(19) Europäisches Patentamt
European Patent Office
Office européen des brevets

(11) EP 2 533 645 B1

(12) **EUROPEAN PATENT SPECIFICATION**

(45) Date of publication and mention of the grant of the patent:
**27.07.2016 Bulletin 2016/30**

(21) Application number: **11742761.7**

(22) Date of filing: **09.02.2011**

(51) Int Cl.:
*A01N 57/00* (2006.01) *A61K 31/66* (2006.01)
*A61K 45/06* (2006.01) *A61K 31/4015* (2006.01)
*A61K 31/445* (2006.01) *A61P 25/28* (2006.01)

(86) International application number:
**PCT/US2011/024256**

(87) International publication number:
**WO 2011/100373 (18.08.2011 Gazette 2011/33)**

# (54) METHODS AND COMPOSITIONS FOR IMPROVING COGNITIVE FUNCTION

VERFAHREN UND ZUSAMMENSETZUNGEN ZUR VERBESSERUNG VON KOGNITIVEN FUNKTIONEN

PROCÉDÉS ET COMPOSITIONS POUR AMÉLIORER LA FONCTION COGNITIVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **09.02.2010 US 302760 P**

(43) Date of publication of application:
**19.12.2012 Bulletin 2012/51**

(73) Proprietor: **The Johns Hopkins University**
**Baltimore, MD 21218 (US)**

(72) Inventors:
- **GALLAGHER, Michela**
  **Baltimore, Maryland 21218 (US)**
- **HABERMAN, Rebecca**
  **Baltimore, Maryland 21218 (US)**
- **KOH, Ming Teng**
  **Baltimore, Maryland 21218 (US)**

(74) Representative: **Evenson, Jane Harriet et al**
**Mathys & Squire LLP**
**The Shard**
**32 London Bridge Street**
**London SE1 9SG (GB)**

(56) References cited:
**WO-A2-2004/051222** **US-A1- 2008 167 291**
**US-A1- 2008 242 698** **US-A1- 2009 176 740**

- **"Abstracts of the 5th World Stroke Conference", Stroke, 1 January 2004 (2004-01-01), XP055060454, Retrieved from the Internet: URL:http://stroke.ahajournals.org/content/35/6/e276.full.pdf#page=1&view=FitH [retrieved on 2013-04-19]**
- **SANSONE M ET AL: "Effects of oxiracetam, physostigmine, and their combination on active and passive avoidance learning in mice", PHARMACOLOGY BIOCHEMISTRY AND BEHAVIOR, ELSEVIER, US, vol. 44, no. 2, 1 February 1993 (1993-02-01), pages 451-455, XP023809221, ISSN: 0091-3057, DOI: 10.1016/0091-3057(93)90490-K [retrieved on 1993-02-01]**
- **HELMSTAEDTER C ET AL: "The effects of levetiracetam on cognition: A non-interventional surveillance study", EPILEPSY AND BEHAVIOR, ACADEMIC PRESS, SAN DIEGO, CA, US, vol. 13, no. 4, 1 November 2008 (2008-11-01), pages 642-649, XP025898342, ISSN: 1525-5050, DOI: 10.1016/J.YEBEH.2008.07.012 [retrieved on 2008-09-10]**
- **CARLA M. YUEDE ET AL: "Anti-dementia drugs and hippocampal-dependent memory in rodents", BEHAVIOURAL PHARMACOLOGY, vol. 18, no. 5-6, 1 September 2007 (2007-09-01), pages 347-363, XP055060403, ISSN: 0955-8810, DOI: 10.1097/FBP.0b013e3282da278d**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the Invention

The disclosure relates to methods and compositions for improving cognitive function by using a combination of a synaptic vesicle protein 2A (SV2A) inhibitor and an acetylcholinesterase inhibitor (AChEI). In particular, it relates to the use of a combination of an SV2A inhibitor and an AChEI in treating central nervous system (CNS) disorders with cognitive impairment in a subject in need or at risk thereof, including, without limitation, subjects having or at risk for age-related cognitive impairment, Mild Cognitive Impairment (MCI), amnestic MCI, Age-Associated Memory Impairment (AAMI), Age Related Cognitive Decline (ARCD), dementia, Alzheimer's Disease(AD), prodromal AD, post traumatic stress disorder (PTSD), schizophrenia, amyotrophic lateral sclerosis (ALS) and cancer-therapy-related cognitive impairment.

### Background of the Invention

Cognitive ability may decline as a normal consequence of aging or as a consequence of a central nervous disorder.

A significant population of elderly adults experiences a decline in cognitive ability that exceeds what is typical in normal aging. Such age-related loss of cognitive function is characterized clinically by progressive loss of memory, cognition, reasoning, and judgment. Mild Cognitive Impairment (MCI), Age-Associated Memory Impairment (AAMI), Age-Related Cognitive Decline (ARCD) or similar clinical groupings are among those related to such age-related loss of cognitive function. According to some estimates, there are more than 16 million people with AAMI in the U.S. alone (Barker et al., 1995), and MCI is estimated to affect 5.5 - 7 million in the U.S. over the age of 65 (Plassman et al., 2008).

Other central nervous system (CNS) disorders, such as dementia, Alzheimer's Disease(AD), prodromal AD, post traumatic stress disorder (PTSD), schizophrenia, amyotrophic lateral sclerosis (ALS), and cancer-therapy-related cognitive impairment, are also associated with cognitive impairment.

There is, therefore, a need for effective treatment for central nervous system (CNS) disorders with cognitive impairment and to improve cognitive function in patients diagnosed with age-related cognitive impairment, MCI, amnestic MCI, AAMI, ARCD, dementia, AD, prodromal AD, PTSD, schizophrenia, amyotrophic lateral sclerosis (ALS), cancer-therapy-related cognitive impairment, and similar central nervous system (CNS) disorders with cognitive impairment or at risk of developing them.

Tacrine hydrochloride ("COGNEX™"), the first FDA approved drug for Alzheimer's disease ("AD") is an AChEI (Cutler et al, 1993). Other examples of clinically used AChEIs include galantamine ("REMINYL™") or rivastigmine ("EXELON™"). These drugs, however, have shown limited success in cognitive improvement in Alzheimer's disease patients and display a use-limiting side effect profile. Another AChEI, donepezil (also known as "ARICEPT™"), appears more effective than tacrine. With donepezil, Alzheimer's disease patients show slight cognitive improvements (Barner et al, 1998; Rogers et al, 1998), but the usefulness of donepezil is limited due to its moderate efficacy and side effects. The long-term therapeutic efficacy of donepezil has also been increasingly questioned (Maggini et al., 2006; Petersen et al., 2006). There is, therefore, a need for more effective treatment for disorders involving cognitive dysfunction and in particular cognitive impairment, in AD patients.

Sansone et al., (1993) Pharmacol. Biochem. Behav., 44(2):451-455 reports on the effects of oxiracetam, physostigmine, and their combination on active and passive avoidance learning in mice.

Helmstaedter et al., (2008) Epilepsy & Behavior, 13(4):642-649 reports on the effects of levetiracetam on cognition based on a non-interventional surveillance study.

### Summary of the Invention

The invention is defined by the claims. Those aspects/ instances of the present disclosure which constitute the invention are defined by the claims.

### Summary of the Disclosure

In accordance with a first aspect of the present disclosure, there is provided a method for treating, or improving cognitive function in, a subject suffering from a central nervous system (CNS) disorder with cognitive impairment, or at risk thereof, the method comprising the step of administering to said subject a therapeutically effective amount of an SV2A inhibitor or a pharmaceutically acceptable salt, hydrate, solvate, or polymorph thereof in combination with a therapeutically effective amount of an AChEI or a pharmaceutically acceptable salt, hydrate, solvate, or polymorph thereof. In some instances of this disclosure, the methods of this disclosure prevent or slow the progression of said CNS

disorder with cognitive impairment in said subject. In other instances of this disclosure, the methods of this disclosure alleviate, ameliorate, or slow the progression, of one or more symptoms associated with said CNS disorder with cognitive impairment in said subject.

In one instance of the disclosure, the SV2A inhibitor and/or the AChEI are administered at doses that are subtherapeutic as compared to the doses at which they are therapeutically effective when administered in the absence of the other.

In some instances of the disclosure, the CNS disorder with cognitive impairment is age-related cognitive impairment, such as Mild Cognitive Impairment (MCI), Age-Associated Memory Impairment (AAMI), Age Related Cognitive Decline (ARCD). In one instance of the disclosure, the MCI is amnestic MCI. In some instances of the disclosure, the CNS disorder with cognitive impairment is dementia, Alzheimer's Disease(AD), prodromal AD, post traumatic stress disorder (PTSD), schizophrenia, amyotrophic lateral sclerosis (ALS), or cancer-therapy-related cognitive impairment. In one instance, the subject that suffers such CNS disorder or such cognitive impairment is a human patient.

The SV2A inhibitor or a pharmaceutically acceptable salt, hydrate, solvate or polymorph thereof that is useful in the methods and compositions of this disclosure include those disclosed in, for example, United States (U.S.) Patent Application 12/580,464, International Patent Application PCT/US2009/005647, U.S. Patent Application 61/105,847, U.S. Patent Application 61/152,631, and U.S. Patent Application 61/175,536. However, any SV2A inhibitor or a pharmaceutically acceptable salt, hydrate, solvate or polymorph thereof may be used in the methods and compositions of the disclosure. In other instances, the SV2A inhibitor is selected from the group of SV2A inhibitors referred to in International Patent Applications WO2010/144712; WO2010/002869; WO2008/132139; WO2007/065595; WO2006/128693; WO2006/128692; WO2005/054188; WO2004/087658; WO2002/094787; WO2001/062726; U.S. Patents 7,465,549; 7,244,747; 5,334,720; 4,696,943; 4,696,942; U.S. Patent Application Publication Numbers 20090312333; 20090018148; 20080081832; 2006258704; and UK Patent Numbers 1,039,113; and 1,309,692 or their pharmaceutically acceptable salts, hydrates, solvates, or polymorphs. In other instances, the SV2A inhibitor is selected from the group consisting of levetiracetam, brivaracetam, and seletracetam or derivatives or analogs or pharmaceutically acceptable salts, hydrates, solvates, or polymorphs thereof. In other instances, the SV2A inhibitor is levetiracetam or a derivative or an analog or a pharmaceutically acceptable salt, hydrate, solvate, or polymorph thereof.

AChEI or a pharmaceutically acceptable salt, hydrate, solvate or polymorph thereof that is useful in the methods and compositions of this disclosure include those disclosed in, for example, International Patent Applications WO2010/057088; WO2009/008769; WO2008/097546; WO2008/074896; WO2008/073452; WO2007/127474; WO2007/107846; WO2006/097588; WO2006/071274; WO2006/070394; WO2006/060082; WO2006/040688; WO2005/092009; WO2005/079789; WO2005/074535; WO2005/072713; WO 2005/042475; WO2005/039580; WO2005/027975; WO2004/084884; WO2004/080393; WO2004/052348; WO2004/037234; WO2004/034963; WO2004/032929; WO2003/101458; WO2003/091220; WO2003/082820; WO2003/082794; WO2003/020289; WO2002/074293; WO2002/032412; WO2001/085145; WO2001/078728; WO2001/066114; WO2001/066096; WO2001/021590; WO2001/000215; WO2000/033840; WO2000/030446; WO2000/023057; WO2000/015205; WO2000/009483; WO2000/007600; WO2000/002549; WO1999/047131; WO1999/008672; WO1999/007359; WO1998/039000; WO1998/030243; WO1997/138993; WO1997/119059; WO1997/038993; WO1997/029750; WO1997/021681; WO1997/013754; WO1997/008146; WO1996/040682; WO1994/029255; WO1994/020476; WO1994/019356; WO1993/116690; WO1993/113100; WO1993/007140; WO1993/003041; WO1993/003034; WO1992/019238; WO1992/017475; WO1991/003467; and WO1988/008708; U.S. Patent No. 7,846,930; 7,732,162; 7,635,709; 7,378,425; 6,495,700; 6,479,523; 6,372,760; 6,245,911; 6,140,321; 5,985,864; 5,965,571; 5,965,569; 5,750,542; 5,744,476; 5,693,668; 5,668,117; 5,663,448; 5,622,976; 5,603,176; 5,602,176; 5,574,046; 5,455,245; 5,391,553; 5,389,629; 5,364,864; 5,338,548; 5,302,593; 5,300,517; 5,288,758; 5,246,947; 5,231,093; 5,187,165; 5,166,181; 5,106,856; 5,102,891; 5,100,901; 4,950,658; 4,948,807; 4,948,807; 4,948,807; 4,914,102; 4,895,841; 4,895,841; 4,816,456; 4,663,318; 4,663,318; 4,663,318; and 2,701,225; Japanese Patent Application Nos. 4-216704 and 4-187674, Canadian Patent No. 2,180,703, European Patent Application Publication Nos. 298202; 236684; 409676; 411534; 468187; 477903; 481429; 487071; 611769; 703901; 1050303; 2018874; and 2260839; U.S. Publication Nos. 20010036949; 20020119963; 20030078252; 2003069289; 20040082644; 20050245504; 20050124642; 20060052428; 20070275959; 20080103105; 20080261950; 20090124659; 20100152108; 20100227852; and 20100311697. However, any AChEI or a pharmaceutically acceptable salt, hydrate, solvate or polymorph may be used in the methods and compositions of this disclosure.

In other instances of the disclosure, the SV2A inhibitor or a pharmaceutically acceptable salt, hydrate, solvate or polymorph thereof can be administered at doses as disclosed, for example, in U.S. Patent Application 12/580,464, International Patent Application PCT/US2009/005647, U.S. Patent Application 61/105,847, U.S. Patent Application 61/152,631, and U.S. Patent Application 61/175,536. In other instances of the disclosure, the SV2A inhibitor or a pharmaceutically acceptable salt, hydrate, solvate or polymorph thereof is administered every 12 or 24 hours at a daily dose of about 0.001 mg/kg to 5 mg/kg. In other instances of the disclosure, the SV2A inhibitor or a pharmaceutically acceptable salt, hydrate, solvate or polymorph thereof is administered every 12 or 24 hours at a daily dose of about 0.1 to 5 mg/kg,

or about 1 to 2 mg/kg, or about 0.1 to 0.2 mg/kg, or about 0.01 to 2.5 mg/kg, or about 0.1 to 2.5 mg/kg, or about 0.4 to 2.5 mg/kg, or about 0.6 to 1.8 mg/kg, or about 0.04 to 2.5 mg/kg, or about 0.06 to 1.8 mg/kg, or about 0.01 to 1 mg/kg, or about 0.001 to 1 mg/kg, or about 0.5 mg/kg to 5 mg/kg, or about 0.05 mg/kg to 0.5 mg/kg. In some instances, a subtherapeutic amount of the SV2A inhibitor or a pharmaceutically acceptable salt, hydrate, solvate or polymorph thereof is used. Such subtherapeutic amount, may be, for example, a daily dose of less than 5 mg/kg, less than 2.5 mg/kg, less than 2 mg/kg, less than 1.5 mg/kg, less than 1 mg/kg, less than 0.5 mg/kg, less than 0.1 mg/kg, less than 0.05 mg/kg, less than 0.01 mg/kg, less than 0.005 mg/kg, or less than 0.001 mg/kg, administered every 12 or 24 hours.

In other instances of the disclosure, the AChEI or a pharmaceutically acceptable salt, hydrate, solvate or polymorph thereof is administered every 12 or 24 hours at a daily dose of about 0.1 to 10 mg. In some instances, a subtherapeutic amount of the AChEI or a pharmaceutically acceptable salt, hydrate, solvate or polymorph thereof is administered. In some instances, the subtherapeutic amount of the AChEI administered is a daily dose of less than about 10 mg, less than about 5 mg, less than about 2 mg, less than about 1 mg, less than about 0.5 mg.

In other instances of the disclosure, the SV2A inhibitor and the AChEI, or their pharmaceutically acceptable salts, hydrates, solvates or polymorphs are administered simultaneously, or sequentially, or in a single formulation or in separate formulations packaged together. In other instances, the SV2A inhibitor and the AChEI, or their pharmaceutically acceptable salts, hydrates, solvates or polymorphs are administered via different routes. As used herein, "combination" includes administration by any of these formulations or routes of administration.

In some instances of the disclosure, the combined treatment has a longer or improved therapeutic effect in the subject than is attained by administering the AChEI or a pharmaceutically acceptable salt, hydrate, solvate or polymorph thereof in the absence of the SV2A inhibitor or a pharmaceutically acceptable salt, solvate, hydrate, or polymorph thereof by at least about 1.5x, or 2.0x, or 2.5x, or 3.0x, or 3.5x, or 4.0x, or 4.5x, or 5.0x, or 5.5x, or 6.0x, or 6.5x, or 7.0x, or 7.5x, or 8.0x, or 8.5x, or 9.0x, or 9.5x, or 10x, or greater than about 10x.

In other instances of the disclosure, the combined treatment has a longer or improved therapeutic effect in the subject than is attained by administering the SV2A inhibitor or a pharmaceutically acceptable salt, hydrate, solvate or polymorph thereof in the absence of the AChEI or a pharmaceutically acceptable salt, hydrate, solvate or polymorph thereof by at least about 1.5x, or 2.0x, or 2.5x, or 3.0x, or 3.5x, or 4.0x, or 4.5x, or 5.0x, or 5.5x, or 6.0x, or 6.5x, or 7.0x, or 7.5x, or 8.0x, or 8.5x, or 9.0x, or 9.5x, or 10x, or greater than about 10x.

In accordance with another aspect of the present disclosure, there is provided a method of increasing the therapeutic index of an AChEI or a pharmaceutically acceptable salt, hydrate, solvate or polymorph thereof in a method of treating a central nervous system (CNS) disorder with cognitive impairment in a subject in need or at risk thereof, comprising administering an SV2A inhibitor or a pharmaceutically acceptable salt, hydrate, solvate or polymorph thereof in combination with an AChEI or a pharmaceutically acceptable salt, hydrate, solvate or polymorph thereof to said subject.

In some instances, the increase in the therapeutic index of the AChEI or a pharmaceutically acceptable salt, hydrate, solvate or polymorph thereof is greater than the therapeutic index of the AChEI when administered in the absence of the SV2A inhibitor or a pharmaceutically acceptable salt, hydrate, solvate or polymorph thereof or a pharmaceutically acceptable salt, hydrate, solvate or polymorph of the AChEI when administered in the absence of the SV2A inhibitor or a pharmaceutically acceptable salt, hydrate, solvate or polymorph thereof by at least about 1.5x, or 2.0x, or 2.5x, or 3.0x, or 3.5x, or 4.0x, or 4.5x, or 5.0x, or 5.5x, or 6.0x, or 6.5x, or 7.0x, or 7.5x, or 8.0x, or 8.5x, or 9.0x, or 9.5x, or 10x, or greater than about 10x.

In accordance with another aspect of the present disclosure, there is provided a method of increasing the therapeutic index of an SV2A inhibitor or a pharmaceutically acceptable salt, hydrate, solvate or polymorph thereof in a method of treating a central nervous system (CNS) disorder with cognitive impairment in a subject in need or at risk thereof, comprising administering an SV2A inhibitor or a pharmaceutically acceptable salt, hydrate, solvate or polymorph thereof in combination with an AChEI or a pharmaceutically acceptable salt, hydrate, solvate or polymorph thereof to said subject.

In some instances, the increase in the therapeutic index of the SV2A inhibitor or a pharmaceutically acceptable salt, hydrate, solvate or polymorph thereof is greater than the therapeutic index of the SV2A inhibitor , hydrate, solvate or polymorph when administered in the absence of the AChEI or a pharmaceutically acceptable salt, hydrate, solvate or polymorph thereof or a pharmaceutically acceptable salt, hydrate, solvate or polymorph of the SV2A inhibitor when administered in the absence of the AChEI or a pharmaceutically acceptable salt, hydrate, solvate or polymorph thereof by at least about 1.5x, or 2.0x, or 2.5x, or 3.0x, or 3.5x, or 4.0x, or 4.5x, or 5.0x, or 5.5x, or 6.0x, or 6.5x, or 7.0x, or 7.5x, or 8.0x, or 8.5x, or 9.0x, or 9.5x, or 10x, or greater than about 10x.

In accordance with another aspect of this disclosure, there is provided a pharmaceutical composition for treating, or improving cognitive function in, a subject suffering from a central nervous system (CNS) disorder with cognitive impairment, or at risk thereof, the composition comprising an SV2A inhibitor and an AChEI or their pharmaceutically acceptable salts, hydrates, solvates or polymorphs. In some instances, the composition is in a solid form. In some instances, the composition is in a liquid form. In some instances, the composition is in an aqueous solution. In some instances, the composition is in a suspension form. In some instances, the composition is in a sustained release form,

or a controlled release form, or a delayed release form, or an extended release form. In some instances, the composition is in a unit dosage form. In other instances, the two components of the compositions are in separate delivery forms packaged together.

In some instances of this disclosure, the composition comprises levetiracetam or a derivative or an analog or a pharmaceutically acceptable salt, hydrate, solvate or polymorph thereof and donepezil a derivative or an analog or a pharmaceutically acceptable salt, hydrate, solvate or polymorph thereof.

In some instances of this disclosure, the SV2A inhibitor or a pharmaceutically acceptable salt, hydrate, solvate or polymorph thereof in the composition is present in an amount of 0.07 - 350 mg, or 50 - 250 mg, or 3 - 50 mg. In some instances, the SV2A inhibitor or a pharmaceutically acceptable salt, hydrate, solvate or polymorph thereof is present in an amount less than 350 mg, less than 250 mg, less than 200 mg, less than 150 mg, less than 100 mg, less than 50 mg, less than 10 mg, less than 5 mg, less than 1 mg, less than 0.5 mg, less than 0.1 mg, or less than 0.07 mg.

In some instances of this disclosure, the AChEI or a pharmaceutically acceptable salt, hydrate, solvate or polymorph thereof is present in an amount of 0.1 mg to 10 mg. In some instances, the AChEI or a pharmaceutically acceptable salt, hydrate, solvate or polymorph thereof is present in an amount of less than 10 mg, less than 5 mg, less than 2 mg, less than 1 mg, or less than 0.5 mg.

## Brief Description of the Drawings

**FIG. 1** depicts the effects of administering levetiracetam on the spatial memory retention of ten aged-impaired rats (AI) in an eight-arm Radial Arm Maze (RAM) test. Six treatment conditions are employed: vehicle control, levetiracetam (1.25 mg/kg/day), levetiracetam (2.5 mg/kg/day), levetiracetam (5 mg/kg/day), levetiracetam (10 mg/kg/day) and levetiracetam (20 mg/kg/day). In the RAM task used, there is a one-hour delay between presentation of a subset of arms (5 arms available and 3 arms blocked) and completion of the eight-arm win-shift task (eight arms available). Rats are pre-treated 30-40 minutes before daily trials with a one-time drug/control treatment. The number of errors made by the rats during the retention phase is used as a measure of spatial memory retention. Errors are defined as instances when rats enter an arm from which food has already been retrieved in the pre-delay component of the trial or when rats re-visit an arm in the post-delay session that had already been visited. Paired t-tests are used to compare the number of errors between different doses of levetiracetam and vehicle control.

**FIG. 2** depicts the effects of administering donepezil only and administering a combination of donepezil and levetiracetam on the spatial memory retention of eight aged-impaired rats (AI) in an eight-arm Radial Arm Maze (RAM) test. Four treatment conditions are employed: vehicle control, donepezil only (1 mg/kg/day), donepezil (2 mg/kg/day), and a combination of donepezil (1 mg/kg/day) and levetiracetam (2.5 mg/kg/day). In the RAM task used, there is a two-hour delay between presentation of a subset of arms (5 arms available and 3 arms blocked) and completion of the eight-arm win-shift task (eight arms available). Rats are pre-treated 30 - 40 minutes before daily trials with a one-time drug/control treatment. The number of errors made by the rats during the retention phase is used as a measure of spatial memory retention. Errors are defined as instances when rats enter an arm from which food has already been retrieved in the pre-delay component of the trial or when rats re-visit an arm in the post-delay session that have already been visited. Paired t-tests are used to compare the number of errors between different doses of drug treatment and vehicle control. Relative to vehicle control treatment, a combination of donepezil (1 mg/kg/day) and levetiracetam (2.5 mg/kg/day) significantly improves memory performance ($t(7) = 2.16$, $p = 0.034$).

**FIG. 3** depicts the experimental design of the human trials for levetiracetam treatment.

**FIG. 4A** depicts the average activity in the left CA3 of aMCI subjects with placebo treatment and age-matched control subjects with placebo treatment during the presentation of lure stimuli that the subject correctly identified as "similar."

**FIG. 4B** depicts the average activity in the left CA3 of aMCI subjects with placebo treatment or levetiracetam treatment (125 mg twice a day for two weeks) during the presentation of lure stimuli that the subject correctly identified as "similar."

**FIG. 4C** is a table of the data represented in **FIGS. 4A** and **4B.**

**FIG. 5A** depicts the average activity in the left entorhinal cortex of age-matched control subjects with placebo treatment and aMCI subjects with placebo treatment during the presentation of lure stimuli that the subject correctly

identified as "similar."

**FIG. 5B** depicts the average activity in the left entorhinal cortex of the same aMCI subjects with placebo treatment or levetiracetam treatment (125 mg twice a day for two weeks) during the presentation of lure stimuli that the subject correctly identified as "similar."

**FIG. 5C** is a table of the data represented in **FIGS. 5A** and **5B.**

**FIG. 6A** depicts an example of the sequence of images shown to subjects in the explicit 3-alternative forced choice task described in Example 2.

**FIG. 6B** shows sample pairs of similar ("lure") images.

**FIG. 7** shows the difference between the aMCI (placebo) subjects and age-matched control (placebo) subjects in their performance of the explicit 3-alternative forced choice task described in Example 2. Each bar represents the proportion of the subject responses (old, similar, or new) when presented with a lure image.

**FIG. 8** shows the difference between the same aMCI subjects with placebo treatment or with levetiracetam treatment (125 mg twice a day for two weeks) in their performance of the explicit 3-alternative forced choice task described in Example 2. Each bar represents the proportion of the subjects responses (old, similar, or new) when presented with a lure image.

**FIG. 9** is a table of the data represented in **FIGS. 7** and **8.**

**FIG. 10A** shows the difference between the age-matched control (placebo) subjects and the aMCI subjects treated with placebo or with levetiracetam (125 mg twice a day for two weeks) in their performance of the Bushke Selective reminding Test - Delayed Recall.

**FIG. 10B** is a table of the data represented in **FIG. 10A.**

**FIG. 11A** shows the difference between the control (placebo) subjects and the aMCI subjects treated with placebo or with levetiracetam (125 mg twice a day for two weeks) in their performance of the Benton Visual Retention Test.

**FIG. 11B** is a table of the data represented in **FIG. 11A.**

**FIG. 12A** shows the difference between the control (placebo) subjects and the aMCI subjects treated with placebo or with levetiracetam (125 mg twice a day for two weeks) in their performance of the Verbal Paired Associates Test - Recognition.

**FIG. 12B** is a table of the data represented in **FIG. 12A.**

**FIG. 13A** shows the difference between the control (placebo) subjects and the aMCI subjects treated with placebo or with levetiracetam (125 mg twice a day for two weeks) in their performance of the Verbal Paired Associates Test - Delayed Recall.

**FIG. 13B** is a table of the data represented in FIG. **13A.**

**FIG. 14A** is a table showing the subject selection process for the human levetiracetam trial described in Example 2.

**FIG. 14B** is a table showing the characteristics of the subjects selected for the human levetiracetam trial described in Example 2.

## Detailed Description of the Disclosure

Unless otherwise defined herein, scientific and technical terms used in this application shall have the meanings that are commonly understood by those of ordinary skill in the art. Generally, nomenclature used in connection with, and techniques of, cell and tissue culture, molecular biology, cell and cancer biology, neurobiology, neurochemistry, virology, immunology, microbiology, pharmacology, genetics and protein and nucleic acid chemistry, described herein,

are those well known and commonly used in the art.

The methods and techniques of the present disclosure are generally performed, unless otherwise indicated, according to conventional methods well known in the art and as described in various general and more specific references that are cited and discussed throughout this specification. See, e.g. "Principles of Neural Science", McGraw-Hill Medical, New York, N.Y. (2000); Motulsky, "Intuitive Biostatistics", Oxford University Press, Inc. (1995); Lodish et al., "Molecular Cell Biology, 4th ed.", W. H. Freeman & Co., New York (2000); Griffiths et al., "Introduction to Genetic Analysis, 7th ed.", W. H. Freeman & Co., N.Y. (1999); Gilbert et al., "Developmental Biology, 6th ed.", Sinauer Associates, Inc., Sunderland, MA (2000).

Chemistry terms used herein are used according to conventional usage in the art, as exemplified by "The McGraw-Hill Dictionary of Chemical Terms", Parker S., Ed., McGraw-Hill, San Francisco, C.A. (1985).

. In case of conflict, the present specification, including its specific definitions, will control.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

The singular forms "a," "an," and "the" include the plurals unless the context clearly dictates otherwise.

The term "including" is used to mean "including but not limited to". "Including" and "including but not limited to" are used interchangeably.

The term "agent" is used herein to denote a chemical compound (such as an organic or inorganic compound, a mixture of chemical compounds), a biological macromolecule (such as a nucleic acid, an antibody, including parts thereof as well as humanized, chimeric and human antibodies and monoclonal antibodies, a protein or portion thereof, e.g., a peptide, a lipid, a carbohydrate), or an extract made from biological materials such as bacteria, plants, fungi, or animal (particularly mammalian) cells or tissues. Agents include, for example, agents which are known with respect to structure, and those which are not known with respect to structure. The SV2A inhibitory activity or the AChEI activity of such agents may render them suitable as "therapeutic agents" in the methods and compositions of this disclosure.

A "patient", "subject", or "individual" are used interchangeably and refer to either a human or a non-human animal. These terms include mammals, such as humans, primates, livestock animals (including bovines, porcines, etc.), companion animals (e.g., canines, felines, etc.) and rodents (e.g., mice and rats).

"Cognitive function" or "cognitive status" refers to any higher order intellectual brain process or brain state, respectively, involved in learning and/or memory including, but not limited to, attention, information acquisition, information processing, working memory, short-term memory, long-term memory, anterograde memory, retrograde memory, memory retrieval, discrimination learning, decision-making, inhibitory response control, attentional set-shifting, delayed reinforcement learning, reversal learning, the temporal integration of voluntary behavior, and expressing an interest in one's surroundings and self-care.

In humans, cognitive function may be measured, for example and without limitation, by the clinical global impression of change scale (CIBIC-plus scale); the Mini Mental State Exam (MMSE); the Neuropsychiatric Inventory (NPI); the Clinical Dementia Rating Scale (CDR); the Cambridge Neuropsychological Test Automated Battery (CANTAB); the Sandoz Clinical Assessment-Geriatric (SCAG), the Buschke Selective Reminding Test (Buschke and Fuld, 1974); the Verbal Paired Associates subtest; the Logical Memory subtest; the Visual Reproduction subtest of the Wechsler Memory Scale-Revised (WMS-R) (Wechsler, 1997); the Benton Visual Retention Test, or the explicit 3-alternative forced choice task. *See* Folstein et al., J Psychiatric Res 12: 189-98, (1975); Robbins et al., Dementia 5: 266-81, (1994); Rey, L'examen clinique en psychologie, (1964); Kluger et al., J Geriatr Psychiatry Neurol 12:168-79, (1999); Marquis et al., 2002 and Masur et al., 1994.

In animal model systems, cognitive function may be measured in various conventional ways known in the art, including using a Morris Water Maze (MWM), Barnes circular maze, elevated radial arm maze, T maze or any other mazes in which the animals use spatial information. Other tests known in the art may also be used to assess cognitive function, such as novel object recognition and odor recognition tasks.

Cognitive function may also be measured using imaging techniques such as Positron Emission Tomography (PET), functional magnetic resonance imaging (fMRI), Single Photon Emission Computed Tomography (SPECT), or any other imaging technique that allows one to measure brain function. In animals, cognitive function may also be measured with electrophysiological techniques.

"Promoting" cognitive function refers to affecting impaired cognitive function so that it more closely resembles the function of a normal, unimpaired subject. Cognitive function may be promoted to any detectable degree, but in humans preferably is promoted sufficiently to allow an impaired subject to carry out daily activities of normal life at the same level of proficiency as a normal, unimpaired subject.

In some cases, "promoting" cognitive function in a subject affected by age-related cognitive refers to affecting impaired cognitive function so that it more closely resembles the function of an aged-matched normal, unimpaired subject, or the function of a young adult subject. Cognitive function of that subject may be promoted to any detectable degree, but in humans preferably is promoted sufficiently to allow an impaired subject to carry out daily activities of normal life

at the same level of proficiency as an aged-matched normal, unimpaired subject or as a young adult subject.

"Preserving" cognitive function refers to affecting normal or impaired cognitive function such that it does not decline or does not fall below that observed in the subject upon first presentation or diagnosis, or delays such decline.

"Improving" cognitive function includes promoting cognitive function and/or preserving cognitive function in a subject.

"Cognitive impairment" refers to cognitive function in subjects that is not as robust as that expected in a normal, unimpaired subject. In some cases, cognitive function is reduced by about 5%, about 10%, about 30%, or more, compared to cognitive function expected in a normal, unimpairmed subject. In some cases, "cognitive impairment" in subjects affected by aged-related cognitive impairment refers to cognitive function in subjects that is not as robust as that expected in an aged-matched normal, unimpaired subject, or the function of a young adult subject (*i.e.* subjects with mean scores for a given age in a cognitive test).

"Age-related cognitive impairment" refers to cognitive impairment in aged subjects, wherein their cognitive function is not as robust as that expected in an age-matched normal subject or as that expected in young adult subjects. In some cases, cognitive function is reduced by about 5%, about 10%, about 30%, or more, compared to cognitive function expected in an age-matched normal subject. In some cases, cognitive function is as expected in an age-matched normal subject, but reduced by about 5%, about 10%, about 30%, about 50% or more, compared to cognitive function expected in a young adult subject. Age-related impaired cognitive function may be associated with Mild Cognitive Impairment (MCI) (including amestic MCI and non-amnestic MCI), Age-Associated Memory Impairment (AAMI), and Age-related Cognitive Decline (ARCD).

"Cognitive impairment" associated with AD or related to AD or in AD refers to cognitive function in subjects that is not as robust as that expected in subjects who have not been diagnosed AD using conventional methodologies and standards.

"Mild Cognitive Impairment" or "MCI" refers to a condition characterized by isolated memory impairment unaccompanied other cognitive abnormalities and relatively normal functional abilities. One set of criteria for a clinical characterization of MCI specifies the following characteristics: (1) memory complaint (as reported by patient, informant, or physician), (2) normal activities of daily living (ADLs), (3) normal global cognitive function, (4) abnormal memory for age (defined as scoring more than 1.5 standard deviations below the mean for a given age), and (5) absence of indicators of dementia (as defined by DSM-IV guidelines). Petersen et al., Srch. Neurol. 56: 303-308 (1999); Petersen, "Mild cognitive impairment: Aging to Alzheimer's Disease." Oxford University Press, N.Y. (2003).

Diagnosis of MCI usually entails an objective assessment of cognitive impairment, which can be garnered through the use of well-established neuropsychological tests, including the Mini Mental State Examination (MMSE), the Cambridge Neuropsychological Test Automated Battery (CANTAB) and individual tests such as Rey Auditory Verbal Learning Test (AVLT), Logical Memory Subtest of the revised Wechsler Memory Scale (WMS-R) and the New York University (NYU) Paragraph Recall Test. *See* Folstein et al., J Psychiatric Res 12: 189-98 (1975); Robbins et al., Dementia 5: 266-81 (1994); Kluger et al., J Geriatric Psychiatry Neurol 12:168-79 (1999).

"Age-Associate Memory Impairment (AAMI)" refers to a decline in memory due to aging. A patient may be considered to have AAMI if he or she is at least 50 years old and meets all of the following criteria: a) The patient has noticed a decline in memory performance, b) The patient performs worse on a standard test of memory compared to young adults, c) All other obvious causes of memory decline, except normal aging, have been ruled out (in other words, the memory decline cannot be attributed to other causes such as a recent heart attack or head injury, depression, adverse reactions to medication, Alzheimer's disease, etc.).

"Age-Related Cognitive Decline (ARCD)" refers to declines in memory and cognitive abilities that are a normal consequence of aging in humans (e.g., Craik & Salthouse, 1992). This is also true in virtually all mammalian species. Age-Associated Memory Impairment refers to older persons with objective memory declines relative to their younger years, but cognitive functioning that is normal relative to their age peers (Crook et al., 1986). Age-Consistent Memory Decline is a less pejorative label which emphasizes that these are normal developmental changes (Crook, 1993; Larrabee, 1996), are not pathophysiological (Smith et al., 1991), and rarely progress to overt dementia (Youngjohn & Crook, 1993). The DSM-IV (1994) has codified the diagnostic classification of ARCD.

Alzheimer's disease (AD) is characterized by memory deficits in its early phase. Later symptoms include impaired judgment, disorientation, confusion, behavior changes, trouble speaking, and motor deficits. Histologically, AD is characterized by beta-amyloid plaques and tangles of protein tau.

Vascular dementia is caused by strokes. Symptoms overlap with those of AD, but without the focus on memory impairment.

Dementia with Lewy bodies is characterized by abnormal deposits of alpha-synuclein that form inside neurons in the brain. Cognitive impairment may be similar to AD, including impairments in memory and judgment and behavior changes.

Frontotemporal dementia is characterized by gliosis, neuronal loss, superficial spongiform degeneration in the frontal cortex and/or anterior temporal lobes, and Picks' bodies. Symptoms include changes in personality and behavior,

including a decline in social skills and language expression/comprehension.

"Post traumatic stress disorder (PTSD)" refers to an anxiety disorder characterized by an immediate or delayed response to a catastrophic event, characterized by re-experiencing the trauma, psychic numbing or avoidance of stimuli associated with the trauma, and increased arousal. Re-experiencing phenomena include intrusive memories, flashbacks, nightmares, and psychological or physiological distress in response to trauma reminders. Such responses produce anxiety and can have significant impact, both chronic and acute, on a patient's quality of life and physical and emotional health. PTSD is also associated with impaired cognitive performance, and older individuals with PTSD have greater decline in cognitive performance relative to control patients.

"Schizophrenia" refers to a chronic debilitating disorder, characterized by a spectrum of psychopathology, including positive symptoms such as aberrant or distorted mental representations (*e.g.*, hallucinations, delusions), negative symptoms characterized by diminution of motivation and adaptive goal-directed action (*e.g.*, anhedonia, affective flattening, avolition), and cognitive impairment. While abnormalities in the brain are proposed to underlie the full spectrum of psychopathology in schizophrenia, currently available antipsychotics are largely ineffective in treating cognitive impairments in patients.

"Amyotrophic lateral sclerosis," also known as ALS, refers to a progressive, fatal, neurodegenerative disease characterized by a degeneration of motor neurons, the nerve cells in the central nervous system that control voluntary muscle movement. ALS is also characterized by neuronal degeneration in the entorhinal cortex and hippocampus, memory deficits, and neuronal hyperexcitability in different brain areas such as the cortex.

"Cancer-therapy-related cognitive impairment" refers to cognitive impairment that develops in subjects that are treated with cancer therapies such as chemotherapy and radiation. Cytotoxicity and other adverse side-effects on the brain of cancer therapies result in cognitive impairment in such functions as memory, learning and attention.

"Treating" a condition or patient refers to taking steps to obtain beneficial or desired results, including clinical results. Beneficial or desired clinical results include, but are not limited to, preventing or slowing the progression of the disease or disorder, or alleviation, amelioration, or slowing the progression, of one or more symptoms associated with CNS disorders with cognitive impairment, such as age-related cognitive impairment, Mild Cognitive Impairment (MCI), amnestic MCI, dementia, Alzheimer's Disease (AD), prodromal AD, PTSD, schizophrenia, amyotrophic lateral sclerosis (ALS), or cancer therapy-related cognitive impairment. Treating age-related cognitive impairment further comprises slowing the conversion of age-related cognitive impairment (including, but not limited to MCI, ARCD and AAMI) into dementia (*e.g.*, AD).

"Treating cognitive impairment" refers to taking steps to improve cognitive function in a subject with cognitive impairment so that the subject's performance in one or more cognitive tests is improved to any detectable degree, or is prevented from further decline. Preferably, that subject's cognitive function, after treatment of cognitive impairment, more closely resembles the function of a normal, unimpaired subject. Treatment of cognitive impairment in humans may improve cognitive function to any detectable degree, but is preferably improved sufficiently to allow the impaired subject to carry out daily activities of normal life at the same level of proficiency as a normal, unimpaired subject. In some cases, "treating cognitive impairment" refers to taking steps to improve cognitive function in a subject with cognitive impairment so that the subject's performance in one or more cognitive tests is improved to any detectable degree, or is prevented from further decline. Preferably, that subject's cognitive function, after treatment of cognitive impairment, more closely resembles the function of a normal, unimpaired subject. In some cases, "treating cognitive impairment" in a subject affecting by age-related cognitive impairment refers to takings steps to improve cognitive function in the subject so that the subject's cognitive function, after treatment of cognitive impairment, more closely resembles the function of an age-matched normal, unimpaired subject, or the function of a young adult subject.

"Administering" or "administration of" a substance, a compound or an agent to a subject can be carried out using one of a variety of methods known to those skilled in the art. For example, a compound or an agent can be administered, intravenously, arterially, intradermally, intramuscularly, intraperitonealy, intravenously, subcutaneously, ocularly, sublingually, orally (by ingestion), intranasally (by inhalation), intraspinally, intracerebrally, and transdermally (by absorbtion, e.g., through a skin duct). A compound or agent can also appropriately be introduced by rechargeable or biodegradable polymeric devices or other devices, e.g., patches and pumps, or formulations, which provide for the extended, slow, or controlled release of the compound or agent. Administering can also be performed, for example, once, a plurality of times, and/or over one or more extended periods. In some aspects, the administration includes both direct administration, including self-administration, and indirect administration, including the act of prescribing a drug. For example, as used herein, a physician who instructs a patient to self-administer a drug, or to have the drug administered by another and/or who provides a patient with a prescription for a drug is administering the drug to the patient.

Appropriate methods of administering a substance, a compound or an agent to a subject will also depend, for example, on the age of the subject, whether the subject is active or inactive at the time of administering, whether the subject is cognitively impaired at the time of administering, the extent of the impairment, and the chemical and biological properties of the compound or agent (e.g. solubility, digestibility, bioavailability, stability and toxicity). In some instances, a compound or an agent is administered orally, e.g., to a subject by ingestion, or intravenously, e.g., to a subject by

injection. In some instances, the orally administered compound or agent is in an extended release or slow release formulation, or administered using a device for such slow or extended release.

As used herein, administration of an SV2A inhibitor and an AChEI or pharmaceutically acceptable salts, hydrates, solvates, or polymorphs thereof "in combination" or "together" includes simultaneous administration and/or administration at different times, such as sequential administration. It also includes administration in a single formulation or in separate formulation packaged together.

The term "simultaneous administration," as used herein, means that the SV2A inhibitor and the AChEI, or their pharmaceutically acceptable salts, hydrates, solvates, or polymorphs, are administered with a time separation of no more than about 15 minutes, and in some instances no more than about 10 minutes. When the drugs are administered simultaneously, the SV2A inhibitor and the AChEI, or their salts, hydrates, solvates, or polymorphs, may be contained in the same dosage (e.g., a unit dosage form comprising both the SV2A inhibitor and the AChEI) or in discrete dosages (e.g., the SV2A inhibitor or its salt, hydrate, solvate, or polymorph is contained in one dosage form and the AChEI or its salt, hydrate, solvate, or polymorph is contained in another dosage form).

The term "sequential administration" as used herein means that the SV2A inhibitor and the AChEI, or their pharmaceutically acceptable salts, hydrates, solvates, polymorphs, are administered with a time separation of more than about 15 minutes, and in some instances more than about one hour, or up to 12 hours. Either the SV2A inhibitor or the AChEI may be administered first. The SV2A inhibitor and the AChEI, or their salts, hydrates, solvents, or polymorphs, for sequential administration may be contained in discrete dosage forms, optionally contained in the same container or package.

A "therapeutically effective amount" of a drug or agent is an amount of a drug or an agent that, when administered to a subject will have the intended therapeutic effect, e.g. improving cognitive function in a subject, e.g., a patient having a CNS disorder with cognitive impairment. The full therapeutic effect does not necessarily occur by administration of one dose, and may occur only after administration of a series of doses. Thus, a therapeutically effective amount may be administered in one or more administrations. The precise effective amount needed for a subject will depend upon, for example, the subject's size, health and age, the nature and extent of the cognitive impairment, and the therapeutics or combination of therapeutics selected for administration, and the mode of administration. The skilled worker can readily determine the effective amount for a given situation by routine experimentation.

"Subtherapeutic amount" refers to an amount administered of an agent or compound of the disclosure that is less than the therapeutic amount, that is, less than the amount normally used when said agent or compound is administered alone (i.e., individually and in the absence of other therapeutic agents or compounds) to treat disorders involving cognitive dysfunction.

"Analog" is used herein to refer to a compound which functionally resembles another chemical entity, but does not share the identical chemical structure. For example, an analog is sufficiently similar to a base or parent compound such that it can substitute for the base compound in therapeutic applications, despite minor structural differences.

"Derivative" is used herein to refer to the chemical modification of a compound. Chemical modifications of a compound can include, for example, replacement of hydrogen by an alkyl, acyl, or amino group. Many other modifications are also possible.

The term "prodrug" is art-recognized and is intended to encompass compounds or agents which, under physiological conditions, are converted into an SV2A inhibitor or an AChEI. A common method for making a prodrug is to select moieties which are hydrolyzed or metabolized under physiological conditions to provide the desired compound or agent. In other instances, the prodrug is converted by an enzymatic activity of the host animal to an inhibitor of SV2A or an AChEI.

"Pharmaceutically acceptable salts" is used herein to refer to an agent or a compound according to the disclosure that is a therapeutically active, non-toxic base and acid salt form of the agents and compounds of the disclosure.

### Description of Methods of the Disclosure

The methods of this disclosure comprise administration of an SV2A inhibitor or a pharmaceutically acceptable salt thereof in combination with administration of an AChEI or a pharmaceutically acceptable salt thereof. The agents or compounds of the SV2A inhibitor or the AChEI and their pharmaceutically acceptable salts also include hydrates, solvates, polymorphs, and prodrugs of those agents, compounds, and salts.

### Methods of Assessing Cognitive Impairment

Animal models serve as an important resource for developing and evaluating treatments for CNS disorders with cognitive impairment. Features that characterize cognitive impairment in animal models typically extend to cognitive impairment in humans. Efficacy in such animal models is, thus, expected to be predictive of efficacy in humans. The extent of cognitive impairment in an animal model for a CNS disorder, and the efficacy of a method of treatment for said

CNS disorder may be tested and confirmed with the use of a variety of cognitive tests.

A Radial Arm Maze (RAM) behavioral task is one example of a cognitive test, specifically testing spacial memory (Chappell et al. Neuropharmacology 37: 481-487, 1998). The RAM apparatus consists of, *e.g.,* eight equidistantly spaced arms. A maze arm projects from each facet of a center platform. A food well is located at the distal end of each arm. Food is used as a reward. Blocks can be positioned to prevent entry to any arm. Numerous extra maze cues surrounding the apparatus may also be provided. After habituation and training phases, spatial memory of the subjects may be tested in the RAM under control or test compound-treated conditions. As a part of the test, subjects are pretreated before trials with a vehicle control or one of a range of dosages of the test compound. At the beginning of each trial, a subset of the arms of the eight-arm maze is blocked. Subjects are allowed to obtain food on the unblocked arms to which access is permitted during this initial "information phase" of the trial. Subjects are then removed from the maze for a delay period, *e.g.,* a 60 second delay, a 15 minute delay, a one-hour delay, a two-hour delay, a six hour delay, a 24 hour delay, or longer) between the information phase and the subsequent "retention test," during which the barriers on the maze are removed, thus allowing access to all eight arms. After the delay period, subjects are placed back onto the center platform (with the barriers to the previously blocked arms removed) and allowed to obtain the remaining food rewards during this retention test phase of the trial. The identity and configuration of the blocked arms vary across trials. The number of "errors" the subjects make during the retention test phase is tracked. An error occurs in the trial if the subjects entered an arm from which food had already been retrieved in the pre-delay component of the trial, or if it re-visits an arm in the post-delay session that had already been visited. A fewer number of errors would indicate better spatial memory. The number of errors made by the test subject, under various test compound treatment regimes, can then be compared for efficacy of the test compound in treating CNS disorders with cognitive impairment.

Another cognitive test that may be used to assess the effects of a test compound on the cognitive impairment of a CNS disorder model animal is the Morris water maze. A water maze is a pool surrounded with a novel set of patterns relative to the maze. The training protocol for the water maze may be based on a modified water maze task that has been shown to be hippocampal-dependent (de Hoz et al., Eur. J. Neurosci., 22:745-54, 2005; Steele and Morris, Hippocampus 9:118-36, 1999). The subject is trained to locate a submerged escape platform hidden underneath the surface of the pool. During the training trial, a subject is released in the maze (pool) from random starting positions around the perimeter of the pool. The starting position varies from trial to trial. If the subject does not locate the escape platform within a set time, the experimenter guides and places the subject on the platform to "teach" the location of the platform. After a delay period following the last training trial, a retention test in the absence of the escape platform is given to assess spatial memory. The subject's level of preference for the location of the (now absent) escape platform, as measured by, *e.g.,* the time spent in that location or the number of crossings of that location made by the mouse, indicates better spatial memory, i.e., treatment of cognitive impairment. The preference for the location of the escape platform under different treatment conditions, can then be compared for efficacy of the test compound in treating CNS disorders with cognitive impairment.

There are various tests known in the art for assessing cognitive function in humans, for example and without limitation, the clinical global impression of change scale (CIBIC-plus scale); the Mini Mental State Exam (MMSE); the Neuropsychiatric Inventory (NPI); the Clinical Dementia Rating Scale (CDR); the Cambridge Neuropsychological Test Automated Battery (CANTAB); the Sandoz Clinical Assessment-Geriatric (SCAG), the Buschke Selective Reminding Test (Buschke and Fuld, 1974); the Verbal Paired Associates subtest; the Logical Memory subtest; the Visual Reproduction subtest of the Wechsler Memory Scale-Revised (WMS-R) (Wechsler, 1997); or the Benton Visual Retention Test. *See* Folstein et al., J Psychiatric Res 12: 189-98, (1975); Robbins et al., Dementia 5: 266-81, (1994); Rey, L'examen clinique en psychologie, (1964); Kluger et al., J Geriatr Psychiatry Neurol 12:168-79, (1999); Marquis et al., 2002 and Masur et al., 1994. Another example of a cognitive test in humans is the explicit 3-alternative forced choice task. In this test, subjects are presented with color photographs of common objects consisting of a mix of three types of image pairs: similar pairs, identical pairs and unrelated foils. The second of the pair of similar objects is referred to as the "lure". These image pairs are fully randomized and presented individually as a series of images. Subjects are instructed to make a judgment as to whether the objects seen are new, old or similar. A "similar" response to the presentation of a lure stimulus indicates successful memory retrieval by the subject. By contrast, calling the lure stimulus "old" or "new" indicates that correct memory retrieval did not occur.

In addition to assessing cognitive performance, the progression of age-related cognitive impairment and dementia, as well as the conversion of age-related cognitive impairment into dementia, may be monitored by assessing surrogate changes in the brain of the subject. Surrogate changes include, without limitation, changes in regional brain volumes, perforant path degradation, and changes seen in brain function through resting state fMRI (R-fMRI) and fluorodeoxyglucose positron emission tomography (FDG-PET). Examples of regional brain volumes useful in monitoring the progression of age-related cognitive impairment and dementia include reduction of hippocampal volume and reduction in volume or thickness of entorhinal cortex. These volumes may be measured in a subject by, for example, MRI. Aisen et al., Alzheimer's & Dementia 6:239-246 (2010). Perforant path degradation has been shown to be linked to age, as well as reduced cognitive function. For example, older adults with more perforant path degradation tend to perform worse

in hippocampus-dependent memory tests. Perforant path degradation may be monitored in subjects through ultrahigh-resolution diffusion tensor imaging (DTI). Yassa et al., PNAS 107:12687-12691 (2010). Resting-state fMRI (R-fMRI) involves imaging the brain during rest, and recording large-amplitude spontaneous low-frequency (<0.1 Hz) fluctuations in the fMRI signal that are temporally correlated across functionally related areas. Seed-based functional connectivity, independent component analyses, and/or frequency-domain analyses of the signals are used to reveal functional connectivity between brain areas, particularly those areas whose connectivity increase or decrease with age, as well as the extent of cognitive impairment and/or dementia. FDG-PET uses the uptake of FDG as a measure of regional metabolic activity in the brain. Decline of FDG uptake in regions such as the posterior cingulated cortex, temporoparietal cortex, and prefrontal association cortex has been shown to relate to the extent of cognitive decline and dementia. Aisen et al., Alzheimer's & Dementia 6:239-246 (2010), Herholz et al., NeuroImage 17:302-316 (2002).

**Age-Related Cognitive Impairment**

This disclosure provides methods and compositions for treating age-related cognitive impairment or the risk thereof using an SV2A inhibitor or a pharmaceutically acceptable salt thereof in combination with an AChEI or a pharmaceutically acceptable salt thereof. In certain instances, treatment comprises preventing or slowing the progression, of age-related cognitive impairment. In certain instances, treatment comprises alleviation, amelioration or slowing the progression, of one or more symptoms associated with age-related cognitive impairment. In certain instances, treatment of age-related cognitive impairment comprises slowing the conversion of age-related cognitive impairment (including, but not limited to MCI, ARCD and AAMI) into dementia (e.g., AD). The methods and compositions may be used for human patients in clinical applications in the treating age-related cognitive impairment in conditions such as MCI, ARCD and AAMI or for the risk thereof. The dose of the composition and dosage interval for the method is, as described herein, one that is safe and efficacious in those applications.

In some instances, a subject to be treated by the methods and compositions of this disclosure exhibits age-related cognitive impairment or is at risk of such impairment. In some instances, the age-related cognitive impairment includes, without limitation, Age-Associated Memory Impairment (AAMI), Mild Cognitive Impairment (MCI) and Age-related Cognitive Decline (ARCD).

Animal models serve as an important resource for developing and evaluating treatments for such age-related cognitive impairments. Features that characterize age-related cognitive impairment in animal models typically extend to age-related cognitive impairment in humans. Efficacy in such animal models is, thus, expected to be predictive of efficacy in humans.

Various animal models of age-related cognitive impairment are known in the art. For example, extensive behavioral characterization has identified a naturally occurring form of cognitive impairment in an outbred strain of aged Long-Evans rats (Charles River Laboratories; Gallagher et al., Behav. Neurosci. 107:618-626, (1993)). In a behavioral assessment with the Morris Water Maze (MWM), rats learn and remember the location of an escape platform guided by a configuration of spatial cues surrounding the maze. The cognitive basis of performance is tested in probe trials using measures of the animal's spatial bias in searching for the location of the escape platform. Aged rats in the study population have no difficulty swimming to a visible platform, but an age-dependent impairment is detected when the platform is camouflaged, requiring the use of spatial information. Performance for individual aged rats in the outbred Long-Evans strain varies greatly. For example, a proportion of those rats perform on a par with young adults. However, approximately 40-50% fall outside the range of young performance. This variability among aged rats reflects reliable individual differences. Thus, within the aged population some animals are cognitively impaired and designated aged-impaired (AI) and other animals are not impaired and are designated aged-unimpaired (AU). *See, e.g.,* Colombo et al., Proc. Natl. Acad. Sci. 94: 14195-14199, (1997); Gallagher and Burwell, Neurobiol. Aging 10: 691-708, (1989); Gallagher et al. Behav. Neurosci. 107:618-626, (1993); Rapp and Gallagher, Proc. Natl. Acad. Sci. 93: 9926-9930, (1996); Nicolle et al., Neuroscience 74: 741-756, (1996); Nicolle et al., J. Neurosci. 19: 9604-9610, (1999); International Patent Publication WO2007/019312 and International Patent Publication WO 2004/048551. Such an animal model of age-related cognitive impairment may be used to assay the effectiveness of the methods and compositions this disclosure in treating age-related cognitive impairment.

The efficacy of the methods and compositions of this disclosure in treating age-related cognitive impairment may be assessed using a variety of cognitive tests, including the Morris water maze and the radial arm maze, as discussed above.

**Dementia**

This disclosure also provides methods and compositions for treating dementia using an SV2A inhibitor or a pharmaceutically acceptable salt thereof in combination with an AChEI or a pharmaceutically acceptable salt thereof. In certain instances, treatment comprises preventing or slowing the progression, of dementia. In certain instances,

treatment comprises alleviation, amelioration, or slowing the progression of one or more symptoms associated with dementia. In certain instances, the symptom to be treated is cognitive impairment. In certain instances, the dementia is Alzheimer's disease (AD), vascular dementia, dementia with Lewy bodies, or frontotemporal dementia. The methods and compositions may be used for human patients in clinical applications in treating dementia. The dose of the composition and dosage interval for the method is, as described herein, one that is safe and efficacious in those applications.

Animal models serve as an important resource for developing and evaluating treatments for dementia. Features that characterize dementia in animal models typically extend to dementia in humans. Thus, efficacy in such animal models is expected to be predictive of efficacy in humans. Various animal models of dementia are known in the art, such as the PDAPP, Tg2576, APP23, TgCRND8, J20, hPS2 Tg, and APP + PS1 transgenic mice. Sankaranarayanan, Curr. Top. Medicinal Chem. 6: 609-627, 2006; Kobayashi et al. Genes Brain Behav. 4: 173-196. 2005; Ashe and Zahns, Neuron. 66: 631-45, 2010. Such animal models of dementia may be used to assay the effectiveness of the methods and compositions of this disclosure of the disclosure in treating dementia.

The efficacy of the methods and compositions of this disclosure in treating dementia, or cognitive impairment associated with dementia, may be assessed in animals models of dementia, as well as human subjects with dementia, using a variety of cognitive tests known in the art, as discussed above.

**Post Traumatic Stress Disorder**

This disclosure also provides methods and compositions for treating post traumatic stress disorder (PTSD) using an SV2A inhibitor or a pharmaceutically acceptable salt thereof in combination with an AChEI or a pharmaceutically acceptable salt thereof. In certain instances, treatment comprises preventing or slowing the progression, of PTSD. In certain instances, treatment comprises alleviation, amelioration, or slowing the progression of one or more symptoms associated with PTSD. In certain instances, the symptom to be treated is cognitive impairment. The methods and compositions may be used for human patients in clinical applications in treating PTSD. The dose of the composition and dosage interval for the method is, as described herein, one that is safe and efficacious in those applications.

Patients with PTSD (and, to a lesser degree trauma-exposed patients without PTSD) have smaller hippocampal volumes (Woon et al., Prog. Neuro-Psychopharm. & Biological Psych. 34, 1181-1188; Wang et al., Arch. Gen. Psychiatry 67:296-303, 2010). PTSD is also associated with impaired cognitive performance. Older individuals with PTSD have greater declines in cognitive performance relative to control patients (Yehuda et al., Bio. Psych. 60: 714-721, 2006) and have a greater likelihood of developing dementia (Yaffe et al., Arch. Gen. Psych. 678: 608-613, 2010).

Animal models serve as an important resource for developing and evaluating treatments for PTSD. Features that characterize PTSD in animal models typically extend to PTSD in humans. Thus, efficacy in such animal models is expected to be predictive of efficacy in humans. Various animal models of PTSD are known in the art.

One rat model of PTSD is Time-dependent sensitization (TDS). TDS involves exposure of the animal to a severely stressful event followed by a situational reminder of the prior stress. The following is an example of TDS. Rats are placed in a restrainer, then placed in a swim tank and made to swim for a period of time, *e.g.,* 20 min. Following this, each rat is then immediately exposed to a gaseous anesthetic until loss of consciousness, and finally dried. The animals are left undisturbed for a number of days, *e.g.,* one week. The rats are then exposed to a "restress" session consisting of an initial stressor, *e.g.,* a swimming session in the swim tank (Liberzon et al., Psychoneuroendocrinology 22: 443-453, 1997; Harvery et al., Psychopharmacology 175:494-502, 2004). TDS results in an enhancement of the acoustic startle response (ASR) in the rat, which is comparable to the exaggerated acoustic startle that is a prominent symptom of PTSD (Khan and Liberzon, Psychopharmacology 172: 225-229, 2004). Such animal models of PTSD may be used to assay the effectiveness of the methods and compositions of this disclosure of the disclosure in treating PTSD.

The efficacy of the methods and compositions of this disclosure in treating PTSD, or cognitive impairment associated with PTSD, may also be assessed in animals models of PTSD, as well as human subjects with PTSD, using a variety of cognitive tests known in the art, as discussed above.

**Schizophrenia**

This disclosure additionally provides methods and compositions for treating schizophrenia using an SV2A inhibitor or a pharmaceutically acceptable salt thereof in combination with an AChEI or a pharmaceutically acceptable salt thereof. In certain instances, treatment comprises preventing or slowing the progression, of schizophrenia. In certain instances, treatment comprises alleviation, amelioration or slowing the progression, of one or more symptoms associated with schizophrenia. In certain instances, the symptom to be treated is cognitive impairment. The methods and compositions may be used for human patients in clinical applications in treating schizophrenia. The dose of the composition and dosage interval for the method is, as described herein, one that is safe and efficacious in those applications.

Cognitive impairments are also associated with schizophrenia. They precede the onset of psychosis and are present in non-affected relatives. The cognitive impairments associated with schizophrenia constitute a good predictor

for functional outcome and are a core feature of the disorder. Cognitive features in schizophrenia reflect dysfunction in frontal cortical and hippocampal circuits. Patients with schizophrenia also present hippocampal pathologies such as reductions in hippocampal volume, reductions in neuronal size and dysfunctional hyperactivity. An imbalance in excitation and inhibition in these brain regions has also been documented in schizophrenic patients suggesting that drugs targeting inhibitory mechanisms could be therapeutic. *See, e.g.,* Guidotti et al., Psychopharmacology 180: 191-205, 2005; Zierhut, Psych. Res. Neuroimag. 183:187-194, 2010; Wood et al., NeuroImage 52:62-63, 2010; Vinkers et al., Expert Opin. Investig. Drugs 19:1217-1233, 2009; Young et al., Pharmacol. Ther. 122:150-202, 2009.

Animal models serve as an important resource for developing and evaluating treatments for schizophrenia. Features that characterize schizophrenia in animal models typically extend to schizophrenia in humans. Thus, efficacy in such animal models is expected to be predictive of efficacy in humans. Various animal models of schizophrenia are known in the art.

One animal model of schizophrenia is protracted treatment with methionine. Methionine-treated mice exhibit deficient expression of GAD67 in frontal cortex and hippocampus, similar to those reported in the brain of postmortem schizophrenia patients. They also exhibit prepulse inhibition of startle and social interaction deficits (Tremonlizzo et al., PNAS, 99: 17095-17100, 2002). Another animal model of schizophrenia is methylaoxymethanol acetate (MAM)-treatment in rats. Pregnant female rats are administered MAM (20 mg/kg, intraperitoneal) on gestational day 17. MAM-treatment recapitulate a pathodevelopmental process to schizophrenia-like phenotypes in the offspring, including anatomical changes, behavioral deficits and altered neuronal information processing. More specifically, MAM-treated rats display a decreased density of parvalbumin-positive GABAergic interneurons in portions of the prefrontal cortex and hippocampus. In behavioral tests, MAM-treated rats display reduced latent inhibition. Latent inhibition is a behavioral phenomenon where there is reduced learning about a stimulus to which there has been prior exposure with any consequence. This tendency to disregard previously benign stimuli, and reduce the formation of association with such stimuli is believed to prevent sensory overload. Low latent inhibition is indicative of psychosis. Latent inhibition may be tested in rats in the following manner. Rats are divided into two groups. One group is pre-exposed to a tone over multiple trials. The other group has no tone presentation. Both groups are then exposed to an auditory fear conditioning procedure, in which the same tone is presented concurrently with a noxious stimulus, e.g. an electric shock to the foot. Subsequently, both groups are presented with the tone, and the rats' change in locomotor activity during tone presentation is monitored. After the fear conditioning the rats respond to the tone presentation by strongly reducing locomotor activity. However, the group that has been exposed to the tone before the conditioning period displays robust latent inhibition: the suppression of locomotor activity in response to tone presentation is reduced. MAM-treated rats, by contrast show impaired latent inhibition. That is, exposure to the tone previous to the fear conditioning procedure has no significant effect in suppressing the fear conditioning. (*see* Lodge et al., J. Neurosci., 29:2344-2354, 2009) Such animal models of schizophrenia may be used to assay the effectiveness of the methods and compositions of the disclosure in treating schizophrenia.

The efficacy of the methods and compositions of this disclosure in treating schizophrenia, or cognitive impairment associated with schizophrenia, may also be assessed in animal models of schizophrenia, as well as human subjects with schizophrenia, using a variety of cognitive tests known in the art, as discussed above.

**Amyotropic Lateral Sclerosis (ALS)**

This disclosure additionally provides methods and compositions for treating ALS using an SV2A inhibitor or a pharmaceutically acceptable salt thereof in combination with an AChEI or a pharmaceutically acceptable salt thereof. In certain instances, treatment comprises preventing or slowing the progression, of ALS. In certain instances, treatment comprises alleviation, amelioration or slowing the progression, of one or more symptoms associated with ALS. In certain instances, the symptom to be treated is cognitive impairment. The methods and compositions may be used for human patients in clinical applications in treating ALS. The dose of the composition and dosage interval for the method is, as described herein, one that is safe and efficacious in those applications.

In addition to the degeneration of motor neurons, ALS is characterized by neuronal degeneration in the entorhinal cortex and hippocampus, memory deficits, and neuronal hyperexcitability in different brain areas such as the cortex.

The efficacy of the methods and compositions of this disclosure in treating ALS, or cognitive impairment associated with ALS, may also be assessed in animal models of ALS, as well as human subjects with ALS, using a variety of cognitive tests known in the art, as discussed above.

**Cancer therapy-related cognitive impairment**

This disclosure additionally provides methods and compositions for treating cancer therapy-related cognitive impairment using an SV2A inhibitor or a pharmaceutically acceptable salt thereof in combination with an AChEI or a pharmaceutically acceptable salt thereof. In certain instances, treatment comprises preventing or slowing the progression,

of cancer therapy-related cognitive impairment. In certain instances, treatment comprises alleviation, amelioration or slowing the progression, of one or more symptoms associated with cancer therapy-related cognitive impairment. The methods and compositions may be used for human patients in clinical applications in treating cancer therapy-related cognitive impairment. The dose of the composition and dosage interval for the method is, as described herein, one that is safe and efficacious in those applications.

Therapies that are used in cancer treatment, including chemotherapy, radiation, or combinations thereof, can cause cognitive impairment in patients, in such functions as memory, learning and attention. Cytotoxicity and other adverse side-effects on the brain of cancer therapies are the basis for this form of cognitive impairment, which can persist for decades. (Dietrich et al., Oncologist 13:1285-95, 2008; Soussain et al., Lancet 374:1639-51, 2009).

Cognitive impairment following cancer therapies reflects dysfunction in frontal cortical and hippocampal circuits that are essential for normal cognition. In animal models, exposure to either chemotherapy or radiation adversely affects performance on tests of cognition specifically dependent on these brain systems, especially the hippocampus (Kim et al., J. Radiat. Res. 49:517-526, 2008; Yang et al., Neurobiol. Learning and Mem. 93:487-494, 2010). Thus, drugs targeting these cortical and hippocampal systems could be neuroprotective in patients receiving cancer therapies and efficacious in treating symptoms of cognitive impairment that may last beyond the interventions used as cancer therapies.

Animal models serve as an important resource for developing and evaluating treatments for cancer therapy-related cognitive impairment. Features that characterize cancer therapy-related cognitive impairment in animal models typically extend to cancer therapy-related cognitive impairment in humans. Thus, efficacy in such animal models is expected to be predictive of efficacy in humans. Various animal models of cancer therapy-related cognitive impairment are known in the art.

Examples of animal models of cancer therapy-related cognitive impairment include treating animals with antineoplastic agents such as cyclophosphamide (CYP) or with radiation, *e.g.,* $^{60}$Co gamma-rays. (Kim et al., J. Radiant. Res. 49:517-526, 2008; Yang et al., Neurobiol. Learning and Mem. 93:487-494, 2010). The cognitive function of animal models of cancer therapy-related cognitive impairment may then be tested with cognitive tests to assay the effectiveness of the methods and compositions of the disclosure in treating cancer therapy-related cognitive impairment. The efficacy of the methods and compositions of this disclosure in treating cancer therapy-related cognitive impairment, as well as human subjects with cancer therapy-related cognitive impairment, using a variety of cognitive tests known in the art, as discussed above.

## SV2A Inhibitor

"Synaptic vesicle protein-2 (SV2)" is a family of synaptic vesicle proteins, which consists of three members, designated SV2A, SV2B, and SV2C. SV2A is the most widely distributed family member, being expressed ubiquitously in the brain. The proteins are integral membrane proteins and have a low-level homology (20-30%) to the twelve transmembrane family of bacterial and fungal transporter proteins that transport sugar, citrate, and xenobiotics (Bajjalieh et al., Science. 257: 1271-1273. (1992)). SV2 family proteins are present in the brain and endocrine cells, and further are present in all synaptic and endocrine vesicles. SV2 proteins are reported to play a role in normal synaptic function, and functions in a maturation step of primed vesicles that converts the vesicles into a Ca($^{2+}$)- and synaptotagmin-responsive state (Sudhof et al., 2009). Functionally, SV2 proteins are reported to enhance synaptic currents and increase the probability of transmitter release by maintaining the size of the readily releasable pool of vesicles (Custer et al., 2006).

"SV2A inhibitor" refers to any agent, substance or compound that binds to SV2A and reduces synaptic function by reducing pre-synaptic vesicle release (See, e.g., Noyer et al. 1995; Fuks et al. 2003; Lynch et al. 2004; Gillard et al. 2006; Custer et al., 2006; Smedt et al., 2007; Yang et al., 2007; and Example 8 of WO 2001/62726) A substance, or a compound or an agent is an SV2A inhibitor even if it does not itself bind to SV2A, as long as it causes, or affects the ability of, another compound or agent to bind SV2A or reduce synaptic function by reducing pre-synaptic vesicle release. SV2A inhibitors, as used herein, include pharmaceutically acceptable salts of the inhibitors thereof. They also include hydrates, polymorphs, prodrugs, salts, and solvates of these inhibitors.

Among the SV2A inhibitors or pharmaceutically acceptable salts, hydrates, solvates and polymorphs thereof that are useful in the methods and compositions of this disclosure are those disclosed, for example, United States (U.S.) Patent Application 12/580,464, International Patent Application PCT/US2009/005647, U.S. Patent Application 61/105,847, U.S. Patent Application 61/152,631, and U.S. Patent Application 61/175,536. However, any SV2A inhibitor or a pharmaceutically acceptable salt, hydrate, solvate or polymorph thereof may be used in the methods and compositions of the disclosure. In some instances, the SV2A inhibitor is selected from the group of SV2A inhibitors referred to in International Patent Applications WO2010/144712; WO2010/002869; WO2008/132139; WO2007/065595; WO2006/128693; WO2006/128692; WO2005/054188; WO2004/087658; WO2002/094787; WO2001/062726; U.S. Patents 7,465,549; 7,244,747; 5,334,720; 4,696,943; 4,696,942; U.S. Patent Application Publication Numbers 20090312333; 20090018148; 20080081832; 2006258704; and UK Patent Numbers 1,039,113; and 1,309,692 or their pharmaceutically acceptable salts, hydrates, solvates, or polymorphs. Other SV2A inhibitors may also be used in this

disclosure. Applicants also refer to methods of preparing these compounds found in the documents cited above. Other synthetic methods may also be used. These methods are well known to those skilled in the art.

In some instances of this disclosure, the SV2A inhibitor is selected from the group consisting of levetiracetam, brivaracetam, and seletracetam or derivatives or analogs or pharmaceutically acceptable salts, solvates, hydrates, polymorphs, or prodrugs thereof.

In some instances of this disclosure, the SV2A inhibitor is levetiracetam or salts, solvates, hydrates, polymorphs or prodrugs thereof. Levetiracetam refers to the International Union of Pure and Applied Chemistry (IUPAC) name of the compound (2S)-2-(2-oxopyrrolidin-1-yl) butanamide). Levetiracetam is a widely used antiepileptic drug. Levetiracetam binds to a specific site in the CNS: the synaptic vesicle protein 2A (SV2A) (See. e.g., Noyer et al. 1995; Fuks et al. 2003; Lynch et al. 2004; Gillard et al. 2006) and has further been shown to directly inhibit synaptic activity and neurotransmission by inhibiting presynaptic neurotransmitter release (Yang et al., 2007).

Among the SV2A inhibitors useful for the methods and compositions of this disclosure are the following:

i) International Patent Application WO 2001/062726:

A compound having the formula I or a pharmaceutically acceptable salt thereof,

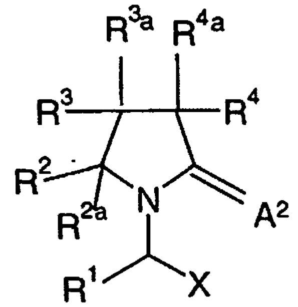

(I)

wherein X is-$CA^1NR^5R^6$ or-$CA^1OR^7$ or-$CA^1$-$R^8$ or CN ;

$A^1$ and $A^2$ are independently oxygen, sulfur or-$NR^9$;

$R^1$ is hydrogen, alkyl, aryl or-$CH_2$-$R^{1a}$ wherein $R^{1a}$ is aryl, heterocycle, halogen, hydroxy, amino, nitro or cyano;

$R^2$, $R^3$ and $R^4$ are the same or different and each is independently hydrogen, halogen, hydroxy, thiol, amino, nitro, nitrooxy, cyano, azido, carboxy, amido, sulfonic acid, sulfonamide, alkyl, alkenyl, alkynyl, ester, ether, aryl, heterocycle, or an oxy derivative, thio derivative, amino derivative, acyl derivative, sulfonyl derivative or sulfinyl derivative;

$R^{2a}$, $R^{3a}$ and $R^{4a}$ are the same or different and each is independently hydrogen, halogen, alkyl, alkenyl, alkynyl or aryl;

$R^5$, $R^6$, $R^7$ and $R^9$ are the same or different and each is independently hydrogen, hydroxy, alkyl, aryl, heterocycle or an oxy derivative; and

$R^8$ is hydrogen, hydroxy, thiol, halogen, alkyl, aryl, heterocycle or a thio derivative;

with the provisos that at least one of as $R^2$, $R^3$, $R^4$, $R^{2a}$ , $R^{3a}$ and $R^{4a}$ is other than hydrogen; and that when the compound is a mixture of all possible isomers, X is-$CONR^5R^6$, $A^2$ is oxygen and $R^1$ is hydrogen, methyl, ethyl or propyl then substitution on the pyrollidine ring is other than mono-, di-, or tri-methyl or mono-ethyl; and that when $R^1$, $R^2$, $R^4$, $R^{2a}$ , $R^{3a}$ and $R^{4a}$ are each hydrogen, $A^2$ is oxygen and X is $CONR^5R^6$ then $R^3$ is different from carboxy, ester, amido, substituted oxo-pyrrolidine, hydroxy, oxy derivative, amino, amino derivatives, methyl, naphthyl, phenyl optionally substituted by oxy derivatives or in the para position by an halogen atom.

In the definitions set forth below, unless otherwise stated, $R^{11}$ and $R^{12}$ are the same or different and each is independently amido, alkyl, alkenyl, alkynyl, acyl, ester, ether, aryl, aralkyl, heterocycle or an oxy derivative, thio derivative, acyl derivative, amino derivative, sulfonyl derivative, or sulfinyl derivative, each optionally substituted with any suitable group, including, but not limited to, one or more moieties selected from lower alkyl or other groups as described below as substituents for alkyl.

The term "oxy derivative", as used herein is defined as including -O-$R^{11}$ groups wherein $R^{11}$ is as defined above

except for "oxy derivative". Non-limiting examples are alkoxy, alkenyloxy, alkynyloxy, acyloxy, oxyester, oxyamido, alkylsulfonyloxy, alkylsulfinyloxy, arylsulfonyloxy, arylsulfinyloxy, aryloxy, aralkoxy or heterocyclooxy such as pentyloxy, allyloxy, methoxy, ethoxy, phenoxy, benzyloxy, 2-naphthyloxy, 2-pyridyloxy, methylenedioxy, carbonate.

The term "thio derivative" as used herein, is defined as including-S-$R^{11}$ groups wherein $R^{11}$ is as defined above except for "thio derivative". Non-limiting examples are alkylthio, alkenylthio, alkynylthio and arylthio.

The term "amino derivative" as used herein, is defined as including-NH$R^{11}$ or -N$R^{11}R^{12}$ groups wherein $R^{11}$ and $R^{12}$ are as defined above. Non-limiting examples are mono- or di-alkyl-, alkenyl-, alkynyl- and arylamino or mixed amino.

The term "acyl derivative" as used herein, represents a radical derived from carboxylic acid and thus is defined as including groups of the formula $R^{11}$-CO-, wherein $R^{11}$ is as defined above and may also be hydrogen. Non-limiting examples are formyl, acetyl, propionyl, isobutyryl, valeryl, lauroyl, heptanedioyl, cyclohexanecarbonyl, crotonoyl, fumaroyl, acryloyl, benzoyl, naphthoyl, furoyl, nicotinoyl, 4-carboxybutanoyl, oxalyl, ethoxalyl, cysteinyl, oxamoyl.

The term "sulfonyl derivative" as used herein, is defined as including a group of the formula -$SO_2$-$R^{11}$, wherein $R^{11}$ is as defined above except for "sulfonyl derivative". Non-limiting examples are alkylsulfonyl, alkenylsulfonyl, alkynylsulfonyl and arylsulfonyl.

The term "sulfinyl derivative" as used herein, is defined as including a group of the formula -SO-$R^{11}$, wherein $R^{11}$ is as defined above except for "sulfinyl derivative". Non-limiting examples are alkylsulfinyl, alkenylsulfinyl, alkynylsulfinyl and arylsulfinyl.

The term "alkyl", as used herein, is defined as including saturated, monovalent hydrocarbon radicals having straight, branched or cyclic moieties or combinations thereof and containing 1-20 carbon atoms, preferably 1-6 carbon atoms for non-cyclic alkyl and 3-6 carbon atoms for cycloalkyl (in these two preferred cases, unless otherwise specified, "lower alkyl"). Alkyl moieties may optionally be substituted by 1 to 5 substituents independently selected from the group consisting of halogen, hydroxy, thiol, amino, nitro, cyano, thiocyanato, acyl, acyloxy, sulfonyl derivative, sulfinyl derivative, alkylamino, carboxy, ester, ether, amido, azido, cycloalkyl, sulfonic acid, sulfonamide, thio derivative, oxyester, oxyamido, heterocycle, vinyl, C1-5-alkoxy, C6-10-aryloxy and C6-10-aryl.

Preferred alkyl groups are methyl, ethyl, propyl, isopropyl, butyl, iso or ter-butyl, and 2,2,2-trimethylethyl each optionally substituted by at least one substituent selected from the group consisting of halogen, hydroxy, thiol, amino, nitro and cyano, such as trifluoromethyl, trichloromethyl, 2,2,2-trichloroethyl, 1,1-dimethyl-2,2-dibromoethyl, 1,1-dimethyl-2,2,2-trichloroethyl.

The term "alkenyl" as used herein, is defined as including both branched and unbranched, unsaturated hydrocarbon radicals having at least one double bond such as ethenyl (= vinyl), 1- methyl-1-ethenyl, 2,2-dimethyl-1-ethenyl, 1-propenyl, 2-propenyl (= allyl), 1-butenyl, 2-butenyl, 3-butenyl, 4-pentenyl, 1-methyl-4-pentenyl, 3-methyl-1-pentenyl, 1-hexenyl, 2-hexenyl, and the like and being optionally substituted by at least one substituent selected from the group consisting of halogen, hydroxy, thiol, amino, nitro, cyano, aryl and heterocycle such as mono- and di-halo vinyl where halo is fluoro, chloro or bromo.

The term "alkynyl" as used herein, is defined as including a monovalent branched or unbranched hydrocarbon radical containing at least one carbon-carbon triple bond, for example ethynyl, 2-propynyl (= propargyl), and the like and being optionally substituted by at least one substituent selected from the group consisting of halogen, hydroxy, thiol, amino, nitro, cyano, aryl and heterocycle, such as haloethynyl.

When present as bridging groups, alkyl, alkenyl and alkynyl represent straight- or branched chains, C1-12, preferably C1-4-alkylene or C2-12-, preferably C2-4-alkenylene or -alkynylene moieties respectively.

Groups where branched derivatives are conventionally qualified by prefixes such as "n", "sec", "iso" and the like (e.g., "n-propyl", "sec-butyl") are in the n-form unless otherwise stated.

The term "aryl" as used herein, is defined as including an organic radical derived from an aromatic hydrocarbon consisting of 1-3 rings and containing 6-30 carbon atoms by removal of one hydrogen, such as phenyl and naphthyl each optionally substituted by 1 to 5 substituents independently selected from halogen, hydroxy, thiol, amino, nitro, cyano, acyl, acyloxy, sulfonyl, sulfinyl, alkylamino, carboxy, ester, ether, amido, azido, sulfonic acid, sulfonamide, alkylsulfonyl, alkylsulfinyl, alkylthio, oxyester, oxyamido, aryl, C1-6-alkoxy, C6-10-aryloxy, C1-6-alkyl, C1-6-haloalkyl. Aryl radicals are preferably monocyclic containing 6-10 carbon atoms. Preferred aryl groups are phenyl and naphthyl each optionally substituted by 1 to 5 substituents independently selected from halogen, nitro, amino, azido, C1-6-alkoxy, C1-6-alkylthio, C1-6-alkyl, C1-6-haloalkyl and phenyl.

The term "halogen", as used herein, includes an atom of Cl, Br, F, I.

The term "hydroxy", as used herein, represents a group of the formula -OH.

The term "thiol", as used herein, represents a group of the formula -SH.

The term "cyano", as used herein, represents a group of the formula -CN.

The term "nitro", as used herein, represents a group of the formula -$NO_2$.

The term "nitrooxy", as used herein, represents a group of the formula - $ONO_2$.

The term "amino", as used herein, represents a group of the formula -$NH_2$.

The term "azido", as used herein, represents a group of the formula $-N_3$.
The term "carboxy", as used herein, represents a group of the formula-COOH.
The term "sulfonic acid", as used herein, represents a group of the formula-$SO_3H$.
The term "sulfonamide", as used herein, represents a group of the formula - $SO_2NH_2$.
The term "ester", as used herein is defined as including a group of formula - COO-R" wherein $R^{11}$ is as defined above except oxy derivative, thio derivative or amino derivative.
The term "ether" is defined as including a group selected from C1-50-straight or branched alkyl, or C2-50-straight or branched alkenyl or alkynyl groups or a combination of the same, interrupted by one or more oxygen atoms.
The term "amido" is defined as including a group of formula $-CONH_2$ or-$CONHR^{11}$ or $-CONR^{11}R^{12}$ wherein $R^{11}$ and $R^{12}$ are as defined above.
The term "heterocycle", as used herein is defined as including an aromatic or non aromatic cyclic alkyl, alkenyl, or alkynyl moiety as defined above, having at least one O, S and/or N atom interrupting the carbocyclic ring structure and optionally, one of the carbon of the carbocyclic ring structure may be replaced by a carbonyl. Non-limiting examples of aromatic heterocycles are pyridyl, furyl, pyrrolyl, thienyl, isothiazolyl, imidazolyl, benzimidazolyl, tetrazolyl, quinazolinyl, quinolizinyl, naphthyridinyl, pyridazinyl, pyrimidinyl, pyrazinyl, quinolyl, isoquinolyl, isobenzofuranyl, benzothienyl, pyrazolyl, indolyl, indolizinyl, purinyl, isoindolyl, carbazolyl, thiazolyl, 1, 2, 4-thiadiazolyl, thieno (2,3-b) furanyl, furopyranyl, benzofuranyl, benzoxepinyl, isooxazolyl, oxazolyl, thianthrenyl, benzothiazolyl, or benzoxazolyl, cinnolinyl, phthalazinyl, quinoxalinyl, phenanthridinyl, acridinyl, perimidinyl, phenanthrolinyl, phenothiazinyl, furazanyl, isochromanyl, indolinyl, xanthenyl, hypoxanthinyl, pteridinyl, 5-azacytidinyl, 5-azauracilyl, triazolopyridinyl, imidazolopyridinyl, pyrrolopyrimidinyl, and pyrazolopyrimidinyl optionally substituted by alkyl or as described above for the alkyl groups. Non-limiting examples of non aromatic heterocycles are tetrahydrofuranyl, tetrahydropyranyl, piperidinyl, piperidyl, piperazinyl, imidazolidinyl, morpholino, morpholinyl, 1-oxaspiro (4.5) dec-2-yl, pyrrolidinyl, 2-oxo-pyrrolidinyl, sugar moieties (i.e. glucose, pentose, hexose, ribose, fructose, which may also be substituted) or the same which can optionally be substituted with any suitable group, including but not limited to one or more moieties selected from lower alkyl, or other groups as described above for the alkyl groups. The term "heterocycle" also includes bicyclic, tricyclic and tetracyclic, spiro groups in which any of the above heterocyclic rings is fused to one or two rings independently selected from an aryl ring, a cyclohexane ring, a cyclohexene ring, a cyclopentane ring, a cyclopentene ring or another monocyclic heterocyclic ring or where a monocyclic heterocyclic group is bridged by an alkylene group, such as quinuclidinyl, 7-azabicyclo (2.2.1)heptanyl, 7- oxabicyclo (2.2.1) heptanyl, 8-azabicyclo (3.2.1)octanyl.
In the above definitions it is to be understood that when a substituent such as $R^2$, $R^3$, $R^4$, $R^{2a}$, $R^{3a}$, $R^{4a}$, $R^5$, $R^6$, $R^7$, $R^8$ is attached to the rest of the molecule *via* a heteroatom or a carbonyl, a straight- or branched chain, C1-12-, preferably C1-4-alkylene or C2-12, preferably C2-4-alkenylene or-alkynylene bridge may optionally be interposed between the heteroatom or the carbonyl and the point of attachment to the rest of the molecule.
Preferred examples of X are -COO $R^7$ or $-CONR^5R^6$, wherein $R^5$, $R^6$ and $R^7$ are preferably hydrogen, C1-4-alkyl, phenyl or alkylphenyl.
Preferably X is carboxy or $-CONR^5R^6$, wherein $R^5$ and $R^6$ are preferably hydrogen, C1-4-alkyl, phenyl or alkylphenyl, especially $-CONH_2$.
Preferably $A^1$ and $A^2$ are each oxygen.
Preferably $R^1$ is hydrogen, alkyl, especially C1-12 alkyl, particularly lower alkyl or aryl especially phenyl.
Examples of preferred $R^1$ groups are methyl, ethyl, propyl, isopropyl, butyl, iso- or ter-butyl, 2,2,2-trimethylethyl each optionally attached *via* a methylene bridge or the same substituted by at least one halogen atom such as trifluoromethyl, trichloromethyl, 2,2,2-trichloroethyl, 1,1-dimethyl-2,2-dibromoethyl, 1,1-dimethyl-2,2,2-trichloroethyl.
$R^1$ as ethyl is especially preferred.
Preferably $R^2$ and $R^{2a}$ are independently hydrogen, halogen or alkyl, especially lower alkyl.
Examples of preferred $R^2$ and $R^{2a}$ groups are independently hydrogen, halogen or methyl, ethyl, propyl, isopropyl, butyl, iso or ter-butyl, 2,2,2-trimethylethyl or the same substituted by at least one halogen atom such as trifluoromethyl, trichloromethyl, 2,2,2-trichloroethyl, 1,1-dimethyl-2,2-dibromoethyl, 1,1-dimethyl-2,2,2-trichloroethyl.
Especially at least one and most preferably both of $R^2$ and $R^{2a}$ are hydrogen.
Preferably $R^{3a}$, $R^4$ and $R^{4a}$ are independently hydrogen, alkyl, especially methyl or ethyl or aryl especially phenyl or aralkyl, especially benzyl.
Examples of preferred $R^{3a}$, $R^4$ and $R^{4a}$ groups are independently hydrogen, halogen or methyl, ethyl, propyl, isopropyl, butyl, iso or ter-butyl, 2,2,2-trimethylethyl or the same substituted by at least one halogen atom such as trifluoromethyl, trichloromethyl, 2,2,2-trichloroethyl, 1,1-dimethyl-2, 2-dibromoethyl, 1,1-dimethyl-2,2,2-trichloroethyl.
Especially at least one and most preferably both of $R^4$ and $R^{4a}$ are hydrogen.
$R^{3a}$ is particularly hydrogen or alkyl, especially lower alkyl and is most preferably hydrogen.
Preferably $R^3$ is hydrogen, C1-12-alkyl, especially C1-6-alkyl, each optionally substituted by one or more substituents selected from hydroxy, halogen, cyano, thiocyanato or alkoxy and attached to the ring either directly or *via* a thio, sulfinyl, sulfonyl, carbonyl or oxycarbonyl group and optionally, a C1-4-alkylene bridge, particularly methylene ;

C2-6-alkenyl or -alkynyl, especially C2-3-alkenyl or-alkynyl each optionally substituted by one or more halogens ; azido ; cyano ; amido ; carboxy ; triazolyl, tetrazolyl, pyrrolidinyl, pyridyl, 1- oxidopyridyl, thiomorpholinyl, benzodioxolyl, furyl, oxazolyl, pyrimidinyl, pyrrolyl, thiadiazolyl, thiazolyl, thienyl or piperazinyl each optionally substituted by one or more substituents selected from halogen, C1-6-alkyl and phenyl and attached to the ring either directly or *via* a carbonyl group or a C1-4-alkylene bridge, particularly methylene ; naphthyl; or phenyl, phenylalkyl or phenylalkenyl each optionally substituted by one or more substituents selected from halogen, C1-6-alkyl, C1-6 haloalkyl, C1-6-alkoxy, C1-6-alkylthio, amino, azido, phenyl and nitro and each attached to the ring either directly or *via* an oxy, sulfonyl, sulfonyloxy, carbonyl or carbonyloxy group and optionally additionally a C1-4-alkylene bridge, particularly methylene.

Also, preferably, $R^3$ is C1-6-alkyl optionally substituted by one or more substituents selected from halogen, thiocyanato, azido, alkoxy, alkylthio, phenylsulfonyl ; nitrooxy ; C2-3- alkenyl or-alkynyl each optionally substituted by one or more halogens or by acetyl; tetrazolyl, pyridyl, furyl, pyrrolyl, thiazolyl or thienyl ; or phenyl or phenylalkyl each optionally substituted by one or more substituents selected from halogen, C1-6-alkyl, C1-6 haloalkyl, C1-6-alkoxy, amino, azido, phenyl and nitro and each attached to the ring either directly or *via* a sulfonyloxy and optionally additionally a C1-4-alkylene bridge, particularly methylene.

Other examples of preferred $R^3$ groups are hydrogen, halogen or methyl, ethyl, propyl, isopropyl, butyl, iso or ter-butyl, 2,2,2-trimethylethyl or the same substituted by at least one halogen atom such as trifluoromethyl, trichloromethyl, 2,2,2-trichloroethyl, 1,1-dimethyl-2, 2-dibromoethyl, 1,1-dimethyl-2,2,2-trichloroethyl.

$R^3$ is especially C1-4-alkyl optionally substituted by one or more substituents selected from halogen, thiocyanato or azido; C2-5-alkenyl or-alkynyl, each optionally substituted by one or more halogens; thienyl; or phenyl optionally substituted by one or more substituents selected from halogen, C1-6-alkyl, C1-6 haloalkyl or azido.

Further examples of preferred $R^3$ groups are C1-6 alkyl and C2-6 haloalkenyl.

Preferably $R^5$ and $R^6$ are independently hydrogen, methyl, ethyl, propyl, isopropyl, butyl, iso or ter-butyl, 2,2,2-trimethylethyl, especially hydrogen or methyl.

Especially at least one and most preferably both of $R^5$ and $R^6$ are hydrogen.

Preferably $R^7$ is hydrogen, methyl, ethyl, propyl, isopropyl, butyl, iso or tert-butyl, 2,2,2-trimethylethyl, methoxy, ethoxy, phenyl, benzyl or the same substituted by at least one halogen atom such as trifluoromethyl, chlorophenyl.

Preferably $R^7$ is hydrogen, methyl or ethyl especially hydrogen.

Preferably $R^8$ is hydrogen, methyl, ethyl, propyl, isopropyl, butyl, iso or ter-butyl, 2,2,2-trimethylethyl, phenyl, benzyl or the same substituted by at least one halogen atom such as trifluoromethyl, chlorobenzyl.

Preferably $R^8$ is hydrogen or methyl.

Combinations of one or more of these preferred compound groups are especially preferred.

A particular group of compounds of formula I (Compounds 1A) comprises those wherein,

$A^2$ is oxygen;

X is-CONR$^5$R$^6$ or-COOR$^7$ or-CO-R$^8$ or CN ;

$R^1$ is hydrogen or alkyl, aryl, halogen, hydroxy, amino, nitro, cyano;

$R^2$, $R^3$, $R^4$, are the same or different and each is independently hydrogen or halogen, hydroxy, amino, nitro, cyano, acyl, acyloxy, a sulfonyl derivative, a sulfinyl derivative, an amino derivative, carboxy, ester, ether, amido, sulfonic acid, sulfonamide,,, alkoxycarbonyl,,, a thio derivative,, alkyl, alkoxy, oxyester, oxyamido, aryl,, an oxy derivative, heterocycle, vinyl and $R^3$ may additionally represent C2-5 alkenyl, C2-5 alkynyl or azido each optionally substituted by one or more halogen, cyano, thiocyano, azido,, cyclopropyl, acyl and/or phenyl; or phenylsulfonyloxy whereby any phenyl moiety may be substituted by one or more halogen, alkyl, haloalkyl, alkoxy, nitro, amino, and/or phenyl; most preferably methyl, ethyl, propyl, isopropyl, butyl, or isobutyl.

$R^{2a}$, $R^{3a}$ and $R^{4a}$ are hydrogen;

$R^5$, $R^6$, $R^7$ are the same or different and each is independently hydrogen, hydroxy, alkyl, aryl, heterocycle or oxy derivative; and

$R^8$ is hydrogen, hydroxy, thiol, halogen, alkyl, aryl, heterocycle, alkylthio or thio derivative.

Within these Compounds 1A, $R^1$ is preferably methyl, ethyl, propyl, isopropyl, butyl, or isobutyl; most preferably methyl, ethyl or n-propyl.

$R^2$ and $R^4$ are preferably independently hydrogen or halogen or methyl, ethyl, propyl, isopropyl, butyl, isobutyl; and, most preferably, are each hydrogen.

$R^3$ is preferably C1-5 alkyl, C2-5 alkenyl, C2-C5 alkynyl, cyclopropyl, azido, each optionally substituted by one or more halogen, cyano, thiocyano, azido, alkylthio, cyclopropyl, acyl and/or phenyl; phenyl; phenylsulfonyl ; phenylsulfonyloxy, tetrazole, thiazole, thienyl, furyl, pyrrole, pyridine, whereby any phenyl moiety may be substituted by one or more halogen, alkyl, haloalkyl, alkoxy, nitro, amino, and/or phenyl; most preferably methyl, ethyl, propyl, isopropyl, butyl, or isobutyl.

X is preferably -COOH or -COOMe or -COOEt or -$CONH_2$ ; most preferably -$CONH_2$.

A further particular group of compounds of formula I (Compounds 1 B) comprises those wherein,

X is-CA$^1$$NH_2$,-CA$^1$$NHCH_3$ or-CA'N $(CH_3)_2$ ;

$R^1$ is alkyl or phenyl;
$R^3$ is alkyl, alkenyl, alkynyl, cyano, isothiocyanato, ether, carboxyl, amido, aryl, heterocycle ; or
$R^3$ is $CH_2R^{10}$ wherein $R^{10}$ is hydrogen, cycloalkyl, oxyester, oxyalkylsulfonyl, oxyarylsufonyl, aminoalkylsulfonyl, aminoarylsulfonyl, nitrooxy, cyano, isothiocyanato, azido, alkylthio, arylthio, alkylsulfinyl, alkylsulfonyl, heterocycle, aryloxy, alkoxy or trifluoroethyl;
$R^{3a}$ is hydrogen, alkyl or aryl (especially with the proviso that when $R^{3a}$ is hydrogen, $R^3$ other than methyl);
or $R^3R^{3a}$ form a cycloalkyl ;
and $R^2$, $R^{2a}$, $R^4$ and $R^{4a}$ are each hydrogen.

Within the compounds of formula I,
$R^1$ is preferably alkyl especially C1-12- more particularly C1-6-alkyl and is most preferably ethyl;
$R^2$, $R^{2a}$, $R^{3a}$ and $R^{4a}$ are preferably hydrogen;
$R^3$ is preferably selected from hydrogen; C1-12-alkyl, especially C1-6-alkyl, each optionally substituted by one or more substituents selected from hydroxy, halogen, cyano, thiocyanato or alkoxy and attached to the ring either directly or *via* a thio, sulfinyl, sulfonyl, carbonyl or oxycarbonyl group and optionally additionally a C1-4-alkylene bridge, particularly methylene; C2-6-alkenyl or-alkynyl, especially C2-3-alkenyl or-alkynyl, each optionally substituted by one or more halogens ; azido ; cyano ; amido ; carboxy ; triazolyl, tetrazolyl, pyrrolidinyl, pyridyl, 1-oxidopyridyl, thiomorpholinyl, benzodioxolyl, furyl, oxazolyl, pyrimidinyl, pyrrolyl, thiadiazolyl, thiazolyl, thienyl or piperazinyl each optionally substituted by one or more substituents selected from halogen, C1-6-alkyl and phenyl and attached to the ring either directly or *via* a carbonyl group or a C1-4-alkylene bridge, particularly methylene ; naphthyl; or phenyl, phenylalkyl or phenylalkenyl each optionally substituted by one or more substituents selected from halogen, C1-6-alkyl, Cl-6 haloalkyl, C1-6-alkoxy, C1-6-alkylthio, amino, azido, phenyl and nitro and each attached to the ring either directly or *via* an oxy, sulfonyl, sulfonyloxy, carbonyl or carbonyloxy group and optionally additionally a C1-4- alkylene bridge, particularly methylene ;
$R^{3a}$ is preferably hydrogen or C1-4-alkyl ;
$R^4$ and $R^{4a}$ are preferably, independently hydrogen, C1-4-alkyl, phenyl or benzyl.

A further group of compounds of formula I (Compounds 1C) comprises those in racemic form wherein, when X is-$CONR^5R^6$ and $R^1$ is hydrogen, methyl, ethyl or propyl, then substitution on the pyrrolidine ring is other than mono-, di-, or tri-methyl or mono-ethyl.

A further group of compound of formula I (Compounds 1D) comprises those in racemic form wherein, when X is-$CONR^5R^6$ and $R^1$ is hydrogen or C1-6-alkyl, C2-6-alkenyl or- alkynyl or cycloalkyl, each unsubstituted, then substitution in the ring is other than by alkyl, alkenyl or alkynyl, each unsubstituted.

A further particular group of compounds of formula I (Compounds IE) comprises those wherein,
X is-$CA^1NH_2$;
$R^1$ is H ;
$R^3$ is azidomethyl, iodomethyl, ethyl optionally substituted by 1 to 5 halogen atoms, n- propyl optionally substituted by 1 to 5 halogen atoms, vinyl optionally subsituted by one or two methyl, and/or 1 to 3 halogen atoms, acetylene optionally substituted by Cl-4-alkyl, phenyl or halogen ;
$R^{3a}$ is hydrogen or halogen, preferably fluorine ;
and $R^2$, $R^{2a}$, $R^4$ and $R^{4a}$ are each hydrogen ;
as their racemates or in enantiomerically enriched form, preferably the pure enantiomers.

A further particular group of compounds of formula I (Compounds 1F) comprises those wherein,
X is-$CA^1NH_2$ ;
$R^1$ is H ;
$R^3$ is C1-6-alkyl, C2-6-alkenyl or C2-6-alkynyl optionally substituted by azido, oxynitro, 1 to 6 halogen atoms ;
$R^{3a}$ is hydrogen or halogen, preferably fluorine ;
and $R^2$, $R^{2a}$ , $R^4$ and $R^{4a}$ are each hydrogen ; as their racemates or in enantiomerically enriched form, preferably the pure enantiomers.

In all the above mentioned scopes when the carbon atom to which $R^1$ is attached is asymmetric it is preferably in the "S"-configuration.

In some instances, compounds useful in the methods and compositions of this disclosure are selected from the group consisting of:

(2S)-2-[4-(bromomethyl)-2-oxo-1-pyrrolidinyl]butanamide;
(2S)-2-[(4R)-4-(iodomethyl)-2-oxopyrrolidinyl]butanamide;
(2S)-2-(2-oxo-4-phenyl-1-pyrrplidinyl)butanamide;
(2S)-2-[4-(iodomethyl)-2-oxo-1-pyrrolidinyl]butanamide;
(2S)-2-[4-(chloromethyl)-2-oxo-1-pyrrolidinyl]butanamide;
{1-[(1S)-1-(aminocarbonyl)propyl]-5-oxo-3-pyrrolidinyl}methyl4-methylbenzenesulfonate;
(2S)-2-[(4R)-4-(azidomethyl)-2-oxopyrrolidinyl]butanamide;

2-[4-(2,2-dibromovinyl)-2-oxo-1-pyrrolidinyl]butanamide;
{1-[(1S)-1-(aminocarbonyl)propyl]-5-oxo-3-pyrrolidinyl}methyl nitrate;
(2S)-2-[2-oxo-4-(1H-tetraazol-1-ylmethyl)-1-pyrrolidinyl]butanamide;
2-(2-oxo-4-vinyl-1-pyrrolidinyl)butanamide;
2-{2-oxo-4-[(phenylsulfonyl)methyl]-1-pyrrolidinyl]butanamide;
(2S)-2-[(4R)-4-(2, 2-dibromovinyl)-2-oxopyrrolidinyl]butanamide;
(2S)-2-[(4S)-4-(2, 2-dibromovinyl)-2-oxopyrrolidinyl]butanamide;
(2S)-2-[4-(isothiocyanatomethyl)-2-oxo-1-pyrrolidinyl]butanamide;
2-[2-oxo-4-(1,3-thiazol-2-yl)-1-pyrrolidinyl]butanamide;
(2S)-2-[2-oxo-4-(2-thienyl)-1-pyrrolidinyl]butanamide;
(2S)-2-[4-(2-methoxyphenyl)-2-oxo-1-pyrrolidinyl]butanamide;
(2S)-2-[4-(3-methoxyphenyl)-2-oxo-1-pyrrolidinyl]butanamide;
(2S)-2-[4-(4-azidophenyl)-2-oxo-1-pyrrolidinyl]butanamide;
(2S)-2-[2-oxo-4-(3-thienyl)-1-pyrrolidinyl]butanamide;
(2S)-2-[4-(3-azidophenyl)-2-oxo-1-pyrrolidinyl]butanamide;
(2S)-2-[2-oxo-4-(3-thienyl)-1-pyrrolidinyl]butanamide;
(2S)-2-[(4S)-2-oxo-4-vinylpyrrolidinyl]butanamide;
(2S)-2-[(4R)-2-oxo-4-vinylpyrrolidinyl]butanamide;
2-[4-(2-bromophenyl)-2-oxo-1-pyrrolidinyl]butanamide;
2-[2-oxo-4-(3-pyridinyl)-1-pyrrolidinyl]butanamide;
(2S)-2-(4-[1,1'-biphenyl]-4-yl-2-oxo-1-pyrrolidinyl)butanamide;
(2S)-2-{4-[(methylsulfanyl)methyl]-2-oxo-1-pyrrolidinyl}butanamide;
2-[4-(iodomethyl)-2-oxo-1-pyrrolidinyl]butanamide;
(2S)-2-[(4R)-4-(iodomethyl)-2-oxo-1-pyrrolidinyl]pentanamide;
(2S)-2-[(4R)-4-(iodomethyl)-2-oxopyrrolidinyl]propanamide;
2-(2-oxo-4-propyl-1-pyrrolidinyl)propanamide;
2-(2-oxo-4-propyl-1-pyrrolidinyl)butanamide;
2-(2-oxo-4-pentyl-1-pyrrolidinyl)butanamide;
(2S)-2-[(4R)-4-(iodomethyl)-2-oxopyrrolidinyl]-N-methylbutanamide;
(2S)-2-(4-neopentyl-2-oxo-1-pyrrolidinyl)butanamide;
(2S)-2-(4-ethyl-2-oxo-1-pyrrolidinyl)butanamide;
2-[4-(2,2-difluorovinyl)-2-oxo-1-pyrrolidinyl]butanamide;
2-[4-(2,2-difluoroethyl)-2-oxo-1-pyrrolidinyl]butanamide;
(2S)-2-[(4S)-2-oxo-4-propylpyrrolidinyl]butanamide;
(2S)-2-[(4R)-2-oxo-4-propylpyrrolidinyl]butanamide;
2-{4-[(Z)-2-fluoroethenyl]-2-oxo-1-pyrrolidinyl}butanamide;
2-[4-(2-methyl-1-propenyl)-2-oxo-1-pyrrolidinyl]butanamide;
2-(4-butyl-2-oxo-1-pyrrolidinyl)butanamide;
2-[4-(cyclopropylmethyl)-2-oxo-1-pyrrolidinyl]butanamide;
2-(4-isobutyl-2-oxo-1-pyrrolidinyl)butanamide;
2-[4-(4-chlorophenyl)-2-oxo-1-pyrrolidinyl]butanamide;
2-[4-(3-chlorophenyl)-2-oxo-1-pyrrolidinyl]butanamide;
2-{2-oxo-4-[2-(trifluoromethyl)phenyl]-1-pyrrolidinyl}butanamide;
2-[4-(2-fluorophenyl)-2-oxo-1-pyrrolidinyl]butanamide;
2-[4-(3-methylphenyl)-2-oxo-1-pyrrolidinyl]butanamide;
(2S)-2-[2-oxo-4-(2-phenylethyl)-1-pyrrolidinyl]butanamide;
(2S)-2-[4-(3-bromophenyl)-2-oxo-1-pyrrolidinyl]butanamide;
2-{4-[3,5-bis(trifluoromethyl)phenyl]-2-oxo-1-pyrrolidinyl}butanamide;
2-[4-(3,4-dichlorophenyl)-2-oxo-1-pyrrolidinyl]butanamide;
2-[4-(2,4-dichlorophenyl)-2-oxo-1-pyrrolidinyl]butanamide;
2-[4-(2-furyl)-2-oxo-1-pyrrolidinyl]butanamide;
(2S)-2-[2-oxo-4-(3-phenylpropyl)-1-pyrrolidinyl]butanamide;
(2S)-2-[4-(3,5-dibromophenyl)-2-oxo-1-pyrrolidinyl]butanamide;
2-[4-(3,4-dichlorophenyl)-2-oxo-1-pyrrolidinyl]butanamide;
2-(2-oxo-4-propyl-1-pyrrolidinyl)butanamide;
2-[4-(3-chlorophenyl)-2-oxo-1-pyrrolidinyl]butanamide;
2-(4-ethynyl-2-oxo-1-pyrrolidinyl) butanamide;
2-[4-(2-fluorophenyl)-2-oxo-1-pyrrolidinyl]butanamide;

(2S)-2-[4-(cyclopropylmethyl)-2-oxo-1-pyrrolidinyl}butanamide;
(2S)-2-[(4S)-4-(2, 2-difluorovinyl)-2-oxopyrrolidinyl]butanamide;
(2S)-2-[2-oxo-4-(3,3, 3-trifluoropropyl)-1-pyrrolidinyl]butanamide;
2-[4-(3-methylphenyl)-2-oxo-1-pyrrolidinyl]butanamide;
(2S)-2-[4-(cyclopropylmethyl)-2-oxo-1-pyrrolidinyl]butanamide;
(2S)-2-[(4R)-4-(2, 2-difluorovinyl)-2-oxopyrrolidinyl]butanamide;
(2S)-2-[2-oxo-4-(1 H-pyrrol-1-yl)-1-pyrrolidinyl]butanamide;
(2S)-2-(4-allyl-2-oxo-1-pyrrolidinyl)butanamide;
(2S)-2-[4-(2-iodopropyl)-2-oxo-1-pyrrolidinyl}butanamide;
(2S)-2-(4-allyl-2-oxo-1-pyrrolidinyl)butanamide;
(2S)-2-[2-oxo-4-(2-oxopropyl)-1-pyrrolidinyl]butanamide;
(2S)-2-[4-(2-bromo-1H-pyrrol-1-yl)-2-oxo-1-pyrrolidinyl]butanamide;
(2S)-2-(4-methyl-2-oxo-4-propyl-1-pyrrolidinyl)butanamide;
(2R)-2-[4-(2, 2-dichlorovinyl)-2-oxo-1-pyrrolidinyl]butanamide;
2-[4-(bromoethynyl)-2-oxo-1-pyrrolidinyl]butanamide;
2-[(4S)-4-(2, 2-difluoropropyl)-2-oxopyrrolidinyl]butanamide;
(2S)-2-[4-(bromoethynyl)-2-oxo-1-pyrrolidinyl]butanamide;
2-(2-oxo-4-propyl-1-pyrrolidinyl)pentanamide;
3-cyclopropyl-2-(2-oxo-4-propyl-1-pyrrolidinyl)propanamide;
2-(2-oxo-4-propyl-1-pyrrolidinyl)-3-(1,3-thiazol-4-yl)propanamide;
2-(2-oxo-4-propyl-1-pyrrolidinyl)-4-pentenamide;
(2S)-2-[(4R)-2-oxo-4-vinylpyrrolidinyl]butanamide;

including all isomeric forms and mixtures thereof or a pharmaceutically acceptable salt thereof.

In some instances, compounds useful in the methods and compositions of this disclosure are selected from the group consisting of:

(2S)-2-[(4S)-4-(2, 2-difluorovinyl)-2-oxopyrrolidinyl]butanamide;

(2S)-2-[(4S)-2-oxo-4-propylpyrrolidinyl]butanamide;

(2S)-2-[(4R)-2-oxo-4-propylpyrrolidinyl]butanamide.

ii) International Patent Application WO 2002/094787:

Compounds of the formula I

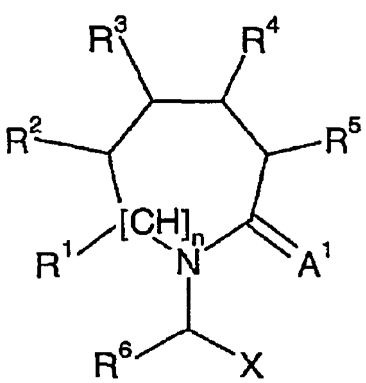

(I)

wherein n represents 0 or 1 whereby $R^1$ is not existent when n=0 and $R^1$ is existent when n= 1 ;

$A^1$ represents an oxygen or a sulfur atom;

X is-$CONR^7R^8$,-$COOR^9$,-CO-$R^{10}$ or CN;

$R^1$ when existent, $R^2$, $R^3$, $R^4$ and $R^5$ are the same or different and each is independently hydrogen, halogen, hydroxy, thiol, amino, nitro, nitrooxy, cyano, azido, carboxy, amido, sulfonic acid, sulfonamide, alkyl, alkenyl, alkynyl, ester, ether, aryl, heterocycle, or an oxy derivative, thio derivative, amino derivative, acyl derivative, sulfonyl derivative or sulfinyl derivative,

provided that at least one of the substituents R chosen from $R^1$ when existent, $R^2$, $R^3$, $R^4$ or $R^5$ is not hydrogen;

$R^6$ is hydrogen, alkyl, aryl or-$CH_2$-$R^{6a}$ wherein $R^{6a}$ is aryl, heterocycle, halogen, hydroxy, amino, nitro or cyano;

$R^7$, $R^8$ and $R^9$ are the same or different and each is independently hydrogen, hydroxy, alkyl, aryl, heterocycle or an oxy derivative; and

$R^{10}$ is hydrogen, hydroxy, thiol, halogen, alkyl, aryl, heterocycle or a thio derivative;

their pharmaceutically acceptable salts, geometrical isomers (including cis and trans, Z and E isomers), enantiomers, diastereoisomers and mixtures thereof (including all possible mixtures of stereoisomers).

In the above formula, at least one substituent $R^1$ to $R^5$ is different from hydrogen. Some non-substituted compounds are referred to in US Patent No. 5,468,733 and 5,516, 759. US Patent No. 5,468,733 refers to non-ring substituted 2-oxo-1-pyrrolidinyl and 2-oxo-1-piperidinyl derivatives as inhibitors of the oncogene Ras protein. In particular, these compounds block the ability of Ras to transform normal cells to cancer cells, and therefore can be included in several chemotherapeutic compositions for treating cancer.

US Patent No. 5,516,759 refers to non-ring substituted 2-oxo-1-pyrrolidinyl, 2-oxo-1- piperidinyl and azepanyl derivatives present at the N-terminus of dodecapeptides possessing LHRH (luteinizing hormone-releasing hormone) antagonistic activity. Such LHRH antagonists are useful in the treatment of a variety of conditions in which suppression of sex steroids plays a key role including contraception, delay of puberty, treatment of benign prostatic hyperplasia a. o.

In the definitions set forth below, unless otherwise stated, $R^{11}$ and $R^{12}$ are the same or different and each is independently amido, alkyl, alkenyl, alkynyl, acyl, ester, ether, aryl, aralkyl. heterocycle or an oxy derivative, thio derivative, acyl derivative, amino derivative, sulfonyl derivative, or sulfinyl derivative, each optionally substituted with any suitable group, including, but not limited to, one or more moieties selected from lower alkyl or other groups as described below as substituents for alkyl.

The term "oxy derivative", as used herein, is defined as including-O-$R^{11}$ groups wherein $R^{11}$ is as defined above except for "oxy derivative". Non-limiting examples are alkoxy, alkenyloxy, alkynyloxy, acyloxy, oxyester, oxyamido, alkylsulfonyloxy, alkylsulfinyloxy, arylsulfonyloxy, arylsulfinyloxy, aryloxy, aralkoxy or heterocyclooxy such as pentyloxy, allyloxy, methoxy, ethoxy, phenoxy, benzyloxy, 2-naphthyloxy, 2-pyridyloxy, methylenedioxy, carbonate.

The term "thio derivative", as used herein, is defined as including-S-$R^{11}$ groups wherein $R^{11}$ is as defined above except for "thio derivative". Non-limiting examples are alkylthio, alkenylthio, alkynylthio and arylthio.

The term "amino derivative", as used herein, is defined as including-$NHR^{11}$ or-$NR^{11}R^{12}$ groups wherein $R^{11}$ and $R^{12}$ are as defined above. Non-limiting examples are mono- or di-alkyl-, alkenyl-, alkynyl-and arylamino or mixed amino.

The term "acyl derivative", as used herein, represents a radical derived from carboxylic acid and thus is defined as including groups of the formula $R^{11}$-CO-, wherein $R^{11}$ is as defined above and may also be hydrogen. Preferred are acyl derivatives of formula -$COR^{11}$ wherein $R^{11}$ is selected from hydrogen, C1-12 alkyl, C2-12 alkenyl, C2-12 alkenyl, heterocyle and aryl. Non-limiting examples are formyl, acetyl, propionyl, isobutyryl, valeryl, lauroyl, heptanedioyl, cyclohexanecarbonyl, crotonoyl, fumaroyl, acryloyl, benzoyl, naphthoyl, furoyl, nicotinoyl, 4-carboxybutanoyl, oxalyl, ethoxalyl, cysteinyl, oxamoyl.

The term "sulfonyl derivative", as used herein, is defined as including a group of the formula -$SO_2$-$R^{11}$, wherein $R^{11}$ is as defined above except for "sulfonyl derivative". Non-limiting examples are alkylsulfonyl, alkenylsulfonyl, alkynylsulfonyl and arylsulfonyl.

The term "sulfinyl derivative", as used herein, is defined as including a group of the formula -SO-$R^{11}$, wherein $R^{11}$ is as defined above except for "sulfinyl derivative". Non-limiting examples are alkylsulfinyl, alkenylsulfinyl, alkynylsulfinyl and arylsulfinyl.

The term "alkyl", as used herein, is defined as including saturated, monovalent hydrocarbon radicals having straight, branched or cyclic moieties or combinations thereof and generally containing 1-20 carbon atoms, most often 1 to 12 carbon atoms, preferably 1-7 carbon atoms for non-cyclic alkyl and 3-7 carbon atoms for cycloalkyl (in these two preferred cases, unless otherwise specified," lower alkyl"), each optionally substituted by, preferably 1 to 5, substituents independently selected from the group consisting of halogen, hydroxy, thiol, amino, nitro, cyano, thiocyanato, acyl,

acyloxy, sulfonyl derivative, sulfinyl derivative, alkylamino, carboxy, ester, ether, amido, azido, cycloalkyl, sulfonic acid, sulfonamide, thio derivative, alkylthio, oxyester, oxyamido, heterocycle, vinyl, alkoxy (preferably C1-5), aryloxy (preferably C6-10) and aryl (preferably C6-10).

Preferred are alkyl groups containing 1 to 7 carbon atoms, each optionally substituted by one or more substituents selected from hydroxy, halogen, cyano, thiocyanato, alkoxy, azido, alkylthio, cyclopropyl, acyl and phenyl. Most preferred are C1-4 alkyl and C3-7 cycloalkyl, each optionally substituted by one or more hydroxy, halogen, lower alkyl or/and azido.

Most preferred alkyl groups are hydroxymethyl, propyl, butyl, 2, 2,2-trifluoroethyl, 2- bromo-2,2-difluoroethyl, 2-chloro-2,2-difluoroethyl, 3,3,3-trifluoropropyl, cyclopropylmethyl, iodomethyl, azidomethyl, 2,2-difluoropropyl, 2-iodo-2,2-difluoroethyl.

The term "lower alkyl", as used herein, and unless otherwise specified, refers to $C_1$ to $C_7$ saturated straight, branched or cyclic hydrocarbon. Non limiting examples are methyl, ethyl, propyl, isopropyl, butyl, tertiobutyl, pentyl, cyclopropyl, cyclopentyl, isopentyl, neopentyl, hexyl, isohexyl, cyclohexyl, 3-methypentyl, 2,2-dimethylbutyl, optionally substituted with any suitable group, including but not limited to one or more moieties selected from groups as described above for the alkyl groups. Preferably, lower alkyl is methyl.

The term "alkenyl", as used herein, is defined as including both branched and unbranched, unsaturated hydrocarbon radicals having at least one double bond, and being optionally substituted by at least one substituent selected from the group consisting of halogen, hydroxy, thiol, amino, thiocyanato, azido, alkylthio, cycloalkyl, acyl, nitro, cyano, aryl and heterocycle.

Prefered alkenyl groups are C2-C12 alkenyls, especially C2-6 alkenyls, such as ethenyl (= vinyl), 1-methyl-1-ethenyl, 2,2-dimethyl-1-ethenyl, 1-propenyl, 2-propenyl (= allyl), 1-butenyl, 2- butenyl, 3-butenyl, 4-pentenyl, 1-methyl-4-pentenyl, 3-methyl-1-pentenyl, 1-hexenyl, 2-hexenyl and the like, optionally being substituted by one or more substituents selected from halogen, cyano, thiocyanato, azido, alkylthio, cycloalkyl, phenyl and acyl. Most prefered is vinyl, optionally substituted by one or more halogen or/and lower alkyl, and especially 2,2- difluorovinyl, 2,2-dibromovinyl and 2,2-dichlorovinyl.

The term "alkynyl" as used herein, is defined as including a monovalent branched or unbranched hydrocarbon radical containing at least one carboncarbon triple bond, for example ethynyl, 2-propynyl (= propargyl), and the like, and being optionally substituted by at least one substituent selected from the group consisting of halogen, hydroxy, thiol, amino, nitro, cyano, aryl, heterocycle, thiocyanato, azido, alkylthio, alkyl and acyl.

Preferred alkynyl groups are C2-12 alkynyl, especially C2-6 alkynyl, optionally being substituted by one or more substituents selected from halogen, cyano, thiocyanato, azido, alkylthio, acyl, aryl such as phenyl and alkyl, preferably cycloalkyl.

Most preferred are ethynyl, propynyl and butynyl, optionally substituted by lower alkyl or/and halogen, and especially 1-propynyl, cyclopropylethynyl, 3-methyl-1-butynyl and 3,3,3- trifluoro-1-propynyl.

When present as bridging groups, alkyl, alkenyl and alkynyl represent straight- or branched chains, C1-12, preferably C1-4-alkylene or C2-12-, preferably C2-4-alkenylene or- alkynylene moieties respectively.

Groups where branched derivatives are conventionally qualified by prefixes such as "n", "sec", "iso" and the like (e. g."n-propyl","sec-butyl") are in the n-form unless otherwise stated.

The term "aryl", as used herein, is defined as including an organic radical derived from an aromatic hydrocarbon consisting of at least one ring, most often 1 to 3 rings and generally containing 6-30 carbon atoms by removal of one hydrogen, such as phenyl and naphthyl, each optionally substituted by one or more substituents independently selected from halogen, hydroxy, thiol, amino, nitro, cyano, acyl, acyloxy, sulfonyl, sulfinyl, alkylamino, carboxy, ester, ether, amido, azido, sulfonic acid, sulfonamide, alkylsulfonyl, alkylsulfinyl, C1-6-alkylthio, oxyester, oxyamido, aryl, C1-6-alkoxy, C6-10-aryloxy, C1-6-alkyl, C1-6-haloalkyl. Aryl radicals are preferably monocyclic or bicyclic containing 6-10 carbon atoms. Preferred aryl groups are phenyl and naphthyl each optionally substituted by one or more substituents independently selected from halogen, nitro, amino, azido, C1-6-alkoxy, C1-6-alkyl, C1-6-haloalkyl, sulfonyl and phenyl.

Preferred aryl is phenyl, optionally substituted by one or more halogen, lower alkyl, azido or nitro, such as 3-chlorophenyl and 3-azidophenyl.

The term "halogen", as used herein, includes an atom of Cl, Br, F, I.

The term "hydroxy", as used herein, represents a group of the formula -OH.

The term "thiol", as used herein, represents a group of the formula -SH.

The term "cyano", as used herein, represents a group of the formula -CN.

The term "nitro", as used herein, represents a group of the formula $-NO_2$.

The term "nitrooxy", as used herein, represents a group of the formula $- ONO_2$.

The term "amino", as used herein, represents a group of the formula $-NH_2$.

The term "azido", as used herein, represents a group of the formula $-N_3$.

The term "carboxy", as used herein, represents a group of the formula - COOH.

The term "sulfonic acid", as used herein, represents a group of the formula $- SO_3H$.

The term "sulfonamide", as used herein, represents a group of the formula - $SO_2NH_2$.

The term "ester", as used herein, is defined as including a group of formula -COO-$R^{11}$ wherein $R^{11}$ is as defined above except oxy derivative, thio derivative or amino derivative. Preferred are esters of formula -COO$R^{11}$ wherein $R^{11}$ is selected from C1-12 alkyl, C2-12 alkenyl, C2-12 alkynyl and aryl. Most preferred are esters where $R^{11}$ is a lower alkyl, especially methyl.

The term "ether" is defined as including a group selected from C1-50-straight or branched alkyl, or C2-50-straight or branched alkenyl or alkynyl groups or a combination of the same, interrupted by one or more oxygen atoms.

The term "amido" is defined as including a group of formula -$CONH_2$ or - CONH$R^{11}$ or -CON$R^{11}R^{12}$ wherein $R^{11}$ and $R^{12}$ are as defined above.

The term "heterocycle", as used herein, is defined as including an aromatic or non aromatic cyclic alkyl, alkenyl, or alkynyl moiety as defined above, having at least one O, S and/or N atom interrupting the carbocyclic ring structure and optionally, one of the carbon of the carbocyclic ring structure may be replaced by a carbonyl, and optionally being substituted with any suitable group, including but not limited to one or more moieties selected from lower alkyl, or other groups as described above for the alkyl groups. Non-limiting examples of heterocycles are pyridyl, furyl, pyrrolyl, thienyl, isothiazolyl, triazolyl, imidazolyl, benzimidazolyl, tetrazolyl, quinazolinyl, quinolizinyl, naphthyridinyl, pyridazinyl, pyrimidinyl, pyrazinyl, quinolyl, isoquinolyl, isobenzofuranyl, benzothienyl, pyrazolyl, indolyl, indolizinyl, purinyl, isoindolyl, carbazolyl, thiazolyl, 1,2,4-thiadiazolyl, thiomorpholinyl, thieno (2,3-b) furanyl, furopyranyl, benzofuranyl, benzoxepinyl, isooxazolyl, oxazolyl, thianthrenyl, benzothiazolyl, or benzoxazolyl, cinnolinyl, phthalazinyl, quinoxalinyl, 1-oxidopyridyl, phenanthridinyl, acridinyl, perimidinyl, phenanthrolinyl, phenothiazinyl, furazanyl, benzodioxolyl, isochromanyl, indolinyl, xanthenyl, hypoxanthinyl, pteridinyl, 5-azacytidinyl, 5-azauracilyl, triazolopyridinyl, imidazolopyridinyl, pyrrolopyrimidinyl, pyrazolopyrimidinyl, tetrahydrofuranyl, tetrahydropyranyl, piperidinyl, piperidyl, piperazinyl, imidazolidinyl, morpholino, morpholinyl, 1-oxaspiro (4.5) dec-2-yl, pyrrolidinyl, 2-oxo-pyrrolidinyl, sugar moieties (i. e. glucose, pentose, hexose, ribose, fructose, which may also be substituted) optionally substituted by alkyl or as described above for the alkyl groups. The term"heterocycle"also includes bicyclic, tricyclic and tetracyclic, spiro groups in which any of the above heterocyclic rings is fused to one or two rings independently selected from an aryl ring, a cyclohexane ring, a cyclohexene ring, a cyclopentane ring, a cyclopentene ring or another monocyclic heterocyclic ring or where a monocyclic heterocyclic group is bridged by an alkylene group, such as quinuclidinyl, 7-azabicyclo (2.2.1) heptanyl, 7-oxabicyclo (2.2.1) heptanyl, 8-azabicyclo (3.2.1) octanyl.

The heterocycle is preferably selected from triazolyl, tetrazolyl, pyrrolidinyl, pyridyl, 1- oxidopyridyl, thiomorpholinyl, benzodioxolyl, furyl, oxazolyl, pyrimidinyl, pyrrolyl, thiadiazolyl, thiazolyl, thienyl and piperazinyl, each optionally substituted by one or more substituents selected from halogen, alkyl, substituted alkyl, alkoxy, nitro, amino, acyl and phenyl.

More preferably the heterocycle is selected from tetrazolyl, pyrrolidinyl, pyridyl, furyl, pyrrolyl, thiazolyl and thienyl, each optionally substituted by one or more substituents selected from halogen, alkyl, halogen substituted alkyl, acyl, alkoxy, nitro, amino and phenyl, and especially from 2-and 3-thienyl, optionally substituted by one or more halogen, acyl such as formyl, cyano and/or lower alkyl, such as methyl.

In the above definitions it is to be understood that when a substituent such as $R^1$, $R^2$, $R^3$, $R^4$, $R^5$, $R^7$, $R^8$, $R^9$, $R^{10}$ is attached to the rest of the molecule *via* a heteroatom or a carbonyl, a straight- or branched chain, C1-12-, preferably C1-4-alkylene or C2-12, preferably C2-4-alkenylene or-alkynylene bridge may optionally be interposed between the heteroatom or the carbonyl and the point of attachment to the rest of the molecule.

The term"R substituent"refers to $R^1$, $R^2$, $R^3$, $R^4$ or $R^5$, independently.

According to a preferred instance, a compound of formula I is as defined above wherein n represents 0. The compound is a 6-ring structure (2- thioxo- or 2-oxo-piperidinyl derivative) wherein $R^1$ is not existent since n=0, and is depicted by the formula (I-A).

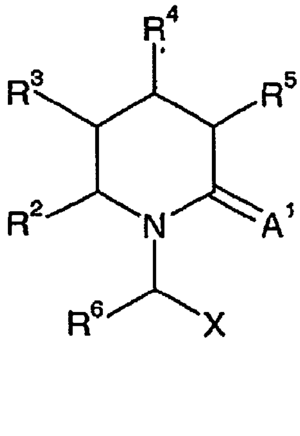

(I-A)

According to a following instance, the compound of formula I is as defined above wherein n represents 1. The compound is a 7-ring structure (2-thioxo- or 2-oxo-azepanyl derivative) wherein $R^1$ is existent since n=1 and depicted by the formula (I-B).

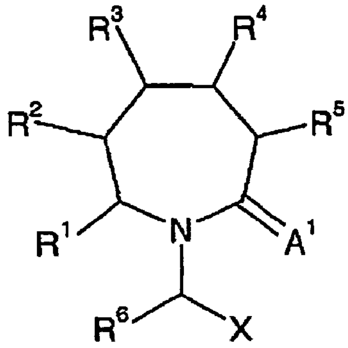

(I-B)

According to a more preferred instance, said compound is as defined above wherein n=0, $R^3$ and/or $R^4$ are different from hydrogen and $R^2$ and $R^5$ represent hydrogen.

According to another more preferred instance, said compound is as defined above wherein n=1, $R^2$, $R^3$ and/or $R^4$ are different from hydrogen and wherein $R^1$ and $R^5$ represent hydrogen.

According to a yet more preferred instance, said compound is as defined above wherein only one R substituent chosen from $R^3$ or $R^4$ when n=0 or from $R^2$, $R^3$ or $R^4$ when n=1, is different from hydrogen and the remaining R substituent(s) is/are hydrogen. We hereby refer to a mono-substituted 2-thioxo-or 2-oxo-piperidinyl or 2-thioxo- or 2-oxo-azepanyl derivatives.

According to another preferred instance, compounds of formula I are as defined above wherein $A^1$ represents an oxygen atom. We hereby refer to 2-oxo-piperidinyl or 2-oxo-azepanyl derivatives.

According to another preferred instance, compounds of formula I are as defined above wherein X is $CONR^7R^8$, especially $CONH_2$. We hereby refer to amido derivatives of 2-oxo (or thioxo)-piperidinyl or 2-oxo (or thioxo) - azepanyl.

According to another preferred instance, compounds of formula I are as defined above wherein $R^6$ represents hydrogen, C1-4 alkyl, or a $CH_2$-$R^{6a}$ group wherein $R^{6a}$ represents a heterocycle. Most preferably $R^6$ is a C1-4 alkyl, especially ethyl. When $R^6$ is ethyl we refer to 2- (2-oxo (or thioxo)-1-piperidinyl) butanamide or 2- (2-oxo (or thioxo)-1-azepanyl) butanamide derivatives.

According to another preferred instance, compounds of formula I are as defined above wherein the carbon atom to which $R^6$ is attached is of the S configuration. In case where $R^6$ is ethyl, A is oxygen and X is $CONR^7R^8$ we refer then to (2S)-2-(2-oxo-1-piperidinyl) butanamide or (2S)-2- (2-oxo-1-azepanyl) butanamide derivatives.

According to a prefered instance, the compound is as defined above wherein $R^2$ when n=1, $R^3$ and $R^4$ are the same or different and each is independently hydrogen, halogen, nitro, nitrooxy, cyano, carboxy, amido, sulfonic acid, sulfonamide, alkyl, alkenyl, alkynyl, ester, ether, aryl, heterocycle, acyl derivative, sulfonyl derivative or sulfinyl derivative;
$R^1$ when existent, $R^2$ when n=0 and $R^5$ are hydrogen;
$R^6$ is hydrogen, alkyl, aryl or-$CH_2$-$R^{6a}$ wherein $R^{6a}$ is aryl, heterocycle, halogen, hydroxy, amino, nitro or cyano;

According to this preferred instance, the compound is generally such that when $R^6$ is benzyl, X is-$COOCH_3$ and n=1, $R^2$ is different from methyl when $R^3$ and $R^4$ are both hydrogen and $R^4$ is different from methyl when $R^2$ and $R^3$ are both hydrogen.

According to another preferred instance, the compound is as defined above wherein $R^2$ when n=1, $R^3$ and $R^4$ are the same or different and each is independently hydrogen; cyano; carboxy; amido ;
C1-12 alkyl, each optionally substituted by one or more substituents selected from hydroxy, halogen, cyano, thiocyanato, alkoxy, azido, alkyltio, cycloalkyl, acyl, aryl and heterocycle;
C2-12 alkenyl, each optionally substituted by one or more substituents selected from halogen, cyano, thiocyanato, azido, alkylthio, alkyl, aryl and acyl;
C2-12 alkynyl, each optionally substituted by one or more substituents selected from halogen, cyano, thiocyanato, azido, alkylthio, alkyl, aryl and acyl ; acyl derivative of formula -CO-$R^{11}$, wherein $R^{11}$ is selected from C1-12 alkyl, C2-12 alkenyl, C2-12 alkynyl, heterocycle and aryl;
ester of formula -CO-O-$R^{11}$ wherein $R^{11}$ is selected from C1-12 alkyl, C2-12 alkenyl, C2-12 alkynyl and aryl;
heterocycle selected from triazolyl, tetrazolyl, pyrrolidinyl, pyridyl, 1-oxidopyridyl, thiomorpholinyl, benzodioxolyl, furyl, oxazolyl, pyrimidinyl, pyrrolyl, thiadiazolyl, thiazolyl, thienyl and piperazinyl, each optionally substituted by one or more substituents selected from halogen, alkyl, substituted alkyl, alkoxy, nitro, amino, acyl and phenyl;
aryl, each optionally substitued by one or more substituents selected from C1-6 alkyl, C1-6 haloalkyl, C1-6 alkoxy, C1-6

alkylthio, amino, azido, sulfonyl, aryl and nitro.

According to another preferred instance, the compound is as defined above, wherein $R^2$ when n= 1, $R^3$ and $R^4$ are the same or different and each is independently hydrogen;

C1-7 alkyl, each optionally substituted by one or more substituents selected from hydroxy, halogen, cyano, thiocyanato, alkoxy, azido, alkyltio, cyclopropyl, acyl and phenyl;

C2-6 alkenyl, each optionally substituted by one or more substituents selected from halogen, cyano, thiocyanato, azido, alkylthio, cycloalkyl, phenyl and acyl ;

C2-6 alkynyl, each optionally substituted by one or more substituents selected from halogen, cyano, thiocyanato, azido, alkylthio, cycloalkyl, phenyl and acyl;

heterocycle selected from tetrazolyl, pyrrolidinyl, pyridyl, furyl, pyrrolyl, thiazolyl and thienyl, each optionally substituted by one or more substituents selected from halogen, alkyl, halogen substituted alkyl, acyl, alkoxy, nitro, amino and phenyl;

phenyl, each optionally substitued by one or more substituents selected from C1-6 alkyl, halogen substituted alkyl, halogen, alkoxy, amino, azido, sulfonyl, phenyl and nitro.

According to another preferred instance, the compound is as defined above wherein at least one of the R substituents chosen from the group $R^2$, $R^3$ and $R^4$ when n=1 or from the group $R^3$ and $R^4$ when n=0, represents independently C1-4-alkyl or C3-7-cycloalkyl, optionally substituted by one or more halogen, hydroxy, lower alkyl and/or azido.

According to another preferred instance, the compound is as defined above wherein at least one of the R substituents chosen from the group $R^2$, $R^3$ and $R^4$ when n=1 or from the group $R^3$ and $R^4$ when n=0, represents independently vinyl, optionally substituted by one or more halogen or/and lower alkyl.

According to another preferred instance, the compound is as defined above wherein at least one of the R substituents chosen from the group $R^2$, $R^3$ and $R^4$ when n=1 or from the group $R^3$ and $R^4$ when n=0, represents independently ethynyl, propynyl or butynyl, optionally substituted by one or more halogen and/or lower alkyl.

According to another preferred instance, the compound is as defined above wherein at least one of the R substituents chosen from the group $R^2$, $R^3$ and $R^4$ when n= 1 or from the group $R^3$ and $R^4$ when n=0, represents independently phenyl, optionally substituted by one or more halogen, lower alkyl, azido and/or nitro.

According to another preferred instance, the compound is as defined above wherein at least one of the R substituents chosen from the group $R^2$, $R^3$ and $R^4$ when n=1 or from the group $R^3$ and $R^4$ when n=0, represents independently 2-or 3-thienyl, optionally substituted by one or more halogen, acyl, cyano or/and lower alkyl.

According to a particular preferred instance, the compound is as defined above wherein at least one of the R substituents chosen from the group $R^3$, $R^4$ and $R^2$ when n= 1 or from the group $R^3$ and $R^4$ when n=0, is hydroxymethyl, propyl, butyl, 3,3,3-trifluoropropyl, 2,2,2-trifluoroethyl, cyclopropylmethyl, iodomethyl, azidomethyl, 2- thienyl, 3-thienyl, phenyl, 3-chlorophenyl, 3-azidophenyl, 2,2-difluorovinyl, 2,2-dibromovinyl, 2, 2-dichlorovinyl, 2-ethynyl, 5-methyl-2-thienyl, 5-formyl-2-ethynyl, 5-cyano-2-thienyl, 3-bromo-2-thienyl, 4-methyl-2-thienyl, 3,3,3-trifluoro-1-propynyl, 1-propynyl, cyclopropylethynyl, 3- methyl-1-butynyl, 1-butynyl, 2,2-difluoropropyl, 2-chloro-2,2-difluoroethyl, 2-bromo-2,2- difluoroethyl and 2-iodo-2,2-difluoroethyl.

According to yet another preferred instance, the compound is as defined above wherein $R^1$, $R^2$, $R^4$ and $R^5$ are hydrogen.

According to even another preferred instance, the compound is as defined above wherein $R^1$, $R^2$, $R^3$ and $R^5$ are hydrogen.

According to even another preferred instance, the compound is as defined above wherein n=1 and $R^1$, $R^3$, $R^4$ and $R^5$ are hydrogen.

In all the above-mentioned scopes when the carbon atom to which $R^6$ is attached is asymmetric it is preferably in the"S"-configuration.

Representative compounds useful in the methods and compositions of this disclosure as defined above are selected from the group consisting of

2-[5-(hydroxymethyl)-2-oxo-1-piperidinyl]butanamide,
2-(2-oxo-5-propyl-1-piperidinyl)butanamide,
2-[2-oxo-5-(3,3,3-trifluoropropyl)-1-piperidinyl]butanamide,
2-[5-(cyclopropylmethyl)-2-oxo-1 -piperidinyl]butanamide,
2-[5-(iodomethyl)-2-oxo-1-piperidinyl] butanamide,
2-[5-(azidomethyl)-2-oxo-1-piperidinyl]butanamide,
2-(2-oxo-5-phenyl-1-piperidinyl)butanamide,
2-[2-oxo-5-(2-thienyl)-1-piperidinyl]butanamide,
2-[2-oxo-5-(3-thienyl)-1-piperidinyl]butanamide,
2-[5-(3-chlorophenyl)-2-oxo-1-piperidinyl]butanamide,
2-[5-(3-azidophenyl)-2-oxo-1-piperidinyl]butanamide,

2-[5-(2, 2-difluorovinyl)-2-oxo-1-piperidinyl]butanamide,
2-[5-(2, 2-dibromovinyl)-2-oxo-1-piperidinyl]butanamide,
2-[5-(2, 2- dichlorovinyl)-2-oxo-1-piperidinyl]butanamide,
2-(5-ethynyl-2-oxo-1-piperidinyl)butanamide,
2[5-(5-methyl-2-thienyl)-2-oxo-1-piperidinyl]butanamide,
2-[5-(5-formyl-2-thienyl)-2-oxo-1-piperidinyl]butanamide,
2-[5-(5-cyano-2-thienyl)-2-oxo-1-piperidinyl]butanamide,
2-[5-(3-bromo-2-thienyl)-2-oxo-1-piperidinyl]butanamide,
2-[5-(4-methyl-2-thienyl)-2-oxo-1-piperidinyl]butanamide,
2-[2-oxo-5-(3,3,3-trifluoro-1-propynyl)-1-piperidinyl]butanamide,
2-[2-oxo-5-(1-propynyl)-1-piperidinyl]butanamide,
2-[5-(cyclopropylethynyl)-2-oxo-1-piperidinyl]butanamide,
2-[5-(3-methyl-1-butynyl)-2-oxo-1-piperidinyl]butanamide,
2-[5-(1-butynyl)-2-oxo-1-piperidinyl]butanamide,
2-[5-(2,2-difluoropropyl)-2-oxo-1-piperidinyl]butanamide,
2-[5-(2-chloro-2,2-difluoroethyl)-2-oxo-1-piperidinyl]butanamide,
2-[5-(2-bromo-2,2-difluoroethyl)-2-oxo-1-piperidinyl]butanamide,
2-[4-(hydroxymethyl)-2-oxo-1-piperidinyl]butanamide,
2-(2-oxo-4-propyl-1-piperidinyl)butanamide,
2-[2-oxo-4-(3,3,3trifluoropropyl)-1-piperidinyl]butanamide,
2-[4-(cyclopropylmethyl)-2-oxo-1-piperidinyl]butanamide,
2-[4-(iodomethyl)-2-oxo-1-piperidinyl]butanamide,
2-[4-(azidomethyl)-2-oxo-1-piperidinyl]butanamide,
2-(2-oxo-4-phenyl-1-piperidinyl)butanamide,
2-[2-oxo-4-(2-thienyl)-1-piperidinyl]butanamide,
2-[2-oxo-4-(3-thienyl)-1-piperidinyl]butanamide,
2-[4-(3-chtorophenyl)-2-oxo-1-piperidinyl]butanamide,
2-[4-(3-azidophenyl)-2-oxo-1-piperidinyl]butanamide,
2-[4-(2,2-difluorovinyl)-2-oxo-1-piperidinyl]butanamide,
2-[4-(2,2-dibromovinyl)-2-oxo-1-piperidinyl]butanamide,
2-[4-(2,2-dichlorovinyl)-2-oxo-1-piperidinyl]butanamide,
2-(4-ethynyl-2-oxo-1-piperidinyl)butanamide,
2-[4-(5-methyl-2-thienyl)-2-oxo-1-piperidinyl]butanamide,
2-[4-(5-formyl-2-thienyl)-2-oxo-1-piperidinyl]butanamide,
2-[4-(5-cyano-2-thienyl)-2-oxo-1-piperidinyl]butanamide,
2-[4-(3-bromo-2-thienyl)-2-oxo-1-piperidinyl]butanamide,
2-[4-(4-methyl-2-thienyl)-2-oxo-1-piperidinyl]butanamide,
2-[2-oxo-4-(3,3,3-trifluoro-1-propynyl)-1-piperidinyl]butanamide,
2-[2-oxo-4-(1-propynyl)-1-piperidinyl]butanamide,
2-[4-(cyclopropylethynyl)-2-oxo-1-piperidinyl]butanamide,
2-[4-(3-methyl-1-butynyl)-2-oxo-1-piperidinyl]butanamide,
2-[4-(1-butynyl)-2-oxo-1-piperidinyl]butanamide,
2-[4-(2, 2-difluoropropyl)-2-oxo-1-piperidinyl]butanamide,
2-[4-(2-chloro-2,2-difluoroethyl)-2-oxo-1-piperidinyl]butanamide,
2-[4-(2-bromo-2,2-difluoroethyl)-2-oxo-1-piperidinyl]butanamide,
2[4-(2,2,2-trifluoroethyl)-2-oxo-1-piperidinyl]butanamide,
2-[5-(hydroxymethyl)-2-oxo-1-azepanyl]butanamide,
2-(2-oxo-5-propyl-1-azepanyl)butanamide,
2-[2-oxo-5-(3,3,3-trifluoropropyl)-1-azepanyl]butanamide,
2-[5-(cyclopropylmethyl)-2-oxo-1-azepanyl]butanamide,
2-[5-(iodomethyl)-2-oxo-1-azepanyl]butanamide,
2-[5-(azidomethyl)-2-oxo-1-azepanyl]butanamide,
2-(2-oxo-5-phenyl-1-azepanyl)butanamide,
2-[2-oxo-5-(2-thienyl)-1-azepanyl]butanamide,
2-[2-oxo-5-(3-thienyl)-1-azepanyl]butanamide,
2-[5-(3-chlorophenyl)-2-oxo-1-azepanyl]butanamide,
2-[5-(3-azidophenyl)-2-oxo-1-azepanyl]butanamide,
2-[5-(2,2-dinuorovinyl)-2-oxo-1-azepanyl]butanamide,

2-[5-(2,2-dibromovinyl)-2-oxo-1-azepanyl]butanamide,
2-[5-(2,2-dichlorovinyl)-2-oxo-1-azepanyl]butanamide,
2-(5-ethynyl-2-oxo-1-azepanyl)butanamide,
2-[5-(5-methyl-2-thienyl)-2-oxo-1-azepanyl]butanamide,
2-[5-(5-formyl-2-thienyl)-2-oxo-1-azepanyl]butanamide,
2-[5-(5-cyano-2-thienyl)-2-oxo-1-azepanyl]butanamide,
2-[5-(3-bromo-2-thienyl)-2-oxo-1-azepanyl]butanamide,
2-[5-(4-methyl-2-thienyl)-2-oxo-1-azepanyl]butanamide,
2-[2-oxo-5-(3,3,3-trifluoro-1-propynyl)-1-azepanyl]butanamide,
2-[2-oxo-5-(1-propynyl)-1-azepanyl]butanamide,
2-[5-(cyclopropylethynyl)-2-oxo-1-azepanyl]butanamide,
2-[5-(3-methyl-1-butynyl)-2-oxo-1-azepanyl]butanamide,
2-[5-(1-butynyl)-2-oxo-1-azepanyl]butanamide,
2-[5-(2,2-difluoropropyl)-2-oxo-1-azepanyl]butanamide,
2-[5-(2-chloro-2,2-difluoroethyl)-2-oxo-1-azepanyl]butanamide,
2-[5-(2-bromo-2,2-difluoroethyl)-2-oxo-1-azepanyl]butanamide,
2-[5-(2,2,2-trifluoroethyl)-2-oxo-1-azepanyl]butanamide,
2-[6-(hydroxymethyl)-2-oxo-1-azepanyl]butanamide,
2-(2-oxo-6-propyl-1-azepanyl)butanamide,
2-[2-oxo-6-(3,3,3-trifluoropropyl)-1-azepanyl]butanamide,
2-[6-(cyclopropylmethyl)-2-oxo-1-azepanyl]butanamide,
2-[6-(iodomethyl)-2-oxo-1-azepanyl]butanamide,
2-[6-(azidomethyl)-2-oxo-1-azepanyl]butanamide,
2-(2-oxo-6-phenyl-1-azepanyl)butanamide,
2-[2-oxo-6-(2-thienyl)-1-azepanyl]butanamide,
2-[2-oxo-6-(3-thienyl)-1-azepanyl]butanamide,
2-[6-(3-chlorophenyl)-2-oxo-1-azepanyl]butanamide,
2-[6-(3-azidophenyl)-2-oxo-1-azepanyl]butanamide,
2-[6-(2,2-difluorovinyl)-2-oxo-1-azepanyl]butanamide,
2-[6-(2,2-dibromovinyl)-2-oxo-1-azepanyl]butanamide,
2-[6-(2,2-dichlorovinyl)-2-oxo-1-azepanyl]butanamide,
2-(6-ethynyl-2-oxo-1-azepanyl)butanamide,
2-[6-(5-methyl-2-thienyl)-2-oxo-1-azepanyl]butanamide,
2-[6-(5-formyl-2-thienyl)-2-oxo-1-azepanyl]butanamide,
2-[6-(5-cyano-2-thienyl)-2-oxo-1-azepanyl]butanamide,
2-[6-(3-bromo-2-thienyl)-2-oxo-1-azepanyl]butanamide,
2-[6-(4-methyl-2-thienyl)-2-oxo-1-azepanyl]butanamide,
2-[2-oxo-6-(3,3, 3-trifluoro-1-propynyl)-1-azepanyl]butanamide,
2-[2-oxo-6-(1-propynyl)-1-azepanyl]butanamide,
2-[6-(cyclopropylethynyl)-2-oxo-1-azepanyl]butanamide,
2-[6-(3-methyl-1-butynyl)-2-oxo-1-azepanyl]butanamide,
2-[6-(1-butynyl)-2-oxo-1-azepanyl]butanamide,
2-[6-(2, 2-difluoropropyl)-2-oxo-1-azepanyl]butanamide,
2-[6-(2-chloro-2,2-difluoroethyl)-2-oxo-1-azepanyl]butanamide,
2-[6-(2-bromo-2,2-difluoroethyl)-2-oxo-1-azepanyl]butanamide,
2-[6-(2,2,2-trifluoroethyl)-2-oxo-1-azepanyl]butanamide,
2-[4-(hydroxymethyl)-2-oxo-1-azepanyl]butanamide,
2-(2-oxo-4-propyl-1-azepanyl)butanamide,
2-[2-oxo-4-(3,3,3-trifluoropropyl)-1-azepanyl]butanamide,
2-[4-(cyclopropylmethyl)-2-oxo-1-azepanyl]butanamide,
2-[4-(iodomethyl)-2-oxo-1-azepanyl]butanamide,
2-[4-(azidomethyl)-2-oxo-1-azepanyl]butanamide,
2-(2-oxo-4-phenyl-1-azepanyl)butanamide,
2-[2-oxo-4-(2-thienyl)-1-azepanyl]butanamide,
2-[2-oxo-4-(3-thienyl)-1-azepanyl]butanamide,
2-[4-(3-chlorophenyl)-2-oxo-1-azepanyl]butanamide,
2-[4-(3-azidophenyl)-2-oxo-1-azepanyl]butanamide,
2-[4-(2, 2-difluorovinyl)-2-oxo-1-azepanyl]butanamide,

2-[4-(2, 2-dibromovinyl)-2-oxo-1-azepanyl]butanamide,
2-[4-(2,2-dichlorovinyl)-2-oxo-1-azepanyl]butanamide,
2-(4-ethynyl-2-oxo-1-azepanyl)butanamide,
2-[4-(5-methyl-2-thienyl)-2-oxo-1-azepanyl]butanamide,
2-[4-(5-formyl-2-thienyl)-2-oxo-1-azepanyl]butanamide,
2-[4-(5-cyano-2-thienyl)-2-oxo-1-azepanyl]butanamide,
2-[4-(3-bromo-2-thienyl)-2-oxo-1-azepanyl]butanamide,
2-[4-(4-methyl-2-thienyl)-2-oxo-1-azepanyl]butanamide,
2-[2-oxo-4-(3,3,3-trifluoro-1-propynyl)-1-azepanyl]butanamide,
2-[2-oxo-4-(1-propynyl)-1-azepanyl]butanamide,
2-[4-(cyclopropylethynyl)-2-oxo-1-azepanyl]butanamide,
2-[4-(3-methyl-1-butynyl)-2-oxo-1-azepanyl]butanamide,
2-[4-(1-butynyl)-2-oxo-1-azepanyl]butanamide,
2-[4-(2,2-difluoropropyl)-2-oxo-1-azepanyl]butanamide,
2-[4-(2-chloro-2,2-difluoroethyl)-2-oxo-1-azepanyl]butanamide,
2-[4-(2-bromo-2,2-difluoroethyl)-2-oxo-1-azepanyl]butanamide,
2-[4-(2,2,2-tritluoroethyl)-2-oxo-1-azepanyl]butanamide.

In some instances, compounds useful in the methods and compositions of this disclosure are selected from the group consisting of:

(2S)-2-[5-(iodomethyl)-2-oxo-1-piperidinyl]butanamide,

(2S)-2-[5-(azidomethyl)-2-oxo-1-piperidinyl]butanamide,

2-(2-oxo-5-phenyl-1-piperidinyl]butanamide,

(2S)-2-[4-(iodomethyl)-2-oxo-1-piperidinyl]butanamide,

2-[5-(iodomethyl)-2-oxo-1-azepanyl]butanamide.

iii) International Patent Application WO 2004/087658:

A compound having the formula I or a pharmaceutically acceptable salt thereof or stereoisomeric forms thereof,

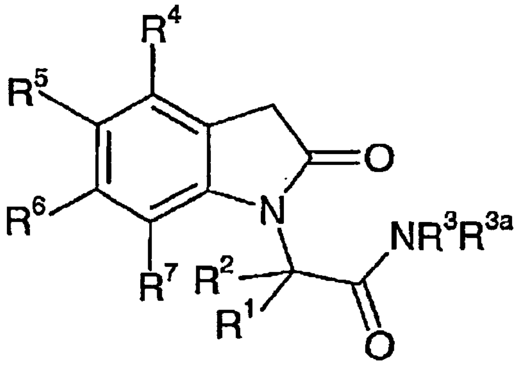

(I)

wherein

$R^1$ is hydrogen,

$R^2$ is hydrogen or C1-20-alkyl,

$R^3$ is hydrogen, C1-20-alkyl, C4-8-cycloalkyl, C5-8-cycloalkenyl, aryl, aromatic or non aromatic heterocycle, C1-20-alkoxy, or a group of formula - W-$R^8$, $R^{3a}$ is hydrogen, C1-20-alkyl or a group of formula :

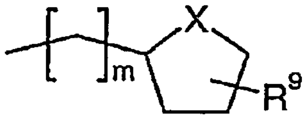

,

or $NR^3R^{3a}$ is a group of formula

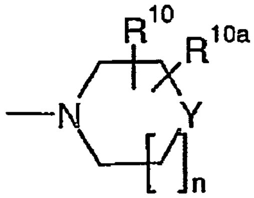

or

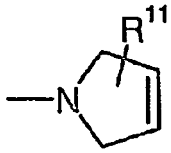

,

$R^4$ is hydrogen,

$R^5$ is hydrogen; nitro; halogen; azido; cyano; -S-C1-4-alkyl; -SO-C1-4-alkyl; $-SO_2$-C1-4-alkyl; $-SONH_2$; C1-20-alkyl unsubstituted or substituted by halogen; or C1-20-alkoxy unsubstituted or substituted by halogen,

$R^6$ is hydrogen, C1-20-alkyl or halogen,

$R^7$ is hydrogen, C 1-20-alkyl or halogen,

W is C1-12-alkylene, -NH- or -NHC(=O)-,

X is O, S or NH,

Y is O, S, $-CR^{12}R^{13}$-, $-NR^{14}$- or -C(=O)-,

$R^8$ is aryl or heterocycle,

$R^9$, $R^{10}$, $R^{10a}$ and $R^{11}$ are independently selected from hydrogen, C1-4-alkyl, halogen, hydroxy or methoxycarbonyl,

or $R^{10}$ and $R^{10a}$ together form a C3-6-alkylene,

$R^{12}$ is hydrogen, C1-4-alkyl, halogen or hydroxy,

$R^{13}$ is hydrogen,

or $CR^{12}R^{13}$ is dioxolanyl,

$R^{14}$ is aryl, heterocycle or a group of formula $-V-R^{15}$,

V is $C_{1-12}$-alkylene,

$R^{15}$ is aryl or heterocycle,

m is 1 to 4,

n is 0 or 1,

and at least one of $R^5$, $R^6$ or $R^7$ is different from hydrogen when $R^2$ is hydrogen, $R^3$ is H or 2, 6-diisopropylphenyl, and $R^{3a}$ is H.

In another aspect, the compound has the formula I or a pharmaceutically acceptable salt thereof or stereoisomeric forms thereof,

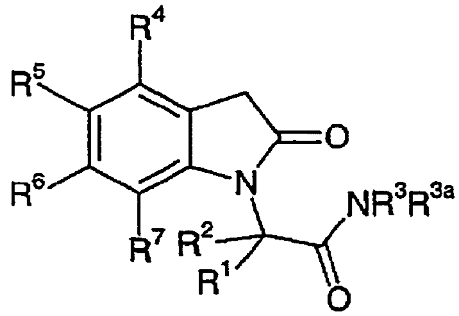 (I)

wherein

$R^1$ is hydrogen,

$R^2$ is hydrogen or C1-20-alkyl,

$R^3$ is hydrogen, C1-20-alkyl, C4-8-cycloalkyl, C5-8-cycloalkenyl, aryl, aromatic or non aromatic heterocycle, C1-20-alkoxy, or a group of formula - W-$R^8$,

$R^{3a}$ is hydrogen, C1-20-alkyl or a group of formula:

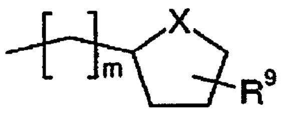,

or $NR^3R^{3a}$ is a group of formula

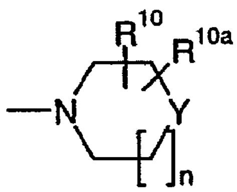

or

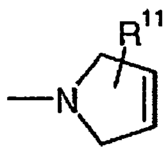,

$R^4$ is hydrogen,

$R^5$ is hydrogen; nitro; halogen; C1-20-alkyl unsubstituted or substituted by halogen; or C1-20-alkoxy unsubstituted or substituted by halogen,

$R^6$ is hydrogen, C1-20-alkyl or halogen,

$R^7$ is hydrogen, C1-20-alkyl or halogen,

W is C1-12-alkylen -NH- or -NHC(=O)-,

X is O, S or NH,

Y is O, S, -$CR^{12}R^{13}$-, -$NR^{14}$- or -C(=O)-,

$R^8$ is aryl or heterocycle,

$R^9$, $R^{10}$ , $R^{10a}$ and $R^{11}$ are independently selected from hydrogen, C1-4-alkyl, halogen, hydroxy or methoxycarbonyl,

or $R^{10}$ and $R^{10a}$ together form a C3-6-alkylene,

$R^{12}$ is hydrogen, C1-4-alkyl, halogen or hydroxy,

$R^{13}$ is hydrogen,

or $CR^{12}R^{13}$ is dioxolanyl,

$R^{14}$ is aryl, heterocycle or a group of formula -V-$R^{15}$,

V is C1-12-alkylene,

$R^{15}$ is aryl or heterocycle,

m is 1 to 4,

n is 0 or 1,

and at least one of $R^5$, $R^6$ or $R^7$ is different from hydrogen when $R^2$ is hydrogen, $R^3$ is H or 2,6-diisopropylphenyl, and $R^{3a}$ is H.

The term "alkyl", as used herein, is defined as including saturated, monovalent hydrocarbon radicals having straight, branched or cyclic moieties or combinations thereof and containing 1-20 carbon atoms, preferably 1-6 carbon atoms and more preferably 1-4 carbon atoms for non-cyclic alkyl and 3-8 carbon atoms for cycloalkyl. Alliyl moieties may optionally be substituted by 1 to 5 substituents independently selected from halogen, hydroxy, alkoxy, alkoxycarbonyl, ester or alkylamino. Preferred alkyl groups are methyl, ethyl, n-propyl, isopropyl, trifluoromethyl, n-butyl, 2- fluoroethyl, 3-hydroxypropyl, 3-hydroxy-2, 2-dimethylpropyl, 1-(hydroxymethyl) propyl, 3,3, 3-trifluoro-2-hydroxypropyl, 3-ethoxypropyl, 2-ethoxy-2-oxoethyl and 3- (dimethylamino) propyl.

The term "cycloalkyl", as used herein, refers to a monovalent group of 3 to 18 carbon atoms, preferably 4-8 carbon atoms, derived from a saturated cyclic or polycyclic hydrocarbon which may be substituted by any suitable group including but not limited to one or more moieties selected from groups as described above for the alkyl groups. Preferred cycloalkyl group is cycloheptyl.

The term "alkylene", as used herein, represents a divalent alkyl group, having straight or branched moieties, containing 1-12 carbon atoms, preferably 1-6 carbon atoms, and being optionally substituted with any suitable group, including but not limited to one or more moieties selected from groups as described above for the alkyl groups. Preferred alkylene groups are methylene, ethylene, hydroxyethylene, trimethylene or propylene.

The term "cycloalkenyl", as used herein, is defined as a cyclic unsaturated hydrocarbon radical having at least one double bond, containing 4-20 carbon atoms, preferably 5-8 carbon atoms, and being optionally substituted with any suitable group, including but not limited to one or more moieties selected from groups as described above for the alkyl groups. Preferred cycloalkenyl group is 6- (hydroxymethyl) cyclohex-3-en-1-yl.

The term "aryl", as used herein, is defined as including an organic radical derived from an aromatic hydrocarbon consisting of 1-3 rings and containing 6-30 carbon atoms by removal of one hydrogen, such as phenyl and naphthyl each optionally substituted by 1 to 5 substituents independently selected from halogen, hydroxy, nitro, C1-6-alkyl, C1-6-alkoxy, C1-6-alkylsulfonyl, trifluoromethylthio or pyridinylalkyl. Aryl radicals are preferably phenyl radicals. Preferred aryl groups are phenyl, 3-hydroxyphenyl, 3-fluorophenyl, 3-methylphenyl, 4-methylphenyl, 4- hydroxyphenyl, 4-hydroxy-3-methoxyphenyl, 3-(2-pyridin-2-ylethyl) phenyl, 3,4- dimethylphenyl, 4-tert-butylphenyl, 4-methylsulfonylphenyl, 2-nitrophenyl, 2-chloro- 6-fluorophenyl, 2-[(trifluoromethyl) thio] phenyl, 2-chlorophenyl or 4-bromophenyl.

The term "halogen", as used herein, includes an atom of Cl, Br, F, I.

The term "nitro", as used herein, represents a group of the formula -$NO_2$.

The term "hydroxy", as used herein, represents a group of the formula -OH.

The term "alkoxy", as used herein, represents a group of formula -$OR^b$ wherein $R^b$ is an alkyl group, as defined above.

The term "ester", as used herein, represents a group of formula -$COOR^C$ wherein $R^c$ is an alkyl group or an aryl group, as defined above.

The term "alkoxycarbonyl", as used herein, represents a group of formula - $COOR^d$ wherein $R^d$ is an alkyl group, as defined above.

The term "amino", as used herein, represents a group of the formula -$NH_2$.

The term "alkylamino", as used herein, represents a group of formula - $NHR^e$ or -$NR^eR^f$ wherein $R^e$ and $R^f$ are alkyl group as defined above.

The term alkylsulfonyl, as used herein is defined as representing a group of formula $-SO_2-R^g$ wherein $R^g$ is C1-4-alkyl.

The term "heterocycle", as used herein is defined as including an aromatic or non aromatic cycloalkyl or cycloalkenyl moiety as defined above, having at least one O, S and/or N atom interrupting the carbocyclic ring structure and optionally, one of the carbon of the carbocyclic ring structure may be replaced by a carbonyl.

Non-limiting examples of aromatic heterocycles are pyrazolyl, furyl, imidazolyl, triazolyl, oxazolyl, pyridinyl, pyrrolyl, thienyl, isothiazolyl, benzimidazolyl, tetrazolyl, isooxazolyl, oxazolyl, thiazolyl, 1,2, 4-thiadiazolyl, oxadiazole, pyridazinyl, pyrimidinyl, pyrazinyl, isoindolyl, triazolopyridinyl, imidazolopyridinyl, pyrrolopyrimidinyl, pyrazolopyrimidinyl, quinazolinyl, quinolizinyl, naphthyridinyl, quinolyl, isoquinolyl, isobenzofuranyl, benzothienyl, indolyl, indolizinyl, purinyl, carbazolyl, thieno (2,3- b) furanyl, thianthrenyl, benzothiazolyl, benzoxazolyl, cinnolinyl, quinoxalinyl, phenothiazinyl, isochromanyl and xanthenyl, optionally substituted by 1 to 5 substituents independently selected from halogen, hydroxy, thiol, amino, nitro, cyano, azido, C1-6-alkoxy, C1-6-alkylthio, C1-6-alkyl, C1-6-haloalkyl, formyl or ester. More preferred aromatic heterocycles are pyrazolyl, furyl, imidazolyl, triazolyl, oxazolyl and pyridinyl.

Non-limiting examples of non aromatic heterocycles are tetrahydrofuranyl, piperidinyl, piperidyl, piperazinyl, imidazolidinyl, morpholinyl, thiomorpholinyl, pyrrolidinyl, thiazolidinyl, indolinyl, tetrahydrobenzazocinyl, dihydroisochromenyl, tetrahydropyranyl, oxooctahydroquinolinyl, dioxolanyl, 1-oxaspiro (4.5) dec-2-yl, pyrrolidinyl, 2-oxo-pyrrolidinyl, 8-thiabicyclo [3.2. 1] cyclooctanyl, 1,4-dithiepanyl, tetrahydro-2H-thiopyranyl, azepanyl and azocanyl, optionally substituted by 1 to 5 substituents independently selected from halogen, hydroxy, thiol, amino, nitro, cyano, azido, C1-6-alkoxy, C1-6-alkylthio, C1-6-alkyl, C1-6-haloalkyl, formyl or ester. More preferred non aromatic heterocycles are tetrahydrofuranyl, piperidinyl, piperidyl, piperazinyl, imidazolidinyl, morpholinyl, thiomorpholinyl, pyrrolidinyl, thiazolidinyl, indolinyl, tetrahydro-1-benzazocin-1 (2H)-yl, 3, 4-dihydro-1H-isochromen-1-yl, tetrahydropyranyl, oxooctahydroquinolinyl and dioxolanyl. The term"heterocycle"also includes bicyclic, tricyclic and tetracyclic, spiro groups in which any of the above heterocyclic rings is fused to one or two rings independently selected from an aryl ring, a cycloalkyl ring, a cycloalkenyl ring or another monocyclic heterocyclic ring or where a monocyclic heterocyclic group is bridged by an alkylene group, such as quinuclidinyl, 7-azabicyclo (2.2.1)heptanyl, 7-oxabicyclo (2.2.1)heptanyl and 8- azabicyclo (3.2.1)octanyl.

The term "pyridinylalkyl", as used herein, represents a group of formula - $R^h$- pyridinyl in which $R^h$ is C1-4-alkylene.

The term "azido" as used herein, represents a group of the formula $-N_3$.

The term "cyano" as used herein, represents a group of the formula -CN.

Generally, $R^2$ is hydrogen or C1-4-alkyl.

Preferably, $R^2$ is hydrogen, methyl or ethyl. More preferably, $R^2$ is hydrogen or methyl.

Generally, $R^3$ is hydrogen; C1-6-alkyl unsubstituted or substituted by 1 to 5 substituents selected from halogen, hydroxy, alkoxy, alkoxycarbonyl or alkylamino; C5-7-cycloalkyl; (hydroxymethyl) cyclohexenyl; phenyl unsubstituted or substituted by 1 to 5 substituents selected from halogen, C1-4-alkyl, hydroxy, methoxy, nitro, methylsulfonyl, trifluoromethylthio or pyridinylalkyl ; pyridinyl unsubstituted or substituted by methoxy; triazolyl; C1-4-alkoxy ; or a group of formula $-W-R^8$ wherein:

Generally, W is C1-4-alkylene unsubstituted or substituted by halogen, hydroxy, C1-4-alkyl or alkoxy ;-NH- ; or-NHC (=O)- ; and

$R^8$ is phenyl unsubstituted or substituted by 1 to 5 substituents selected from halogen, C1-4-alkyl, hydroxy, methoxy, nitro, methylsulfonyl or trifluoromethylthio; furyl unsubstituted or substituted by methyl; pyrazolyl; pyridinyl; morpholinyl ; tetrahydrobenzazocinyl; piperidinyl unsubstituted or substituted by methyl; dihydroisochromenyl or dihydroimidazolyl.

Preferably, $R^3$ is hydrogen, n-butyl, cycloheptyl, 2-fluoroethyl, 3-hydroxypropyl, 3-hydroxy-2, 2-dimethylpropyl, 1-(hydroxymethyl) propyl, 3,3, 3- trifluoro-2-hydroxypropyl, 3-ethoxypropyl, 2-ethoxy-2-oxoethyl, 3-(dimethylamino) propyl, 6-(hydroxymethyl) cyclohex-3-en-1-yl, 3-hydroxyphenyl, 3- fluorophenyl, 3- (2-pyridin-2-ylethyl) phenyl, 3, 4-dimethylphenyl, 4-tert-butylphenyl, benzyl, 4-hydroxy-3-methoxybenzyl, 4-methylsulfonylbenzyl, 2-nitrobenzyl, 2-chloro- 6-fluorobenzyl, 2-[(trifluoromethyl) thio] benzyl, 2-hydroxy-2-phenylethyl, 2- (3,4-dimethoxyphenyl) ethyl, 2- (2-chlorophenyl) ethyl, 2- (4-methylphenyl) ethyl, (4- bromophenyl) amino, pyridin-3-yl, 6-methoxypyridin-3-yl, 4H-1, 2, 4-triazol-3-yl, pyridin-4-ylmethyl, (5-methyl-2-furyl) methyl, 3-(1H-pyrazol-1-yl)propyl, 2-morpholin- 4-ylethyl, 2- ((3, 4,5, 6-tetrahydro-1-benzazocin-1 (2H)-yl) propyl, 2- (2-methylpiperidin-1-yl) ethyl, 3, 4-dihydro-1H-isochromen-1-ylmethyl, methoxy, (4-pyridinylcarbonyl) amino or 4, 5-dihydro-1H-imidazol-2-ylamino. More preferably, $R^3$ is hydrogen.

Generally, $R^{3a}$ is hydrogen, C1-4-alkyl or a group of formula

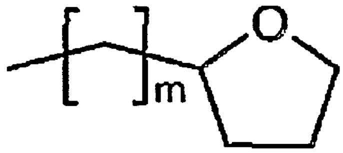

wherein m is 1 to 4.

Preferably, $R^{3a}$ is hydrogen, methyl or tetrahydrofuran-2-ylmethyl. More preferably, $R^{3a}$ is hydrogen.

In another instance, $NR^3R^{3a}$ is piperidinyl unsubstituted or substituted by hydroxy; thiomorpholinyl; thiazolidinyl unsubstituted or substituted by C1-4-alkoxycarbonyl ; 2, 5-dihydro-1H-pyrrol-1-yl ; 1, 4-dioxa-8-azaspiro [4.5] dec-8-yl; 4- oxooctahydro-1(2H)-quinolinyl; or a group of formula

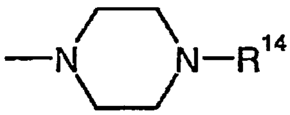

wherein $R^{14}$ is pyridinyl ; phenyl unsubstituted or substituted by halogen, hydroxy, C1-4-alkyl; or a group of formula -V-$R^{15}$ wherein V is unsubstituted C1-4- alkylene and $R^{15}$ is phenyl or morpholinyl.

In a preferred instance, $NR^3R^{3a}$ is 4-pyridin-2-ylpiperazin-1-yl, 4-(3-methylphenyl) piperazin-1-yl, 4- (4-hydroxyphenyl) piperazin-1-yl, 4- (2-phenylethyl) piperazin-1-yl, 4- (2-morpholin-4-ylethyl) piperazin-1-yl, 3-hydroxypiperidin-1-yl, thiomorpholin-4-yl, 4-methoxycarbonyl-1,3-thiazolidin-3-yl, 2, 5-dihydro-1H-pyrrol-1-yl, 1, 4-dioxa-8-azaspiro [4.5] dec-8-yl or 4-oxooctahydro-1 (2H)-quinolinyl.

Generally, $R^5$ is hydrogen, nitro, halogen, C1-4-alkyl, unsubstituted or substituted by halogen, or C1-4-alkoxy unsubstituted or substituted by halogen.

Preferably, $R^5$ is hydrogen, methyl, ethyl, trifluoromethyl, trifluoromethoxy, n- propyl, isopropyl, nitro, or halogen. More preferably, $R^5$ is halogen or trifluoromethyl.

Generally, $R^6$ is hydrogen, C1-6-alkyl or halogen.

Preferably, $R^6$ is hydrogen, methyl or Cl. More preferably, $R^6$ is hydrogen.

Generally, $R^7$ is hydrogen, methyl or halogen.

Preferably, $R^7$ is hydrogen, methyl, Br, F or Cl. More preferably, $R^7$ is hydrogen, Br or F.

Combinations of one or more of these preferred compound groups are especially preferred.

In a preferred instance, the compound has the formula I or a pharmaceutically acceptable salt thereof or stereoisomeric forms thereof,

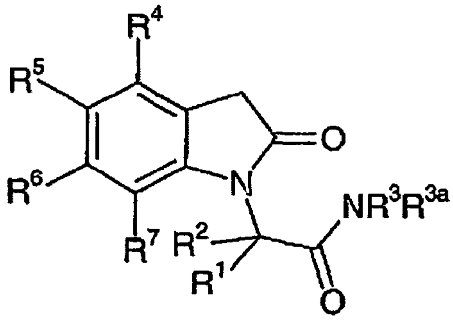

(I)

wherein $R^1$ is hydrogen,

$R^2$ is hydrogen or C1-4-alkyl,

$R^3$ is hydrogen; C1-6-alkyl unsubstituted or substituted by 1 to 5 substituents selected from halogen, hydroxy, alkoxy, alkoxycarbonyl or alkylamino ; C5-7-cycloalkyl ; (hydroxymethyl) cyclohexenyl; phenyl unsubstituted or substituted by 1 to 5 substituents selected from halogen, C1-4-alkyl, hydroxy, methoxy, nitro, methylsulfonyl, trifluoromethylthio or pyridinylalkyl ; pyridinyl unsubstituted or substituted by methoxy; triazolyl; C1-4-alkoxy ; or a group of formula-W-$R^8$,

$R^{3a}$ is hydrogen, C1-4-alkyl or a group of formula

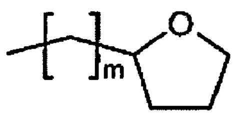

or $NR^3R^{3a}$ is piperidinyl unsubstituted or substituted by hydroxy; thiomorpholinyl ; thiazolidinyl unsubstituted or substituted by C1-4-alkoxycarbonyl; 2,5-dihydro-1H-pyrrol-1-yl; 1,4-dioxa-8-azaspiro [4.5] dec-8-yl; 4-oxooctahydro-1(2H)-quinolinyl ; or a group of formula

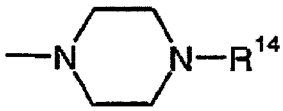

$R^4$ is hydrogen,

$R^5$ is hydrogen; nitro; halogen; C1-4-alkyl, unsubstituted or substituted by halogen; or C1-4-alkoxy unsubstituted or substituted by halogen,

R6 is hydrogen, C1-6-allyl or halogen,

R7 is hydrogen, methyl or halogen,

W is C1-4-alkylene unsubstituted or substituted by halogen, hydroxy, C1-4-alkyl or alkoxy ;-NH- ; or-NHC (=O)-,

R8 is phenyl unsubstituted or substituted by 1 to 5 substituents selected from halogen, C1-4-alkyl, hydroxy, methoxy, nitro, methylsulfonyl or trifluoromethylthio ; furyl unsubstituted or substituted by methyl; pyrazolyl; pyridinyl ; morpholinyl; tetrahydrobenzazocinyl ; piperidinyl unsubstituted or substituted by methyl; dihydroisochromenyl or dihydroimidazolyl,

$R^{14}$ is pyridinyl; phenyl unsubstituted or substituted by halogen, hydroxy, C1-4-alkyl ; or a group of formula-V-$R^{15}$,

V is unsubstituted C1-4-alkylene,

$R^{15}$ is phenyl or morpholinyl, m is 1 to 4,

and at least one of $R^5$, $R^6$ or $R^7$ is different from hydrogen when $R^2$ is hydrogen, $R^3$ is H or 2,6-diisopropylphenyl, and $R^{3a}$ is H.

In a more preferred instance, the compound has the formula I or a pharmaceutically acceptable salt thereof or stereoisomeric forms thereof,

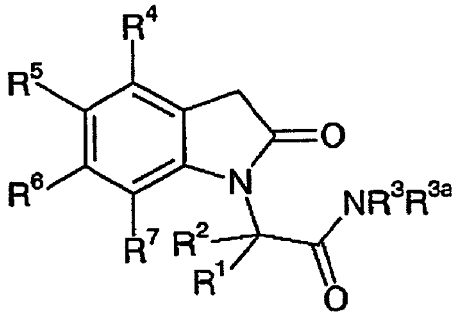

(I)

wherein

$R^1$ is hydrogen,

$R^2$ is hydrogen, methyl or ethyl,

$R^3$ is hydrogen, n-butyl, cycloheptyl, 2-fluoroethyl, 3-hydroxypropyl, 3-hydroxy-2,2- dimethylpropyl, 1-(hydroxymethyl) propyl, 3,3, 3-trifluoro-2-hydroxypropyl, 3- ethoxypropyl, 2-ethoxy-2-oxoethyl, 3- (dimethylamino) propyl, 6-(hydroxymethyl) cyclohex-3-en-1-yl, 3-hydroxyphenyl, 3-fluorophenyl, 3- (2-pyridin-2-ylethyl) phenyl, 3,4-dimethylphenyl, 4-tert-butylphenyl, benzyl, 4-hydroxy-3- methoxybenzyl, 4-methylsulfonylbenzyl, 2-nitrobenzyl, 2-chloro-6-fluorobenzyl, 2- [(trifluoromethyl)thio] benzyl, 2-hydroxy-2-phenylethyl, 2- (3, 4-dimethoxyphenyl) ethyl, 2- (2-chlorophenyl) ethyl, 2- (4-methylphenyl) ethyl, (4-bromophenyl) amino, pyridin-3-yl, 6-methoxypyridin-3-yl, 4H-1,2,4-triazol-3-yl, pyridin-4-ylmethyl, (5-methyl-2-furyl) methyl, 3- (1H-pyrazol-1-yl) propyl, 2-morpholin-4-ylethyl, 2- ((3, 4,5, 6-tetrahydro-1-benzazocin-1 (2H) -yl) propyl, 2- (2-methylpiperidin-1-yl) ethyl, 3, 4-dihydro-1H-isochromen-1-ylmethyl, methoxy, (4-pyridinylcarbonyl) amino or 4, 5-dihydro-1H- imidazol-2-ylamino,

$R^{3a}$ is hydrogen, methyl or tetrahydrofuran-2-ylmethyl, or $NR^3R^{3a}$ 4-pyridin-2-ylpiperazin-1-yl, 4-(3-methylphenyl) piperazin-1-yl, 4-(4-hydroxyphenyl) piperazin-1-yl, 4-(2-phenylethyl) piperazin-1-yl, 4-(2-morpholin-4- ylethyl) piperazin-1-yl, 3-hydroxypiperidin-1-yl, thiomorpholin-4-yl, 4- methoxycarbonyl-1, 3-thiazolidin-3-yl, 2, 5-dihydro-1H-pyrrol-1-yl, 1,4-dioxa-8- azaspiro [4.5]dec-8-yl or 4-oxooctahydro-1(2H)-quinolinyl,

$R^4$ is hydrogen,

R5 is hydrogen, methyl, ethyl, trifluoromethyl, trifluoromethoxy, n-propyl, isopropyl, nitro or halogen,

$R^6$ is hydrogen, methyl or Cl,

$R^7$ is hydrogen, methyl, Br, F or Cl,

and at least one of $R^5$, $R^6$ or $R^7$ is different from hydrogen when $R^2$ is hydrogen, $R^3$ is H or 2,6-diisopropylphenyl, and $R^{3a}$ is H.

More preferably, $R^2$ is hydrogen or methyl, $R^3$ is hydrogen, $R^{3a}$ is hydrogen, $R^5$ is halogen or trifluoromethyl, $R^6$ is hydrogen and $R^7$ is hydrogen, Br or F.

In all the above-mentioned scopes, when $R^2$ is C1-20-alkyl, the carbon atom to which $R^2$ is attached is preferably in the"S"-configuration.

In some instances, compounds useful in the methods and compositions of this disclosure are selected from the group consisting of: 2-(5-iodo-2-oxo-2,3-dihydro-1H-indol-1-yl) acetamide ; 2- (5-chloro-2-oxo-2, 3-dihydro-1H-indol-1-yl) acetamide ; 2- (5, 7-dibromo- 2-oxo-2, 3-dthydro-1H-indol-1-yl) acetamide ; 2-(5-nitro-2-oxo-2,3-dihydro-1H-indol-1-yl) acetamide ; 2-(5-methyl-2-oxo-2,3-dihydro-1H-indol-1-yl) acetamide; 2- (5-chloro-2- oxo-2, 3-dihydro-1H-indol-1-yl) propanamide ; (2R)-2- (5-chloro-2-oxo-2, 3-dihydro-1H- indol-1-yl) propanamide ; (2S)-2-(5-chloro-2-oxo-2,3-dihydro-1H-indol-1-yl) propanamide; 2-[2-oxo-5-(trifluoromethoxy)-2, 3-dihydro-1H-indol-1-yl] acetamide ; 2- (5-isopropyl-2-oxo-2, 3-dihydro-1H-indol-1-yl)acetamide ; 2-(5-ethyl-2-oxo-2, 3-dihydro- 1H-indol-1-yl) acetamide ; 2-(5-fluoro-2-oxo-2,3-dihydro-1H-indol-1-yl) acetamide; 2- (5,7-dimethyl-2-oxo-2, 3-dihydro-1H-indol-1-yl) acetamide; 2- (5-bromo-2-oxo-2,3-dihydro-1H-indol-1-yl) acetamide ; 2-(2-oxo-5-propyl-2, 3-dihydro-1H-indol-1-yl) acetamide ; 2-[2-oxo-5-(trifluoromethyl)-2, 3-dihydro-1H-indol-1-yl] acetamide ; 2- (5, 6-dimethyl-2-oxo-2, 3-dihydro-1H-indol-1-yl) acetamide; 2- (7-chloro-2-oxo-2, 3-dihydro-IH-indol-1-yl) acetamide; 2- (6-chloro-2-oxo-2, 3-dihydro-IH-indol-1-yl) acetamide; 2- (5- chloro-2-oxo-2, 3-dihydro-1H-indol-1-yl) butanamide ; (+)-2- (5-chloro-2-oxo-2, 3- dihydro-1H-indol-1-yl) butanamide; (-)-2- (5-chloro-2-oxo-2, 3-dihydro-IH-indol-1-yl) butanamide; 2-(5-methyl-2-oxo-2,3-dihydro-1H-indol-1-yl)propanamide ; (+)-2-(5- methyl-2-oxo-2, 3-dihydro-1H-indol-1-yl) propanamide; (-)-2- (5-methyl-2-oxo-2, 3- dihydro-1H-indol-1-yl) propanamide ; 2-(5-bromo-2-oxo-2,3-dihydro-1H-indol-1- yl) propanamide ; (-)-2- (5-bromo-2-oxo-2,3-dihydro-1H-indol-1-yl) propanamide ; (+)-2- (5-bromo-2-oxo-2,3-dihydro-1H-indol-1-yl) propanamide; 2- (5-chloro-7-fluoro-2-oxo- 2, 3-dihydro-1H-indol-1-yl) acetamide; 2-(5-chloro-2-oxo-2,3-dihydro-1H-indol-1-yl)-N-(3-hydroxyphenyl) acetamide ; 2- (5-chloro-2-oxo-2, 3-dihydro-1H-indol-1-yl)-N-(3- fluorophenyl) acetamide ; 2-(5-chloro-2-oxo-2, 3-dihydro-1H-indol-1-yl)-N-[3- (2-pyridin- 2-ylethyl) phenyllacetarnide ; 2-(5-chloro-2-oxo-2,3-dihydro-1H-indol-1-yl)-N-[6-(hydroxymethyl) cyclohex-3-en-1-yl]acetanuide ; 5-chloro-1-[2-oxo-2-(4-pyridin-2- ylpiperazin-1-yl) ethyl3-1, 3-dihydro-2H-indol-2-one ; 5-chloro-1-{2- [4- (3- methylphenyl) piperazin-1-yl]-2-oxoethyl}-1,3-dihydro-2H-indol-2-one ; 2- (5-chloro-2- oxo-2, 3-dihydro-1H-indol-1-yl)-N-(4-hydroxy-3-methoxybenzyl)acetamide ; 2- (5-chloro- 2-oxo-2, 3-dihydro-1H-indol-1-yl)-N-(pyridin-4-ylmethyl)-N- (tetrahydrofuran-2- ylmethyl) acetamide ; 5-chloro-1-[2-(3-hydroxypiperidin-1-yl)-2-oxoethyl]-1,3-dihydro- 2H-indol-2-one; 2-(5-chloro-2-oxo-2,3-dihydro-1H-indol-1-yl)-N’-isonicotinoylacetohydrazide ; 5-chloro-1-(2-oxo-2-thiomorpholin-4-ylethyl)-1,3-dihydro- 2H-indol-2-one; 2-(5-chloro-2-oxo-2,3-dihydro-1H-indol-1-yl)-N-(4H-1,2, 4-triazol-3- yl) acetamide; 2- (5-chloro-2-oxo-2, 3-dihydro-1H-indol-1-yl)-N-[4-(methylsulfonyl) benzyl] acetamide ; 1-[(5-chloro-2-oxo-2,3-dihydro-1H-indol-1- yl) acetyl] octahydroquinolin-4 (1H)-one ; N'- (4-bromophenyl)-2- (5-chloro-2-oxo-2, 3-dihydro-1H-indol-1-yl) acetohydrazide; 2-(5-chloro-2-oxo-2,3-dihydro-1H-indol-1-yl)-N-(6-methoxypyridin-3-yl) acetamide; N-butyl-2-(5-chloro-2-oxo-2,3-dihydro-1H-indol-1-yl) acetamide ; 2-(5-chloro-2-oxo-2,3-dihydro-1H-indol-1-yl)-N-(3-hydroxypropyl) acetamide ; 2-(5-chloro-2-oxo-2,3-dihydro-1H-indol-1-yl)-N-[3- (dimethylamino) propyl] acetamide ; 5-chloro-1-{2-oxo-2[4-(2-phenylethyl)pperazin-1-yl] ethyl}-1,3-dihydro-2H-indol-2-one; ethyl {[(5-chloro-2-oxo-2, 3-dihydro-1H-indol-1-yl) acetyl]amino}acetate ; 2-(5-chloro-2-oxo-2,3-dihydro-1H-indol-1-yl)-N-(3-ethoxypropyl) acetamide ; 2- (5-chloro-2-oxo-2, 3-dihydro-1H-indol-1-yl)-N-(2- fluoroethyl) acetamide ; 2- (5-chloro-2-oxo-2, 3-dihydro-1H-indol-1-yl)-N-methoxy-N- methylacetamide ; 2-(5-chloro-2-oxo-2,3-dihydro-1H-indol-1-yl)-N-(3, 4- dimethylphenyl) acetamide ; N- (4-tert-butylphenyl)-2- (5-chloro-2-oxo-2,3-dihydro-1H-indol-1-yl) acetamide; 2- (5-chloro-2-oxo-2, 3-dihydro-1H-indol-1-yl)-N- (3-hydroxy-2,2- dimethylpropyl) acetamide ; 2-(5-chloro-2-oxo-2,3-dihydro-1H-indol-1-yl)-N-[1-(hydroxymethyl) propyl] acetamide ; 2-(5-chloro-2-oxo-2, 3-dihydro-1H-indol-1-yl)-N-(3,3, 3-trifluoro-2-hydroxypropyl) acetamide; 2- (5-chloro-2-oxo-2, 3-dihydro-1H-indol-1-yl)-N-(2-hydroxy-2-phenylethyl) acetamide ; 5-chloro-1-{2-[4- (4- hydroxyphenyl) piperazin-1-yl]-2-oxoethyl}-1, 3-dihydro-2H-indol-2-one; 2- (5-chloro-2- oxo-2, 3-dihydro-1H-indol-1-yl)-N-(pyridin-4-ylmethyl)acetamide ; 2- (5-chloro-2-oxo- 2, 3-dihydro-1H-indol-1-yl)-N-[(5-methyl-2-furyl)methyl]acetamide ; 2-(5-chloro-2-oxo- 2, 3-dihydro-1H-indol-1-yl)-N- [3- (1H-pyrazol-1-yl) propyl] acetamide ; methyl 3- [(5- chloro-2-oxo-2, 3-dihydro-1H-indol-1-yl] acetyl]-1, 3-thiazolidine-4-carboxylate ; 5- chloro-1-[2-(2,5-dihydro-1H-pyrrol-1-yl)-2-oxoethyl]-1,3-dihydro-2H-indol-2-one; 2- (5- chloro-2-oxo-2, 3-dihydro-1H-indol-1-yl)-N'-(4, 5-dihydro-1H-imidazol-2- yl) acetohydrazide ; 2- (5-chloro-2-oxo-2,3-dihydro-1H-indol-1-yl)-N-[2- (3, 4- dimethoxyphenyl) ethyl] acetamide ; 2-(5-chloro-2-oxo-2,3-dihydro-1H-indol-1-yl)-N-[2- (2-chlorophenyl) etl-lyllacetaniide ; 2-(5-chloro-2-oxo-2,3-dihydro-1H-indol-1-yl)-N-[2-(4- methylphenyl) ethyl] acetamide ; 2-(5-chloro-2-oxo-2,3-dihydro-1H-indol-1-yl)-N-(2-morpholin-4-ylethyl) acetamide ; 2- (5-chloro-2-oxo-2, 3-dihydro-1H-indol-1-yl)-N-[2- (3,4, 5, 6-tetrahydro-1-benzazocin-1 (2H) -yl) propyl] acetamide ; 2- (5-chloro-2-oxo-2, 3- dihydro-1H-indol-1-yl)-N-[2-(2-methylpiperidin-1-yl) ethyl] acetamide ; 2- (5-chloro-2- oxo-2, 3-dihydro-1H-indol-1-yl)-N-(2-nitrobenzyl) acetamide ; 2- (5-chloro-2-oxo-2, 3- dihydro-1H-indol-1-yl)-N- (3, 4-dihydro-1H-isochromen-1-ylinethyl) acetamide ; N- (2-chloro-6-fluorobenzyl)-2- (5-chloro-2-oxo-2, 3-dihydro-1H-indol-1-yl) acetamide ; N- benzyl-2- (5-chloro-2-oxo-2, 3-dihydro-1H-indol-1-yl)-N-methylacetamide ; 2-(5-chloro- 2-oxo-2, 3-dthydro-1H-indol-1-yl)-N-{2-[(trifluoromethyl) thio] benzyl} acetamide ; 5- chloro-1- [2- (1, 4-dioxa-8-azaspiro [4.5] dec-8-yl)-2-oxoethyl]-1,3-dihydro-2H-indol-2- one; 2-(5-chloro-2-oxo-2, 3-dihydro-1H-indol-1-yl)-N-cycloheptylacetamide ; 5-chloro-1- {2-[4- (2-morpholin-4-ylethyl) piperazin-1-yl]-2-oxoethyl}-1,3-dihydro-2H-indol-2-one ; and 2-(5-chloro-2-oxo-2,3-dihydro-1H-indol-1-yl)-N-pyridin-3-ylacetamide.

In some instances, compounds useful in the methods and compositions of this disclosure are selected from the group consisting of: 2-(5-iodo-2-oxo-2,3-dihydro-1H-indol-1- yl) acetamide ; 2- (5-chloro-2-oxo-2, 3-dihydro-1H-indol-1-yl) acetamide ; 2- (5, 7-dibromo- 2-oxo-2, 3-dihydro-1H-indol-1-yl)acetamide; (2S)-2-(5-chloro-2-oxo-2,3-dihydro-1H-indol-1-yl) propanamide ; 2-[2-oxo-5-(trifluoromethyl)-2, 3-dihydro-1H-indol-1- yl] acetamide and 2-(5-chloro-7-fluoro-2-oxo-2,3-dihydro-1H-indol-1-yl) acetamide.

In another instance, compounds useful in the methods and compositions of this disclosure are selected from the group consisting of: 2- (5-chloro-2-oxo-2, 3-dihydro-1H-indol-1-yl) acetamide and (2S) -2- (5-chloro-2- oxo-2, 3-dihydro-1H-indol-1-yl) propanamide.

iv) US Patent No. 7,244,747:

A compound having the formula I or a pharmaceutically acceptable salt thereof,

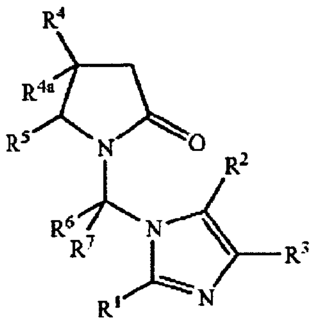

(I)

wherein $R^1$ is hydrogen, $C_{1-20}$ alkyl, $C_{3-8}$ cycloalkyl, halogen, hydroxy, alkoxy, aryloxy, ester, amido, cyano, nitro, amino, guanidine, amino derivative, alkylthio, arylthio, alkylsulfonyl, arylsulfonyl, alkylsulfinyl, arylsulfinyl, aryl or heterocycle;

$R^2$ is hydrogen, $C_{1-20}$ alkyl, alkoxy, amino, halogen, hydroxy, ester, amido, nitro, cyano, carbamate, or aryl;

$R^3$ is hydrogen, $C_{1-20}$ alkyl, alkoxy, amino, halogen, hydroxy, ester, amido, nitro, cyano, carbamate, or aryl;

or $R^2$ and $R^3$ can form together with the imidazole ring the following 1 H-benzimidazole cycle

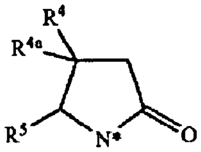

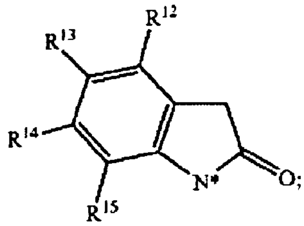

$R^4$ is hydrogen, $C_{1-20}$ alkyl, $C_{2-12}$ alkenyl, $C_{2-12}$ alkynyl, aryl, azido, alkoxycarbonylamino, arylsulfonyloxy or heterocycle;

$R^{4a}$ is hydrogen or $C_{1-20}$ alkyl;

or $R^4$ and $R^{4a}$ can form together a $C_{3-8}$ cycloalkyl;

$R^5$ is hydrogen;

or $R^4$, $R^{4a}$ and $R^5$ can form together with the 2-oxo-1-pyrrolidine ring the following 1,3-dihydro-2H-indol-2-one cycle

$R^6$ is hydrogen or $C_{1-20}$ alkyl;

$R^7$ is hydrogen;

or $R^6$ and $R^7$ are linked together to form a $C_{3-6}$ cycloalkyl;

$R^8$ is hydrogen, halogen, nitro, cyano, $C_{1-20}$ alkyl or alkoxy;

$R^9$ is hydrogen, $C_{1-20}$ alkyl, halogen, hydroxy, alkoxy, aryloxy, ester, amido, cyano, nitro, amino, amino derivative, alkylthio, arylthio, alkylsulfonyl, arylsulfonyl, alkylsulfinyl or arylsulfinyl;

$R^{10}$ is hydrogen, $C_{1-20}$ alkyl, halogen, hydroxy, alkoxy, aryloxy, ester, amido, cyano, nitro, amino, amino derivative, alkylthio, arylthio, alkylsulfonyl, arylsulfonyl, alkylsulfinyl or arylsulfinyl;

$R^{11}$ is hydrogen, halogen, nitro, cyano, $C_{1-20}$ alkyl or alkoxy;

$R^{12}$ is hydrogen or halogen;

$R^{13}$ is hydrogen, nitro, halogen, heterocycle, amino, aryl, $C_{1-20}$ alkyl unsubstituted or substituted by halogen, or alkoxy unsubstituted or substituted by halogen;

$R^{14}$ is hydrogen, $C_{1-20}$ alkyl or halogen;

$R^{15}$ is hydrogen, $C_{1-20}$ alkyl or halogen;

with the proviso that $R^4$ is different from hydrogen when represents a group of formula

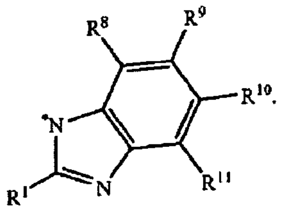

The asterisk * indicates the point of attachment of the substituents.

In a preferred instance, the compounds have the formula I, their tautomers, geometrical isomers (including cis and trans, Z and E isomers), enantiomers, diastereoisomers and mixtures thereof (including all possible mixtures of stereoisomers), or pharmaceutically acceptable salts thereof,

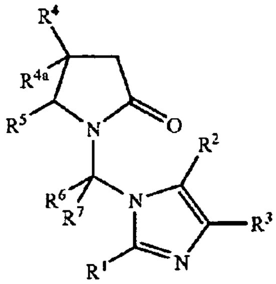

(I)

wherein $R^1$ is hydrogen, $C_{1-20}$ alkyl, $C_{3-8}$ cycloalkyl, halogen, hydroxy, ester, amido, cyano, nitro, amino, guanidine, alkylthio, alkylsulfonyl, alkylsulfinyl, aryl or heterocycle;

$R^2$ is hydrogen, $C_{1-20}$ alkyl, halogen, cyano, ester, carbamate or amido;

$R^3$ is hydrogen, cyano, $C_{1-20}$ alkyl, halogen or ester;

or $R^2$ and $R^3$ can form together with the imidazole ring the following 1H-benzimidazole cycle

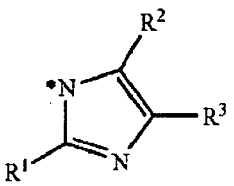
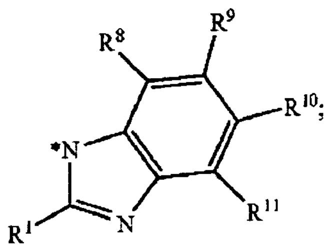

$R^4$ is hydrogen, $C_{1-20}$ alkyl, $C_{2-12}$ alkenyl or aryl;

$R^{4a}$ is hydrogen;

$R^5$ is hydrogen;

or $R^4$, $R^{4a}$ and $R^5$ can form together with the 2-oxo-1-pyrrolidine ring the following 1,3-dihydro-2H-indol-2-one cycle

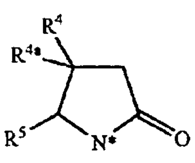
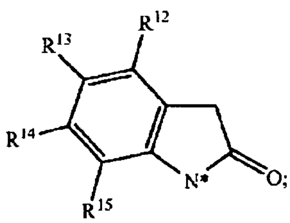

$R^6$ is hydrogen or $C_{1-20}$ alkyl;

$R^7$ is hydrogen; or $R^6$ and $R^7$ are linked together to form a $C_{3-6}$ cycloalkyl;

$R^8$ is hydrogen;

$R^9$ is hydrogen, $C_{1-20}$ alkyl, halogen or alkoxy;

$R^{10}$ is hydrogen, $C_{1-20}$ alkyl, halogen or cyano;

$R^{11}$ is hydrogen;

$R^{12}$ is hydrogen or halogen;

$R^{13}$ is hydrogen, halogen, heterocycle or $C_{1-20}$ alkyl;

$R^{14}$ is hydrogen;

$R^{15}$ is hydrogen;

with the proviso that $R^4$ is different from hydrogen when

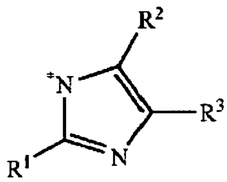

represents a group of formula

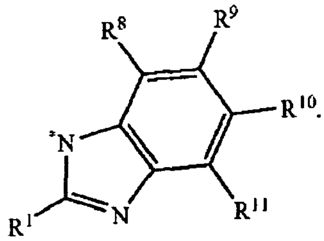

The term "alkyl", as used herein, represents saturated, monovalent hydrocarbon radicals having straight (unbranched) or branched or cyclic or combinations thereof and containing 1-20 carbon atoms, preferably 1-10 carbon atoms, more pre preferred alkyl groups have 1-3 carbon atoms. Alkyl moieties may optionally be substituted by 1 to 5 substituents independently selected from the group consisting of halogen, hydroxy, cyano, azido, aryloxy, alkoxy, alkythio, alkanoylamino, arylcarbonylamino, aminocarbonyl, methylaminocarbonyl, dimethylaminocarbonyl or aryl. Usually alkyl groups, in the present case, are methyl, ethyl, n-propyl, i-propyl, n-butyl, i-butyl, t-butyl, 1-ethylpropyl, n-heptyl, 2,4,4-trimethylpentyl, n-decyl, chloromethyl, trifluoromethyl, 2-bromo-2,2-difluoroethyl, 2,2,2-trifluoroethyl, 3,3,3-trifluoropropyl, hydroxymethyl, cyanomethyl, azidomethyl, (acetylamino)methyl, (propionylamino)methyl, (benzoylamino)methyl, (4-chlorophenoxy)methyl, benzyl, 2-phenylethyl or 2-(methylthio)ethyl. Preferred alkyl groups are methyl, ethyl, n-propyl, i-propyl, n-butyl, i-butyl, t-butyl, 1-ethylpropyl, 2,4,4-trimethylpentyl, chloromethyl, trifluoromethyl, 2,2,2-trifluoroethyl, hydroxymethyl, cyanomethyl, azidomethyl, (acetylamino)methyl, (propionylamino)methyl, (benzoylamino)methyl or 2-(methylthio)ethyl. More preferred alkyl groups are methyl, ethyl, n-propyl, i-propyl, n-butyl, azidomethyl or trifluoromethyl. Most preferred alkyl groups are methyl or n-propyl.

The term "cycloalkyl", as used herein, represents a monovalent group of 3 to 8 carbon atoms, usually 3-6 carbon atoms derived from a saturated cyclic hydrocarbon, which may be substituted by any suitable group including but not limited to one or more moieties selected from groups as described above for the alkyl groups. Preferred cycloalkyl groups are cyclopropyl and cyclohexyl.

The term "alkenyl" as used herein, represents straight, branched or cyclic unsaturated hydrocarbon radicals

or combinations thereof having at least one carbon-carbon double bond, containing 2-12 carbon atoms, preferably usually 2-4 carbon atoms. Alkenyl groups are being optionally substituted with any suitable group, including but not limited to one or more moieties selected from groups as described above for the alkyl groups. Usually an alkenyl group is ethenyl (vinyl) optionally substituted by 1 to 3 halogens. Preferred alkenyl group, in the present case, is 2, 2-difluorovinyl.

The term a"alkynyl" as used herein, represents straight, branched or cyclic hydrocarbon radicals or combinations thereof containing at least one carbon-carbon triple bond, containing 2-12 carbon atoms, preferably 2-6 carbon atoms, and being optionally substituted by any suitable group, including but not limited to one or more moieties selected from groups as described above for the alkyl groups. Preferably an alkynyl group is a halogenoalkynyl group (haloalkynyl group).

Groups qualified by prefixes such as "s", "i", "t" and the like (e.g. "i-propyl", "s-butyl") are branched derivatives.

The term "aryl" as used herein, is defined as phenyl optionally substituted by 1 to 4 substituents independently selected from halogen, cyano, alkoxy, alkylthio, $C_{1-3}$ alkyl or azido, preferably halogen or azido. Usually aryl groups, in the present case are phenyl, 3-chlorophenyl, 3-fluorophenyl, 4-chlorophenyl, 4-fluorophenyl, 3,4-difluorophenyl, 3,5-difluorophenyl, 3-chloro-4-fluorophenyl, 2,3,4-trifluorophenyl, 2,4,5-trifluorophenyl, 2,3,5-trifluorophenyl, 3,4,5-trifluorophenyl, 3-azido-2,4-difluorophenyl or 3-azido-2,4,6-trifluorophenyl. Preferably, aryl groups are phenyl, 3-chlorophenyl, 3-fluorophenyl, 4-chlorophenyl, 4-fluorophenyl, 3,4-difluorophenyl, 3,5-difluorophenyl, 3-chloro-4-fluorophenyl, 2,3,4-trifluorophenyl, 2,4,5-trifluorophenyl, 2,3,5-trifluorophenyl, 3,4,5-trifluorophenyl or 3-azido-2,4-difluorophenyl. Most preferred aryl groups are phenyl, 3-chlorophenyl, 3-fluorophenyl, 3,5-difluorophenyl, 2,3,4-trifluorophenyl, 2,4,5-trifluorophenyl, 2,3,5-trifluorophenyl, 3,4,5-trifluorophenyl or 3-azido-2,4-difluorophenyl.

The term "heterocycle", as used herein, is defined as including an aromatic or non aromatic cycloalkyl moiety as defined above, having at least one O, S and/or N atom interrupting the carbocyclic ring structure. Heterocyclic ring moieties can be optionally substituted by alkyl groups or halogens and optionally, one of the carbon of the carbocyclic ring structure may be replaced by a carbonyl. Usually heterocycles are 2-pyridyl, 3-pyridyl, 4-pyridyl, 2-furyl, 3-furyl, 2-thienyl, 3-thienyl, 2-tetrahydrofuranyl, 1H-pyrrol-2-yl, 1-methyl-1H-pyrrol-2-yl, 1H-pyrazol-2-yl, 1H-pyrazol-3-yl, 4-chloro-1-methyl-1H-pyrazol-3-yl, 5-chloro-1,3-dimethyl-1H-pyrazol-4-yl, 1,2,3-thiadiazol-4-yl, 3,5-dimethyl-4-isothiazyl, 1H-imidazol-2-yl, 1-methyl-1H-imidazol-2-yl, 4-methyl-1H-imidazol-5-yl, or 2-methyl-1,3-thiazol-4-yl. Preferred heterocycles are 1H-imidazol-2-yl, 1,2,3-thiadiazol-4-yl, 1H-pyrazol-3-yl, 2-furyl, 3-furyl, 2-thienyl, 1-methyl-1H-pyrrol-2-yl, 1H-pyrrol-2-yl.

The term "halogen", as used herein, includes an atom of chlorine, bromine, fluorine, iodine. Usually halogens are chlorine, bromine and fluorine. Preferred halogens are fluorine, bromine and chlorine.

The term "hydroxy", as used herein, represents a group of formula --OH.

The term "alkoxy", as used herein, represents a group of formula $-OR^a$ wherein $R^a$ is an alkyl group, as defined above. Preferred alkoxy group is methoxy.

The term "aryloxy", as used herein, represents a group of formula $--OR^b$ wherein $R^b$ is an aryl group, as defined above. Preferred aryloxy group is phenoxy.

The term "ester", as used herein, represents a group of formula $--COOR^c$ wherein $R^c$ is an alkyl group or aryl group, as defined above. Preferred ester group is methoxycarbonyl.

The term "amido", as used herein, represents a group of formula $--CONH_2$.

The term "amino", as used herein, represents a group of formula $--NH_2$.

The term "aminoderivative", as used herein, represents an alkylamino or an arylamino group, wherein the terms "alkyl" and "aryl" are defined as above.

The term "cyano", as used herein, represents a group of formula --CN.

The term "nitro", as used herein, represents a group of formula $-NO_2$.

The term "azido", as used herein, represents a group of formula $--N_3$.

The term "guanidine", as used herein, represents a group of formula-$NHC(=NH)NH_2$.

The term "alkylthio", as used herein, represents a group of formula $--SR^d$ wherein $R^d$ is an alkyl group, as defined above. Preferred alkylthio group is methylthio.

The term "alkylsulfonyl", as used herein, represents a group of formula-$S(=O)_2R^e$ wherein $R^e$ is an alkyl group, as defined above. Preferred alkylsulfonyl group is methylsulfonyl.

The term "alkylsulfinyl", as used herein, represents a group of formula-$S(=O)R^f$ wherein $R^f$ is an alkyl group, as defined above. Preferred alkylsulfinyl group is methylsulfinyl.

The term "arylthio", as used herein, represents a group of formula $--SR^g$ wherein $R^g$ is an aryl group, as defined above.

The term "arylsulfonyl", as used herein, represents a group of the formula--$S(=O)_2R^h$ wherein $R^h$ is an aryl group, as defined above.

The term "arylsulfinyl", as used herein, represents a group of the formula-$S(=O)R^1$ wherein $R^1$ is an aryl group, as defined above.

The term "carbamate" as used herein, represents a group of formula-$N(H)C(O)OR^j$, wherein $R^j$ is an alkyl or

an aryl, as defined above. Usually carbamate groups are (propoxycarbonyl)amino or (benzyloaxycarbonyl)amino. Preferred carbamate group is (benzyloaxycarbonyl)amino.

The term "alkanoylamino" as used herein, represents a group of the formula --NHC(=O)$R^k$ wherein $R^k$ is an alkyl group, as defined above.

The term "(arylcarbonyl)amino" as used herein, represents a group of the formula --NHC(=O)$R^m$ wherein $R^m$ is an aryl group, as defined above.Preferred (arylcarbonyl)amino is benzoylamino.

Usually, $R^1$ is hydrogen; $C_{1-10}$ alkyl unsubstituted or substituted by halogen, hydroxy, cyano, methylthio, phenyl or 4-chlorophenoxy; hydroxy; $C_{3-6}$ cycloalkyl; halogen; ester; amido; nitro; cyano; amino; phenyl; alkylthio; alkylsulfonyl; alkylsulfinyl; heterocycle unsubstituted or substituted by alkyl groups; or guanidine. Preferably, $R^1$ is hydrogen; methyl; ethyl; i-propyl; n-propyl; cyclopropyl; n-butyl; i-butyl; t-butyl; 1-ethylpropyl; 2,4,4-trimethylpentyl; hydroxymethyl; chloromethyl; trifluoromethyl; 2,2,2-trifluoroethyl; cyanomethyl; 2-(methylthio)ethyl; chloro; bromo; nitro; cyano; amino; aminocarbonyl; methoxycarbonyl; methylthio; methylsulfinyl; methylsulfonyl; phenyl; 2-furyl; 3-furyl; 1H-pyrrol-2-yl; 1-methyl-1H-pyrrol-2-yl; 2-thienyl; 1H-pyrazol-3-yl; 1,2,3-thiadiazol-4-yl or 1H-imidazol-2-yl. More preferably, $R^1$ is hydrogen; methyl; ethyl; i-propyl; n-propyl; n-butyl; methylthio; nitro; cyano; amino; chloro or 1H-pyrrol-2-yl. Most preferably, $R^1$ is hydrogen; methyl; methylthio; nitro; cyano; amino or chloro.

Usually, $R^2$ is hydrogen; $C_{1-4}$ alkyl unsubstituted or substituted by hydroxy, alkanoylamino or benzoylamino; halogen; ester; cyano; alkyl carbamate; [(N-methoxy-N-methyl)amino]carbonyl. Preferably, $R^2$ is hydrogen; methyl; hydroxymethyl; (acetylamino)methyl; (propionylamino)methyl; (benzoylamino)methyl; [(benzyloxy)carbonyl]amino; chloro or cyano. More preferably, $R^2$ is hydrogen; chloro or cyano.

Usually, $R^3$ is hydrogen; $C_1$-$_4$ alkyl unsubstituted or substituted by hydroxy; halogen; ester or cyano. Preferably, $R^3$ is hydrogen; hydroxymethyl; chloro; cyano. More preferably, $R^3$ is hydrogen or cyano. Most preferred $R^3$ is hydrogen.

Usually, $R^4$ is hydrogen; $C_{1-4}$ alkyl unsubstituted or substituted by halogens; $C_{2-4}$ alkenyl substituted by halogens or phenyl group unsubstituted or substituted by azido or/and halogens. Preferably, $R^4$ is hydrogen; n-propyl; 2,2-difluorovinyl; phenyl; 3-chlorophenyl; 3-fluorophenyl; 4-chlorophenyl; 4-fluorophenyl; 3,5-difluorophenyl; 3,4-difluorophenyl; 3-chloro-4-fluorophenyl; 2,3,4-trifluorophenyl; 2,4,5-trifluorophenyl; 2,3,5-trifluorophenyl; 3,4,5-trifluorophenyl; 3-azido-2,4-difluorophenyl or 3-azido-2,4,6-trifluorophenyl. More preferably, $R^4$ is hydrogen; n-propyl; 2,2-difluorovinyl; phenyl; 3-chlorophenyl; 3-fluorophenyl; 4-chlorophenyl; 4-fluorophenyl; 3,5-difluorophenyl; 3,4-difluorophenyl; 3-chloro-4-fluorophenyl; 2,3,4-trifluorophenyl; 2,4,5-trifluorophenyl; 2,3,5-trifluorophenyl; 3,4,5-trifluorophenyl or 3-azido-2,4-difluorophenyl. Most preferably, $R^4$ is n-propyl; 2,2-difluorovinyl; phenyl; 3-chlorophenyl; 3-fluorophenyl; 3,5-difluorophenyl; 2,3,4-trifluorophenyl; 2,4,5-trifluorophenyl; 2,3,5-trifluorophenyl; 3,4,5-trifluorophenyl or 3-azido-2,4-difluorophenyl.

Usually, $R^{4a}$ is hydrogen.

Usually, $R^5$ is hydrogen.

Usually, $R^6$ is hydrogen or $C_{1-10}$ alkyl unsubstituted or substituted by hydroxy or azido. Preferably, $R^6$ is hydrogen or azidomethyl. More preferably $R^6$ is hydrogen.

Usually $R^7$ is hydrogen.

In other preferred instances, $R^6$ and $R^7$ are linked to form a cyclopropyl.

In other preferred instances, $R^2$ and $R^3$ can form together with the imidazole ring the following 1H-benzimidazole cycle

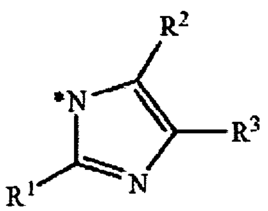

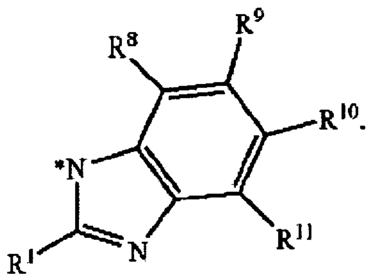

Usually, $R^8$ is hydrogen.

Usually, $R^9$ is hydrogen; halogen; $C_{1-3}$ alkyl or alkoxy. Preferably, $R^9$ is hydrogen; methyl; chloro or methoxy. More preferred $R^9$ is hydrogen.

Usually, $R^{10}$ is hydrogen; halogen; cyano; $C_{1-3}$ alkyl unsubstituted or substituted by halogens; or alkoxy. Preferably, $R^{10}$ is methyl; hydrogen; trifluoromethyl; fluoro; cyano or methoxy. More preferred $R^{10}$ is hydrogen; trifluoromethyl; fluoro or cyano.

Usually, $R^{11}$ is hydrogen.

In other preferred instances, $R^4$, $R^{4a}$ and $R^5$ can form together with the 2-oxo-1-pyrrolidine ring the following 1,3-dihydro-2H-indol-2-one cycle

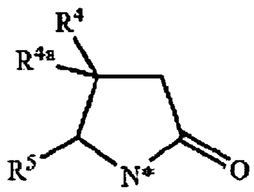
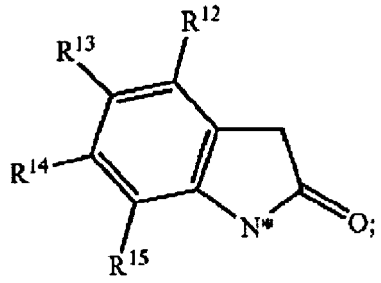

Usually, $R^{12}$ is hydrogen or halogen. Preferably $R^{12}$ is hydrogen; chloro or fluoro. More preferred $R^{12}$ is hydrogen.

Usually, $R^{13}$ is hydrogen; $C_{1-3}$ alkyl; halogen or thiazolyl unsubstituted or substituted by alkyl groups, such as methylthiazolyl. Preferably $R^{13}$ is hydrogen; chloro; bromo or methyl. Most preferred $R^{13}$ is chloro; bromo or methyl.

Usually $R^{14}$ is hydrogen.

Usually, $R^{15}$ is hydrogen.

Combinations of one or more of these preferred compound groups are especially preferred.

Generally, among the instances, the compounds of formula I, or pharmaceutically acceptable salts thereof, are those wherein

$R^1$ is selected from hydrogen; $C_{1-10}$ alkyl unsubstituted or substituted by halogen, hydroxy, cyano, methylthio, phenyl or 4-chlorophenoxy; $C_{3-6}$ cycloalkyl; halogen; ester; amido; nitro; cyano; amino; phenyl; alkylthio; alkylsulfonyl; alkylsulfinyl; heterocycle unsubstituted or substituted by alkyl group; or guanidine;

$R^2$ is selected from hydrogen; $C_{1-4}$ alkyl unsubstituted or substituted by hydroxy, alkanoylamino or benzoylamino; halogen; ester; cyano; alkyl carbamate or [(N-methoxy-N-methyl)amino]carbonyl.

$R^3$ is selected from hydrogen; $C_{1-4}$ alkyl unsubstituted or substituted by hydroxy; halogen; ester or cyano;

$R^4$ is selected from hydrogen; $C_{1-4}$ alkyl unsubstituted or substituted by halogens; $C_{2-4}$ alkenyl substituted by halogens or phenyl group unsubstituted or substituted by azido or /and halogens;

$R^{4a}$ is hydrogen;

$R^5$ is hydrogen;

$R^6$ is selected from hydrogen or $C_{1-10}$ alkyl unsubstituted or substituted by hydroxy or azido;

$R^7$ is hydrogen;

or $R^6$ and $R^7$ can be linked to form a cyclopropyl;

or $R^2$ and $R^3$ can form together with the imidazole ring the following 1H-benzimidazole cycle

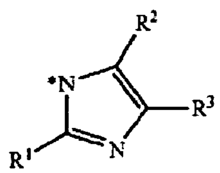
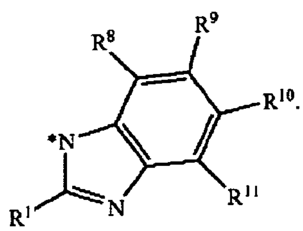

$R^8$ is hydrogen;

$R^9$ is selected from hydrogen; halogen; $C_{1-3}$ alkyl; alkoxy;

$R^{10}$ is selected from hydrogen; halogen; cyano or $C_{1-3}$ alkyl unsubstituted or substituted by halogens; or alkoxy;

$R^{11}$ is hydrogen;

or $R^4$, $R^{4a}$ and $R^5$ can form together with the 2-oxo-1-pyrrolidine ring the following 1,3-dihydro-2H-indol-2-one cycle

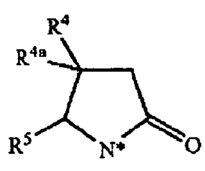
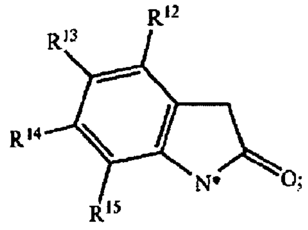

$R^{12}$ is selected from hydrogen or halogen;

$R^{13}$ is selected from hydrogen; $C_{1-3}$ alkyl; halogen; thiazolyl unsubstituted or substituted by alkyl groups, such as methylthiazolyl;

$R^{14}$ is hydrogen;

$R^{15}$ is hydrogen;

with the proviso that $R^4$ is different from hydrogen when

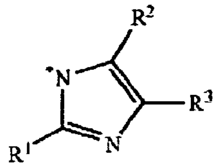

represents a group of formula

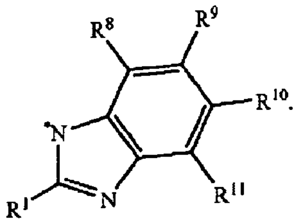

In a preferred instance, the compounds of formula I, or pharmaceutically acceptable salt thereof, are those wherein
$R^1$ is selected from hydrogen; methyl; ethyl; i-propyl; n-propyl; cyclopropyl; n-butyl; i-butyl; t-butyl; 1-ethylpropyl; 2,4,4-trimethylpentyl; trifluoromethyl; 2,2,2-trifluoroethyl; hydroxymethyl; chloromethyl; cyanomethyl; 2-(methylthio)ethyl; chloro; bromo; nitro; cyano; amino; aminocarbonyl; methoxycarbonyl; methylthio; methylsulfinyl; methylsulfonyl; phenyl; 2-furyl; 3-furyl; 1H-pyrrol-2-yl; 1-methyl-1H-pyrrol-2-yl; 2-thienyl; 1H-pyrazol-3-yl; 1,2,3-thiadiazol-4-yl; or 1H-imidazol-2-yl;
$R^2$ is selected from hydrogen; methyl; hydroxymethyl; (acetylamino)methyl; (propionylamino)methyl; (benzoylamino)methyl; (benzyloxycarbonyl)amino; chloro; or cyano;
$R^3$ is selected from hydrogen; hydroxymethyl; chloro; cyano;
or $R^2$ and $R^3$ can form together with the imidazole ring the following 1H-benzimidazole cycle

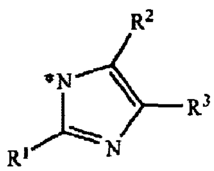 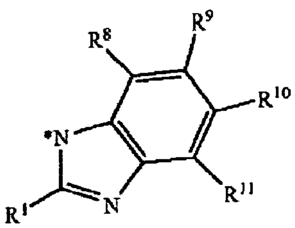

$R^8$ is hydrogen;

$R^9$ is selected from hydrogen; methyl; choro; methoxy;

$R^{10}$ is selected from methyl; hydrogen; trifluoromethyl; fluoro; cyano; or methoxy;

$R^{11}$ is hydrogen;

$R^4$ is selected from hydrogen; n-propyl; 2,2-difluorovinyl; phenyl; 3-chlorophenyl; 3-fluorophenyl; 4-chlorophenyl; 4-fluorophenyl; 3,5-difluorophenyl; 3,4-difluorophenyl; 3-chloro-4-fluorophenyl; 2,3,4-trifluorophenyl; 2,4,5-trifluorophenyl; 2,3,5-trifluorophenyl; 3,4,5-trifluorophenyl; 3-azido-2,4-difluorophenyl; or 3-azido-2,4,6-trifluorophenyl.

$R^{4a}$ is hydrogen;$R^5$ is hydrogen;

or $R^4$, $R^{4a}$ and $R^5$ can form together with the 2-oxo-1-pyrrolidine ring the following 1,3-dihydro-2H-indol-2-one cycle

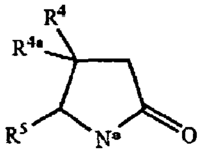

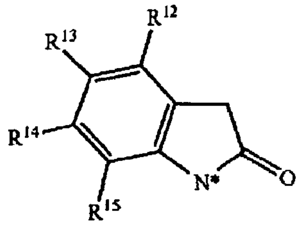

$R^{12}$ is selected from hydrogen; chloro; fluoro;
$R^{13}$ is selected from hydrogen; chloro; bromo; methyl;
$R^{14}$ is hydrogen;
$R^{15}$ hydrogen;
$R^6$ is selected from hydrogen; azidomethyl;
$R^7$ is hydrogen;
or $R^6$ and $R^7$ are linked to form a cyclopropyl;

with the proviso that $R^4$ is different from hydrogen when

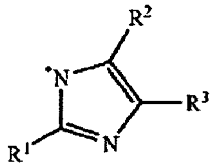

represents a group of formula

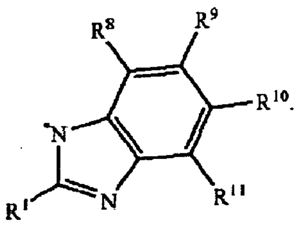

In a more preferred instance, the compounds of formula I, or pharmaceutically acceptable salt thereof, are those wherein
$R^1$ is selected from hydrogen; methyl; ethyl; i-propyl; n-propyl; n-butyl;methylthio; nitro; cyano; amino; chloro; or 1H-pyrrol-2-yl;
$R^2$ is selected from hydrogen; chloro; cyano;
$R^3$ is selected from hydrogen; cyano;
or $R^2$ and $R^3$ can form together with the imidazole ring the following 1H-benzimidazole cycle

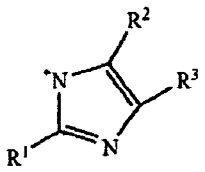

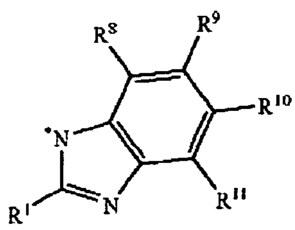

$R^8$ is hydrogen;

$R^9$ is hydrogen;

$R^{10}$ is selected from hydrogen; trifluoromethyl; fluoro; cyano;

$R^{11}$ is hydrogen;

$R^4$ is selected from hydrogen; n-propyl; 2,2-difluorovinyl; phenyl; 3-chlorophenyl; 3-fluorophenyl; 4-chlorophenyl; 4-fluorophenyl; 3,5-difluorophenyl; 3,4-difluorophenyl; 3-chloro-4-fluorophenyl; 2,3,4-trifluorophenyl; 2,4,5-trifluorophenyl; 2,3,5-trifluorophenyl; 3,4,5-trifluorophenyl; or 3-azido-2,4-difluorophenyl;

$R^{4a}$ is hydrogen;

$R^5$ is hydrogen;

or $R^4$, $R^{4a}$ and $R^5$ can form together with the 2-oxo-1-pyrrolidine ring the following 1,3-dihydro-2H-indol-2-one cycle

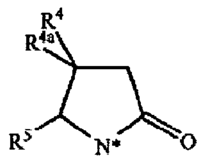 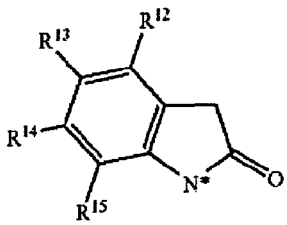

wherein

$R^{12}$ is hydrogen;

$R^{13}$ is selected from methyl; chloro; bromo;

$R^{14}$ is hydrogen;

$R^{15}$ hydrogen;

$R^6$ is hydrogen;

$R^7$ is hydrogen;

with the proviso that $R^4$ is different from hydrogen when

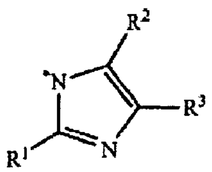

represents a group of formula

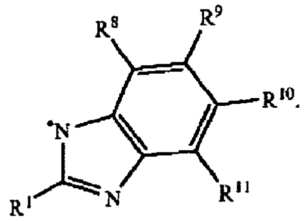

In a most preferred instance, the compounds of formula I, or pharmaceutically acceptable salt thereof, are those wherein
$R^1$ is selected from hydrogen; methyl; methylthio; nitro; cyano; amino; chloro;
$R^2$ is selected from hydrogen; chloro; cyano;
$R^3$ is hydrogen;
$R^4$ is selected from n-propyl; 2,2-difluorovinyl; phenyl; 3-chlorophenyl; 3-fluorophenyl; 3,5-difluorophenyl; 2,3,4-trifluorophenyl; 2,4,5-trifluorophenyl; 2,3,5-trifluorophenyl; 3,4,5-trifluorophenyl; 3-azido-2,4-difluorophenyl;
$R^{4a}$ is hydrogen;
$R^5$ is hydrogen;
or $R^4$, $R^{4a}$ and $R^5$ can form together with the 2-oxo-1-pyrrolidine ring the following 1,3-dihydro-2H-indol-2-one cycle

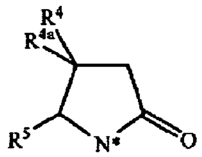 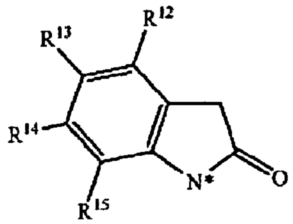

$R^{12}$ is hydrogen;
$R^{13}$ is selected from chloro; bromo; methyl;
$R^{14}$ is hydrogen;
$R^{15}$ hydrogen;
$R^6$ is hydrogen;
$R^7$ is hydrogen.

In some instances, compounds useful in the methods and compositions of this disclosure are selected from the group consisting of: 1-(1H-imidazol-1-ylmethyl)pyrrolidin-2-one; 1-(1H-imidazol-1-ylmethyl)-4-phenylpyrrolidin-2-one; 4-(3-azido-2,4,6-trifluorophenyl)-1-(1H-imidazol-1-ylmethyl)pyrrolidin-2-- one; 1-(1H-imidazol-1-ylmethyl)-4-propylpyrrolidin-2-one; (-)-4-(3-azido-2,4-difluorophenyl)-1-(1H-imidazol-1-ylmethyl)pyrrolidin-2- -one; (+)-4-(3-azido-2,4-difluorophenyl)-1-(1H-imidazol-1-ylmethyl)pyrrolidin-2-one; 1-[(2-ethyl-1H-imidazol-1-yl)methyl]-4-propylpyrrolidin-2-one; 1-[(2-isopropyl-1H-imidazol-1-yl)methyl]-4-propylpyrrolidin-2-one; 1-[(2-methyl-1H-imidazol-1-yl)methyl]-4-propylpyrrolidin-2-one; 1-[(2-phenyl-1H-imidazol-1-yl)methyl]-4-propylpyrrolidin-2-one; 4-propyl-1-[(2-propyl-1H-imidazol-1-yl)methyl]pyrrolidin-2-one; (+)-1-(1 H-imidazol-1-ylmethyl)-4-propylpyrrolidin-2-one; (-)-1-(1H-imidazol-1-ylmethyl)-4-propylpyrrolidin-2-one; 4-(2,2-difluorovinyl)-1-(1H-imidazol-1-ylmethyl)pyrrolidin-2-one; 4-(3-chlorophenyl)-1-(1H-imidazol-1-ylmethyl)pyrrolidin-2-one; 1-{[2-(methylthio)-1H-imidazol-1-yl]methyl}-4-propylpyrrolidin-2-one; 1-{[2-(methylsulfinyl)-1H-imidazol-1-yl]methyl}-4-propylpyrrolidin-2-one; 1-[(2-tert-butyl-1H-imidazol-1-yl)methyl]-4-propylpyrrolidin-2-one; 1-[1-(1H-imidazol-1-yl)cyclopropyl]pyrrolidin-2-one; 1-[(2-methyl-1H-imidazol-1-yl)methyl]-4-phenylpyrrolidin-2-one; 1-{[2-(methylsulfonyl)-1H-imidazol-1-yl]methyl}-propylpyrrolidin-2-one; 1-[(2-oxo-4-propylpyrrolidin-1-yl)methyl]-1H-imidazole-2-carboxamide, 4-(4-fluorophenyl)-1-(1H-imidazol-1-ylmethyl)pyrrolidin-2-one; 1-(1H-imidazol-1-ylmethyl)-4-(3,4,5-trifluorophenyl)pyrrolidin-2-one; 4-(3-fluorophenyl)-1-(1H-imidazol-1-ylmethyl)pyrrolidin-2-one; 4-(3,5-difluorophenyl)-1-(1H-imidazol-1-ylmethyl)pyrrolidin-2-one; 4-(3,4-difluorophenyl)-1-(1H-imidazol-1-ylmethyl)pyrrolidin-2-one; 4-(3-chloro-4-fluorophenyl)-1-(1H-imidazol-1-ylmethyl)pyrrolidin-2-one; 4-(4-chlorophenyl)-1-(1H-imidazol-1-ylmethyl)pyrrolidin-2-one; 1-(1H-imidazol-1-ylmethyl)-4-(2,3,4-trifluorophenyl)pyrrolidin-2-one; 1-(1H-imidazol-1-ylmethyl)-4-(2,3,5-trifluorophenyl)pyrrolidin-2-one; 1-(1H-imidazol-1-ylmethyl)-4-(2,4,5-trifluorophenyl)pyrrolidin-2-one; 1-{[2-(hydroxymethyl)-1H-imidazol-1-yl]methyl}-4-propylpyrrolidin-2-one; methyl 1-[(2-oxo-4-propylpyrrolidin-1-yl)methyl]-1H-imidazole-2-carboxyla- te; 1-[(2-nitro-1H-imidazol-1-yl)methyl]-4-(3,4,5-trifluorophenyl)pyrrolidin-2-one; 1-{[2-oxo-4-(3,4,5-trifluorophenyl)pyrrolidin-1-yl]methyl}-1H-imidazole-2-carbonitrile; 1-[(2-amino-1 H-imidazol-1-yl)methyl]-4-pro-pylpyrrolidin-2-one; 1-[(2,4-dichloro-1H-imidazol-1-yl)methyl]-4-(3,4,5-trifluorophenyl)pyrrolidin-2-one; 1-[(5-chloro-1H-imidazol-1-yl)methyl]-4-(3,4,5-trifluorophenyl)pyrrolidin- -2-one; 1-{[2-oxo-4-(3,4,5-trifluorophenyl)pyrrolidin-1-yl]methyl}-1H-imidazole-4-carbonitrile; 1-{[2-oxo-4-(3,4,5-trifluorophenyl)pyrrolidin-1-yl]methyl}-1H-imidazole-5-carbonitrile; (+)-1-(1H-imidazol-1-ylmethyl)-4-phenylpyrrolidin-2-one; (-)-1-(1H-imidazol-1-ylmethyl)-4-phenylpyrrolidin-2-one; 1-{[2-oxo-4-(2,3,5-trifluorophenyl)pyrrolidin-1-yl]methyl}-1H-imidazole-5-carbonitrile; (-)-1-{[2-oxo-4-(2,3,4-trifluorophenyl)pyrrolidin-1-yl]methyl}-1H-imidazole-5-carbonitrile; (+)-1-{[2-oxo-4-(2,3,4-trifluorophenyl)pyrrolidin-1-yl]methyl}-1H-imidazole-5-carbonitrile; (-)-1-{[2-oxo-4-(2,3,4-trifluorophenyl)pyrrolidin-1-yl]methyl}-1H-imidazole-4-carbonitrile; (+)-1-{[2-oxo-4-(2,3,4-trifluorophenyl)-1- pyrrolidinyl]methyl}-1H-imidazole-4-carbonitrile; (-)-1-{[2-oxo-4-(3,4,5-trifluorophenyl)pyrrolidin-1-yl]methyl}-1H-imidazole-4-carbonitrile; (+)-1-{[2-oxo-4-(3,4,5-trifluorophenyl)pyrrolidin-1-yl]methyl}-1H-imidazole-4-carbonitrile; (+)-1-{[2-oxo-4-(2,4,5-trifluorophenyl)pyrrolidin-1-yl]methyl}-1H-imidazole-4-carbonitrile; (-)-1-{[2-oxo-4-(2,4,5-trifluorophenyl)pyrrolidin-1-yl]methyl}-1H-imidazole-4-carbonitrile; (-)-1-{[2-oxo-4-(2,3,5-trifluorophenyl)pyrrolidin-1-yl]methyl}-1H-imidazole-4-carbonitrile; (-)-1-{[2-oxo-4-(3,4,5-trifluorophenyl)pyrrolidin-1-yl]methyl}-1H-imidazole-5-carbonitrile; 1-{[2-oxo-4-(2,3,5-trifluorophenyl)pyrrolidin-1-yl]methyl}-1H-imidazole-5- -carbonitrile; 1-{[2-oxo-4-(2,3,5-trifluorophenyl)pyrrolidin-1-yl]methyl}-1H-imidazole-5- -carbonitrile; 1-[(5-methyl-2-phenyl-1H-imidazol-1-yl)methyl]-4-propylpyrrolidin-2-one; 1-[(5-methyl-1H-imidazol-1-yl)methyl]-4-propylpyrrolidin-2-one; 1-[(5-phenyl-1H-imidazol-1-yl)methyl]-4-propylpyrrolidin-2-one; 1-[(2-ethyl-5-methyl-1H-imidazol-1-yl)methyl]-4-propylpyrrolidin-2-one; 1-[(2,5-dimethyl-1H-imidazol-1-yl)methyl]-4-propylpyrrolidin-2-one; 1-[(2-chloro-1H-imidazol-1-yl)methyl]-4-(3,4,5-trifluorophenyl)pyrrolidin- -2-one; 1-[2-azido-1-(1H-imidazol-1-yl)ethyl]-4-propylpyrrolidin-2-one; 1-[(4-chloro-1H-imidazol-1-yl)methyl]-4-(3,4,5-trifluorophenyl)pyrrolidin- -2-one; 1-[(2-bromo-4,5-dichloro-1H-imidazol-1-yl)methyl]-4-propylpyrrolidin-2-one; 1-[(2-chloro-1H-imidazol-1-yl)methyl]-4-propylpyrrolidin-2-one; (+)-1-{[2-oxo-4-(3,4,5-trifluorophenyl)pyrrolidin-1-yl]methyl}-1H-imidazole-5-carbonitrile; 1-{[5-(hydroxymethyl)-1H-imidazol-1-yl]methyl}-4-propylpyrrolidin-2-one; 1-{[4-(hydroxymethyl)-1H-imidazol-1-yl]methyl}-4-propylpyrrolidin-2-one; benzyl 1-[(2-oxo-4-propylpyrrolidin-1-yl)methyl]-1H-imidazol-5-ylcarbamat- e; N-[(1-{[2-oxo-4-(3,4,5-trifluorophenyl)pyrrolidin-1-yl]methyl}-1H-imidazol-5-yl)methyl]acetamide; N-[(1-{[2-oxo-4-(3,4,5-trifluorophenyl)pyrrolidin-1-yl]methyl}-1H-imidazol-5-yl)methyl]benzamide; N-[(1-{[2-oxo-4-(3,4,5-trifluorophenyl)pyrrolidin-1-yl]methyl}-1H-imidazol-5-yl)methyl]propanamide; 1-(1H-benzimidazol-1-ylmethyl)-4-propylpyrrolidin-2-one; 1-[(2-methyl-1H-benzimidazol-1-yl)methyl]-4-propylpyrrolidin-2-one; 4-propyl-1-[(2-propyl-1H-benzimidazol-1-yl)methyl]pyrrolidin-2-one; 1-[(2-isopropyl-1H-benzimidazol-1-yl)methyl]-4-propylpyrrolidin-2-one; 4-propyl-1-{[2-(trifluoromethyl)-1H-benzimidazol-1-yl]methyl}pyrrolidin-2- -one; 1-{[2-(methylthio)-1H-benzimidazol-1-yl]methyl}-4-propylpyrrolidin-2- -one; 1-[(2-amino-1H-benzimidazol-1-yl)methyl]-4-propylpyrrolidin-2-one; 1-{[2-(chloromethyl)-1H-benzimidazol-1-yl]methyl}-4-propylpyrrolidin-2-on- e; {1-[(2-oxo-4-propylpyrrolidin-1-yl)methyl]-1H-benzimidazol-2-yl}acetoni- trile; 1-[(5-methoxy-1H-benzimidazol-1-yl)methyl]-4-propylpyrrolidin-2-one- ; 1-[(5-methyl-1H-benzimidazol-1-yl)methyl]-4-propylpyrrolidin-2-one; 1-[(5,6-dimethyl-1H-benzimidazol-1-yl)methyl]-4-propylpyrrolidin-2-one; 1-{[2-isopropyl-5-(trifluoromethyl)-1 H-benzimidazol-1-yl]methyl} -4-propylpyrrolidin-2-one; 1-[(6-chloro-1H-benzimidazol-1-yl)methyl]-4-propylpyrrolidin-2-one; 1-[(2-oxo-4-propylpyrrolidin-1-yl)methyl]-2-propyl-1 H-benzimidazole-5-car- bonitrile; 1-{[2-ethyl-5-(trifluoromethyl)-1H-benzimidazol-1-yl]methyl}-4-- propylpyrrolidin-2-one; 4-propyl-1-{[2-(1H-pyrrol-2-yl)-1H-benzimidazol-1-yl]methyl}pyrrolidin-2-- one; 1-[(5-fluoro-2-propyl-1H-benzimidazol-1-yl)methyl]-4-propylpyrrolidin- -2-one; 1-{[6-methyl-2-(1 H-pyrrol-2-yl)-1 H-benzimidazol-1-yl]methyl} -4-pro-pylpyrrolidin-2-one; 1-[(6-methoxy-2-propyl-1H-benzimidazol-1-yl)methyl]-4-propylpyrrolidin-2-- one ; 2-butyl-1-[(2-oxo-4-propylpyrrolidin-1-yl)methyl]-1H-benzimidazole-5--carbonitrile; 1-{[2-[2-(methylthio)ethyl]-5-(trifluoromethyl)-1H-benzimidazol-1-yl]methyl}-4-propylpyrrolidin-2-one; 1-[(5-fluoro-2-isobutyl-1H-benzimidazol-1-yl)methyl]-4-propylpyrrolidin-2- -one; 1-{[5-fluoro-2-(2,4,4-trimethylpentyl)-1H-benzimidazol-1-yl]methyl}-- 4-propylpyrrolidin-2-one; 2-cyclopropyl-1-[(2-oxo-4-propylpyrrolidin-1-yl)methyl]-1H-benzimidazole-- 5-carbonitrile; 1-[(2-oxo-4-propylpyrrolidin-1-yl)methyl]-2-(1H-pyrazol-3-yl)-1H-benzimidazole-5-carbonitrile; 1-[(2-cyclopropyl-5-fluoro-1 H-benzimidazol-1-yl)methyl]-4-propylpyrrolidin-2-one; 1-[(5-fluoro-2-isopropyl-1H-benzimidazol-1-yl)methyl]-4-propylpyrrolidin-2-one; 1-{[2-(3-furyl)-6-methoxy-1H-benzimidazol-1-yl]methyl}-4-propylpyrrolidin- -2-one; 1-[(2-cyclopropyl-6-methoxy-1H-benzimidazol-1-yl)methyl]-4-propylp- yrrolidin-2-one; 1-[(2-isopropyl-6-methoxy-1H-benzimidazol-1-yl)methyl]-4-propylpyrrolidin- -2-one; 1-[(2-oxo-4-propylpyrrolidin-1-yl)methyl]-2-(1,2,3-thiadiazol-4-yl-)-1H-benzimidazole-5-carbonitrile; 1-{[2-(1H-imidazol-2-yl)-5-(trifluoromethyl)-1H-benzimidazol-1-yl]methyl}--4-propylpyrrolidin-2-one; 1-{[5-fluoro-2-(2,2,2-trifluoroethyl)-1H-benzimidazol-1-yl]methyl}-4-propylpyrrolidin-2-one; 1-{[2-(1-ethylpropyl)-6-methoxy-1H-benzimidazol-1-yl]methyl}-4-propylpyrrolidin-2-one; 1-{[6-methoxy-2-(1-methyl-1H-pyrrol-2-yl)-1H-benzimidazol-1-yl]methyl}-4-- propylpyrrolidin-2-one; 1-{[2-(2-furyl)-5-(trifluoromethyl)-1H-benzimidazol-1-yl]methyl} -4-propyl- pyrrolidin-2-one; 4-propyl-1-{[2-thien-2-yl-5-(trifluoromethyl)-1H-benzimidazol-1-yl]methyl- }pyrrolidin-2-one; 1-{[2-(3-furyl)-5-(trifluoromethyl)-1H-benzimidazol-1-yl]methyl}-4-propylpyrrolidin-2-one; 1-{[2-cyclopropyl-5-(trifluoromethyl)-1H-benzimidazol-1-yl]methyl}-4-propylpyrrolidin-2-one; 4-propyl-1-{[2-(1H-pyrrol-2-yl)-5-(trifluoromethyl)-1H-benzimidazol-1-yl]-methyl}pyrrolidin-2-one; 1-(1H-imidazol-1-ylmethyl)-1,3-dihydro-2H-indol-2-one; 5-bromo-1-(1H-imidazol-1-ylmethyl)-1,3-dihydro-2H-indol-2-one; 5-chloro-1-(1H-imidazol-1-ylmethyl)-1,3-dihydro-2H-indol-2-one; 4-fluoro-1-(1H-imidazol-1-ylmethyl)-1,3-dihydro-2H-indol-2-one; 4-chloro-1-(1H-imidazol-1-ylmethyl)-1,3-dihydro-2H-indol-2-one; 1-(1H-imidazol-1-ylmethyl)-5-methyl-1,3-dihydro-2H-indol-2-one; 1-[(2-oxo-2,3-dihydro-1H-indol-1-yl)methyl]-1H- imidazole-5-carbonitrile; and 1-[(5-chloro-2-oxo-2,3-di-

hydro-1-H-indol-1-yl)methyl]-1H-imidazole-5-c- arbonitrile.

In some instances, compounds useful in the methods and compositions of this disclosure are selected from the group consisting of: 1-(1H-imidazol-1-ylmethyl)pyrrolidin-2-one, 1-(1H-imidazol-1-ylmethyl)-4-phenylpyrrolidin-2-one; 1-(1 H-imidazol-1-ylmethyl)-4-propylpyrrolidin-2-one; (-)-4-(3-azido-2,4-difluorophenyl)-1-(1H-imidazol-1-ylmethyl)pyrrolidin-2- -one; (+)-4-(3-azido-2,4-difluorophenyl)-1-(1H-imidazol-1-ylmethyl)pyrrolidin-2-one; 1-[(2-ethyl-1H-imidazol-1-yl)methyl]-4-propylpyrrolidin-2-one; 1-[(2-isopropyl-1H-imidazol-1-yl)methyl]-4-propylpyrrolidin-2-one; 1-[(2-methyl-1H-imidazol-1-yl)methyl]-4-propylpyrrolidin-2-one; 4-propyl-1-[(2-propyl-1H-imidazol-1-yl)methyl]pyrrolidin-2-one; (+)-1-(1H-imidazol-1-ylmethyl)-4-propylpyrrolidin-2-one; (-)-1-(1H-imidazol-1-ylmethyl)-4-propylpyrrolidin-2-one; 4-(2,2-difluorovinyl)-1-(1H-imidazol-1-ylmethyl)pyrrolidin-2-one; 4-(3-chlorophenyl)-1-(1H-imidazol-1-ylmethyl)pyrrolidin-2-one; 1-{[2-(methylthio)-1H-imidazol-1-yl]methyl}-4-propylpyrrolidin-2-one; 1-[(2-methyl-1H-imidazol-1-yl)methyl]-4-phenylpyrrolidin-2-one; 4-(4-fluorophenyl)-1-(1H-imidazol-1-ylmethyl)pyrrolidin-2-one; 1-(1H-imidazol-1-ylmethyl)-4-(3,4,5-trifluorophenyl)pyrrolidin-2-one; 4-(3-fluorophenyl)-1-(1H-imidazol-1-ylmethyl)pyrrolidin-2-one; 4-(3,5-difluorophenyl)-1-(1H-imidazol-1-ylmethyl)pyrrolidin-2-one; 4-(3,4-difluorophenyl)-1-(1H-imidazol-1-ylmethyl)pyrrolidin-2-one; 4-(3-chloro-4-fluorophenyl)-1-(1H-imidazol-1-ylmethyl)pyrrolidin-2-one; 4-(4-chlorophenyl)-1-(1H-imidazol-1-ylmethyl)pyrrolidin-2-one; 1-(1H-imidazol-1-ylmethyl)-4-(2,3,4-trifluorophenyl)pyrrolidin-2-one; 1-(11H-imidazol-1-ylmethyl)-4-(2,3,5-trifluorophenyl)pyrrolidin-2-one; 1-(1H-imidazol-1-ylmethyl)-4-(2,4,5-trifluorophenyl)pyrrolidin-2-one; 1-[(2-nitro-1H-imidazol-1-yl)methyl]-4-(3,4,5-trifluorophenyl)pyrrolidin-- 2-once; 1-{[2-oxo-4-(3,4,5-trifluorophenyl)pyrrolidin-1-yl]methyl}-1H-imidazole-2-carbonitrile; 1-[(2-amino-1H-imidazol-1-yl)methyl]-4-propylpyrrolidin-2-one; 1-[(5-chloro-1H-imidazol-1-yl)methyl]-4-(3,4,5-trifluorophenyl)pyrrolidin- -2-one; 1-{[2-oxo-4-(3,4,5-trifluorophenyl)pyrrolidin-1-yl]methyl} -1 H-imidazole-4-carbonitrile; 1-{[2-oxo-4-(3,4,5-trifluorophenyl)pyrrolidin-1-yl]methyl}-1H-imidazole-5-carbonitrile; (+)-1-(H-imidazol-1-ylmethyl)-4-phenylpyrrolidin-2-one; (-)-1-(1H-imidazol-1-ylmethyl)-4-phenylpyrrolidin-2-one; (+); 1-{[2-oxo-4-(3,4,5-trifluorophenyl)pyrrolidin-1-yl]methyl}-1H-imidazole-4- -carbonitrile; 1-[(2-chloro-1H-imidazol-1-yl)methyl]-4-(3,4,5-trifluorophenyl)pyrrolidin- -2-one; 1-[2-azido-1-(1H-imidazol-1-yl)ethyl]-4-propylpyrrolidin-2-one; 1-[(2-chloro-1H-imidazol-1-yl)methyl]-4-propylpyrrolidin-2-one; (+)-1-{[2-oxo-4-(3,4,5-trifluorophenyl)pyrrolidin-1-yljmethyl}-1H-imidazole-5-carbonitrile; 1-[(2-oxo-4-propylpyrrolidin-1-yl)methyl]-2-propyl-1H-benzimidazole-5-carbonitrile; 1-{[2-ethyl-5-(trifluoromethyl)-1H-benzimidazol-1-yl]methyl}-4-propylpyrrolidin-2-one; 4-propy1-1-{[2-(1H-pyrrol-2-y1)-1H-benzimidazol-1-yl]methyl}pyrrolidin-2-- one; 1-[(5-fluoro-2-propyl-1H-benzimidazol-1-yl)methyl]-4-propylpyrrolidin- -2-one; 2-butyl-1-[(2-oxo-4-propylpyrrolidin-1-yl)methyl]-1H-benzimidazole--5-carbonitrile; 1-[(5-fluoro-2-isopropyl-1H-benzimidazol-1-yl)methyl]-4-propylpyrrolidin-- 2-one; 1-(1H-imidazol-1-ylmethyl)-1,3-dihydro-2H-indol-2-one; 5-bromo-1-(1H-imidazol-1-ylmethyl)-1,3-dihydro-2H-indol-2-one; 5-chloro-1-(1H-imidazol-1-ylmethyl)-1,3-dihydro-2H-indol-2-one; 1-(1H-imidazol-1-ylmethyl)-5-methyl-1,3-dihydro-2H-indol-2-one; 1-[(5-chloro-2-oxo-2,3-dihydro-1H-indol-1-yl)methyl]-1H-imidazole-5-carbo- nitrile.

In some instances, compounds useful in the methods and compositions of this disclosure are selected from the group consisting of: 1-(1H-imidazol-1-ylmethyl)-4-phenylpyrrolidin-2-one; 1-(1H-imidazol-1-ylmethyl)-4-propylpyrrolidin-2-one; (-)-4-(3-azido-2,4-difluorophenyl)-1-(1H-imidazol-1-ylmethyl)pyrrolidin-2- -one; (+)-4-(3-azido-2,4-difluorophenyl)-1-(1H-imidazol-1-ylmethyl)pyrrolidin-2-one; 4-(2,2-difluorovinyl)-1-(1H-imidazol-1-ylmethyl)pyrrolidin-2-on- e; 4-(3-chlorophenyl)-1-(1H-imidazol-1-ylmethyl)pyrrolidin-2-one; 1-{[2-(methylthio)-1H-imidazol-1-yl]methyl}-4-propylpyrrolidin-2-one; 1-[(2-methyl-1H-imidazol-1-yl)methyl]-4-phenylpyrrolidin-2-one; 1-(1H-imidazol-1-ylmethyl)-4-(3,4,5-trifluorophenyl)pyrrolidin-2-one; 4-(3-fluorophenyl)-1-(1H-imidazol-1-ylmethyl)pyrrolidin-2-one; 4-(3,5-difluorophenyl)-1-(1H-imidazol-1-ylmethyl)pyrrolidin-2-one; 1-(1H-imidazol-1-ylinethyl)-4-(2,3,4-trifluorophenyl)pyrrolidin-2-one; 1-(1H-imidazol-1-ylmethyl)-4-(2,3,5-trifluorophenyl)pyrrolidin-2-one; 1-(1H-imidazol-1-ylmethyl)-4-(2,4,5-trifluorophenyl)pyrrolidin-2-one; 1-[(2-nitro-1H-imidazol-1-yl)methyl]-4-(3,4,5-trifluorophenyl)pyrrolidin-- 2-one; 1-{[2-oxo-4-(3,4,5-trifluorophenyl)pyrrolidin-1-yl]methyl}-1H-imidazole-2-carbonitrile; 1-[(2-amino-1H-imidazol-1-yl)methyl]-4-propylpyrrolidin-2-one; 1-[(5-chloro-1H-imidazol-1-yl)methyl]-4-(3,4,5-trifluorophenyl)pyrrolidin- -2-one; (+)-1-(1H-imidazol-1-ylmethyl)-4-phenylpyrrolidin-2-one; (-)-1-(1H-imidazol-1-ylmethyl)-4-phenylpyrrolidin-2-one; 1-[(2-chloro-1H-imidazol-1-yl)methyl]-4-(3,4,5-trifluorophenyl)pyrrolidin- -2-one; 1-[(2-chloro-1H-imidazol-1-yl)methyl]-4-propylpyrrolidin-2-one; (+)-1-{[2-oxo-4-(3,4,5-trifluorophenyl)pyrrolidin-1-yl]methyl}-1H-imidazole-5-carbonitrile; 5-bromo-1-(1H-imidazol-1-ylmethyl)-1,3-dihydro-2H-indol-2-one; 5-chloro-1-(1H-imidazol-1-ylmethyl)-1,3-dihydro-2H-indol-2-one; 1-(1H-imidazol-1-ylmethyl)-5-methyl-1,3-dihydro-2H-indol-2-one; 1-[(5-chloro-2-oxo-2,3-dihydro-1H-indol-1-yl)methyl]-1H-imidazole-5-carbo- nitrile.

In some instances, compounds useful in the methods and compositions of this disclosure are selected from the group consisting of: (-)-4-(3-azido-2,4-difluorophenyl)-1-(1H-imidazol-1-ylmethyl)pyrrolidin-2- -one; (+)-4-(3-azido-2,4-difluorophenyl)-1-(1H-imidazol-1-ylmethyl)pyrrolidin-2-one; 4-(3-azido-2,4-difluorophenyl)-1-(1H-imidazol-1-ylmethyl)pyrrolidin-2-one.

v) International Patent Application WO 2007/065595:

Compounds having formula I, their enantiomers, diastereoisomers and mixtures thereof (including all possible mixtures of stereoisomers), or pharmaceutically acceptable salts thereof,

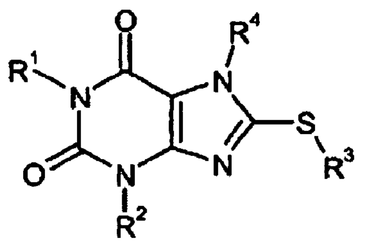

(I)

wherein

$R^1$ is hydrogen or $C_{1-6}$ alkyl;

$R^2$ is hydrogen or $C_{1-4}$ alkyl;

$R^3$ is a group of formula $-CHR^5R^6$ or a benzyl group;

$R^4$ is $C_{1-8}$ alkyl optionally substituted by alkoxycarbonyl, C3-6 cycloalkyl, aryl or heterocycle;

$R^5$ is C2-4 alkyl;

$R^6$ is C2-4 alkyl, amido or $-COOR^7$;

$R^7$ is $C_{1-4}$ alkyl;

Usually when $R^3$ is a benzyl group, then $R^4$ is $C_{1-8}$ alkyl optionally substituted by alkoxycarbonyl.

Usually when $R^3$ is a group of formula $-CHR^5R^6$ then $R^4$ is $C_{1-8}$ alkyl optionally substituted by $C_{3-6}$ cycloalkyl, aryl or heterocycle.

The term "alkyl", as used herein, is a group which represents saturated, monovalent hydrocarbon radicals having straight (unbranched) or branched moieties, or combinations thereof, and containing 1-8 carbon atoms, preferably 1-6 carbon atoms; more preferably alkyl groups have 1-4 carbon atoms. Alkyl moieties may optionally be substituted by 1 to 5 substituents independently selected from the group consisting of hydroxy, alkoxy, cyano, ethynyl, alkoxycarbonyl, acyl, aryl or heterocycle. Alkyl moieties may be optionally substituted by a cycloalkyl as defined hereafter. Preferred alkyl groups are methyl, cyanomethyl, ethyl, 2-ethoxy-2-oxoethyl, 2- methoxyethyl, n-propyl, 2-oxopropyl, 3-hydroxypropyl, 2-propynyl, n-butyl, i-butyl, n-pentyl, 3-pentyl, n-hexyl, cyclohexylmethyl, benzyl, 2-bromobenzyl, 3-bromobenzyl, 4-bromobenzyl, 3-methoxybenzyl, 3-nitrobenzyl, 3-aminobenzyl, 4-(aminosulfonyl)benzyl, 1- phenylethyl, 2-phenylethyl, (3,5-dimethylisoxazol-4-yl)methyl or (5-nitro-2-furyl)methyl. More preferred alkyl groups are methyl, ethyl, cyanomethyl, 2-methoxyethyl, n-propyl, 3- hydroxypropyl, 2-propynyl, n-butyl, 3-pentyl, n-hexyl, benzyl, 3-bromobenzyl, 3- methoxybenzyl, 3-nitrobenzyl, 3-aminobenzyl, (3,5-dimethylisoxazol-4-yl)methyl or (5-nitro- 2-furyl)methyl. Most preferred alkyl groups are methyl, ethyl, 3-methoxybenzyl, 3- nitrobenzyl or (5-nitro-2-furyl)methyl.

The term "cycloalkyl", as used herein, represents a monovalent group of 3 to 8, preferably 3 to 6 carbon atoms derived from a saturated cyclic hydrocarbon, which may be substituted by any suitable group including but not limited to one or more moieties selected from groups as described above for the alkyl groups. Preferred cycloalkyl group is cyclohexyl.

The term "aryl" as used herein, is defined as a phenyl group optionally substituted by 1 to 4 substituents independently selected from halogen, amino, nitro, alkoxy or aminosulfonyl. Preferred aryl groups are phenyl, 2-bromophenyl, 3-bromophenyl, 4- bromophenyl, 3-methoxyphenyl, 3-nitrophenyl, 3-aminophenyl or 4-(aminosulfonyl)phenyl.

The term "phenyl", as used herein, represents an aromatic hydrocarbon group of formula $-C_6H_5$.

The term "benzyl group", as used herein, represents a group of formula-$CH_2$-aryl. Preferred benzyl groups are benzyl, 2-bromobenzyl, 3-bromobenzyl, 4-bromobenzyl, 3- methoxybenzyl, 3-nitrobenzyl, 3-aminobenzyl or 4-(aminosulfonyl)benzyl. More preferred benzyl groups are benzyl, 3-bromobenzyl, 3-methoxybenzyl, 3-nitrobenzyl or 3- aminobenzyl. Most preferred alkyl groups are 3-methoxybenzyl or 3-nitrobenzyl.

The term "halogen", as used herein, represents an atom of fluorine, chlorine, bromine, or iodine. Preferred halogen is bromine.

The term "hydroxy", as used herein, represents a group of formula -OH.

The term "cyano", as used herein, represents a group of formula -CN.

The term "amino", as used herein, represents a group of formula $-NH_2$.

The term "ethynyl", as used herein, represents a group of formula -C=CH.

The term "alkoxy", as used herein, represents a group of formula $-OR^a$ wherein $R^a$ is an alkyl group, as defined above. Preferred alkoxy group is methoxy.

The term "nitro", as used herein, represents a group of formula $-NO_2$.

The term "amido", as used herein, represents a group of formula - C(=O)NH2.

The term "acyl", as used herein, represents a group of formula $-C(=O)R^b$ wherein $R^b$ is an alkyl group, as defined here above. Preferred acyl group is acetyl (-C(=O)Me).

The term "alkoxycarbonyl (or ester)", as used herein, represents a group of formula $-COOR^c$ wherein $R^c$ is an alkyl group; with the proviso that $R^c$ does not represent an alkyl alpha-substituted by hydroxy. Preferred alkoxycarbonyl group is ethoxycarbonyl.

The term "heterocycle", as used herein, represents a 5-membered ring containing one or two heteroatoms selected from O or N. The heterocycle may be substituted by one or two $C_{1-4}$ alkyl or nitro. Preferred heterocycles are (3, 5-dimethylisoxazol-4-yl) or (5-nitro- 2-furyl). Most preferred heterocycle is (5-nitro-2-furyl).

Generally $R^1$ is hydrogen or $C_{1-6}$ alkyl. Usually $R^1$ is hydrogen or $C_{1-6}$ alkyl optionally substituted by hydroxy, alkoxy, cyano, ethynyl, alkoxycarbonyl or acyl. Preferably $R^1$ is hydrogen, methyl, cyanomethyl, 2-ethoxy-2-oxoethyl, 2-methoxyethyl, n- propyl, 2-oxopropyl, 3-hydroxypropyl, 2-propynyl, n-pentyl or n-hexyl. More preferably $R^1$ is hydrogen, methyl, cyanomethyl, 2-methoxyethyl, n-propyl, 3-hydroxypropyl or 2- propynyl. Most preferably $R^1$ is hydrogen.

Generally $R^2$ is hydrogen or $C_{1-4}$ alkyl. Usually $R^2$ is hydrogen or unsubstituted $C_{1-4}$ alkyl. Preferably $R^2$ is hydrogen, methyl or n-butyl. More preferably, $R^2$ is methyl.

Generally $R^3$ is a group of formula $-CHR^5R^6$ or a benzyl group. Preferably $R^3$ is 3-pentyl, 1-(aminocarbonyl)propyl, 1-(ethoxycarbonyl)propyl or 3-bromobenzyl. Most preferably $R^3$ is 1-(ethoxycarbonyl)propyl.

Generally $R^4$ is $C_{1-8}$ alkyl optionally substituted by alkoxycarbonyl, $C_{3-6}$ cycloalkyl, aryl or heterocycle. Usually $R^4$ is $C_{1-8}$ alkyl optionally substituted by cyclohexyl, phenyl, bromophenyl, aminophenyl, methoxyphenyl, nitrophenyl, aminosulfonylphenyl, 3,5-dimethylisoxazol-4-yl, 5-nitro-2-furyl or ethoxycarbonyl. Preferably $R^4$ is n-butyl, i-butyl, n-pentyl, n-hexyl, cyclohexylmethyl, benzyl, 2- bromobenzyl, 3-bromobenzyl, 4-bromobenzyl, 3-methoxybenzyl, 3-nitrobenzyl, 3- aminobenzyl, 4-(aminosulfonyl)benzyl, 1-phenylethyl, 2-phenylethyl, (3,5-dimethylisoxazol-4-yl)methyl, (5-nitro-2-furyl)methyl or 1-(ethoxycarbonyl)propyl. More preferably $R^4$ is n- butyl, n-hexyl, benzyl, 3-bromobenzyl, 3-methoxybenzyl, 3-nitrobenzyl, 3-aminobenzyl, (3,5-dimethylisoxazol-4-yl)methyl, (5-nitro-2-furyl)methyl or 1 - (ethoxycarbonyl)propyl. Most preferably $R^4$ is 3-methoxybenzyl, 3-nitrobenzyl or (5-nitro-2-furyl)methyl.

Generally $R^5$ is $C_{2-4}$ alkyl. Usually $R^5$ is unsubstituted $C_{2-4}4$ alkyl. Preferably $R^5$ is ethyl.

Generally $R^6$ is $C_{2-4}$ alkyl, amido or $-COOR^7$. Usually $R^6$ is unsubstituted $C_{2-4}$ alkyl, amido or $-COOR^7$. Preferably $R^6$ is ethyl, amido or ethoxycarbonyl. Most preferably $R^6$ is ethoxycarbonyl.

Generally $R^7$ is $C_{1-4}$ alkyl. Usually $R^7$ is unsubstituted $C_{1-4}$ alkyl. Preferably, $R^7$ is ethyl.

In some instances, the compounds are those having formula I, and their enantiomers, diastereoisomers and mixtures thereof (including all possible mixtures of stereoisomers), or pharmaceutically acceptable salts thereof,

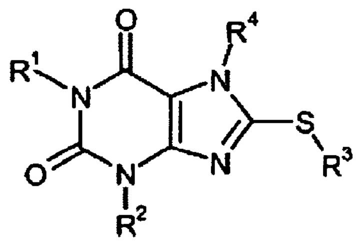
(I)

wherein

$R^1$ is hydrogen, $C_{1-6}$ alkyl optionally substituted by hydroxy, alkoxy, cyano, ethynyl, alkoxycarbonyl or acyl;

$R^2$ is hydrogen or unsubstituted $C_{1-4}$ alkyl;

$R^3$ is a group of formula $-CHR^5$ $R^6$ or a benzyl group;

$R^4$ is $C_{1-8}$ alkyl optionally substituted by cyclohexyl, phenyl, bromophenyl, aminophenyl, methoxyphenyl, nitrophenyl, aminosulfonylphenyl, 3,5-dimethylisoxazol-4-yl, 5-nitro-2-furyl or ethoxycarbonyl;

$R^5$ is unsubstituted $C_{2-4}$ alkyl;

$R^6$ is unsubstituted $C_{2-4}$ alkyl, amido or -$COOR^7$;

$R^7$ is unsubstituted $C_{1-4}$ alkyl;

with the proviso that when $R^1$ is hydrogen, $R^2$ is methyl, $R^3$ is -$CHR^5R^6$, $R^6$ is ethoxycarbonyl and $R^5$ is ethyl, then $R^4$ is different from n-propyl, i-propyl, n-pentyl, n- heptyl, 3-bromobenzyl, 4-chlorobenzyl, 4-methylbenzyl or 2-phenylethyl.

In the above instance, preferably, when $R^3$ is a benzyl group, then $R^4$ is $C_{1-8}$ alkyl optionally substituted by alkoxycarbonyl.

In the above instance, preferably, when $R^3$ is a group of formula -$CHR^5R^6$, then $R^4$ is $C_{1-8}$ alkyl optionally substituted by $C_{3-6}$ cycloalkyl, aryl or heterocycle.

In a preferred instance,
$R^1$ is hydrogen, methyl, cyanomethyl, 2-ethoxy-2-oxoethyl, 2-methoxyethyl, n- propyl, 2-oxopropyl, 3-hydroxypropyl, 2-propynyl, n-pentyl or n-hexyl;
$R^2$ is hydrogen, methyl or n-butyl;
$R^3$ is 3-pentyl, 1-(aminocarbonyl)propyl, 1 -(ethoxycarbonyl)propyl or 3-bromobenzyl;
$R^4$ is n-butyl, i-butyl, n-pentyl, n-hexyl, cyclohexylmethyl, benzyl, 2-bromobenzyl, 3- bromobenzyl, 4-bromobenzyl, 3-methoxybenzyl, 3-nitrobenzyl, 3-aminobenzyl, 4- (aminosulfonyl)benzyl, 1-phenylethyl, 2-phenylethyl, (3,5-dimethylisoxazol-4-yl)methyl, (5- nitro-2-furyl)methyl or 1 - (ethoxycarbonyl)propyl;
with the proviso that when $R^1$ is hydrogen, $R^2$ is methyl and $R^3$ is 1-(ethoxycarbonyl)propyl, then $R^4$ is different from n-pentyl, 3-bromobenzyl or 2- phenylethyl.

In the above instance, preferably, when $R^3$ is 3-bromobenzyl, then $R^4$ is $C_{1-8}$ alkyl optionally substituted by alkoxycarbonyl.

In the above instance, preferably, when $R^3$ is 3-pentyl, 1-(aminocarbonyl)propyl or 1-(ethoxycarbonyl)propyl, then $R^4$ is different from 1- (ethoxycarbonyl)propyl.

In a more preferred instance,
$R^1$ is hydrogen, methyl, cyanomethyl , 2-methoxyethyl, n-propyl, 3-hydroxypropyl or 2-propynyl;
$R^2$ is methyl;
$R^3$ is 3-pentyl, 1-(aminocarbonyl)propyl, 1-(ethoxycarbonyl)propyl or 3-bromobenzyl;
$R^4$ is n-butyl, n-hexyl, benzyl, 3-bromobenzyl, 3-methoxybenzyl, 3-nitrobenzyl, 3- aminobenzyl, (3,5-dimethylisoxazol-4-yl)methyl, (5-nitro-2-furyl)methyl or 1- (ethoxycarbonyl)propyl;
with the proviso that when $R^1$ is hydrogen, $R^2$ is methyl and $R^3$ is 1-(ethoxycarbonyl)propyl, then $R^4$ is different from 3-bromobenzyl.

In the above instance, preferably, when $R^3$ is 3-bromobenzyl, then $R^4$ is 1-(ethoxycarbonyl)propyl;

In the above instance, preferably, when $R^3$ is 3-pentyl, 1-(aminocarbonyl)propyl or 1-(ethoxycarbonyl)propyl, then $R^4$ is different from 1- (ethoxycarbonyl)propyl;

In a most preferred instance, $R^1$ is hydrogen; $R^2$ is methyl; $R^3$ is 1-(ethoxycarbonyl)propyl; and $R^4$ is 3-methoxybenzyl, 3-nitrobenzyl or (5-nitro-2- furyl)methyl.

A further instance consists in compounds wherein $R^2$ is methyl, $R^3$ is a group of formula -$CHR^5R^6$ with $R^5$ being $C_{2-4}$ alkyl, $R^6$ being amido or-$COOR^7$ and $R^7$ being methyl or ethyl.

In some instances, compounds useful in the methods and compositions of this disclosure are selected from the group consisting of: ethyl 2-[(7-benzyl-1 ,3-dimethyl-2,6-dioxo-2,3,6,7- tetrahydro-1H-purin-8-yl)thio]butanoate; ethyl 2-{[7-(3-bromobenzyl)-1-(2-ethoxy-2- oxoethyl)-3-methyl-2,6-dioxo-2,3,6,7-tetrahydro-1H-purin-8-yl]thio}butanoate; ethyl 2-{[7-(3-bromobenzyl)-1-(2-methoxyethyl)-3-methyl-2,6-dioxo-2,3,6,7-tetrahydro-1 H-purin-8-yl]thio}butanoate; ethyl 2-{[7-(3-bromobenzyl)-2,6-dioxo-2,3,6,7-tetrahydro-1 H-purin-8- yl]thio}butanoate; ethyl 2-{[7-(3-bromobenzyl)-1 ,3-dimethyl-2,6-dioxo-2,3,6,7-tetrahydro- 1 H-purin-8-yl]thio}butanoate; ethyl2-{[7-(2-bromobenzyl)-1 ,3-dimethyl-2,6-dioxo-2,3,6,7- tetrahydro-1 H-purin-8-yl]thio}butanoate; ethyl 2-{[7-(3-bromobenzyl)-1-(cyanomethyl)-3- methyl-2,6-dioxo-2,3,6,7-tetrahydro-1 H-purin-8-yl]thio}butanoate; ethy12-{[7-(3-bromobenzyl)-3-methyl-2,6-dioxo-1-propyl-2,3,6,7-tetrahydro-1H-purin-8-yl]thio}butanoate; ethyl 2-{[7-(3-bromobenzyl)-3-methyl-2,6-dioxo-1-(2-oxopropyl)-2,3,6,7-tetrahydro-1H- purin-8-yl]thio}butanoate; ethyl 2-{[7-(3-bromobenzyl)-1-(3-hydroxypropyl)-3-methyl-2,6- dioxo-2,3,6,7-tetrahydro-1 H-purin-8-yl]thio}butanoate; ethyl 2-{[7-(3-bromobenzyl)-3- methyl-2,6-dioxo-1-(2-propynyl)-2,3,6,7-tetrahydro-1 H-purin-8-yl]thio}butanoate; ethyl 2- {[7-(3-methoxybenzyl)-3-methyl-2,6-dioxo-2,3,6,7-tetrahydro-1 H-purin-8-yl]thio}butanoate; ethyl 2-{[3-methyl-7-(3-nitrobenzyl)-2,6-dioxo-2,3,6,7-tetrahydro-1 H-purin-8- yl]thio}butanoate; ethyl 2-{[7-(3-aminobenzyl)-3-methyl-2,6-dioxo-2,3,6,7-tetrahydro-1 H-purin-8-yl]thio}butanoate; ethyl 2-({7-[4-(aminosulfonyl)benzyl]-3-methyl-2,6-dioxo-2, 3,6,7- tetrahydro-1H-purin-8-yl}thio)butanoate; ethyl 2-{[7-(4-bromobenzyl)-1,3-dimethyl-2,6-dioxo-2,3,6,7-tetrahydro-1 H-purin-8-yl]thio}butanoate; ethyl 2-{[7-(cyclohexylmethyl)-1 ,3- dimethyl-2,6-dioxo-2,3,6,7-tetrahydro-1H-purin-8-yl]thio}butanoate; ethyl 2-{[1 ,3-dimethyl- 2,6-dioxo-7-(1 -phenylethyl)-2,3,6,7-tetrahydro-

1 H-purin-8-yl]thio}butanoate; ethy12-{[1,3-dimethyl-2,6-dioxo-7-(2-phenylethyl)-2,3,6,7-tetrahydro-1H-purin-8-yl]thio}butanoate; ethyl 2-({7-[(3,5-dimethylisoxazol-4-yl)methyl]-3-methyl-2,6-dioxo-2,3,6,7-tetrahydro-1 H-purin-8-yl}thio)butanoate; ethyl 2-({3-methyl-7-[(5-nitro-2-furyl)methyl]-2,6-dioxo-2,3,6,7-tetrahydro- 1 H-purin-8-yl}thio)butanoate; ethyl 2-[(7-butyl-3-methyl-2,6-dioxo-2,3,6,7-tetrahydro-1 H- purin-8-yl)thio]butanoate; ethyl 2-{[7-(3-bromobenzyl)-2,6-dioxo-2,3,6,7-tetrahydro-1H- purin-8-yl]thio}butanoate; ethyl 2-[(1,7-dihexyl-3-methyl-2,6-dioxo-2,3,6,7-tetrahydro-1H-purin-8-yl)thio]butanoate; ethyl 2-[(7-hexyl-3-methyl-2,6-dioxo-2,3,6,7-tetrahydro-1 H-purin- 8-yl)thio]butanoate; ethyl 2-[(3-methyl-2,6-dioxo-1,7-dipentyl-2,3,6,7-tetrahydro-1H-purin-8-yl)thio]butanoate; 2-{[7-(3-bromobenzyl)-3-methyl-2,6-dioxo-2,3,6,7-tetrahydro-1H-purin-8- yl]thio}butanamide; 2-[(7-butyl-3-methyl-2,6-dioxo-2,3,6,7-tetrahydro-1 H-purin-8- yl)thio]butanamide; 7-(3-bromobenzyl)-8-[(1-ethylpropyl)thio]-3-methyl-3,7-dihydro-1 H- purine-2,6-dione; ethyl 2-{8-[(3-bromobenzyl)thio]-1 ,3-dimethyl-2,6-dioxo-1 ,2,3,6- tetrahydro-7H-purin-7-yl}butanoate; and ethyl 2-[(7-isobutyl-3-methyl-2,6-dioxo-2,3,6,7- tetrahydro-1 H-purin-8-yl)thio]butanoate.

In some instances, compounds useful in the methods and compositions of this disclosure are selected from the group consisting of: ethyl 2-[(7-benzyl-1 ,3-dimethyl-2,6-dioxo-2,3,6,7-tetrahydro-1H-purin-8-yl)thio]butanoate; ethyl 2-{[7-(3-bromobenzyl)-1-(2-methoxyethyl)-3- methyl-2,6-dioxo-2,3,6,7-tetrahydro-1 H-purin-8-yl]thio}butanoate; ethyl 2-{[7-(3- bromobenzyl)-1 ,3-dimethyl-2,6-dioxo-2,3,6,7-tetrahydro-1 H-purin-8-yl]thio}butanoate; ethyl 2-{[7-(3-bromobenzyl)-1-(cyanomethyl)-3-methyl-2,6-dioxo-2,3,6,7-tetrahydro-1 H-purin-8- yl]thio}butanoate; ethyl 2-{[7-(3-bromobenzyl)-3-methyl-2,6-dioxo-1-propyl-2,3,6,7- tetrahydro-1 H-purin-8-yl]thio}butanoate; ethyl 2-{[7-(3-bromobenzyl)-1-(3-hydroxypropyl)-3- methyl-2,6-dioxo-2,3,6,7-tetrahydro-1 H-purin-8-yl]thio}butanoate; ethyl 2-{[7-(3- bromobenzyl)-3-methyl-2,6-dioxo-1-(2-propynyl)-2,3,6,7-tetrahydro-1 H-purin-8- yl]thio}butanoate; ethyl 2-{[7-(3-methoxybenzyl)-3-methyl-2,6-dioxo-2,3,6,7-tetrahydro-1H-purin-8-yl]thio}butanoate; ethyl 2-{[3-methyl-7-(3-nitrobenzyl)-2,6-dioxo-2,3,6,7-tetrahydro- 1 H-purin-8-yl]thio}butanoate; ethyl 2-{[7-(3-aminobenzyl)-3-methyl-2,6-dioxo-2,3,6,7- tetrahydro-1 H-purin-8-yl]thio}butanoate; ethyl 2-({7-[(3,5-dimethylisoxazol-4-yl)methyl]-3- methyl-2,6-dioxo-2,3,6,7-tetrahydro-1H-purin-8-yl}thio)butanoate; ethyi 2-({3-methyi-7-[(5- nitro-2-furyl)methyl]-2,6-dioxo-2,3,6,7-tetrahydro-1 H-purin-8-yl}thio)butanoate; ethyl 2-[(7- butyl-3-methyl-2,6-dioxo-2,3,6,7-tetrahydro-1 H-purin-8-yl)thio]butanoate; ethyl 2-[(7-hexyl-3-methyl-2,6-dioxo-2,3,6,7-tetrahydro-1 H-purin-8-yl)thio]butanoate; 2- {[7-(3-bromobenzyl)- 3-methyl-2,6-dioxo-2,3,6,7-tetrahydro-1 H-purin-8-yl]thio}butanamide; 7-(3-bromobenzyl)-8- [(1-ethylpropyl)thio]-3-methyl-3,7-dihydro-1 H-purine-2,6-dione; and ethyl 2-{8-[(3-bromobenzyl)thio]-1,3-dimethyl-2,6-dioxo-1,2,3,6-tetrahydro-7H-purin-7-yl}butanoate.

In some instances, compounds useful in the methods and compositions of this disclosure are selected from the group consisting of: ethyl 2-{[7-(3-methoxybenzyl)-3-methyl-2,6-dioxo-2,3,6,7-tetrahydro-1 H-purin-8-yl]thio}butanoate; ethyl 2-{[3-methyl-7-(3-nitrobenzyl)-2,6- dioxo-2,3,6,7-tetrahydro-1 H-purin-8-yl]thio}butanoate; and ethyl 2-({3-methyl-7-[(5-nitro-2- furyl)methyl]-2,6-dioxo-2,3,6,7-tetrahydro-1 H-purin-8-yl}thio)butanoate.

In some instances, the compounds are those having formula II, their enantiomers, diastereoisomers and mixtures thereof (including all possible mixtures of stereoisomers), or pharmaceutically acceptable salts:

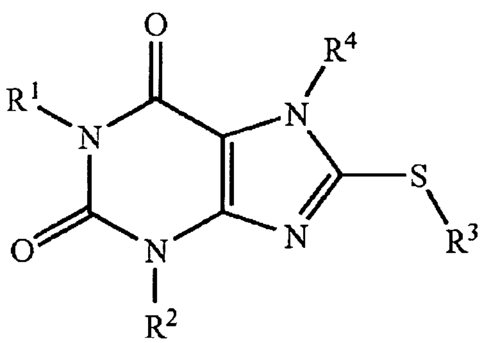

(II)

wherein R.sup.1 is hydrogen or C.sub.1-6 alkyl;

R.sup.2 is hydrogen or C.sub.1-4 alkyl;

R.sup.3 is a group of formula --CHR.sup.SR.sup.6 or a benzyl group;

R.sup.4 is C.sub.1-8 alkyl optionally substituted by alkoxycarbonyl, C.sub.3-6 cycloalkyl, aryl or heterocycle;

R.sup.5 is hydrogen or C.sub.1-4 alkyl;

R.sup.6 is C.sub.1-4 alkyl, amido or --COOR.sup.7;

R.sup.7 is C.sub.1-4 alkyl;

In the above instance, in some cases, when R.sup.3 is a benzyl group, then R.sup.4 is C.sub.1-8 alkyl optionally substituted by alkoxycarbonyl.

In the above instance, in some cases, when R.sup.3 is a group of formula -- CHR.sup.5R.sup.6, then R.sup.4 is C.sub.1-8 alkyl optionally substituted by C.sub.3-6 cycloalkyl, aryl or heterocycle.

In some instances, the compounds are those compounds of formula II, their enantiomers, diastereoisomers and mixtures thereof (including all possible mixtures of stereoisomers), or pharmaceutically acceptable salts

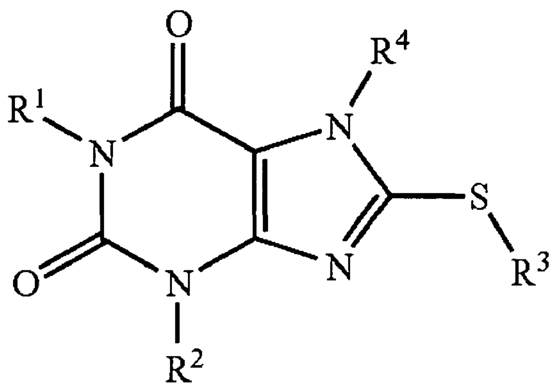

(II)

wherein

R.sup.1 is hydrogen or C.sub.1-6 alkyl;

R.sup.2 is hydrogen or C.sub.1-4 alkyl;

R.sup.3 is a group of formula --CHR.sup.5R.sup.6 or a benzyl group;

R.sup.4 is C.sub.1-8 alkyl optionally substituted by alkoxycarbonyl, C.sub.3-6 cycloalkyl, aryl or heterocycle;

R.sup.5 is hydrogen or C.sub.1-4 alkyl;

R.sup.6 is C.sub.1-4 alkyl, amido or --COOR.sup.7;

R.sup.7 is C.sub.1-4 alkyl.

In some instances, the compounds are compounds of formula II selected from ethyl 2-[(7-heptyl-3-methyl-2,6-dioxo-2,3,6,7-tetrahydro-1H-purin-8-yl)thio]but- anoate; 7-(3-bromobenzyl)-3-methyl-8-(propylthio)-3,7-dihydro-1H-purine-2,- 6-dione; ethyl 2-[(3-methyl-2,6-dioxo-7-pentyl-2,3,6,7-tetrahydro-1H-purin-8-yl)thio]but-anoate; ethyl 2-{[7-(3-bromobenzyl)-3-methyl-2,6-dioxo-2,3,6,7-tetrahydro-1H-purin-8-yl-]thio}butanoate; ethyl 2-[(3-methyl-2,6-dioxo-7-propyl-2,3,6,7-tetrahydro-1H-purin-8-yl)thio]but- anoate; 7-(3-bromobenzyl)-8-[(3-chloro-2-hydroxypropyl)thio]-3-methyl-3,7-- dihydro-1H-purine-2,6-dione; and ethyl 2-{[7-(3-bromobenzyl)-3-methyl-2,6-dioxo-2,3,6,7-tetrahydro-1H-purin-8-yl-]thio}propanoate.

In some instances, the compounds are compounds of formula I, their enantiomers, diastereoisomers and mixtures thereof (including all possible mixtures of stereoisomers), or pharmaceutically acceptable salts

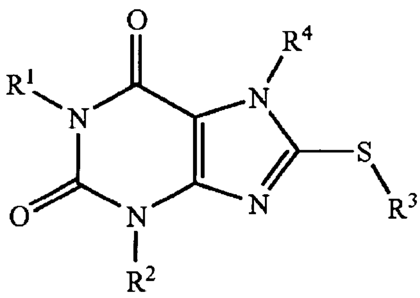

(I)

wherein

R.sup.1 is hydrogen or C.sub.1-6 alkyl;

R.sup.2 is hydrogen or C.sub.1-4 alkyl;

R.sup.3 is a group of formula --CHR.sup.5R.sup.6 or a benzyl group;

R.sup.4 is C.sub.1-8 alkyl optionally substituted by alkoxycarbonyl, C.sub.3-6 cycloalkyl, aryl or heterocycle;

R.sup.5 is C.sub.2-4 alkyl;

R.sup.6 is C.sub.2-4 alkyl, amido or --COOR.sup.7;

R.sup.7 is C.sub.1-4 alkyl;

In another instance, the compounds are compounds having formula II, their enantiomers, diastereoisomers and mixtures thereof (including all possible mixtures of stereoisomers), or pharmaceutically acceptable salts thereof,

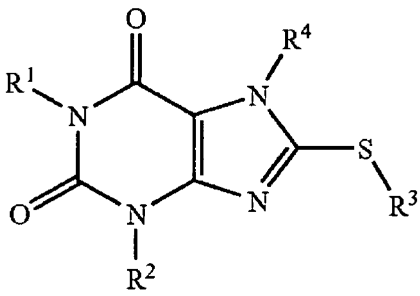

(II)

wherein

R.sup.1 is hydrogen or C.sub.1-6 alkyl;

R.sup.2 is hydrogen or C.sub.1-4 alkyl;

R.sup.3 is a group of formula --CHR.sup.5R.sup.6 or a benzyl group;

R.sup.4 is C.sub.1-8 alkyl optionally substituted by alkoxycarbonyl, C.sub.3-6 cycloalkyl, aryl or heterocycle;

R.sup.5 is hydrogen or C.sub.1-4 alkyl;

R.sup.6 is C.sub.1-4 alkyl, amido or --COOR.sup.7;

R.sup.7 is C.sub.1-4 alkyl;

vi) International Patent Application Publication No. WO2010/14471

In one instance, a chemical composition that includes a LEV derivative of Formula 1 or Formula 2 is disclosed.

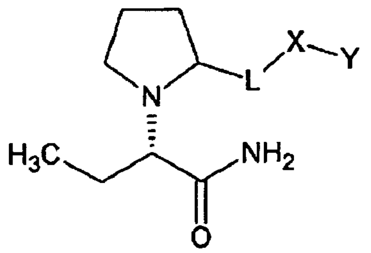

Formula 1

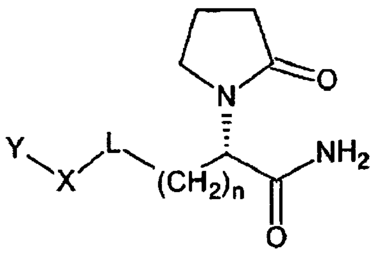

Formula 2

n of Formula 2 and L, X, and Y of Formulas 1 and 2 are defined as follows: a) n is an integer with a value of 0 to 8; b) L is one of the group consisting of CH2, CO, NHCO, NHCOO, CONH, NH, O, or S, and combinations thereof; c) X is an end group, an aromatic group, an aryl group, or a saturated, unsaturated, substituted, unsubstituted, straight chain, or branched chain aliphatic group having from 1 to 10 carbon and/or hetero chain atoms, the hetero chain atoms being selected from the group consisting of oxygen, nitrogen, sulfur, or phosphorus, and combinations thereof; and d) Y is optional and if present is one of a functional group selected from group consisting of alcohol amine, amide, carboxylic acid, aldehyde, ester, iminoester, isocyanate, isothiocyanate, anhydride, thiol, thiolacetone, diazonium, NHS, CO-NHS, O-NHS, maleimido; or e) Y is a Yi-Z where Yi is selected from the group consisting of COO, CO, O, CONH, NHCO, or NH and Z is an operative group.

In one instance of the method, the operative group of Z is selected from the group consisting of detectable labels, antigenic carriers, coupling agents, end groups, proteins, lipoproteins, glycoproteins, polypeptides, polysaccharides, nucleic acids, polynucleotides, teichoic acids, radioactive isotopes, enzymes, enzyme fragments, enzyme donor fragments, enzyme acceptor fragments, enzyme substrates, enzyme inhibitors, coenzymes, fluorescent moieties, phosphorescent moieties, anti-stokes up-regulating moieties, chemiluminescent moieties, luminescent moieties, dyes, sensitizers, particles, microparticles, magnetic particles, solid supports, liposomes, ligands, receptors, hapten radioactive isotopes, and combinations thereof.

vii) International Patent Application Publication No. WO2010/0028

The present disclosure provides a compound of Formula I:

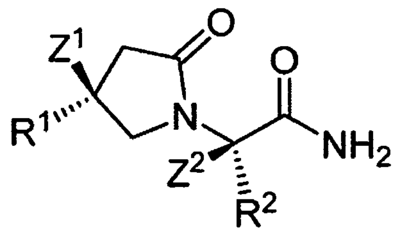

(I),

or a pharmaceutically acceptable salt thereof, wherein: each Z is independently selected from hydrogen and deuterium; R1 is an n-propyl group having zero to seven deuterium atoms; R2 is an ethyl group having zero to five deuterium atoms, and when each R has zero deuterium atoms, at least one Z is deuterium.

One instance of this disclosure provides compounds of Formula I wherein R1 is selected from CD3CH2CH2-, CD3CD2CH2-, CD3CH2CD2-, CH3CH2CD2-, CH3CD2CD2-, CD3CD2CD2- or CH3CH2CH2-. In a more specific instance, R1 is CD3CD2CD2- or

CD3CD2CH2-. In one aspect of these instances, Z1 and Z2 are both hydrogen. In another aspect of these instances, Z1 and Z2 are both deuterium.

In another instance, R2 is selected from CH3CH2-, CD3CH2-, CH3CD2-, or CD3CD2-. In a more specific instance, R2 is selected from CH3CH2- or CD3CD2-. In one aspect of these instances, Z1 and Z2 are both hydrogen. In another aspect of these instances, Z1 and Z2 are both deuterium.

The R and Z variables as described above may be selected and taken together to provide more specific instances of this disclosure. For example, in one instance, R1 is CD3CH2CH2-, CD3CD2CH2-, CD3CH2CD2-, CH3CH2CD2-, CH3CD2CD2-, CD3CD2CD2- or CH3CH2CH2-; and R2 is selected from CH3CH2-, CD3CH2-, CH3CD2-, or CD3CD2-. In one aspect of this instance, R2 is CH3CH2- or CD3CD2-. [0039] In another instance, R1 is CD3CD2CD2- or CD3CD2CH2-; and R2 is selected from CH3CH2-, CD3CH2-, CH3CD2-, or CD3CD2-. In one aspect of this instance, R2 is CH3CH2- or CD3CD2-.

Examples of specific compounds of this disclosure include the following:

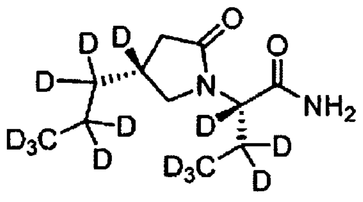
Compound 100;
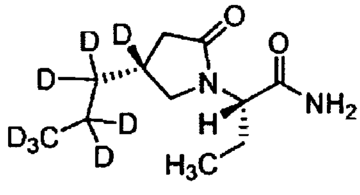
Compound 101;

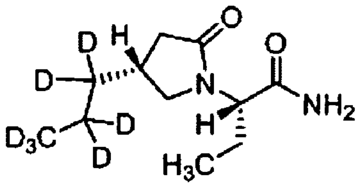
Compound 102; and
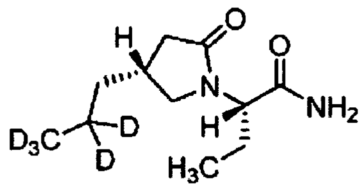
Compound 103

viii) 20090312333

The compounds of the present disclosure are those covered by formula (I), their diastereomers and mixtures, or a pharmaceutically acceptable salt thereof.

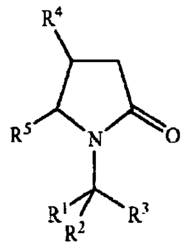
(I)

R1 is hydrogen, substituted or unsubstituted C1-12 alkyl, substituted or unsubstituted aryl or substituted or unsubstituted 3-8 membered heterocycle.

R2 is hydrogen. Alternatively, R1 and R2 may be linked together in such a way to form a C3-6 cycloalkyl.

R3 is either

(a) a substituted or unsubstituted heterocycle linked to the rest of the molecule via one of its C atoms, said heterocycle is selected from the group consisting of:

1H-benzimidazol-6-yl;

1H-benzimidazol-7-yl;

imidazo [1,2-a]pyridin-3-yl;

imidazo[11,2-a]pyrimidin-3-yl;

imidazo[1,2-b][11,2,4]triazin-7-yl;

imidazo[1,2-b]pyridazin-3-yl;

5,6,7,8-tetrahydroimidazo[1,2-b]pyridazin-3-yl;

imidazo[2,1-b][1,3,4]thiadiazol-5-yl;

imidazo[2,1-b][1,3]thiazol-5-yl;

3H-imidazo[4,5-b]pyridin-7-yl;

1H-imidazol-4-yl;

1H-imidazol-5-yl;

1H-indol-2-yl;

1H-indol-3-yl;

1H-indol-4-yl;

1H-indol-7-yl;

isoxazol-4-yl;

1H-pyrazol-4-yl;

1H-pyrazol-5-yl;

1H-pyrazolo[1,5-a]pyrimidin-3-yl;

1H-pyrazolo[3,4-b]pyridin-3-yl;

pyridazin-4-yl;

pyridin-2-yl;

pyridin-3-yl;

pyridin-4-yl;

1H-pyrrolo[2,3-b]pyridin-3-yl;

1H-pyrrolo[2,3-b]pyridin-4-yl;

1H-pyrrolo[2,3-b]pyridin-5-yl;

1H-pyrrolo[2,3-c]pyridin-2-yl;

1H-pyrrolo[2,3-c]pyridin-3-yl;

1H-pyrrolo[3,2-b]pyridin-3-yl;

1 H-pyrrolo[3,2-c]pyridin-2-yl;

1H-pyrrolo[3,2-c]pyridin-3-yl;

1,3,4-thiadiazol-2-yl;

1,3-thiazol-5-yl;

[1,2,4]triazolo[4,3-b]pyridazin-7-yl;

[1,2,4]triazolo[4,3-b]pyridazin-8-yl;

indolizin-3-yl;

or R3 is

(b) a substituted or unsubstituted heterocycle linked to the rest of the molecule via one of its N atoms, said heterocycle is selected from the group consisting of:

1H-1,2,3-benzotriazol-1-yl;

1H-imidazo[4,5-b]pyridin-1-yl;

3H-imidazo[4,5-b]pyridin-3-yl;

7H-imidazo[4,5-c]pyridazin-7-yl;

1H-indol-1-yl;

2,3-dihydro-1H-indol-1-yl;

9H-purin-9-yl;

1H-pyrazolo[3,4-b]pyridin-1-yl;

2H-pyrazolo[3,4-b]pyridin-2-yl;

1H-pyrrolo[2,3-b]pyridin-1-yl;

1H-pyrrolo[3,2-b]pyridin-1-yl;

3,4-dihydroquinolin-1(2H)-yl;

8H-isothiazolo[5,4-b]indol-8-yl;

1H-1,2,4-triazol-1-yl;

1H-pyrrol-1-yl;

2-chloro-1H-benzimidazol-1-yl.

R4 in formula (I) is selected from the group comprising or consisting of hydrogen; C1-12 alkyl optionally substituted by halogen, C1-4 alkoxy, C1-4 alkylthio, azido, nitrooxy or an aryl; C2-12 alkenyl optionally substituted by halogen; C2-12 alkynyl optionally substituted by halogen; azido; alkoxycarbonylamino; arylsulfonyloxy; a substituted or unsubstituted aryl; or a 3-8 membered substituted or unsubstituted heterocycle;

In a specific instance R4 is hydrogen; or R4 is C1-12 alkyl or a C1-6 alkyl, optionally substituted by halogen, C1-4 alkoxy, C1-4 alkylthio, azido or nitrooxy; or R4 is C2-12 alkenyl or a C1-6 alkenyl optionally substituted by halogen; or R4 is C2-12 alkynyl or a C1-6 alkynyl optionally substituted by halogen; or R4 is alkoxycarbonylamino.
R5 is hydrogen;
Alternatively R4 may form together with R5 and the 2-oxo-1-pyrrolidine ring a 1,3-dihydro-2H-indol-2-one ring of the following structure:

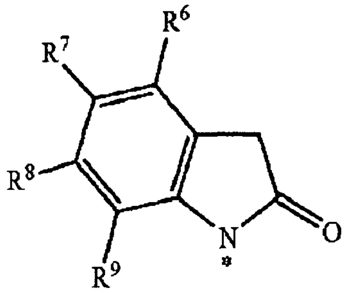

The asterisk * indicates the point of attachment of the substituents;
R6 is hydrogen or halogen.

R7 in formula (I) is selected from the group comprising or consisting of hydrogen; nitro; halogen; heterocycle; amino; aryl; C1-12 alkyl optionally substituted by at least one halogen; or C1-12 alkoxy optionally substituted by at least one halogen.

R8 in formula (I) is selected from the group comprising or consisting of hydrogen, C1-12 alkyl optionally substituted by halogen, or halogen.

R9 in formula (I) is selected from the group comprising or consisting of hydrogen, C1-12 alkyl optionally substituted by halogen, or halogen.

A further aspect of the present disclosure consists in compounds of formula (I) wherein
R1 and R2 are both hydrogen.

R3 is:

(a) a substituted or unsubstituted heterocycle linked to the rest of the molecule via one of its C atoms selected from the group consisting of:

1 H-benzimidazol-6-yl;
1H-benzimidazol-7-yl;
imidazo[1,2-a]pyridin-3-yl;
imidazo[1,2-a]pyrimidin-3-yl;
imidazo[1,2-b][1,2,4]triazin-7-yl;
imidazo[1,2-b]pyridazin-3-yl;
5,6,7,8-tetrahydroimidazo[1,2-b]pyridazin-3-yl;
imidazo[2,1-b][1,3,4]thiadiazol-5-yl;
imidazo[2,1-b][1,3]thiazol-5-yl;
3H-imidazo[4,5-b]pyridin-7-yl;
1H-imidazol-4-yl;
1H-imidazol-5-yl;
1H-indol-2-yl;
1H-indol-3-yl;
1H-indol-4-yl;
1H-indol-7-yl;
isoxazol-4-yl;
1H-pyrazol-4-yl;
1H-pyrazol-5-yl;
1H-pyrazolo[1,5-a]pyrimidin-3-yl;
1H-pyrazolo[3,4-b]pyridin-3-yl;
pyridazin-4-yl;
pyridin-2-yl;
pyridin-3-yl;
pyridin-4-yl;
1H-pyrrolo[2,3-b]pyridin-3-yl;
1H-pyrrolo[2,3-b]pyridin-4-yl;
1H-pyrrolo[2,3-b]pyridin-5-yl;
1H-pyrrolo[2,3-c]pyridin-2-yl;
1H-pyrrolo[2,3-c]pyridin-3-yl;
1 H-pyrrolo[3,2-b]pyridin-3-yl;

1 H-pyrrolo[3,2-c]pyridin-2-yl;
1 H-pyrrolo [3,2-c]pyridin-3-yl;
1,3,4-thiadiazol-2-yl;
1,3-thiazol-5-yl;
[1,2,4]triazolo[4,3-b]pyridazin-7-yl;
[1,2,4]triazolo[4,3-b]pyridazin-8-yl;
indolizin-3-yl.

Alternatively R3 is:

(b) a substituted or unsubstituted heterocycle linked to the rest of the molecule via one of its N atoms selected from the group consisting of:

1H-1,2,3-benzotriazol-1-yl;

1H-imidazo[4,5-b]pyridin-1-yl;

3H-imidazo[4,5-b]pyridin-3-yl;

7H-imidazo[4,5-c]pyridazin-7-yl;

1H-indol-1-yl;

2,3-dihydro-1H-indol-1-yl;

9H-purin-9-yl;

1H-pyrazolo[3,4-b]pyridin-1-yl;

2H-pyrazolo[3,4-b]pyridin-2-yl;

1H-pyrrolo[2,3-b]pyridin-1-yl;

1H-pyrrolo[3,2-b]pyridin-1-yl;

3,4-dihydroquinolin-1(2H)-yl;

8H-isothiazolo[5,4-b]indol-8-yl;

1H-1,2,4-triazol-1-yl;

1H-pyrrol-1-yl;

2-chloro-1H-benzimidazol-1-yl.

R4 in formula (I) is selected from the group comprising or consisting of hydrogen; C1-12 alkyl optionally substituted by halogen or C1-4 alkoxy; C2-12 alkenyl optionally substituted by halogen; C2-12 alkynyl optionally substituted by halogen.

In a further specific instance R4 is n-propyl, 2,2,2-trifluoroethyl, 2-chloro-2,2-difluoroethyl, 2 bromo-2,2-difluoroethyl, 2,2-difluorovinyl.

In another specific instance R4 is phenyl, 2,3,5-trifluorophenyl or 3-chloro-4-fluorophenyl.

R5 is hydrogen;

A further instance of the present disclosure consists in compounds of formula (I) wherein R4 forms together with R5a 1,3-dihydro-2H-indol-2-one ring

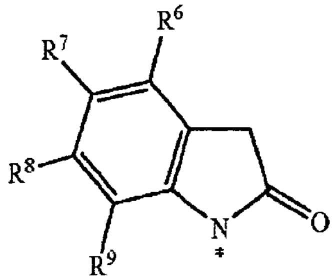

The asterisk * indicates the point of attachment of the heteroaryl alkylene substituent, and wherein
R6 is hydrogen;
R7 is chlorine;
R8 is hydrogen;
R9 is hydrogen.

A further instance of the present disclosure consists in compounds of formula (I) wherein R3 is a substituted or unsubstituted heterocycle linked to the rest of the molecule via one of its C atoms and is selected from the group consisting of:

imidazo[1,2-a]pyrimidin-3-yl;

imidazo[1,2-b][1,2,4]triazin-7-yl;

imidazo[1,2-b]pyridazin-3-yl;

5,6,7,8-tetrahydroimidazo[1,2-b]pyridazin-3-yl;

imidazo[2,1-b][1,3,4]thiadiazol-5-yl;

imidazo[2,1-b][1,3]thiazol-5-yl;

3H-imidazo[4,5-b]pyridin-7-yl;

1H-imidazol-4-yl;

1H-imidazol-5-yl;

isoxazol-4-yl;

1 H-pyrazol-4-yl;

1H-pyrazol-5-yl;

1H-pyrazolo[1,5-a]pyrimidin-3-yl;

1 H-pyrazolo[3,4-b]pyridin-3-yl;

pyridin-3-yl;

1H-pyrrolo[2,3-b]pyridin-3-yl;

1 H-pyrrolo[2,3-b]pyridin-4-yl;

1H-pyrrolo[2,3-b]pyridin-5-yl;

1 H-pyrrolo[2,3-c]pyridin-2-yl;

1 H-pyrrolo[2,3-c]pyridin-3-yl;

1,3-thiazol-5-yl;

[1,2,4]triazolo[4,3-b]pyridazin-8-yl;

indolizin-3-yl.

In a further specific instance R3 is a heterocycle linked to the rest of the molecule via one of its C atoms and is selected from the group consisting of:

imidazo[1,2-b]pyridazin-3-yl;

imidazo[2,1-b][1,3,4]thiadiazol-5-yl;

imidazo[2,1-b][1,3]thiazol-5-yl;

3H-imidazo[4,5-b]pyridin-7-yl;

1H-imidazol-4-yl;

1H-imidazol-5-yl;

1H-pyrazol-4-yl;

1H-pyrazolo[1,5-a]pyrimidin-3-yl;

pyridin-3-yl;

1H-pyrrolo[2,3-b]pyridin-3-yl;

1H-pyrrolo[2,3-b]pyridin-4-yl;

1,3-thiazol-5-yl;

Said heterocycles are optionally substituted by e.g. a methyl, n-propyl, trifluoromethyl, cyclopropyl, bromine, chlorine, fluorine, iodine, methoxy, ethoxy, propoxy, isopropoxy, cyclopropyloxy, cyclopropylmethoxy, cyclobutylmethoxy, amino, methylamino, cyclopropylamino, cyclobutylamino, 1-pyrrolidinyl, cyano, phenyl, benzyl or 3-thienyl.

In a further specific instance R3 is a heterocycle linked to the rest of the molecule via one of its C atoms and is selected from the group consisting of: 6-chloro-2-cyclopropylimidazo[1,2-b]pyridazin-3-yl, 6-(cyclopropyloxy)-2-(trifluoromethyl)imidazo[1,2-b]pyridazin-3-yl, 6-propoxy-2-(trifluoromethyl)imidazo[1,2-b]pyridazin-3-yl, 6-chloroimidazo[2,1-b][1,3]thiazol-5-yl, 2,6-dichloroimidazo[2,1-b][1,3]thiazol-5-yl, 5-chloro-1H-imidazol-4-yl, 5-bromo-1H-imidazol-4-yl, 4-bromo-1H-imidazol-5-yl, 4-chloro-1H-imidazol-5-yl, 1H-imidazol-5-yl, 1-methyl-1H-imidazol-5-yl, 4-chloro-1-methyl-1H-imidazol-5-yl, 1H-pyrazol-4-yl, 1H-pyrrolo[2,3-b]pyridin-3-yl.

A further instance of the present disclosure consists in compounds of formula (I) wherein R3 is a heterocycle linked to the rest of the molecule via one of its C atoms and is a substituted or unsubstituted imidazo[1,2-a]pyridin-3-yl.

Said imidazo[1,2-a]pyridin-3-yl is optionally substituted by e.g. a methyl, cyclopropyl, bromine, chlorine, fluorine, iodine.

In a further specific instance R3 is a heterocycle linked to the rest of the molecule via one of its C atoms and is selected from the group consisting of: imidazo[1,2-a]pyridin-3-yl, 6-methylimidazo[1,2-a]pyridin-3-yl, 2-chloroimidazo[1,2-a]pyridin-3-yl.

A further instance of the present disclosure consists in compounds of formula (I) wherein R3 is a substituted or unsubstituted heterocycle linked to the rest of the molecule via one of its N atoms and is selected from the group consisting of:

3H-imidazo[4,5-b]pyridin-3-yl;

1H-indol-1-yl;

1H-pyrrolo[2,3-b]pyridin-1-yl;

1H-pyrrolo[3,2-b]pyridin-1-yl;

1H-pyrrol-1-yl;

2-chloro-1H-benzimidazol-1-yl.

A specific further instance of the present disclosure consists in compounds of formula (I) wherein R3 is a heterocycle linked to the rest of the molecule via one of its N atoms and is selected from the group consisting of:

3H-imidazo[4,5-b]pyridin-3-yl;

1H-pyrrolo[3,2-b]pyridin-1-yl;

1H-pyrrol-1-yl;

2-chloro-1H-benzimidazol-1-yl;

Said heterocycles may optionally be substituted by trifluoromethyl, cyclopropyl, bromine, chlorine, fluorine, methoxy or cyano.

In a further specific instance R3 is a heterocycle linked to the rest of the molecule via one of its C atoms and is selected from the group consisting of 6-bromo-2-chloro-3H-imidazo[4,5-b]pyridin-3-yl, 6-bromo-2-cyclopropyl-3H-imidazo[4,5-b]pyridin-3-yl, 1H-pyrrolo[3,2-b]pyridin-1-yl, 2,5-dichloro-1H-pyrrol-1-yl, 2-chloro-5-methoxy-1H-benzimidazol-1-yl, 5-bromo-2-chloro-1H-benzimidazol-1-yl or 2,5-dichloro-1H-benzimidazol-1-yl.

A further instance of the present disclosure consists in compounds of formula (I) wherein R1, R2 and R5 are hydrogen.

R4 is a C1-6 alkyl optionally substituted by halogen, a C2-6 alkenyl optionally substituted by halogen or C2-12 alkynyl optionally substituted by halogen.

R3 is selected from the group consisting of;

imidazo[1,2-b]pyridazin-3-yl;

imidazo[2,1-b][1,3,4]thiadiazol-5-yl;

imidazo[2,1-b][1,3]thiazol-5-yl;

3H-imidazo[4,5-b]pyridin-7-yl;

1H-imidazol-4-yl;

1H-imidazol-5-yl;

1H-pyrazol-4-yl;

1H-pyrazolo[1,5-a]pyrimidin-3-yl;

pyridin-3-yl;

1H-pyrrolo[2,3-b]pyridin-3-yl;

1H-pyrrolo[2,3-b]pyridin-4-yl;

1,3-thiazol-5-yl;

and optionally substituted by methyl, n-propyl, trifluoromethyl, cyclopropyl, bromine, chlorine, fluorine, iodine, methoxy, ethoxy, propoxy, isopropoxy, cyclopropyloxy, cyclopropylmethoxy, cyclobutylmethoxy, amino, methylamino, cyclopropylamino, cyclobutylamino, 1-pyrrolidinyl, cyano, phenyl, benzyl or 3-thienyl.

A further instance of the present disclosure consists in compounds of formula (I) wherein R1, R2 and R5 are hydrogen.

R4 is a C1-6 alkyl optionally substituted by halogen, a C2-6 alkenyl optionally substituted by halogen or C2-12 alkynyl optionally substituted by halogen.

R3 is selected from the group consisting of

3H-imidazo[4,5-b]pyridin-3-yl;

1H pyrrolo[3,2-b]pyridin-1-yl;

1H-pyrrol-1-yl;

2-chloro-1H-benzimidazol-1-yl;

optionally substituted by trifluoromethyl, cyclopropyl, bromine, chlorine, fluorine, methoxy or cyano.

A further instance of the disclosure consists in compounds of formula (I), their diastereomers and mixtures, or a pharmaceutically acceptable salt thereof.

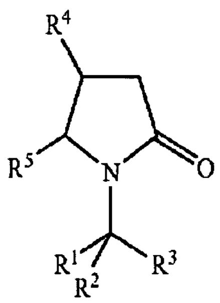

(I)

R1, R2 and R5 are hydrogen.

R3 is a substituted or unsubstituted heterocycle linked to the rest of the molecule via one of its C atoms, said heterocycle is selected from the group consisting of:

1 H-benzimidazol-6-yl;
1 H-benzimidazol-7-yl;
imidazo[1,2-a]pyridin-3-yl;
imidazo[1,2-a]pyrimidin-3-yl;
imidazo[11,2-b][1,2,4]triazin-7-yl;
imidazo[1,2-b]pyridazin-3-yl;
5,6,7,8-tetrahydroimidazo[1,2-b]pyridazin-3-yl;
imidazo[2,1-b][1,3,4]thiadiazol-5-yl;
imidazo[2,1-b][1,3]thiazol-5-yl;
3H-imidazo[4,5-b]pyridin-7-yl;
1H-imidazol-4-yl;
1H-imidazol-5-yl;
1H-indol-2-yl;
1H-indol-3-yl;
1H-indol-4-yl;
1H-indol-7-yl;
isoxazol-4-yl;
1H-pyrazol-4-yl;
1H-pyrazol-5-yl;
1H-pyrazolo[1,5-a]pyrimidin-3-yl;
1H-pyrazolo[3,4-b]pyridin-3-yl;
pyridazin-4-yl;
pyridin-2-yl;

pyridin-3-yl;
pyridin-4-yl;
1H-pyrrolo[2,3-b]pyridin-3-yl;
1 H-pyrrolo[2,3-b]pyridin-4-yl;
1H-pyrrolo[2,3-b]pyridin-5-yl;
1H-pyrrolo[2,3-c]pyridin-2-yl;
1 H-pyrrolo[2,3-c]pyridin-3-yl;
1 H-pyrro lo[3,2-b]pyridin-3-yl;
1 H-pyrrolo[3,2-c]pyridin-2-yl;
1H-pyrrolo[3,2-c]pyridin-3-yl;
1,3,4-thiadiazol-2-yl;
1,3-thiazol-5-yl;
[1,2,4]triazolo[4,3-b]pyridazin-7-yl;
[1,2,4]triazolo[4,3-b]pyridazin-8-yl;
indolizin-3-yl;
Particularly preferred are imidazo[1,2-a]pyridin-3-yl; imidazo[1,2-a]pyrimidin-3-yl; imidazo[1,2-b]pyridazin-3-yl; 1H-imidazol-4-yl; 1H-imidazol-5-yl;
R4 is a substituted or unsubstituted phenyl moiety;
A further instance of the present disclosure consists in compounds of formula (I) wherein R1 is hydrogen or C1-12 alkyl;
R2 is hydrogen;
R3 is an aromatic 5-membered heterocycle linked to the rest of the molecule via one of its C atoms;
R4 is hydrogen, C1-12 alkyl or aryl;
R5 is hydrogen;

Alternatively, R4 can form together with R5 and the 2-oxo-1-pyrrolidine ring the following 1,3-dihydro-2H-indol-2-one cycle

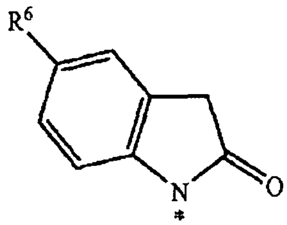

wherein the asterisk * indicates the point of attachment of the substituents;
R6 is hydrogen or halogen;

In this instance R4 may not be hydrogen when R3 is substituted 1H-pyrazol-5-yl. Also this instance does not comprise 5-(2'-oxo-1'-pyrrolidinyl)methyl-1,3,4-tricarbomethoxy-pyrazole which is disclosed in A. Padwa et al J. Org. Chem. 2000, 65, 5223-5232 without any biological activity though.

In this instance wherein R3 is an aromatic 5-membered heterocycle linked to the rest of the molecule via one of its C atoms, specific moieties R3 may be selected from 1,3-thiazol-5-yl, 1H-imidazol-4-yl, 1H-imidazol-5-yl, 1H-pyrazol-4-yl, 1H-pyrazol-5-yl, 2-oxo-2,3-dihydro-1,3-thiazol-5-yl, each of them being optionally substituted by 1 to 3 substituents independently selected from methyl, chlorine, bromine, amino, methylamino, dimethylamino, (2-oxo-4-propyl-pyrrolidin-1-yl)methyl, 1-pyrrolidinyl, amido, cyano, methoxy, phenyl, 4-methylphenylsulfonyl, benzyl or 2-(benzylamino)-2-oxoethyl.

In this instance, more specific moieties R3 are selected from 2-(methylamino)-1,3-thiazol-5-yl; 2-pyrrolidin-1-yl-1,3-thiazol-5-yl; 5-bromo-1H-imidazol-4-yl; 5-chloro-1H-imidazol-4-yl; 1H-imidazol-5-yl; 1-methyl-1H-imidazol-5-yl; 4-bromo-1-methyl-1H-imidazol-5-yl; 4-chloro-1H-imidazol-5-yl; 4-chloro-1-methyl-1H-imidazol-5-yl; 4-cyano-1-methyl-1H-imidazol-5-yl; 1H-pyrazol-4-yl; 3,5-dimethyl-1H-pyrazol-4-yl; 3-methyl-1H-pyrazol-4-yl.

In this instance, most specific moieties R3 are selected from 5-bromo-1H-imidazol-4-yl; 5-chloro-1H-imidazol-4-yl; 1H-imidazol-5-yl; 4-bromo-1-methyl-1H-imidazol-5-yl; 4-chloro-1-methyl-1H-imidazol-5-yl; 1H-pyrazol-4-yl.

Still in this instance, a specific moiety R1 is selected from hydrogen or ethyl.

Still in this instance, a specific moiety R4 is selected from hydrogen, n-propyl, 2,3,5-trifluorophenyl or phenyl.

A further instance of the present disclosure consists in compounds having the specific formula (Ia).

(Ia)

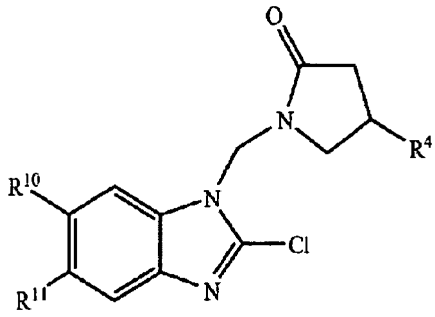

In formula (Ia) the substituent R10 is hydrogen; halogen; C14 alkyl optionally substituted by at least one halogen; C1-4 alkoxy; methoxycarbonyl; nitro; amino; alkylamino; amido; or alkanoyl-amino. Preferably R10 is hydrogen.

R11 is hydrogen; halogen; C1-4 alkyl optionally substituted by at least one halogen; C1-4 alkoxy; methoxycarbonyl; nitro; amino; alkylamino; amido; or alkanoylamino. Preferably R11 is hydrogen.

R4 is C1-4 alkyl optionally substituted by at least one halogen; or C2-4 alkenyl optionally substituted by at least one halogen. Preferably R4 is n-propyl.

Still in this aspect of the disclosure a specific instance relates to an instance wherein R10 is selected from hydrogen; methyl; fluorine; chlorine; bromine; methoxy; methoxycarbonyl; nitro; or trifluoromethyl, while R11 is selected from hydrogen; methyl; fluorine; chlorine; bromine; methoxy; methoxycarbonyl; nitro; or trifluoromethyl; and R3 is n-propyl.

Specific compounds of the present disclosure are those selected from the group consisting of:

1-[(1-methyl-1H-benzimidazol-6-yl)methyl]-4-propylpyrrolidin-2-one;

1-(1H-benzimidazol-7-ylmethyl)-4-propylpyrrolidin-2-one;

1-(imidazo[1,2-a]pyridin-3-ylmethyl)-4-propylpyrrolidin-2-one;

1-{[6-chloro-2-(4-methylphenyl)imidazo[1,2-a]pyridin-3-yl]methyl} -4-propylpyrrolidin-2-one;

1-{[2-(4-chlorophenyl)-6-methylimidazo[1,2-a]pyridin-3-yl]methyl} -4-propylpyrrolidin-2-one;

1-[(5-methylimidazo[1,2-a]pyridin-3-yl)methyl]-4-phenylpyrrolidin-2-one;

1-(imidazo[1,2-a]pyridin-3-ylmethyl)-4-phenylpyrrolidin-2-one;

1-[(6-methylimidazo[1,2-a]pyridin-3-yl)methyl]-4-propylpyrrolidin-2-one;

1-[(6-bromoimidazo[1,2-a]pyridin-3-yl)methyl]-4-propylpyrrolidin-2-one;

1-[(8-methylimidazo[1,2-a]pyridin-3-yl)methyl]-4-propylpyrrolidin-2-one;

1-[(6-iodoimidazo[1,2-a]pyridin-3-yl)methyl]-4-propylpyrrolidin-2-one;

1-{[8-chloro-6-(trifluoromethyl)imidazo[1,2-a]pyridin-3-yl]methyl} -4-propylpyrrolidin-2-one;

1-[(7-methylimidazo[1,2-a]pyridin-3-yl)methyl]-4-propylpyrrolidin-2-one;

1-[(6,8-dibromoimidazo[1,2-a]pyridin-3-yl)methyl]-4-propylpyrrolidin-2-one;

1-[(6,8-dichloroimidazo[1,2-a]pyridin-3-yl)methyl]-4-propylpyrrolidin-2-one;

1-[(6-chloroimidazo[1,2-a]pyridin-3-yl)methyl]-4-propylpyrrolidin-2-one;

1-[(2-chloroimidazo[1,2-a]pyridin-3-yl)methyl]-4-propylpyrrolidin-2-one;

1-[(2-cyclopropyl-6-fluoroimidazo[1,2-a]pyridin-3-yl)methyl]-4-(2,2-difluorovinyl)pyrrolidin-2-one;

1-[(6-chloro-2-cyclopropylimidazo[1,2-a]pyridin-3-yl)methyl]-4-(2,2-difluorovinyl)pyrrolidin-2-one;

1-(imidazo[1,2-a]pyrimidin-3-ylmethyl)-4-propylpyrrolidin-2-one;

1-{[2-(4-chlorophenyl)imidazo[1,2-a]pyrimidin-3-yl]methyl}-4-propyl pyrrolidin-2-one;

1-(imidazo[1,2-a]pyrimidin-3-ylmethyl)-4-phenylpyrrolidin-2-one;

1-[(6-chloroimidazo[1,2-a]pyrimidin-3-yl)methyl]-4-propylpyrrolidin-2-one;

1-{[6-chloro-2-(trifluoromethyl)imidazo[1,2-a]pyrimidin-3-yl]methyl} -4-propylpyrrolidin-2-one;

1-[(6-phenylimidazo[1,2-b][1,2,4]triazin-7-yl)methyl]-4-propylpyrrolidin-2-one;

1-{[6-chloro-2-(4-methylphenyl)imidazo[1,2-b]pyridazin-3-yl]methyl} -4-propylpyrrolidin-2-one;

1-{[6-chloro-2-(4-chlorophenyl)imidazo[1,2-b]pyridazin-3-yl]methyl} -4-propylpyrrolidin-2-one;

1-[(6-chloroimidazo[1,2-b]pyridazin-3-yl)methyl]-4-propylpyrrolidin-2-one;

1-[(6-chloroimidazo[1,2-b]pyridazin-3-yl)methyl]-4-phenylpyrrolidin-2-one;

1-{[6-chloro-2-(trifluoromethyl)imidazo[1,2-b]pyridazin-3-yl]methyl} -4-propylpyrrolidin-2-one;

1-{[6-chloro-2-(trifluoromethyl)imidazo[1,2-b]pyridazin-3-yl]methyl}-4-(2,3,5-trifluorophenyl)pyrrolidin-2-one;

1-{[6-chloro-2-(trifluoromethyl)imidazo[1,2-b]pyridazin-3-yl]methyl}pyrrolidin-2-one;

1-{[6-chloro-2-(trifluoromethyl)imidazo[1,2-b]pyridazin-3-yl]methyl} -4-(2,2-difluorovinyl)pyrrolidin-2-one;

1-{[6-chloro-2-(trifluoromethyl)imidazo[1,2-b]pyridazin-3-yl]methyl}-4-phenylpyrrolidin-2-one;

5-chloro-1-{[6-chloro-2-(trifluoromethyl)imidazo[1,2-b]pyridazin-3-yl]methyl}-1,3-dihydro-2H-indol-2-one;

1-{[6-methoxy-2-(trifluoromethyl)imidazo[1,2-b]pyridazin-3-yl]methyl-4-propylpyrrolidin-2-one;

1-[(6-chloro-2-cyclopropylimidazo[1,2-b]pyridazin-3-yl)methyl]-4-propylpyrrolidin-2-one;

1-{[6-isopropoxy-2-(trifluoromethyl)imidazo[1,2-b]pyridazin-3-yl]methyl}-4-propylpyrrolidin-2-one;

1-{[6-(benzyloxy)-2-(trifluoromethyl)imidazo[1,2-b]pyridazin-3-yl]methyl} -4-propylpyrrolidin-2-one;

1-{[6-cyclopropyl-2-(trifluoromethyl)imidazo[1,2-b]pyridazin-3-yl]methyl}-4-propylpyrrolidin-2-one;

1-{[6-(dimethylamino)-2-(trifluoromethyl)imidazo[1,2-b]pyridazin-3-yl]methyl}-4-propylpyrrolidin-2-one;

4-(2,2-difluorovinyl)-1-{[6-methoxy-2-(trifluoromethyl)imidazo[1,2-b]pyridazin-3-yl]methyl}pyrrolidin-2-one;

4-(2-chloro-2,2-difluoroethyl)-1-{[6-chloro-2-(trifluoromethyl)imidazo[ 1,2-b]pyridazin-3-yl]methyl}pyrrolidin-2-one;

1-{[6-(methylamino)-2-(trifluoromethyl)imidazo[1,2-b]pyridazin-3-yl]methyl} -4-propylpyrrolidin-2-one;

1-{[6-hydroxy-2-(trifluoromethyl)imidazo[1,2-b]pyridazin-3-yl]methyl} -4-propylpyrrolidin-2-one;

1-{[6-(methylthio)-2-(trifluoromethyl)imidazo[1,2-b]pyridazin-3-yl]methyl}-4-propylpyrrolidin-2-one;

4-(2-bromo-2,2-difluoroethyl)-1-{[6-chloro-2-(trifluoromethyl)imidazo[1,2-b]pyridazin-3-yl]methyl}pyrrolidin-2-one;

1-{[6-(methylsulfonyl)-2-(trifluoromethyl)imidazo[1,2-b]pyridazin-3-yl]methyl}-4-propylpyrrolidin-2-one;

1-{[6-(methylsulfinyl)-2-(trifluoromethyl)imidazo[1,2-b]pyridazin-3-yl]methyl}-4-propylpyrrolidin-2-one;

1-{[6-chloro-2-(trifluoromethyl)imidazo[1,2-b]pyridazin-3-yl]methyl}-4-(2,2,2-trifluoroethyl)pyrrolidin-2-one;

1-[(6-chloro-2-cyclobutylimidazo[1,2-b]pyridazin-3-yl)methyl]-4-propylpyrrolidin-2-one;

1-{[6-chloro-2-(4-methylphenyl)imidazo1,2-b]pyridazin-3-yl]methyl} -4-(2,2-difluorovinyl)pyrrolidin-2-one;

1-{[6-amino-2-(trifluoromethyl)imidazo[1,2-b]pyridazin-3-yl]methyl}-4-propylpyrrolidin-2-one;

1-{[6-(ethylamino)-2-(trifluoromethyl)imidazo[1,2-b]pyridazin-3-yl]methyl}-4-propylpyrrolidin-2-one;

4-propyl-1-[6-(propylamino)-2-(trifluoromethyl)imidazo[1,2-b]pyridazin-3-yl]methyl}pyrrolidin-2-one;

4-(2-bromo-2,2-difluoroethyl)-1-{[6-(propylamino)-2-(trifluoromethyl)imidazo[1,2-b]pyridazin-3-yl]methyl}pyrrolidin-2-one;

4-(2,2-difluorovinyl)-1-{[6-(propylamino)-2-(trifluoromethyl)imidazo[ 1,2-b]pyridazin-3-yl]methyl}pyrrolidin-2-one;

4-(2,2-difluorovinyl)-1-{[6-methoxy-2-(4-methylphenyl)imidazo[1,2-b]pyridazin-3-yl]methyl}pyrrolidin-2-one;

4-propyl-1-{[6-pyrrolidin-1-yl-2-(trifluoromethyl)imidazo[1,2-b]pyridazin-3-yl]methyl}pyrrolidin-2-one;

4-(2-bromo-2,2-difluoroethyl)-1-{[6-methoxy-2-(trifluoromethyl)imidazo[1,2-b]pyridazin-3-yl]methyl}pyrrolidin-2-one;

1-{[6-(cyclopropylamino)-2-(trifluoromethyl)imidazo[1,2-b]pyridazin-3-yl]methyl}-4-(2,2-difluorovinyl)pyrrolidin-2-one;

1-[(6-chloro-2-cyclopropylimidazo[1,2-b]pyridazin-3-yl)methyl]-4-(2,2-difluorovinyl)pyrrolidin-2-one;

4-(2,2-difluorovinyl)-1-{[6-(isopropylamino)-2-(trifluoromethyl)imidazo[1,2-b]pyridazin-3-yl]methyl}pyrrolidin-2-one;

4-(2,2-difluorovinyl)-1-{[2-(trifluoromethyl)imidazo[1,2-b]pyridazin-3-yl]methyl}pyrrolidin-2-one;

1-{[2-cyclopropyl-6-(propylamino)imidazo[1,2-b]pyridazin-3-yl]methyl -4-(2,2-difluorovinyl)pyrrolidin-2-one;

1-({2-cyclopropyl-6-[(2-fluoroethyl)amino]imidazo[1,2-b]pyridazin-3-yl}methyl)-4-(2,2-difluorovinyl)pyrrolidin-2-one;

1-({2-cyclopropyl-6-[(2,2-difluoroethyl)amino]imidazo[1,2-b]pyridazin-3-yl}methyl)-4-(2,2-difluorovinyl)pyrrolidin-2-one;

1-({2-cyclopropyl-6-[(2,2,2-trifluoroethyl)amino]imidazo[1,2-b]pyridazin-3-yl}methyl)-4-(2,2-difluorovinyl)pyrrolidin-2-one;

4-(2,2-difluoroethyl)-1-{[2-(trifluoromethyl)imidazo[1,2-b]pyridazin-3-yl]methyl}pyrrolidin-2-one;

1-{[2-cyclopropyl-6-(cyclopropylamino)imidazo[1,2-b]pyridazin-3-yl]methyl}-4-(2,2-difluorovinyl)pyrrolidin-2-one;

1-[(6-chloro-2-cyclobutylimidazo[1,2-b]pyridazin-3-yl)methyl]-4-(2,2-difluorovinyl)pyrrolidin-2-one;

1-[(6-chloro-2-cyclopropylimidazo[1,2-b]pyridazin-3-yl)methyl]-4-(3-chloro-4-fluorophenyl)pyrrolidin-2-one;

1-{[6-(butylamino)-2-(trifluoromethyl)imidazo[1,2-b]pyridazin-3-yl]methyl -4-(2,2-difluorovinyl)pyrrolidin-2-one;

1-{[6-(cyclobutylamino)-2-(trifluoromethyl)imidazo[1,2-b]pyridazin-3-yl]methyl}-4-(2,2-difluorovinyl)pyrrolidin-2-one;

1-[(2-cyclopropyl-6-methoxyimidazo[1,2-b]pyridazin-3-yl)methyl]-4-(2,2-difluorovinyl)pyrrolidin-2-one;

4-(2,2-difluorovinyl)-1-{[6-ethoxy-2-(trifluoromethyl)imidazo[1,2-b]pyridazin-3-yl]methyl}pyrrolidin-2-one;

4-(2,2-difluorovinyl)-1-{[6-isopropoxy-2-(trifluoromethyl)imidazo[1,2-b]pyridazin-3-yl]methyl}pyrrolidin-2-one;

1-{[6-(cyclopropylmethoxy)-2-(trifluoromethyl)imidazo[1,2-b]pyridazin-3-yl]methyl}-4-(2,2-difluorovinyl)pyrrolidin-2-one;

1-{[6-(cyclobutylmethoxy)-2-(trifluoromethyl)imidazo[1,2-b]pyridazin-3-yl]methyl}-4-(2,2-difluorovinyl)pyrrolidin-2-one;

1-{[6-(cyclopropyloxy)-2-(trifluoromethyl)imidazo[1,2-b]pyridazin-3-yl]methyl}-4-(2,2-difluorovinyl)pyrrolidin-2-one;

4-(2,2-difluorovinyl)-1-{[6-propoxy-2-(trifluoromethyl)imidazo[1,2-b]pyridazin-3-yl]methyl}pyrrolidin-2-one;

3-{[4-(2,2-difluorovinyl)-2-oxopyrrolidin-1-yl]methyl} -2-(trifluoromethyl)imidazo[1,2-b]pyridazine-6-carbonitrile;

4-(2,2-difluorovinyl)-1-{[6-thien-3-yl-2-(trifluoromethyl)imidazo[1,2-b]pyridazin-3-yl]methyl}pyrrolidin-2-one;

4-(2,2-difluorovinyl)-1-{[6-phenyl-2-(trifluoromethyl)imidazo[1,2-b]pyridazin-3-yl]methyl}pyrrolidin-2-one;

4-(2,2-difluorovinyl)-1-{[6-methyl-2-(trifluoromethyl)imidazo[1,2-b]pyridazin-3-yl]ethyl}pyrrolidin-2-one;

4-(2, 2-difluorovinyl)-1-{[6-pyridin-3-yl-2-(trifluoromethyl)imidazo[1,2-b]pyridazin-3-yl]methyl}pyrrolidin-2-one;

4-propyl-1-{2-(trifluoromethyl)-5,6,7,8-tetrahydroimidazo[1,2-b]pyridazin-3-yl]methyl}pyrrolidin-2-one;

1-[(6-methylimidazo[2,1-b][1,3,4]thiadiazol-5-yl)methyl]-4-propylpyrrolidin-2-one;

1-{[6-(4-methylphenyl)imidazo[2,1-b][1,3,4]thiadiazol-5-yl]methyl}-4-propylpyrrolidin-2-one;

1-[(2-cyclopropyl-6-phenylimidazo[2,1-b][1,3,4]thiadiazol-5-yl)methyl]-4-propylpyrrolidin-2-one;

1-[(6-methylimidazo[2,1-b][1,3]thiazol-5-yl)methyl]-4-propylpyrrolidin-2-one;

1-[(6-chloroimidazo[2,1-b][1,3]thiazol-5-yl)methyl]-4-propylpyrrolidin-2-one;

1-[(2,6-dichloroimidazo[2,1-b][1,3]thiazol-5-yl)methyl]-4-propylpyrrolidin-2-one;

1-(3H-imidazo[4,5-b]pyridin-7-ylmethyl)-4-propylpyrrolidin-2-one;

1-(3H-imidazo[4,5-b]pyridin-7-ylmethyl)-4-phenylpyrrolidin-2-one;

4-phenyl-1-[(5-phenyl-3H-imidazo[4,5-b]pyridin-7-yl)methyl]pyrrolidin-2-one;

4-phenyl-1-{[5-(trifluoromethyl)-3H-imidazo[4,5-b]pyridin-7-yl]methyl}pyrrolidin-2-one;

1-[(6-bromo-3H-imidazo[4,5-b]pyridin-7-yl)methyl]-4-propylpyrrolidin-2-one;

1-[(2-phenyl-3H-imidazo[4,5-b]pyridin-7-yl)methyl]-4-propylpyrrolidin-2-one;

1-[(5-methyl-3H-imidazo[4,5-b]pyridin-7-yl)methyl]-4-propylpyrrolidin-2-one;

1-[(2-methyl-3H-imidazo[4,5-b]pyridin-7-yl)methyl]-4-propylpyrrolidin-2-one;

4-propyl-1-{[5-(trifluoromethyl)-3H-imidazo[4,5-b]pyridin-7-yl]methyl}pyrrolidin-2-one;

1-[(6-methyl-3H-imidazo[4,5-b]pyridin-7-yl)methyl]-4-propylpyrrolidin-2-one;

1-[(6-phenyl-3H-imidazo[4,5-b]pyridin-7-yl)methyl]-4-propylpyrrolidin-2-one;

1-[1-(1H-imidazol-4-yl)propyl]pyrrolidin-2-one;

1-[(5-methyl-1H-imidazol-4-yl)methyl]pyrrolidin-2-one;

1-[(2-methyl-1H-imidazol-4-yl)methyl]pyrrolidin-2-one;

1-(1 H-imidazol-4-ylmethyl)-4-propylpyrrolidin-2-one;

1-({1-[(2-oxo-4-propylpyrrolidin-1-yl)methyl]-1H-imidazol-4-yl}methyl)-4-propylpyrrolidin-2-one;

1-[(5-chloro-1H-imidazol-4-yl)methyl]-4-(2,3,5-trifluorophenyl)pyrrolidin-2-one;

1-[(5-bromo-1H-imidazol-4-yl)methyl]-4-(2,3,5-trifluorophenyl)pyrrolidin-2-one;

1-[(5-bromo-1H-imidazol-4-yl)methyl]-5-chloro-1,3-dihydro-2H-indol-2-one;

1-(H-imidazol-5-ylmethyl)pyrrolidin-2-one;

1-[(-methyl-1H-imidazol-5-yl)methyl]pyrrolidin-2-one;

1-methyl-5-[(2-oxopyrrolidin-1-yl)methyl]-1H-imidazole-4-carbomtrile;

1-(1H-imidazol-5-ylmethyl)-4-phenylpyrrolidin-2-one;

1-[(-methyl-1H-imidazol-5-yl)methyl]-4-phenylpyrrolidin-2-one;

1-[(4-methoxy-1-methyl-1H-imidazol-5-yl)methyl]pyrrolidin-2-one;

1-[(1-methyl-1H-imidazol-5-yl)methyl]-4-propylpyrrolidin-2-one;

1-methyl-5-[(2-oxo-4-propylpyrrolidin-1-yl)methyl]-1H-imidazole-4-carbonitrile;

1-methyl-5-[(2-oxo-4-propylpyrrolidin-1-yl)methyl]-1 H-imidazole-4-carboxamide;

N-benzyl-2-{5-[(2-oxo-4-propylpyrrolidin-1-yl)methyl]-1H-imidazol-1-yl}acetamide;

1-methyl-5-[(2-oxo-4-propylpyrrolidin-1-yl)methyl]-1 H-imidazole-2-carbonitrile;

1-[(4-chloro-1H-imidazol-5-yl)methyl]-4-propylpyrrolidin-2-one;

1-methyl-5-{[2-oxo-4-(2,3,5-trifluorophenyl)pyrrolidin-1-yl]methyl}-1H-imidazole-4-carbonitrile;

1-[(4-bromo-1-methyl-1H-imidazol-5-yl)methyl]-4-propylpyrrolidin-2-one;

1-[(2,4-dichloro-1-methyl-1H-imidazol-5-yl)methyl]-4-propylpyrrolidin-2-one;

benzyl 1-methyl-5-[(2-oxo-4-propylpyrrolidin-1-yl)methyl]-1H-imidazol-2-ylcarbamate;

1-[(4-chloro-1-methyl-1H-imidazol-5-yl)methyl]-4-propylpyrrolidin-2-one;

1-[(2-chloro-1-methyl-1H-imidazol-5-yl)methyl]-4-propylpyrrolidin-2-one;

5-chloro-1-(1H-imidazol-5-ylmethyl)-1,3-dihydro-2H-indol-2-one;

1-[(2,4-dichloro-1H-imidazol-5-yl)methyl]-4-(2,3,5-trifluorophenyl)pyrrolidin-2-one;

1-[(2,4-dichloro-1-methyl-1H-imidazol-5-yl)methyl]-4-(2,3,5-trifluorophenyl)pyrrolidin-2-one;

1-[(2-chloro-1-methyl-1H-imidazol-5-yl)methyl]-4-(2,3,5-trifluorophenyl)pyrrolidin-2-one;

1-[(4-bromo-1-methyl-1H-imidazol-5-yl)methyl]-4-(2,3,5-trifluorophenyl)pyrrolidin-2-one;

5-chloro-1-[(1-methyl-1H-imidazol-5-yl)methyl]-1,3-dihydro-2H-indol-2-one;

1-[(4-chloro-1-methyl-1H-imidazol-5-yl)methyl]-4-(2,3,5-trifluorophenyl)pyrrolidin-2-one;

1-(1H-indol-2-ylmethyl)-4-propylpyrrolidin-2-one;

1-(H-indol-3-ylmethyl)-4-propylpyrrolidin-2-one;

3-[(2-oxo-4-propylpyrrolidin-1-yl)methyl]-1H-indole-5-carbonitrile;

1-[(2-methyl-1H-indol-3-yl)methyl]-4-propylpyrrolidin-2-one;

1-[(7-methoxy-1H-indol-3-yl)methyl]-4-propylpyrrolidin-2-one;

1-[(6-nitro-1H-indol-3-yl)methyl]-4-propylpyrrolidin-2-one;

4-propyl-1-{[6-(trifluoromethyl)-1 H-indol-3-yl]methyl} pyrrolidin-2-one;

1-[(5-nitro-1H-indol-3-yl)methyl]-4-propylpyrrolidin-2-one;

1-[(7-fluoro-1H-indol-3-yl)methyl]-4-propylpyrrolidin-2-one;

1-[(5-chloro-2-methyl-1H-indol-3-yl)methyl]-4-propylpyrrolidin-2-one;

1-[1H-indol-3-yl(phenyl)methyl]-4-propylpyrrolidin-2-one;

1-[1-(H-indol-3-yl)propyl]-4-propylpyrrolidin-2-one;

1-[2-furyl(1H-indol-3-yl)methyl]-4-propylpyrrolidin-2-one;

3-[(2-oxo-4-propylpyrrolidin-1-yl)(phenyl)methyl]-1H-indole-5-carbonitrile;

1-(1H-indol-4-ylmethyl)-4-propylpyrrolidin-2-one;

1-(1H-indol-7-ylmethyl)-4-propylpyrrolidin-2-one;

1-(isoxazol-4-ylmethyl)-4-propylpyrrolidin-2-one;

1-[(1-phenyl-1H-pyrazol-4-yl)methyl]-4-(2,3,5-trifluorophenyl)pyrrolidin-2-one;

1-[(1-methyl-1H-pyrazol-4-yl)methyl]-4-(2,3,5-trifluorophenyl)pyrrolidin-2-one;

1-[(1-benzyl-1H-pyrazol-4-yl)methyl]-4-(2,3,5-trifluorophenyl)pyrrolidin-2-one;

4-(2,3,5-trifluorophenyl)-1-[(1,3,5-trimethyl-1H-pyrazol-4-yl)methyl]pyrrolidin-2-one;

4-phenyl-1-(1H-pyrazol-4-ylmethyl)pyrrolidin-2-one;

1-({1-[(4-methylphenyl)sulfonyl]-1H-pyrazol-4-yl}methyl)-4-(2,3,5-trifluorophenyl)pyrrolidin-2-one;

1-(1H-pyrazol-4-ylmethyl)-4-(2,3,5-trifluorophenyl)pyrrolidin-2-one;

1-[(5-chloro-1,3-dimethyl-1H-pyrazol-4-yl)methyl]-4-(2,3,5-trifluorophenyl)pyrrolidin-2-one;

1-[(1-chloro-1H-pyrazol-4-yl)methyl]-4-(2,3,5-trifluorophenyl)pyrrolidin-2-one;

1-[(3,5-dimethyl-1H-pyrazol-4-yl)methyl]-4-(2,3,5-trifluorophenyl)pyrrolidin-2-one;

1-[(3-methyl-1H-pyrazol-4-yl)methyl]-4-(2,3,5-trifluorophenyl)pyrrolidin-2-one;

1-[(5-amino-1,3-dimethyl-1H-pyrazol-4-yl)methyl]-4-(2,3,5-trifluorophenyl)pyrrolidin-2-one;

1-[(5-amino-1-methyl-1H-pyrazol-4-yl)methyl]-4-propylpyrrolidin-2-one;

(-)-1-(1H-pyrazol-4-ylmethyl)-4-(2,3,5-trifluorophenyl)pyrrolidin-2-one;

(+)-1-(1H-pyrazol-4-ylmethyl)-4-(2,3,5-trifluorophenyl)pyrrolidin-2-one;

1-(1H-pyrazol-4-ylmethyl)-1,3-dihydro-2H-indol-2-one;

5-chloro-1-(1H-pyrazol-4-ylmethyl)-1,3-dihydro-2H-indol-2-one;

5-chloro-1-({1-[(4-methylphenyl)sulfonyl]-1H-pyrazol-4-yl}methyl)-1,3-dihydro-2H-indol-2-one;

1-{[5-chloro-1-methyl-3-(trifluoromethyl)-1H-pyrazol-4-yl]methyl} -4-propylpyrrolidin-2-one;

1-[(5-amino-1H-pyrazol-4-yl)methyl]-4-(2,3,5-trifluorophenyl)pyrrolidin-2-one;

1-[(1-benzyl-5-chloro-1H-pyrazol-4-yl)methyl]-4-propylpyrrolidin-2-one;

1-[(1,3-dimethyl-1H-pyrazol-5-yl)methyl]-4-(2,3,5-trifluorophenyl)pyrrolidin-2-one;

1-(1H-pyrazol-5-ylmethyl)-4-(2,3,5-trifluorophenyl)pyrrolidin-2-one;

1-[(4-bromo-1-methyl-1H-pyrazol-5-yl)methyl]-4-(2,3,5-trifluorophenyl)pyrrolidin-2-one;

1-[(-methyl-1H-pyrazol-5-yl)methyl]-4-(2,3,5-trifluorophenyl)pyrrolidin-2-one;

1-[(6-bromo-2-methylpyrazolo[1,5-a]pyrimidin-3-yl)methyl]-4-propylpyrrolidin-2-one;

1-[(2-methylpyrazolo[1,5-a]pyrimidin-3-yl)methyl]-4-propylpyrrolidin-2-one;

1-[(6-bromo-2-phenylpyrazolo[1,5-a]pyrimidin-3-yl)methyl]-4-propylpyrrolidin-2-one;

1-[(6-bromo-2-thien-2-ylpyrazolo[1,5-a]pyrimidin-3-yl)methyl]-4-propylpyrrolidin-2-one;

4-propyl-1-[(2-thien-2-ylpyrazolo[1,5-a]pyrimidin-3-yl)methyl]pyrrolidin-2-one;

1-[(6-bromo-2-cyclopropylpyrazolo[1,5-a]pyrimidin-3-yl)methyl]-4-propylpyrrolidin-2-one;

1-[(6-bromo-2-tert-butylpyrazolo[1,5-a]pyrimidin-3-yl)methyl]-4-propylpyrrolidin-2-one;

1-[(2-phenylpyrazolo[1,5-a]pyrimidin-3-yl)methyl]-4-propylpyrrolidin-2-one;

1-[(2-tert-butyl-6-cyclopropylpyrazolo[1,5-a]pyrimidin-3-yl)methyl]-4-propylpyrro lidin-2 -one;

1-{[2-(2-furyl)pyrazolo[1,5-a]pyrimidin-3-yl]methyl}-4-propylpyrrolidin-2-one;

1-[(2-methyl-6-thien-2-ylpyrazolo[1,5-a]pyrimidin-3-yl)methyl]-4-propylpyrrolidin-2-one;

1-[(2-methyl-6-phenylpyrazolo[1,5-a]pyrimidin-3-yl)methyl]-4-propylpyrrolidin-2-one;

1-{[2-methyl-6-(1H-pyrrol-2-yl)pyrazolo[1,5-a]pyrimidin-3-yl]methyl} -4-propylpyrrolidin-2-one;

1-({6-[(1E)-hex-1-enyl]-2-methylpyrazolo[1,5-a]pyrimidin-3-yl}methyl)-4-propylpyrrolidin-2-one;

1-[(6-chloro-2-phenylpyrazolo[1,5-a]pyrimidin-3-yl)methyl]-4-propylpyrrolidin-2-one;

1-{[2-methyl-6-(phenylethynyl)pyrazolo[1,5-a]pyrimidin-3-yl]methyl} -4-propylpyrrolidin-2-one;

1-[(6-bromo-2-phenylpyrazolo[1,5-a]pyrimidin-3-yl)methyl]-4-(2,2-difluorovinyl)pyrrolidin-2-one;

1-[(6-hydroxy-2-methylpyrazolo[1,5-a]pyrimidin-3-yl)methyl]-4-propylpyrrolidin-2-one;

1-[(6-methyl-2-phenylpyrazolo[1,5-a]pyrimidin-3-yl)methyl]-4-propylpyrrolidin-2-one;

4-(2,2-difluorovinyl)-1-[(2-phenylpyrazolo[1,5-a]pyrimidin-3-yl)methyl]pyrrolidin-2-one;

1-[(6-methoxy-2-phenylpyrazolo[1,5-a]pyrimidin-3-yl)methyl]-4-propylpyrrolidin-2-one;

1-[(5-chloropyrazolo[1,5-a]pyrimidin-3-yl)methyl]-4-propylpyrrolidin-2-one;

4-(2,2-difluorovinyl)-1-[(5,6-dimethyl-2-phenylpyrazolo[1,5-a]pyrimidin-3-yl)methyl]pyrrolidin-2-one;

4-(2,2-difluorovinyl)-1-[(6-fluoro-5-methyl-2-phenylpyrazolo[1,5-a]pyrimidin-3-yl)methyl]pyrrolidin-2-one;

1-[(5-methoxypyrazolo[1,5-a]pyrimidin-3-yl)methyl]-4-propylpyrrolidin-2-one;

1-{[2-(4-bromophenyl)pyrazolo[1,5-a]pyrimidin-3-yl]methyl} -4-(2,2-difluorovinyl)pyrrolidin-2-one;

1-{[2-(4-fluorophenyl)pyrazolo[1,5-a]pyrimidin-3-yl]methyl}-4-propylpyrrolidin-2-one;

4-(2,2-difluorovinyl)-1-[(6-methyl-2-phenylpyrazolo[1,5-a]pyrimidin-3-yl)methyl]pyrrolidin-2-one;

4-(2,2-difluorovinyl)-1-[(5-methyl-2-phenylpyrazolo[1,5-a]pyrimidin-3-yl)methyl]pyrrolidin-2-one;

4-(2,2-difluorovinyl)-1-[(2-thien-2-ylpyrazolo[1,5-a]pyrimidin-3-yl)methyl]pyrrolidin-2-one;

1-{[2-(4-chlorophenyl)-6-methylpyrazolo[1,5-a]pyrimidin-3-yl]methyl} -4-propylpyrrolidin-2-one;

1-{[2-(4-chlorophenyl)pyrazolo[1,5-a]pyrimidin-3-yl]methyl}-4-(2,2-difluorovinyl)pyrrolidin-2-one;

1-[(6-chloro-2-phenylpyrazolo[1,5-a]pyrimidin-3-yl)methyl]-4-(2,2-difluorovinyl)pyrrolidin-2-one;

1-{[6-chloro-2-(4-chlorophenyl)pyrazolo[1,5-a]pyrimidin-3-yl]methyl}-4-(2,2-difluorovinyl)pyrrolidin-2-one;

1-[(2-cyclopropyl-5-methylpyrazolo[1,5-a]pyrimidin-3-yl)methyl]-4-(2,2-difluorovinyl)pyrrolidin-2-one;

1-[(5-chloro-2-cyclopropylpyrazolo[1,5-a]pyrimidin-3-yl)methyl]-4-(2,2-difluorovinyl)pyrrolidin-2-one;

1-[(5-chloro-2,6-dimethylpyrazolo[1,5-a]pyrimidin-3-yl)methyl]-4-(2,2-difluorovinyl)pyrrolidin-2-one;

1-[(5-bromo-1H-pyrazolo[3,4-b]pyridin-3-yl)methyl]-4-(2,2-difluorovinyl)pyrrolidin-2-one;

4-propyl-1-(pyridin-3-ylmethyl)pyrrolidin-2-one;

(-)-1-(1-pyridin-3-ylpropyl)pyrrolidin-2-one;

5-chloro-1-[(2-fluoropyridin-3-yl)methyl]-1,3-dihydro-2H-indol-2-one;

1-[(6-chloropyridin-3-yl)methyl]-4-propylpyrrolidin-2-one;

1-{[6-(benzylamino)pyridin-3-yl]methyl}-4-propylpyrrolidin-2-one;

1-[(2-aminopyridin-3-yl)methyl]-4-propylpyrrolidin-2-one;

4-propyl-1-(1H-pyrrolo[2,3-b]pyridin-3-ylmethyl)pyrrolidin-2-one;

1-[(2-isopropyl-1H-pyrrolo[2,3-b]pyridin-3-yl)methyl]-4-propylpyrrolidin-2-one;

1-[(2-phenyl-1H-pyrrolo[2,3-b]pyridin-3-yl)methyl]-4-propylpyrrolidin-2-one;

4-propyl-1-[(2-propyl-1H-pyrrolo[2,3-b]pyridin-3-yl)methyl]pyrrolidin-2-one;

1-[(6-bromo-1H-pyrrolo[2,3-b]pyridin-3-yl)methyl]-4-propylpyrrolidin-2-one;

1-[(1-benzoyl-6-bromo-1H-pyrrolo[2,3-b]pyridin-3-yl)methyl]-4-propylpyrrolidin-2-one;

1-[(6-phenyl-1H-pyrrolo[2,3-b]pyridin-3-yl)methyl]-4-propylpyrrolidin-2-one;

1-[(5-bromo-1H-pyrrolo[2,3-b]pyridin-3-yl)methyl]-4-(2,2-difluorovinyl)pyrrolidin-2-one;

1-[(7-oxido-1H-pyrrolo[2,3-b]pyridin-3-yl)methyl]-4-propylpyrrolidin-2-onc;

4-propyl-1-(H-pyrrolo[2,3-b]pyridin-4-ylmethyl)pyrrolidin-2-one;

4-propyl-1-(1H-pyrrolo[2,3-b]pyridin-5-ylmethyl)pyrrolidin-2-one;

4-propyl-1-(1H-pyrrolo[2,3-c]pyridin-2-ylmethyl)pyrrolidin-2-one;

4-propyl-1-(1H-pyrrolo[2,3-c]pyridin-3-ylmethyl)pyrrolidin-2-one;

4-propyl-1-(1H-pyrrolo[3,2-b]pyridin-3-ylmethyl)pyrrolidin-2-one;

4-propyl-1-(1H-pyrrolo[3,2-c]pyridin-2-ylmethyl)pyrrolidin-2-one;

4-propyl-1-(1H-pyrrolo[3,2-c]pyridin-3-ylmethyl)pyrrolidin-2-one;

4-propyl-1-(1,3,4-thiadiazol-2-ylmethyl)pyrrolidin-2-one;

1-[(2-amino-1,3-thiazol-5-yl)methyl]pyrrolidin-2-one;

1-(1,3-thiazol-5-ylmethyl)pyrrolidin-2-one;

1-[(2-chloro-1,3-thiazol-5-yl)methyl]-4-(2,3,5-trifluorophenyl)pyrrolidin-2-one;

1-{[2-(dimethylamino)-1,3-thiazol-5-yl]methyl}-4-(2,3,5-trifluorophenyl)pyrrolidin-2-one;

1-{[2-(methylamino)-1,3-thiazol-5-yl]methyl}-4-(2,3,5-trifluorophenyl)pyrrolidin-2-one;

1-[(2-pyrrolidin-1-yl-1,3-thiazol-5-yl)methyl]-4-(2,3,5-trifluorophenyl)pyrrolidin-2-one;

5-{[2-oxo-4-(2,3,5-trifluorophenyl)pyrrolidin-1-yl]methyl}-1,3-thiazol-2(3H)-one;

4-phenyl-1-{[3-(trifluoromethyl)[1,2,4]triazolo[4,3-b]pyridazin-7-yl]methyl}pyrrolidin-2-one;

4-phenyl-1-[(3-phenyl[1,2,4]triazolo[4,3-b]pyridazin-7-yl)methyl]pyrrolidin-2-one;

4-phenyl-1-{[3-(trifluoromethyl)[1,2,4]triazolo[4,3-b]pyridazin-8-yl]methyl}pyrrolidin-2-one;

4-propyl-1-{[3-(trifluoromethyl)[1,2,4]triazolo[4,3-b]pyridazin-8-yl]methyl}pyrrolidin-2-one;

4-phenyl-1-[(3-phenyl[1,2,4]triazolo[4,3-b]pyridazin-8-yl)methyl]pyrrolidin-2-one;

1-[(6-chloro-3-phenyl[1,2,4]triazolo[4,3-b]pyridazin-8-yl)methyl]-4-propylpyrrolidin-2-one;

1-[(6-chloro[1,2,4]triazolo[4,3-b]pyridazin-8-yl)methyl]-4-phenylpyrrolidin-2-one;

1-{[6-chloro-3-(trifluoromethyl)[1,2,4]triazolo[4,3-b]pyridazin-8-yl]methyl}-4-phenylpyrrolidin-2-one;

1-[(6-chloro-3-phenyl[1,2,4]triazolo[4,3-b]pyridazin-8-yl)methyl]-4-phenylpyrrolidin-2-one;

1-[(2-fluoroindolizin-3-yl)methyl]-4-propylpyrrolidin-2-one;

1-(1H-1,2,3-benzotriazol-1-ylmethyl)-4-propylpyrrolidin-2-one;

1-[(6-bromo-2-chloro-1H-imidazo[4,5-b]pyridin-1-yl)methyl]-4-propylpyrrolidin-2-one;

1-[(6-bromo-2-phenyl-1H-imidazo[4,5-b]pyridin-1-yl)methyl]-4-propylpyrrolidin-2-one;

1-(3H-imidazo[4,5-b]pyridin-3-ylmethyl)-4-propylpyrrolidin-2-one;

1-[(6-bromo-3H-imidazo[4,5-b]pyridin-3-yl)methyl]-4-propylpyrrolidin-2-one;

1-[(6-bromo-2-chloro-3H-imidazo[4,5-b]pyridin-3-yl)methyl]-4-propylpyrrolidin-2-one;

1-[(6-bromo-2-phenyl-3H-imidazo[4,5-b]pyridin-3-yl)methyl]-4-propylpyrrolidin-2-one;

1-[(6-bromo-2-cyclopropyl-3H-imidazo[4,5-b]pyridin-3-yl)methyl]-4-(2,2-difluorovinyl)pyrrolidin-2-one;

1-[(3-chloro-7H-imidazo[4,5-c]pyridazin-7-yl)methyl]-4-propylpyrrolidin-2-one;

1-[(2-chloro-1H-indol-1-yl)methyl]-4-propylpyrrolidin-2-one;

1-[(5-methyl-1H-indol-1-yl)methyl]-4-propylpyrrolidin-2-one;

1-[(6-methyl-1H-indol-1-yl)methyl]-4-propylpyrrolidin-2-one;

1-[(2-phenyl-1H-indol-1-yl)methyl]-4-propylpyrrolidin-2-one;

1-[(5-fluoro-1H-indol-1-yl)methyl]-4-propylpyrrolidin-2-one;

1-[(5-bromo-1H-indol-1-yl)methyl]-4-propylpyrrolidin-2-one;

1-[(5-chloro-1H-indol-1-yl)methyl]-4-propylpyrrolidin-2-one;

1-(2,3-dihydro-1H-indol-1-ylmethyl)-4-propylpyrrolidin-2-one;

1-[(5-fluoro-2-phenyl-1H-indol-1-yl)methyl]-4-propylpyrrolidin-2-one;

1-[(2-oxo-4-propylpyrrolidin-1-yl)methyl]-1H-indole-2-carbonitrile;

1-[(2-bromo-1H-indol-1-yl)methyl]-4-propylpyrrolidin-2-one;

1-[(2,5-dichloro-1H-indol-1-yl)methyl]-4-propylpyrrolidin-2-one;

1-[(6-amino-9H-purin-9-yl)methyl]-4-propylpyrrolidin-2-one;

4-propyl-1-(9H-purin-9-ylmethyl)pyrrolidin-2-one;

1-{[6-(cyclopropylamino)-9H-purin-9-yl]methyl}-4-propylpyrrolidin-2-one;

1-{[6-(benzylamino)-9H-purin-9-yl]methyl}-4-propylpyrrolidin-2-one;

4-propyl-1-{[6-(propylamino)-9H-purin-9-yl]methyl}pyrrolidin-2-one;

1-({6-[(cyclopropylmethyl)amino]-9H-purin-9-yl}methyl)-4-propylpyrrolidin-2-one;

4-propyl-1-[(6-pyrrolidin-1-yl-9H-purin-9-yl)methyl]pyrrolidin-2-one;

1-[(5-bromo-3-phenyl-1H-pyrazolo[3,4-b]pyridin-1-yl)methyl]-4-propylpyrrolidin-2-one;

1-[(5-bromo-2H-pyrazolo[3,4-b]pyridin-2-yl)methyl]-4-propylpyrrolidin-2-one;

1-[(5-bromo-3-phenyl-2H-pyrazolo[3,4-b]pyridin-2-yl)methyl]-4-propylpyrrolidin-2-one;

1-[(2-chloro-1H-pyrrolo[2,3-b]pyridin-1-yl)methyl]-4-propylpyrrolidin-2-one;

4-propyl-1-(1H-pyrrolo[3,2-b]pyridin-1-ylmethyl)pyrrolidin-2-one;

1-(3,4-dihydroquinolin-1 (2H)-ylmethyl)-4-propylpyrrolidin-2-one;

1-(8H-isothiazolo[5,4-b]indol-8-ylmethyl)-4-propylpyrrolidin-2-one;

1-(1H-1,2,4-triazol-1-ylmethyl)pyrrolidin-2-one;

1-[(2,5-dichloro-1H-pyrrol-1-yl)methyl]-4-propylpyrrolidin-2-one;

1-[(2-chloro-1H-pyrrol-1-yl)methyl]-4-propylpyrrolidin-2-one;

1-[(2-chloro-1 H-benzimidazol-1-yl)methyl]-4-propylpyrrolidin-2-one;

1-[(2-chloro-1H-benzimidazol-1-yl)methyl]-4-phenylpyrrolidin-2-one;

2-chloro-1-[(2-oxo-4-propylpyrrolidin-1-yl)methyl]-1 H-benzimidazole-5-carbonitrile;

2-chloro-1-[(2-oxo-4-propylpyrrolidin-1-yl)methyl]-1H-benzimidazole-6-carbonitrile;

4-propyl-1-[(2,5,6-trichloro-1H-benzimidazol-1-yl)methyl]pyrrolidin-2-one;

1-[(2-chloro-6-methoxy-1H-benzimidazol-1-yl)methyl]-4-propylpyrrolidin-2-one;

1-[(2-chloro-5-methoxy-1 H-benzimidazol-1-yl)methyl]-4-propylpyrrolidin-2-one;

1-[(2-chloro-6-nitro-1H-benzimidazol-1-yl)methyl]-4-propylpyrrolidin-2-one;

1-[(2-chloro-5-nitro-1 H-benzimidazol-1-yl)methyl]-4-propylpyrrolidin-2-one;

1-[(2-chloro-6-methyl-1H-benzimidazol-1-yl)methyl]-4-propylpyrrolidin-2-one;

1-[(2-chloro-1H-benzimidazol-1-yl)methyl]-4-(2,2-difluorovinyl)pyrrolidin-2-one;

1-[(6-bromo-2-chloro-1H-benzimidazol-1-yl)methyl]-4-propylpyrrolidin-2-one;

1-[(5-bromo-2-chloro-1H-benzimidazol-1-yl)methyl]-4-propylpyrrolidin-2-one;

1-[(2-chloro-6-fluoro-1H-benzimidazol-1-yl)methyl]-4-propylpyrrolidin-2-one;

1-[(2-chloro-5-fluoro-1H-benzimidazol-l-yl)methyl]-4-propylpyrrolidin-2-one;

1-[(2,6-dichloro-1H-benzimidazol-1-yl)methyl]-4-propylpyrrolidin-2-one;

1-[(2,5-dichloro-1H-benzimidazol-1-yl)methyl]-4-propylpyrrolidin-2-one;

1-{[2-chloro-6-(trifluoromethyl)-1H-benzimidazol-1-yl]methyl}-4-propylpyrrolidin-2-one;

1-{[2-chloro-5-(trifluoromethyl)-1H-benzimidazol-1-yl]methyl-4-propylpyrrolidin-2-one;

1-[(2-chloro-1H-benzimidazol-1-yl)methyl]pyrrolidin-2-one;

1-[(2-chloro-6-hydroxy-1H-benzimidazol-1-yl)methyl]-4-propylpyrrolidin-2-one;

1-(pyridin-4-ylmethyl)pyrrolidin-2-one, and

1-[(2-chloro-5-hydroxy-1H-benzimidazol-1-yl)methyl]-4-propylpyrrolidin-2-one.

viii) U.S. Patent 4,696,943

The present disclosure relates to the novel compound (S)-alpha-ethyl-2-oxo-1-pyrro lidineacetamide.

ix) U.S. Patent 4,696,942

The present disclosure relates to the novel compound, (R)-alpha-ethyl-2-oxo-1-pyrrolidineacetamide

x) U.S. Patent 5,334,720

According to this disclosure we provide novel compounds of the formula I,

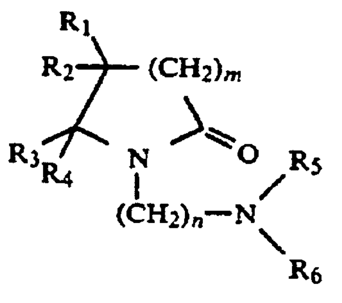

I.

wherein, R1, R2, R3 and R4, which may be the same or different independently represent hydrogen, C1-6 alkyl, phenyl or phenyl substituted by one or more halogen, hydroxyl, nitro, amino, C1-6 alkyl or C1-C6 alkoxy groups; R5 and R6 independently represent hydrogen, C1-C6 alkyl or C3 -C6 cycloalkyl , or R5 and R6 together with the nitrogen form a C4-6 N heterocycle;
m represents an integer from 1-2; and
n represents an integer from 1-3;

provided that,
two of the substituents R1, R2, R3 and R4 independently represent phenyl or substituted phenyl and the other two independently represent hydrogen or C1-6 alkyl;
or a pharmaceutically acceptable acid addition salt thereof.

Pharmaceutically acceptable acid addition salts of the compounds of formula I include salts of mineral acids, for example, hydrohalic acids, e.g. hydrochloric or hydrobromic; or organic acids, e.g. formic, acetic or lactic acids. The acid may be polybasic, for example sulphuric, fumaric, maleic or citric acid.

This disclosure also relates to all stereoisomeric forms and optical enantiomeric forms of the compounds of formula I.

In the compounds of formula I: alkyl groups which R1, R2, R3, R4, R5 and R6 may represent include methyl, ethyl, propyl, isopropyl, n-butyl, iso-butyl and s-butyl;
cycloalkyl groups which R5 and R6 may represent include cyclopropyl, cyclobutyl, cyclopentyl and cyclohexyl;
C1-6 alkoxy groups include methoxy, ethoxy and propoxy;
halogen groups include fluorine, chlorine, bromine or iodine;
We prefer compounds of formula I or a pharmaceutically acceptable acidaddition salt thereof, in which;
R1 is hydrogen, phenyl or substituted phenyl, preferably phenyl;
R2 is hydrogen, phenyl or substituted phenyl, preferably phenyl;
R3 is hydrogen , phenyl or substituted phenyl, preferably hydrogen;
R4 is hydrogen, phenyl or substituted phenyl, preferably hydrogen;
R5 is hydrogen , C1-3 alkyl or cyclopropyl , preferably hydrogen or methyl;
R6 is hydrogen , C1-3 alkyl or cyclopropyl , preferably hydrogen or methyl;
m represents an integer from 1-2 preferably 2;
n represents an integer from 1-2, preferably 1.

We especially prefer compounds of formula I in which R1 and R2 are both phenyl.

We especially prefer compounds of formula I in which one of R5 and R6 is hydrogen and the other is hydrogen or methyl.

xi) International Patent Application Publication No. WO2005/054188

In one aspect the disclosure therefore provides a compound having the formula I or a pharmaceutically acceptable salt thereof,

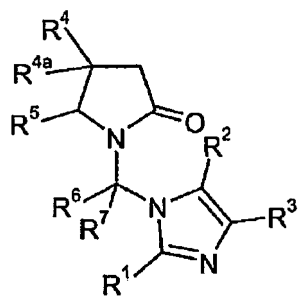

(I)

wherein

RI is hydrogen, CI-20 alkyl, C3 23 cycloalkyl, halogen, hydroxy, alkoxy, aryloxy, ester, amido, cyano, nitro, amino, guanidine, amino derivative, alkylthio, arylthio, alkylsulfonyl, arylsulfonyl, alkylsulfinyl, arylsulfinyl, aryl or heterocycle;
R2 is hydrogen, C1 20 alkyl, alkoxy, amino, halogen, hydroxy, ester, amido, nitro, cyano, carbamate, or aryl;

R3 is hydrogen, C1 20 alkyl, alkoxy, amino, halogen, hydroxy, ester, amido, nitro, cyano, carbamate, or aryl;

or R2 and R3 can form together with the imidazole ring the following 1H-benzimidazole cycle

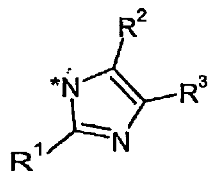

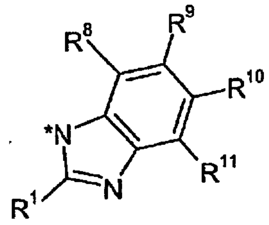

;

R4 is hydrogen, C1-20 alkyl, C2-12 alkenyl,C2-12 alkynyl, aryl, azido, alkoxycarbonylamino, arylsulfonyloxy or heterocycle; R4a is hydrogen or C1-20 alkyl; or R4 and R4a can form together a C3-8 cycloalkyl ; R5 is hydrogen; or R4, R4a and R5 can form together with the 2-oxo-1-pyrrolidine ring the following 1, 3-dihydro-2H-indol-2-one cycle

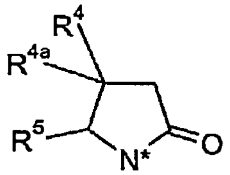

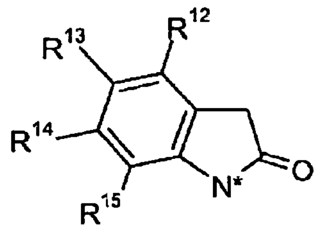

;

R6 is hydrogen or C1 20 alkyl ; R7 is hydrogen; or R6 and R7 are linked together to form a C3-6 cycloalkyl ; R8 is hydrogen, halogen, nitro, cyano, C1 20 alkyl or alkoxy ; R9 is hydrogen, C1-20 alkyl, halogen, hydroxy, alkoxy, aryloxy, ester, amido, cyano, nitro, amino, amino derivative, alkylthio, arylthio, alkylsulfonyl, arylsulfonyl, alkylsulfinyl or arylsulfinyl ;

RIO is hydrogen, C1 20 alkyl, halogen, hydroxy, alkoxy, aryloxy, ester, amido, cyano, nitro, amino, amino derivative, alkylthio, arylthio, alkylsulfonyl, arylsulfonyl, alkylsulfinyl or arylsulfinyl ;

RI1 is hydrogen, halogen, nitro, cyano, C1 20 alkyl or alkoxy ; R12 is hydrogen or halogen;

R13 is hydrogen, nitro, halogen, heterocycle, amino, aryl, C1-20 alkyl unsubstituted or substituted by halogen, or alkoxy unsubstituted or substituted by halogen; R14 is hydrogen, C1-20 alkyl or halogen;

R15 is hydrogen, C1 20 alkyl or halogen;

with the proviso that R4 is different from hydrogen when

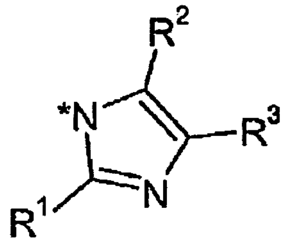

N represents a group of formula

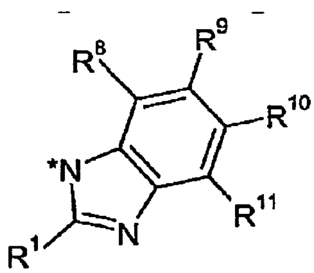

The asterisk * indicates the point of attachment of the substituents.

In a preferred instance, the disclosure concerns a compound having the formula I, their tautomers, geometrical isomers (including cis and trans, Z and E isomers), enantiomers, diastereoisomers and mixtures thereof (including all possible mixtures of stereoisomers), or pharmaceutically acceptable salts thereof,

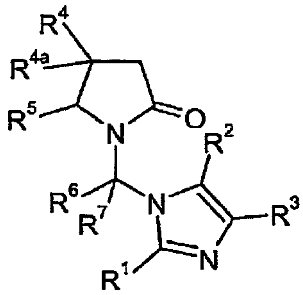

(I)

wherein

RI is hydrogen, C1-20 alkyl, C3-8 cycloalkyl, halogen, hydroxy, ester, amido, cyano, nitro, amino, guanidine, alkylthio, alkylsulfonyl, alkylsulfinyl, aryl or heterocycle; R2 is hydrogen, C1 20 alkyl, halogen, cyano, ester, carbamate or amido; R3 is hydrogen, cyano, C 1 20 alkyl, halogen or ester; or R2 and R3 can form together with the imidazole ring the following 1H- benzimidazole cycle

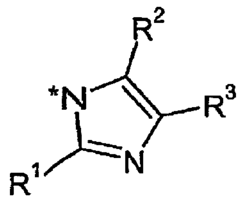

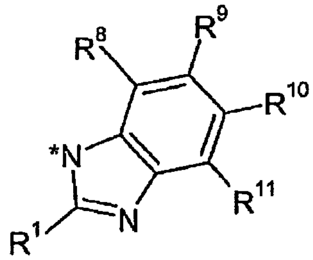

;

R4 is hydrogen, C1 20 alkyl, C2 12 alkenyl or aryl; R4a is hydrogen;

R5 is hydrogen; or R4, R4a and R5 can form together with the 2-oxo-1-pyrrolidine ring the following 1, 3-dihydro-2H-indol-2-one cycle

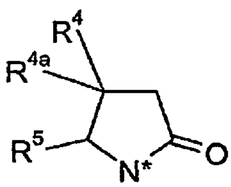

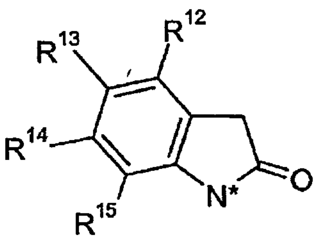

;

R6 is hydrogen or C 1 20 alkyl ; R7 is hydrogen; or R6 and R7 are linked together to form a C3-6 cycloalkyl ; R8 is hydrogen; R9 is hydrogen, C 1-20 alkyl, halogen or alkoxy; RIO is hydrogen, C1 20 alkyl, halogen or cyano; R11 is hydrogen; R12 is hydrogen or halogen; R13 is hydrogen, halogen, heterocycle or C1 20 alkyl; R14 is hydrogen; R15 is hydrogen; with the proviso that R4 is different from hydrogen when

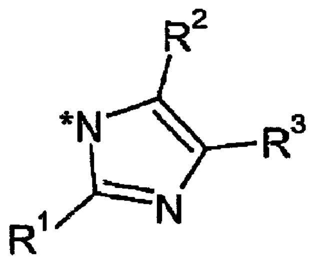

represents a group of formula

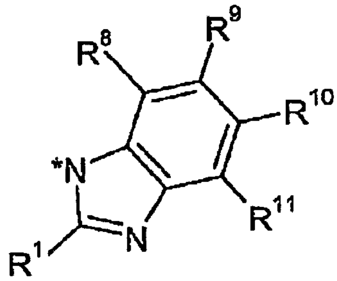

.

The term"alkyl", as used herein, represents saturated, monovalent hydrocarbon radicals having straight (unbranched) or branched or cyclic or combinations thereof and containing 1-20 carbon atoms, preferably 1-10 carbon atoms, more preferably 1-4 carbon atoms; most preferred alkyl groups have 1-3 carbon atoms. Alkyl moieties may optionally be substituted by 1 to 5 substituents independently selected from the group consisting of halogen, hydroxy, cyano, azido, aryloxy, alkoxy, alkylthio, alkanoylamino, arylcarbonylamino, aminocarbonyl, methylaminocarbonyl, dimethylaminocarbonyl or aryl. Usually alkyl groups, in the present case, are methyl, ethyl, n-propyl, i-propyl, n-butyl, i-butyl, t-butyl, 1-ethylpropyl, n-heptyl, 2,4, 4-trimethylpentyl, n-decyl, chloromethyl, trifluoromethyl, 2-bromo-2,2-difluoroethyl, 2,2, 2-trifluoroethyl, 3,3, 3-trifluoropropyl, hydroxymethyl, cyanomethyl, azidomethyl, (acetylamino) methyl, (propionylamino) methyl, (benzoylamino) methyl, (4-chlorophenoxy) methyl, benzyl, 2-phenylethyl or 2- (methylthio) ethyl. Preferred alkyl groups are methyl, ethyl, n-propyl, i-propyl, n-butyl, i-butyl, t-butyl, 1-ethylpropyl, 2,4, 4-trimethylpentyl, chloromethyl, trifluoromethyl, 2,2, 2-trifluoroethyl, hydroxymethyl, cyanomethyl, azidomethyl, (acetylamino) methyl, (propionylamino) methyl, (benzoylamino) methyl or 2- (methylthio) ethyl. More preferred alkyl groups are methyl, ethyl, n-propyl, i-propyl, n-butyl, azidomethyl or trifluoromethyl. Most preferred alkyl groups are methyl or n-propyl.

The term"cycloalkyl", as used herein, represents a monovalent group of 3 to 8 carbon atoms, usually 3-6 carbon atoms derived from a saturated cyclic hydrocarbon, which may be substituted by any suitable group including but not limited to one or more moieties selected from groups as described above for the alkyl groups. Preferred cycloalkyl groups are cyclopropyl and cyclohexyl.

The term"alkenyl"as used herein, represents straight, branched or cyclic unsaturated hydrocarbon radicals or combinations thereof having at least one carbon- carbon double bond, containing 2-12 carbon atoms, preferably usually 2-4 carbon atoms. Alkenyl groups are being optionally substituted with any suitable group, including but not limited to one or more moities selected from groups as described above for the alkyl groups. Usually an alkenyl group is ethenyl (vinyl) optionally substituted by 1 to 3 halogens. Preferred alkenyl group, in the present case, is 2,2- difluorovinyl.

The term"alkynyl"as used herein, represents straight, branched or cyclic hydrocarbon radicals or combinations thereof containing at least one carbon-carbon triple bond, containing 2-12 carbon atoms, preferably 2-6 carbon atoms, and being optionally substituted by any suitable group, including but not limited to one or more moities selected from groups as described above for the alkyl groups. Preferably an alkynyl group is a halogenoalkynyl group (haloalkynyl group).

Groups qualified by prefixes such as"s", "i","t"and the like (e. g."i-propyl","s-butyl") are branched derivatives.

The term"aryl"as used herein, is defined as phenyl optionally substituted by 1 to 4 substituents independently selected from halogen, cyano, alkoxy, alkylthio, C1 3 alkyl or azido, preferably halogen or azido. Usually aryl groups, in

the present case are phenyl, 3-chlorophenyl, 3-fluorophenyl, 4-chlorophenyl, 4-fluorophenyl, 3,4-difluorophenyl, 3, 5-difluorophenyl, 3-chloro-4-fluorophenyl, 2,3, 4-trifluorophenyl, 2,4, 5-trifluorophenyl, 2,3, 5-trifluorophenyl, 3,4, 5-trifluorophenyl, 3-azido-2,4- difluorophenyl or 3-azido-2,4, 6-trifluorophenyl. Preferably, aryl groups are phenyl, 3- chlorophenyl, 3-fluorophenyl, 4-chlorophenyl, 4-fluorophenyl, 3,4-difluorophenyl, 3,5- difluorophenyl, 3-chloro-4-fluorophenyl, 2,3, 4-trifluorophenyl, 2,4, 5-trifluorophenyl, 2,3, 5-trifluorophenyl, 3,4, 5-trifluorophenyl or 3-azido-2, 4-difluorophenyl. Most preferred aryl groups are phenyl, 3-chlorophenyl, 3-fluorophenyl, 3,5-difluorophenyl, 2,3, 4-trifluorophenyl, 2,4, 5-trifluorophenyl, 2,3, 5-trifluorophenyl, 3, 4, 5-trifluorophenyl or 3-azido-2,4-difluorophenyl.

The term"heterocycle", as used herein, is defined as including an aromatic or non aromatic cycloalkyl moiety as defined above, having at least one O, S and/or N atom interrupting the carbocyclic ring structure. Heterocyclic ring moities can be optionally substituted by alkyl groups or halogens and optionally, one of the carbon of the carbocyclic ring structure may be replaced by a carbonyl. Usually heterocycles are 2-pyridyl, 3-pyridyl, 4-pyridyl, 2-furyl, 3-furyl, 2-thienyl, 3-thienyl, 2-tetrahydrofuranyl, 1H-pyrrol-2-yl, 1-methyl-1H-pyrrol-2-yl, 1H-pyrazol-2-yl, 1H- pyrazol-3-yl, 4-chloro-1-methyl-1H-pyrazol-3-yl, 5-chloro-1, 3-dimethyl-1H-pyrazol-4-yl, 1, 2,3-thiadiazol-4-yl, 3, 5-dimethyl-4-isothiazyl, 1H-imidazol-2-yl, 1-methyl-1H- imidazol-2-yl, 4-methyl-1H-imidazol-5-yl, or 2-methyl-1, 3-thiazol-4-yl. Preferred heterocycles are 1H-imidazol-2-yl, 1, 2,3-thiadiazol-4-yl, 1H-pyrazol-3-yl, 2-furyl, 3- furyl, 2-thienyl, 1-methyl-1H-pyrrol-2-yl, 1H-pyrrol-2-yl.

The term"halogen", as used herein, includes an atom of chlorine, bromine, fluorine, iodine. Usually halogens are chlorine, bromine and fluorine. Preferred halogens are fluorine, bromine and chlorine.

The term"hydroxy", as used herein, represents a group of formula-OH.

The term"alkoxy", as used herein, represents a group of formula-ORa wherein Ra is an alkyl group, as defined above. Preferred alkoxy group is methoxy.

The term"aryloxy", as used herein, represents a group of formula-ORb wherein Rb is an aryl group, as defined above. Preferred aryloxy group is phenoxy.

The term"ester", as used herein, represents a group of formula-COORC wherein Rc is an alkyl group or aryl group, as defined above. Preferred ester group is methoxycarbonyl.

The term"amido", as used herein, represents a group of formula-CONH2.

The term"amino", as used herein, represents a group of formula-NH2.

The term"aminoderivative", as used herein, represents an alkylamino or an arylamino group, wherein the terms"alkyl"and"aryl"are defined as above.

The term"cyano", as used herein, represents a group of formula-CN.

The term"nitro", as used herein, represents a group of formula-N02.

The term"azido", as used herein, represents a group of formula-N3.

The term"guanidine", as used herein, represents a group of formula- NHC (=NH) NH2.

The term"alkylthio", as used herein, represents a group of formula-SRd wherein Rd is an alkyl group, as defined above. One alkylthio group is methylthio.

The term"alkylsulfonyl", as used herein, represents a group of formula- S (=O) 2Re wherein Re is an alkyl group, as defined above. One alkylsulfonyl group is methylsulfonyl.

The term"alkylsulfinyl", as used herein, represents a group of formula-S (=O) Rf wherein Rf is an alkyl group, as defined above. One alkylsulfinyl group is methylsulfinyl.

The term"arylthio", as used herein, represents a group of formula-SRg wherein Rg is an aryl group, as defined above.

The term"arylsulfonyl", as used herein, represents a group of the formula- S (=O) 2Rh wherein Rh is an aryl group, as defined above.

The term"arylsulfinyl", as used herein, represents a group of the formula- S (=O) Ri wherein Ri is an aryl group, as defined above.

The term"carbamate"as used herein, represents a group of formula- N (H) C (O) OR1, wherein Ri is an alkyl or an aryl, as defined above. Usually carbamate groups are (propoxycarbonyl) amino or (benzyloaxycarbonyl) amino. One carbamate group is (benzyloaxycarbonyl) amino.

The term"alkanoylamino"as used herein, represents a group of the formula- NHC (=O) Rk wherein Rk is an alkyl group, as defined above.

The term"(arylcarbonyl) amino"as used herein, represents a group of the formula-NHC (=O) Rm wherein Rm is an aryl group, as defined above. One (arylcarbonyl) amino is benzoylamino.

Usually, RI is hydrogen; C1 lo alkyl unsubstituted or substituted by halogen, hydroxy, cyano, methylthio, phenyl or 4-chlorophenoxy ; hydroxy ; C3-6 cycloalkyl ; halogen; ester; amido; nitro ; cyano; amino ; phenyl; alkylthio; alkylsulfonyl ; alkylsulfinyl ; heterocycle unsubstituted or substituted by alkyl groups; or guanidine.

In some instances, RI is hydrogen; methyl; ethyl; i-propyl ; n-propyl ; cyclopropyl ; n-butyl; i- butyl; t-butyl; 1-ethylpropyl; 2,4, 4-trimethylpentyl; hydroxymethyl ; chloromethyl; trifluoromethyl ; 2,2, 2-trifluoroethyl ; cyanomethyl; 2-(methylthio) ethyl; chloro; bromo; nitro ; cyano; amino; aminocarbonyl; methoxycarbonyl ; methylthio; methylsulfinyl ;

methylsulfonyl; phenyl; 2-furyl ; 3-furyl; 1H-pyrrol-2-yl; 1-methyl-1H-pyrrol-2-yl ; 2- thienyl; 1H-pyrazol-3-yl ; 1, 2,3-thiadiazol-4-yl or 1H-imidazol-2-yl. More preferably, RI is hydrogen; methyl; ethyl; i-propyl ; n-propyl ; n-butyl; methylthio; nitro ; cyano; amino; chloro or 1H-pyrrol-2-yl. Most preferably, RI is hydrogen; methyl; methylthio ; nitro; cyano; amino or chloro.

Usually, R2 is hydrogen; C1 4 alkyl unsubstituted or substituted by hydroxy, alkanoylamino or benzoylamino; halogen ; ester; cyano ; alkyl carbamate; [(N-methoxy- N-methyl) amino] carbonyl. Preferably, R2 is hydrogen; methyl; hydroxymethyl; (acetylamino) methyl; (propionylamino) methyl; (benzoylamino) methyl; [(benzyloxy) carbonyl] amino ; chloro or cyano. In some instances, R2 is hydrogen; chloro or cyano.

Usually, R3 is hydrogen; C1 4 alkyl unsubstituted or substituted by hydroxy; halogen; ester or cyano. In some instances, R3 is hydrogen; hydroxymethyl; chloro; cyano.

In some instances, R3 is hydrogen or cyano. In some instances R3 is hydrogen.

Usually, R4 is hydrogen; C1 4 alkyl tlnsubstituted or substituted by halogens; C2 4 alkenyl substituted by halogens or phenyl group unsubstituted or substituted by azido or/and halogens. Preferably, R4 is hydrogen; n-propyl ; 2,2-difluorovinyl ; phenyl; 3-chlorophenyl; 3-fluorophenyl; 4-chlorophenyl; 4-fluorophenyl ; 3,5-difluorophenyl; 3,4-difluorophenyl; 3-chloro-4-fluorophenyl ; 2,3, 4-trifluorophenyl ; 2,4, 5-trifluorophenyl; 2,3, 5-trifluorophenyl ; 3,4, 5-trifluorophenyl ; 3-azido-2,4- difluorophenyl or 3-azido-2,4, 6-trifluorophenyl. More preferably, R4 is hydrogen; n- propyl ; 2,2-difluorovinyl ; phenyl; 3-chlorophenyl; 3-fluorophenyl ; 4-chlorophenyl; 4- fluorophenyl ; 3, 5-difluorophenyl ; 3,4-difluorophenyl ; 3-chloro-4-fluorophenyl; 2,3, 4-trifluorophenyl ; 2,4, 5-trifluorophenyl; 2,3, 5-trifluorophenyl; 3,4, 5-trifluorophenyl or 3- azido-2,4-difluorophenyl. Most preferably, R4 is n-propyl ; 2,2-difluorovinyl ; phenyl; 3- chlorophenyl; 3-fluorophenyl; 3,5-difluorophenyl ; 2,3, 4-trifluorophenyl ; 2,4, 5- trifluorophenyl ; 2,3, 5-trifluorophenyl ; 3,4, 5-trifluorophenyl or 3-azido-2,4- difluorophenyl.

Usually, R4a is hydrogen.

Usually, R5 is hydrogen.

Usually, R6 is hydrogen or Cl-1~0 alkyl unsubstituted or substituted by hydroxy or azido. Preferably, R6 is hydrogen or azidomethyl. More preferably R6 is hydrogen.

Usually R7 is hydrogen.

In other instances, R6 and R7 are linked to form a cyclopropyl.

In other instances, R2 and R3 can form together with the imidazole ring the following 1H-benzimidaole cycle

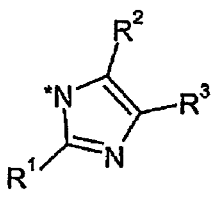

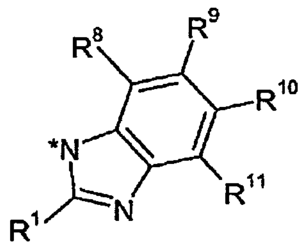

.

Usually, R8 is hydrogen.

Usually, R9 is hydrogen; halogen ; 1-3 alkyl or alkoxy. In some instances, R9 is hydrogen; methyl; chloro or methoxy. In some instances R9 is hydrogen.

Usually, RIO is hydrogen; halogen; cyano; C1 3 alkyl unsubstituted or substituted by halogens; or alkoxy. In some instances, RIO is methyl; hydrogen; trifluoromethyl ; fluoro; cyano or methoxy. In some instances R10 is hydrogen; trifluoromethyl ; fluoro or cyano.

Usually, RI 1 is hydrogen.

In other instances, R4, R4a and R5 can form together with the 2- oxo-1-pyrrolidine ring the following 1, 3-dihydro-2H-indol-2-one cycle

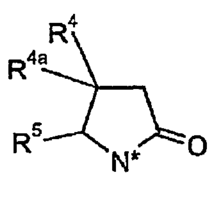

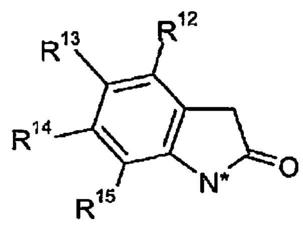

;

Usually, R12 is hydrogen or halogen. In some instances R12 is hydrogen; chloro or fluoro. In some instances R12 is hydrogen.

Usually, R13 is hydrogen; C1 3 alkyl ; halogen or thiazolyl unsubstituted or substituted by alkyl groups, such as methylthiazolyl. In some instances R13 is hydrogen; chloro; bromo or methyl. In some instances R13 is chloro; bromo or methyl.

Usually R14 is hydrogen.

Usually, R15 is hydrogen.

In a general instance of the disclosure, the compounds of formula I, or pharmaceutically acceptable salts thereof, are those wherein

RI is selected from hydrogen; C1 lo alkyl unsubstituted or substituted by halogen, hydroxy, cyano, methylthio, phenyl or 4-chlorophenoxy ; C3 6 cycloalkyl ; halogen; ester; amido; nitro; cyano; amino; phenyl; alkylthio ; alkylsulfonyl ; alkylsulfinyl ; heterocycle unsubstituted or substituted by alkyl group; or guanidine; R2 is selected from hydrogen; C 1-4 alkyl unsubstituted or substituted by hydroxy, alkanoylamino or benzoylamino; halogen; ester; cyano; alkyl carbamate or [ (N-methoxy-N-methyl) amino] carbonyl.

R3 is selected from hydrogen; C1 4 alkyl unsubstituted or substituted by hydroxy ; halogen; ester or cyano; R4 is selected from hydrogen; C1 4 alkyl unsubstituted or substituted by halogens; C2 4 alkenyl substituted by halogens or phenyl group unsubstituted or substituted by azido or/and halogens;

R4a is hydrogen; R5 is hydrogen; R6 is selected from hydrogen or C 1-10 alkyl unsubstituted or substituted by hydroxy or azido;

R7 is hydrogen; or R6 and R7 can be linked to form a cyclopropyl ; or R2 and R3 can form together with the imidazole ring the following 1H- benzimidazole cycle

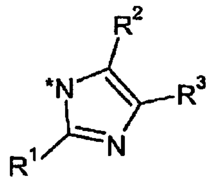

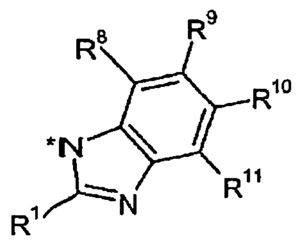

R8 is hydrogen; R9 is selected from hydrogen; halogen; C1-3 alkyl ; alkoxy ;

R10 is selected from hydrogen; halogen; cyano or Cil alkyl unsubstituted or substituted by halogens; or alkoxy ; R1 is hydrogen; or R4, R4a and R5 can form together with the 2-oxo-1-pyrrolidine ring the following 1, 3-dihydro-2H-indol-2-one cycle

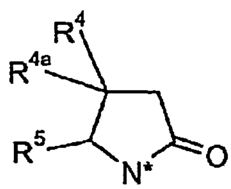

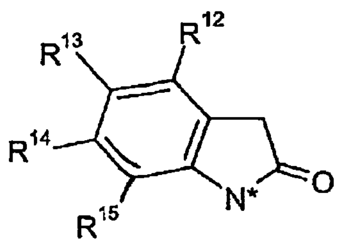

;

R12 is selected from hydrogen or halogen; R13 is selected from hydrogen; CI-3 alkyl ; halogen ; thiazolyl unsubstituted or substituted by alkyl groups, such as methylthiazolyl; R14 is hydrogen; R15 is hydrogen; with the proviso that R4 is different from hydrogen when

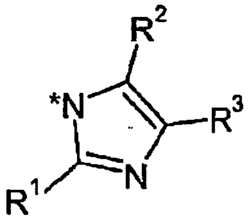

represents a group of formula

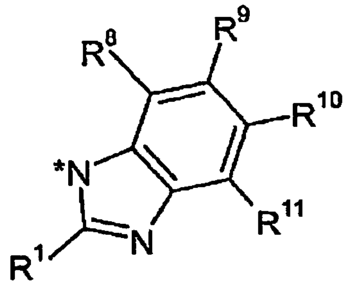

.

In an instance of the disclosure, the compounds of formula I, or pharmaceutically acceptable salt thereof, are those wherein
RI is selected from hydrogen; methyl; ethyl; i-propyl ; n-propyl ; cyclopropyl ; n-butyl; i-butyl; t-butyl; 1-ethylpropyl; 2,4, 4-trimethylpentyl; trifluoromethyl; 2,2, 2- trifluoroethyl; hydroxymethyl; chloromethyl; cyanomethyl ; 2- (methylthio) ethyl; chloro; bromo; nitro; cyano ; amino; aminocarbonyl; methoxycarbonyl ; methylthio; methylsulfinyl; methylsulfonyl; phenyl; 2-furyl ; 3-furyl ; 1H-pyrrol-2-yl; 1-methyl-1H- pyrrol-2-yl ; 2-thienyl; 1H-pyrazol-3-yl ; 1, 2, 3-thiadiazol-4-yl ; or 1H-imidazol-2-yl ; R2 is selected from hydrogen; methyl; hydroxymethyl; (acetylamino) methyl; (propionylamino) methyl; (benzoylamino) methyl; (benzyloxycarbonyl) amino; chloro; or cyano; R3 is selected from hydrogen; hydroxymethyl; chloro; cyano; or R2 and R3 can form together with the imidazole ring the following 1H- benzimidazole cycle

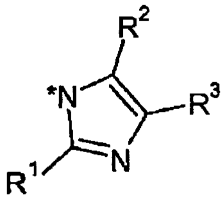

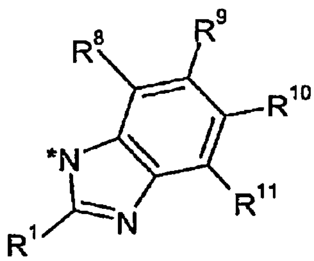

R8 is hydrogen; R9 is selected from hydrogen; methyl; choro ; methoxy;

R10 is selected from methyl; hydrogen; trifluoromethyl; fluoro; cyano; or methoxy; R is hydrogen; R4 is selected from hydrogen; n-propyl ; 2,2-difluorovinyl ; phenyl; 3- chlorophenyl; 3-fluorophenyl; 4-chlorophenyl; 4-fluorophenyl; 3,5-difluorophenyl; 3,4- difluorophenyl; 3-chloro-4-fluorophenyl ; 2,3, 4-trifluorophenyl; 2,4, 5-trifluorophenyl ; 2,3, 5-trifluorophenyl; 3,4, 5-trifluorophenyl ; 3-azido-2,4-difluorophenyl ; or 3-azido- 2,4, 6-trifluorophenyl.

R4a is hydrogen; R5 is hydrogen; or R4, R4a and R5 can form together with the 2-oxo-1-pyrrolidine ring the following 1, 3-dihydro-2H-indol-2-one cycle

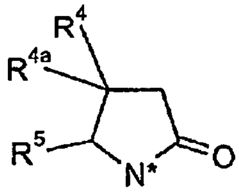

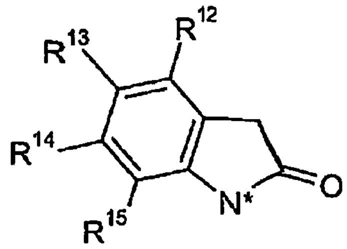

R12 is selected from hydrogen; chloro; fluoro; R13 is selected from hydrogen; chloro; bromo; methyl; R14 is hydrogen; R15 hydrogen; R6 is selected from hydrogen; azidomethyl; R7 is hydrogen; or R6 and R7 are linked to form a cyclopropyl ; with the proviso that R4 is different from hydrogen when

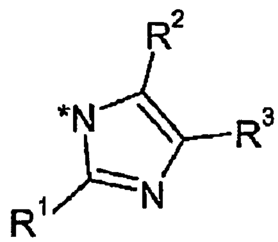

represents a group of formula

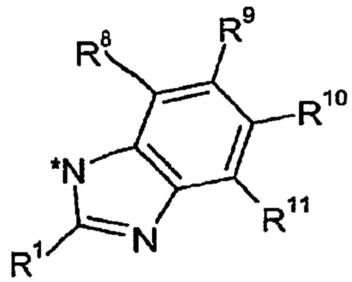

.

In one instance of the disclosure, the compounds of formula I, or pharmaceutically acceptable salt thereof, are those wherein

RI is selected from hydrogen; methyl; ethyl; i-propyl ; n-propyl ; n-butyl; methylthio; nitro; cyano ; amino; chloro ; or 1H-pyrrol-2-yl ; R2 is selected from hydrogen; chloro; cyano; R3 is selected from hydrogen; cyano; or R2 and R3 can form together with the imidazole ring the following 1H- benzimidazole cycle

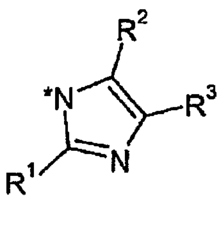

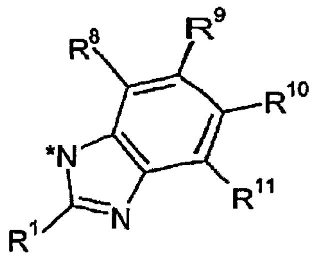

R8 is hydrogen; R9 is hydrogen;

R10 is selected from hydrogen ; trifluoromethyl ; fluoro ; cyano;

RI 1 is hydrogen; R4 is selected from hydrogen; n-propyl ; 2, 2-difluorovinyl ; phenyl; 3- chlorophenyl; 3-fluorophenyl; 4-chlorophenyl; 4-fluorophenyl ; 3, 5-difluorophenyl ; 3,4- difluorophenyl ; 3-chloro-4-fluorophenyl; 2,3, 4-trifluorophenyl ; 2,4, 5-trifluorophenyl ; 2,3, 5-trifluorophenyl; 3,4, 5-trifluorophenyl; or 3-azido-2, 4-difluorophenyl ;

R4a is hydrogen; R5 is hydrogen; or R4, R4a and R5 can form together with the 2-oxo-1-pyrrolidine ring the following 1, 3-dihydro-2H-indol-2-one cycle

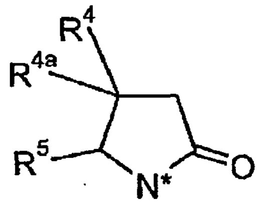

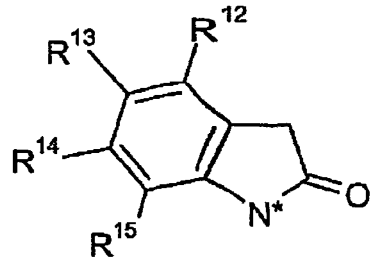

wherein R12 is hydrogen; R13 is selected from methyl; chloro; bromo; R14 is hydrogen; R15 hydrogen; R6 is hydrogen; R7 is hydrogen; with the proviso that R4 is different from hydrogen when

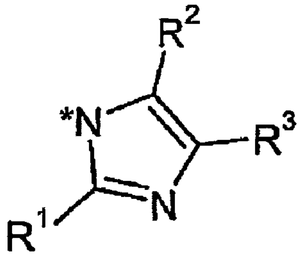

R11 represents a group of formula

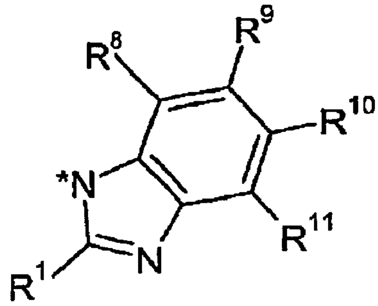

.

In one instance of the disclosure, the compounds of formula I, or pharmaceutically acceptable salt thereof, are those wherein

RI is selected from hydrogen; methyl; methylthio; nitro ; cyano; amino; chloro; R2 is selected from hydrogen; chloro; cyano; R3 is hydrogen; R4 is selected from n-propyl; 2, 2-difluorovinyl; phenyl; 3-chlorophenyl; 3- fluorophenyl; 3,5-difluorophenyl ; 2,3, 4-trifluorophenyl ; 2,4, 5-trifluorophenyl ; 2,3, 5-trifluorophenyl; 3,4, 5-trifluorophenyl; 3-azido-2,4-difluorophenyl ; R4a is hydrogen;

R5 is hydrogen; or R4, R4a and R5 can form together with the 2-oxo-1-pyrrolidine ring the following 1, 3-dihydro-2H-indol-2-one cycle

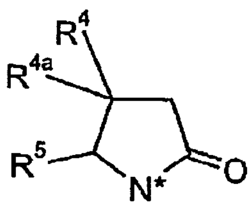

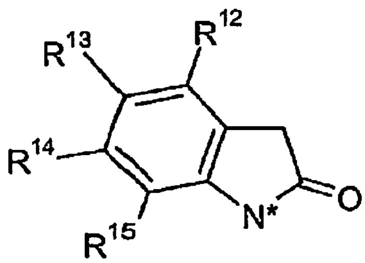

R12 is hydrogen; R13 is selected from chloro; bromo; methyl; R14 is hydrogen;

R15 hydrogen; R6 is hydrogen; R7 is hydrogen.

In some instances, compounds are: 1-(1H-imidazol-1-ylmethyl) pyrrolidin-2-one ; 1- (1H- imidazol-1-ylmethyl)-4-phenylpyrrolidin-2-one ; 4- (3-azido-2, 4, 6-trifluorophenyl)-1-(1H-imidazol-1-ylmethyl) pyrrolidin-2-one; 1- (IH-imidazol-1-ylmethyl)-4- propylpyrrolidin-2-one; (-)-4-(3-azido-2,4-difluorophenyl)-1-(1H-imidazol-1- ylmethyl) pyrrolidin-2-one; (+)-4- (3-azido-2, 4-difluorophenyl)-1-(IH-imidazol-1- ylmethyl) pyrrolidin-2-one ; 1-[(2-ethyl-1H-imidazol-1-yl)methyl]-4-propylpyrrolidin-2- one; 1-[(2-isopropyl-1H-imidazol-1-yl) methyl]-4-propylpyrrolidin-2-one ; 1-[(2-methyl- IH-imidazol-1-yl) methyl]-4-propylpyrrolidin-2-one ; 1-[(2-phenyl-1H-imidazol-1-yl)methyl]-4-propylpyrrolidin-2-one ; 4-propyl-1-[(2-propyl-1H-imidazol-1- yl) methyl] pyrrolidin-2-one ; (+)-1-(1H-imidazol-1-ylmethyl)-4-propylpyrrolidin-2-one ; (- )-1-(1H-imidazol-1-ylmethyl)-4-propylpyrrolidin-2-one ; 4- (2, 2-difluorovinyl)-1- (1H-imidazol-1-ylmethyl) pyrrolidin-2-one ; 4-(3-chlorophenyl)-1-(1H-imidazol-1-ylmethyl) pyrrolidin-2-one; 1-{[2-(methylthio)-1H-imidazol-1-yl] methyl}-4-propylpyrrolidin-2-one; 1-{[2-(methylsulfinyl)-1H-imidazol-1-yl] methyl}-4-propylpyrrolidin-2-one ; 1-[(2-tert-butyl-1H-imidazol-1-yl) methyl]-4-propylpyrrolidin-2- one; 1- [1- (1H-imidazol-1-yl) cyclopropyl] pyrrolidin-2-one ; 1- [(2-methyl-1H-imidazol-1-yl) methyl]-4-phenylpyrrolidin-2-one ; 1-{[2-(methylsulfonyl)-1H-imidazol-1-yl] methyl}-4- propylpyrrolidin-2-one; 1-[(2-oxo-4-propylpyrrolidin-1-yl)methyl]-1H-imidazole-2- carboxamide; 4-(4-fluorophenyl)-1-(1H-imidazol-1-ylmethyl)pyrrolidin-2-one ; 1- (1H- imidazol-1-ylmethyl)-4-(3, 4, 5-trifluorophenyl) pyrrolidin-2-one; 4- (3-fluorophenyl)-1- (1H-imidazol-1-ylmethyl) pyrrolidin-2-one; 4-(3,5-difluorophenyl)-1-(1H-imidazol-1-ylmethyl) pyrrolidin-2-one; 4-(3,4-difluorophenyl)-1-(1H-imidazol-1-ylmethyl) pyrrolidin- 2-one; 4-(3-chloro-4-fluorophenyl)-1-(1H-imidazol-1-ylmelthyl) pyrrolidin-2-one; 4-(4- chlorophenyl)-1-(1H-imidazol-1-ylmelthyl) pyrrolidin-2-one; 1-(1H-imidazol-1-ylmethyl)- 4- (2, 3, 4-trifluorophenyl) pyrrolidin-2-one; 1-(1H-imidazol-1-ylmethyl)-4-(2, 3,5-trifluorophenyl) pyrrolidin-2-one ; 1-(1H-imidazol-1-ylmethyl)-4-(2, 4,5- trifluorophenyl) pyrrolidin-2-one; 1-{[2-(hydroxymethyl)-1H-imidazol-1-yl]methyl}-4- propylpyrrolidin-2-one ; methyl 1-[ (2-oxo-4-propylpyrrolidin-1-yl) methyl]-IH-imidazole- 2-carboxylate ; 1- [(2-nitro-IH-imidazol-1-yl) methyll-4- (3, 4,5-trifluorophenyl) pyrrolidin- 2-one; 1-{[2-oxo-4-(3, 4, 5-trifluorophenyl) pyrrolidin-1-yl] methyl}-1H-imidazole-2-carbonitrile; 1-[(2-amino-1H-imidazol-1-yl)methyl]-4-propylpyrrolidin-2-one ; 1- [ (2, 4- dichloro-IH-imidazol-1-yl) methyl]-4- (3, 4, 5-trifluorophenyl) pyrrolidin-2-one ; 1- [ (5- chloro-1H-imidazol-1-yl) methyl]-4- (3, 4, 5-trifluorophenyl) pyrrolidin-2-one ; 1-{[2-oxo-4- (3,4, 5-trifluorophenyl) pyrrolidin-1-yl] methyl}-1H-imidazole-4-carbonitrile ; 1-{[2-oxo-4- (3,4, 5-trifluorophenyl) pyrrolidin-1-yl] methyl}-1H-imidazole-5-carbonitrile ; (+)-1- (1H- imidazol-1-ylmethyl)-4-phenylpyrrolidin-2-one ; (-)-1-(1H-imidazol-1-ylmethyl)-4- phenylpyrrolidin-2-one ; 1- { [2-oxo-4- (2, 3, 5-trifluorophenyl) pyrrolidin-1-yl] methyl}-1H-imidazole-5-carbonitrile ; (-)-1-{[2-oxo-4-(2, 3, 4-trifluorophenyl) pyrrolidin-1-yl]methyl}-1H-imidazole-5-carbonitrile ; (+)-1-{[2-oxo-4-(2, 3,4-trifluorophenyl) pyrrolidin-1- yl] methyl}-1H-imidazole-5-carbonitrile ; (-)-1-{[2-oxo-4-(2, 3,4-trifluorophenyl) pyrrolidin-1-yl] methyl}-1H-imidazole-4-carbonitrile ; (+)-1-{[2-oxo-4-(2, 3, 4-trifluorophenyl)-1- pyrrolidinyl] methyl}-1H-imidazole-4-carbonitrile ; (-)-1- { [2-oxo-4- (3, 4,5- trifluorophenyl) pyrrolidin-1-yl]methyl}-1H-imidazole-4-carbonitrile; (+)-1-{[2-oxo-4- (3,4, 5-trifluorophenyl) pyrrolidin-1-yl] methyl}-1H-imidazole-4-carbonitrile ; (+)-1-{[2-oxo- 4- (2, 4, 5-trifluorophenyl) pyrrolidin-1-yl] methyl}-1H-imidazole-4-carbonitrile ; (-)-1-{[2-oxo-4- (2, 4,5-trifluorophenyl) pyrrolidin-1-yl] methyl}-1H-imidazole-4-carbonitrile ; (-)-1- {[2-oxo-4-(2, 3, 5-trifluorophenyl) pyrrolidin-1-ylmethyl}-1H-imidazole-4-carbonitrile ; (-)- 1-{[2-oxo-4-(3, 4, 5=trifluorophenyl) pyrrolidin-1-yl] methyl}-1H-imidazole-5-carbonitrile ; 1-{[2-oxo-4-(2, 3, 5-trifluorophenyl) pyrrolidin-1-yl]methyl}-1H-imidazole-5-carbonitrile ; 1-{[2-oxo-4-(2, 3,5-trifluorophenyl) pyrrolidin- methyl}-1H-imidazole-5-carbonitrile ; 1-[(5-methyl-2-phenyl-1H-imidazol-1-yl)methyl]-4-propylpyrrolidin-2-one ; 1- [ (5- methyl-IH-imidazol-1-yl)methyl]-4-propylpyrrolidin-2-one ; 1-[(5-phenyl-1H-imidazol- 1-yl) methyl]-4-propylpyrrolidin-2-one ; 1-[(2-ethyl-5-methyl-1H-imidazol-1-yl)methyl]- 4-propylpyrrolidin-2-one; 1-[(2,5-dimethyl-1H-imidazol-1-yl)methyl]-4- propylpyrrolidin-2-one; 1- [ (2-chloro-1H-imidazol-1-yl) methyll-4- (3, 4,5-trifluorophenyl) pyrrolidin-2-one; 1-[2-azido-1-(1H-imidazol-1-yl) ethyl] -4-propylpyrrolidin-2-one ; 1- [ (4-chloro-IH-imidazol-1-yl) methyll-4- (3, 4,5-trifluorophenyl) pyrrolidin-2-one; 1-[(2-bromo-4,5-dichloro-1H-imidazol-1-yl)methyl]-4- propylpyrrolidin-2-one; 1- [(2-chloro-1H-imidazol-1-yl)methyl]-4-propylpyrrolidin-2- one; (+)-1-1 [2-oxo-4- (3, 4,5-trifluorophenyl) pyrrolidin-1-yl]methyl}-1H-imidazole-5- carbonitrile; 1-{[5-(hydroxymethyl)-1H-imidazol-1-yl]methyl}-4-propylpyrrolidin-2-one ; 1-{[4-(hydroxymethyl)-1H-imidazol-1-yl] methyl}-4-propylpyrrolidin-2-one ; benzyl 1- [ (2- oxo-4-propylpyrrolidin-1-yl) methyl]-1H-imidazol-5-ylcarbamate ; N-[(1-{[2-oxo-4-(3, 4,5-trifluorophenyl) pyrrolidin-1-yl] methyl}-1H-imidazol-5-yl) methyl] acetamide ; N-[(1-{[2- oxo-4-(3, 4, 5-trifluorophenyl) pyrrolidin-1-yl] methyl)-1H-imidazol-5- yl) methyl] benzamide; N-1 (1-1 [2-oxo-4-(3, 4, 5-trifluorophenyl) pyrroldin-1-yl]methyl}-1H- imidazol-5-yl) methyl] propanamide ; 1- (IH-benzimidazol-1-ylmethyl)-4-propylpyrrolidin- 2-one; 1-[(2-methyl-1H-benzimidazol-1-yl)methyl]-4-propylpyrrolidin-2-one ; 4-propyl- 1-[(2-propyl-1H-benzimidazol-1-yl)methyl]pyrrolidin-2-one ; 1-[(2-isopropyl-1H- benzimidazol-1-yl) methyl]-4-propylpyrrolidin-2-one ; 4-propyl-1-{[2-(trifluoromethyl)- 1H-benzimidazol-l-yl] methyl} pyrrolidin-2-one; 1-{[2-(methylthio)-1H-benzimidazol-1-yl] methyl}-4-propylpyrrolidin-2-one ; 1-[(2-amino-1H-benzimidazol-1-yl)methyl]-4-propylpyrrolidin-2-one ; 1-{[2-(chloromethyl)-1H-benzimidazol-1-yl]melthyl}-4-propylpyrrolidin-2-one ; {1-[(2-oxo-4-propylpyrrolidin-1-yl) methyl]-1 H-benzimidazol-2- yl} acetonitrile ; 1- [ (5-methoxy-1H-benzimidazol-1-yl) methyl]-4-propylpyrrolidin-2-one ; 1-[(5-methyl-1H-benzimidazol-1-yl) methyl]-4-propylpyrrolidin-2-one ; 1- [ (5, 6-dimethyl- 1H-benzimidazol-1-yl) methyl]-4-propylpyrrolidin-2-

one ; 1-{[2-isopropyl-5- (trifluoromethyl)-1H-benzimidazol-1-yl] methyl}-4-propylpyrrolidin-2-one ; 1-[(6-chloro- IH-benzimidazol-1-yl) methyl]-4-propylpyrrolidin-2-one ; 1-[(2-oxo-4-propylpyrrolidin-1-yl) methyl]-2-propyl-1H-benzimidazole-5-carbonitrile ; 1-{[2-ethyl-5-(trifluorornethyl)- 1H-benzimidazol-1-yl] methyl}-4-propylpyrrolidin-2-one ; 4-propyl-1-{[2-(1H-pyrrol-2-yl)- 1H-benzimidazol-1-yl] methyl} pyrrolidin-2-one ; 1- [ (5-fluoro-2-propyl-1H-benzimidazol- 1-yl) methyl]-4-propylpyrrolidin-2-one ; 1-{[6-methyl-2-(1H-pyrrol-2-yl)-1H- benzimidazol-1-yl] methyl}-4-propylpyrrolidin-2-one ; 1-[(6-methoxy-2-propyl-1H- benzimidazol-1-yl) methyl]-4-propylpyrrolidin-2-one ; 2-butyl-1- [ (2-oxo-4- propylpyrrolidin-1-yl) methyl]-1H-benzimidazole-5-carbonitrile ; 1-{[2-[2- (methylthio) ethyl]-5-(trifluoromethyl)-1H-benzimidazol-1-yl] methyl}-4-propylpyrrolidin- 2-one; 1-[(5-fluoro-2-isobutyl-1H-benzimidazol-1-yl)methyl]-4-propylpyrrolidin-2-one ; 1-{[5-fluoro-2-(2, 4, 4-trimethylpentyl)-1 H-benzimidazol-1-yl] methyl}-4-propylpyrrolidin- 2-one; 2-cyclopropyl-1-[(2-oxo-4-propylpyrrolidin-1-yl)methyl]-1H-benzimidazole-5- carbonitrile ; 1- [ (2-oxo-4-propylpyrrolidin-1-yl) methyl]-2- (IH-pyrazol-3-yl)-IH-benzimidazole-5-carbonitrile; 1-[(2-cyclopropyl-5-fluoro-1H-benzimidazol-1-yl)methyl]-4-propylpyrrolidin-2-one ; 1-[(5-fluoro-2-isopropyl-1H-benzimidazol-1-yl)methyl]-4- propylpyrrolidin-2-one ; 1-{[2-(3-furyl)-6-methoxy-1H-benzimidazol-1-ylmethyl}-4- propylpyrrolidin-2-one; 1- [(2-cyclopropyl-6-methoxy-1H-benzimidazol-1-yl) methyl]-4- propylpyrrolidin-2-one; 1- [(2-isopropyl-6-methoxy-1H-benzimidazol-1-yl) methyl]-4- propylpyrrolidin-2-one ; 1- [(2-oxo-4-propylpyrrolidin-1-yl) methyl]-2-(1, 2,3-thiadiazol-4- yl)-1H-benzimidazole-5-carbonitrile ; 1-{[2-(1H-imidazol-2-yl)-5-(trifluoromethyl)-1H-benzimidazol-1-yl] methyl}-4-propylpyrrolidin-2-one ; 1-{[5-fluoro-2-(2, 2,2- trifluoroethyl)-1H-benzimidazol-1-yl] methyl}-4-propylpyrrolidin-2-one ; 1- { [2- (1- ethylpropyl)-6-methoxy-IH-benzimidazol-1-yl] methyl}-4-propylpyrrolidin-2-one ; 1-{[6-methoxy-2- (1-methyl-1H-pyrrol-2-yl)-IH-benzimidazol-1-yl] methyl}-4-propylpyrrolidin- 2-one; 1-{[2-(2-furyl)-5-(trifluoromethyl)-1H-benzimidazol-1-yl]methyl}-4- propylpyrrolidin-2-one; 4-propyl-1-{[2-thien-2-yl-5-(trifluoromethyl)-1H-benzimidazol- 1-yl]melthyl}pyrrolidin-2-one ; 1-1 [2- (3-furyl)-5- (trifluoromethyl)-IH-benzimidazol-1- yl] methyl}-4-propylpyrrolidin-2-one ; 1- { [2-cyclopropyl-5- (trifluoromethyl)-1H- benzimidazol-1-yl] methyl}-4-propylpyrrolidin-2-one ; 4-propyl-1-{[2-(1H-pyrrol-2-yl)-5- (trifluoromethyl)-1H-benzimidazol-1-yl] methyl} pyrrolidin-2-one ; 1- (IH-imidazol-1- ylmethyl)-1, 3-dihydro-2H-indol-2-one ; 5-bromo-1-(1H-imidazol-1-ylmethyl)-1, 3- dihydro-2H-indol-2-one; 5-chloro-1- (1H-imidazol-1-ylmethyl)-1,3-dihydro-2H-indol-2- one; 4-fluoro-1-(1H-imidazol-1-ylmethyl)-1,3-dihydro-2H-indol-2-one; 4-chloro-1-(1H-imidazol-1-ylmethyl)-1, 3-dihydro-2H-indol-2-one ; 1-(1H-imidazol-1-ylmethyl)-5-methyl-1, 3-dihydro-2H-indol-2-one ; 1- [ (2-oxo-2, 3-dihydro-1H-indol-1-yl) methyl]-1H- imidazole-5-carbonitrile; and 1- [ (5-chloro-2-oxo-2, 3-dihydro-1H-indol-1-yl) methyl]-1H- imidazole-5-carbonitrile.

In some instances, compounds are: 1- (1H-imidazol-1-ylmethyl) pyrrolidin-2-one, 1- (1H-imidazol-1-ylmethyl)-4-phenylpyrrolidin-2-one ; 1-(1H-imidazol-1-ylmethyl)-4- propylpyrrolidin-2-one; (-)-4- (3-azido-2, 4-difluorophenyl)-1-(1H-imidazol-1- ylmethyl) pyrrolidin-2-one; (+)-4-(3-azido-2,4-difluorophenyl)-1-(1H-imidazol-1- ylmethyl) pyrrolidin-2-one ; 1-[(2-ethyl-1H-imidazol-1-yl)methyl]-4-propylpyrrolidin-2- one; 1-[(2-isopropyl-1H-imidazol-1-yl)methyl]-4-propylpyrrolidin-2-one ; 1- [ (2-methyl- 1H-imidazol-1-yl) methyl]-4-propylpyrrolidin-2-one ; 4-propyl-1-[(2-propyl-1H-imidazol- 1-yl) methyl] pyrrolidin-2-one ; (+)-1- (1H-imidazol-1-ylmethyl)-4-propylpyrrolidin-2-one ; (-)-1-(1H-imidazol-1-ylmethyl)-4-propylpyrrolidin-2-one ; 4-(2, 2-difluorovinyl)-1-(1 H- imidazol-1-ylmethyl) pyrrolidin-2-one; 4- (3-chlorophenyl)-1-(1H-imidazol-1-ylmethyl) pyrrolidin-2-one ; 1-{[2-(methylthio)-1H-imidazol-1-yl]methyl}-4-propylpyrrolidin-2-one ; 1-[(2-methyl-1H-imidazol-1-yl)methyl]-4-phenylpyrrolidin-2- one; 4-(4-fluorophenyl)-1-(1H-imidazol-1-ylmethyl) pyrrolidin-2-one; 1-(1H-imidazol-1- ylmethyl)-4- (3, 4, 5-trifluorophenyl) pyrrolidin-2-one; 4-(3-fluorophenyl)-1-(1H-imidazol- 1-ylmethyl) pyrrolidin-2-one; 4-(3,5-difluorophenyl)-1-(1H-imidazol-1- ylmethyl) pyrrolidin-2-one ; 4-(3,4-difluorophenyl)-1-(1H-imidazol-1-ylmethyl) pyrrolidin- 2-one; 4-(3-chloro-4-fluorophenyl)-1-(1H-imidazol-1-ylmethyl) pyrrolidin-2-one; 4- (4-chlorophenyl)-1-(1H-imidazol-1-ylmethyl) pyrrolidin-2-one; 1- (1H-imidazol-1-ylmethyl)- 4- (2, 3, 4-trifluorophenyl) pyrrolidin-2-one; 1-(1H-imidazol-1-ylmethyl)-4-(2, 3,5-trifluorophenyl) pyrrolidin-2-one; 1- (1 H-imidazol-1-ylmethyl)-4- (2, 4,5-trifluorophenyl) pyrrolidin-2-one; 1-[(2-nitro-1H-imidazol-1-yl) methyl]-4-(3, 4,5-trifluorophenyl) pyrrolidin-2-one ; 1-{ [2-oxo-4- (3, 4, 5-trifluorophenyl) pyrrolidin-1- yl] methyl}-1H-imidazole-2-carbonitrile ; 1-[(2-amino-1H-imidazol-1-yl)methyl]-4- propylpyrrolidin-2-one; 1-1 (5-chloro-IH-imidazol-1-yl) methyl]-4- (3, 4,5-trifluorophenyl) pyrrolidin-2-one; 1-{[2-oxo-4-(3, 4,5-trifluorophenyl) pyrrolidin-1- yl] methyl}-1H-imidazole-4-carbonitrile ; 1-{[2-oxo-4-(3, 4, 5-trifluorophenyl) pyrrolidin-1- yl] methyl}-1H-imidazole-5-carbonitrile ; (+)-1-(1H-imidazol-1-ylmethyl)-4- phenylpyrrolidin-2-one ; (-)-1-(1H-imidazol-1-ylmethyl)-4-phenylpyrrolidin-2-one ; (+); 1-{[2-oxo-4-(3, 4,5-trifluorophenyl) pyrrolidin-1-yl]methyl}-1H-imidazole-4-carbonitrile ; 1-[(2-chloro-1H-imidazol-1-yl) methyl]-4-(3, 4, 5-trifluorophenyl) pyrrolidin-2-one ; 1- [2- azido-1-(1H-imidazol-1-yl) ethyl]-4-propylpyrrolidin-2-one ; 1-[(2-chloro-1H-imidazol-1- yl) methyl]-4-propylpyrrolidin-2-one ; (+)-1-1 [2-oxo-4- (3, 4, 5-trifluorophenyl) pyrrolidin-1-yl]methyl}-1H-imidazole-5-carbonitrile ; 1-[(2-oxo-4-propylpyrrolidin-1-yl) methyl]-2- propyl-1H-benzimidazole-5-carbonitrile ; 1-{[2-ethyl-5-(trifluoromethyl)-1H- benzimidazol-1-yl]methyl}-4-propylpyrrolidin-2-one ; 4-propyl-1-{[2-(1H-pyrrol-2-yl)-1H- benzimidazol-1-yl]methyl}pyrrolidin-2-one ; 1-[(5-fluoro-2-propyl-1H-benzimidazol-1- yl) methyl]-4-propylpyrrolidin-2-one ; 2-butyl-1- [(2-oxo-4-propylpyrrolidin-1-yl) methyl]- 1H-benzimidazole-5-carbonitrile ; 1- [ (5-fluoro-2-isopropyl-1H-benzimidazol-1- yl) methyl]-4-propylpyrrolidin-2-one ; 1-(1H-imidazol-1-ylmethyl)-1, 3-dihydro-2H-indol- 2-one; 5-bromo-1- (1H-imidazol-1-ylmethyl)-1, 3-dihydro-2H-indol-2-one ; 5-chloro-1- (1H-imidazol-1-ylmethyl)-1, 3-dihydro-2H-indol-2-one ; 1-(1H-imidazol-1-ylmethyl)-5- methyl-1,3-dihydro-2H-indol-2-one ; 1-[(5-chloro-2-oxo-2,3-dihydro-1H-indol-1- yl) methyl]-1H-imidazole-5-carbonitrile.

In some instances, compounds are: 1-(1H-imidazol-1-ylmethyl)-4-phenylpyrrolidin- 2-one; 1-(1H-imidazol-1-ylmethyl)-4-propylpyrrolidin-2-one ; (-)-4- (3-azido-2, 4- difluorophenyl)-1-(1H-imidazol-1-ylmethyl) pyrrolidin-2-one; (+)-4- (3-azido-2, 4- difluorophenyl)-1-(1H-imidazol-1-ylmethyl) pyrrolidin-2-one; 4-(2,2-difluorovinyl)-1- (1H-imidazol-1-ylmethyl) pyrrolidin-2-one; 4-(3-chlorophenyl)-1-(1H-imidazol-1- ylmethyl) pyrrolidin-2-one; 1-{[2-(methylthio)-1H-imidazol-1-yl] methyl}-4- propylpyrrolidin-2-one; 1-[(2-methyl-1H-imidazol-1-yl)methyl]-4-phenylpyrrolidin-2- one; 1- (1H-imidazol-1-ylmethyl)-4- (3, 4,5-trifluorophenyl) pyrrolidin-2-one; 4- (3- fluorophenyl)-1-(1H-imidazol-1-ylmethyl) pyrrolidin-2-one; 4-(3,5-difluoromethyl)-1- (IH-imidazol-1-ylmetliyl) pyrrolidin-2-one; 1-(1H-imidazol-1-ylmethyl)-4-(2, 3,4- trifluorophenyl) pyrrolidin-2-one; 1-(1H-imidazol-1-ylmethyl)-4-(2, 3,5- trifluorophenyl) pyrrolidin-2-one; 1- H-imidazol-1-ylmethyl)-4-(2, 4,5- trifluorophenyl) pyrrolidin-2-one; 1-[(2-nitro-1H-imidazol-1-yl) methyl]-4-(3, 4,5- trifluorophenyl) pyrrolidin-2-one; 1-{[2-oxo-4-(3, 4, 5-trifluorophenyl) pyrrolidin-1- yl] methyl}-1H-imidazole-2-carbonitrile ; 1-[(2-amino-1H-imidazol-1-yl) methyl]-4- propylpyrrolidin-2-one ; 1-[(5-chloro-1H-imidazol-1-yl)methyl]-4-(3, 4,5- trifluorophenyl) pyrrolidin-2-one; (+)-1-(1H-imidazol-1-ylmethyl)-4-phenylpyrrolidin-2- one; (-)-1-(1H-imidazol-1-ylmethyl)-4-phenylpyrrolidin-2-one ; 1-[(2-chloro-1H- imidazol-1-yl) methyl]-4- (3, 4,5-trifluorophenyl) pyrrolidin-2-one 1-[(2-chloro-1H-imidazol-1-yl) methyl]-4-propylpyrrolidin-2-one ; (+)-1-1 [2-oxo-4- (3, 4,5- trifluorophenyl) pyrrolidin-1-yl] methyl}-1H-imidazole-5-carbonitrile ; 5-bromo-1- (1H- imidazol-1-ylmethyl)-1, 3-dihydro-2H-indol-2-one; 5-chloro-1-(1H-imidazol-1-ylmethyl)- 1, 3-dihydro-2H-indol-2-one; 1- (1H-imidazol-1-ylmethyl)-5-methyl-1, 3-dihydro-2H- indol-2-one; 1-[(5-chloro-2-oxo-2, 3-dihydro-1H-indol-1-yl) methyl]-1H-imidazole-5-carbonitrile.

Some compounds are: (-)-4- (3-azido-2, 4-difluorophenyl)-1- (1H-imidazol-1-ylmethyl) pyrrolidin-2-one ; (+)-4-(3-azido-2, 4-difluorophenyl)-1-(1H-imidazol-1-ylmethyl) pyrrolidin-2-one; 4-(3-azido-2, 4-difluorophenyl)-1-(1H-imidazol-1-ylmethyl) pyrrolidin-2-one.

The acid addition salt form of a compound of formula I that occurs in its free form as a base can be obtained by treating the free base with an appropriate acid such as an inorganic acid, for example, a hydrohalic such as hydrochloric or hydrobromic, sulfuric, nitric, phosphoric and the like; or an organic acid, such as, for example, acetic, trifluoroacetic, hydroxyacetic, propanoic, lactic, pyruvic, malonic, succinic, maleic, fumaric, malic, tartaric, citric, methanesulfonic, ethanesulfonic, benzenesulfonic, p-toluenesulfonic, cyclic, salicylic, p-aminosalicylic, pamoic and the like.

The compounds of formula I containing acidic protons may be converted into their therapeutically active, non-toxic base addition salt forms, e. g. metal or amine salts, by treatment with appropriate organic and inorganic bases. Appropriate base salt forms include, for example, ammonium salts, alkali and earth alkaline metal salts, e. g. lithium, sodium, potassium, magnesium, calcium salts and the like, salts with organic bases, e. g. N-methyl-D-glucamine, hydrabamine salts, and salts with amino acids such as, for example, arginine, lysine and the like.

Conversely said salt forms can be converted into the free forms by treatment with an appropriate base or acid.

Compounds of the formula I and their salts can be in the form of a solvate, which is included within the scope of the present disclosure. Such solvates include for example hydrates, alcoholates and the like.

Many of the compounds of formula I and some of their intermediates have at least one stereogenic center in their structure. This stereogenic center may be present in a R or a S configuration, said R and S notation is used in correspondence with the rules described in Pure Appl. Chem. , 45 (1976) 11-30.

The disclosure also relates to all stereoisomeric forms such as enantiomeric and diastereoisomeric forms of the compounds of formula I or mixtures thereof (including all possible mixtures of stereoisomers).

Some of the compounds of formula I may also exist in tautomeric forms. Such forms although not explicity indicated in the above formula are intended to be included within the scope of the present disclosure.

In another preferred instance, the present disclosure concerns also compounds of formula IA and their tautomeric form IB

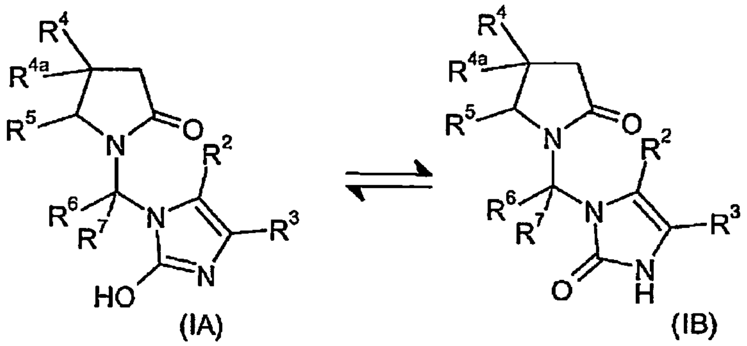

With respect to the present disclosure reference to a compound or compounds is intended to encompass that compound in each of its possible isomeric forms and mixtures thereof, unless the particular isomeric form is referred to specifically.

Compounds according to the present disclosure may exist in different polymorphic forms. Although not explicitly indicated in the above formula, such forms are intended to be included within the scope of the present disclosure.
The disclosure also includes within its scope pro-drug forms of the compounds of formula I and its various sub-scopes and sub-groups.

xii) U.S. Patent Application Publication No. 20090018148

In one aspect the disclosure provides compounds having formula I, their enantiomers, diastereoisomers and mixtures thereof (including all possible mixtures of stereoisomers), or pharmaceutically acceptable salts thereof,

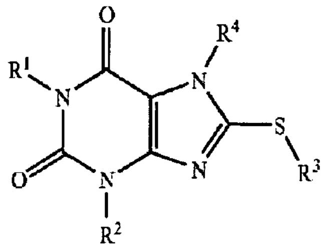

(I)

wherein

R1 is hydrogen or C1-6 alkyl;

R2 is hydrogen or C1-4 alkyl;

R3 is a group of formula -CHR5R6 or a benzyl group;

R4 is C1-8 alkyl optionally substituted by alkoxycarbonyl, C3-6 cycloalkyl, aryl or heterocycle;

R5 is C2-4 alkyl;

R6 is C2-4 alkyl, amido or -COOR7;

R7 is C1-4 alkyl;

In one aspect, the disclosure provides compounds:

When R1 is hydrogen, R2 is methyl, R3 is -CHRSR6, R6 is ethoxycarbonyl and

R5 is ethyl, then R4 is different from methyl, n-propyl, i-propyl, n-pentyl, n-heptyl, 3-bromobenzyl, 4-chlorobenzyl, 4-methylbenzyl or 2-phenylethyl;

When R1 is hydrogen, R2 is methyl, R3 is benzyl, then R4 is different from i-propyl, n-butyl, 3-methylbutyl, benzyl, phenylethyl-, or 3-phenylpropyl;

When R1 and R2 are methyl, R3 is benzyl, R4 is different from methyl, 3-methylbutyl, benzyl, 3-phenylpropyl or 4-chlorophenylmethyl;

Finally 8-(2-chloro-benzylsulfanyl)-3-methyl-7-octyl-3,7-dihydro-purine-2,6-dione is considered.

Usually when R3 is a benzyl group, then R4 is C1-8 alkyl optionally substituted by alkoxycarbonyl.

Usually when R3 is a group of formula -CHRSR6, then R4 is C1-8 alkyl optionally substituted by C3-6 cycloalkyl, aryl or heterocycle.
The term "alkyl", as used herein, is a group which represents saturated, monovalent hydrocarbon radicals having straight (unbranched) or branched moieties, or combinations thereof, and containing 1-8 carbon atoms, preferably 1-6 carbon atoms; more preferably alkyl groups have 1-4 carbon atoms. Alkyl moieties may optionally be substituted by

1 to 5 substituents independently selected from the group consisting of hydroxy, alkoxy, cyano, ethynyl, alkoxycarbonyl, acyl, aryl or heterocycle. Alkyl moieties may be optionally substituted by a cycloalkyl as defined hereafter. Preferred alkyl groups according to the present disclosure are methyl, cyanomethyl, ethyl, 2-ethoxy-2-oxoethyl, 2-methoxyethyl, n-propyl, 2-oxopropyl, 3-hydroxypropyl, 2-propynyl, n-butyl, i-butyl, n-pentyl, 3-pentyl, n-hexyl, cyclohexylmethyl, benzyl, 2-bromobenzyl, 3-bromobenzyl, 4-bromobenzyl, 3-methoxybenzyl, 3-nitrobenzyl, 3-aminobenzyl, 4-(aminosulfonyl)benzyl, 1-phenylethyl, 2-phenylethyl, (3,5-dimethylisoxazol-4-yl)methyl or (5-nitro-2-furyl)methyl. More preferred alkyl groups are methyl, ethyl, cyanomethyl, 2-methoxyethyl, n-propyl, 3-hydroxypropyl, 2-propynyl, n-butyl, 3-pentyl, n-hexyl, benzyl, 3-bromobenzyl, 3-methoxybenzyl, 3-nitrobenzyl, 3-aminobenzyl, (3,5-dimethylisoxazol-4-yl)methyl or (5-nitro-2-furyl)methyl. Most preferred alkyl groups are methyl, ethyl, 3-methoxybenzyl, 3-nitrobenzyl or (5-nitro-2-furyl)methyl.

The term "cycloalkyl", as used herein, represents a monovalent group of 3 to 8, preferably 3 to 6 carbon atoms derived from a saturated cyclic hydrocarbon, which may be substituted by any suitable group including but not limited to one or more moieties selected from groups as described above for the alkyl groups. Preferred cycloalkyl group according to the present disclosure is cyclohexyl.

The term "aryl" as used herein, is defined as a phenyl group optionally substituted by 1 to 4 substituents independently selected from halogen, amino, nitro, alkoxy or aminosulfonyl. Preferred aryl groups are phenyl, 2-bromophenyl, 3-bromophenyl, 4-bromophenyl, 3-methoxyphenyl, 3-nitrophenyl, 3-aminophenyl or 4-(aminosulfonyl)phenyl.

The term "phenyl", as used herein, represents an aromatic hydrocarbon group of formula $-C6H5$.

The term "benzyl group", as used herein, represents a group of formula -CH2-aryl. Preferred benzyl groups are benzyl, 2-bromobenzyl, 3-bromobenzyl, 4-bromobenzyl, 3-methoxybenzyl, 3-nitrobenzyl, 3-aminobenzyl or 4-(aminosulfonyl)benzyl. More preferred benzyl groups are benzyl, 3-bromobenzyl, 3-methoxybenzyl, 3-nitrobenzyl or 3-aminobenzyl. In some instances alkyl groups are 3-methoxybenzyl or 3-nitrobenzyl.

The term "halogen", as used herein, represents an atom of fluorine, chlorine, bromine, or iodine. In some instances the halogen is bromine.

The term "hydroxy", as used herein, represents a group of formula -OH.

The term "cyano", as used herein, represents a group of formula -CN.

The term "amino", as used herein, represents a group of formula -NH2.

The term "ethynyl", as used herein, represents a group of formula $-C\equiv CH$.

The term "alkoxy", as used herein, represents a group of formula -ORa wherein Ra is an alkyl group, as defined above. In some instances the alkoxy group is methoxy.

The term "nitro", as used herein, represents a group of formula -NO2.

The term "amido", as used herein, represents a group of formula -C(=O)NH2.

The term "acyl", as used herein, represents a group of formula -C(=O)Rb wherein Rb is an alkyl group, as defined here above. In some instances the acyl group is acetyl (-C(=O)Me).

The term "alkoxycarbonyl (or ester)", as used herein, represents a group of formula -COORc wherein Rc is an alkyl group; with the proviso that Rc does not represent an alkyl alpha-substituted by hydroxy. In some instances the alkoxycarbonyl group is ethoxycarbonyl.

The term "heterocycle", as used herein, represents a 5-membered ring containing one or two heteroatoms selected from O or N. The heterocycle may be substituted by one or two C1-4 alkyl or nitro. In some instances the heterocycles are (3,5-dimethylisoxazol-4-yl) or (5-nitro-2-furyl). Most preferred heterocycle is (5-nitro-2-furyl).

Generally R1 is hydrogen or C1-6 alkyl. Usually R1 is hydrogen or C1-6 alkyl optionally substituted by hydroxy, alkoxy, cyano, ethynyl, alkoxycarbonyl or acyl. In some instances R1 is hydrogen, methyl, cyanomethyl, 2-ethoxy-2-oxoethyl, 2-methoxyethyl, n-propyl, 2-oxopropyl, 3-hydroxypropyl, 2-propynyl, n-pentyl or n-hexyl. In some instances R1 is hydrogen, methyl, cyanomethyl, 2-methoxyethyl, n-propyl, 3-hydroxypropyl or 2-propynyl. In some instances R1 is hydrogen.

Generally R2 is hydrogen or C1-4 alkyl. Usually R2 is hydrogen or unsubstituted C1-4 alkyl. In some instances R2 is hydrogen, methyl or n-butyl. In some instances, R2 is methyl.

Generally R3 is a group of formula -CHR5R6 or a benzyl group. In some instances R3 is 3-pentyl, 1-(aminocarbonyl)propyl, 1-(ethoxycarbonyl)propyl or 3-bromobenzyl. In some instances R3 is 1-(ethoxycarbonyl)propyl.

Generally R4 is C1-8 alkyl optionally substituted by alkoxycarbonyl, C3-6 cycloalkyl, aryl or heterocycle. Usually R4 is C 1-8 alkyl optionally substituted by cyclohexyl, phenyl, bromophenyl, aminophenyl, methoxyphenyl, nitrophenyl, aminosulfonylphenyl, 3,5-dimethylisoxazol-4-yl, 5-nitro-2-furyl or ethoxycarbonyl. In some instances R4 is n-butyl, i-butyl, n-pentyl, n-hexyl, cyclohexylmethyl, benzyl, 2-bromobenzyl, 3-bromobenzyl, 4-bromobenzyl, 3-methoxybenzyl, 3-nitrobenzyl, 3-aminobenzyl, 4-(aminosulfonyl)benzyl, 1-phenylethyl, 2-phenylethyl, (3,5-dimethylisoxazol-4-yl)methyl, (5-nitro-2-furyl)methyl or 1-(ethoxycarbonyl)propyl. In some instances R4 is n-butyl, n-hexyl, benzyl, 3-bromobenzyl, 3-methoxybenzyl, 3-nitrobenzyl, 3-aminobenzyl, (3,5-dimethylisoxazol-4-yl)methyl, (5-nitro-2-furyl)methyl or 1-(ethoxycarbonyl)propyl. In some instances R4 is 3-methoxybenzyl, 3-nitrobenzyl or (5-nitro-2-furyl)methyl.

Generally R5 is C2-4 alkyl. Usually R5 is unsubstituted C2-4 alkyl. In some instances R5 is ethyl.

Generally R6 is C2-4 alkyl, amido or -COOR7. Usually R6 is unsubstituted C2-4 alkyl, amido or -COOR7. In some instances R6 is ethyl, amido or ethoxycarbonyl. In some instances R6 is ethoxycarbonyl.
Generally R7 is C1-4 alkyl. Usually R7 is unsubstituted C1-4 alkyl. In some instances, R7 is ethyl.
Usually the disclosure provides compounds having formula I, their enantiomers, diastereoisomers and mixtures thereof (including all possible mixtures of stereoisomers), or pharmaceutically acceptable salts thereof,

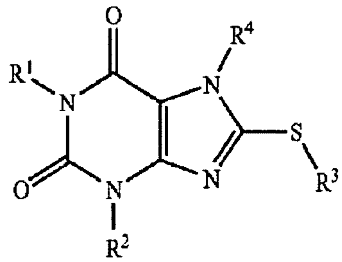

(I)

wherein

R1 is hydrogen, C1-6 alkyl optionally substituted by hydroxy, alkoxy, cyano, ethynyl, alkoxycarbonyl or acyl;

R2 is hydrogen or unsubstituted C1-4 alkyl;

R3 is a group of formula -CHR5R6 or a benzyl group;

R4 is C1-8 alkyl optionally substituted by cyclohexyl, phenyl, bromophenyl, aminophenyl, methoxyphenyl, nitrophenyl, aminosulfonylphenyl, 3,5-dimethylisoxazol-4-yl, 5-nitro-2-furyl or ethoxycarbonyl;

R5 is unsubstituted C2-4 alkyl;

R6 is unsubstituted C2-4 alkyl, amido or -COOR7;

R7 is unsubstituted C1-4 alkyl;

with the proviso that when R1 is hydrogen, R2 is methyl, R3 is -CHR5R6, R6 is ethoxycarbonyl and R5 is ethyl, then R4 is different from n-propyl, i-propyl, n-pentyl, n-heptyl, 3-bromobenzyl, 4-chlorobenzyl, 4-methylbenzyl or 2-phenylethyl.

In the above instance, sometimes, when R3 is a benzyl group, then R4 is C1-8 alkyl optionally substituted by alkoxycarbonyl.
In the above instance, sometimes, when R3 is a group of formula -CHR5R6, then R4 is C1-8 alkyl optionally substituted by C3-6 cycloalkyl, aryl or heterocycle.
In one instance,
R1 is hydrogen, methyl, cyanomethyl, 2-ethoxy-2-oxoethyl, 2-methoxyethyl, n-propyl, 2-oxopropyl, 3-hydroxypropyl, 2-propynyl, n-pentyl or n-hexyl;
R2 is hydrogen, methyl or n-butyl;
R3 is 3-pentyl, 1-(aminocarbonyl)propyl, 1-(ethoxycarbonyl)propyl or 3-bromobenzyl;
R4 is n-butyl, i-butyl, n-pentyl, n-hexyl, cyclohexylmethyl, benzyl, 2-bromobenzyl, 3-bromobenzyl, 4-bromobenzyl, 3-methoxybenzyl, 3-nitrobenzyl, 3-aminobenzyl, 4-(aminosulfonyl)benzyl, 1-phenylethyl, 2-phenylethyl, (3,5-dimethylisoxazol-4-yl)methyl, (5-nitro-2-furyl)methyl or 1-(ethoxycarbonyl)propyl;
with the proviso that when R1 is hydrogen, R2 is methyl and R3 is 1-(ethoxycarbonyl)propyl, then R4 is different from n-pentyl, 3-bromobenzyl or 2-phenylethyl.
In the above instance, sometimes, when R3 is 3-bromobenzyl, then R4 is C1-8 alkyl optionally substituted by alkoxycarbonyl.
In the above instance, sometimes, when R3 is 3-pentyl, 1-(aminocarbonyl)propyl or 1-(ethoxycarbonyl)propyl, then R4 is different from 1-(ethoxycarbonyl)propyl.
In a more preferred instance, R1 is hydrogen, methyl, cyanomethyl, 2-methoxyethyl, n-propyl, 3-hydroxypropyl or 2-propynyl;

R2 is methyl;
R3 is 3-pentyl, 1-(aminocarbonyl)propyl, 1-(ethoxycarbonyl)propyl or 3-bromobenzyl;
R4 is n-butyl, n-hexyl, benzyl, 3-bromobenzyl, 3-methoxybenzyl, 3-nitrobenzyl, 3-aminobenzyl, (3,5-dimethylisoxazol-4-yl)methyl, (5-nitro-2-furyl)methyl or 1-(ethoxycarbonyl)propyl;
with the proviso that when R1 is hydrogen, R2 is methyl and R3 is 1-(ethoxycarbonyl)propyl, then R4 is different from 3-bromobenzyl.

In the above instance, sometimes, when R3 is 3-bromobenzyl, then R4 is 1-(ethoxycarbonyl)propyl;
In the above instance, sometimes, when R3 is 3-pentyl, 1-(aminocarbonyl)propyl or 1-(ethoxycarbonyl)propyl, then R4 is different from 1-(ethoxycarbonyl)propyl;
In one instance, R1 is hydrogen; R2 is methyl; R3 is 1-(ethoxycarbonyl)propyl; and R4 is 3-methoxybenzyl, 3-nitrobenzyl or (5-nitro-2-furyl)methyl.

A further instance consists in compounds wherein R2 is methyl, R3 is a group of formula -CHR5R6 with R5 being C2-4 alkyl, R6 being amido or -COOR7 and R7 being methyl or ethyl.

In some instances, compounds are ethyl 2-[(7-benzyl-1,3-dimethyl-2,6-dioxo-2,3,6,7-tetrahydro-1H-purin-8-yl)thio]butanoate; ethyl 2-{[7-(3-bromobenzyl)-1-(2-ethoxy-2-oxoethyl)-3-methyl-2,6-dioxo-2,3,6,7-tetrahydro-1H-purin-8-yl]thio}butanoate; ethyl 2-{[7-(3-bromobenzyl)-1-(2-methoxyethyl)-3-methyl-2,6-dioxo-2,3,6,7-tetrahydro-1H-purin-8-yl]thio}butanoate; ethyl 2-{[7-(3-bromobenzyl)-2,6-dioxo-2,3,6,7-tetrahydro-1H-purin-8-yl]thio}butanoate; ethyl 2-{[7-(3-bromobenzyl)-1,3-dimethyl-2,6-dioxo-2,3,6,7-tetrahydro-1H-purin-8-yl]thio}butanoate; ethyl 2-{[7-(2-bromobenzyl)-1,3-dimethyl-2,6-dioxo-2,3,6,7-tetrahydro-1H-purin-8-yl]thio}butanoate; ethyl 2-{[7-(3-bromobenzyl)-1-(cyanomethyl)-3-methyl-2,6-dioxo-2,3,6,7-tetrahydro-1H-purin-8-yl]thio}butanoate; ethyl 2-{[7-(3-bromobenzyl)-3-methyl-2,6-dioxo-1-propyl-2,3,6,7-tetrahydro-1H-purin-8-yl]thio}butanoate; ethyl 2-{[7-(3-bromobenzyl)-3-methyl-2,6-dioxo-1-(2-oxopropyl)-2,3,6,7-tetrahydro-1H-purin-8-yl]thio}butanoate; ethyl 2-{[7-(3-bromobenzyl)-1-(3-hydroxypropyl)-3-methyl-2,6-dioxo-2,3,6,7-tetrahydro-1H-purin-8-yl]thio}butanoate; ethyl 2-{[7-(3-bromobenzyl)-3-methyl-2,6-dioxo-1-(2-propynyl)-2,3,6,7-tetrahydro-1H-purin-8-yl]thio} butanoate; ethyl 2-{[7-(3-methoxybenzyl)-3-methyl-2,6-dioxo-2,3,6,7-tetrahydro-1H-purin-8-yl]thio}butanoate; ethyl 2-{[3-methyl-7-(3-nitrobenzyl)-2,6-dioxo-2,3,6,7-tetrahydro-1H-purin-8-yl]thio}butanoate; ethyl 2-{[7-(3-aminobenzyl)-3-methyl-2,6-dioxo-2,3,6,7-tetrahydro-1H-purin-8-yl]thio}butanoate; ethyl 2-({7-[4-(aminosulfonyl)benzyl]-3-methyl-2,6-dioxo-2,3,6,7-tetrahydro-1H-purin-8-yl}thio)butanoate; ethyl 2-{[7-(4-bromobenzyl)-1,3-dimethyl-2,6-dioxo-2,3,6,7-tetrahydro-1H-purin-8-yl]thio}butanoate; ethyl 2-{[7-(cyclohexylmethyl)-1,3-dimethyl-2,6-dioxo-2,3,6,7-tetrahydro-1H-purin-8-yl]thio}butanoate; ethyl 2-{[1,3-dimethyl-2,6-dioxo-7-(1-phenylethyl)-2,3,6,7-tetrahydro-1H-purin-8-yl]thio}butanoate; ethyl 2-{[1,3-dimethyl-2,6-dioxo-7-(2-phenylethyl)-2,3,6,7-tetrahydro-1H-purin-8-yl]thio}butanoate; ethyl 2-({7-[(3,5-dimethylisoxazol-4-yl)methyl]-3-methyl-2,6-dioxo-2,3,6,7-tetrahydro-1H-purin-8-yl}thio)butanoate; ethyl 2-({3-methyl-7-[(5-nitro-2-furyl)methyl]-2,6-dioxo-2,3,6,7-tetrahydro-1H-purin-8-yl}thio)butanoate; ethyl 2-[(7-butyl-3-methyl-2,6-dioxo-2,3,6,7-tetrahydro-1H-purin-8-yl)thio]butanoate; ethyl 2-{[7-(3-bromobenzyl)-2,6-dioxo-2,3,6,7-tetrahydro-1H-purin-8-yl]thio}butanoate; ethyl 2-[(1,7-dihexyl-3-methyl-2,6-dioxo-2,3,6,7-tetrahydro-1H-purin-8-yl)thio]butanoate; ethyl 2-[(7-hexyl-3-methyl-2,6-dioxo-2,3,6,7-tetrahydro-1H-purin-8-yl)thio]butanoate; ethyl 2-[(3-methyl-2,6-dioxo-1,7-dipentyl-2,3,6,7-tetrahydro-1H-purin-8-yl)thio]butanoate; 2-{[7-(3-bromobenzyl)-3-methyl-2,6-dioxo-2,3,6,7-tetrahydro-1H-purin-8-yl]thio}butanamide; 2-[(7-butyl-3-methyl-2,6-dioxo-2,3,6,7-tetrahydro-1H-purin-8-yl)thio]butanamide; 7-(3-bromobenzyl)-8-[(1-ethylpropyl)thio]-3-methyl-3,7-dihydro-1H-purine-2,6-dione; ethyl 2- {8-[(3-bromobenzyl)thio]-1,3-dimethyl-2,6-dioxo-1,2,3,6-tetrahydro-7H-purin-7-yl}butanoate; and ethyl 2-[(7-isobutyl-3-methyl-2,6-dioxo-2,3,6,7-tetrahydro-1H-purin-8-yl)thio]butanoate.

In some instances compounds are: ethyl 2-[(7-benzyl-1,3-dimethyl-2,6-dioxo-2,3,6,7-tetrahydro-1H-purin-8-yl)thio]butanoate; ethyl 2-{[7-(3-bromobenzyl)-1-(2-methoxyethyl)-3-methyl-2,6-dioxo-2,3,6,7-tetrahydro-1H-purin-8-yl]thio}butanoate; ethyl 2-{[7-(3-bromobenzyl)-1,3-dimethyl-2,6-dioxo-2,3,6,7-tetrahydro-1H-purin-8-yl]thio}butanoate; ethyl 2-{[7-(3-bromobenzyl)-1-(cyanomethyl)-3-methyl-2,6-dioxo-2,3,6,7-tetrahydro-1H-purin-8-yl]thio}butanoate; ethyl 2-{[7-(3-bromobenzyl)-3-methyl-2,6-dioxo-1-propyl-2,3,6,7-tetrahydro-1H-purin-8-yl]thio}butanoate; ethyl 2-{[7-(3-bromobenzyl)-1-(3-hydroxypropyl)-3-methyl-2,6-dioxo-2,3,6,7-tetrahydro-1H-purin-8-yl]thio}butanoate; ethyl 2-{[7-(3-bromobenzyl)-3-methyl-2,6-dioxo-1-(2-propynyl)-2,3,6,7-tetrahydro-1H-purin-8-yl]thio}butanoate; ethyl 2-{[7-(3-methoxybenzyl)-3-methyl-2,6-dioxo-2,3,6,7-tetrahydro-1H-purin-8-yl]thio}butanoate; ethyl 2-{[3-methyl-7-(3-nitrobenzyl)-2,6-dioxo-2,3,6,7-tetrahydro-1H-purin-8-yl]thio}butanoate; ethyl 2-{[7-(3-aminobenzyl)-3-methyl-2,6-dioxo-2,3,6,7-tetrahydro-1H-purin-8-yl]thio}butanoate; ethyl 2-({7-[(3,5-dimethylisoxazol-4-yl)methyl]-3-methyl-2,6-dioxo-2,3,6,7-tetrahydro-1H-purin-8-yl}thio)butanoate; ethyl 2-({3-methyl-7-[(5-nitro-2-furyl)methyl]-2,6-dioxo-2,3,6,7-tetrahydro-1H-purin-8-yl}thio)butanoate; ethyl 2-[(7-butyl-3-methyl-2,6-dioxo-2,3,6,7-tetrahydro-1H-purin-8-yl)thio]butanoate; ethyl 2-[(7-hexyl-3-methyl-2,6-dioxo-2,3,6,7-tetrahydro-1H-purin-8-yl)thio]butanoate; 2-{[7-(3-bromobenzyl)-3-methyl-2,6-dioxo-2,3,6,7-tetrahydro-1H-purin-8-yl]thio}butanamide; 7-(3-bromobenzyl)-8-[(1-ethylpropyl)thio]-3-methyl-3,7-dihydro-1H-purine-2,6-dione; and ethyl 2-{8-[(3-bromobenzyl)thio]-1,3-dimethyl-2,6-dioxo-1,2,3,6-tetrahydro-7H-purin-7-yl}butanoate.

In some instances compounds are: ethyl 2-{[7-(3-methoxybenzyl)-3-methyl-2,6-dioxo-2,3,6,7-tetrahydro-1H-purin-8-yl]thio}butanoate; ethyl 2-{[3-methyl-7-(3-nitrobenzyl)-2,6-dioxo-2,3,6,7-tetrahydro-1H-purin-8-yl]thio}butanoate; and ethyl 2-({3-methyl-7-[(5-nitro-2-furyl)methyl]-2,6-dioxo-2,3,6,7-tetrahydro-1H-purin-8-yl}thio)butanoate.

The acid addition salt form of a compound of formula I that occurs in its free form as a base can be obtained by treating the free base with an appropriate acid such as an inorganic acid, for example, a hydrohalic such as hydrochloric or hydrobromic, sulfuric, nitric, phosphoric and the like; or an organic acid, such as, for example, acetic, trifluoroacetic, hydroxyacetic, propanoic, lactic, pyruvic, malonic, succinic, maleic, fumaric, malic, tartaric, citric, methanesulfonic, ethanesulfonic, benzenesulfonic, p-toluenesulfonic, cyclamic, salicylic, p-aminosalicylic, pamoic and the like.

The compounds of formula I containing acidic protons may be converted into their therapeutically active, non-toxic base addition salt forms, e.g. metal or amine salts, by treatment with appropriate organic and inorganic bases. Appropriate base salt forms include, for example, ammonium salts, alkali and earth alkaline metal salts, e.g. lithium, sodium, potassium, magnesium, calcium salts and the like, salts with organic bases, e.g. N-methyl-D-glucamine, hydrabamine salts, and salts with amino acids such as, for example, arginine, lysine and the like.

Conversely said salt forms can be converted into the free forms by treatment with an appropriate base or acid.

Compounds of the formula I and their salts can be in the form of a solvate, which is included within the scope of the present disclosure. Such solvates include for example hydrates, alcoholates and the like.

Many of the compounds of formula I and some of their intermediates have at least one stereogenic center in their structure. This stereogenic center may be present in a R or a S configuration, said R and S notation is used in correspondence with the rules described in Pure Appl. Chem., 45 (1976) 11-30.

The disclosure also relates to all stereoisomeric forms such as enantiomeric and diastereoisomeric forms of the compounds of formula I or mixtures thereof (including all possible mixtures of stereoisomers).

With respect to the present disclosure reference to a compound or compounds is intended to encompass that compound in each of its possible isomeric forms and mixtures thereof, unless the particular isomeric form is referred to specifically.

Compounds according to the present disclosure may exist in different polymorphic forms. Although not explicitly indicated in the above formula, such forms are intended to be included within the scope of the present disclosure.

xiii) U.S. Patent 7,465,549

In some instances, the compound includes optionally substituted N-alkylated 2-oxo-pyrrolidine derivatives. In some instances, those compounds are alkyl amides derivatives substituted on the positions 4 and/or 5 of the pyrrolidone ring. Examples of optionally substituted N-alkylated 2-oxo-pyrrolidine derivatives include, but are not limited to, compounds such as (2S)-2-[(4S)-4-(2,2-difluorovinyl)-2-oxopyrrolidinyl]butanamide, (2S)-2-[(4R)-2-oxo-4-propylpyrrolidinyl]butanamide, (2S)-2-[(4S)-2-oxo-4-propylpyrrolidinyl]butanamide, and (2S)-2-[4-(3-azidophenyl)-2-oxopyrrolidin-1-yl]butanamide.

In some instances, the compounds further include optionally substituted N-alkylated 2-oxo-piperidinyl derivatives. In some instances, those compounds are alkyl amides derivatives substituted on the position 4 and/or 5 and/or 6 of the 2-oxo-piperidinyl ring. Examples of optionally substituted N-alkylated 2-oxo-pyrrolidine derivatives include, but are not limited to, compounds such as those referred to in international patent application PCT/EP02/05503 such as (2S)-2-[5-(iodomethyl)-2-oxo-1-piperidinyl]butanamide, (2S)-2-[5-(azidomethyl)-2-oxo-1-piperidinyl]butanamide, 2-(2-oxo-5-phenyl-1-piperidinyl]butanamide, (2S)-2-[4-(iodomethyl)-2-oxo-1-piperidinyl]butanamide, and (2S)-2-[4-(2-fluoro-2-methylpropyl)-2-oxo-1-pyrrolidinyl]butanamide.

In some instances, the compounds include any acetam compound of formula I, in racemic or isomeric form, or a pharmaceutically acceptable salt thereof,

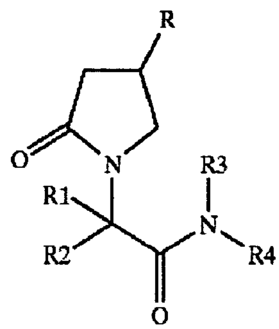

(I)

wherein

R represents hydrogen or hydroxy;

R1 and R2 represent independently hydrogen or an alkyl group of 1-4 carbon atoms; and

R3 and R4 represent independently hydrogen, an alkyl group of 1-4 carbon atoms or -(CH2)n-NR5R6 wherein n is 1, 2 or 3 and R5 and R6 represent independently hydrogen or an alkyl group of 1-4 carbon atoms.

An example of such an acetam compound includes, but is not limited to, a compound of formula I wherein R, R1, R2, R3 and R4 are hydrogen, 2-oxo-pyrrolidineacetamide, known by the generic name piracetam as described in UK Patents Nos. 1,039,113 and 1,309,692.

In some instances, the compounds also include optionally substituted N-alkylated 2-oxo-azepanyl derivatives. Preferably, those compounds are alkyl amides derivatives substituted on the positions 4 and/or 5 and/or 6 and/or 7 of the 2-oxo-azepanyl ring. Examples of optionally substituted N-alkylated 2-oxo-azepanyl derivatives include, but are not limited to, compounds such as those referred to in international patent application PCT/EP02/05503 such as 2-[5-(iodomethyl)-2-oxo-1-azepanyl]butanamide.

xiv) U.S. Patent Application Publication No. 2006258704

This disclosure provides novel compounds of the formula I

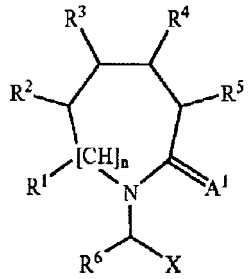

(I)

wherein

n represents 0 or 1 whereby R<1 > is not existent when n=0 and R<1 > is existent when n=1;

A<1 > represents an oxygen or a sulfur atom;

X is -CONR<7> R<8> , -COOR<9> , -CO-R<10 > or CN;

R< 1 > when existent, R<2> , R<3> , R<4 > and R<5 > are the same or different and each is independently hydrogen, halogen, hydroxy, thiol, amino, nitro, nitrooxy, cyano, azido, carboxy, amido, sulfonic acid, sulfonamide, alkyl, alkenyl, alkynyl, ester, ether, aryl, heterocycle, or an oxy derivative, thio derivative, amino derivative, acyl derivative, sulfonyl derivative or sulfinyl derivative,

provided that at least one of the substituents R chosen from R<1 > when existent, R<2> , R<3> , R<4 > or R<5 > is not hydrogen;

R<6 > is hydrogen, alkyl, aryl or -CH2-R<6a > wherein R<6a > is aryl, heterocycle, halogen, hydroxy, amino, nitro or cyano;

R<7> , R<8 > and R<9 > are the same or different and each is independently hydrogen, hydroxy, alkyl, aryl, heterocycle or an oxy derivative; and

R<10 > is hydrogen, hydroxy, thiol, halogen, alkyl, aryl, heterocycle or a thio derivative;

their pharmaceutically acceptable salts, geometrical Isomers (including cis and trans, Z and E isomers), enantiomers, diastereoisomers and mixtures thereof (including all possible mixtures of stereoisomers).

In the above formula, at least one substituent R<1 > to R<5 > is different from hydrogen. Some non-substituted compounds are referred to in U.S. Pat. Nos. 5,468,733 and 5,516,759. U.S. Pat. No. 5,468,733 discloses non-ring

substituted 2-oxo-1-pyrrolidinyl and 2-oxo-1-piperidinyl derivatives as inhibitors of the oncogene Ras protein. In particular, these compounds block the ability of Ras to transform normal cells to cancer cells, and therefore can be included in several chemotherapeutic compositions for treating cancer.

US Patent No. 5,516,759 discloses non-ring substituted 2-oxo-1-pyrrolidinyl, 2-oxo-1-piperidinyl and azepanyl derivatives present at the N-terminus of dodecapeptides possessing LHRH (luteinizing hormone-releasing hormone) antagonistic activity. Such LHRH antagonists are useful in the treatment of a variety of conditions in which suppression of sex steroids plays a key role including contraception, delay of puberty, treatment of benign prostatic hyperplasia a.o.

In the definitions set forth below, unless otherwise stated, R<11 > and R<12 > are the same or different and each is independently amido, alkyl, alkenyl, alkynyl, acyl, ester, ether, aryl, aralkyl, heterocycle or an oxy derivative, thio derivative, acyl derivative, amino derivative, sulfonyl derivative, or sulfinyl derivative, each optionally substituted with any suitable group, including, but not limited to, one or more moieties selected from lower alkyl or other groups as described below as substituents for alkyl.

The term "oxy derivative", as used herein, is defined as including -O-R<11 > groups wherein R<11 > is as defined above except for "oxy derivative". Non-limiting examples are alkoxy, alkenyloxy, alkynyloxy, acyloxy, oxyester, oxyamido, alkylsulfonyloxy, alkylsulfinyloxy, arylsulfonyloxy, arylsulfinyloxy, aryloxy, aralkoxy or heterocyclooxy such as pentyloxy, allyloxy, methoxy, ethoxy, phenoxy, benzyloxy, 2-naphthyloxy, 2-pyridyloxy, methylenedioxy, carbonate.

The term "thio derivative", as used herein, is defined as including -S-R<11 > groups wherein R<11 > is as defined above except for "thio derivative". Non-limiting examples are alkylthio, alkenylthio, alkynylthio and arylthio.

The term "amino derivative", as used herein, is defined as including -NHR<11 > or -NR<11> R<12 > groups wherein R<11 > and R<12 > are as defined above. Non-limiting examples are mono- or di-alkyl-, alkenyl-, alkynyl- and arylamino or mixed amino.

The term "acyl derivative", as used herein, represents a radical derived from carboxylic acid and thus is defined as including groups of the formula R<11> -CO-, wherein R<11 > is as defined above and may also be hydrogen. Preferred are acyl derivatives of formula -COR<11 > wherein R<11 > is selected from hydrogen, C1-12 alkyl, C2-12 alkenyl, C2-12 alkenyl, heterocyle and aryl. Non-limiting examples are formyl, acetyl, propionyl, isobutyryl, valeryl, lauroyl, heptanedioyl, cyclohexanecarbonyl, crotonoyl, fumaroyl, acryloyl, benzoyl, naphthoyl, furoyl, nicotinoyl, 4-carboxybutanoyl, oxalyl, ethoxalyl, cysteinyl, oxamoyl.

The term "sulfonyl derivative", as used herein, is defined as including a group of the formula -SO-R<11> , wherein R<11 > is as defined above except for "sulfonyl derivative". Non-limiting examples are alkylsulfonyl, alkenylsulfonyl, alkynylsulfonyl and arylsulfonyl.

The term "sulfinyl derivative", as used herein, is defined as including a group of the formula -SO-R<11> , wherein R<11 > is as defined above except for "sulfinyl derivative". Non-limiting examples are alkylsulfinyl, alkenylsulfinyl, alkynylsulfinyl and arylsulfinyl.

The term "alkyl", as used herein, is defined as including saturated, monovalent hydrocarbon radicals having straight, branched or cyclic moieties or combinations thereof and generally containing 1-20 carbon atoms, most often 1 to 12 carbon atoms, preferably 1-7 carbon atoms for non-cyclic alkyl and 3-7 carbon atoms for cycloalkyl (in these two preferred cases, unless otherwise specified, "lower alkyl"), each optionally substituted by, preferably 1 to 5, substituents independently selected from the group consisting of halogen, hydroxy, thiol, amino, nitro, cyano, thiocyanato, acyl, acyloxy, sulfonyl derivative, sulfinyl derivative, alkylamino, carboxy, ester, ether, amido, azido, cycloalkyl, sulfonic acid, sulfonamide, thio derivative, alkylthio, oxyester, oxyamido, heterocycle, vinyl, alkoxy (preferably C1-5), aryloxy (preferably C6-10) and aryl(preferably C6-10).

In some instances are alkyl groups containing 1 to 7 carbon atoms, each optionally substituted by one or more substituents selected from hydroxy, halogen, cyano, thiocyanato, alkoxy, azido, alkylthio, cyclopropyl, acyl and phenyl. Most preferred are C1-4 alkyl and C3-7 cycloalkyl, each optionally substituted by one or more hydroxy, halogen, lower alkyl or/and azido.

In some instances are alkyl groups are hydroxymethyl, propyl, butyl, 2,2,2-trifluoroethyl, 2-bromo-2,2-difluoroethyl, 2-chloro-2,2-difluoroethyl, 3,3,3-trifluoropropyl, cyclopropylmethyl, iodomethyl, azidomethyl, 2,2-difluoropropyl, 2-iodo-2,2-difluoroethyl.

The term "lower alkyl", as used herein, and unless otherwise specified, refers to C1 to C7 saturated straight, branched or cyclic hydrocarbon. Non limiting examples are methyl, ethyl, propyl, isopropyl, butyl, tertiobutyl, pentyl, cyclopropyl, cyclopentyl, isopentyl, neopentyl, hexyl, isohexyl, cyclohexyl, 3-methypentyl, 2,2-dimethylbutyl, optionally substituted with any suitable group, including but not limited to one or more moieties selected from groups as described above for the alkyl groups. Preferably, lower alkyl is methyl.

The term "alkenyl", as used herein, is defined as including both branched and unbranched, unsaturated hydrocarbon radicals having at least one double bond, and being optionally substituted by at least one substituent selected from the group consisting of halogen, hydroxy, thiol, amino, thiocyanato, azido, alkylthio, cycloalkyl, acyl, nitro, cyano, aryl and heterocycle.

In some instances are alkenyl groups are C2-C12 alkenyls, especially C2-6alkenyls, such as ethenyl (=vinyl),

1-methyl-1-ethenyl, 2,2-dimethyl-1-ethenyl, 1-propenyl, 2-propenyl (=allyl), 1-butenyl, 2-butenyl, 3-butenyl, 4-pentenyl, 1-methyl-4-pentenyl, 3-methyl-1-pentenyl, 1-hexenyl, 2-hexenyl and the like, optionally being substituted by one or more substituents selected from halogen, cyano, thiocyanato, azido, alkylthio, cycloalkyl, phenyl and acyl. Most prefered is vinyl, optionally substituted by one or more halogen or/and lower alkyl, and especially 2,2-difluorovinyl, 2,2-dibromovinyl and 2,2-dichlorovinyl.

The term "alkynyl" as used herein, is defined as including a monovalent branched or unbranched hydrocarbon radical containing at least one carbon-carbon triple bond, for example ethynyl, 2-propynyl (=propargyl), and the like, and being optionally substituted by at least one substituent selected from the group consisting of halogen, hydroxy, thiol, amino, nitro, cyano, aryl, heterocycle, thiocyanato, azido, alkylthio, alkyl and acyl.

In some instances are alkynyl groups are C2-12 alkynyl, especially C2-6 alkynyl, optionally being substituted by one or more substituents selected from halogen, cyano, thiocyanato, azido, alkylthio, acyl, aryl such as phenyl and alkyl, preferably cycloalkyl.

In some instances are ethynyl, propynyl and butynyl, optionally substituted by lower alkyl or/and halogen, and especially 1-propynyl, cyclopropylethynyl, 3-methyl-1-butynyl and 3,3,3-trifluoro-1-propynyl.

When present as bridging groups, alkyl, alkenyl and alkynyl represent straight- or branched chains, C1-12, preferably C1-4-alkylene or C2-12-, preferably C2-4-alkenylene or -alkynylene moieties respectively.

Groups where branched derivatives are conventionally qualified by prefixes such as "n", "sec", "iso" and the like (e.g. "n-propyl", "sec-butyl") are in the n-form unless otherwise stated.

The term "aryl", as used herein, is defined as including an organic radical derived from an aromatic hydrocarbon consisting of at least one ring, most often 1 to 3 rings and generally containing 6-30 carbon atoms by removal of one hydrogen, such as phenyl and naphthyl, each optionally substituted by one or more substituents independently selected from halogen, hydroxy, thiol, amino, nitro, cyano, acyl, acyloxy, sulfonyl, sulfinyl, alkylamino, carboxy, ester, ether, amido, azido, sulfonic acid, sulfonamide, alkylsulfonyl, alkylsulfinyl, C1-6-alkylthio, oxyester, oxyamido, aryl, C1-6-alkoxy, C6-10-aryloxy, C1-6-allcyl, C1-6-haloalkyl. Aryl radicals are preferably monocyclic or bicyclic containing 6-10 carbon atoms. Preferred aryl groups are phenyl and naphthyl each optionally substituted by one or more substituents independently selected from halogen, nitro, amino, azido, C1-6-alkoxy, C1-6-alkyl, C1-6-haloalkyl, sulfonyl and phenyl.

In some instances the aryl is phenyl, optionally substituted by one or more halogen, lower alkyl, azido or nitro, such as 3-chlorophenyl and 3-azidophenyl.

The term "halogen", as used herein, includes an atom of Cl, Br, F, I.

The term "hydroxy", as used herein, represents a group of the formula -OH.

The term "thiol", as used herein, represents a group of the formula -SH.

The term "cyano", as used herein, represents a group of the formula -CN.

The term "nitro", as used herein, represents a group of the formula -NO2.

The term "nitrooxy", as used herein, represents a group of the formula -ONO2.

The term "amino", as used herein, represents a group of the formula -NH2.

The term "azido", as used herein, represents a group of the formula -N3.

The term "carboxy", as used herein, represents a group of the formula -COOH.

The term "sulfonic acid", as used herein, represents a group of the formula -SO3H.

The term "sulfonamide", as used herein, represents a group of the formula-SO2NH2.

The term "ester", as used herein, is defined as including a group of formula -COO-R<11 > wherein R<11 > is as defined above except oxy derivative, thio derivative or amino derivative. Preferred are esters of formula -COOR<11 > wherein R<11 > is selected from C1-12 alkyl, C2-12 alkenyl, C2-12 alkenyl and aryl. Most preferred are esters where R<11 > is a lower alkyl, especially methyl.

The term "ether" is defined as including a group selected from C1-50-straight or branched alkyl, or C2-50-straight or branched alkenyl or alkynyl groups or a combination of the same, interrupted by one or more oxygen atoms.

The term "amido" is defined as including a group of formula -CONH2 or - CONHR<11 > or -CONR<11> R<12 > wherein R<11 > rand R<12 > are as defined above.

The term "heterocycle", as used herein, is defined as including an aromatic or non aromatic cyclic alkyl, alkenyl, or alkynyl moiety as defined above, having at least one O, S and/or N atom interrupting the carbocyclic ring structure and optionally, one of the carbon of the carbocyclic ring structure may be replaced by a carbonyl, and optionally being substituted with any suitable group, including but not limited to one or more moieties selected from lower alkyl, or other groups as described above for the alkyl groups. Non-limiting examples of heterocycles are pyridyl; furyl, pyrrolyl, thienyl, isothiazolyl, triazolyl, imidazolyl, benzimidazolyl, tetrazolyl, quinazolinyl, quinolizinyl, naphthyridinyl, pyridazinyl, pyrimidinyl, pyrazinyl, quinolyl, isoquinolyl, isobenzofuranyl, benzothienyl, pyrazolyl, indolyl, indolizinyl, purinyl, isoindolyl, carbazolyl, thiazolyl, 1,2,4-thiadiazolyl, thiomorpholinyl, thieno(2,3-b)furanyl, furopyranyl, benzofuranyl, benzoxepinyl, isooxazolyl, oxazolyl, thianthrenyl, benzothiazolyl, or benzoxazolyl, cinnolinyl, phthalazinyl, quinoxalinyl, 1-oxidopyridyl, phenanthridinyl, acridinyl, perimidinyl, phenanthrolinyl, phenothiazinyl, furazanyl, benzodioxolyl, isochromanyl, indolinyl, xanthenyl, hypoxanthinyl, pteridinyl, 5-azacytidinyl, 5-azauracilyl, triazolopyridinyl, imidazolopyridinyl, pyrrolopyrimidinyl,

pyrazolopyrimidinyl, tetrahydrofuranyl, tetrahydropyranyl, piperidinyl, piperidyl, piperazinyl, imidazolidinyl, morpholino, morpholinyl, 1-oxaspiro(4.5)dec-2-yl, pyrrolidinyl, 2-oxo-pyrrolidinyl, sugar moieties (i.e. glucose, pentose, hexose, ribose, fructose, which may also be substituted) optionally substituted by alkyl or as described above for the alkyl groups. The term "heterocycle" also includes bicyclic, tricyclic and tetracyclic, spiro groups in which any of the above heterocyclic rings is fused to one or two rings independently selected from an aryl ring, a cyclohexane ring, a cyclohexene ring, a cyclopentane ring, a cyclopentene ring or another monocyclic heterocyclic ring or where a monocyclic heterocyclic group is bridged by an alkylene group, such as quinuclidinyl, 7-azabicyclo(2.2.1)heptanyl, 7-oxabicyclo(2.2. 1)heptanyl, 8-azabicyclo(3.2.1)octanyl.

The heterocycle may be selected from triazolyl, tetrazolyl, pyrrolidinyl, pyridyl, 1-oxidopyridyl, thiomorpholinyl, benzodioxolyl, furyl, oxazolyl, pyrimidinyl, pyrrolyl, thiadiazolyl, thiazolyl, thienyl and piperazinyl, each optionally substituted by one or more substituents selected from halogen, alkyl, substituted alkyl, alkoxy, nitro, amino, acyl and phenyl. In some instances the heterocycle is selected from tetrazolyl, pyrrolidinyl, pyridyl, furyl, pyrrolyl, thiazolyl and thienyl, each optionally substituted by one or more substituents selected from halogen, alkyl, halogen substituted alkyl, acyl, alkoxy, nitro, amino and phenyl, and especially from 2-and 3-thienyl, optionally substituted by one or more halogen, acyl such as formyl, cyano and/or lower alkyl, such as methyl.

In the above definitions it is to be understood that when a substituent such as R<1> , R<2> , R<3> , R<4> , R<5> , R<7> , R<8> , R<9> , R<10 > is attached to the rest of the molecule via a heteroatom or a carbonyl, a straight- or branched chain, C1-12-, preferably C1-4-alkylene or C2-12, preferably C2-4-alkenylene or-alkynylene bridge may optionally be interposed between the heteroatom or the carbonyl and the point of attachment to the rest of the molecule.

The acid addition salt form of a compound of formula (I) that occurs in its free form as a base can be obtained by treating said free base form with an appropriate acid such as an inorganic acid, for example, a hydrohalic such as hydrochloric or hydrobromic, sulfuric, nitric, phosphoric and the like; or an organic acid, such as, for example, acetic, hydroxyacetic, propanoic, lactic, pyruvic, malonic, succinic, maleic, fumaric, malic, tartaric, citric, methanesulfonic, ethanesulfonic, benzenesulfonic, p-toluenesulfonic, cyclamic, salicylic, p-aminosalicylic, pamoic and the like.

The compounds of formula (I) containing acidic protons may be converted into their therapeutically active, non-toxic base addition salt form, e.g. metal or amine salts, by treatment with appropriate organic and inorganic bases. Appropriate base salt forms include, for example, ammonium salts, alkali and earth alkaline metal salts, e.g. lithium, sodium, potassium, magnesium, calcium salts and the like, salts with organic bases, e.g. N-methyl-D-glucamine, hydrabamine salts, and salts with amino acids such as, for example, arginine, lysine and the like.

Conversely said salt forms can be converted into the free forms by treatment with an appropriate base or acid.

Compounds of the formula I and their salts can be in the form of a solvate, which is included within the scope of the present disclosure. Such solvates include for example hydrates, alcoholates and the like.

Many of the compounds of formula I and some of their intermediates have at least one stereogenic center in their structure. This stereogenic center may be present in a R or a S configuration, said R and S notation is used in correspondence with the rules described in Pure Appl. Chem. (1976), 45, 11-30.

The disclosure also relates to all stereoisomeric forms such as enantiomeric and diastereoisomeric forms of the compounds of formula I or mixtures thereof (including all possible mixtures of stereoisomers).

Furthermore, certain compounds of formula I which contain alkenyl groups may exist as Z (zusammen) or E (entgegen) isomers. In each instance, the disclosure includes both mixture and separate individual isomers.

Multiple substituents on the piperidinyl or the azepanyl ring can also stand in either cis or trans relationship to each other with respect to the plane of the piperidinyl or the azepanyl ring.

Some of the compounds of formula I may also exist in tautomeric forms. Such forms although not explicitly indicated in the above formula are intended to be included within the scope of the present disclosure.

With respect to the present disclosure reference to a compound or compounds is intended to encompass that compound in each of its possible isomeric forms and mixtures thereof unless the particular isomeric form is referred to specifically.

The disclosure also includes within its scope prodrug forms of the compounds of formula I and Its various sub-scopes and sub-groups.

The term "prodrug" as used herein includes compound forms which are rapidly transformed in vivo to the parent compound according to the disclosure, for example, by hydrolysis in blood. Prodrugs are compounds bearing groups which are modified by biotransformation prior to exhibiting their pharmacological action. Such groups include moieties which are readily oxidised, cyclised or cleaved, which compound after biotransformation remains or becomes pharmacologically active. For example, metabolically cleavable groups form a class of groups well known to practitioners of the art. They include, but are not limited to such groups as alkanoyl (i.e. acetyl, propionyl, butyryl, and the like), unsubstituted and substituted carbocyclic aroyl (such as benzoyl, substituted benzoyl and 1- and 2-naphthoyl), alkoxycarbonyl (such as ethoxycarbonyl), trialkylsilyl (such as trimethyl- and triethylsilyl), monoesters formed with dicarboxylic acids (such as succinyl), phosphate, sulfate, sulfonate, sulfonyl, sulfinyl and the like. The compounds bearing the biotransformable groups have the advantage that they may exhibit improved bioavailability as a result of enhanced solubility and/or rate

of absorption conferred upon the parent compound by virtue of the presence of the biotransformable group. T. Higuchi and V. Stella, "Pro-drugs as Novel Delivery System", Vol. 14 of the A.C.S. Symposium Series; "Bioreversible Carriers in Drug Design", ed. Edward B. Roche, American Pharmaceutical Association and Pergamon Press, 1987.

The term "R substituent" refers to R<1> , R<2> , R<3> , R<4 > or R<5 > , independently.

According to one instance, the present disclosure relates to a compound of formula I as defined above wherein n represents 0. The compound is a 6-ring structure (2-thioxo- or 2-oxo-piperidinyl derivative) wherein R<1 > is not existent since n=0, and is depicted by the formula (I-A).

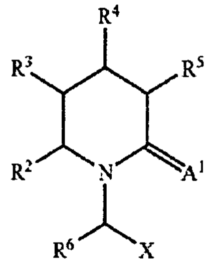

(I-A)

According to a following instance, the present disclosure relates to a compound of formula I according to the disclosure as defined above wherein n represents 1. The compound is a 7-ring structure (2-thioxo- or 2-oxo-azepanyl derivative) wherein R<1 > is existent since n=1 and depicted by the formula (I-B).

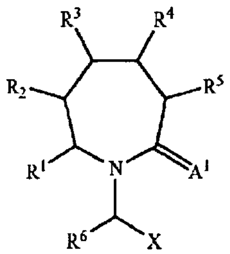

(I-B)

According to one instance, the disclosure relates to said compound as defined above wherein n=0, R<3 > and/or R<4 > are different from hydrogen and R<2 > and R<5 > represent hydrogen.

According to another instance, the disclosure relates to said compound as defined above wherein n=1, R<2> , R<3 > and/or R<4 > are different from hydrogen and wherein R<1 > and R<5 > represent hydrogen.

According to another instance, the disclosure relates to said compound as defined above wherein only one R substituent chosen from R<3 > or R<4 > when n=0 or from R<2> , R<3 > or R<4 > when n=1, is different from hydrogen and the remaining R substituent(s) is/are hydrogen. We hereby refer to a mono-substituted 2-thioxo- or 2-oxo-piperidinyl or 2-thioxo- or 2-oxo-azepanyl derivatives.

According to another instance, the present disclosure relates to compounds of formula I according to the disclosure as defined above wherein A<1 > represents an oxygen atom. We hereby refer to 2-oxo-piperidinyl or 2-oxo-azepanyl derivatives.

According to another instance, the present disclosure relates to compounds of formula I according to the disclosure as defined above wherein X is CONR<7> R<8> , especially CONH2. We hereby refer to amido derivatives of 2-oxo(or thioxo)-piperidinyl or 2-oxo(or thioxo)-azepanyl.

According to another instance, the present disclosure relates to compounds of formula I according to the disclosure as defined above wherein R<6 > represents hydrogen, C1-4 alkyl, or a CH2-R<6a > group wherein R<6a > represents a heterocycle. Most preferably R<6 > is a C1-4 alkyl, especially ethyl. When R<6 > is ethyl we refer to 2-(2-oxo(or thioxo)-1-piperidinyl)butanamide or 2-(2-oxo(or thioxo)-1-azepanyl)butanamide derivatives.

According to another instance, the present disclosure relates to compounds of formula I according to the disclosure as defined above wherein the carbon atom to which R<6 > is attached is of the S configuration. In case where R<6 > is ethyl, A is oxygen and X is CON R<7 > R<8> , we refer then to (2S)-2-(2-oxo-1-piperidinyl)butanamide or (2S)-2-(2-oxo-1-azepanyl)butanamide derivatives.

According to one instance, the present disclosure relates to a compound as defined above wherein R<2 > when n=1, R<3 > and R<4 > are the same or different and each is independently hydrogen, halogen, nitro, nitrooxy, cyano, carboxy, amido, sulfonic acid, sulfonamide, alkyl, alkenyl, alkynyl, ester, ether, aryl, heterocycle, acyl derivative, sulfonyl

derivative or sulfinyl derivative:

R<1 > when existent, R<2 > when n=0 and R<5 > are hydrogen;

R<6 > is hydrogen, alkyl, aryl or -CH2-R<6a > wherein R<6a > is aryl, heterocycle, halogen, hydroxy, amino, nitro or cyano;

provided that, when R<6 > is hydrogen, X is -CONR<7> R<8 > and that the compound is

neither methyl (2R)-2-[(6R)-6-methyl-2-oxoazepanyl]-3-phenylpropanoate nor methyl (2S)-2-[(4R)-4-methyl-2-oxoazepanyl]-3-phenylpropanoate.

According to this instance, the compound is generally such that when R<6 > is benzyl, X is -COOCH3 and n=1, R<2 > is different from methyl when R<3 > and R<4 > are both hydrogen and R<4 > is different from methyl when R<2 > and R<3 > are both hydrogen.

According to another instance, the present disclosure relates to a compound as defined above wherein R<2 > when n=1, R<3 > and R<4 > are the same or different and each is independently hydrogen; cyano; carboxy; amido;

C1-12 alkyl, each optionally substituted by one or more substituents selected from hydroxy, halogen, cyano, thiocyanato, alkoxy, azido, alkyltio, cycloalkyl, acyl, aryl and heterocycle;

C2-12 alkenyl, each optionally substituted by one or more substituents selected from halogen, cyano, thiocyanato, azido, alkylthio, alkyl, aryl and acyl;

C2-12 alkynyl, each optionally substituted by one or more substituents selected from halogen, cyano, thiocyanato, azido, alkylthio, alkyl, aryl and acyl; acyl derivative of formula -CO-R<11> , wherein R<11 > is selected from C1-12 alkyl, C2-12 alkenyl, C2-12 alkynyl, heterocycle and aryl;

ester of formula -CO-O-R<11 > wherein R<11 > is selected from C1-12 alkyl, C2-12 alkenyl, C2-12 alkynyl and aryl;

heterocycle selected from triazolyl, tetrazolyl, pyrrolidinyl, pyridyl, 1-oxidopyridyl, thiomorpholinyl, benzodioxolyl, furyl, oxazolyl, pyrimidinyl, pyrrolyl, thiadiazolyl, thiazolyl, thienyl and piperazinyl, each optionally substituted by one or more substituents selected from halogen, alkyl, substituted alkyl, alkoxy, nitro, amino, acyl and phenyl;

aryl, each optionally substitued by one or more substituents selected from C1-6 alkyl, C1-6 haloalkyl, C1-6 alkoxy, C1-6 alkylthio, amino, azido, sulfonyl, aryl and nitro.

According to another instance, the present disclosure relates to a compound as defined above, wherein R<2 > when n=1, R<3 > and R<4 > are the same or different and each is independently hydrogen;

C1-7 alkyl, each optionally substituted by one or more substituents selected from hydroxy, halogen, cyano, thiocyanato, alkoxy, azido, alkyltio, cyclopropyl, acyl and phenyl;

C2-6 alkenyl, each optionally substituted by one or more substituents selected from halogen, cyano, thiocyanato, azido, alkylthio, cycloalkyl, phenyl and acyl:

C2-6 alkynyl, each optionally substituted by one or more substituents selected from halogen, cyano, thiocyanato, azido, alkylthio, cycloalkyl, phenyl and acyl:

heterocycle selected from tetrazolyl, pyrrolidinyl, pyridyl, furyl, pyrrolyl, thiazolyl and thienyl, each optionally substituted by one or more substituents selected from halogen, alkyl, halogen substituted alkyl, acyl, alkoxy, nitro, amino and phenyl;

phenyl, each optionally substitued by one or more substituents selected from C1-6 alkyl, halogen substituted alkyl, halogen, alkoxy, amino, azido, sulfonyl, phenyl and nitro.

According to another instance, the present disclosure relates to a compound as defined above wherein at least one of the R substituents chosen from the group R<2> , R<3 > and R<4 > when n=1 or from the group R<3 > and R<4

> when n=0, represents independently C1-4-alkyl or C3-7-cycloalkyl, optionally substituted by one or more halogen, hydroxy, lower alkyl and/or azido.

According to another instance, the present disclosure relates to a compound as defined above wherein at least one of the R substituents chosen from the group R<2> , R<3 > and R<4 > when n=1 or from the group R<3 > and R<4 > when n=0, represents independently vinyl, optionally substituted by one or more halogen or/and lower alkyl.

According to another instance, the present disclosure relates to a compound as defined above wherein at least one of the R substituents chosen from the group R<2> , R<3 > and R<4 > when n=1 or from the group R3 and R<4 > when n=0, represents independently ethynyl, propynyl or butynyl, optionally substituted by one or more halogen and/or lower alkyl.

According to another instance, the present disclosure relates to a compound as defined above wherein at least one of the R substituents chosen from the group R<2> , R<3 > and R<4 > when n=1 or from the group R<3 > and R<4 > when n=0, represents independently phenyl, optionally substituted by one or more halogen, lower alkyl, azido and/or nitro.

According to another instance, the present disclosure relates to a compound as defined above wherein at least one of the R substituents chosen from the group R<2> , R<3 > and R<4 > when n=1 or from the group R<3 > and R<4 > when n=0, represents independently 2- or 3-thienyl, optionally substituted by one or more halogen, acyl, cyano or/and lower alkyl.

According to a particular instance, the present disclosure relates to a compound as defined above wherein at least one of the R substituents chosen from the group R<3> , R<4 > and R<2 > when n=1 or from the group R<3 > and R<4 > when n=0, is hydroxymethyl, propyl, butyl, 3,3,3-trifluoropropyl, 2,2,2-trifluoroethyl, cyclopropylmethyl, iodomethyl, azidomethyl, 2-thienyl, 3-thienyl, phenyl, 3-chlorophenyl, 3-azidophenyl, 2,2-difluorovinyl, 2,2-dibromovinyl, 2,2-dichlorovinyl, 2-ethynyl, 5-methyl-2-thienyl, 5-formyl-2-ethynyl, 5-cyano-2-thienyl, 3-bromo-2-thienyl, 4-methyl-2-thienyl, 3,3,3-trifluoro-1-propynyl, 1-propynyl, cyclopropylethynyl, 3-methyl-1-butynyl, 1-butynyl, 2,2-difluoropropyl, 2-chloro-2,2-difluoroethyl, 2-bromo-2,2-difluoroethyl and 2-iodo-2,2-difluoroethyl.

According to yet another instance, the present disclosure relates to a compound as defined above wherein R<1> , R<2> , R<4 > and R<5 > are hydrogen.

According to another instance, the present disclosure relates to a compound as defined above wherein R<1> , R<2> , R<3 > and R<5 > are hydrogen.

According to another instance, the present disclosure relates to a compound as defined above wherein n=1 and R<1> , R<3> , R<4 > and R<5 > are hydrogen.

In all the above-mentioned scopes when the carbon atom to which R<6 > is attached is asymmetric it may be in the "S"-configuration.

Representative compounds of this disclosure as defined above are selected from the group consisting of 2-[5-(hydroxymethyl)-2-oxo-1-piperidinyl]butanamide, 2-(2-oxo-5-propyl-1-piperidinyl)butanamide, 2-12-oxo-5-(3,3,3-trifluoropropyl)-1-piperidinyl]butanamide, 2-[5-(cyclopropylmethyl)-2-oxo-1-piperidinyl]butanamide, 2-[5-(iodomethyl)-2-oxo-1-piperidinyl]butanamide, 2-[5-(azidomethyl)-2-oxo-1-piperidinyl]butanamide, 2-(2-oxo-5-phenyl-1-piperidinyl)butanamide, 2-[2-oxo-5-(2-thienyl)-1-piperidinyl]butanamide, 2-[2-oxo-5-(3-thienyl)-1-piperidinyl]butanamide, 2-[5-(3-chlorophenyl)-2-oxo-1-piperidinyl]butanamide, 2-[5-(3-azidophenyl)-2-oxo-1-piperidinyl]butanamide, 2-[5-(2,2-difluorovinyl)-2-oxo-1-piperidinyl]butanamide, 2-[5-(2,2-dibromovinyl)-2-oxo-1-piperidinyl]butanamide, 2-[5-(2,2-dichlorovinyl)-2-oxo-1-piperidinyl]butanamide, 2-(5-ethynyl-2-oxo-1-piperidinyl)butanamide, 2-[5-(5-methyl-2-thienyl)-2-oxo-1-piperidinyl]butanamide, 2-[5-(5-formyl-2-thienyl)-2-oxo-1-piperidinyl]butanamide, 2-[5-(5-cyano-2-thienyl)-2-oxo-1-piperidinyl]butanamide, 2-[5-(3-bromo-2-thienyl)-2-oxo-1-piperidinyl]butanamide, 2-[5-(4-methyl-2-thienyl)-2-oxo-1-piperidinyl]butanamide, 2-[2-oxo-5-(3,3,3-trifluoro-1-propynyl)-1-piperidinyl]butanamide, 2-[2-oxo-5-(1-propynyl)-1-piperidinyl]butanamide, 2-[5-(cyclopropylethynyl)-2-oxo-1-piperidinyl]butanamide, 2-[5-(3-methyl-1-butynyl)-2-oxo-1-piperidinyl]butanamide, 2-[5-(1-butynyl)-2-oxo-1-piperidinyl]butanamide, 2-[5-(2,2-difluoropropyl)-2-oxo 1-piperidinyl]butanamide, 2-[5-(2-chloro-2,2-difluoroethyl)-2-oxo-1-piperidinyl]butanamide, 2-[5-(2-bromo-2,2-difluoroethyl)-2-oxo-1-piperidinyl]butanamide, 2-[4-(hydroxymethyl)-2-oxo-1-piperidinyl]butanamide, 2-(2-oxo-4-propyl-1-piperidinyl)butanamide, 2-[2-oxo-4-(3,3,3-trifluoroproyl)-1-piperidinyl]butanamide, 2-14-(cyclopropylmethyl)-2-oxo-1-piperidinyl]butanamide, 2-[4-(iodomethyl)-2-oxo-1-piperldinyl]butanamide, 2-[4-(azidomethyl)-2-oxo-1-piperidinyl]butanamide, 2-(2-oxo-4-phenyl-1-piperidinyl)butanamide, 2-12-oxo-4-(2-thienyl)-1-piperidinyl]butanamide, 2-[2-oxo-4-(3-thienyl)-1-piperidinyl]butanamide, 2-[4-(3-chlorophenyl)-2-oxo-1-piperidinyl]butanamide, 2-[4-(3-azidophenyl)-2-oxo-1-piperidinyl]butanamide, 2-[4-(2,2-difluorovinyl)-2-oxo-1-piperidinyl]butanamide, 2-[4-(2,2-dibromovinyl)-2-oxo-1-piperidinyl]butanamide, 2-[4-(2,2-dichlorovinyl)-2-oxo-1-piperidinyl]butanamide, 2-(4-ethynyl-2-oxo-1-piperidinyl)butanamide, 2-[4-(5-methyl-2-thienyl)-2-oxo-1-piperidinyl]butanamide, 2-[4-(5-formyl-2-thienyl)-2-oxo-1-piperidinyl]butanamide, 2-[4-(5-cyano-2-thienyl)-2-oxo-1-piperidinyl]butanamide, 2-[4-(3-bromo-2-thienyl)-2-oxo-1-piperidinyl]butanamide, 2-[4-(4-methyl-2-thienyl)-2-oxo-1-piperidinyl]butanamide, 2-[2-oxo-4-(3,3,3-trifluoro-1-propynyl)-1-piperidinyl]butanamide, 2-[2-oxo-4-( 1-propynyl)-1-piperidinyl]butanamide, 2-[4-(cyclopropylethynyl)-2-oxo-1-piperidinyl]butanamide, 2-[4-(3-methyl-1-butynyl)-2-oxo-1-piperidinyl]butanamide, 2-[4-(1-butynyl)-2-oxo-1-piperidinyl]butanamide, 2-[4-(2,2-difluoropropyl)-2-oxo-1-piperidinyl]butanamide, 2-[4-(2-chloro-2,2-difluoroethyl)-2-oxo-1-piperidinyl]butanamide, 2-14-(2-bromo-2,2-difluoroethyl)-2-oxo-1-piperidinyl]butanamide, 2-[4-(2,2,2-trifluoroethyl)-2-oxo-1-piperidinyl]butanamide, 2-[5-(hydroxymethyl)-2-oxo-1-azepanyl]butanamide, 2-(2-oxo-5-propyl-1-azepanyl)butanamide, 2-[2-oxo-5-(3,3,3-trifluoropropyl)-1-azepanyl]butanamide, 2-(5-(cyclopropylmethyl)-2-oxo-1-azepanyl]butanamide, 2-[5-(iodomethyl)-2-oxo-1-azepanyl]butanamide, 2-[5-(azidomethyl)-2-oxo-1-azepanyl]butanamide, 2-(2-oxo-5-phenyl-1-azepanyl)butanamide, 2-[2-oxo-5-(2-thienyl)-1-azepanyl]butanamide, 2-[2-oxo-5-(3-thienyl)-1-azepanyl]butanamide, 2-[5-(3-chlorophenyl)-2-oxo-1-azepanyl]butanamide, 2-[5-(3-azidophenyl)-2-oxo-1-azepanyl]butanamide, 2-[5-(2,2-difluorovinyl)-2-oxo-1-azepanyl]butanamide, 2-[5-(2,2-dibromovinyl)-2-oxo-1-azepanyl]butanamide, 2-[5-(2,2-dichlorovinyl)-2-oxo-1-azepanyl]butanamide, 2-(5-ethynyl-2-oxo-1-azepanyl)butanamide, 2-[5-(5-methyl-2-thienyl)-2-oxo-1-azepanyl]butanamide, 2-[5-(5-formyl-2-thienyl)-2-oxo-1-azepanyl]butanamide, 2-[5-(5-cyano-2-thienyl)-2-oxo-1-azepanyl]butanamide, 2-[5-(3-bromo-2-thienyl)-2-oxo-1-azepanyl]butanamide, 2-[5-(4-methyl-2-thienyl)-2-oxo-1-azepanyl]butanamide, 2-[2-oxo-5-(3,3,3-trifluoro-1-propynyl)-1-azepanyl]butanamide, 2-[2-oxo-5-(1-propynyl)-1-azepanyl]butanamide, 2-[5-(cyclopropylethynyl)-2-oxo-1-azepanyl]butanamide 2-[5-(3-methyl-1-butynyl)-2-oxo-1-azepanyl]butanamide, 2-[5-(1-butynyl)-2-oxo-1-azepanyl]butanamide, 2-[5-(2,2-difluoropropyl)-2-oxo-1-azepanyl]butanamide, 2-[5-(2-chloro-2,2-difluoroethyl)-2-oxo-1-azepanyl]butanamide, 2-[5-(2-bromo-2,2-difluoroethyl)-2-oxo-1-azepanyl]butanamide, 2-[5-(2,2,2-trifluoroethyl)-2-oxo-1-azepanyl]butanamide, 2-[6-(hydroxymethyl)-2-oxo-1-azepanyl]butanamide, 2-(2-oxo-6-propyl-1-azepanyl)butanamide, 2-[2-oxo-6-(3,3,3-trifluoropropyl)-1-azepanyl]butanamide, 2-[6-(cyclopropylmethyl)-2-oxo-1-azepanyl]butanamide, 2-[6-(iodomethyl)-2-oxo-1-azepanyl]butanamide 2-16-(azidomethyl)-2-oxo-1-azepanyl]butanamide, 2-(2-oxo-6-phenyl-1-azepanyl)butanamide, 2-[2-oxo-6-(2-thienyl)-1-azepanyl]butanamide, 2-[2-oxo-6-(3-thienyl)-1-azepanyl]butanamide, 2-[6-(3-chlorophenyl)-2-oxo-1-azepanyl]butanamide, 2-[6-(3-azidophenyl)-2-oxo-1-azepanyl]butanamide, 2-[6-(2,2-difluorovinyl)-2-oxo-1-azepanyl]butanamide, 2-[6-(2,2-dibromovinyl)-2-oxo-1-azepanyl]butanamide, 2-[6-(2,2-dichlorovinyl)-2-oxo-1-azepanyl]butanamide, 2-(6-ethynyl-2-oxo-1-azepanyl)butanamide, 2-[6-(5-methyl-2-thienyl)-2-oxo-1-azepanyl]butanamide, 2-[6-(5-formyl-2-thienyl)-2-oxo-1-azepanyl]butanamide, 2-[6-(5-cyano-2-thienyl)-2-oxo-1-azepanyl]butanamide, 2-[6-(3-bromo-2-thienyl)-2-oxo-1-azepanyl]butanamide, 2-[6-(4-methyl-2-thienyl]-2-oxo-1-azepanyl]butanamide, 2-[2-oxo-6-(3,3,3-trifluoro-1-propynyl)-1-azepanyl]butanamide, 2-[2-oxo-6-(1-propynyl)-1-azepanyl]butanamide, 2-[6-(cyclopropylethynyl)-2-oxo-1-azepanyl]butanamide, 2-[6-(3-methyl-1-butynyl)-2-oxo-1-azepanyl]butanamide, 2-[6-(1-butynyl)-2-oxo-1-azepanyl]butanamide, 2-[6-(2,2-difluoropropyl)-2-oxo-1-azepanyl]butanamide, 2-[6-(2-chloro-2,2-difluoroethyl)-2-oxo-1-azepanyl]butanamide, 2-[6-(2-bromo-2,2-difluoroethyl)-2-oxo-1-azepanyl]butanamide, 2-[6-(2,2,2-trifluoroethyl)-2-oxo-1-azepanyl]butanamide, 2-[4-(hydroxymethyl)-2-oxo-1-azepanyl]butanamide, 2-(2-oxo-4-propyl-1-azepanyl)butanamide, 2-[2-oxo-4-(3,3,3-trifluoropropyl)-1-azepanyl]butanamide, 2-14-(cyclopropylmethyl)-2-oxo-1-azepanyl]butanamide, 2-[4-(iodomethyl)-2-oxo-1-azepanyl]butanamide, 2-[4-(azidomethyl)-2-oxo-1-azepanyl]butanamide, 2-(2-oxo-4-phenyl-1-azepanyl)butanamide, 2-[2-oxo-4-(2-thienyl)-1-azepanyl]butanamide, 2-[2-oxo-4-(3-thienyl)-1-azepanyl]butanamide, 2-f4-(3-chlorophenyl)-2-oxo-1-azepanyl]butanamide, 2-[4-(3-azidophenyl)-2-oxo-1-azepanyl]butanamide, 2-[4-(2,2-difluorovinyl)-2-oxo-1-azepanyl]butanamide, 2-[4-(2,2-dibromovinyl)-2-oxo-1-azepanyl]butanamide, 2-[4-(2,2-dichlorovinyl)-2-oxo-1-azepanyl]butanamide, 2-(4-ethynyl-2-oxo-1-azepanyl)butanamide, 2-[4-(5-methyl-2-thienyl)-2-oxo-1-azepanyl]butanamide, 2-[4-(5-formyl-2-thienyl)-2-oxo-1-azepanyl]butanamide, 2-[<4> -(5-cyano-<2> -thienyl)-2-oxo-1-azepanyl]butanamide, 2-[4-(3-bromo-2-thienyl)-2-oxo-1-azepanyl]butanamide, 2-[4-(4-methyl-2-thienyl)-2-oxo-1-azepanyl]butanamide, 2-[2-oxo-4-(3,3,3-trifluoro-1-propynyl)-1-azepanyl]butanamide, 2-[2-oxo-4-(1-propynyl)-1-azepanyl]butanamide, 2-[4-(cyclopropylethynyl)-2-oxo-1-azepanyl]butanamide, 2-[4-(3-methyl-1-butynyl)-2-oxo-1-azepanyl]butanamide, 2-[4-( 1-butynyl)-2-oxo-1-azepanyl]butanamide, 2-[4-(2,2-difluoropropyl)-2-oxo-1-azepanyl]butanamide, 2-[4-(2-chloro-2,2-difluoroethyl]-2-oxo-1-azepanyl]butanamide, 2-[4-(2-bromo-2,2-difluoroethyl)-2-oxo-1-azepanyl]butanamide, 2-[4-(2,2,2-trifluoroethyl)-2-oxo-1-azepanyl]butanamide.

Results have been obtained with the following compounds:

(2S)-2-[5-(iodomethyl)-2-oxo-1-piperidinyl]butanamide,

(2S)-2-[5-(azidomethyl)-2-oxo-1-piperidinyl]butanamide,

2-(2-oxo-5-phenyl-1-piperidinyl]butanamide,

(2S)-2-[4-(iodomethyl)-2-oxo-1-piperidinyl]butanamide,

2-[5-(iodomethyl)-2-oxo-1-azepanyl]butanamide.

xv) International Patent Application Publication No. WO2008/132139

In some instances, the compounds are of formula (I) as follows:

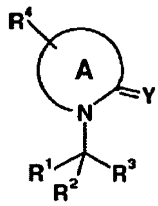

(I)

wherein

Y is O or S. In some instances Y is O. R1 is hydrogen or C-|.g alkyl;

R2 is hydrogen;

R3 is -CONR5R6, -COR7, an imidazolyl, an imidazopyridinyl, an imidazopyridazinyl; R5, R6 are the same or different and are independently selected from hydrogen and C-|_6 alkyl;

R7 is C<;|_6 alkyl;

A is a monocyclic or bicyclic heterocyclic moiety selected from the group consisting of imidazolidin-1-yl, 1 ,3-oxazolidin-3-yl, 2,5-dihydro-1 H-pyrrol-1-yl, 1 ,3-thiazol-3(2H)-yl, 1 ,3- thiazolidin-3-yl, piperidin-1-yl, azepan-1-yl, 5,6-dihydro-4H-thieno[3,2-b]pyrrol-4-yl, hexahydro-4H-thieno[3,2-b]pyrrol-4-yl, 2,3-dihydro-1 H-thieno[3,4-b]pyrrol-1-yl, 1 ,3- benzothiazol-3(2H)-yl, 1 ,3-benzoxazol-3(2H)-yl, pyrazolo[1 ,5-a]pyridin-1 (2H)-yl, 3,4- dihydroisoquinolin-2(1 H)-yl, 3,4-dihydroquinolin-1 (2H)-yl, 1 ,3,4,5-tetrahydro-2H-2- benzazepin-2-yl, 1 ,2,4,5-tetrahydro-3H-3-benzazepin-3-yl; R4 is either R^a or R^b depending on whether A being is a monocyclic or a bicyclic heterocycle:

where A is a monocyclic heterocyclic moiety, R^ is R^a which is selected from the group consisting of hydrogen; C-|.g alkyl optionally substituted by a substituent selected from halogen, C-1.4 alkoxy, C-1.4 alkylthio, azido, nitrooxy or an aryl; C2-6 alkenyl optionally substituted by halogen; C2-6 alkynyl optionally substituted by halogen; azido; alkoxycarbonylamino; arylsulfonyloxy; a substituted or unsubstituted aryl; or a 3-8 membered substituted or unsubstituted heterocycle;

where A is a bicyclic heterocyclic moiety R^ is R^ which is selected from the group comprising or consisting of hydrogen; nitro; cyano; halogen; heterocycle;

amino; aryl; C-|.g alkyl optionally substituted by at least one halogen; or C-|.g alkoxy optionally substituted by at least one halogen;

In some instances the compounds are as follows:

For compounds where A=Y is selected from a 2-oxo-piperidin-1-yl, a 2-oxoazepan-1-yl, a 2-oxo-1 ,3-benzothiazol-3(2H)-yl or a 2-oxo-1 ,3-benzoxazol-3(2H)- yl, R3 must beselected from an imidazolyl, an imidazopyridinyl or an imidazopyridazinyl.

For compounds where A=Y is a 5-oxoimidazolidin-1-yl, R^ and R^ are hydrogen, R3 is -CONR5R6, R5 anc| R6 are as above defined, then R^a may not be an alkyl, aralkyl or substituted aralkyl.

Where A=Y is either of a 2-oxo-piperidin-1-yl and a 2-oxo-azepan-1-yl, R^ , R^ and R^a are all hydrogen, then R^ could not be a 2-phenylimidazo[1 ,2-a]pyridin-3-yl.

In a specific instance A=Y is selected from the list consisting of:

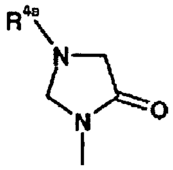

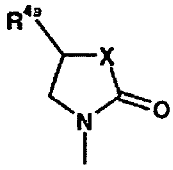

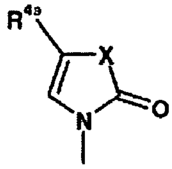

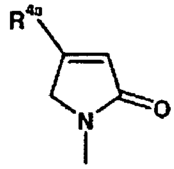

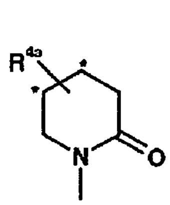
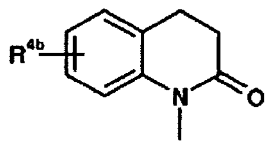
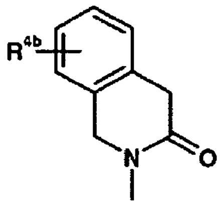

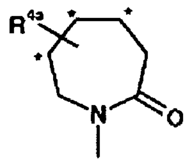
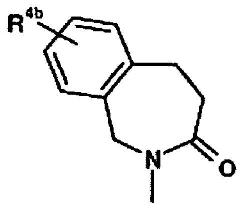
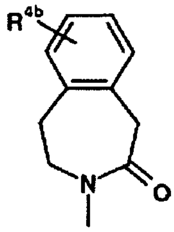
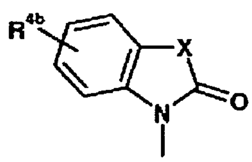

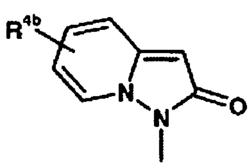
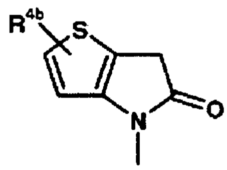
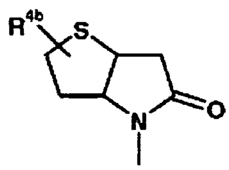
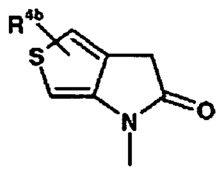

wherein X is O or S, in a more specific instance O; in another instance, X is S.

The asterisks in the above illustration indicate the attachment sites of the substituent R^a.

In a specific instance, when R^ is -CONR5R6 and R^ is C-µg alkyl, the carbon atom to which R-I and R^ are attached is preferably in the "S"-configuration.

In a specific instance R^ is hydrogen, methyl, ethyl and R^ is hydrogen. In a specific instance R3 is -CONH2.

In a further specific instance R^ is 1H-imidazol-1-yl, 1H-imidazol-4-yl, 1H-imidazol-5-yl, imidazo[1,2-a]pyridin-3-yl or imidazo[1,2-b]pyridazin-3-yl. In a specific instance R^a is a C-|.g alkyl which may optionally be substituted by a halogen; or a phenyl.

In another specific instance R^b is hydrogen, halogen, nitro, cyano or a C-µg alkyl optionally substituted by a halogen.

In still a further instance compounds may be used in the treatment of the above mentioned disorders, in particular of epilepsy, having the formula (I-E), as wells as its geometrical isomers, enantiomers, diastereomers and mixtures, or a pharmaceutically acceptable salt thereof,

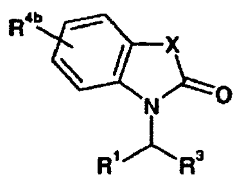

**(I-E)**

wherein

X is O or S;

R-I is hydrogen or C-|.g alkyl, in a more specific instance hydrogen;

R3 is an imidazolyl, an imidazopyridinyl, an imidazopyridazinyl; R^b is hydrogen; nitro; cyano; halogen; C-|.g alkyl optionally substituted by halogen; C-|.g alkoxy optionally substituted by halogen.

A further aspect of the present disclosure consists in novel compounds having the formula (I-A), their geometrical isomers, enantiomers, diastereomers and mixtures, or a pharmaceutically acceptable salt thereof,

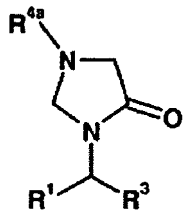

(I-A)

wherein

R1 is hydrogen or C-|.g alkyl, preferably hydrogen, methyl or ethyl; in a more specific instance R^ is ethyl.

R3 is -CONH2, an imidazolyl, an imidazopyridinyl, an imidazopyridazinyl, preferably R^ is -CONH2.

R^a is either hydrogen or an aryl; with the proviso that 2-(5-oxoimidazolidin-1-yl)acetamide is excluded. Preferably R^a is an aryl, e.g. a phenyl which may be substituted preferably by halogen, nitro, alkoxy, in particular by nitro.

In a particular instance, when R^ is -CONH2 and R^ is C-|.g alkyl, the carbon atom to which R1 and R^ are attached is preferably in the "S"-configuration.

A further aspect of the present disclosure consists in novel compounds having the formula (I-B1 or I-B2), their geometrical isomers, enantiomers, diastereomers and mixtures, or a pharmaceutically acceptable salt thereof,

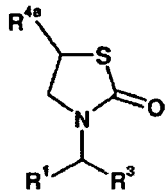

(I-B1)

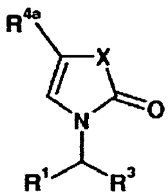

(I-B2)

wherein X in formula (I-B2) is either S or O, in a more specific instance S;

R1 is hydrogen or C-|.g alkyl, preferably hydrogen, methyl or ethyl; in a more specific instance R^ is ethyl.

R3 is -CONH2, an imidazolyl, an imidazopyridinyl, an imidazopyridazinyl; preferably R^ is -CONH2 R^a is hydrogen; C-|.g alkyl optionally substituted by halogen or C-1.4 alkoxy; an aryl; or C2.g alkenyl optionally substituted by halogen. Preferably, R^a is C-|.g alkyl optionally substituted by halogen or C2-6 alkenyl optionally substituted by halogen or an aryl. In a more specific instance R^a is C-|.g alkyl optionally substituted by halogen or aryl.

In a particular instance, when R^ is -CONH2 and R^ is C-|.g alkyl, the carbon atom to which R-l and R^ are attached is preferably in the "S"-configuration.

A further aspect of the present disclosure consists in novel compounds having the formula (I-B3), their geometrical isomers, enantiomers, diastereomers and mixtures, or a pharmaceutically acceptable salt thereof,

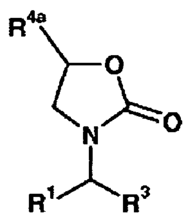

(I-B3)

wherein

R1 is either hydrogen or C-µg alkyl, preferably hydrogen, methyl or ethyl; more preferably R1 is ethyl.

R3 is -CONH2, an imidazolyl, an imidazopyridinyl, an imidazopyridazinyl; preferably R^ is -CONH2 R^a is C-|_5 alkyl optionally substituted by halogen or C-1.4 alkoxy; an aryl; or C2_g alkenyl optionally substituted by halogen. Preferably, R^a is C-|.g alkyl optionally substituted by halogen or C2_g alkenyl optionally substituted by halogen.

In a particular instance, when R^ is -CONH2 and R^ is C-|.g alkyl, the carbon atom to which R-l and R^ are attached is preferably in the "S"-configuration.

A further aspect of the present disclosure consists in novel compounds having the formula (I-C), their geometrical isomers, enantiomers, diastereomers and mixtures, or a pharmaceutically acceptable salt thereof,

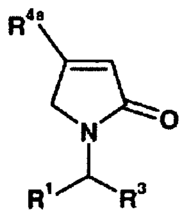

(I-C)

wherein

R1 is hydrogen or C-|.g alkyl, in particular hydrogen, methyl or ethyl.

R3 is -CONH2, an imidazolyl, an imidazopyridinyl, an imidazopyridazinyl; in particular R^ is -CONH2 R^a is methyl, ethyl, butyl optionally substituted by halogen or C-1.4 alkoxy, an unsubstituted phenyl or a phenyl substituted by halogen, a C-|.g alkyl optionally substituted by halogen or a C-1.4 alkoxy; or R^a is a C2-6 alkenyl optionally substituted by halogen. Preferably, R^a is methyl, optionally substituted by halogen, an unsubstituted phenyl or a phenyl substituted by halogen.

In a particular instance, when R^ is -CONH2 and R^ is C-|.g alkyl, the carbon atom to which R1 and Rβ are attached is preferably in the "S"-configuration.

A further aspect of the present disclosure consists in compounds having the formula (I-D1 or I-D2), their geometrical isomers, enantiomers, diastereomers and mixtures, or a pharmaceutically acceptable salt thereof,

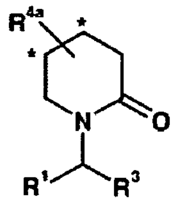

(I-D1)

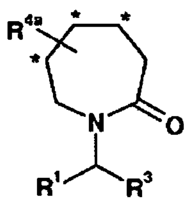

(I-D2)

wherein

R-I is hydrogen or C-|.g alkyl, in particular hydrogen; R3 is an imidazolyl, an imidazopyridinyl or an imidazopyridazinyl. In one instance, R^ is 1H-imidazol-1-yl, 1H-imidazol-4-yl, 1H-imidazol-5-yl, imidazo[1,2-a]pyridin-3-yl or imidazo[1 ,2-b]pyridazin-3-yl. In a more specific instance, R^ is 1H-imidazol-1-yl, 1H-imidazol-4-yl, 1H-imidazol-5-yl, imidazo[1,2-a]pyridin-3-yl; R^a is hydrogen, C-|.g alkyl optionally substituted by halogen or C-1.4 alkoxy; aryl; or C2- g alkenyl optionally substituted by halogen. In a specific instance, R^a is C-|.g alkyl optionally substituted by halogen; aryl; or C2-6 alkenyl optionally substituted by halogen. In a more specific instance R^a is C-|.g alkyl optionally substituted by halogen; or aryl; e.g, propyl or phenyl;

with the proviso that when R^ and R^a are hydrogen, R^ is not 2-phenylimidazo[1 ,2- a]pyridin-3-yl.

A further aspect of the present disclosure consists in compounds having the formula (I-F1, I-F2 or I-F3), their geometrical isomers, enantiomers, diastereomers and mixtures, or a pharmaceutically acceptable salt thereof,

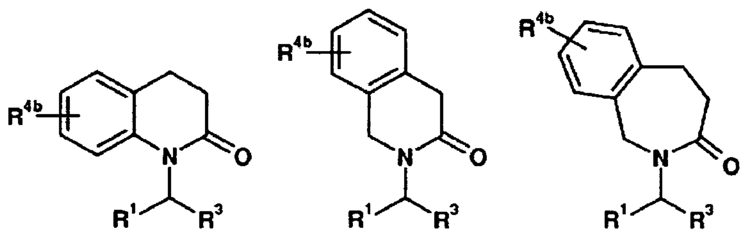

(I-F1) (I-F2) (I-F3)

wherein

R-I is hydrogen or C-|.g alkyl, preferably hydrogen, methyl or ethyl; more preferably, R^ is hydrogen.

R3 is -CONH2, an imidazolyl, an imidazopyridinyl or an imidazopyridazinyl; in a more specific instance R3 is -CONH2, 1H-imidazol-1-yl, 1H-imidazol-4-yl, 1H-imidazol-5-yl, imidazo[1,2-a]pyridin-3-yl or imidazo[1,2-b]pyridazin-3-yl. R^b is hydrogen; halogen; nitro; cyano; C1.4 alkyl optionally substituted by halogen; C-1.4 alkoxy optionally substituted by halogen. In a more specific instance R^ is hydrogen, halogen or cyano, more specifically halogen.

In a particular instance, when R^ is -CONH2 and R^ is C-|.g alkyl, the carbon atom to which R1 and Rβ are attached is preferably in the "S"-configuration.

A further aspect of the present disclosure consists in compounds having the formula (I-F4), their geometrical isomers, enantiomers, diastereomers and mixtures, or a pharmaceutically acceptable salt thereof,

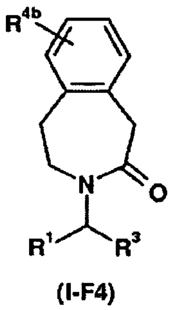

(I-F4)

wherein

R-I is hydrogen or C-|.g alkyl, preferably hydrogen;

R3 is an imidazolyl, an imidazopyridinyl or an imidazopyridazinyl; more specifically R^ is 1H-imidazol-1-yl, 1H-imidazol-4-yl, 1H-imidazol-5-yl, imidazo[1,2-a]pyridin-3-yl or imidazo[1,2-b]pyridazin-3-yl. More specifically R^ is 1H-imidazol-4-yl or imidazo[1,2- a]pyridin-3-yl.

R^b is hydrogen; halogen; nitro; cyano; C-1.4 alkyl optionally substituted by halogen; C-1.4 alkoxy optionally substituted by halogen; specifically R^ is hydrogen, halogen or cyano,.

In a particular instance, when R^ is -CONH2 and R^ is C-|.g alkyl, the carbon atom to which R-I and R^ are attached is preferably in the "S"-configuration.

A further aspect of the present disclosure consists in compounds having either of the formula (I-G1, I-G2 or I-G3), their geometrical isomers, enantiomers, diastereomers and mixtures, or a pharmaceutically acceptable salt thereof,

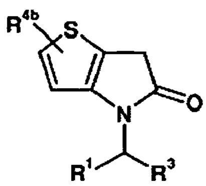

(I-G1)

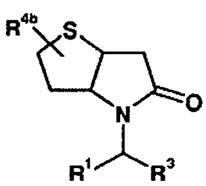

(I-G2)

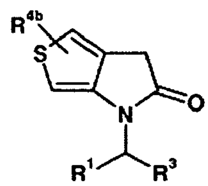

(I-G3)

wherein

R-I is hydrogen or C-|.g alkyl; preferably hydrogen;

R3 is -CONH2, an imidazolyl, an imidazopyridinyl, an imidazopyridazinyl; in a more specific instance R^ is -CONH2, 1H-imidazol-1-yl, 1H-imidazol-4-yl, 1H-imidazol-5-yl, imidazo[1,2-a]pyridin-3-yl or imidazo[1,2-b]pyridazin-3-yl. In a even more specific instance R3 is an 1 H-imidazol-4-yl or imidazo[1,2-a]pyridin-3-yl;

R4D js hydrogen; halogen; C-1.4 alkyl optionally substituted by halogen; C-1.4 alkoxy optionally substituted by halogen.

Specific compounds of the present disclosure are those selected from the group consisting of: (2S)-2-[3-(4-nitrophenyl)-5-oxoimidazolidin-1-yl]butanamide; (2S)-2-[3-(2,4-dinitrophenyl)-5-oxoimidazolidin-1-yl]butanamide; (2S)-2-(5-oxo-3-phenylimidazolidin-1-yl)butanamide; 2-[5-(iodomethyl)-2-oxo-1,3-oxazolidin-3-yl]butanamide; 2-(2-oxo-2,5-dihydro-1H-pyrrol-1-yl)butanamide; 2-(2-oxo-4-phenyl-2,5-dihydro-1H-pyrrol-1-yl)butanamide; 2-(4-methyl-2-oxo-2,5-dihydro-1 H-pyrrol-1-yl)butanamide; (2S)-2-(2-oxo-5-propyl-1,3-thiazol-3(2H)-yl)butanamide; 2-(2-oxo-5-propyl-1,3-thiazol-3(2H)-yl)propanamide; 2-(5-butyl-2-oxo-1,3-thiazolidin-3-yl)butanamide; 2-(5-butyl-2-oxo-1,3-thiazolidin-3-yl)propanamide; 2-(2-oxo-5-phenyl-1,3-thiazolidin-3-yl)propanamide; 2-(2-oxo-5-propyl-1,3-thiazolidin-3-yl)butanamide; 2-(2-oxo-5-phenyl-1,3-thiazolidin-3-yl)butanamide; 2-(2-oxo-5-propyl-1,3-thiazolidin-3-yl)propanamide; (2S)-2-[2-oxo-5-(2,2,2-trifluoroethyl)-1,3-thiazolidin-3-yl]butanamide; 1-{[6-chloro-2-(trifluoromethyl)imidazo[1,2- b]pyridazin-3-yl]methyl}piperidin-2-one; 1-(1H-imidazol-4-ylmethyl)-5-propylpiperidin-2-one; 1-(1H-imidazol-1-ylmethyl)-5-propylpiperidin-2-one; 1-(imidazo[1,2-a]pyridin-3-ylmethyl)-5-propylpiperidin-2-one; 1-(1H-imidazol-1-ylmethyl)-5-phenylpiperidin-2-one; 1-(imidazo[1,2-a]pyridin-3-ylmethyl)-5-phenylpiperidin-2-one; 1-(imidazo[1,2-a]pyridin-3-ylmethyl)-4-phenylpiperidin-2-one; 1-(1H-imidazol-1-ylmethyl)-4-phenylpiperidin-2-one; 1-(imidazo[1,2-a]pyridin-3-ylmethyl)-4-propylpiperidin-2-one; 1-(1H-imidazol-5-ylmethyl)-4-propylpiperidin-2-one; 1-(1H-imidazol-1-ylmethyl)-4-propylpiperidin-2-one; 1-{[6-chloro-2-(trifluoromethyl)imidazo[1,2-b]pyridazin-3-yl]methyl}azepan-2-one; 1-(1H-imidazol-5- ylmethyl)-5-propylazepan-2-one; 5-propyl-1-{[2-(trifluoromethyl)imidazo[1,2-a]pyridin-3-yl]methyl}azepan-2-one; 5-phenyl-1-{[2-(trifluoromethyl)imidazo[1,2-a]pyridin-3-yl]methyl}azepan-2-one; 1-(1H-imidazol-5-ylmethyl)-6-propylazepan-2-one; 1-(1H-imidazol-4-ylmethyl)-4-propylazepan-2-one; 4-(1H-imidazol-4-ylmethyl)-4,6-dihydro-5H-thieno[3,2-b]pyrrol-5-one; 2-(5-oxo-5,6-dihydro-4H-thieno[3,2-b]pyrrol-4-yl)acetamide; 4-{[2-(trifluoromethyl)imidazo[1,2-a]pyridin-3-yl]methyl}-4,6-dihydro-5H-thieno[3,2-b]pyrrol-5-one; 4-{[2-(trifluoromethyl)imidazo[1,2-a]pyridin-3-yl]methyl}hexahydro-5H-thieno[3,2-b]pyrrol-5-one; 1-(1H-imidazol-4-ylmethyl)-1H-thieno[3,4-b]pyrrol-2(3H)-one; 2-(6-chloro-2-0X0-1,3-benzothiazol-3(2H)-yl)acetamide; 6-bromo-3-(1H-imidazol-1-ylmethyl)-1,3- benzothiazol-2(3H)-one; 2-(6-bromo-2-oxo-1,3-benzothiazol-3(2H)-yl)propanamide; 2-(6-bromo-2-oxo-1,3-benzothiazol-3(2H)-yl)propanamide; 2-(6-fluoro-2-oxo-1,3-benzothiazol- 3(2H)-yl)acetamide; 2-(6-methyl-2-oxo-1,3-benzothiazol-3(2H)-yl)acetamide; 6-fluoro-3-(1H-imidazol-1-ylmethyl)-1,3-benzoxazol-2(3H)-one; 1-(1H-imidazol-4-ylmethyl)pyrazolo[1,5-a]pyridin-2(1H)-one; 2-(6-chloro-3-oxo-3,4-dihydroisoquinolin- 2(1 H)-yl)propanamide; 5-chloro-2-(1H-imidazol-4-ylmethyl)-1,4-dihydroisoquinolin-3(2H)- one; 2-(6-chloro-2-oxo-3,4-dihydroquinolin-1(2H)-yl)acetamide; 2-(6-bromo-2-oxo-3,4-dihydroquinolin-1(2H)-yl)acetamide; 1-(1H-imidazol-4-ylmethyl)-3,4-dihydroquinolin-2(1H)-one; 2-(6-iodo-2-oxo-3,4-dihydroquinolin-1 (2H)-yl)acetamide; 2-(6-cyano-2-oxo-3,4-dihydroquinolin-1(2H)-yl)acetamide; 7-chloro-2-{[2-(trifluoromethyl)imidazo[1,2-a]pyridin-3-yl]methyl}-1,2,4,5-tetrahydro-3H-2-benzazepin-3-one; 7-chloro-2-(1H-imidazol-4-ylmethyl)-1 ,2,4,5-tetrahydro-3H-2-benzazepin-3-one; 7-chloro-3-(1H-imidazol-4-ylmethyl)-1 ,3,4,5-tetrahydro-2H-3-benzazepin-2-one; and 7-chloro-3-{[2-(trifluoromethyl)imidazo[1,2-a]pyridin-3-yl]methyl}-1,3,4,5-tetrahydro-2H-3-benzazepin-2- one.

In some instances, compounds of the present disclosure are those selected from the group consisting of: 1-(1H-imidazol-4-ylmethyl)-5-propylpiperidin-2-one; 1-(1H-imidazol-1-ylmethyl)-5-propylpiperidin-2-one; 1-(imidazo[1,2-a]pyridin-3-ylmethyl)-5-propylpiperidin-2-one; 1-(1H-imidazol-1-ylmethyl)-5-phenylpiperidin-2-one; 1-(imidazo[1,2-a]pyridin-3-ylmethyl)-4-phenylpiperidin-2-one; 1-(1H-imidazol-1-ylmethyl)-4-phenylpiperidin-2-one; 1-(imidazo[1,2-a]pyridin-3-ylmethyl)-4-propylpiperidin-2-one; 1-(1H-imidazol-5-ylmethyl)-4-propylpiperidin-2-one; 1-(1H-imidazol-1-ylmethyl)-4-propylpiperidin-2-one; 1-(1 H-imidazol-4-ylmethyl)-1H-thieno[3,4-b]pyrrol-2(3H)-one; 6-bromo-3-(1H-imidazol-1-ylmethyl)-1,3- benzothiazol-2(3H)-one; 2-(6-bromo-2-oxo-1,3-benzothiazol-3(2H)-yl)propanamide; and 5-chloro-2-(1H-imidazol-4-ylmethyl)-1 ,4-dihydroisoquinolin-3(2H)-one.

The following paragraphs provide definitions of the various chemical moieties that make up the compounds according to the disclosure and are intended to apply uniformly through- out the specification and claims unless an otherwise expressly set out definition provides a broader definition.

"C-|_β alkyl" refers to alkyl groups having 1 to 6, or 1 to 4 carbon atoms. This term is exemplified by groups such as methyl, ethyl, n-propyl, isopropyl, n-butyl, isobutyl, tert- butyl, n-pentyl, n-hexyl, trifluoromethyl and the like. "Aryl" refers to an unsaturated aromatic carbocyclic group of from 6 to 14 carbon atoms having a single ring (e.g., phenyl) or multiple condensed rings (e.g., naphthyl). Preferred aryl include phenyl, naphthyl, phenantrenyl and the like.

"Heterocycle" refers to a saturated or unsaturated ring system containing, in addition to carbon atoms, at least one hetero atom, such as nitrogen, oxygen and/or sulfur. "Heterocycle" includes both "heteroaryl" and "heterocycloalkyl".

"Heteroaryl" refers to a monocyclic heteroaromatic, or a bicyclic or a tricyclic fused-ring heteroaromatic group. Particular examples of heteroaromatic groups include optionally substituted pyridyl, pyrrolyl, furyl, thienyl, imidazolyl, oxazolyl, isoxazolyl, thiazolyl, isothiazolyl, pyrazolyl, 1,2,3-triazolyl, 1,2,4-triazolyl, 1 ,2,3-oxadiazolyl, 1,2,4-oxadia-zolyl, 1,2,5-oxadiazolyl, 1,3,4-oxadiazolyl, 1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazopyridinyl, benzothiazolyl, benzoxazolyl, quinolizinyl, quinazolinyl, pthalazinyl, quinoxalinyl, cinnolinyl, napthyridinyl, pyrido[3,4-b]pyridyl, pyrido[3,2-b]pyridyl, pyrido[4,3-b]pyridyl, quinolyl, isoquinolyl, tetrazolyl, 5,6,7,8- tetrahydroquinolyl, 5,6,7,8-tetrahydroisoquinolyl, purinyl, pteridinyl, carbazolyl, xanthenyl,benzoquinolyl, imidazopyrimidinyl, imidazopyridazinyl, imidazothiazolyl or imidazothiadiazolyl.

"C2-6 alkenyl" refers to alkenyl groups preferably having from 2 to 6 carbon atoms and having at least 1 or 2 sites of alkenyl unsaturation. Preferable alkenyl groups include ethenyl (vinyl, -CH=CH2), n-2-propenyl (allyl, -CH2CH=CH2) and the like.

"C2-6 alkynyl" refers to alkynyl groups preferably having from 2 to 6 carbon atoms and having at least 1-2 sites of alkynyl unsaturation, preferred alkynyl groups include ethynyl (-C=CH), propargyl (-CH2C≡CH), and the like.

"C3.8 cycloalkyl" refers to a saturated carbocyclic group of from 3 to 8 carbon atoms having a single ring (e.g., cyclohexyl) or multiple condensed rings (e.g., norbornyl). Preferred cycloalkyl include cyclopropyl, cyclobutyl, cyclopentyl, cyclohexyl, norbornyl and the like.

"Heterocycloalkyl" refers to a C3.8 cycloalkyl group according to the definition above, in which 1 to 3 carbon atoms are replaced by hetero atoms chosen from the group consisting of O, S, NR, R being defined as hydrogen or C-|.g alkyl. "Alkoxy" refers to the group -O-R where R includes " C-μg alkyl", "C2-6 alkenyl", "C2-6 alkynyl", "C3.8 cycloalkyl", "heterocycloalkyl", "aryl", "heteroaryl".

"Amino" refers to the group -NRR' where each R, R' is independently hydrogen, "C-|.g alkyl", "C2-6 alkenyl", "C2-6 alkynyl", "C3-8 cycloalkyl", "heterocycloalkyl", "aryl", "heteroaryl", and where R and R', together with the nitrogen atom to which they are attached, can optionally form a 3-8-membered heterocycloalkyl ring.

"Amido" refers to the group -C(=O)NRR' where each R, R' is independently hydrogen, " C-|_5 alkyl", "C2-6 alkenyl", " C2-6 alkynyl", " C3.8 cycloalkyl", "heterocycloalkyl", "aryl",

"heteroaryl", and where R and R', together with the nitrogen atom to which they are attached, can optionally form a 3-8-membered heterocycloalkyl ring.

"Acylamino" refers to the group -NRC(O)R' wherein R and R' are as defined hereabove for the amino group.

"Ureido" refers to the group -NR"C(O)NRR' wherein R and R' are as defined hereabove for the amino group, and R" is as defined hereabove. "Sulfanyl" refers to the group -SR where R is "C-|.g alkyl", "C2-6 alkenyl", "C2-6 alkynyl", "C3.8 cycloalkyl", "heterocycloalkyl", "aryl" or "heteroaryl".

"Sulfinyl" refers to the group -S(=O)R where R is "C-|.g alkyl", "C2-6 alkenyl", "C2-6 alkynyl", "C3.8 cycloalkyl", "heterocycloalkyl", "aryl" or "heteroaryl".

"Sulfonyl" refers to the group -S(=O)2R where R is "C-|.g alkyl", "C2-6 alkenyl", "C2-6 alkynyl", "C3.8 cycloalkyl", "heterocycloalkyl", "aryl" or "heteroaryl".

"Halogen" refers to fluoro, chloro, bromo and iodo atoms.

"Substituted or unsubstituted" : Unless otherwise constrained by the definition of the individual substituent, the above set out groups, like "alkyl", "alkenyl", "alkynyl", "aryl" and

"heteroaryl" etc. groups can optionally be substituted with from 1 to 5 substituents selected from the group consisting of "C-|.g alkyl", "C2-6 alkenyl", "C2-6 alkynyl"

"cycloalkyl", "heterocycloalkyl", "amino", "amido", "acylamino", "ureido", "aryl", "heteroaryl", "alkoxy", "halogen", cyano, hydroxy, mercapto, nitro, "amido", "sulfanyl", "sulfinyl", "sulfonyl" and the like.

The acid addition salt form of a compound of formula (I) that occurs in its free form as a base can be obtained by treating the free base with an appropriate acid such as an inorganic acid, for example, a hydrohalic such as hydrochloric or hydrobromic, sulfuric, nitric, phosphoric and the like; or an organic acid, such as, for example, acetic, trifluoroacetic, hydroxyacetic, propanoic, lactic, pyruvic, malonic, succinic, maleic, fumaric, malic, tartaric, citric, methanesulfonic, ethanesulfonic, benzenesulfonic, p-toluenesulfonic, cyclamic, salicylic, p-aminosalicylic, pamoic and the like.

The compounds of formula (I) containing acidic protons may be converted into their therapeutically active, non-toxic base addition salt forms, e.g. metal or amine salts, by treatment with appropriate organic and inorganic bases. Appropriate base salt forms include, for example, ammonium salts, alkali and earth alkaline metal salts, e.g. lithium, sodium, potassium, magnesium, calcium salts and the like, salts with organic bases, e.g. N-methyl-D-glucamine, hydrabamine salts, and salts with amino acids such as, for example, arginine, lysine and the like.

Conversely said salt forms can be converted into the free forms by treatment with an appropriate base or acid.

Compounds of the formula (I) and their salts can be in the form of a solvate, which is included within the scope of the present disclosure. Such solvates include for example hydrates, alcoholates and the like.

Many of the compounds of formula (I) and some of their intermediates have at least one stereogenic center in their structure. This stereogenic center may be present in a R or a S configuration, said R and S notation is used in correspondence with the rules described in Pure Appl. Chem., 45 (1976) 11-30.

The disclosure also relates to all stereoisomeric forms such as enantiomeric and diastereoisomeric forms of the compounds of formula (I) or mixtures thereof (including all possible mixtures of stereoisomers). With respect to the present disclosure reference to a compound or compounds is intended to encompass that compound in each of its possible isomeric forms and mixtures thereof, unless the particular isomeric form is referred to specifically.

Compounds according to the present disclosure may exist in different polymorphic forms. Although not explicitly indicated in the above formula, such forms are intended to be included within the scope of the present disclosure.

Some of the compounds of formula (I) may also exist in tautomeric forms. Such forms although not explicity indicated in the above formula are intended to be included within the scope of the present disclosure.

The disclosure also includes within its scope pro-drug forms of the compounds of formula (I) and its various sub-scopes and sub-groups.

In a specific instance, the present disclosure concerns a compound selected from the group consisting of: (2S)-2-[3-(4-nitrophenyl)-5-oxoimidazolidin-1-yl]butanamide; (2S)-2-[3-(2,4-dinitrophenyl)-5-oxoimidazolidin-1-yl]butanamide; (2S)-2-(5-oxo-3-phenylimidazolidin-1-yl)butanamide; 2-[5-(iodomethyl)-2-oxo-1 ,3-oxazolidin-3-yl]butanamide;

2-(2-oxo-2,5-dihydro-1H-pyrrol-1-yl)butanamide; 2-(2-oxo-4-phenyl-2,5-dihydro-1H-pyrrol-1-yl)butanamide; 2-(4-methyl-2-oxo-2,5-dihydro-1H-pyrrol-1-yl)butanamide; (+)-(2S)-2-(2-oxo-4-propyl-2,5-dihydro-1H-pyrrol-1-yl)butanamide; (2S)-2-(2-oxo-5-propyl-1,3-thiazol-3(2H)-yl)butanamide; 2-(2-oxo-5-propyl-1,3-thiazol-3(2H)-yl)propanamide; 2-(5-butyl-2-oxo-1,3-thiazolidin-3-yl)butanamide; 2-(5-butyl-2-oxo-1,3-thiazolidin-3-yl)propanamide; 2-(2-oxo-5-phenyl-1,3-thiazolidin-3-yl)propanamide; 2-(2-oxo-5-propyl-1,3-thiazolidin-3-yl)butanamide; 2-(2-oxo-5-phenyl-1,3-thiazolidin-3-yl)butanamide; 2-(2-oxo-5-propyl-1,3-thiazolidin-3-yl)propanamide; (2S)-2-[2-oxo-5-(2,2,2-trifluoroethyl)-1 ,3-thiazolidin-3-yl]butanamide; 1-{[6-chloro-2-(trifluoromethyl)imidazo[1,2-b]pyridazin-3-yl]methyl}piperidin-2-one; 1-(1H-imidazol-4-ylmethyl)-5-propylpiperidin-2-one; 1-(1H-imidazol-1-ylmethyl)-5-propylpiperidin-2-one; 1-(imidazo[1,2-a]pyridin-3-ylmethyl)-5-propylpiperidin-2-one; 1-(1H-imidazol-1-ylmethyl)-5-phenylpiperidin-2-one; 1-(imidazo[1,2-a]pyridin-3-ylmethyl)-5-phenylpiperidin-2-one; 1-(imidazo[1,2-a]pyridin-3-ylmethyl)-4-phenylpiperidin-2-one; 1-(1H-imidazol-1-ylmethyl)-4-phenylpiperidin-2-one; 1-(imidazo[1,2-a]pyridin-3-ylmethyl)-4-propylpiperidin-2-one; 1-(1H-imidazol-5-ylmethyl)-4-propylpiperidin-2-one; 1-(1H-imidazol-1-ylmethyl)-4-propylpiperidin-2-one; 1-{[6-chloro-2-(trifluoromethyl)imidazo[1,2-b]pyridazin-3-yl]methyl}azepan-2-one; 1-(1H-imidazol-5- ylmethyl)-5-propylazepan-2-one; 5-propyl-1-{[2-(trifluoromethyl)imidazo[1,2-a]pyridin-3-yl]methyl}azepan-2-one; 1-(1H-imidazol-5-ylmethyl)-5-phenylazepan-2-one; 5-phenyl-1-{[2-(trifluoromethyl)imidazo[1,2-a]pyridin-3-yl]methyl}azepan-2-one; 1-(1H-imidazol-5-ylmethyl)-6-propylazepan-2-one; 1-(1H-imidazol-4-ylmethyl)-4-propylazepan-2-one; 4-(1H-imidazol-4-ylmethyl)-4,6-dihydro-5H-thieno[3,2-b]pyrrol-5-one; 2-(5-oxo-5,6-dihydro-4H-thieno[3,2-b]pyrrol-4-yl)acetamide; 4-{[2-(trifluoromethyl)imidazo[1,2-a]pyridin-3-yl]methyl}-4,6-dihydro-5H-thieno[3,2-b]pyrrol-5-one; 4-{[2-(trifluoromethyl)imidazo[1,2-a]pyridin-3-yl]methyl}hexahydro-5H-thieno[3,2-b]pyrrol-5-one; 1-(1H-imidazol-4-ylmethyl)-1H-thieno[3,4-b]pyrrol-2(3H)-one; 2-(6-bromo-2-oxo-1,3-benzothiazol-3(2H)-yl)acetamide; 2-(2-oxo-1,3-benzothiazol-3(2H)-yl)acetamide; 2-(6-chloro-2-oxo-1,3-benzothiazol-3(2H)-yl)acetamide; 6-bromo-3-(1H-imidazol-1-ylmethyl)-1,3-benzothiazol-2(3H)-one; 6-bromo-3-(2-oxopropyl)-1,3-benzothiazol-2(3H)-one; 2-(6-nitro-2-oxo-1,3-benzothiazol-3(2H)- yl)acetamide; 2-(6-bromo-2-oxo-1,3-benzothiazol-3(2H)-yl)propanamide; 2-(6-bromo-2-oxo-1,3-benzothiazol-3(2H)-yl)propanamide; 2-(6-fluoro-2-oxo-1 ,3-benzothiazol-3(2H)-yl)acetamide; 2-(6-methyl-2-oxo-1,3-benzothiazol-3(2H)-yl)acetamide; 6-fluoro-3-(1H-imidazol-1-ylmethyl)-1,3-benzoxazol-2(3H)-one; 1-(1H-imidazol-4-ylmethyl)pyrazolo[1,5-a]pyridin-2(1H)-one; 2-(6-chloro-3-oxo-3,4-dihydroisoquinolin-2(1H)-yl)propanamide; 5-chloro-2-(1H-imidazol-4-ylmethyl)-1,4-dihydroisoquinolin-3(2H)-one; 2-(6-chloro-2-oxo-3,4-dihydroquinolin-1(2H)-yl)acetamide; 2-(6-bromo-2-oxo-3,4-dihydroquinolin-1 (2H)-yl)acetamide; 1-(1H-imidazol-4-ylmethyl)-3,4-dihydroquinolin-2(1H)-one; 2-(6-iodo-2-oxo-3,4-dihydroquinolin-1(2H)-yl)acetamide; 2-(6-cyano-2-oxo-3,4-dihydroquinolin-1(2H)-yl)acetamide; 7-chloro-2-{[2-(trifluoromethyl)imidazo[1 ,2-a]pyridin-3-yl]methyl}-1,2,4,5-tetrahydro-3H-2-benzazepin-3-one; 7-chloro-2-(1H-imidazol-4-ylmethyl)-1,2,4,5-tetrahydro-3H-2-benzazepin-3-one; 7-chloro-3-(1H-imidazol-4-ylmethyl)-1,3,4,5-tetrahydro-2H-3-benzazepin-2-one; and 7-chloro-3-{[2-(trifluoromethyl)imidazo[1,2-a]pyridin-3-yl]methyl}-1,3,4,5-tetrahydro-2H-3-benzazepin-2-one.

xvi) UK Patent 1,039,113

The new compounds according to the present disclosure are N-substituted lactams of the general formula:

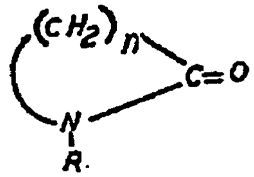

wherein N is a whole number of from 3 to 5 and R represents a

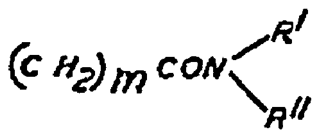

radical in which m is 0, 1 or 2 and R’ is a hydrogen atom or an alkyl, cycloalkyl, alkenyl or alkynyl radical, which may contain 3 to 6 carbon atoms, or an aryl radical, and R" is a hydrogen atom or an alkyl radical, or both R’ and R", together with the nitrogen atom to which they are attached, form a heterocyclic ring, such as 5 a pyrrolidine ring.

xvii) UK Patent 1,309,692

According to the present disclosure, there are provided new N-substituted lactams of the general formula:

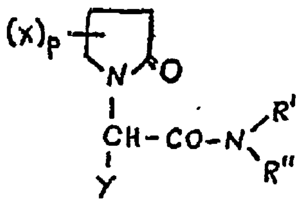

wherein X is a hydrogen atom or an alkyl, alkenyl or alkynyl radical containing 1 to 6 carbon atoms, p is a whole number of from 1 to 6, Y is a hydrogen atom or an alkyl, alkenyl or alkynyl radical containing 1 to 6 carbon atoms or a cycloalkyl radical and R' and R", which may be the same or different, are hydrogen atoms or alkyl, alkenyl, alkynyl, cycloalkyl or aryl radicals or R' and R", together with the nitrogen atom to which they are attached, form a heterocyclic radical which may contain further heteroatoms, with the proviso that at least one of the symbols X and Y is other than a hydrogen atom.

**Acetylcholinesterase Inhibitor**

Among the AChEIs useful for the methods and compositions this disclosure are the following:

zanapezil, ganstigmine, phenethylnorcymserine (PENC), cymserine, thiacymserine, SPH 1371 (galantamine plus), ER 127528, RS 1259, F3796, tetrahydroaminoacridine,DFP (diisopropylfluorophosphate), Ladostigil, Memoquin, SP-004, BGC-20-1259, NP-0361, ZT-1, INM-176, pyridostigmine, ambenonium, demarcarium, edrophonium, pralidoxime, Amirine, SW-10888, MF-217, Ro 45-5934, HP-290, ENA 713, CP 118.954, ONO 1603, eptastigmine, extract of magnolia, for example, magnolol, honokiol, taspine and asimilobine, alacepril, benazepril, captopril, ceronapril, cilazapril, delapril, enalapril, enalaprilat, fosinopril, imidapril, lisinopril, moexipril, moveltipril, omapatrilat, perindopril, quinapril, ramipril, sampatrilat, sprirapril, temocaptril, trandolapril, piperidinyl-alkanoyl heterocyclic compounds, N-benzyl-piperidine derivatives, 4-(1-benzylpiperidyl)-substituted fused quinoline derivatives, monoamine acridines and their derivatives, cyclic amide derivatives, carbonic acid derivatives, saliclate, obidoxime, trimedoxime, diacetyl monoxim, demecarium, icopezil (5,7-dihydro-3-(2-(1-(phenylmethyl)-4-piperidinyl)ethyl)-6H-pyrrolo(3,2-f)-1,2-benzisoxazol-6-one), heptylphysostigmine, E-2020, Citicoline, velnacrine maleate, sulfonyl fluorides such as methanesulfonyl fluoride (Moos and Hershenson, 1989) and phenylmethylsulfonyl fluoride (Ferris, 1990; Pope and Padilla, 1990), huperzine B, miotine, suronacrine and its maleate, 7-methoxytacrine, SM-10888 and its citrate, ENA-713, TAK-147, CP-118954, zifrosilone, (SR,9R)-5-(r-chloro-2-hydroxy-3-methoxybenzylidene-amino)-11-ethlidene-7-methyl-1,2,5,6,9,10-hexahydro-5,9-methanocycloocta[b]pyridin-2-one (ZT 1), stacofylline, SPH 1371, SPH 1373 and SPH 1375, tolserine, 1-(3-fluorobenzyl)-4-[(2-fluoro-5,6-dimethoxy-1-indanone-2-yl)methyl]piperidine hydrochloride (ER 127528), thiatolserine, (-)-12-amino-3-chloro-9-ethyl-6,7,10,11-tetrahydro-7,11-methanocycloocta [b] quinoline hydrochloride (huperine X), N,N-dimethylcarbamic acid 4-[1(S)-(methylamino)-3-(4-nitrophenoxy) propyl]phenyl ester hemifumarate (RS 1259), 2-[2-(1-benzylpiperidin-4-yl)ethyl]-2,3-dihydro-9-methoxy-1H-pyrrolo[3,4-b] quinolin-1-one hemifumerate (T 82), 1,3-dichloro-6,7,8,9,10,12-hexahydroazepino[2,1-b]-quinazoline (CI 1002), N-heptylcarbamic acid, 2,4a,9-trimethyl-2,3,4,4a,9,9a-hexahydro-1,2-oxazino[6,5-b]indol-6-yl ester-L-tartrate (CHF 2060), 3-(2-[1-(1,3-dioxolan-2-ylmethyl)piperidin-4-yl]ethyl)-3, 4-dihydro-2H-1,3benzoxazine-2,4-dione hydrochloride (E 2030), N-[10-(diethylamino)decyl]carbamic acid (3aS,8aR)-1,3a,8-trimethyl-1,2,3,3a,8,8a-hexahydropyrrolo[2,3-b]indol-5-yl ester (MF 247), 5-amino-6-chloro-4-hydroxy-3,4-dihydro-1H-thiopyrano-[3,4-b]quinoline (MF 8615), N-[8-(cis-2,6-dimethylmorpholin-4-yl)octyl]carbamic acid (3aS,8aR)-1,3a,8trimethyl-1,2,3, 3a,8,8a-hexahydropyrrolo[2,3-b]indol-5-yl ester L-bitartrate hydrate (MF 268), (-) N-(3-piperidinopropyl)-N-demethylgalantamine (SPH 1286), N-propargyl-3Raminoindan-5-yl-ethyl methyl carbamate (TV 3326), cryptotanshinone, dihydrotanshinone I, and tanshinone I.

Among the acetylcholinesterase inhibitors useful for the methods and compositions of this disclosure are those compounds or agents as referred to in:

i) U.S. Patent Application Publication No. 20080103105

In one instance, an AChel is metrifonate, which is also known as metriphonate or trichlorfon or its active metabolite, 2,2-dimethyldichlorovinyl phosphate (or dichlorvos or DDVP). Metrifonate is represented by the following

formula:

(CH3O)2-PO-CHOH-OCl3.

Echothiophate is also known as ecothiopate, echothiophate iodide, phospholine iodide, (2-Mercaptoethyl)trimethylammonium S-ester with O,O'-diethylphosphorothioate, BRN 1794025, ecothiopatum, or phospholine. Echothiophate is referenced by CAS Registry Number 6736-03-4.

In other instances, an ACHE inhibitor is an aminoacridine such as tacrine or ipidacrine as non-limiting examples. Tacrine/COGNEX® (1,2,3,4-tetrahydro-9-aminoacridine hydrochloride) is also known as tetrahydroaminoacridine or THA. Tacrine is referenced by CAS Registry Number 321-64-2. Ipidacrine is also known as Amiridin.

In additional instances, an AChE inhibitor is a carbamate such as physostigmine, neostigmine, or rivastigmine as non-limiting examples.

Physostigmine, also known as 1,2,3,3a,8,8a-hexahydro-1,3a,8-trimethyl-, methylcarbamate (ester) or (3aS,8aR)-pyrrolo[2,3-b]indol-5-ol, is referenced by CAS number 57-47-6. It is a tertiary amine capable of crossing the blood-brain barrier.

Neostigmine, or m-hydroxyphenyl)trimethyl-dimethylcarbamate(ester) ammonium, is referenced by CAS number 59-99-4.

Rivastigmine is also known as rivastigmine tartrate or (S)-N-Ethyl-N-methyl-3-[1-(dimethylamino)ethyl]-phenyl carbamate hydrogen-(2R,3R)-tartrate or SDZ ENA 713 or ENA 713. The reference for rivastigmine is CAS Registry Number 123441-03-2.

In further instances, an AChE inhibitor is a carbamate phenanthrine derivative such as galantamine or its hydrogen bromide form as non-limiting examples.

Galantamine is also known as (4aS,6R,8aS)-4a,5,9,10,11,12-hexahydro-3-methoxy-11-methyl-6H-benzofuro(3a,3,2-ef)(2)benzazepin-6-ol and is often used in its hydrogen bromide form. Galantamine is referenced by CAS number 357-70-0.

An AChE inhibitor may also be a piperidine derivative, such as donepezil as a non-limiting example. Donepezil is also known as 2,3-dihydro-5,6-dimethoxy-2-((1-(phenylmethyl)-4-piperidinyl)methyl)-1H-inden-1-one, and is referenced by CAS number 120014-06-4.

Itopride may also be an AChE inhibitor for use in instances disclosed herein.

Itopride HCl is referenced by CAS Registry Number 122898-67-3. In one instance, a total daily dose range for itopride HCl is from about 25 mg to about 1000 mg, or between about 100 mg to about 300 mg. In some instances, the AChE inhibitor, or neurogenic agent, is the N-oxide derivative of itopride, which is the primary human metabolite of itopride HCl.

Another AChE inhibitor for use in the disclosed instances is (-)-huperzine A, which is also referred to as HupA and 1-amino-13-ethylidene-11-methyl-6-aza-tricyclo[7.3.1.02,7]trideca-2(7),3,10-trien-5-one. It is referenced by CAS number 102518-79-6.

A further instance of an ACHE inhibitor is phenserine, the structure and synthesis of which is described in U.S. Pat. No. 6,495,700.

Although galantamine is only an example of an acetylcholinesterase inhibitor for use in the present disclosure, other acetylcholinesterase inhibitors may equally be used. Examples of such compounds include galantamine derivatives, such as those compounds of formula (I):

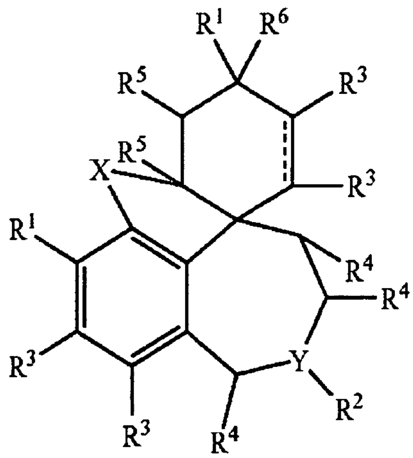

(I)

in which:

the dotted line indicates that there is a single or double carbon-carbon bond;
the two symbols R1 are the same as or different from each other and each represents a hydrogen atom, a hydroxy group, an alkyl group, an aryl group, an aralkyl group, a hydroxyalkyl group, a thioalkyl group, a carboxyalkyl group, a carboxyalkylamino group, an alkylamino group, an acyl group, a cyano group, a sulphhydryl group, a C1-C6 alkoxy group, an alkylthio group, an aryloxy group, an arylthio group, an aliphatic or aromatic carbamoyl group, an aralkoxy group, an aralkylthio group, an aryloxymethyl group, an alkanoyloxy group, a hydroxyalkanoyloxy group, a benzoyloxy group, a benzoyloxy group substituted by one or more groups R3, as defined below, or an aryloxycarbonyl group;
R2 represents a hydrogen atom, a C1-C6 alkyl group, a C2-C6 alkenyl group (e.g. an allyl group), an aralkyl group, said alkyl, alkenyl and aralkyl groups being unsubstituted or being substituted by at least one halogen atom, a cycloalkyl group, a hydroxy group, an alkoxy group, a nitro group, an amino group, an aminoalkyl group, an acylamino group, an aromatic or non-aromatic heterocyclic group (e.g. an α - or β-furyl group, an α - or β-thienyl group, an α - or β-thenyl group, a pyridyl group, a pyrazinyl group, or a pyrimidyl group), an alkyl group substituted by an aromatic heterocyclic group, an aryl group (e.g. a phenyl group), an aralkyl group, a cyano group, an aroyl group, or a cycloalkylmethyl group;
the symbols R3 are the same as or different from each other and each represents a hydrogen atom, a hydroxy group, an alkyl group, an aryl group, an aralkyl group, a hydroxyalkyl group, a thioalkyl group, a sulphhydryl group, a C1-C6 alkoxy group, an aryloxy group, an arylthio group, an aralkoxy group, an aralkylthio group, a nitro group, an amino group, an alkylamino group, an arylamino group, an aralkylamino group, a halogen atom or a trifluoromethyl group;
the symbols R4 are the same as or different from each other and each represents a hydrogen atom, a halogen atom, a trifluoromethyl group, or a C1-C4 alkyl group; the two symbols R5 are the same as or different from each other and each represents a hydrogen atom, or a hydroxymethyl group;
R6 represents a hydrogen atom or a C1-C6 alkyl group, or, when the symbol R1 attached to the same carbon atom as R6 represents a hydrogen atom, R6 represents a group of formula (Ia):

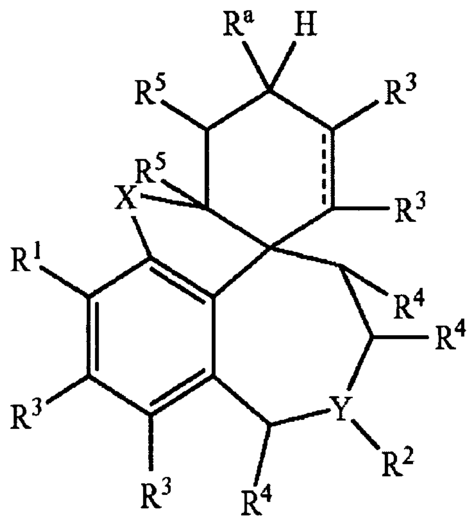

where Ra represents a linking bond, and R1, R2, R3, R4, R5, X and Y are as defined for formula (I);
or R6 and the symbol R1 attached to the same carbon atom as R6 together represent a semicarbazone;
X represents an oxygen atom or a group of formula -NR3, where R3 is as defined above;
Y represents a nitrogen atom or a phosphorus atom;
and pharmaceutically acceptable salts thereof.

In the compounds of formula (I) above, except where otherwise indicated, alkyl groups may be straight or branched chain and preferably have from 1 to 10 carbon atoms, and examples of these include the methyl, ethyl, propyl, isopropyl, butyl, isobutyl, sec-butyl, t-butyl, pentyl, hexyl, heptyl, octyl, nonyl and decyl groups. Alkoxy groups and other simple derivatives of the alkyl groups likewise preferably have from 1 to 10, more preferably from 1 to 6, carbon atoms.
Aryl and heterocyclic groups may be unsubstituted or they may be substituted by one or more substituents selected from the groups and atoms defined for R3, provided that any R3 substituent may not itself be further substituted by a substituted aryl or heterocyclic group.

Preferred halogen atoms are the fluorine, chlorine, bromine and iodine atoms. Preferred compounds of formula (I) are those in which the alkyl groups contain from 1 to 8, more preferably from 1 to 6, carbon atoms, halogen atoms are fluorine, chlorine or bromine atoms, aryl groups are the phenyl group (which may be substituted or unsubstituted, preferably unsubstituted), cycloalkyl groups contain from 3 to 7 ring carbon atoms (preferably cyclopropyl or cyclobutyl), acyl groups are lower (e.g. C2-C6 alkanoyl groups) and heterocyclic groups are aromatic and contain from 5 to 8 ring atoms (e.g. the thienyl, furyl, pyridyl, pyrrolyl or pyrazinyl groups).

Particularly preferred compounds for use in the present disclosure are those compounds of formula (II):

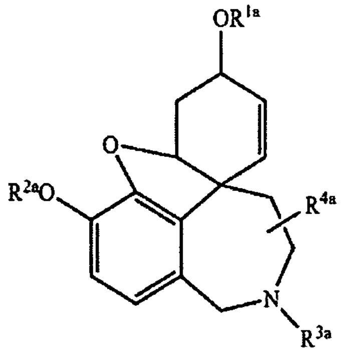

(II)

where:

R1a and R2a are the same as or different from each other and each represents a hydrogen atom, an acyl group (preferably a lower alkanoyl group, such as an acetyl group) or a C1-C6 alkyl group (such as a methyl, ethyl, propyl or isopropyl group);

R3a represents an alkyl, alkenyl or aralkyl group, any of which may be unsubstituted or substituted by one or more halogen atoms, or it represents a cycloalkyl, hydroxy, alkoxy, nitro, amino, aminoalkyl, acylamino, aromatic heterocyclic, aroyl, aroylalkyl or cyano group; and

R4a represents a hydrogen or halogen atom;

and pharmaceutically acceptable salts thereof, especially the hydrobromide, hydrochloride, methylsulphonate or methiodide.

Of these compounds, we particularly prefer galantamine and its salts or donepezil and its salts, especially the halides, such as galantamine hydrobromide or donepezil hydrochloride.

Galantamine derivatives which may be used in the present disclosure include norgalantamine, norgalantamine derivatives and epigalantamine.

Other compounds which may be used as the acetylcholinesterase inhibitor include: physostigmine, tacrine and tacrine analogues, fasciculin, metrifonate, heptylphysostigmine, norpyridostigmine, norneostigmine, neostigmine, pyridostigmine, huperzine or a prodrug therefor, rivastigmine or a prodrug therefor, gossypol or phenserine, or a prodrug therefor.

Another particularly preferred acetylcholinesterase inhibitor is donepezil and its salts, especially the halides, such as donepezil hydrochloride.

ii) U.S. Patent Application Publication No. 20070275959

In one instance, the disclosure is a carbamoyl ester having the following structure:

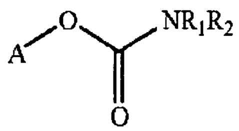

wherein A is selected from the group consisting of an unsubstituted aryl, a substituted aryl, an unsubstituted heteroaryl and a substituted heteroaryl; and R1 and R2 are each, independently or in combination, selected from the group consisting of a hydrogen, an unsubstituted alkyl, a substituted alkyl, an unsubstituted aralkyl, a substituted aralkyl, an unsubstituted

heteroalkyl, a substituted heteroalkyl, an unsubstituted heteroaralkyl, a substituted heteroaralkyl, an unsubstituted aryl, a substituted aryl, an unsubstituted heteroaryl, a substituted heteroaryl, an unsubstituted cycloalkyl, a substituted cycloalkyl, an unsubstituted heterocycloalkyl and a substituted heterocycloalkyl.

In an additional instance, the disclosure is a carbamoyl ester selected from the group consisting of:

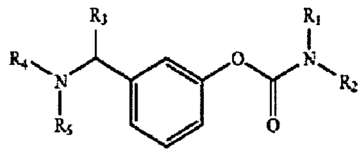

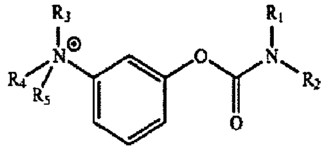

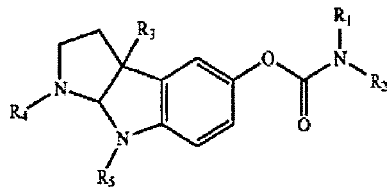

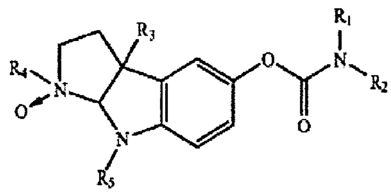

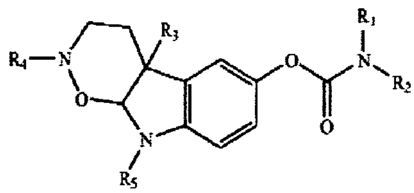

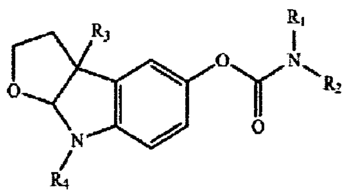

and

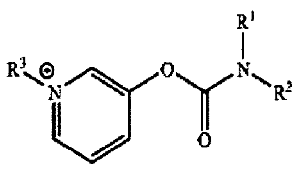

wherein R3, R4 and R5 are each, independently or in combination, selected from the group consisting of a hydrogen, an unsubstituted alkyl, a substituted alkyl, an unsubstituted aralkyl, a substituted aralkyl, an unsubstituted heteroalkyl, a substituted heteroalkyl, an unsubstituted heteroaralkyl, a substituted heteroaralkyl, an unsubstituted aryl, a substituted aryl, an unsubstituted heteroaryl, a substituted heteroaryl, an unsubstituted cycloalkyl, a substituted cycloalkyl, an unsubstituted heterocycloalkyl and a substituted heterocycloalkyl.

In still another instance, the disclosure is a carbamoyl ester selected from the group consisting of:

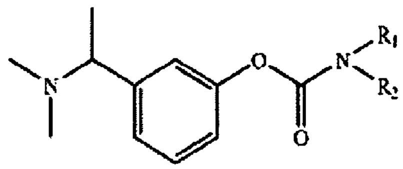

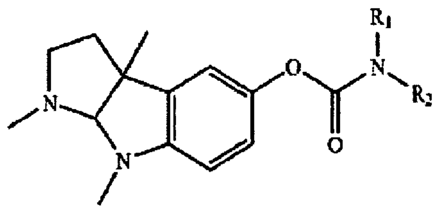

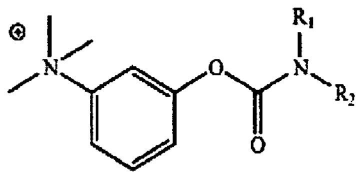

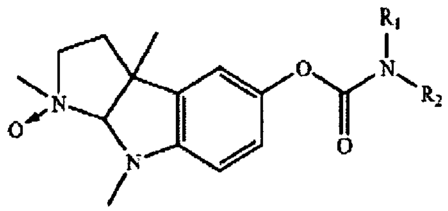

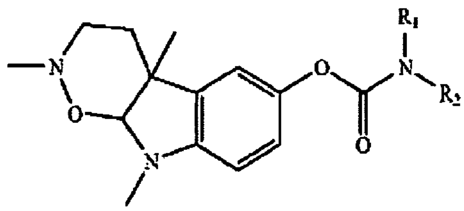

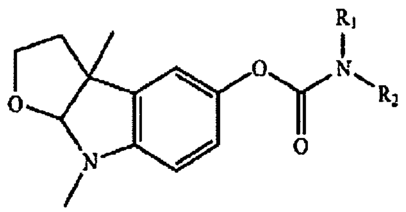

and

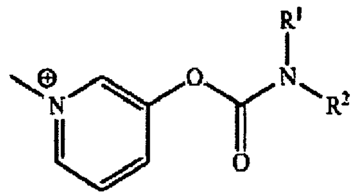

iii) European Patent Application No. EP1050303

In some instances of the disclosure, the acetylcholinesterase inhibitor is a compound of Formula 1:

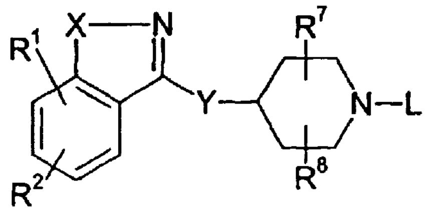

wherein $R^1$ and $R^2$ are each independently selected from the group consisting of hydrogen; (C1-C6) alkoxy; benzyloxy; phenoxy; hydroxy; phenyl; benzyl; halo; nitro; cyano; $-COR^5$; $-COOR^5$; $-CONHR^5$; $-NR^5R^6$; $-NR^5COR^6$; $-OCONR^5R^6$; $NHCOOR^5$; (C1-C6) alkyl which may be substituted with from 1 to 3 fluorine atoms; SOpCH2-phenyl or SOp(C1-C6) alkyl, wherein p is 0, 1 or 2; pyridylmethyloxy or thienylmethyloxy; 2-oxazolyl; 2-thiazolyl; and benzenesulfonamide; wherein the phenyl moieties of said phenoxy, benzyloxy, phenyl, benzyl and benzenesulfonamide groups, the pyridyl and thienyl moieties of said pyridylmethyloxy or thienylmethyloxy groups, and the oxazolyl and thiazolyl moieties of said 2-oxazolyl and 2-thiazolyl groups may be substituted with 1 or 2 substituents independently selected from the group consisting of halo, (C1-C4) alkyl, trifluoromethyl, (C1-C4) alkoxy, cyano, nitro and hydroxy; or $R^1$ and $R^2$ are attached to adjacent carbon atoms and form, together with the carbon atoms to which they are attached, a group of Formula 2:

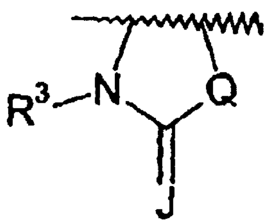

wherein $R^3$ is hydrogen or (C1-C6) alkyl; J is oxygen, sulfur or $NR^4$; $R^4$ is hydrogen or (C1-C4) alkyl; and Q is oxygen, sulfur, NH, CHCH3, C(CH3)2,-CH=CH-, or $(CH2)_l$ wherein $_l$ is an integer from 1 to 3;

X is oxygen or sulfur;

Y is -(CH2)m-, -CH=CH(CH2)n-, -NR<4)<CH2)m-, or -O(CH2)m-, wherein n is an integer from 0 to 3, and m is an integer from 1 to 3;

$R^5$ and $R^6$ are each independently selected from the group consisting of hydrogen, (C1-C6) alkyl, phenyl, and benzyl, wherein the phenyl moieties of said phenyl and benzyl groups may be substituted with 1 or 2 substituents independently selected from the group consisting of fluoro, chloro, bromo, iodo, (C1-C4) alkyl, trifluoromethyl, (C1-C4) alkoxy, cyano, nitro and hydroxy; or $NR^5R^6$ together form a 4 or 5 membered ring wherein one atom of the ring is nitrogen and the others are carbon, oxygen or nitrogen; or $NR^5COR^6$ together form a 4 or 5 membered lactam ring;

L is phenyl, phenyl-(C1-C6) alkyl, cinnamyl or pyridylmethyl, wherein the phenyl moieties of said phenyl and phenyl-(C1-C6) alkyl may be substituted with 1 to 3 substituents independently selected from the group consisting of (C1-C6) alkyl, (C1-C6) alkoxy, (C1-C4) alkoxycarbonyl, (C1-C6) alkylcarbonyl, $-OCONR^5R^6$, $- NHCOOR^5$, and halo; or L is a group of Formula 3:

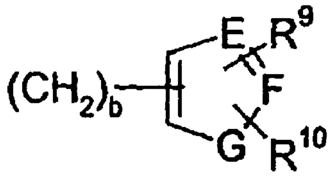

wherein b is an integer from 1 to 4; $R^9$ and $R^{10}$ are independently selected from the group consisting of hydrogen, (C1-C4) alkyl, halo, and phenyl; E and F are independently -CH- or nitrogen; and G is oxygen, sulfur or $NR^4$, with the proviso that when E and F are both nitrogen, one of $R^9$ and $R^{10}$ is absent; and $R^7$ and $R^8$ are independently selected from the group consisting of hydrogen, (C1-C6) alkyl, (C1-C6) alkoxycarbonyl, (C1-C6) alkylcarbonyl, and (C1-C6) alkoxy, with the proviso that said (C1-C6) alkoxy is not attached to a carbon that is adjacent to a nitrogen;

or a pharmaceutically acceptable salt or solvate thereof.

In some instances of the disclosure, the compound of Formula 1 is selected from the group consisting of:

5,7-dihydro-7-methyl-3-[2-[1-(phenylmethyl)-4-piperidinyl]ethyl]-6H-pyrrolo[4,5-f]-1,2-benzisoxazol-6-one;
5,7-dihydro-7-ethyl-3-[2[1-(phenylmethyl)-4-piperidinyl]ethyl]-6H-pyrrolo[4,5-f]-1,2-benzisoxazol-6-one;
5,7-dihydro-3-[2-[1-(2-chloro-5-thiophenemethyl)-4-piperidinyl]ethyl]6H-pyrrolo[4,5-f]-1,2-benzisoxazal-6-one;
5,7-dihydro-3-[2-[1-(2-methyl-4-thiazolemethyl)-4-piperidinyl]ethyl]-6H-pyrrolo[4,5-f]-1,2-benzisoxazol-6-one;
3-[2-[1-(3-bromophenylmethyl)-4-piperidinyl]ethyl]-5,7-dihydro-6H-pyrrolo[4,5-f]-1,2-benzisoxazol-6-one;
3-[2-[1-(4-bromophenylmethyl)-4-piperidinyl]ethyl]-5,7-dihydro-6H-pyrrolo[4,5-f]-1,2-benzisoxazol-6-one;
5,7-dihydro-3-[3-[1-(phenylmethyl)-4-piperidinyl]propyl]-6H-pyrrolo[4,5-f]-1,2-benzisoxazol-6-one;
6,8-dihydro-3-[2-[1-(phenylmethyl)-4-piperidinyl]ethyl]-7H-pyrrolo[5,4-g]-1,2-benzisoxazal-7-one; and
5,7-dihydro-3-[3-[1-(phenylmethyl)-4-piperidinyl]ethyl]-6H-pyrrolo[4,5-f]-1,2-benzisoxazol-6-one;
In some instances of the disclosure, the compound of Formula 1 is 5,7-dihydro-3-[3-[1-(phenylmethyl)-4-piperidinyl]ethyl]-6H-pyrrolo[4,5-f]-1,2-benzisoxazol-6-one.

iv) International Patent Application Publication No. WO2000/030446

a) An acetylcholinesterase inhibitor selected from the group consisting of galanthamine, lycoramine and analogs of said compounds wherein the methoxy group thereof is replaced by another alkoxy group of from one to six carbon atoms, a hydroxy group, hydrogen, an alkanoyloxy group, a benzoyloxy or substituted benzoyloxy group, a carbonate group or a carbamate group.
b) An acetylcholinesterase inhibitor is selected from the group consisting of analogs of galanthamine and lycoramine wherein the hydroxy group of galanthamine or lycoramine is replaced by an alkoxy group of from one to six carbon atoms, hydrogen, an alkanoyloxy group, a benzoyloxy or substituted benzoyloxy group, a carbonate group or a carbamate group.
c) An acetylcholinesterase inhibitor is selected from the group consisting of analogs of galanthamine and lycoramine wherein the N-methyl group of galanthamine or lycoramine is replaced by hydrogen, alkyl, benzyl or a cyclopropylmethyl group or a substituted or unsubstituted benzoyloxy group.
d) An acetylcholinesterase inhibitor as in a) selected from the group consisting of galanthamine, lycoramine and analogs thereof wherein the methoxy group of such compounds is replaced by a hydrogen, hydroxy or alkoxy group of from two to six carbon atoms or an acyloxy group of from one to seven carbon atoms.
e) An acetylcholinesterase inhibitor as in a) wherein said acetylcholinesterase inhibitor is selected from the group consisting of analogs of galanthamine or lycoramine wherein the methoxy group thereof is replaced by a mono or dialkyl carbamate or carbonate group wherein the alkyl groups contain from 1 to 8 carbon atoms
f) An acetylcholinesterase inhibitor as in e) wherein the alkyl group or groups of said carbonate or carbamate groups comprise from 4 to 6 carbon atoms
g) An acetylcholinesterase inhibitor as in a) wherein said acetylcholinesterase inhibitor is selected from the group consisting of analogs of galanthamine or lycoramine wherein the hydroxy group thereof is replaced by a mono or dialkyl carbamate or carbonate group wherein the alkyl groups contain from 1 to 8 carbon atoms.
h) An acetylcholinesterase inhibitor as in f) wherein the alkyl group or groups of said carbonate or carbamate groups comprise from 4 to 6 carbon atoms.
i) An acetylcholinesterase inhibitor as in a) wherein said acetylcholinesterase inhibitor is selected from the group consisting of analogs of galanthamine or lycoramine wherein the methoxy group thereof is replaced by an aryl

carbamate or carbonate group wherein said aryl group is selected from phenyl, naphthyl, substituted phenyl and substituted naphthyl groups wherein said substituent is selected from alkyl and alkoxy groups of from 1 to 6 carbon atoms, trifluoro methyl groups and halo groups.

j) An acetylcholinesterase inhibitor as in b) wherein said acetylcholinesterase inhibitor is selected from the group consisting of analogs of galanthamine and lycoramine wherein the hydroxy group thereof is replaced by an aryl carbamate or carbonate group wherein said aryl group is selected from phenyl, naphthyl, substituted phenyl and substituted naphthyl groups wherein said substituent is selected from alkyl and alkoxy groups of from 1 to 6 carbon atoms, trifluoro methyl groups and halo groups.

k) An acetylcholinesterase inhibitor as in b) wherein said acetylcholinesterase inhibitor is selected from the group consisting of galanthamine, lycoramine and analogs thereof wherein the hydroxy group of such compounds is replaced by a hydrogen or alkoxy group of from one to six carbon atoms or an acyl group of from one to seven carbon atoms.

l) An acetylcholinesterase inhibitor as in a) wherein said acetylcholinesterase inhibitor is selected from the group consisting of analogs of galanthamine and lycoramine wherein the hydroxy group of galanthamine or lycoramine is replaced by an alkoxy group of from one to six carbon atoms, hydrogen, an alkanoyloxy group, a benzoyloxy or substituted benzoyloxy group, a carbonate group or a carbamate group.

v) International Patent Application Publication No. WO2006/070394

An AChEI that is a quinoline derivative represented by formula 1 and 2 below:

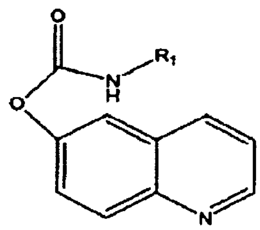

1

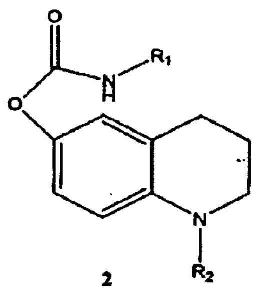

2

wherein Ri= alkyl, aryl; R2= H, alkyl, aralkyl.

In one instance, substituted carbamic acid quinolinyl esters obtained are selected from the group consisting of: Ia. hexyl-carbamic acid quinolin-6-yl ester Ib. heptyl-carbamic acid quinolin-6-yl ester Ic. (2-chloro-phenyl)-carbamic acid quinolin-6-yl ester Id. (3-bromo-phenyl)-carbamic acid quinolin-6-yl ester 2a. hexyl-carbamic acid 1, 2, 3, 4-tetrahydro-quinolin-6-yl ester 2b. heptyl-carbamic acid 1, 2, 3, 4-tetrahydro-quinolin-6-yl ester 2c. (3-bromo-phenyl)-carbamic acid 1,2,3,4-tetrahydro-quinolin-6-yl ester 2d. (2-chloro-phenyl)-carbamic acid 1,2,3,4-tetrahydro-quinolin-6-yl ester 2e. (4-chloro-3-trifluoromethyl-phenyl)-carbamic acid 1,2,3,4-tetrahydro-quinolin-6-yl ester 2f. (4-bromo-phenyl)-carbamic acid 1,2,3,4-tetrahydro-quinolin-6-yl ester 2g. heptyl-carbamic acid 1-benzyl-1,2,3,4-tetrahydro-quinolin-6-yl ester 2h. hexyl-carbamic acid 1-benzyl-1,2,3,4-tetrahydro-quinolin-6-yl ester 2i. (2-chloro-phenyl)-carbamic acid 1-benzyl-1,2,3,4-tetrahydro-quinolin-6-yl ester 2j. (3-bromo-phenyl)-carbamic acid 1-benzyl-1,2,3,4-tetrahydro-quinolin-6-yl ester 2k. (4-chloro-3-trifluoromethyl-phenyl)-carbamic acid 1-benzyl-1,2,3,4-tetrahydro-quinolin-6-yl ester 21. (4-bromo-phenyl)-carbamic acid 1-benzyl-1,2,3,4-tetrahydro-quinolin-6-yl ester 2m. hexyl-carbamic acid 1-methyl-1,2,3,4-tetrahydro-quinolin-6-yl ester 2n. heptyl-carbamic acid 1-methyl-1,2,3,4-tetrahydro-quinolin-6-yl ester 2o. (2-chloro-phenyl)-carbamic acid 1-methyl-1,2,3,4-tetrahydro-quinolin-6-yl ester 2p. (3-bromo-phenyl)-carbamic acid 1-methyl-1,2,3,4-tetrahydro-quinolin-6-yl ester 2q. (4-chloro-3-trifluoromethyl-phenyl)-carbamic acid 1-methyl-1,2,3, 4-tetrahydro-quinolin-6-yl ester 2r. (4-bromo-phenyl)-carbamic acid 1-methyl-1,2, 3,4-tetrahydro-quinolin-6-yl ester

In one instance of the disclosure, when R1 is aryl it is selected from the group consisting of.2- chloro, 3- bromo, 4-bromo and 4-chloro-3-trifluoromethyl-phenyl. In another instance of the disclosure, R2 is selected from the group consisting of methyl and benzyl.

vi) International Patent Application Publication No. WO2004/032929

An instance is directed to the compounds represented by the general formula I

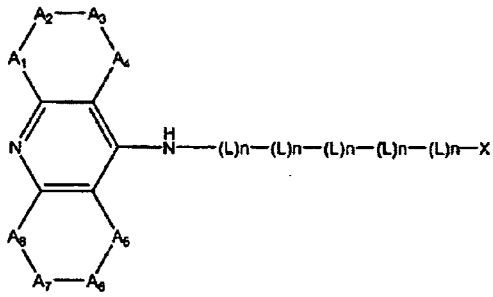

Formula I

wherein: X is one of the following radicals:

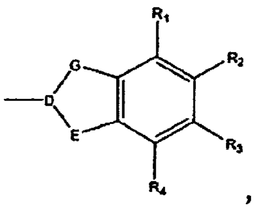

,

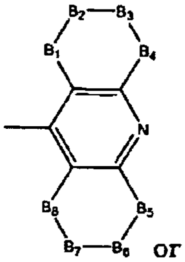

or

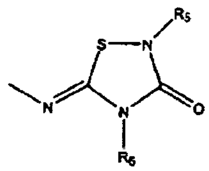

L is independently selected from-C (R) (R")-,-CO-,-0-or-NR'- n is zero, one, two, three, four, five, six, seven, eight, nine or ten R and R"are independently selected from hydrogen, alkyl, aryl, heteroaryl, halo, haloalkyl, alkoxy, hydroxyl, nitro and alkylthio D is independently selected from-C (R9)-, =C-, or-N- Al, A2, As, A4, As, A7, As, Bi, B2, Bs, B4, Bs, B6, B7, Bs, G and E are independently selected from-CO-,-C (Rio) (Rn)-, =C (Rio)-,-N (R12)-, =N-,-O-,-S (O) t- Ri, R2, R3, R4, R9, Rio and Ril are independently selected from hydrogen, alkyl, alkoxy, hydroxyl, alkylthio, cycloalkyl, haloalkyl, halo, aryl,- (Z) n-, aryl, heteroaryl,-O (R7),-C (O) R7, -C (O) OR7,-S (O) t, cyano, nitro and mercapto, aryl substituted by alkyl, alkoxy, hydroxy, halo, haloalkyl, nitro or alkylthio; and heteroaryl substituted by alkyl, alkoxy, hydroxy, halo, haloalkyl, nitro or alkylthio R5, R6, and R12 are independently selected from hydrogen, alkyl, alkoxy, hydroxyl, cycloalkyl, haloalkyl, aryl, heteroaryl, aryl substituted by alkyl, alkoxy, hydroxy, halo, haloalkyl, or alkylthio; and heteroaryl substituted by alkyl, alkoxy, hydroxy, halo, haloalkyl, nitro or alkylthio Z is independently selected from-C (R7) (R8)-,-C (O)-,-O-,-C (=NR7)-,-S (O) t, N (R7)- R7 and Rs are independently selected from hydrogen, alkyl, alkoxy, alkylthio, cycloalkyl, haloalkyl, halo, aryl, heteroaryl, cyano, nitro, mercapto, aryl substituted by alkyl, alkoxy, hydroxy, halo, haloalkyl, nitro or alkylthio; and heteroaryl substituted by alkyl, alkoxy, hydroxy, halo, haloalkyl, nitro or alkylthio t is zero, one or two.

In general, we have the proviso that when X is:

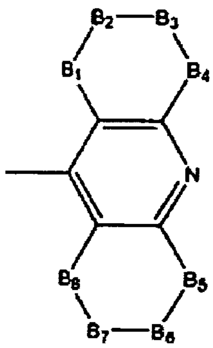

a) in no case each of the atoms in the groups Al-A4, As-As, Bl-B4, and Bs-Bs are at the same time =C (Rio)-, and
b) in no case each of the atoms in one of the two groups Ai-A4 and

As-As, and each of the atoms in one of the two groups Bi-B4 and
Bs-Bs are at the same time =C (Rio)-.

In a related aspect, the disclosure is directed to the compounds within the formula (I) and represented by the general formula Ia and IIa:

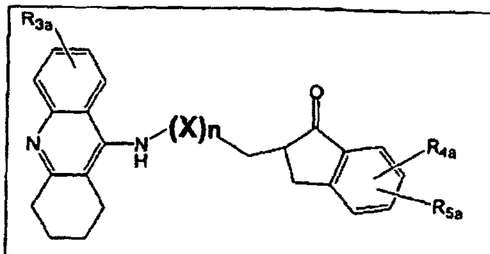

Formula Ia

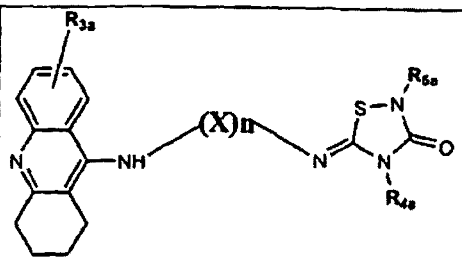

Formula IIa

Where: X is-C (R1a) (R2a)-, -CO-, -O- or -NR1a-; n is zero, one, two, three, four, five, six, seven, eight, nine or ten; R1a and R2a are independently selected from hydrogen, alkyl, aryl, halo, haloalkyl ; R3a, R4a and R5a are independently selected from hydrogen, alkyl, cycloaklyl, haloalkyl, halo, aryl,- (Z) n-aryl, heteroaryl, OR3a,-C (O) R3a,-C (O) OR3a,-S (O) t- ; t is zero, one or two; Z is independently selected from C (R3a) (R4a)-,-C (O)-,-O-,-C (=NR3a)-, -S(O)t-, N (R3a)-.

Definitions

In vi), the following terms have the following meaning: ; "alkyl"refers to a straight-line or branched hydrocarbon chain comprising only atoms of carbon and hydrogen and containing no unsaturated bonds, having from one to eight carbon atoms and bound to the remainder of the molecule by a single bond, e. g. methyl, ethyl, n-propyl, i-propyl, n-butyl, t-butyl, n-pentyl, etc. The alkyl radicals may optionally be substituted by one or more substituents chosen independently from the group comprising halogens, hydroxyl, alcoxides, carboxy, cyano, carbonyl, acyl, alkoxycarbonyl, amino, nitro, mercapto and alkylthio.

Preferably, alkyl is C1-C6 alkyl.

"alkoxy"refers to a radical of formula-ORa, where Ra is an alkyl radical as described above, e. g. methoxy, ethoxy, propoxy, etc.

"alkoxycarbonyl"refers to a radical of formula-C (O) ORa, where Ra is an alkyl radical as described above, e. g. methoxycarbonyl, ethoxycarbonyl, propoxycarbonyl, etc. alkylthio"refers to a radical of formula-SRa, where Ra is an alkyl radical as described above, e. g. methylthio, ethylthio, propylthio, etc.

"amino refers to a radical of formula-NH2 "aryl"refers to a phenyl or naphthyl radical. The aryl radical may optionally be substituted by one or more substituents selected from among the group comprising hydroxy, mercapto, halogens, alkyl, phenyl, alkoxy, haloalkyl, nitro, cyano, dialkylamino, aminoalkyl, acyl and alkoxycarbonyl as they are defined here.

"acyl"refers to a radical of formula-C (O)-Ra and-C (O)-Rb, where Ra is an alkyl radical as described above and Rb is an aryl radical as described above, e. g. acetyl, propionyl, benzoyl, and similar.

"carboxy"refers to a radical of formula-C (O) OH "cyano"refers to a radical of formula-CN "cycloalkyl"refers to stable cycles of 3 to 10 monocyclic or bicyclic members that are saturated or partially saturated and consist exclusively of carbon and hydrogen atoms. This term also includes cycloalkylo radicals, which may optionally be substituted by one or more substituents chosen independently from the group comprising alkyl, halogen, hydroxy, amino, cyano, nitro, alkoxy, carboxy and alkoxycarbonyl "halogens"refers to bromine, chlorine, iodine or fluorine "haloalkyl"refers to an alkyl radical, as defined above, which is substituted by one or more halogens, also as defined above, e. g. trifluoromethyl, trichloromethyl, 2,2, 2-trifluoroethyl, 1-fluoromethyl-2-fluoroethyl, and similar. "heterocycle"refers to a heterocyclic radical. The heterocycle refers to a stable cycle of 3 to 15 members comprising carbon atoms and one to five heteroatoms chosen from the group comprising nitrogen, oxygen and sulphur. For the purposes of this disclosure, the heterocycle may be a monocyclic, bicyclic or tricyclic system that may include fused rings, and the nitrogen, carbon or sulphur atoms may optionally be oxidised, the nitrogen atom may optionally be quaternised, and the heterocycle may be partly or totally saturated or aromatic. Examples of these heterocycles include, but are not limited to, azepines, benzimidazole, benzothiazole, furan, isothiazole, imidazole, indole, piperidine, piperazine, purine, quinoline, thiadiazole, tetrahydrofuran. The heterocycle may optionally be substituted by R3 and R4 as defined in the summary of the disclosure. "mercapto"refers to a radical of formula-SH "nitro"refers to a radical of formula-N02.

In the chain- (L) n- (L) n- (L) n- (L) n- (L) n-, the or each group- (L) n- is preferably- (CH2) n- (where n is not zero),-CO--NH-or-NCH3-.

Preferably there are at least one or two groups- (L) n- where n is not zero. Suitably the chain is of the formula- (CH2) n-,- (CH2) n-NRa- (CH2) n-,- (CH2) n-NRa-CO-,- (CH2) n-NRa-CO- (CH2) n-or-(CH2) n-NRa- (CH2) n-NRa-CO-, where the or each n is not zero, and the or each Ra is- NH-or-NCHs-, usually preferably-NH-. The total for the sum of the n integers is preferably in the range 2 to 15.

In the formula:

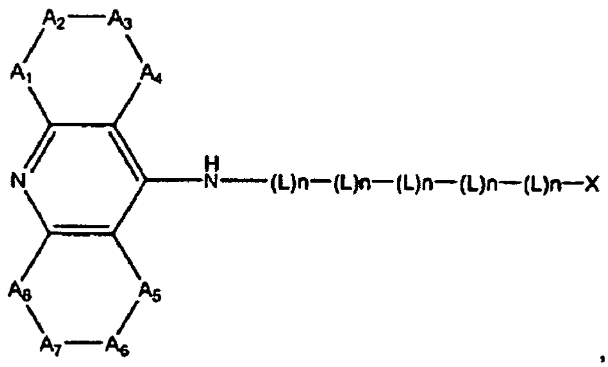

,

each A group (that it, Al to As) is preferably =CH-or-CH2-, though one or both of A2 and A7 can be halo, especially chloro, when the remaining A groups are =CH-. Some AChEIs are those in which X is represented by the following formula:

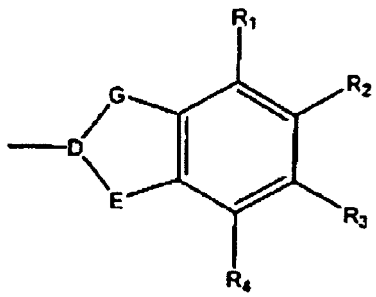

Preferably D is-CH-, =C-or-N-. Preferably E is-CO-,-CH2-. =CH-, =N--O-or-S-. Preferably G is-CO-,-CH2-. =CH-, or =N-.

Preferably Ri to R4 are hydrogen.

In some instances, within these compounds are those in which X is: phthalimidyl (1,3-dioxo-1,3-dihydro-isoindol-2-yl), indol-2-yl, indanon-2-yl, benzimidazol-2-yl, indandion-2-yl, indazol-2-yl, benzofuran-2-yl, benzothiophen-2-yl or benzotriazol-2-yl.

In some instances are those in which X is phthalimide (1,3-dioxo-1,3-dihydro-isoindol-2-yl) and the cyclic part of formula I represents 9-acridinyl, 1,2,3,4-tetrahydro-acridin-9-yl or 6chloro, 1,2,3,4-tetrahydro-acridin-9-yl. Some compounds are: 2-[6-(acridin-9-ylamino)-hexyl]-isoindole-1,3-dione (6), 2-[7-(acridin-9-ylamino)-heptyl]-isoindole-1,3-dione (7), 2-[8-(acridin-9-ylamino)-octyl]-isoindole-1,3-dione (8), 2-[9-(acridin-9-ylamino)-nonyl]-isoindole-1,3-dione (9), N-[7-(6-Chloro-1,2,3,4-tetrahydro-acridin-9-ylamino)-heptyl]-2-(1, 3-dioxo-1,3-dihydro-isoindol-2-yl)-acetamide (10), N-(3-{[3-(6-Chloro-1,2,3, 4-tetrahydro-acridin-9-ylamino)-propyl]-methyl-amino}-propyl)-2-(1,3-dioxo-1, 3-dihydro-isoindol-2-yl)-acetamide (11), N-[6-(6-Chloro-1,2,3,4-tetrahydro-acridin-9-ylamino)-hexyl]-2-(1,3-dioxo-1,3-dihydro-isoindol-2-yl)-acetamide (12), 2-[6-(1,2,3,4-Tetrahydro-acridin-9-ylamino)-hexylamino]-indan-1,3-dione (3), 2-[7-(1,2,3,4-Tetrahydro-acridin-9-ylamino)-heptyl]-isoindole-1,3-dione (4), and 2-[8-(1,2,3,4-Tetrahydro-acridin-9-ylamino)-octyl]-isoindole-1,3-dione (5).

Some compounds are those in which X is 1-indanon-2-yl and the cyclic part of formula I represents 1,2,3,4-tetrahydro-acridin-9yl; within these compounds are included, among others, the following compounds: 5,6-Dimethoxy-2-{[7-(1,2,3, 4-tetrahydro-acridin-9-ylamino)-heptylamino]-methyl}-indan-1-one (1), and 5,6-Dimethoxy-2-{[6-(1,2,3,4-tetrahydro-acridin-9-ylamino)-hexylamino]-methyl}-indan-1-one (2)

From those compounds in which X is represented by the following formula:

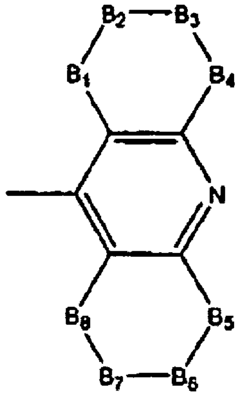

each B group (that it, Bi to Bs) is preferably =CH-or-CH2-, though one or both of B2 and B7 can be halo, especially chloro, when the remaining B groups are =CH-. Some compounds are those in which X is 9-acridinyl, 6-chloro-1,2,3,4-tetrahydro-acridin-9-yl and 1,2, 3,4-tetrahydro-acridin-9-yl. Within these compounds are those in which the cyclic part of formula I represents: 9-acridinyl, 6chloro-1,2,3,4-tetrahydro-acridin-9-yl or 1,2, 3,4-tetrahydro-acridin-9-yl; within these compounds are included, among others, the following compounds: -N-[2-(6-Chloro-1,2,3,4,4a,9a-hexahydro-acridin-9-ylamino)-ethyl]-N'-(6-chloro-1,2,3, 4-tetrahydro-acridin-9-yl)-N-methyl-ethane-1,2-diamine (19), -N-Acridin-9-yl-N'-(1,2,3,4-tetrahydro-acridin-9-yl)-nonane-1,9-diamine (20) -N-Acridin-9-yl-N'[2-(1,2,3,4,4a,9a-hexahydro-acridin-9-ylamino)-ethyl]-N'-methyl-ethane-1, 2-diamine (21), -N-[2-(Acridin-9-ylamino)-ethyl]-N'-(6-chloro-1,2,3,4-tetrahydro-acridin-9-yl)-N-methyl-ethane-1,2-diamine (22), - N-Acridin-9-yl-N'-(6-chloro-1,2,3,4-tetrahydro-acridin-9-yl)-heptane-1,7-diamine (23), and -N-Acridin-9-yl-N'-(6-chloro-1,2,3,4-tetrahydro-acridin-9-yl)-octane-1,8-diamine (24).

From those compounds in which X is represented by the following formula:

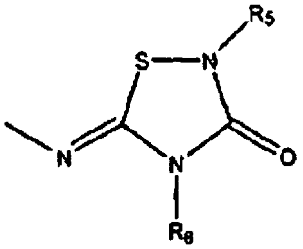

the groups Rs and R6 are suitably alkyl or substituted alkyl, notably alkoxycarbonylalkyl. The preferred compounds are those in which the cyclic part of formula I represents: 9-acridinyl, 6-chloro, 1,2,3,4-tetrahydro-acridin-9-yl or 1,2,3,4-tetrahydro-acridin-9-yl ; within these compounds are included, among others, the following compounds: 2-Ethyl-4-iso-propyl-5-[7-(1,2,3,4-tetrahydro-acridin-9-ylamino)-heptyl-iminio]-[1,2,4]thiadiazolidin-3-one (13), - 2-Ethyl-4-isopropyl-5-[9-(1,2,3,4-tetrahydro-acridin-9-ylamino)-nonyl-iminio]-[1,2,4] thiadiazolidin-3-one (14), 4-isopropyl-3-oxo-5-[9-(1,2,3,4-tetrahydro-acridin-9-ylamino)-nonyl-iminio]-[1,2,4]thiadiazolidine-2-carboxylic acid ethyl ester (15), 4-Ethyl-2-propyl-5-[7-(1,2,3,4-tetrahydro-acridin-9-ylamino)-heptylimino]-[1,2,4]thiadiazolidin-3-one (16), 4-Ethyl-2-isopropyl-5-[8-(1,2,3,4-tetrahydro-acridin-9-ylamino)-octylimino]-[1,2,4] thiadiazolidin-3-one (17), and 4-Ethyl-2-isopropyl-5-[6-(1,2,3,4-tetrahydro-acridin-9-ylamino)-hexyl-imino]-[1,2,4]thiadiazolidin-3-one (18).

vii) U.S. Patent Application Publication No. 20050124642

In some instances, an AChEI is:

1) an (1-indanone)-(1,2,3,6-tetrahydropyridine) compound represented by the formula:

(I)

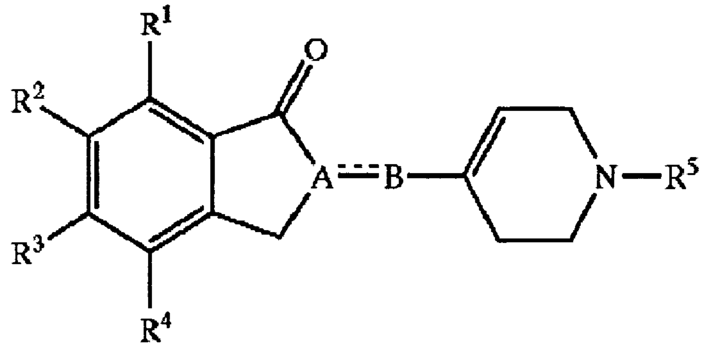

(in the formula (I), R<1>, R<2>, R<3> and R<4> are the same as or different from each other and each represents a group selected from hydrogen atom, a halogen atom, hydroxyl group, nitrile group, an alkyl group having one to six carbon atoms which may be substituted, a cycloalkyl group having three to eight carbon atoms which may be substituted, an alkoxy group having one to six carbon atoms which may be substituted, a cycloalkoxy group having three to eight carbon atoms which may be substituted, an acyl group having one to six carbon atoms which may be substituted, an C1-C6 alkoxy-carbonyl group which may be substituted, an C1-C6 alkyl-aminocarbonyloxy group which may be substituted, a di-(C1-C6 alkyl)-aminocarbonyloxy group which may be substituted, nitro group, an amino group which may be substituted, an amide group which may be substituted, mercapto group and a thioalkoxy group having one to six carbon atoms which may be substituted, where R<1 > and R<2> , or R<2 > and R<3> , or R<3 > and R<4 > may together form an aliphatic ring, an aromatic ring, a heterocyclic ring or an alkylenedioxy ring; the partial structure:

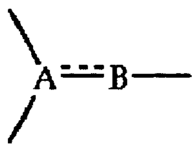

is a group represented by >C-CH-(CH2)m- or >C(R<6>)-CH(R<6>)-(CH2)m-(wherein m is 0 or an integer from 1 to 5; and R<6 > is hydrogen atom, a halogen atom, hydroxyl group, an alkyl group having one to six carbon atoms, an alkoxy group having one to six carbon atoms, nitrile group, a haloalkyl group having one to six carbon atoms, a hydroxyalkyl group having one to six carbon atoms, a cyano-C1-C6 alkyl group, an aminoalkyl group having one to six carbon atoms, nitro group, azide group, an amino group which may be substituted, carbamoyl group which may be substituted, carboxyl group which may be substituted, mercapto group or a thioalkoxy group having one to six carbon atoms); and R<5 > is hydrogen atom, an alkyl group having one to six carbon atoms which may be substituted, an alkenyl group having two to six carbon atoms which may be substituted, an alkynyl group having two to six carbon atoms which may be substituted, a cycloalkyl group having three to eight carbon atoms which may be substituted, an 2,2-(alkylenedioxy)ethyl group or a group represented by the formula:

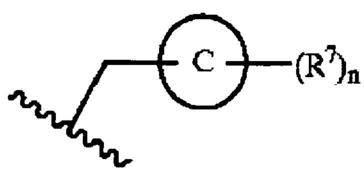

(wherein the ring C is benzene ring, an aliphatic ring or a heterocyclic ring; R<7> s are the same as or different from each other and each represents hydrogen atom, a halogen atom, hydroxyl group, nitrile group, an alkyl group having one to six carbon atoms which may be substituted, an alkenyl group having two to six carbon atoms which may be substituted, an alkynyl group having two to six carbon atoms which may be substituted, a cycloalkyl group having three to eight carbon atoms which may be substituted, an alkoxy group having one to six carbon atoms which may be substituted, an C1-C6 alkoxy-alkoxy group which may be substituted, an aryloxy group which may be substituted, an aralkyloxy group which may be substituted or the like, where two of R<7> s may together form an aliphatic ring, an aromatic ring, a heterocyclic ring or an alkylenedioxy ring; and n is an integer from 1 to 5)), a pharmacologically

acceptable salt thereof or a hydrate of them;

2) the (1-indanone)-(1,2,3,6-tetrahydropyridine) compound described in 1), a pharmacologically acceptable salt thereof or a hydrate of them, wherein R<1 > and R<4 > are hydrogen atoms; and R<2 > and R<3 > are the same as or different from each other and each represents hydrogen atom, a halogen atom, an alkyl group having one to six carbon atoms which may be substituted, a cycloalkyl group having three to eight carbon atoms which may be substituted, an alkoxy group having one to six carbon atoms which may be substituted or a cycloalkoxy group having three to eight carbon atoms which may be substituted, or R<2 > and R<3 > may together form an aliphatic ring, an aromatic ring, a heterocyclic ring or an alkylenedioxy ring;

3) the (1-indanone)-(1,2,3,6-tetrahydropyridine) compound described in 1) or 2), a pharmacologically acceptable salt thereof or a hydrate of them, wherein R<6 > is hydrogen atom, a halogen atom or an alkyl group having one to six carbon atoms;

4) the (1-indanone)-(1,2,3,6-tetrahydropyridine) compound described in any one of 1) to 3), a pharmacologically acceptable salt thereof or a hydrate of them, wherein R<5 > is a group represented by the formula:

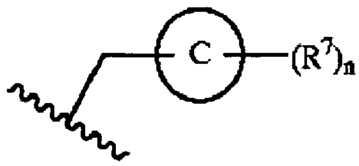

wherein the ring C, R<7 > and n have the same meanings as defined above;

5) the (1-indanone)-(1,2,3,6-tetrahydropyridine) compound described in 4), a pharmacologically acceptable salt thereof or a hydrate of them, wherein the ring C is benzene ring or a cycloalkyl ring having three to eight carbon atoms;

6) the (1-indanone)-(1,2,3,6-tetrahydropyridine) compound described in 4), a pharmacologically acceptable salt thereof or a hydrate of them, wherein R<7> s are the same as or different from each other and each represents hydrogen atom, a halogen atom, an alkyl group having one to six carbon atoms which may be substituted, an alkenyl group having two to six carbon atoms which may be substituted, an alkynyl group having two to six carbon atoms which may be substituted, a cycloalkyl group having three to eight carbon atoms which may be substituted, or two of R<7> s may together form an aliphatic ring, an aromatic ring, a heterocyclic ring or an alkylenedioxy ring;

7) the (1-indanone)-(1,2,3,6-tetrahydropyridine) compound described in 4), a pharmacologically acceptable salt thereof or a hydrate of them, wherein n is an integer of 1 or 2;

8) the (1-indanone)-(1,2,3,6-tetrahydropyridine) compound described in any one of 1) to 7), a pharmacologically acceptable salt thereof or a hydrate of them, wherein the partial structure:

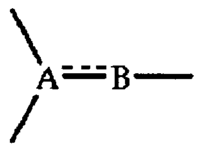

is a group represented by >C(R<6>)-CH(R<6>)-(CH2)m- (wherein m is 0 or an integer from 1 to 5; and R<6 > is hydrogen atom);

9) the (1-indanone)-(1,2,3,6-tetrahydropyridine) compound described in any one of 1) to 7), a pharmacologically acceptable salt thereof or a hydrate of them, wherein the partial structure:

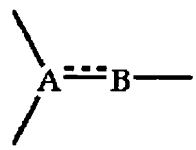

is a group represented by >C(R<6>)-CH(R<6>)-(CH2)m- (wherein m is 0 or an integer from 1 to 5; and R<6 > is a halogen atom, hydroxyl group, an alkyl group having one to six carbon atoms, an alkoxy group having one to six carbon atoms, nitrile group, a haloalkyl group having one to six carbon atoms, a hydroxyalkyl group having one to six carbon atoms, a cyano-C1-C6 alkyl group, an aminoalkyl group having one to six carbon atoms, nitro group, azide group, an amino group which may be substituted, carbamoyl group which may be substituted, carboxyl group which may be substituted, mercapto group or a thioalkoxy group having one to six carbon atoms;

10) the (1-indanone)-(1,2,3,6-tetrahydropyridine) compound described in 1), a pharmacologically acceptable salt thereof or a hydrate of them, wherein the (1-indanone)-(1,2,3,6-tetrahydropyridine) compound represented by the formula (I) is one selected from:

(1) 1-benzyl-4-[(5,6-dimethoxy-1-indanon)-2-yl]methyl-1,2,3,6-tetrahydropyridine,
2) 1-benzyl-4-[(5,6-diethoxy-1-indanon)-2-yl]methyl-1,2,3,6-tetrahydropyridine, and
(3) 1-benzyl-4-[(5,6-diethoxy-2-fluoro-1-indanon)-2-yl]methyl-1,2,3,6-tetrahydropyridine;

See also, for example, those AChE inhibitors set forth in Brufani et al, Alzheimer Disease: From Molecular Biology to Therapy, eds. Becker et al., pp. 171-177 (1996); Schmidt et al., Alzheimer Disease: From Molecular Biology to Therapy, eds. Becker et al., pp. 217-221 (1996); Vargas et al., Alzheimer Disease: From Molecular Biology to Therapy, eds. Becker et al., pp. 261-255 (1996); Greig et al., Alzheimer Disease: From Molecular Biology to Therapy, eds. Becker et al., pp. 231-237 (1996); and Giacobini, Alzheimer Disease: From Molecular Biology to Therapy eds. Becker et al., pp. 187-204 (1996).

viii) U.S. Patent 7,378,425

In some instances, the following compounds ((1) to (4)) are compounds having an acetylcholinesterase inhibitory action.

(1) A cyclic amine derivative represented by the following formula, a pharmacologically acceptable salt thereof or a hydrate of them.

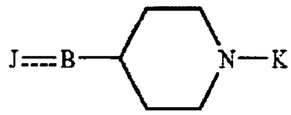

Wherein J is, for example, the formula:

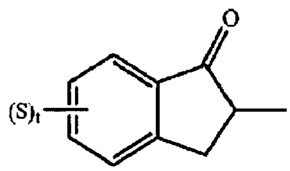

(wherein S is, for example, a lower alkoxy group having one to six carbon atoms; and t is 0 or an integer of 1 to 4); B is, for example, methylene chain; K is, for example, a benzyl group which may be substituted; and the partial structure: - is a single bond or double bond, provided that a compound wherein J is 5,6-dimethoxy-1-indanon-2-yl group; B is -CH2- group; and K is unsubstituted benzyl group, a pharmacologically acceptable salt thereof or a hydrate of them are excluded (JP-B2 2733203).

(2) A compound represented by the following formula:

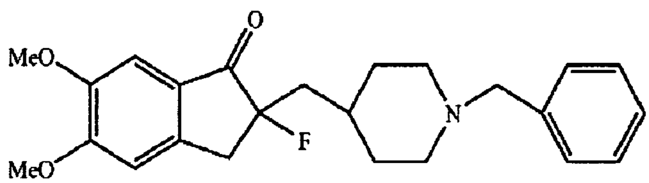

or a pharmacologically acceptable salt thereof (JP-A 2000-319257).

(3) A 4-substituted piperidine derivative fluoride represented by the following formula:

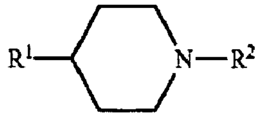

(wherein R<1 > is, for example, a substituent represented by:

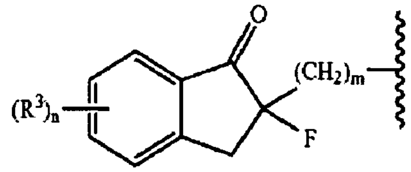

(wherein R<3> s are the same as or different from each other and each represents, for example, an alkoxy group having one to six carbon atoms; m is 0 or an integer of 1 to 6; and n is an integer of 1 to 4); and R<2 > is, for example, a benzyl group which may be substituted, provided that 1-benzyl-4-[(5,6-dimethoxy-2-fluoro-1-indanon)-2-yl]methylpiperidine or a pharmacologically acceptable salt thereof is excluded), a pharmacologically acceptable salt thereof or a hydrate of them (JP-A 2000-319258).

(4) A 4-substituted piperidine derivative represented by the following formula:

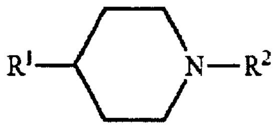

(wherein R<1 > is, for example, a group represented by the formula:

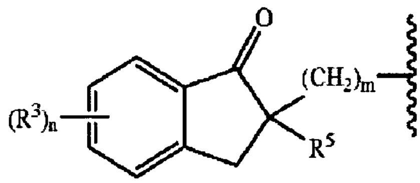

(wherein R<3> s are the same as or different from each other and each represents, for example, an alkoxy group having one to six carbon atoms; R<5 > is, for example, a halogen atom except for fluorine atom; m is 0 or an integer of 1 to 6; and n is an integer of 1 to 4); and R<2 > is, for example, benzyl group which may be substituted), a pharmacologically acceptable salt or a hydrate of them (JP-A 2001-139547).

In some instances, the present disclosure relates to:

1) an (1-indanone)-(1,2,3,6-tetrahydropyridine) compound represented by the formula:

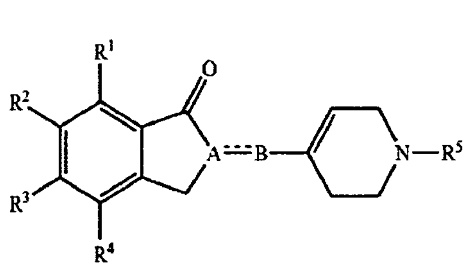

(I)

(in the formula (I), R<1>, R<2>, R<3> and R<4> are the same as or different from each other and each represents a group selected from hydrogen atom, a halogen atom, hydroxyl group, nitrile group, an alkyl group having one to

six carbon atoms which may be substituted, a cycloalkyl group having three to eight carbon atoms which may be substituted, an alkoxy group having one to six carbon atoms which may be substituted, a cycloalkoxy group having three to eight carbon atoms which may be substituted, an acyl group having one to six carbon atoms which may be substituted, an C1-C6 alkoxy-carbonyl group which may be substituted, an C1-C6 alkyl-aminocarbonyloxy group which may be substituted, a di-(C1-C6 alkyl)-aminocarbonyloxy group which may be substituted, nitro group, an amino group which may be substituted, an amide group which may be substituted, mercapto group and a thioalkoxy group having one to six carbon atoms which may be substituted, where R<1 > and R<2> , or R<2 > and R<3> , or R<3 > and R<4 > may together form an aliphatic ring, an aromatic ring, a heterocyclic ring or an alkylenedioxy ring; the partial structure:

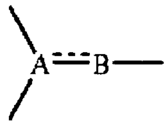

is a group represented by >C-CH-(CH2)m- or >C(R<6>)-CH(R<6>)-(CH2)m-(wherein m is 0 or an integer from 1 to 5; and R<6 > is hydrogen atom, a halogen atom, hydroxyl group, an alkyl group having one to six carbon atoms, an alkoxy group having one to six carbon atoms, nitrile group, a haloalkyl group having one to six carbon atoms, a hydroxyalkyl group having one to six carbon atoms, a cyano-C1-C6 alkyl group, an aminoalkyl group having one to six carbon atoms, nitro group, azide group, an amino group which may be substituted, carbamoyl group which may be substituted, carboxyl group which may be substituted, mercapto group or a thioalkoxy group having one to six carbon atoms); and R<5 > is hydrogen atom, an alkyl group having one to six carbon atoms which may be substituted, an alkenyl group having two to six carbon atoms which may be substituted, an alkynyl group having two to six carbon atoms which may be substituted, a cycloalkyl group having three to eight carbon atoms which may be substituted, an 2,2-(alkylenedioxy)ethyl group or a group represented by the formula:

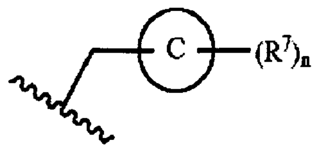

(wherein the ring C is benzene ring, an aliphatic ring or a heterocyclic ring; R<7> s are the same as or different from each other and each represents hydrogen atom, a halogen atom, hydroxyl group, nitrile group, an alkyl group having one to six carbon atoms which may be substituted, an alkenyl group having two to six carbon atoms which may be substituted, an alkynyl group having two to six carbon atoms which may be substituted, a cycloalkyl group having three to eight carbon atoms which may be substituted, an alkoxy group having one to six carbon atoms which may be substituted, an C1-C6 alkoxy-alkoxy group which may be substituted, an aryloxy group which may be substituted, an aralkyloxy group which may be substituted or the like, where two of R<7> s may together form an aliphatic ring, an aromatic ring, a heterocyclic ring or an alkylenedioxy ring; and n is an integer from 1 to 5)), a pharmacologically acceptable salt thereof or a hydrate of them;

2) the (1-indanone)-(1,2,3,6-tetrahydropyridine) compound described in 1), a pharmacologically acceptable salt thereof or a hydrate of them, wherein R<1 > and R<4 > are hydrogen atoms; and R<2 > and R<3 > are the same as or different from each other and each represents hydrogen atom, a halogen atom, an alkyl group having one to six carbon atoms which may be substituted, a cycloalkyl group having three to eight carbon atoms which may be substituted, an alkoxy group having one to six carbon atoms which may be substituted or a cycloalkoxy group having three to eight carbon atoms which may be substituted, or R<2 > and R<3 > may together form an aliphatic ring, an aromatic ring, a heterocyclic ring or an alkylenedioxy ring;

3) the (1-indanone)-(1,2,3,6-tetrahydropyridine) compound described in 1) or 2), a pharmacologically acceptable salt thereof or a hydrate of them, wherein R<6 > is hydrogen atom, a halogen atom or an alkyl group having one to six carbon atoms;

4) the (1-indanone)-(1,2,3,6-tetrahydropyridine) compound described in any one of 1) to 3), a pharmacologically acceptable salt thereof or a hydrate of them, wherein R<5 > is a group represented by the formula:

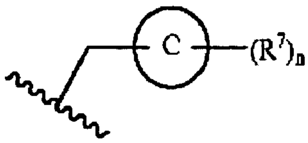

wherein the ring C, R<7 > and n have the same meanings as defined above;

5) the (1-indanone)-(1,2,3,6-tetrahydropyridine) compound described in 4), a pharmacologically acceptable salt thereof or a hydrate of them, wherein the ring C is benzene ring or a cycloalkyl ring having three to eight carbon atoms;

6) the (1-indanone)-(1,2,3,6-tetrahydropyridine) compound described in 4), a pharmacologically acceptable salt thereof or a hydrate of them, wherein R<7> s are the same as or different from each other and each represents hydrogen atom, a halogen atom, an alkyl group having one to six carbon atoms which may be substituted, an alkenyl group having two to six carbon atoms which may be substituted, an alkynyl group having two to six carbon atoms which may be substituted, a cycloalkyl group having three to eight carbon atoms which may be substituted, or two of R<7> s may together form an aliphatic ring, an aromatic ring, a heterocyclic ring or an alkylenedioxy ring;

7) the (1-indanone)-(1,2,3,6-tetrahydropyridine) compound described in 4), a pharmacologically acceptable salt thereof or a hydrate of them, wherein n is an integer of 1 or 2;

8) the (1-indanone)-(1,2,3,6-tetrahydropyridine) compound described in any one of 1) to 7), a pharmacologically acceptable salt thereof or a hydrate of them, wherein the partial structure:

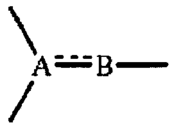

is a group represented by >C(R<6>)-CH(R<6>)-(CH2)m- (wherein m is 0 or an integer from 1 to 5; and R<6 > is hydrogen atom);

9) the (1-indanone)-(1,2,3,6-tetrahydropyridine) compound described in any one of 1) to 7), a pharmacologically acceptable salt thereof or a hydrate of them, wherein the partial structure:

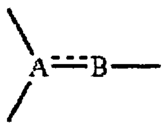

is a group represented by >C(R<6>)-CH(R<6>)-(CH2)m- (wherein m is 0 or an integer from 1 to 5; and R<6 > is a halogen atom, hydroxyl group, an alkyl group having one to six carbon atoms, an alkoxy group having one to six carbon atoms, nitrile group, a haloalkyl group having one to six carbon atoms, a hydroxyalkyl group having one to six carbon atoms, a cyano-C1-C6 alkyl group, an aminoalkyl group having one to six carbon atoms, nitro group, azide group, an amino group which may be substituted, carbamoyl group which may be substituted, carboxyl group which may be substituted, mercapto group or a thioalkoxy group having one to six carbon atoms;

10) the (1-indanone)-(1,2,3,6-tetrahydropyridine) compound described in 1), a pharmacologically acceptable salt thereof or a hydrate of them, wherein the (1-indanone)-(1,2,3,6-tetrahydropyridine) compound represented by the formula (I) is one selected from:

(1) 1-benzyl-4-[(5,6-dimethoxy-1-indanon)-2-yl]methyl-1,2,3,6-tetrahydropyridine,
(2) 1-benzyl-4-[(5,6-diethoxy-1-indanon)-2-yl]methyl-1,2,3,6-tetrahydropyridine, and
(3) 1-benzyl-4-[(5,6-diethoxy-2-fluoro-l-indanon)-2-yl]methyl-1,2,3,6-tetrahydropyridine.

ix) International Patent Application Publication No. WO2003/020289

The compounds of formula I of the present disclosure are compounds of the following general formula 1 :

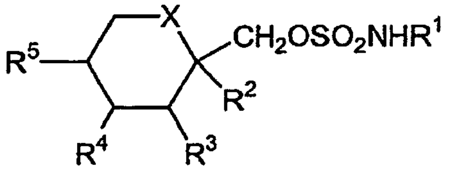

I

wherein

X is CH2 or oxygen;
RI is hydrogen or alkyl ; and R2, R3, R4 and R5 are independently hydrogen or lower alkyl and, when X is CH2, R4 and R5 may be alkene groups joined to form a benzene ring and, when X is oxygen, R2 and R3 and/or R4 and R5 together may be a methylenedioxy group of the following formula II :

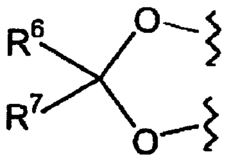

II

wherein

R6 and R7 are the same or different and are hydrogen, lower alkyl or are alkyl and are joined to form a cyclopentyl or cyclohexyl ring.
$R^1$ in particular is hydrogen or alkyl of about 1 to 4 carbons, such as methyl, ethyl and iso-propyl. Alkyl throughout this specification includes straight and branched chain alkyl. Alkyl groups for R2, R3, R4, R5, R6 and R7 are of about 1 to 3 carbons and include methyl, ethyl, iso-propyl and n-propyl.
When X is CH2, R and R5 may combine to form a benzene ring fused to the 6-membered X-containing ring, i. e., R4 and R5 are defined by the alkatrienyl group =C-CH=CH-CH=.

A particular group of compounds of formula I is that wherein X is oxygen and both R2 and R3 and R4 and R5 together are methylenedioxy groups of the formula il, wherein R6 and R7 are both hydrogen, both alkyl or combine to form a spiro cyclopentyl or cyclohexyl ring, in particular where R6 and R7 are both alkyl such as methyl. A second group of compounds is that wherein X is CH2 and R4 and R5 are joined to form a benzene ring. A third group of compounds of formula I is that wherein both R2 and R3 are hydrogen.

x) U.S. Patent Application Publication No. 2003069289

In view of the circumstances described above, the present inventors made extensive study through which they found that both a compound represented by the formula:

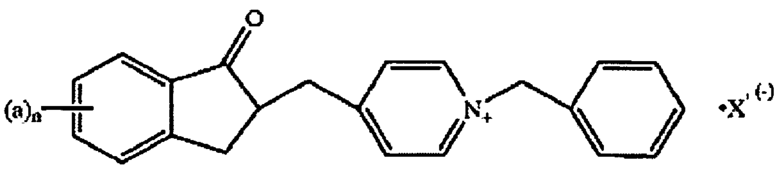

(wherein the "a" moieties are the same as or different from each other and each represents a hydrogen atom or a C1-6 alkoxy group; n is an integer from 1 to 4; and X<(-) >represents a halide ion) and a compound represented by the formula:

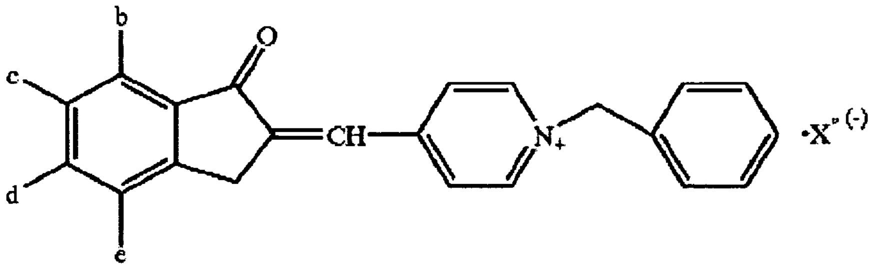

(wherein b, c, d and e are the same as or different from each other and each represents a hydrogen or a linear or branched C1-6 alkyl, C1-6alkoxy, C1-6alkoxy carbonyl, C1-6alkyl aminocarbonyloxy, di (C1-6alkyl)-aminocarbonyloxy or a halogen; and X"<(-) >represents an anion in the series of chloride, bromide, iodide and sulfate) exhibit an excellent inhibitory action on acetylcholinesterase. Further, on the basis of these findings, they found that a 1-benzylpyridinium salt represented by the formula:

(I)

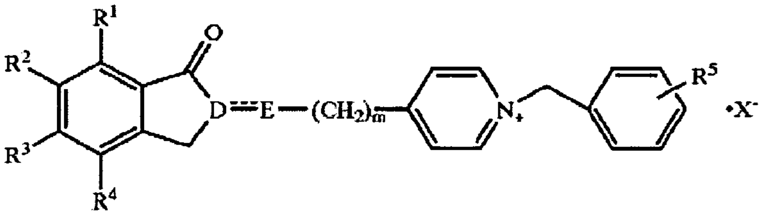

(wherein R<1>, R<2>, R<3 >and R<4 >are the same or different, and represent a hydrogen atom, a halogen atom, a hydroxyl group, a nitrile group, a C1-6 alkyl group, a C1-6 alkoxy group, a C1-6alkoxy carbonyl group, a C1-6 alkyl aminocarbonyloxy group or a di (C1-6 alkyl)-aminocarbonyloxy group; R<5 >represents a hydrogen atom, a halogen atom, a C1-6alkyl group, a C2-6 alkenyl group or a C2-6 alkynyl group; the partial structure:

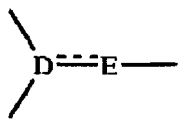

is a group represented by the formula >C(R<6>)-CH2- (wherein R<6 >is a hydrogen atom or a halogen atom) or >C-CH-; X<-> represents a halide ion or organic sulfonic acid ion; and m is 0 or an integer from 1 to 5) exhibits an excellent inhibitory action on acetylcholinesterase, and accomplished the present disclosure. That is, the first aspect according to the present disclosure is (1) an acetylcholinesterase inhibitor comprising a 1-benzylpyridinium salt represented by the above formula (I). Further, (2) in the above item (1), R<1>, R<2>, R<3 >and R<4 >may be the same as or different from each other and each represents a hydrogen atom or a C1-6 alkoxy group; (3) in the above item (1), R<1>, R<2 >R<3 >and R<4 >may be the same as or different from each other and each represents a hydrogen atom or a methoxy group; (4) in the above item (1), R<1 >and R<4 >may represent a hydrogen atom; and R<2 >and R<3 >may represent a methoxy group; (5) in the above item (1), R<5 >may be a hydrogen atom; (6) in the above item (1), the partial structure:

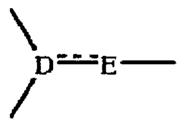

may be a group represented by the formula >C (R<6>) -CH2- (wherein R<6 >is a hydrogen atom or a halogen atom) ; (7) in the above item (1), m may be 0, 2 or 4; (8) in the above item (1), the halide ion represented by X<-> maybe a chloride ion, bromide ion or iodide ion, preferably a chloride ion or bromide ion; (9) in the above item (1), the organic sulfonic acid ion represented by X<-> may be a methanesulfonate ion, trifluoromethanesulfonate ion, ethanesulfonate ion, benzenesulfonate ion, toluenesulfonate ion or camphor sulfonate ion; (10) in the above item (1), the 1-benzylpyridinium salt may be a compound represented by the formula:

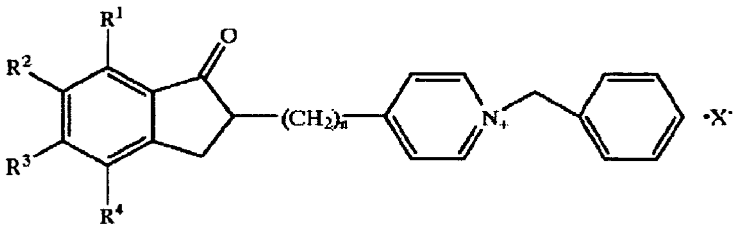

wherein R<1>, R<2>, R<3>, R<4 >and X<-> have the same meanings as defined above; and n is an integer from 1 to 6; (11) in the above item (1), the 1-benzylpyridinium salt may be a compound represented by the formula:

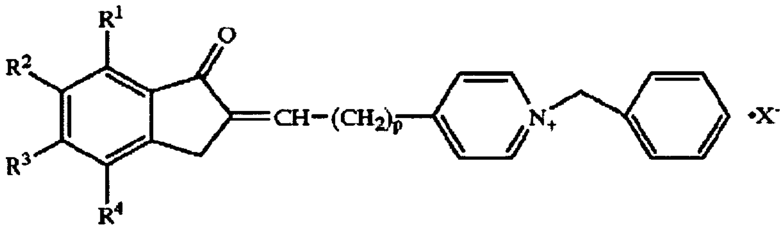

wherein R<1>, R<2>, R<3>, R<4 >and X<-> have the same meanings as defined above; and p is 0 or an integer from 1 to 5; (12) in the above item (1), the preparation may be an agent for treating, preventing or improving senile dementia, cerebrovascular dementia or attention deficit hyperactivity disorder; and (13) in the above item (12), the senile dementia may be Alzheimer type senile dementia.

The second aspect according to the present disclosure is (14) a pharmaceutical preparation comprising a 1-benzylpyridinium salt represented by the formula:

(I)

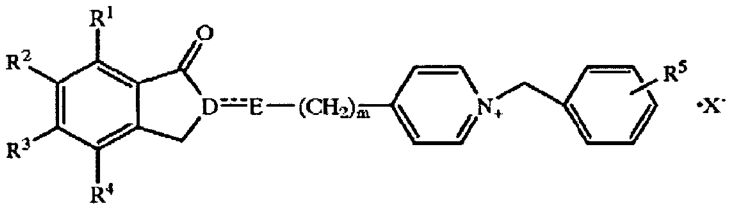

wherein R<1>, R<2>, R<3>, R<4>, R<5>, the partial structure:

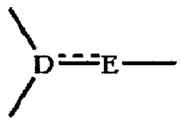

X<-> and m have the same meanings as defined above. Further, the third aspect according to the present disclosure is (15) use of a 1-benzylpyridinium salt represented by the formula:

(I)

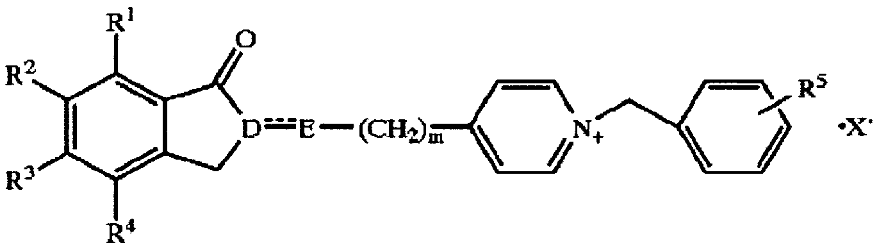

(wherein R<1>, R<2>, R<3>, R<4>, R<5>, the partial structure:

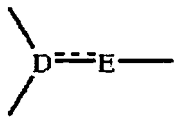

X<-> and m have the same meanings as defined above) for producing an acetylcholinesterase inhibitor.

The present disclosure provides a method of preventing, treating or improving a disease against which an inhibitory action on acetylcholinesterase is efficacious for prevention, treatment or improvement, by administering a pharmacologically effective dose of the 1-benzylpyridinium salt represented by the above formula (I) to a patient.

In the present disclosure, the disease against which an inhibitory action on acetylcholinesterase is efficacious for prevention, treatment or improvement includes senile dementia such as Alzheimer type senile dementia, and cerebrovascular dementia and attention deficit hyperactivity disorder. Hereinafter,, the meanings of symbols, terms etc. used in the specification are described, and the present disclosure is described in detail.

In the specification, the structural formulae of the compound may, for convenience' sake, indicate a certain isomer, but the present disclosure encompasses all possible isomers which can occur in the structures of the compound, for example geometric isomer, optical isomer based on asymmetrical carbon, stereoisomer and tautomer, and a mixture of such isomers, so the compound according to the present disclosure may be any isomers or a mixture thereof without limitation to the formulae shown for convenience' sake. Accordingly, the compound according to the present disclosure can have an intramolecular asymmetrical carbon, thus occurring as optically active isomers or racemic modifications, and any of such compounds are included in the present disclosure without limitation. When there is crystal polymorphism, the compound according to the present disclosure may be in a single crystal form or a mixed crystal form without limitation. Compound (I) or salts thereof may be anhydrides or hydrates, any of which fall under the claims in the specification. Further, metabolites formed by decomposition of Compound (I) in vivo, and prodrugs of Compound (I) or salts thereof, also fall under the claims in the specification.

The "halogen atom" used in the specification refers to an atom such as a fluorine atom, a chlorine atom, a bromine atom or an iodine atom, preferably a fluorine atom, a chlorine atom or a bromine atom.

The "C1-6 alkyl group" represented by R<1>, R<2>, R<3 >and R<4 >in the specification refers to an alkyl group having 1 to 6 carbon atoms, and examples thereof include a methyl group, ethyl group, n-propyl group, iso-propyl group, n-butyl group, iso-butyl group, sec-butyl group, tert-butyl group, n-pentyl group, 1,1-dimethylpropyl group, 1,2-dimethylpropyl group, 2,2-dimethylpropyl group, 1-ethylpropyl group, 2-ethylpropyl group, n-hexyl group, 1-methyl-2-ethylpropyl group, 1-ethyl-2-methylpropyl group, 1,1,2-trimethylpropyl group, 1-propylpropyl group, 1-methylbutyl group, 2-methylbutyl group, 1,1-dimethylbutyl group, 1,2-dimethylbutyl group, 2,2-dimethylbutyl group, 1,3-dimethylbutyl group, 2,3-dimethylbutyl group, 2-ethylbutyl group, 2-methylpentyl group, 3-methylpentyl group etc., preferably a methyl group, ethyl group, n-propyl group, iso-propyl group, n-butyl group, iso-butyl group and tert-butyl group.

The "C1-6 alkoxy group" represented by R<1>, R<2>, R<3 >and R<4 >in the specification refers to an alkoxy group having 1 to 6 carbon atoms, and examples thereof include a methoxy group, ethoxy group, n-propoxy group, iso-propoxy group, sec-propoxy group, n-butoxy group, iso-butoxy group, sec-butoxy group, tert-butoxy group, n-pentyloxy group, iso-pentyloxy group, sec-pentyloxy group, n-hexoxy group, iso-hexoxy group, 1,1-dimethylpropyloxy group, 1,2-dimethylpropoxy group, 2,2-dimethylpropyloxy group, 2-ethylpropoxy group, 1-methyl-2-ethylpropoxy group, 1-ethyl-2-methylpropoxy group, 1,1,2-trimethylpropoxy group, 1,1,2-trimethylpropoxy group, 1,1-dimethylbutoxy group, 1,2-dimethylbutoxy group, 2,2-dimethylbutoxy group, 2,3-dimethylbutyloxy group, 1,3-dimethylbutyloxy group, 2-ethylbutoxy group, 1,3-dimethylbutoxy group, 2-methylpentoxy group, 3-methylpentoxy group, hexyloxy group etc., preferably a methoxy group, ethoxy group, n-propoxy group and iso-propoxy group.

The "C1-6 alkoxycarbonyl group" represented by R<1>, R<2>, R<3 >and R<4 >in the specification. refers to a group in which a C1-6 alkoxy group having the same meaning as defined above bound to a carbonyl group. For example, a methoxycarbonyl group, ethoxycarbonyl group, n-propoxycarbonyl group, iso-propoxycarbonyl group, n-butoxycarbonyl group, iso-butoxycarbonyl group, tert-butoxycarbonyl group, pentyloxycarbonyl group, hexyloxycarbonyl group etc., preferably a methoxycarbonyl group, ethoxycarbonyl group, n-propoxycarbonyl group and iso-propoxycarbonyl group.

The "C1-6alkyl aminocarbonyloxy group" represented by R<1>, R<2>, R<3 >and R<4 >in the specification refers to an aminocarbonyloxy group whose nitrogen atom has been substituted with a C1-6alkyl group having the same meaning as defined above, and examples thereof include a methylaminocarbonyloxy group, ethylaminocarbonyloxy group, n-propylaminocarbonyloxy group, iso-propylaminocarbonyloxy group, n-butylaminocarbonyloxy group, iso-butylaminocarbonyloxy group, tert-butylaminocarbonyloxy group, n-pentylaminocarbonyloxy group, iso-pentylaminocarbonyloxy group, neopentylaminocarbonyloxy group, hexylaminocarbonyloxy group, 1-methylpropylaminocarbonyloxy group, 1-methyl butyl aminocarbonyloxy group, 2-methylbutylaminocarbonyloxy group etc.

The "di (C1-6 alkyl)-aminocarbonyloxy group" represented by R<1>, R<2>, R<3 >and R<4 >in the specification refers to an aminocarbonyloxy group whose nitrogen atom has been substituted with two C1-6 alkyl groups, and examples thereof include a dimethylaminocarbonyloxy group, diethylaminocarbonyloxy group, di(n-propyl)-aminocarbonyloxy group, di-(iso-propyl)-aminocarbonyloxy group, di(n-butyl)-aminocarbonyloxy group, di(iso-butyl)-aminocarbonyloxy group, di-(tert-butyl)-aminocarbonyloxy group, di(n-pentyl)-aminocarbonyloxy group, di-(iso-pentyl)-aminocarbonyloxy group, di-(neopentyl)-aminocarbonyloxy group, di-(n-hexyl)-aminocarbonyloxy group, di-(1-methylpropyl)-aminocarbonyloxy group, di-(1-methylbutyl)-aminocarbonyloxy group, di-(2-methylbutyl)-aminocarbonyloxy group etc.

As the preferable mode of R<1>, R<2>, R<3 >and R<4 >in the specification, R<1>, R<2, >R<3 >and R<4 >are the same as or different from each other and each represents a hydrogen atom, a halogen atom, a hydroxyl group, a nitrile group, a C1-6alkyl group or a C1-6 alkoxy group; more preferably, they are the same as or different from each other and each represents a hydrogen atom, a halogen atom, a hydroxyl group, a nitrile group or a C1-6alkoxy group; further preferably, they are the same as or different from each other and each represents a hydrogen atom or a C1-6alkoxy group; and the most preferably, R<1> and R<4 >represent a hydrogen atom while R<2 >and R<3 >are the same as or different from each other and each represents a C1-6 alkoxy group (for example, a methoxy group, ethoxy group etc.).

The "halogen atom" and "C1-6 alkyl group" represented by R<5 >in the specification refer to a halogen atom and C1-6alkyl group each having the same meaning as defined above.

The "C2-6 alkenyl group" represented by R<5 >in the specification refers to an alkenyl group having 2 to 6 carbon atoms, and examples thereof include linear or branched C2-6 alkenyl groups such as a vinyl group, allyl group, 1-propenyl group, isopropenyl group, 1-buten-1-yl group, 1-buten-2-yl group, 1-buten-3-yl group, 2-buten-1-yl group and 2-buten-2-yl group, preferably a vinyl group, allyl group and isopropenyl group.

The "C2-6 alkynyl group" represented by R<5 >in the specification refers to an alkenyl group having 2 to 6 carbon atoms, and examples thereof include linear or branched C2-6 alkynyl groups such as an ethynyl group, 1-propynyl group, 2-propynyl group, butynyl group, pentynyl group and hexynyl group.

Preferable examples of R<5 >in the specification include a hydrogen atom and a halogen atom (for example, a fluorine atom, chlorine atom, bromine atom etc.). The partial structure:

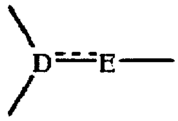

may be either a group represented by the formula >C(R<6>)-CH2- (wherein R<6 >is a hydrogen atom or a halogen atom) or >C-CH-, preferably a group represented by the formula >C(R<6>)-CH2- wherein R<6 >is a hydrogen atom or a halogen atom. Herein, the "halogen atom" represented by R<6 >means an atom having the same meaning as the halogen atom in the above definition, and R<6 >is preferably a hydrogen atom, fluorine atom, chlorine atom or bromine atom, more preferably a hydrogen atom or fluorine atom. That is, the group which can be represented by the partial structure is more preferably the formula >CH-CH2-, >C(F)-CH2-, >C(C1)-CH2- or >C(Br)-CH2-, further preferably the formula >CH-CH2- or >C(F)-CH2-.

The "halide ion" represented by X<-> in the specification refers to a fluoride ion, chloride ion, bromide ion, iodide ion etc., preferably a chloride ion, bromide ion and iodide ion, more preferably a chloride ion and bromide ion, most preferably a chloride ion. The "organic sulfonic acid ion" represented by X<-> refers to a methanesulfonate ion, trifluoromethanesulfonate ion, ethanesulfonate ion, benzenesulfonate ion, toluenesulfonate ion and camphor sulfonate ion etc. In the specification, m is 0 or an integer from 1 to 5, whereupon the partial structure:

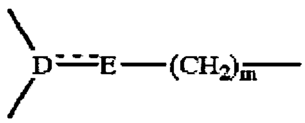

represents (1) the formula >C(R<6>)-CH2- or >C-CH- when m is 0, (2) the formula >C(R<6>)-(CH2)2- or >C=CH-(CH2)- when m is 1, (3) the formula >C(R<6>)-(CH2)3- or >C-CH-(CH2)2- when m is 2, (4) the formula >C(R<6>)-(CH2)4- or >C-CH-(CH2)3- when m is 3, (5) the formula >C(R<6>)-(CH2)5- or >C-CH-(CH2)4- when m is 4, and (6) the formula >C(R<6>)-(CH2)6- or >C-CH-(CH2)5- when m is 5, respectively (in the formula, R<6 >has the same meaning as defined above). m is preferably 0, 2 or 4, more preferably 0 or 2.

One instances of the acetylcholinesterase inhibitor in the present disclosure includes, for example, those acetylcholinesterase inhibitors comprising the following compounds. However, it goes without saying that the instances of the present disclosure are not limited to those acetylcholine esterase inhibitors comprising these compounds.

The acetylcholinesterase inhibitor comprises any one or two or more 1-benzylpyridinium salts selected from:

1-benzyl-4-(1-indanone-2-yl)methylpyridinium chloride;
1-benzyl-4-[(4-methoxy-1-indanone)-2-yl]methylpyridinium chloride;
1-benzyl-4-[(5-methoxy-1-indanone)-2-yl]methylpyridinium chloride;
1-benzyl-4-[(6-methoxy-1-indanone)-2-yl]methylpyridinium chloride;
1-benzyl-4-[(7-methoxy-1-indanone)-2-yl]methylpyridinium chloride;
1-benzyl-4-[(5,6-dimethoxy-1-indanone)-2-yl]methylpyridinium chloride;
1-benzyl-4-[(5,7-dimethoxy-1-indanone)-2-yl]methylpyridinium chloride;
1-benzyl-4-[(4,7-dimethoxy-1-indanone)-2-yl]methylpyridinium chloride;
1-benzyl-4-[(4,5-dimethoxy-1-indanone)-2-yl]methylpyridinium chloride;
1-benzyl-4-[(6,7-dimethoxy-1-indanone)-2-yl]methylpyridinium chloride;
1-benzyl-4-[(5,6,7-trimethoxy-1-indanone)-2-yl]methylpyridinium chloride;
1-benzyl-4-[(5,6-diethoxy-1-indanone)-2-yl]methylpyridinium chloride;
1-benzyl-4-(1-indanone-2-yl)methylpyridinium bromide;
1 -benzyl-4-[(4-methoxy-1-indanone)-2-yl]methylpyridinium bromide;
1-benzyl-4-[(5-methoxy-1-indanone)-2-yl]methylpyridinium bromide;
1-benzyl-4-[(6-methoxy-1-indanone)-2-yl]methylpyridinium bromide;
1-benzyl-4-[(7-methoxy-1-indanone)-2-yl]methylpyridinium bromide;
1-benzyl-4-[(5,6-dimethoxy-1-indanone)-2-yl]methylpyridinium bromide;
1-benzyl-4-[(5,7-dimethoxy-1-indanone)-2-yl]methylpyridinium bromide;
1-benzyl-4-[(4,7-dimethoxy-1-indanone)-2-yl]methylpyridinium bromide;
1-benzyl-4-[(4,5-dimethoxy-1-indanone)-2-yl]methylpyridinium bromide;
1-benzyl-4-[(6,7-dimethoxy-1-indanone)-2-yl]methylpyridinium bromide;
1-benzyl-4-[(5,6,7-trimethoxy-1-indanone)-2-yl]methylpyridinium bromide; and
1-benzyl-4-[(5,6-diethoxy-1-indanone)-2-yl]methylpyridinium bromide.

xi) International Patent Application Publication No. WO2000/033840

In some instances compounds include donepezil, rivastigmine, galantamine, lycoramine and the analogs of galantamine and lycoramine. Suitable compounds are galanthamine, lycoramine and their analogs wherein wherein at least one of the methoxy, hydroxy or methyl groups of the galanthamine or lycoramine is replaced as follows:

the methoxy group by another alkoxy group of from one to six carbon atoms, a hydroxy group, hydrogen, an alkanoyloxy group, a benzoyloxy or substituted benzoyloxy group, a carbonate group or a carbamate groupor a trialkylsilyloxy group;
the hydroxy group by an alkoxy group of from one to six carbon atoms, hydrogen, an alkanoyloxy group, a benzoyloxy or substituted benzoyloxy group, a carbonate group or a carbamate group;
the N-methyl group by hydrogen, alkyl, benzyl, cyclopropylmethyl group or a substituted or unsubstituted benzoyloxy group.

One or more of the methoxy, hydroxy and methyl groups of galanthamine or lycoramine may be replaced by the groups noted above.
When reference is made to a substituent group, said group may be selected from alkyl or alkoxy groups of from 1 to 6 carbon atoms, halo groups, and haloalkyl groups such as trifluoromethyl. When reference is made to alkyl groups, where the context permits, the term also includes groups which are or contain cycloalkyl groups including adamantyl. Aryl groups are typically phenyl or naphthyl but may include heteroatyl such as morpholino.
Galanthamine and lycoramine have the following formulae:

Galanthamine

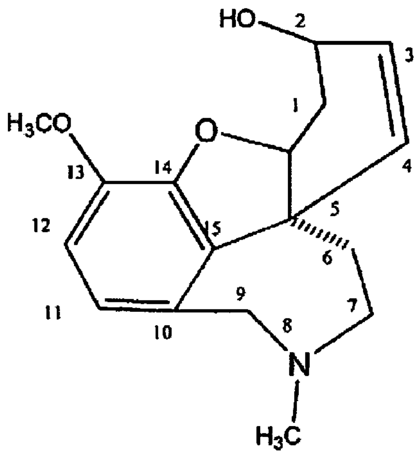

Lycoramine

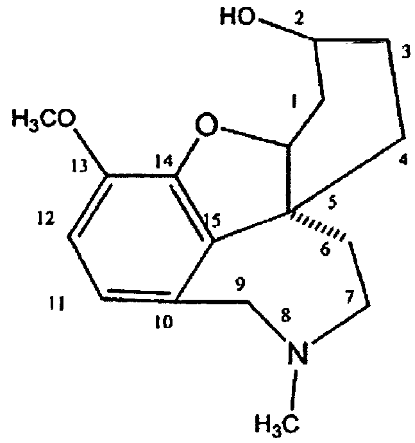

Suitable analogs are described for example in International Patent Publication WO88/08708 and an article by Bores and Kosley in Drugs of the Future 21: 621-631 (1996).

Other useful pharmacologic agents for such preparations include rivastigmine, and other pharmacologic agents with half lives of 1-11 hours.

Particularly useful analogs of galanthamine and lycoramine that are of use in the present disclosure include analogs thereof wherein the methoxy group of such compounds is replaced by a hydrogen, hydroxy or alkoxy group of from two to six carbon atoms or an acyloxy group, for example an alkanoyloxy of from one to seven carbon atoms or benzoyl group, or where the methoxy group is replaced by a mono or dialkyl carbamate or a mono or dialkyl carbonate group wherein the alkyl groups contain from 1 to 8 carbon atoms, preferably of from 4 to 8 carbon atoms or wherein the methoxy group thereof is replaced by an aryl carbamate or carbonate group wherein said aryl group is selected from phenyl, naphthyl, substituted phenyl and substituted naphthyl groups wherein said substituent is selected from alkyl and alkoxy groups of from 1 to 6 carbon atoms, trifluoro methyl groups and halo groups.

Other useful analogs include compounds wherein, independently of whether or not the methoxy group has been replaced, the hydroxy group is replaced by an alkoxy group of from one to six carbon atoms, hydrogen, an acyloxy group, for example an alkanoyloxy group, typically of from 1 to 7 carbon atoms, a benzoyloxy or substituted benzoyloxy group wherein said substituent is selected from alkyl and alkoxy groups of from 1 to 6 carbon atoms, trifluoro methyl groups and halo groups, a carbonate group or a carbamate group which may be a mono or dialkyl or an aryl carbamate or carbonate wherein the alkyl groups contain from 1 to 8 carbon atoms, preferably of from 4 to 6 carbon atoms or said aryl group is selected from phenyl, naphthyl, substituted phenyl and substituted naphthyl groups wherein said substituent is selected from alkyl and alkoxy groups of from 1 to 6 carbon atoms, trifluoromethyl groups and halo groups.

xii) International Patent Application Publication No. WO1999/007359

In some instances, cholinesterase inhibitors may include, but are not limited to, physostigmine, tacrine and

tacrine analogues, fasiculin, metrifonate, heptyl-physostigmine, norpyridostigmine, norneostigmine, huperazine, donepezil and prodrugs of any of these in which the inhibitor is modified in accordance with principles of pro-drug construction known in the art. Examples of such modifications include the introduction of hydrophilic or lipophilic groups to enhance solubility, or penetration through cell membranes, respectively.

In some instances, cholinesterase inhibitors are acetylcholinesterase inhibitors, particularly those which are capable of crossing the blood brain barrier. Particularly preferred cholinesterase inhibitors for use according to the disclosure include galantamine, epigalantamine and norgalantamine, and analogues, salts and derivatives of any of these. Galantamine was previously known as galanthamine. It is a tertiary alkaloid which can be extracted from various snowdrop bulbs e.g. the Caucasian snowdrop galanthus woronowii(Amaryllidaceae) and related species and daffodil bulbs or made by chemical synthesis. It has a high selectivity for acetylcholinesterase as opposed to butyrylcholinesterase. It is active substantially selectively at nicotinic receptor sites with substantially little effect on muscarinic receptor sites.

Particularly preferred cholinesterase inhibitors for use in the disclosure are galantamine and its derivatives of formula (I):

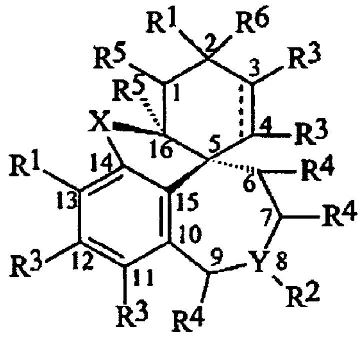

wherein the broken line represents an optionally present double bond between carbon atoms 3 and 4, each R1 is independently selected from hydrogen, hydroxyl, straight or branched chain alkyl, hydroxyalkyl, carboxyalkyl amino, alkylamino, acyl, lower alkanoyl, cyano, sulfhydryl, C16alkoxy, alkylthio, aryloxy, arylthio, R3substituted aryloxy, R3-substituted arylthio, aralkoxy, an optionally R3-substituted aliphatic or aryl carbamyl group, aralkylthio, R3-substituted aralkoxy, R3substituted aralkylthio, aryloxymethyl, R3-substituted aryloxymethyl, alkanoyloxy, hydroxy-substituted alkanoyloxy, benzoyloxy, R3-substituted benzoyloxy, aryloxycarbonyl and R3- substituted aryloxycarbonyl,

R2 is selected from hydrogen, straight or branched chain C16alkyl, alkenyl or alkaryl group, optionally substituted by a halogen atom or a cycloalkyl, hydroxy, alkoxy, nitro, amino, aminoalkyl, acylamino, heteroaryl, heteroaryl-alkyl, aryl, arylalkyl, cyano, amyl, aroyl, cycloalkylmethyl, allyl, phenyl, R3-substituted phenyl, alkylphenyl, R3-substituted alkylphenyl, heterocyclyl selected from a- or ss-furyl, a- or ss-thienyl, thenyl, pyridyl, pyrazinyl, and pyrimidyl, alkyl-heterocyclyl or $R^1$-substituted heterocyclyl, where $R^1$ is alkyl or alkoxy,

each R3 is independently selected from hydrogen, hydroxyl, sulfhydryl, alkyl, hydroxyalkyl, aryl, aralkyl, alkoxy, mercaptoalkyl, aryloxy, thiaryloxy, alkaryloxy, mercaptoalkaryl, nitro, amino, N-alkylamino, N-arylamino, N-alkarylamino, fluoro, chloro, bromo, iodo, and trifluoromethyl,

each R4 is independently selected from hydrogen, halo, trifluoromethyl or C14-alkyl,

each R5 is independently selected from hydrogen or hydroxymethyl,

R6 is hydrogen or C16alkyl, or when R1 at carbon atom 2 is hydroxyl, R6 may be a moiety of formula I wherein R6 is hydrogen and R1 is a linking bond; or

R1 at carbon atom 2 and R6 may jointly form semicarbazone,

X is oxygen or NR3,

Y is nitrogen or phosphorus,

and methylenedioxy derivatives thereof and pharmaceutically acceptable acid addition salts thereof.

Of the compounds of formula I which may be used in the method of the disclosure, some compounds are those in which the alkyl moieties contain 1 to 8 carbon atoms, halogen atoms are preferably fluorine, bromine, chlorine, aryl moieties are preferably phenyl, cycloalkyl groups are preferably 3- to 7-membered rings, especially cyclopropyl or cyclobutyl, acyl groups are preferably lower alkanoyl groups and heteroaryl moieties are preferably 5- to 8-membered rings, e.g., thienyl, furyl, pyridyl, pyrrolyl, or pyrizanyl.

In some instances, compounds of formula I are the compounds of formula II

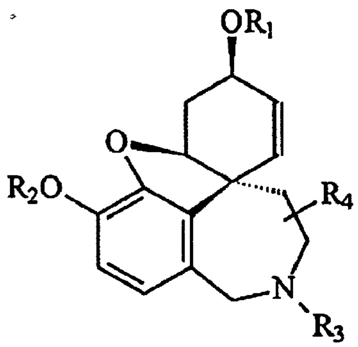

wherein R1 and R2 which may be the same or different each represents a hydrogen atom or an acyl group, such as a lower alkanoyl group, e.g. an acetyl group or a straight-chained or branched alkyl group, e.g. methyl, ethyl, propyl, or isopropyl; R3 is a straight or branched chain alkyl, alkenyl or alkaryl group which is optionally substituted by a halogen atom or a cycloalkyl, hydroxy, alkoxy, nitro, amino, aminoalkyl, acylamino, heteroaryl, heteroarylalkyl, aroyl, aroylalkyl or cyano group; and

R4 represents a hydrogen or a halogen atom attached to at least one of the ring carbons of the tetracyclic skeleton,

and pharmaceutically acceptable salts thereof, such as a hydrobromide, hydrochloride, methylsulphate or methiodide.

Formula II includes galantamine itself.

In some instances, a compound is galantamine itself, and salts thereof such as halides, for example galantamine hydrobromide.

xiii) International Patent Application Publication No. WO1997/038993

This application relates to compounds of the formula

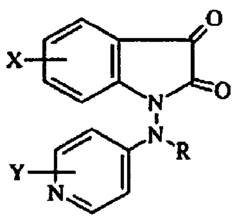

(I)

wherein

R is hydrogen, (C1-C6)alkyl or hydroxy (C-C6)alkyl;

X is hydrogen, hydroxy, (Ci-C6)alkoxy, phenyl(C1-C6)alkoxy, or -O(C=O)NRR; and

Y is hydrogen or halogen;

where R is (C1-C6)alkyl, phenyl, or phenyl(Cl-C6)alkyl, where the phenyl group is optionally substituted by (C1-C6)alkyl, (C,-C6)alkoxy, halogen or trifluoromethyl;

R2 is hydrogen or (C,-C6)alkyl; or

R and R2 taken together with the nitrogen to which they are attached form a tetrahydroisoquinoline group; and its pharmaceutically acceptable acid addition salts.

The term "alkyl" shall mean a straight or branched alkyl group of the stated number of carbon atoms. Examples include, but are not limited to methyl, ethyl, n-propyl, iso-propyl, n-butyl, isobutyl, sec-butyl, t-butyl, and straight and branched chain pentyl and hexyl.

The term "halo" shall mean chloro, fluoro, bromo and iodo.

The term "phenyl" shall mean phenyl having 0, 1, 2 or 3 substituents independently selected from the group of (C1-C6)alkyl, (Cl-C6)alkoxy, halo or trifluoromethyl. In a particular instance of the disclosure are compounds of the Formula (I)

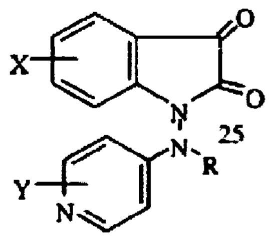

wherein

R is hydrogen, (C1-C6)alkyl or hydroxy(C1-C6)alkyl;
X is hydrogen, hydroxy, (C1-C6)alkoxy, benzyloxy, or -O(C=O)NR1R2 ; and
Y is hydrogen or halogen;
where R1 is (C1-C6)alkyl, phenethyl, or benzyl, where the phenyl group is optionally substituted by (C,-C6)alkyl, (C1-C6)alkoxy, halogen or trifluoromethyl; and
R2 is hydrogen or (C,-C6)alkyl; and its pharmaceutically acceptable acid addition salts.

In one instance, when R1 is (C-C6)alkyl, R2 is hydrogen.
In one instance are compounds of Formula I wherein R is (C1-C6)alkyl.
In one instance X is hydrogen, Y is hydrogen and R is hydrogen, methyl, ethyl or propyl.
In another instance are compounds of Formula I

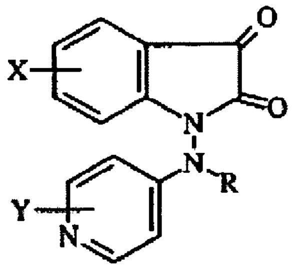

(I)

wherein

R is hydrogen, (Cs-C6)alkyl or hydroxy(CI-C6)alkyl;
X is hydrogen, hydroxy, (C1-C6)alkoxy, benzyloxy, or -O(C=O)NRIR2 ; and
Y is hydrogen or halogen; wherein
R and R2 taken together with the nitrogen to which they are attached form a tetrahydroisoquinoline group; and its pharmaceutically acceptable acid addition salts.

In one instance X is -O(C=O)NR'R2 and Y is hydrogen.

xiv) U.S. Patent 5,965,569

The new aminopyridine compounds which are the subject of the present disclosure correspond to the general formula (I):

(I)

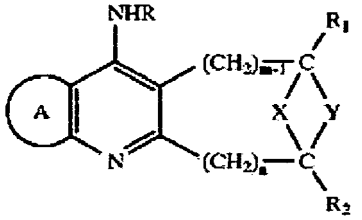

in which R can be hydrogen, alkyl, aralkyl or acyl;

R1 and R2 can be, independently, hydrogen, alkyl, aralkyl, alkoxy, alkoxycarbonyl, amino or amino substituted with one or two alkyl, aralkyl or acyl groups;

m and n can adopt the values 1, 2 or 3;

X and Y can be, independently, a bond between two carbons, an oxygen or sulphur atom, a group N--R3 or an alkylene or alkenylene bridge containing from 1 to 5 carbon atoms and which can contain one or more substituents R4. When X is an alkenylene group, the latter can be fused to a saturated or unsaturated carbocyclic or heterocyclic ring system, it being possible for the ring to be substituted with one or more groups R5 ; for example, X can be an ortho-phenylene group;

and

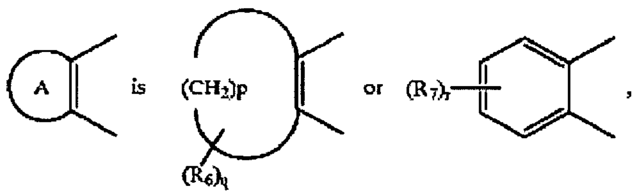

p, q and r having a value equal to or greater than one and R6 and R7 being substituents which can individually be hydrogen, halogen, preferably fluorine or chlorine, lower alkoxy or lower alkyl.

In the above definitions:

The term "alkyl" represents a hydrocarbon residue having one to six carbon atoms with linear, branched, substituted cyclic or cycloalkyl chains, for example methyl, ethyl, propyl, isopropyl, butyl, sec-butyl, pentyl, cyclopentyl, cyclopentylmethyl, cyclohexyl, and the like.

The term "aralkyl" means phenylalkyl or phenylalkyl substituted on the phenyl, containing from 7 to 12 carbon atoms. The term alkyl in "phenylalkyl" or "phenylalkyl substituted on the phenyl" means an alkylene group having a linear chain containing from one to four carbon atoms, for example methylene, ethylene, trimethylene or tetramethylene. The substituted phenyl in "phenylalkyl substituted on the phenyl" is a phenyl group containing one or more substituents selected from the group consisting of halogen, for example fluorine, chlorine, bromine and iodine, lower alkyl which includes alkyl groups containing from one to four carbon atoms with linear or branched chains, for example methyl, ethyl, propyl, isopropyl, butyl, sec-butyl and tert-butyl, and lower alkoxy which includes an alkoxy group having a linear or branched chain containing from one to four carbon atoms, for example methoxy, ethoxy, propoxy, isopropoxy, butoxy and sec-butoxy. Examples of such aralkyl groups include benzyl, phenethyl, 3-phenylpropyl, 4-phenylbutyl, 2-(4-methoxyphenyl)ethyl, 2-(2-methylphenyl)ethyl, 2-(4-fluorophenyl)ethyl and 4-(4-chlorophenyl)butyl.

The term "acyl" means an alkylcarbonyl or aralkylcarbonyl group in which the alkyl and aralkyl residues can adopt the meanings defined before.

In the context of R1 and R2, the terms alkyl and aralkyl have the meaning given above for R. The alkoxy substituent and the alkoxy group of the alkoxycarbonyl substituent can adopt the meanings given above for the lower alkoxy group. The alkyl, aralkyl and acyl substituents of the amino group can also adopt the meanings given above in the context of R.

The group R3 of N--R3 can adopt the meanings defined above for R.

The groups R4 attached to the alkylene or alkenylene bridge can be, independently, hydrogen, lower alkyl, alkenyl or alkylidene having one to four carbon atoms with a linear or branched chain, phenyl, phenyl substituted with one or more lower alkyl groups having one to four carbon atoms, lower alkoxy groups having one to four carbon atoms or halogen (fluorine, chlorine, bromine or iodine) groups, aralkyl as defined above in the context of R, lower alkoxy containing from one to four carbons, and hydroxyl.

The groups R5 which are substituents of the ring fused to X or Y can be hydrogen, lower alkyl or lower alkoxy having one to four carbon atoms, or halogen (fluorine, chlorine, bromine and iodine).

Since the compounds of general formula (I) have at least two chiral centres, that is to say two asymmetric carbons, capable of generating optical isomerism, the present disclosure relates both to the racemic compounds and to all the possible enantiomers of these compounds or to the mixtures thereof in different proportions. The pharmaceutically acceptable addition salts can be with organic or inorganic acids, such as hydrochloric, hydrobromic, sulphuric and nitric acids among inorganic acids and tartaric, succinic, maleic, fumaric and citric acids among organic acids.

xv) U.S. Patent 5,663,448

Compounds of the present disclosure have the following Formula I

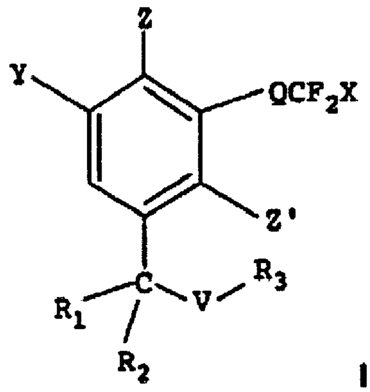

I

stereoisomers and pharmaceutically acceptable salts thereof, wherein each of Z and Z' are independently H or F;
Q is

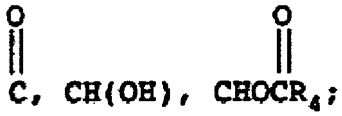

X is H, Br, Cl, F or CF3 ; Y is H, Br, Cl, F, OH, OR5, OC(O)R4, N3, CN, NO2, SO3 H, CO2 R4, NH2, NHR9, NR9 R'9, C(R6)(R7)(V'R8) or C(O)R7, provided that when both Z and Z' are F, then Y is H or F;
V and V' are each independently CH2 or O;
R1 is H or CH3 ;
R2, R9 and R'9 are each independently (C1-6)alkyl, or R2 and V--R3 taken together with the carbon atom to which they are attached form a 3-6 membered ring;
R3, R6, R7 and R8 are each independently H, (C1-6)alkyl, or (C3-6)cycloalkyl; R4 is H, (C1-10)alkyl, (C0-4)alkylene aryl or (C3-8)cycloalkyl; and
R5 is (C1-10)alkyl, benzyl, phenethyl or (C3-6)cycloalkyl.

The terms "(C1-6)alkyl" and "(C1-10)alkyl" mean straight or branched chain alkyl radicals containing respectively from 1 to 6 carbon atoms and from 1 to 10 carbon atoms, including, but not limited to, methyl, ethyl, n-propyl, iso-propyl, n-butyl, iso-butyl, sec-butyl, t-butyl, n-pentyl, 1-methylbutyl, 2,2-dimethylbutyl, 2-methylpentyl, 2,2-dimethylpropyl, n-hexyl and so on. Likewise, the term "(C0-4)alkylene aryl" can mean straight or branched chain alkylene groups up to 4 carbon atoms such as ethylethylene, 2-methyltrimethylene, and so on. C0 of course means no alkylene moiety attached to the aryl.

"Hydroxy(C1-6)alkyl" means a (C1-6)alkyl group having from 1 to 3 hydroxy substituents thereon. Preferably, there is only one hydroxy substituent at the alpha position (attached to the carbon atom which is directly attached to the phenyl). "Ts" or "tosyl" means ##STR5## Tosyl derivatives mean ##STR6## wherein R is C1-6 alkyl.

"Aryl" includes both carbocyclic and heterocyclic moieties of which phenyl, pyridyl, indolyl, indazolyl, furyl and thienyl are of primary interest; these moieties being inclusive of their position isomers such as, for example, 2-, 3-, or 4-pyridyl, 2- or 3-furyl and thienyl, 1-, 2-, or 3-indolyl or the 1- and 3-indazolyl, as well as the dihydro and tetrahydro analogs of the furyl and thienyl moieties. Also included within the term "aryl" are such fused carbocyclic moieties as pentalenyl, indenyl, naphthalenyl, azulenyl, heptalenyl, acenaphthylenyl, fluorenyl, phenalenyl, phenanthrenyl, anthracenyl, acephenanthrylenyl, aceanthrylenyl, triphenylenyl, pyrenyl, chrysenyl and naphthacenyl. Also included within the term "aryl" are such other heterocyclic radicals as 2- or 3-benzo[b]thienyl, 2- or 3-naphtho[2,3-b]thienyl, 2- or 3-thianthrenyl, 2H-pyran-3-(or 4- or 5-)yl, 1-isobenzofuranyl, 2H-chromenyl-3-yl, 2- or 3-phenoxathiinyl, 2- or 3-pyrrolyl, 4- or 3-pyrazolyl, 2-pyrazinyl, 2-pyrimidinyl, 3-pyridazinyl, 2-indolizinyl, 1-isoindolyl, 4H-quinolizin-2-yl, 3-isoquinolyl, 2-quinolyl, 1-phthalazinyl, 1,8-naphthyridinyl, 2-quinoxalinyl, 2-quinazolinyl, 3-cinnolinyl, 2-pteridinyl, 4aH-carbazol-2-yl, 2-carbazolyl, .beta.-carbolin-3-yl, 3-phenanthridinyl, 2-acridinyl, 2-perimidinyl, 1-phenazinyl, 3-isothiazolyl, 2-phenothiazinyl, 3-isoxazolyl, 2-phenoxazinyl, 3-iso-chromanyl, 7-chromanyl, 2-pyrrolin-3-yl, 2-imidazolidinyl, 2-imidazolin-4-yl, 2-pyrazolidinyl, 3-pyrazolin-3-yl, 2-piperidyl, 2-piperazinyl, 1-indolinyl, 1-isoindolinyl, 3-morpholinyl, benzo[h]isoquinolinyl, and benzo[b]furanyl, including the position isomers thereof except that the heterocyclic moieties cannot be attached

directly through their nitrogen atoms. Aryl groups can be substituted or unsubstituted with one, two or three substituents independently selected from C1-6 alkyl, haloalkyl, alkoxy, thioalkoxy, aminoalkylamino, dialkylamino, hydroxy, halo, mercapto, nitro, carboxaldehyde, carboxy, carboalkoxy and carboxamide.

When R2 and V--R3 are taken together, they may form a 3 membered ring which includes the carbon atom to which R2 and V are attached (when R3 is H). Other rings formed may have 4, 5 and 6 members to the ring. The term 3-6 membered ring refers to the number of carbon atoms, and oxygen atoms (when V is O) comprising the structure of the ring.

"Stereoisomers" for the compounds of Formula I is a general term for all isomers of individual molecules that differ only in the orientation of their atoms in space. It includes mirror image isomers (enantiomers), geometric isomers (cis/trans), and isomers of compounds with more than one chiral center that are not mirror images of one another (diastereoisomers), whichever forms are applicable to the compound.

xvi) European Patent Application No. EP0611769

Compounds of the present disclosure have the following Formula I:

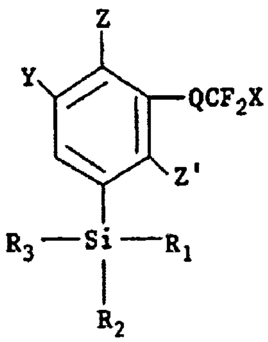

I

stereoisomers or mixtures thereof, and pharmaceutically acceptable salts thereof, wherein:

each of Z and Z' are independently H or F, provided that at least one of Z or Z' is F;

Q is

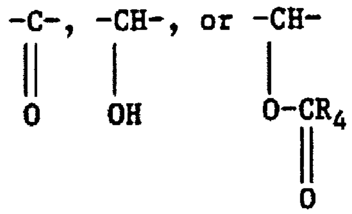

X is H, Br, Cl, F or CF3;

Y is H, OH, (C1-6) alkyl, -(CH2)mOR5, hydroxy (C1-6) alkyl, (CH2)nNR6R6', azido, CN, CO2R4, COR6, SO3H, Br, Cl, F, NO2 or -(CH2)n SiR1'R2'R3', provided that when both Z and Z' are F then Y is H or F;

R1, R2, R3, R1', R2'and R3' are each independently C1-10 alkyl or (CH2)n aryl;

R4 is H, (C1-10) alkyl, phenyl, benzyl or phenethyl;

R5 is H, (C1-10) alkyl, benzyl or phenethyl;

R6 and R6' are independently hydrogen or C1-10 alkyl;

m is an integer of 0, 1, 2, 3 or 4; and

n is an integer of 0, 1 or 2.

The terms "(C1-6) alkyl" and " (C1-10) alkyl" mean straight or branched chain alkyl radicals containing respectively from 1 to 6 carbon atoms and from 1 to 10 carbon atoms, including, but not limited to, methyl, ethyl, n-propyl, isopropyl, n-butyl, iso-butyl, sec-butyl, t-butyl, n-pentyl, 1-methylbutyl, 2,2-dimethylbutyl, 2-methylpentyl, 2,2-dimethylpropyl, n-hexyl and so on. Likewise, the terms "(CH2)n" or "(CH2)m" may represent alkylene chains which may be branched or straight-chained.

"Hydroxy(C1-6) alkyl" means a (C1-6) alkyl group having from 1 to 3 hydroxy substituents thereon. Preferably, there is only one hydroxy substituent at the alpha position (attached to the carbon atom which is directly attached to the

phenyl). "Ts" or "tosyl" means EMI3.1 Tosyl derivatives mean EMI3.2 wherein R is C1-6 alkylene.

"Aryl" includes both carbocyclic and heterocyclic moieties of which phenyl, pyridyl, indolyl, indazolyl, furyl and thienyl are of primary interest; these moieties being inclusive of their position isomers such as, for example, 2-, 3-, or 4-pyridyl, 2- or 3-furyl and thienyl, 1-, 2-, or 3-indolyl or the 1- and 3-indazolyl, as well as the dihydro and tetrahydro analogs of the furyl and thienyl moieties. Also included within the term "aryl" are such fused carbocyclic moieties as pentalenyl, indenyl, naphthalenyl, azulenyl, heptalenyl, acenaphthylenyl, fluorenyl, phenalenyl, phenanthrenyl, anthracenyl, acephenanthrylenyl, aceanthrylenyl, triphenylenyl, pyrenyl, chrysenyl and naphthacenyl.Also included within the term "aryl" are such other heterocyclic radicals as 2- or 3-benzo[b]thienyl, 2- or 3-naphtho-[2,3-b]thienyl, 2- or 3-thianthrenyl, 2H-pyran-3-(or 4- or 5-)yl, 1-isobensofuranyl, 2H-chromenyl-3-yl, 2-or 3-phenoxathiinyl, 2- or 3-pyrrolyl, 4- or 3-pyrazolyl, 2-pyrazinyl, 2-pyrimidinyl, 3-pyridazinyl, 2-indolizinyl, 1-isoindolyl, 4H-quinolizin-2-yl, 3-isoquinolyl, 2-quinolyl, 1-phthalazinyl, 1,8-naphthyridinyl, 2-quinoxalinyl, 2-quinazolinyl, 3-cinnolinyl, 2-pteridinyl, 4aH-carbazol-2-yl, 2-carbazolyl, beta-carbolin-3-yl, 3-phenanthridinyl, 2-acridinyl, 2-perimidinyl, 1-phenazinyl, 3-isothiazolyl, 2-phenothiazinyl, 3-isoxasolyl, 2-phenoxasinyl, 3-isochromanyl, 7-chromanyl, 2-pyrrolin-3-yl, 2-imidazolidinyl, 2-imidazolin-4-yl, 2-pyrazolidinyl, 3-pyrazolin-3-yl, 2-piperidyl, 2-piperazinyl, 1-indolinyl, 1-isoindolinyl, 3-morpholinyl, benzo[h]isoquinolinyl, and benzo[b]furanyl, including the position isomers thereof except that the heterocyclic moieties cannot be attached directly through their nitrogen atoms. Aryl groups can be substituted or unsubstituted with one, two or three substituents independently selected from C1-6 alkyl, haloalkyl, alkoxy, thioalkoxy, aminoalkylamino, dialkylamino, hydroxy, halo, mercapto, nitro, carboxaldehyde, carboxy, carboalkoxy and carboxamide.

R1, R2, R3, R1', R2', and R3' are each independently selected from C1-10 alkyl or (CH2)n aryl which means that, for example, R1 could be benzyl while R2 is methyl. In other words, none of R1, R2, R3, R1', R2', or R3' have to be the same moiety, although this may be the case.

"Stereoisomers" for the compounds of Formula I is a general term for all isomers of individual molecules that differ only in the orientation of their atoms in space. It includes mirror image isomers (enantiomers), geometric isomers (cis/trans), and isomers of compounds with more than one chiral center that are not mirror images of one another (diastereoisomers), whichever forms are applicable to the compound.

xvii) International Patent Application Publication No. WO1993/03034

The present disclosure also includes novel compounds of the formula

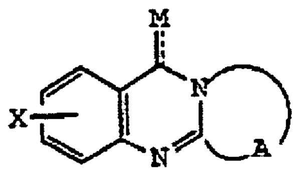

I

wherein A represents

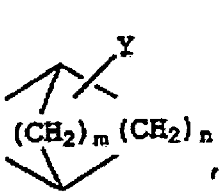 ,

IIa

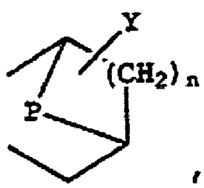 ,

IIb

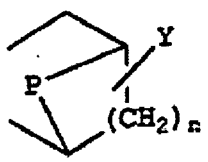 ,

IIc

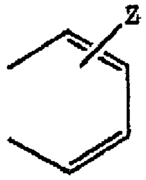 or 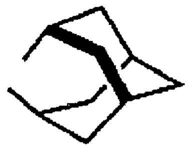

IId IIe

in which n is 1-10, P is a bond or (CH2) m in which m is 0-10, wherein a nitrogen, oxygen, or sulfur atom may replace a methylene group in ring A, which is not adjacent to the quinazoline moiety, and attached to a carbon atom in ring A is Y, in which Y is hydrogen, hydroxy, halogen, carboxy, lower alkoxy, lower alkyl, aryl, heteroaryl, keto, lower alkoxy carbonyl or lower alkanoyl;

M is =S, =NR,

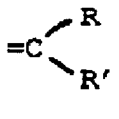 or 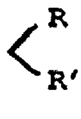

in which R and R' are independently hydrogen, hydroxy, lower alkyl,
lower alkoxy, lower alkenyl, lower alkynyl, aryl, aryloxy, aryllower alkyl, heteroaryl or
heteroaryllower alkyl and, when taken together, may form a three- to six-membered ring optionally containing one to three heteroatoms selected from nitrogen, oxygen, and sulfur; and

X is absent or one to four substituents selected from halogen, alkyl(C1-22), straight or branched,
saturated or alkenyl or alkynyl, if alkyl of appropriate size can form a ring, saturated or
unsaturated, containing (or not containing) one or more heteroatoms, such as O, S, N, Se, P, and
the like, or an aromatic or heteroaromatic ring containing (or not containing) one or more heteroatoms, such as O, S, N, Se, and the like, primary, secondary, or tertiary amino nitro-lower alkylthio, or aryl (or heteroaryl)thio, mercapto, hydroxy, carboxy, lower alkoxy, or aryl (or heteroaryl)oxy, alkyl (C1-22) or aryl (or heteroaryl)sulfinyl, alkyl (C1-22), or aryl (or heteroaryl) sulfonyl, perfluoroalkyl (C1-22), such as trifluoromethyl, perfluoroalkoxy (C1-22) r such as trifluoromethoxy, perfluoroalkylthio (C122), such as trifluoromethylthio, perfluoroalkyl sulfinyl(C122), such as trifluoromethylsulfinyl, perfluoroalkylsulfonyl (C1-22) r such as trifluoromethylsulfonyl, alkyl (C1-22), or aryl (or heteroaryl)carbamoyl, or diacylamino, including cyclic imido, such as succinimido, alkyl(C1~22), or aryl (or heteroaryl)sulfinylamido,
alkyl(C122), or aryl (or heteroaryl) sulfonylamido, perfluoroalkyl (C1-22) sulfinylamido, such as trifluoromethyl sulfinylamido, perfluoroalkyl (C1-22) sulfonylamido, such as trifluoromethyl sulfonylamido above, trialkylsilyl, such as trimethylsilyl, or triethylsilyl, acyl, such as acetyl, benzoyl, phenylacetyl, hydrocinnamoyl, and the like, perfluoroacyl, such as trifluoroacetyl, heptafluorobutyryl, and the like, acyl-lower alkyl, such as acetylmethyl, benzoylmethyl, phenylacetylmethyl, hydrocinnamoylmethyl, and the like, perfluoroacyl-lower alkyl, such as trifluoroacetylmethyl, heptafluorobutyrylmethyl, and the like, alkyl (C1-22), or aryl (or heteroaryl)carbamoyloxy, dialkyl(C122), or diaryl (or diheteroaryl) carbamoyloxy, alkyl(C1-22) or aryl (or heteroaryl) carbamoylthio, alkyl(C1-22) r or aryl (or heteroaryl)carbamoylalkyl, or diacylaminoalkyl, including cyclic imidoalkyl, such as acetamidomethyl, octanamidomethyl, or succinimidomethyl, aryl or aryl lower alkyl, including substituted aryl with groups such as halogen and groups described heteroaryl or heteroaryllower alkyl, such as furan, thiophene, pyrrole, pyridine and the like, including substituted derivatives with groups such as halogen and groups described above; Z is hydrogen, halogen, alkyl (C1-12)r straight or branched, saturated or alkenyl or alkynyl if alkyl of appropriate size can form a ring, saturated or unsaturated, containing or not containing one or more heteroatoms, selected from O, S, and N, can also form an aromatic or heteroaromatic ring containing or not containing one or more heteroatoms, selected from 0, S, and N, primary, secondary, or tertiary aminolower alkylthio-, aryl-, heteroarylthio, mercapto, hydroxy, carboxy, carbalkoxy in which alkyl is C1-C22, lower alkoxy, aryl, or heteroaryloxy, perfluoroalkyl in which alkyl is C1-C22, perfluoroalkoxy in which alkyl portion is C1-C22), alkyl (C122), aryl or heteroarylcarbamoyl, diacylamino, cyclic imido, or acyl, or a pharmaceutically acceptable acid addition salt thereof; with the proviso that when A is

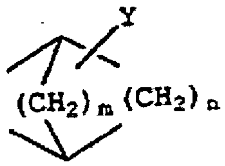

IIa

in which n is 1-3, m is 0, Y is hydrogen, and M is RR' where R and R' are both hydrogen, X cannot be absent or a single hydroxy or methoxy group; and when A if of the Formula IId, X is 1,3-dihalogeno or 2,4-dihalogeno.

xviii) International Patent Application Publication No. WO1992/019238

The present disclosure provides a number of compounds of general formula (I), which are formally derivatives of huperzine A:

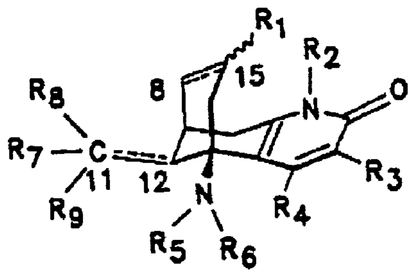

(I)

wherein R1 is H, (C1 -C8)alkyl or halo; R2 is H or (C1 -C8)alkyl; R3 and R4 are individually H, (C1 -C8)alkyl, NO2, hydroxy or halo; R5 and R6 are individually H, (C1 -C8)alkyl, aryl or aralkyl; R7 is H, halo or (C1 -C8)alkyl, R8 is halo or (C1 -C8)alkyl, R9 is absent or is H; and the bonds represented by--are individually absent or, together with the adjacent bond, form the unit C.dbd.C, with the proviso that if both of the bonds represented by--are present, R3 and R4 cannot both be H unless R7 or R8 is halo; and the pharmaceutically acceptable salts thereof. Therefore, the genus of compounds of formula I does not include huperzine A itself, or the simple N-alkylated derivatives thereof. Preferably, R1 is H, halo (Cl, Br, I or F, most preferably F) or methyl. Preferably, R2 is H, preferably R3 is nitro or halo, preferably R4 is (C1 -C4)alkyl or OH, and preferably R5 .dbd.R6 .dbd.H. Preferably R7 and R9 are H and R8 is (C1 -C4)alkyl. Preferably, at least one of the bonds represented by--is absent. Therefore, the preferred compounds of formula I are dihydro or bis(dihydro)analogs of huperzine A which can also comprise substituents on the pyridone ring, or are pyridone ring-substituted analogs of huperzine A.

The compounds of the general formula I or II may exist in the form of optical isomers, and these isomers, as well as racemic (.+-.) mixtures are included within the disclosure. The present disclosure also includes both the 12R and 12S, and both the 15R and 15S enantiomers of the present compounds, as well as unresolved or partially resolved mixtures thereof. The term "alkyl" includes linear or branched alkyl. The terms "aryl" and "aralkyl" are fully defined hereinbelow. In some instances aryl groups include phenyl, tolyl, xylyl, anisyl and the like. In some instances aralkyl groups include arlyoxy- and aryl-(C1 -C3)alkyl moieties.

The C15 --R1 bond is waved to indicate that the R1 substituent, when present may be equatorial or axial, or a mixture thereof. Although for convenience, the C11 - C12 bond is positioned equatorially, it may be either equatorial, axial or a mixture thereof.

xix) European Patent Application No. EP0236684

Galanthamine is generally regarded as having the structure:

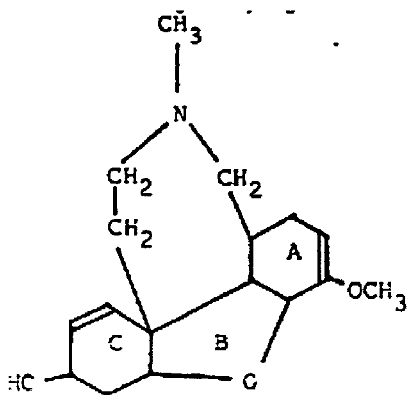

Compounds of a similar structure wherein the hydroxy is replaced by methoxy, ethoxy, lower alkanoyl oxy such as acetyloxy or oxy, the methoxy group is replaced by hydrogen, methoxy, ethoxy, or lower alkanoyloxy such as acetyloxy and the methyl group substituted on the nitrogen atom is replaced by other straight or branch chain lower alkyl groups such as ethyl, cyclopropylmethyl or cyclobutylmethyl, allyl, lower alkyl phenyl or substituted lower alkyl phenyl wherein the substituents are fluoro, chloro, bromo, lower alkoxy, hydroxy, nitro, amino lower alkyl or acylamino of from 1 to 5 carbon atoms, heteroaryl lower alkyl in which the heteroaryl group is thienyl, furyl, pyridyl, pyrrolyl, or pyrazinyl, or a cyano radical; or unsubstituted and halogen substituted benzoyl lower alkyl in which the substituents are on the phenyl ring, and compounds wherein hydrogen atoms in the "core" structure have been replaced by fluoro or chloro groups or the carbon to carbon single bond between the carbons common to the B and C rings is replaced by a double bond are likely to have similar properties to galanthamine.

and xx) U.S. Patent 4,895,841

The disclosure provides a cyclic amine compound having the following formula (XXV) and a pharmacologically acceptable salt thereof:

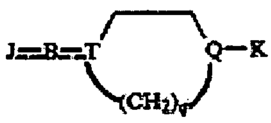

(XXV)

in which J is

(a) a group, substituted or unsubstituted, selected from the group consisting of (1) phenyl, (2) pyridyl, (3) pyrazyl, (4) quinolyl, (5) cyclohexyl, (6) quinoxalyl and (7) furyl;
(b) a monovalent or divalent group, in which the phenyl may have a substituent(s), selected from the group consisting of (1) indanyl, (2) indanonyl, (3) indenyl, (4) indenonyl, (5) indanedionyl, (6) tetralonyl, (7) benzosuberonyl, (8) indanolyl and (9) C6 H5 --CO--CH(CH3)--;
(c) a monovalent group derived from a cyclic amide compound;
(d) a lower alkyl or
(e) a group of R21 --CH.dbd.CH-- in which R21 is hydrogen or a lower alkoxycarbonyl (dbd stands for a double bond);

B is --(CHR22)r --, --CO--(CHR22)r --, --NR4 --(CHR22)r --, R4 being hydrogen, a lower alkyl, an acyl, a lower alkylsulfonyl, phenyl, a substituted phenyl, benzyl or a substituted benzyl, --CO--NR5 --(CHR22)r --, R5 being hydrogen, a lower alkyl or phenyl, --CH.dbd.CH--(CHR22)r --, --OCOO--(CHR22)r --, --OOC--NH-(CHR22)r --, --NH--CO--(CHR22)r --, --CH2 --CO--NH--(CHR22)r --, --(CH2)2--NH--(CHR22)r --, --CH(OH)--(CHR22)r --, r being zero or an integer of 1 to 10, R22 being hydrogen or methyl so that one alkylene group may have no methyl branch or one or more methyl branch, .dbd.(CH--CH.dbd.CH)b--, b being an integer of 1 to 3, .dbd.CH--(CH2)c --, c being zero or an integer of 1 to 9, .dbd.(CH--CH)d .dbd., d being zero or an integer of 1 to 5; --CO--CH.dbd.CH-CH2 --, --CO--CH2 --CH(OH)--CH2 --, --CH(CH3)--CO--NH--CH2 --,-CH.dbd.CH--CO--NH--(CH2)2 --, --NH--, --O--, --S--, a dialkylaminoalkylcarbonyl or a lower alkoxycarbonyl;
T is a nitrogen or carbon;

Q is nitrogen, carbon or

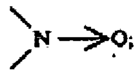

and

q is an integer of 1 to 3;

K is hydrogen, phenyl, a substituted phenyl, an arylalkyl in which the phenyl may have a substituent, cinnamyl, a lower alkyl, pyridylmethyl, a cycloalkylalkyl, adamantanemethyl, furylmenthyl, a cycloalkyl, a lower alkoxycarbonyl or an acyl; and shows a single bond or a double bond.

In the compounds having the formula (XXV), it is preferable that J is (a) or (b). In the definition (b), monovalent groups of (2), (3) and (5) and divalent groups of (2) are preferable. In the definition of B, --(CHR22) r--, .dbd.(CH--CH.dbd.CH)b--, .dbd.CH--(CH2)c-- and .dbd.(CH--CH)d.dbd. are preferable. These preferable groups of (B) may be connected with (b) of J, in particular (2) of (b).

It is preferable in the formula (XXV) that Q is nitrogen, T is carbon and q is 1 or 3; and Q is carbon, T is nitrogen and q is 2. It is most preferable that Q is nitrogen, T is carbon and q is 2.

It is preferable that K is a phenylalkyl or a phenylalkyl having a substituent(s) on the phenyl.

Preferable compounds of the disclosure include:

1-benzyl-4-((5,6-dimethoxy-1-indanon)-2-yl)methylpiperidine,
1-benzyl-4-((5,6-dimethoxy-1-indanon)-2-ylidenyl)methylpiperidine,
1-benzyl-4-((5-methoxy-1-indanon)-2-yl)methylpiperidine,
1-benzyl-4-((5,6-diethoxy-1-indanon)-2-yl)methylpiperidine,
1-benzyl-4-((5,6-methnylenedioxy-1-indanon)-2-yl)methylpiperidine,
1-(m-nitrobenzyl)-4-((5,6-dimethoxy-1-indanon)-2-yl)methylpiperidine,
1-cyclohexymethyl-4-((5,6-dimethoxy-1-indanon)-2-yl)methylpiperidine,
1-(m-florobenzyl)-4-((5,6-dimethoxy-1-indanon)-2-yl)methylpiperidine,
1-benzyl-4-((5,6-dimethoxy-1-indanon)-2-yl)propylpiperidine,
1-benzyl-4-((5-isopropoxy-6-methoxy-1-indanon)-2-yl)methylpiperidine and
1-benzyl-4-((5,6-dimethoxy-1-oxoindanon)-2-yl)propenylpiperidine, having the below shown formula.

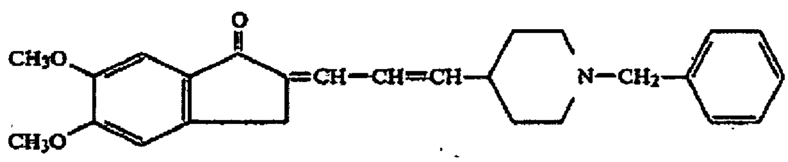

In addition, the disclosure provides a therapeutical composition which comprises a pharmacologically effective amount of the cyclic amine compound having the formula (XXV) or a pharmacologically acceptable salt thereof and a pharmacologically acceptable carrier and then a method for preventing and treating a disease due to the acetylcholinesterase activity by administering to a human patient the cyclic amine compound having the formula (XXV) or a pharmacologically acceptable salt thereof.

The preferable compound has the above shown formula in which J is (b). The group (b) includes ten groups having the respective formulae shown below. S is hydrogen or a substituent such as a lower alkyl having 1 to 6 carbon atoms and a lower alkoxy having 1 to 6 carbon atoms. Among the substituents, methoxy is most preferable. t is an integer of 1 to 4. The phenyl is most preferred to have 1 to 3 methoxy groups thereon. (S)t may form methylene dioxy group or ethylene dioxy group on two adjacent carbon atoms of the phenyl group.

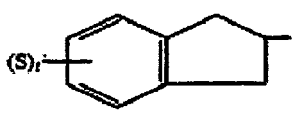

indanyl

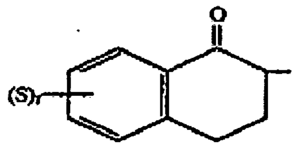

tetralonyl

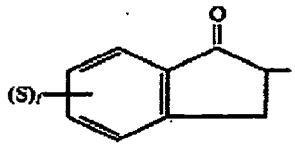

indanonyl

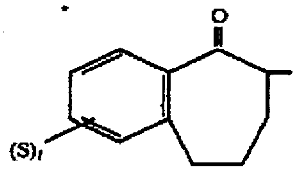

benzosuberonyl

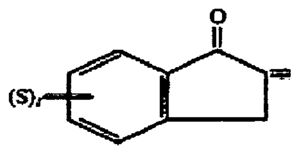

indanonylidenyl

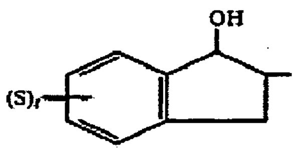

indanolyl

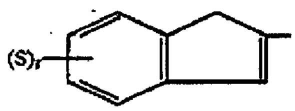

indenyl

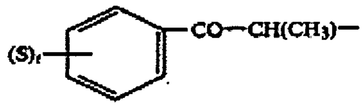

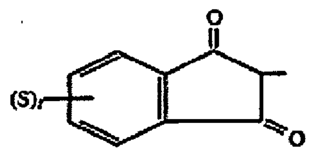

indanedionyl

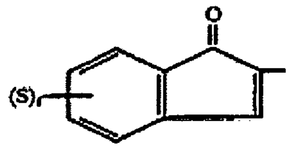

indenonyl

A preferable definition of B includes --(CHR22)r --, --CO--(CHR22)r --, .dbd.(CH--CH.dbd.CH)b --, .dbd.CH--(CH2)c -- and .dbd.(CH--CH)d .dbd.. The group of-(CHR22)r -- in which R22 is hydrogen and r is an integer of 1 to 3 and then the group of .dbd.CH--(CH2)c --are most preferable.

In the above defined cyclic amine compound of the disclosure, it is preferable that J in the formula is (b) the monovalent or divalent group. In the definition (b), indanonyl, indanedionyl and indenyl are most preferable, optionally having a substituent(s) on the phenyl.

In the definition B, --(CHR22)r-- and .dbd.CH--(CH2)c -- are preferable.

In the ring including T and Q, it may be a 5-, 6-or 7-membered ring. It is preferable that Q is nitrogen, T is carbon or nitrogen and n is 2; Q is nitrogen, T is carbon and n is 1 or 3; and Q is carbon, T is nitrogen and n is 2.

In the definition K, phenyl, an arylalkyl and cinnamyl are preferable, optionally having a substituent(s) on the phenyl.

The disclosure will be explained in detail in view of the piperidine compounds which fall within the scope of the above defined cyclic amine compound. The explanation applies to the entire disclosure of the cyclic amine compound.

The piperidine compound is defined by the formula (I):

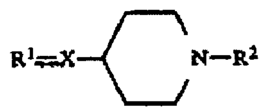

(I)

wherein R1 is the following substituted or unsubstituted group: .circle.1 a phenyl group, .circle.2 a pyridyl group, .circle.3 a pyrazyl group, .circle.4 a quinolyl group, .circle.5 an indanyl group, .circle.6 a cyclohexyl group, .circle.7 a quinoxalyl group, or 11 .circle.8 a furyl group; a monovalent or divalent group derived from an indanone having an unsubstituted or substituted phenyl ring; a monovalent group derived from a cyclic amide compound; a lower alkyl group or a group represented by the formula R3 --CH.dbd.C-- (wherein R3 is a hydrogen atom or a lower alkoxycarbonyl group), X is a group represented by the formula --(CH2)n --, a group represented by the formula

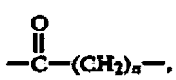

a group represented by the formula

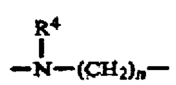

(wherein R4 is a hydrogen atom, a lower alkyl group, an acyl group, a lower alkylsulfonyl group, or a substituted or unsubstituted phenyl or benzyl group), a group represented by the formula

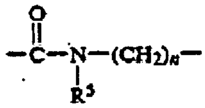

(wherein R5 is a hydrogen atom, a lower alkyl group, or a phenyl group), a group represented by the formula --CH.dbd.CH--(CH2)n --, a group represented by the formula

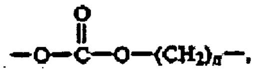

a group represented by the formula

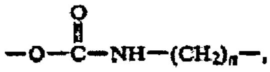

a group represented by the formula -CH=CH-CH=CO-, a group represented by the formula

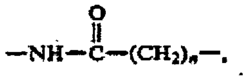

a group represented by the formula

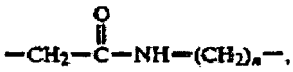

a group represented by the formula

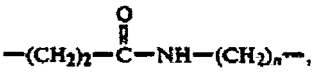

a group represented by the formula

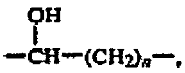

a group represented by the formula

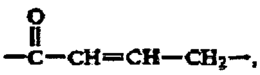

a group represented by the formula

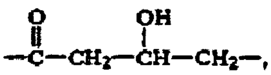

a group represented by the formula

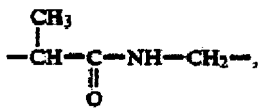

a group represented by the formula

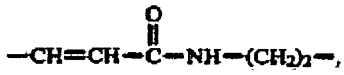

a dialkylaminoalkylcarbonyl group, or a lower alkoxycarbonyl group,
provided that n's in the above definition of X are each independently an integer of 0 to 6,
R2 is a substituted or unsubstituted phenyl group, a substituted or unsubstituted arylalkyl group, a cinnamyl group, a lower alkyl group, a pyridylmethyl group, a cycloalkylalkyl group, an adamantanemethyl group, or a furoylmethyl group, and a symbol, ---- in the above general formula, means a single bond or a double bond.

The term "lower alkyl group" used in the above definition of R1, R2, R4 and R5 with respect to the compound (I) of the present disclosure is intended to mean a straight-chain or branched alkyl group having 1 to 6 carbon atoms, and examples thereof include methyl, ethyl, propyl, isopropyl, butyl, isobutyl, sec-butyl, tert-butyl, pentyl (amyl), isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimenthylbutyl, 2,2-dimethylbutyl, 1,3-dimethylbutyl, 2,3-dimenthylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl groups. Among them, methyl, ethyl, propyl, isopropyl groups etc, are preferable. A methyl group is the most preferable.

Examples of the substituent involved in the expression "the following substituted or unsubstituted group: (1) a phenyl group, (2) a pyridyl group, (3) a pyrazyl group, (4) a quinolyl group, (5) an indanyl group, (6) a cyclohexyl group, (7) a quinoxalyl group, or (8) a furyl group" in the definition of R1 include lower alkyl groups having 1 to 6 carbon atoms, such as methyl, ethyl, n-propyl, isopropyl, n-butyl, isobutyl, and tert-butyl groups; lower alkoxy group corresponding to the above-described lower alkyl groups, such as methoxy and ethoxy groups; a nitro group; halogen atoms such as chlorine, bromine, and fluorine; a carboxyl group; lower alkoxycarbonyl groups corresponding to the above-described lower alkoxy groups, such as methoxycarbonyl, ethoxycarbonyl, isopropoxycarbonyl, n-propoxycarbonyl, and n-butyloxycarbonyl groups; an amino group; a lower monoalkylamino group; a lower dialkylamino group, a carbamoyl group; acylamino groups derived from aliphatic saturated monocarboxylic acids having 1 to 6 carbon atoms, such as acetylamino, propionylamino, butyrylamino, isobutyrylamino, valerylamino, and pivaloylamino groups; cycloalkyloxycarbonyl groups such as a cyclohexyloxycarbonyl group; lower alkylaminocarbonyl groups such as methylaminocarbonyl and ethylaminocarbonyl groups; lower alkylcarbonyloxy groups corresponding to the above-defined lower alkyl groups, such as methylcarbonyloxy, ethylcarbonyloxy, and n-propylcarbonyloxy groups; halogentated lower alkyl groups including a trifluoromethyl group; a hydroxyl group; a formyl group; and lower alkoxy lower alkyl groups such as ethoxymethyl, methoxymethyl, and methoxyethyl groups. The "lower alkyl groups" and "lower alkoxyl groups" in the above description of the substituent include all the groups derived from the above-mentioned groups. The substitutent may be one to three of them which may be the same or different.

Further when the substitutent is a phenyl group, the following group is within the scope of the substituted phenyl group:

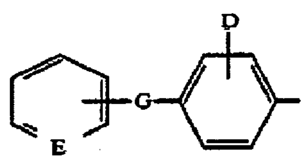

wherein G is a group represented by the formula

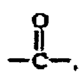

a group represented by the formula

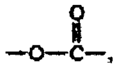

a group represented by the formula --O--, a group represented by the formula

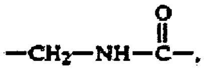

a group represented by the formula --CH2 --O--, a group represented by the formula --CH2 --SO2 --, a group represented by the formula

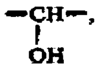

and a group represented by the formula

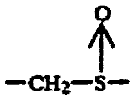

and E is a carbon or nitrogen atom.

Preferable examples of the substituents for the phenyl group among them include lower alkyl, lower alkoxy, nitro, halogenated lower alkyl, lower alkoxycarbonyl, formyl, hydroxyl, and lower alkoxy lower alkyl groups, halogen atoms, and benzoyl and benzylsulfonyl groups. The substituent may be two or more of them which may be the same or different.

Preferable examples of the substituent for the pyridyl group include loweralkyl and amino groups and halogen atoms.

Preferable examples of the substituent for the pyrazyl group include lower alkoxycarbonyl, carboxyl, acylamino, carbamoyl, and cycloalkyloxycarbonyl groups.

With respect to R1, the pyridyl group is preferably a 2-pyridyl, 3-pyridyl, or 4-pyridyl group; the pyrazyl group is preferably a 2-pyrazinyl group; the quinolyl group is preferably a 2-quinolyl or 3-quinolyl group; the quinoxalinyl group is preferable a 2-quinoxalinyl or 3-quinoxalinyl group; and the furyl group is preferably a 2-furyl group.

Specific examples of preferable monovalent or divalent group derived from an indanone having an unsubstituted or substituted phenyl ring include those represented by the following formulae (II) and (III):

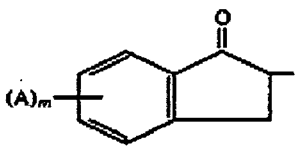

(II)

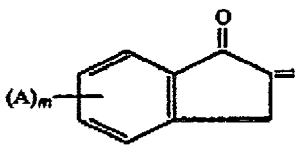

(III)

wherein m's are each an integer of 1 to 4 and A's which may be the same or different are each one of the substituents described in the above items (1) to (8) of the definition of R1 or a hydrogen atom, preferably a hydrogen atom (i.e. unsubstituted), a lower alkyl group, or a lower alkoxy group, and most preferably the indanone group is unsubstituted or substituted with 1 to 3 methoxy groups. Examples of the monovalent group derived from a cyclic amide compound

include quinazolone, tetrahydroisoquinolinone, tetrahydrobenzodiazepinone, and hexahydrobenzazocinone. However, the monovalent group may be any one having a cyclic amide group in the structural formula thereof and is not limited to the above-described specific examples only. The cyclic amide group may be one derived from a monocyclic or condensed heterocyclic ring. The condensed heterocylcic ring is preferably one formed by condensation with a phenyl ring. In this case, the phenyl ring may be substituted with a lower alkyl group having 1 to 6 carbon atoms, preferably a methyl group, or a lower alkoxy group having 1 to 6 carbon atoms, preferably a methoxy group.

Preferable examples of the monovalent group include the following groups:

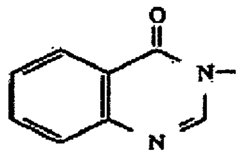

(a)

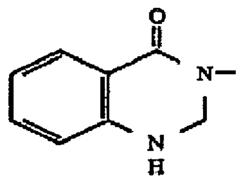

(b)

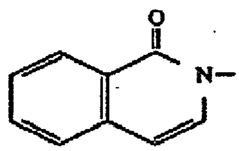

(c)

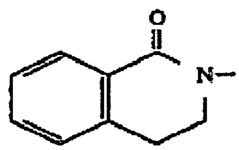

(d)

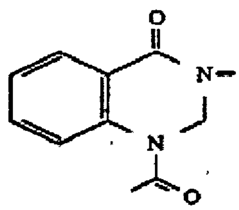

(e)

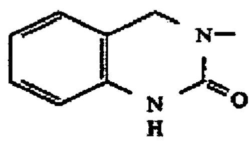

(f)

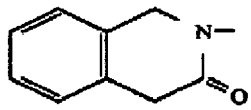

(g)

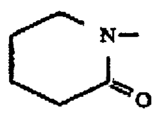

(h)

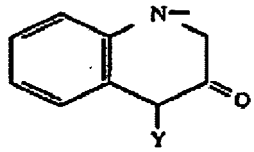

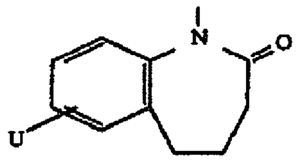

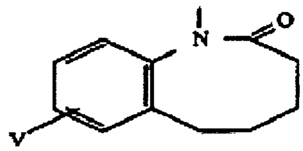

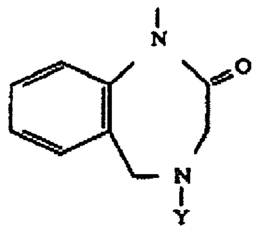

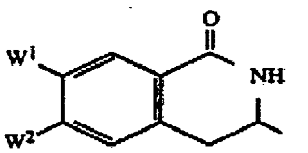

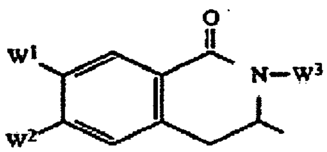

In the above formulae, Y's in the formulae (i) and (1) are each a hydrogen atom or a lower alkyl group, V in the formula (k) is a hydrogen atom or a lower alkoxy group, W1 and W2 in the formulae (m) and (n) are each a hydrogen atom, a lower alkyl group, or a lower alkoxy group and W3 is a hydrogen atom or a lower alkyl group.

The right-hand ring in each of the formulae (j) and (1) is a seven-membered ring, while the right-hand ring in the formula (k) is an eight-membered ring.

Certain examples of the above-defined R1 include a monovalent group derived from an indanone having an unsubstituted or substituted phenyl group and a monovalent group derived from a cyclic amide compound.

Certain examples of the above-defined X include a group represented by the formula --(CH2)n --, a group having an amide group, and groups represented by the above formulae wherein n is 2. Therefore, it is most preferable that any portion of a group represented by the formula

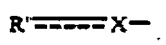

have a carbonyl or amide group.

The substituents involved in the expressions "a substituted or unsubstituted phenyl group" and "a substituted or unsubstituted arylalkyl group" in the above definition of R2 are the same as those described in the above items (1)

to (8) in the above definition of R1.

The term "arylalkyl group" is intended to mean an unsubstituted benzyl or phenethyl group, etc.

Specific examples of the pyridylmethyl group include 2-pyridylmethyl, 3-pyridylmethyl, and 4-pyridylmethyl groups.

Preferable examples of R2 include benzyl and phenethyl groups. The symbol



means either a single or a double bond. This bond is a double bond only when R1 is the above-described divalent group (III) derived from an indanone having an unsubstituted or substituted phenyl ring, while it is a single bond in other cases.

The compounds of the present disclosure may have an asymmetric carbon atom depending upon the kind of the substituent and, therefore, have stereoisomers.

They are, of course, within the scope of the present disclosure.

One specific example thereof will now be described. When R1 has an indanone skeleton, the compound of the present disclosure has an asymmetric carbon atom and, therefore, may have stereoisomers, optical isomers, diastereomers, etc. All of these isomers are within the scope of the present disclosure.

**Method of Treating CNS Disorders with Cognitive Impairment with the Administration of an SV2A Inhibitor and an AChEI or Pharmaceutically Acceptable Salts Thereof**

In one aspect, the disclosure provides methods and compositions for treating, or improving cognitive function in, a subject suffering from a central nervous system (CNS) disorder with cognitive impairment (e.g., age-related cognitive impairment, MCI, amnestic MCI, dementia, AD, prodromal AD, PTSD, schizophrenia, ALS, and cancer therapy-related cognitive impairment), or the risk thereof in a subject in need thereof by administering an SV2A inhibitor or a pharmaceutically acceptable salt, hydrate, solvate or polymorph thereof in combination with an AChEI or a pharmaceutically acceptable salt, hydrate, solvate or polymorph thereof. In some instances, the SV2A inhibitor is selected from the group consisting of levetiracetam, seletracetam, and brivaracetam or derivatives or analogs or pharmaceutically acceptable salts, or solvates, or hydrates, or polymorphs, or prodrugs thereof. In other instances, the SV2A inhibitor is levetiracetam or a derivative or an analog or a pharmaceutically acceptable salt, or a solvate, or a hydrate, or a polymorph, or a prodrug thereof. In some instances, the AChEI is donepezil, tacrine, rivatigmine, physostigmine, galantamine, or metrifonate or derivatives or analogs or pharmaceutically acceptable salts, or solvates, or hydrates, or polymorphs, or prodrugs thereof. In other instances, the AChEI is donepezil or a derivative or an analog or a pharmaceutically acceptable salt or a solvate, or a hydrate, or a polymorph, or a prodrug thereof. In some instances, the CNS disorder with cognitive impairment is age-related cognitive impairment, such as Mild Cognitive Impairment (MCI), Age-Associated Memory Impairment (AAMI), Age Related Cognitive Decline (ARCD). In one instance of the disclosure, the MCI is amnestic MCI. In some instances of the disclosure, the CNS disorder with cognitive impairment is dementia, post traumatic stress disorder (PTSD), schizophrenia, amyotrophic lateral sclerosis (ALS), or cancer-therapy-related cognitive impairment. In one instance, the subject that suffers such cognitive impairment is a human patient. The subject may be a human or other mammal such as a non-human primate, or rodent (e.g., rat). In some instances, the subject is a human patient.

When used clinically, donepezil shows a "cholinergic" side effect profile, and the dosage administered to patients is limited by such side effects. The use of the SV2A inhibitors and pharmaceutically acceptable salts, hydrates, solvates and polymorphs thereof in combination with donepezil or other AChEIs and their pharmaceutically acceptable salts, hydrates, solvates and polymorphs reduces the amount of donepezil or other AChEIs necessary for the treatment of CNS disorders involving cognitive dysfunction and other affective disorders, including MCI, amnestic MCI, AAMI, ARCE, dementia, AD, PTSD, schizophrenia, amyotrophic lateral sclerosis (ALS), or cancer-therapy-related cognitive impairment. In one instance, the subject that suffers such cognitive impairment is a human patient, and thus reduce the side effects caused by donepezil or other AChEIs without diminishing efficacy. Further, the efficacy of a combination of the SV2A inhibitor and donepezil or other AChEIs and pharmaceutically acceptable salts, solvates, hydrates, and polymorphs thereof exceeds the efficacy of either drug administered alone at its optimal dose and thus is an improved treatment for CNS disorders with cognitive impairment.

It will be appreciated that compounds and agents used in the compositions and methods of this disclosure preferably should readily penetrate the blood-brain barrier when peripherally administered. Compounds which cannot penetrate the blood-brain barrier, however, can still be effectively administered directly into the central nervous system, e.g., by an intraventricular or other neuro-compatible route.

As used herein, administration of an SV2A inhibitor and an AChEI or pharmaceutically acceptable salts, hydrates, solvates and polymorphs thereof "in combination" includes simultaneous administration and/or administration at

different times, such as sequential administration. Simultaneous administration of the SV2A inhibitor and the AChEI or their pharmaceutically acceptable salts, hydrates, solvates and polymorphs can optionally be combined with supplemental doses of the SV2A inhibitor and/or the AChEI and their salts, hydrates, solvates and polymorphs. Simultaneous administration of drugs encompasses administration as co-formulation or, alternatively, as separate compositions.

In accordance with this disclosure, the SV2A inhibitor and the AChEI, and pharmaceutically acceptable salts, solvates, hydrates, polymorphs thereof, can be administered to a subject via any suitable route or routes. In some instances, the drugs are administered orally; however, administration intravenously, subcutaneously, intra-arterially, intramuscularly, intraspinally, rectally, intrathoracically, intraperitoneally, intracentricularly, or transdermally, topically, or by inhalation is also contemplated. The agents can be administered orally, for example, in the form of tablets, troches, capsules, elixirs, suspensions, syrups, wafers, or the like, prepared by art recognized procedures. In certain instances, the SV2A inhibitor and the AChEI, and pharmaceutically acceptable salts, solvates, hydrates, polymorphs thereof, can be administered to a subject via different routes. For example, the SV2A inhibitor or its salt, solvate, hydrate, or polymorph is administered intravenously and the AChEI or its salt, solvate, hydrate, or polymorph is administered orally.

In some instances, the administration is a slow or extended release. The term "extended release" is widely recognized in the art of pharmaceutical sciences and is used herein to refer to a controlled release of an active compound or agent from a dosage form to an environment over (throughout or during) an extended period of time, e.g. greater than or equal to one hour. An extended release dosage form will release drug at substantially constant rate over an extended period of time or a substantially constant amount of drug will be released incrementally over an extended period of time. The term "extended release" used herein includes the terms "controlled release," "prolonged release," "sustained release," "delayed release," or "slow release" as these terms are used in the pharmaceutical sciences. In some instances, the extended release dosage is administered in the form of a patch or a pump.

When a solid carrier is used for administration, the preparation may be in a tablet, placed in a hard gelatin capsule in powder or pellet form, or it may be in the form of a troche or lozenge. If a liquid carrier is used, the preparation may be in the forms of a syrup, emulsion, soft gelatin capsule, or sterile injectable liquid such as an aqueous or non-aqueous liquid suspension or solution.

Dosage schedules of the agents and compositions according to the methods of the disclosure will vary according to the particular compound or compositions selected, the route of administration, the nature of the condition being treated, the age, and condition of the patient, the course, or stage of treatment, and will ultimately be at the discretion of the attending physician. It will be understood that the amount of the SV2A inhibitor and the AChEI and their pharmaceutically acceptable salts, hydrates, solvates and polymorphs thereof administered will be amounts effective to produce a desired biological effect, such as beneficial results, including clinical results (e.g., an amount that increases GABAergic activity, reduces excitatory neurotransmission, and blocks, suppresses, or reduces acetylcholinesterase activity, and/or amounts that in combination result in an improvement in cognitive function). It will be understood that an effective amount can be administered in more than one dose and over a course of treatment.

Desired duration of administration of the SV2A inhibitor and the AChEI and their pharmaceutically acceptable salts, hydrates, solvates and polymorphs thereof can be determined by routine experimentation by one skilled in the art. For example, the SV2A inhibitor and the AChEI and their pharmaceutically acceptable salts, hydrates, solvates and polymorphs thereof may be administered for a period of 1-4 weeks, 1-3 months, 3-6 months, 6-12 months, 1-2 years, or more, up to the lifetime of the patient.

It is known in the art that normalization to body surface area is an appropriate method for extrapolating doses between species. The human equivalent dose (HED) for this dosage can be estimated using the following formula that accounts for differences in body surface area (see Estimating the Safe Starting Dose in Clinical Trials for Therapeutics in Adult Healthy Volunteers, December 2002, Center for Biologics Evaluation and Research):

$$\text{HED} = \text{animal dose X (Km animal / Km human)}$$

where the Km factor is body weight divided by body surface area (Km rat has been determined as 6, and Km human is 37; see Reagan-Saw, Nihal, Ahmad, 2007). Thus, a dosage of 10 mg/kg in rats is equivalent to 1.6 mg/kg in humans (10 mg/kg X (6 / 37) = 1.6 mg/kg). For human subjects, to calculate a dose in mg from the dose in mg/kg, the dose in mg/kg is multiplied bya typical adult weight of 70 kg.

In certain instances of the disclosure, the SV2A inhibitor or a pharmaceutically acceptable salt, hydrate, solvate and polymorph thereof can be administered at doses according to, for example, U.S. Patent Application 12/580,464, International Patent Application PCT/US2009/005647, U.S. Patent Application 61/105,847, U.S. Patent Application 61/152,631, and U.S. Patent Application 61/175,536. In certain instances of the disclosure, the SV2A inhibitor or a pharmaceutically acceptable salt, hydrate, solvate and polymorph thereof is administered every 12 or 24 hours at a daily dose of about 0.001 mg/kg to 5 mg/kg. In some instances, the SV2A inhibitor or a pharmaceutically acceptable salt,

hydrate, solvate and polymorph thereof is administered every 12 or 24 hours at a daily dose of about 0.1 to 5 mg/kg, or about 1 to 2 mg/kg, or about 0.1 to 0.2 mg/kg, or about 0.01 to 2.5 mg/kg, or about 0.1 to 2.5 mg/kg, or about 0.4 to 2.5 mg/kg, or about 0.6 to 1.8 mg/kg, or about 0.04 to 2.5 mg/kg, or about 0.06 to 1.8 mg/kg, or about 0.01 to 1 mg/kg, or about 0.001 to 1 mg/kg, or about 0.5 to 5 mg/kg, or about 0.05 to 0.5 mg/kg. For repeated administrations over several days or weeks or longer, depending on the condition, the treatment is sustained until a sufficient level of cognitive function is achieved.

The SV2A inhibitor or its pharmaceutically acceptable salt, hydrate, solvate and polymorph may be administered at a subtherapeutic dosage levels when provided in combination with an AChEI or its pharmaceutically acceptable salt, hydrate, solvate and polymorph, due to an AChEI-dependent increase in the therapeutic index of the SV2A inhibitor. In some instances, the increase in the therapeutic index of the SV2A inhibitor, due to the combination with an AChEI, is greater than the therapeutic index of the SV2A inhibitor administered in the absence of the AChEI by at least about 1.5x or 2.0x or 2.5x or 3.0x or 3.5x or 4.0x or 4.5x or 5.0x or 5.5x or 6.0x or 6.5x or 7.0x or 7.5x or 8.0x or 8.5x or 9.0x or 9.5x or 10x, or greater than about 10x. In some instances, combinations of an SV2A inhibitor with an AChEI reduces the dosage of the SV2A inhibitor required for its therapeutic effect. In some instances, the amount of the SV2A inhibitor administered in combination with the AChEI is a subtherapeutic amount. In some instances, the SV2A inhibitor or a pharmaceutically acceptable salt, hydrate, solvate and polymorph thereof is administered every 12 or 24 hours at a daily dose of less than 5 mg/kg, less than 2.5 mg/kg, less than 2 mg/kg, less than 1.5 mg/kg, less than 1 mg/kg, less than 0.5 mg/kg, less than 0.1 mg/kg, less than 0.05 mg/kg, less than 0.01 mg/kg, less than 0.005 mg/kg, or less than 0.001 mg/kg.

The AChEI or its pharmaceutically acceptable salt, hydrate, solvate and polymorph may be administered at a dosage level up to conventional dosage levels. Suitable dosage levels will depend upon the specific AChEI that is chosen. The AChEI may be administered on a regimen of up to 2 times per day, 1 time per day, or it may be administered less often. For ARICEPT®, a typical daily dosage when administered alone is about 5 to 10 mg. In certain instances of the disclosure, the amount of ARICEPT® administered in combination with an SV2A inhibitor is about 0.1 to 10 mg. In some instances, the amount of ARICEPT® administered in combination with the SV2A inhibitor is a subtherapeutic amount. In some instances, the amount of ARICEPT® administered in combination with the SV2A inhibitor is less than 10 mg daily, less than 5 mg daily, less than 1 mg daily, less than 0.5 mg daily, or less than 0.1 mg daily.

The AChEI or a salt, hydrate, solvate or polymorph thereof may be administered at dosage levels distinct from conventional levels when provided in combination with an SV2A inhibitor, due to an SV2A inhibitor-dependent increase in the AChEI's therapeutic index. In some instances, the increase in the AChEI's therapeutic index due to the combination with an SV2A inhibitor thereof is greater than the therapeutic index of the AChEI administered in the absence of an SV2A inhibitor by at least about 1.5x or 2.0x or 2.5x or 3.0x or 3.5x or 4.0x or 4.5x or 5.0x or 5.5x or 6.0x or 6.5x or 7.0x or 7.5x or 8.0x or 8.5x or 9.0x or 9.5x or 10x, or greater than about 10x. In some instances, combinations of an AChEI with the SV2A inhibitor reduces the dosage of the AChEI required for its therapeutic effect. In some instances, the amount of the AChEI administered in combination with the SV2A inhibitor thereof is about 0.1 to 10 mg. In some instances, the amount of the AChEI administered in combination with the SV2A inhibitor is a subtherapeutic amount. In some instances, the amount of the AChEI administered in combination with the SV2A inhibitor is less than 10 mg daily, less than 5 mg daily, less than 1 mg daily, less than 0.5 mg daily, or less that 0.1 mg daily.

The frequency of administration of the composition of this disclosure may be adjusted over the course of the treatment, based on the judgment of the administering physician. It will be clear that the SV2A inhibitor and the AChEI and their salts, hydrates, solvates and polymorphs can be administered at different dosing frequencies or intervals. For example, an SV2A inhibitor can be administered daily (including multiple doses per day) or less frequently. An AChEI can be administered daily (including multiple doses per day) or less frequently. In some instances, sustained continuous release formulations of an SV2A inhibitor and an AChEI may be desired. Various formulations and devices for achieving sustained release are known in the art.

As described above, some AChEIs (such as donepezil) and their salts, hydrates, solvates and polymorphs can cause cholinergic side effects. The use of a combination of an SV2A inhibitor and an AChEI may reduce the amount of the AChEI necessary for treatment of CNS disorders with cognitive impairment, and may thus reduce the side effects caused by the AChEIs. In particular, the combination of an SV2A inhibitor with a reduced amount of AChEI may reduce the cholinergic side effects without negatively impacting efficacy. Accordingly, in some instances, a subtherapeutic amount of AChEI is administered.

In some instances, a suitable amount of the SV2A inhibitor is administered so as to reduce the dose of the AChEI (e.g., a dose required to effect a degree of cognitive function improvement or treat age-associated cognitive impairment) by at least about 20%, at least about 30%, at least about 40%, or at least about 50%, at least about 60%, at least about 70%, at least about 80%, at least about 90% or more from to the dose of AChEI normally used when administered alone (i.e., individually and not in combination with other therapeutic agents or compounds). The reduction may be reflected in terms of amount administered at a given administration and/or amount administered over a given period of time (reduced frequency).

In certain instances of the disclosure, the combined administration of an SV2A inhibitor or a salt, hydrate,

solvate and polymorph thereof and an AChEI or a salt, hydrate, solvate and polymorph thereof can attain a longer or improved therapeutic effect in the subject than that attained by administering only the AChEI or only the SV2A inhibitor, by at least about 1.5x, or 2.0x, or 2.5x, or 3.0x, or 3.5x, or 4.0x, or 4.5x, or 5.0x, or 5.5x, or 6.0x, or 6.5x, or 7.0x, or 7.5x, or 8.0x, or 8.5x, or 9.0x, or 9.5x, or 10x, or greater than about 10x.

**Compositions of this Disclosure**

In one aspect, the disclosure provides compositions comprising an SV2A inhibitor and an AChEI and their salts, hydrates, solvates and polymorphs. In some instances, the SV2A inhibitor and the AChEI may be present in a single dosage unit (e.g., combined together in one capsule, tablet, powder, or liquid, etc.). In some instances, the AChEI in the composition is donepezil. In some instances, the composition includes levetiracetam, or seletracetam, or brivaracetam or a derivative or an analog or a pharmaceutically acceptable salt, hydrate, solvate, or polymorph thereof as the SV2A inhibitor, and includes donepezil or a derivative or an analog or a pharmaceutically acceptable salt, hydrate, solvate, or polymorph thereof as the AChE1. In some instances, the composition includes levetiracetam or a derivative or an analog or a pharmaceutically acceptable salt, hydrate, solvate and polymorph thereof and donepezil or a pharmaceutically acceptable salt, hydrate, solvate and polymorph thereof. The composition described herein can contain more than one SV2A inhibitor and/or more than one AChEI. In some instances, the SV2A inhibitor and the AChEI are in separate formulations packaged together.

The compositions described herein can further contain pharmaceutically acceptable excipient(s) and may contain other agents that serve to enhance and/or complement the effectiveness of the SV2A inhibitor and/or the AChEI. The compositions may also contain additional agents known to be useful for treating cognitive function disorder.

The composition in the present disclosure may be in solid dosage forms such as capsules, tablets, dragrees, pills, lozenges, powders and granule. Where appropriate, they may be prepared with coatings such as enteric coatings or they may be formulated so as to provide controlled releases of one or more active ingredient such as sustained or prolonged release according to methods well known in the art. In certain instances, the composition is in form of a slow, controlled, or extended release. The term "extended release" is widely recognized in the art of pharmaceutical sciences and is used herein to refer to a controlled release of an active compound or agent from a dosage form to an environment over (throughout or during) an extended period of time, e.g. greater than or equal to one hour. An extended release dosage form will release drug at substantially constant rate over an extended period of time or a substantially constant amount of drug will be released incrementally over an extended period of time. The term "extended release" used herein includes the terms "controlled release", "prolonged release", "sustained release", or "slow release", as these terms are used in the pharmaceutical sciences. In some instances, the extended release dosage is administered in the form of a patch or a pump. The composition may also be in liquid dosage forms including solutions, emulsions, suspensions, syrups, and elixirs.

The compositions may be specifically formulated for administration by any suitable route as described herein and known in the art. Compositions for parental administration include sterile aqueous and nonaqueous injectable solutions, dispersions, suspensions or emulsions as well as sterile powders to be reconstituted in sterile injectable solutions or dispersions prior to use. Compositions for intraoral and oral delivery (including sublingual and buccal administration, e.g. Danckwerts et al, and oral) include but are not limited to bioadhesive polymers, tablets, patches, liquids and semisolids (see e.g., Smart et al). Compositions for respiratory delivery (pulmonary and nasal delivery) include but are not limited to a variety of pressurized metered dose inhalers, dry powder inhalers, nebulizers, aqueous mist inhalers, drops, solutions, suspensions, sprays, powders, gels, ointments, and specialized systems such as liposomes and microspheres (see e.g. Owens et al, "Alternative Routes of Insulin Delivery" and Martini et al). Compositions for transdermal delivery include but are not limited to colloids, patches, and microemulsions. Other suitable administration forms for the above and other include depot injectable formulations, suppositories, sprays, ointments, cremes, gels, inhalants, dermal patches, implants etc.

The compositions may also contain adjuvants, such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption, such as aluminum monostearate and gelatin.

Therapeutic formulations can be prepared by methods well known in the art of pharmacy, see, e.g., Goodman et al., 2001; Ansel, et al., 2004; Stoklosa et al., 2001; and Bustamante, et al., 1993.

In certain instances of the disclosure, a composition containing an SV2A inhibitor and an AChEI and their salts, hydrates, solvates and polymorphs comprises an amount of the SV2A inhibitor between 0.07 and 350 mg, or between 50 and 200 mg, or between 3 and 50 mg. In some instances, the amount of the SV2A inhibitor is less than 350 mg, less than 250 mg, less than 200 mg, less than 150 mg, less than 100 mg, less than 50 mg, less than 10 mg, less than 5 mg,

less than 1 mg, less than 0.5 mg, less than 0.1 mg, or less than 0.07 mg. In certain instances, the amount of the AChEI in the composition is about 0.1 to 10 mg. In some instances, the amount of the AChEI in the composition is less than 10 mg, less than 5 mg, less than 2 mg, less than 1 mg, less than 0.5 mg, or less than 0.1 mg.

It will be understood by one of ordinary skill in the art that the compositions and methods described herein may be adapted and modified as is appropriate for the application being addressed and that the compositions and methods described herein may be employed in other suitable applications, and that such other additions and modifications will not depart from the scope hereof.

This disclosure will be better understood from the Experimental Details which follow. However, one skilled in the art will readily appreciate that the specific methods and results discussed are merely illustrative of the disclosure as described more fully in the instances which follow thereafter.

## Examples

### Introduction and Models of Cognitive Impairment

A variety of conditions characterized by cognitive impairment, e.g., Age-Associated Memory Impairment (AAMI), Mild Cognitive Impairment (MCI) and Age-related Cognitive Decline (ARCD) are believed to be related to aging. Others are related to disease, for example, AD. Animal models serve as an important resource for developing and evaluating treatments for such age-related cognitive impairments. Features that characterize age-related cognitive impairment in animal models typically extend to age-related cognitive impairment in humans. Efficacy in such animal models is, thus, predictive of efficacy in humans.

Of available models, a Long-Evans rat model of cognitive impairment is particularly well suited for distinguishing the difference between cognitive impairment related to illness and that related to aging. Indeed, extensive behavioral characterization has identified a naturally occurring form of cognitive impairment in an outbred strain of aged Long-Evans rats (Charles River Laboratories; Gallagher et al., Behav. Neurosci. 107:618-626, (1993)). In a behavioral assessment with the Morris Water Maze (MWM), rats learn and remember the location of an escape platform guided by a configuration of spatial cues surrounding the maze. The cognitive basis of performance is tested in probe trials using measures of the animal's spatial bias in searching for the location of the escape platform. Aged rats in the study population have no difficulty swimming to a visible platform, but an age-dependent impairment is detected when the platform is camouflaged, requiring the use of spatial information. Performance for individual aged rats in the outbred Long-Evans strain varies greatly. For example, a proportion of those rats perform on a par with young adults. However, approximately 40-50% falls outside the range of young performance. This variability among aged rats reflects reliable individual differences. Thus, within the aged population some animals are cognitively impaired and designated aged-impaired (AI) and other animals are not impaired and are designated aged-unimpaired (AU). *See,* e.g., Colombo et al., Proc. Natl. Acad. Sci. 94: 14195-14199, (1997); Gallagher and Burwell, Neurobiol. Aging 10: 691-708, (1989); Rapp and Gallagher, Proc. Natl. Acad. Sci. 93: 9926-9930, (1996); Nicolle et al., Neuroscience 74: 741-756, (1996); and Nicolle et al., J. Neurosci. 19: 9604-9610, (1999).

We used the above-described rat model to identify individual AI and AU rats. We then conducted behavioral assessment on AI rats while administering various pharmacological treatments.

### Example 1: Behavioral Assessment of Levetiracetam, Donepezil, and Combined Treatments in a Radial Arm Maze Task

Aged, male Long-Evans rats were obtained at 8 to 9 months of age from Charles River Laboratories (Raleigh, NC) and housed in a vivarium until 24 to 26 months of age. All rats were individually housed at 25°C and maintained on a 12 h light/dark cycle. Food and water were provided *ad libitum* unless noted otherwise. The rats were examined for health and pathogen-free status throughout the experiments, as well as necropsies at the time of killing.

All rats were screened in a standardized assessment of spatial cognition. This background assessment used the above-described well-established MWM protocol. Aged rats that demonstrated impaired memory performance in a standardized assessment of spatial cognition, i.e., AI rats, were selected for the drug intervention studies. The selected AI rats were tested for their memory of new spatial information in a Radial Arm Maze (RAM) task, under different drug/control treatment conditions: vehicle control, donepezil only, levetiracetam only, and a combination of donepezil and levetiracetam. The RAM task also extended the behavioral assessment to spatial memory using appetitive motivation. We used a protocol that allowed within-subject assessment across drugs at different doses and in combinations (Koh et al., Neuropsychopharmacology, epub. 1-10, (2009)).

**Radial Arm Maze Task**

The radial maze consisted of eight arms projecting from each side of an octagonal center platform, with food well located at the distal end of each arm. Plexiglas™ blocks could be positioned to prevent entry into any arm. Extra-maze cues were provided in the room surrounding the maze and illumination was provided by an overhead light. Pre-training, as described in detail in Chappell et al. Neuropharmacology 37: 481-487 (1998), consisted of habituation, standard win-shift training, and win-shift training with delays interposed between information and memory test phases. Drug treatments began 2 days after the completion of pre-training. Three arms were blocked at the beginning of each trial (information phase). The identity and configuration of the blocked arms varied across trials. Food-deprived rats were allowed to retrieve food reward (Kellogg's Froot Loops™ cereal) from the five unblocked arms. The rat was then removed from the maze for 60 min (retention interval), during which time the barriers on the blocked arms were removed allowing access to all eight arms. Rats were then placed back onto the center platform and allowed to retrieve the remaining food rewards (memory test phase).

The number of "errors" the AI rats made during the retention test phase was tracked. An error consisted of returning to an arm (all four paws on the arm) from which food had already been obtained. For example, an error occurs in the trials if the rats enter an arm from which food has already been retrieved in the pre-delay component of the trial or if it re-visits an arm in the post-delay session that has already been visited.

**Levetiracetam Treatment**

An independent set of AI rats (n=10) are first tested with levetiracetam (Tecoland, Edison, NJ). The AI rats are pretreated 30-40 minutes before daily trials with a one-time shot of the following six conditions: 1) vehicle control (0.9% saline solution); 2) levetiracetam (1.25 mg/kg/day); 3) levetiracetam (2.5 mg/kg/day); 4) levetiracetam (5 mg/kg/day); 5) levetiracetam (10 mg/kg/day); 6) levetiracetam (20 mg/kg/day); through intraperitoneal (i.p.) injection. Injections are given every other day with intervening washout days. To counterbalance any potential bias, drug effect is assessed using ascending-descending dose series, i.e., the dose series are given first in an ascending order and then repeated in a descending order. Therefore, each dose has two determinations.

Parametric statistics (paired t-tests) are used to compare the retention test performance of the AI rats in the one-hour delay version of the RAM task in the context of different doses of levetiracetam and vehicle control (*see* **FIG. 1).** The average numbers of errors in the trials are significantly fewer with levetiracetam treatment of 5 mg/kg/day (average no. of errors $\pm$ standard error of the mean (SEM) = 0.75 $\pm$ 0.32) and 10 mg/kg/day (average no. of errors $\pm$ SEM = 0.80 $\pm$ 0.27) than using vehicle control (average no. of errors $\pm$ SEM = 2.00 $\pm$ 0.42). Relative to vehicle control treatment, levetiracetam significantly improves memory performance at 5 mg/kg/day ($t(9) = 2.18$, $p = 0.057$) and 10 mg/kg/day ($t(9) = 2.37$, $p = 0.042$).

**Donepezil Treatment**

After the completion of levetiracetam assessment, a new set of AI rats (n=8) are tested with donepezil. The AI rats are pretreated 30-40 minutes before daily trials with a one-time shot of the following three conditions: 1) vehicle control (0.9% saline solution); 2) donepezil (1 mg/kg/day); 3) donepezil (2 mg/kg/day); through intraperitoneal (i.p.) injection. Injections are given every day. To counterbalance any potential bias, drug effect is assessed using ascending-descending dose series, i.e., the dose series are given first in an ascending order and then repeated in a descending order. Therefore, each dose has two determinations.

Parametric statistics (paired t-tests) are used to compare the retention test performance of the AI rats in a two-hour delay version of the RAM task in the context of different doses of donepezil and vehicle control (*see* **FIG. 2**). The average numbers of errors that occur in the trials of using donepezil treatment of 1 mg/kg/day (average no. of errors $\pm$ standard error of the mean (SEM) = 2.88 $\pm$ 0.46) and 2 mg/kg/day (average no. of errors $\pm$ SEM = 2.81 $\pm$ 0.43) are not significantly different from using vehicle control (average no. of errors $\pm$ SEM = 2.88 $\pm$ 0.67). Relative to vehicle control treatment, donepezil does not significantly improve memory performance at 1 mg/kg/day ($t(7) = 0$, $p = 1.00$) or 2 mg/kg/day ($t(7) = 0.91$ , $p = 0.930$).

**Combination of Levetiracetam and Donepezil**

We use the same AI rats (n=8) to test the memory improvement effects of the combination of levetiracetam and donepezil, when administered at subtherapeutic doses. In this experiment, the subtherapeutic doses are 2.5 mg/kg/day for levetiracetam and 1 mg/kg/day for donepezil. As described above, when levetiracetam is administered individually at a dose of 2.5 mg/kg/day in the absence of donepezil, it does not significantly improve memory performance in AI rats (*See* **FIG. 1**). When donepezil is administered individually at a dose of 1 mg/kg/day in the absence of levetiracetam, it does not significantly improve memory performance in AI rats (*See* **FIG. 2**).

The AI rats are pretreated 30-40 minutes before daily trials with a one-time shot of the following two conditions: 1) vehicle control (0.9% saline solution); 2) donepezil (1 mg/kg/day) and levetiracetam (2.5 mg/kg/day); through intraperitoneal (i.p.) injection. Injections are given every day. To counterbalance any potential bias, drug effect is assessed using ascending-descending dose series, i.e., the dose series are given first in an ascending order and then repeated in a descending order. Therefore, each dose has two determinations.

Parametric statistics (paired t-tests) are used to compare the retention test performance of the AI rats in a two-hour delay version of the RAM task in the context of a combination of levetiracetam and donepezil and vehicle control (*see* **FIG. 2**). The average numbers of errors that occur in the trials are significantly fewer with the combined administration of donepezil (1 mg/kg/day) and levetiracetam (2.5 mg/kg/day) (average no. of errors $\pm$ standard error of the mean (SEM) = 1.69 $\pm$ 0.53) than using vehicle control (average no. of errors $\pm$ SEM = 2.88 $\pm$ 0.67). Relative to vehicle control treatment, a combination of donepezil (1 mg/kg/day) and levetiracetam (2.5 mg/kg/day) significantly improves memory performance ($t(7) = 2.16$, $p = 0.034$).

Either donepezil or levetiracetam, when administered at the subtherapeutic doses as are tested in this experiment in the absence of the other drug, does not significantly improve the memory performance in AI rats (*See* **FIGS 1 and 2**). However, a combination of donepezil and levetiracetam administered at subtherapeutic doses significantly improves the memory performance in AI rats. Our results suggest super-additive or synergistic interaction between donepezil and levetiracetam when administered together.

## Example 2: Levetiracetam Treatments in Human

A within-subjects trial of 8 weeks duration, involving 17 amnestic MCI (aMCI) subjects and 17 age-matched controls with a low dose treatment of levetiracetam is conducted. During the course of the study, each aMCI subject receives both drug and placebo treatments separately in two periods of two weeks each, with the order of treatments among different aMCI subjects counterbalanced (see **FIG. 3**). Age-matched control subjects treated with placebo serve as a further control. Cognitive testing and fMRI imaging data are obtained from the subjects after each two week period of drug/placebo treatment.

### Participants and clinical characterization

17 right-handed aMCI patients are recruited from the Alzheimer's Disease Research Center (ADRC) at the Johns Hopkins Hospital and other referrals. An additional 17 right-handed healthy volunteers are recruited from the pool of control participants in the ADRC and other referrals. All participants are administered the Telephone Interview of Cognitive Status to determine if they are likely to pass the entry criteria of the study (including criteria for MRI scanning). All participants further undergo neurological, psychiatric, and neuropsychological examination using standardized instruments and methods. The psychiatric evaluation includes administration of the Structured Clinical Interview for DSM-IV Axis I Disorders and the Clinical Dementia Rating (CDR) scale. All aMCI patients have CDR scores of 0.5. Diagnosis of aMCI is based on the criteria proposed by Petersen et al. (e.g., "Mild cognitive impairment: Aging to Alzheimer's Disease," Oxford University Press, N.Y. (2003), which include a memory complaint (corroborated by an informant), impaired memory function on testing (1.5 standard deviations below norm), otherwise preserved cognitive functioning (within 1 standard deviation of norm), no decline in functional ability, and no dementia. Final aMCI diagnoses are reached by clinical consensus. Exclusion criteria include major neurological or psychiatric disorders, head trauma with loss of consciousness, history of drug abuse or dependency, and general contraindications to an MRI examination (e.g. cardiac pacemaker, aneurysm coils, claustrophobia). Each aMCI subject is required to have a study partner (i.e., an informant) who can provide information about the subject's daily function and assure that medications are taken appropriately. See **FIGS. 14A** and **14B.**

**Study Visits:** The study consists of 4 visits over the course of 8 weeks (see **FIG. 3**). The Baseline Visit is for the purpose of performing medical, neurological, psychiatric, and neurocognitive assessments. Visits 1 and 2 are identical to the Baseline Visit but include a fMRI session. The Washout Visit, at the end of a 4 week washout period, is for the purpose of a brief clinical assessment and initiation of the second drug/placebo phase.

**Baseline Visit:** At the screening visit, informed consent is obtained from the subject (and an informant in the case of MCI subjects). The subject and the informant participate in a standardized clinical interview that is used to determine the degree of the subject's functional impairment in daily life, based on the Clinical Dementia Rating (CDR) scale. The subject's medical, neurological, and psychiatric history is obtained (including a review of current medications), as well as the family history of dementia. Brief medical, neurological and psychiatric exams are conducted (including vital signs). Blood is drawn in order to perform standard laboratory tests needed to determine if the subject meets the entry criteria. The subject is re-screened for contraindications to MRI scanning, using the standard form employed at the Kirby Imaging Center. Brief cognitive testing is performed (described in section on neuropsychological assessment

below). These assessments are used to determine if the subject meets the entry criteria. All of the foregoing are completed using standardized forms. If the subject meets entry criteria for the study, the subject is given the study medication (drug or placebo, randomly selected), and instructions about how it should be taken. The subject is advised about the potential for having suicidal thoughts and advised to stop taking the medication and immediately contact the study physician if this occurs.

**Visit 1:** At the end of the first drug/placebo period 2 weeks after the Baseline Visit, the medical, neurological and psychiatric evaluations and cognitive testing are repeated. The subject is also clinically evaluated for suicidal ideation. Blood is drawn again to repeat the standard tests and to determine whether there are any changes related to drug treatment; the subject's blood levetiracetam level is also obtained. All medication dispensed at the Baseline Visit (drug or placebo) is collected and subject compliance with the medication regimen is assessed. The first fMRI session (with cognitive tests) is conducted on the same day, either immediately before or immediately after the clinical assessment. Subjects discontinue first period treatment at this visit.

**Washout Visit:** At the end of a washout period (4 weeks) following Visit 1, the subject receives a brief medical screening, including a medical and psychiatric evaluation. Blood is drawn to obtain the blood levetiracetam level (to confirm washout). The subject is provided with new medication (drug or placebo, alternated from what was assigned in the previous treatment period) for the final phase of the study with instructions about how it should be taken.

**Visit 2:** At approximately 2 weeks after the Washout Visit (i.e., 2 weeks after starting the second treatment period), the medical, neurological and psychiatric evaluations and the cognitive testing are repeated. The subject is clinically evaluated for suicidal ideation. Blood is drawn again to repeat the standard tests and to determine whether there were any changes related to drug treatment; the subject's blood levetiracetam level is also obtained. All medication dispensed at the Washout Visit is collected and subject compliance with the medication regimen is assessed. The second fMRI session (with cognitive tests) is repeated on the same day, either immediately before or immediately after the clinical assessment.

### Neuropsychological assessment

All participants undergo neuropsychological evaluation at the time of assessment for treatment efficacy (Visits 1 and 2), as well as at the Baseline Visit. The evaluation occurs outside of the scanner and includes the Buschke Selective Reminding Test (Buschke and Fuld, 1974) and the Verbal Paired Associates subtest, the Logical Memory subtest, the Visual Reproduction subtest of the Wechsler Memory Scale-Revised (WMS-R) (Wechsler, 1997), and the Benton Visual Retention Test, as these tasks are particularly sensitive to medial temporal lobe function and early memory problems (Marquis et al., 2002 and Masur et al., 1994). Additionally, subjects are asked to complete tests of more general cognitive function such as tests to assess general mental status, executive function, attention and general naming ability. All neuropsychological tests are administered by a trained research assistant during a 60-minute session. As the three neuropsychological assessments in this study occur within a time period of 8 weeks, different versions of the neuropsychological tests are used to minimize test specific practice effects. Breaks are provided to the subject as needed.

### Drug administration

As described above, the drug treatment period is the two weeks preceding Visit 1 or 2 (with the two week period preceding the other Visit being the placebo phase). For the subjects receiving the drug treatment, half a scored 250 mg tablet of levetiracetam is used to achieve a dose of 125 mg/kg twice a day, which is approximately 3.6 mg/kg/day (assuming an average adult human weight of 70 kg).

All drug and placebo preparations are performed on a 1:1 allocation. The pharmacy randomize patients as they enroll, and keep a list of drug assignment.

Levetiracetam is rapidly and almost completely absorbed after oral administration, and its bioavailability is not affected by food. Plasma half-life of levetiracetam is approximately 7 $\pm$ 1 hour (expected to be 9-10 hours in elderly due to decreased renal function). Absorption is rapid, with peak plasma concentrations occurring about 1 hour following oral administration. Steady state can be achieved after 2 days of multiple twice-daily dosing.

A typical starting dose of levetiracetam in treating epilepsy in humans is 500 mg twice a day, which is approximately 14.3 mg/kg/day. The dosage is then is increased until optimal efficacy, up to 50 mg/kg/day. Thus, the dose used in this experiment is a quarter of the lowest human dose used for treating epilepsy.

Even lower dosages, e.g., of 25-60 mg twice a day, are contemplated, based on the results of previous animal studies that indicated low-dose efficacy. The highest effective doses of levetiracetam used in the animal model are 5-10 mg/kg (given acutely). The human equivalent dose (HED), calculated as described above, of this dosage for treatment of age-dependent cognitive impairment in humans is equivalent to 0.8-1.6 mg/kg/day (or 28-56 mg twice a day).

**MRI data acquisition**

Imaging data are obtained through high-resolution methods developed in the Stark laboratory. Data are collected on a Phillips 3 Tesla scanner (Eindhoven, The Netherlands) equipped with an 8-channel SENSE (Sensitivity Encoding) head coil, located at the F.M. Kirby Research Center for Functional Brain Imaging at the Kennedy Krieger Institute (Baltimore, MD). High-resolution echo-planar images are collected using an acquisition matrix of 64 x 64, a repetition time of 1500 milliseconds, an echo time of 30 milliseconds, a flip angle of 70 degrees, a SENSE factor of 2, and an isotropic resolution of 1.5 mm x 1.5 mm x 1.5 mm with no gap. Nineteen oblique slices are acquired parallel to the principal longitudinal axis of the hippocampus and covered the entire medial temporal lobe region bilaterally. In addition to the functional runs, a whole-brain MPRAGE structural scan (parameters: 150 oblique slices, 1mm isotropic resolution) is acquired.

Image analysis

Data analysis is carried out using the Analysis for Functional Neuroimages (AFNI, release 2008_07_18_1710) software. Images are first co-registered to correct for within- and across-scan head motion. Acquisitions in which a significant motion event occur (more than 3 degrees of rotation or 2 mm of translation in any direction relative to prior acquisition), plus and minus one time repetition for 1.5 seconds, are excluded from the analyses. Structural anatomical data are registered to standard stereotaxic space (Talairach & Tournoux, 1988), and the same parameters are subsequently applied to the functional data. Behavioral vectors are produced to model different trial types.

The ROI-LDDMM (large deformation diffeomorphic metric mapping of the region of interest) method, a technique for cross-subject alignment, increases the power of multisubject regional fMRI studies by focusing the alignment power specifically on the ROIs (regions of interest) and not elsewhere in the brain. First, all subjects' anatomical and functional scans are normalized to the Talairach atlas using AFNI. Sub-regions of the medial temporal lobe and the hippocampus (bilateral entorhinal cortex, perirhinal cortex, parahippocampal cortex, CA3/dentate region, CA1 region, and subiculum) are segmented in three dimensions on the MPRAGE scans. The labels for the CA3 region and dentate gyrus (DG) are combined. The anatomically defined ROIs are then used to calculate the ROI-LDDMM 3D vector field transformation for each subject using a customized template based on the mean of the entire sample tested as the target. The ROI-LDDMM transformations for each individual subject's ROIs are then applied to the fit coefficient maps.

Group data are analyzed using a two-way Analysis of Variance (ANOVA) with trial types and group as fixed factors, and subject as a random factor nested within group. A liberal peak threshold of $p<0.05$, along with a spatial extent threshold of 10 voxels are used to define functional ROIs on the overall F statistic. This approach, rather than using a direct pair-wise contrast, reduces voxel selection biases because any differences amongst the various conditions allowed for a voxel to be selected. This threshold is then combined with the anatomical segmentations to only include voxels inside the regions of interest. This serves to exclude voxels that does not change with any of the model's factors, effectively limiting the analysis to voxels showing any changes with task condition or group. Voxels within each functional ROI are collapsed for further analysis.

**Cognitive tests during fMRI scans at Visits 1 and 2**

The activity of the subject's medial temporal lobe is measured by functional MRI during the subject's participation in an explicit 3-alternative forced choice task, where participants view novel, repeated and similar ("lure") stimuli. The Psychophysics Toolbox extensions in Matlab 7.0 (The MathWorks, Natick, MA) is used for stimulus presentation and behavioral data collection. Stimuli are color photographs of common objects. Each participant undergoes a series of testing runs duing the functional imaging sessions, each run consisting of a mix of three types of image pairs: similar pairs, identical pairs and unrelated foils. These image pairs are fully randomized throughout the run and presented individually as a series of images (see **FIG. 6A**). Participants are instructed to make a judgment as to whether each object seen is new, old or similar. Of critical interest are the participants' responses when presented with the second of the pair of similar objects (the "lure"; see **FIG. 6B**). The correct identification by the subject of lure stimuli as "similar," provides behavioral evidence of pattern separation, i.e., the separation of similar experiences into distinct non-overlapping representations. However, an incorrect indentification of lure stimuli as "old" or "new," indicates a failure of pattern separation. Identification of lure stimuli as "old" indicates that the subject focused on the similarities between the lure stimulus and the earlier-shown partner image. Identification of the lure stimulus as "new" indicates that the subject failed to recall the earlier-shown partner image altogether. Each run also contains a number of baseline trials that use a challenging perceptual discrimination task known to provide a lower and more-stable estimate of baseline activity in the medial temporal lobe (Stark & Squire, 2001 PNAS; Law et al, 2005).

A survery of the activity level of various subregions in the medial temporal lobe during the cogitive test, as measured by fMRI, shows that aMCI subjects have hyperactive DG/CA3 regions and a hypoactive entorhinal cortex

during the performance of memory tasks, compared to age-matched control subjects.

We assess the level of activity in DG/CA3 during successful memory judgments in control and aMCI subjects. The mean activity is calculated from the average activity, as measured by fMRI, during the presentation of lure stimuli correctly idenfied by subject as "similar" that is calibrated for baseline activity. **FIG. 4A** shows that aMCI patients exhibit DG/CA3 hyperactivity when making these judgments (p = 0.013). **FIG. 4B,** however, shows that treatment with levetiracetam reduces DG/CA3 hyper-activity in aMCI subjects (p = 0.037). The activity level in the aMCI subject treated with the drug, in fact, is normalized to the extent that that it is statistically indistinguishable from the activity of control subjects treated with placebo. See **FIG. 4C** for the mean activity values shown in **FIGS. 4A and 4B.**

The activity level during successful memory judgments in EC is significantly lower in placebo-treated aMCI subjects compared to controls (p = 0.003). See **FIG. 5A.** However, levetiracetam treatment normalizes activity in aMCI subjects in EC as well. Levetiracetam treatment increases EC activity during memory judgments in aMCI subjects, such that it is statistically indistinguishable from placebo-treated control subjects. See **FIG. 5C** for the mean activity values shown in **FIGS. 5A and 5B.**

The normalization of DG/CA3 and EC activity during memory judgments by levetiracetam treatment is mirrored in the change seen in the aMCI subjects' performace in the cognitive task. With placebo treatment, aMCI patients perform worse that control subjects, correctly identify lure items as "similar" less often and incorrectly identifying them as "old" more often (p = 0.009). See **FIG. 7.** However, the performance of aMCI subjects improves significantly under levetiracetam treatment. See **FIG. 8.** The interaction of more correct "similar" indenficiations with less incorrect "old" identifications under drug treatment results in a significant improvement in the performance of this memory task (p = 0.039). See **FIG. 9** for a table of the data represented in **FIGS. 7** and **8**.

The performance of control-placebo subjects and aMCI subjects with drug or placebo treatment is also compared in other common cognitive tests, such as the Buschke Selective Reminding Test - Delayed Recall (**FIGS. 10A** and **10B),** the Benton Visual Rentention Test (**FIGS. 11A** and **11B**), Verbal Paired Associates Test - Recognition **(FIGS. 12A** and **12B)** and Verbal Paired Associates Test - Delayed Recall **(FIGS. 13A** and **13B).** In all of these tests, aMCI subjects treated with placebo perform worse than placebo-treated control subjects, and levetiracetam treatment fail to rescue performance in aMCI subjects.

There are a number of possible reasons why levetiracetam treatment does not help aMCI subjects with performance in these other cognitive tests. The explicit 3-alternative forced choice task done in the fMRI study is a task that is especially sensitive to DG/CA3 function. As such, the performace of the subjects in this task may be particularly attuned to the changes in DG/CA3 activity resulting from levetiracetam treatment. Further, the aMCI subjects were treated with levetiracetam for only two weeks prior to the administration of the cognitive tests. It is contemplated that a treatment duration of longer than two weeks, e.g., 16 weeks or 8 months, for the drug treatment will result in improved efficacy. Finally, comparative animal studies (see Example 1) indicate that an even lower dose would be more effective. The human dosage of 125 mg twice a day is equivalent to a rat dosage of 22.3 mg/kg/day. As is shown in Example 1 and **FIG.** 1, 20 mg/kg levetiracetam is too high a dose in rats, and it fails to improve the performace of AI rats in the radial maze task. The effective doses of levetiracetam used in the animal model are 5-10 mg/kg. The human equivalent dose (HED) of the optimal rat dose is 0.8-1.6 mg/kg/day. Such a dosage would result in the administration of 28-56 mg twice a day (which is substantially lower than the 125 mg twice a day used in this study). Thus, it is contemplated that aMCI subjects will exhibit a further normalization of DG/CA3 and EC activity, as well as further improved performance in cognitive tests, if they are treated with lower doses equivalent to the effective doses in rat, e.g., 25 - 60 mg twice a day of levetiracetam.

## Claims

1. A composition comprising levetiracetam or a pharmaceutically acceptable salt, hydrate, solvate, or polymorph thereof and donepezil or a pharmaceutically acceptable salt, hydrate, solvate, or polymorph thereof in separate dosage form packaged together or in a unit dosage form.

2. The composition of claim 1, wherein the composition is in a solid form, a liquid form, or a suspension form.

3. The composition of claim 1, wherein the composition is in a sustained release form, a delayed release form, or an extended release form.

4. The composition of claim 1 or 2, wherein the levetiracetam or the pharmaceutically acceptable salt, hydrate, solvate, or polymorph thereof in the composition is formulated for extended release.

5. The composition of any one of claims 1-4, wherein the composition is for once-a-day administration.

6. The composition of any one of claims 1-4, wherein the composition is for oral administration.

7. A composition for use in treating cognitive impairment or for use in treating or improving cognitive function in a subject suffering from a central nervous system (CNS) disorder with cognitive impairment or at risk thereof, wherein the composition comprises levetiracetam or a pharmaceutically acceptable salt, hydrate, solvate, or polymorph thereof and donepezil or a pharmaceutically acceptable salt, hydrate, solvate, or polymorph thereof.

8. The composition for use according to claim 7, wherein the levetiracetam or the pharmaceutically acceptable salt, hydrate, solvate, or polymorph thereof and/or the donepezil or the pharmaceutically acceptable salt, hydrate, solvate, or polymorph thereof are administered to the subject at doses that are subtherapeutic as compared to the doses at which they are therapeutically effective when administered in the absence of the other.

9. The composition for use according to claim 7 or claim 8, wherein the levetiracetam or the pharmaceutically acceptable salt, hydrate, solvate, or polymorph thereof in the composition is formulated for extended release.

10. The composition of any one of claims 1-3, wherein the levetiracetam or the pharmaceutically acceptable salt, hydrate, solvate, or polymorph thereof in the composition is present in an amount of 0.07 - 350 mg.

11. The composition of any one of claims 1-3, wherein the levetiracetam or the pharmaceutically acceptable salt, hydrate, solvate, or polymorph thereof in the composition is present in an amount of 50 - 250 mg.

12. The composition of any one of claims 1-3, wherein the levetiracetam or the pharmaceutically acceptable salt, hydrate, solvate, or polymorph thereof in the composition is present in an amount of less than 350 mg, less than 250 mg, less than 200 mg, less than 150 mg, less than 100 mg, less than 50 mg, less than 10 mg, less than 5 mg, less than 1 mg, less than 0.5 mg, less than 0.1 mg, or less than 0.07 mg.

13. The composition of any one of claims 1-3, wherein the donepezil or the pharmaceutically acceptable salt, hydrate, solvate, or polymorph thereof in the composition is present in an amount of 0.1 mg to 10 mg.

14. The composition of any one of claims 1-3, wherein the donepezil or the pharmaceutically acceptable salt, hydrate, solvate, or polymorph thereof in the composition is present in an amount of less than 10 mg, less than 5 mg, less than 2 mg, less than 1 mg, or less than 0.5 mg.

15. The composition for use according to claim 7 or claim 8, wherein the levetiracetam or the pharmaceutically acceptable salt, hydrate, solvate, or polymorph thereof is administered every 12 or 24 hours at a daily dose of 0.001 mg/kg to 5 mg/kg.

16. A composition for use according to claim 7 or claim 8, wherein the levetiracetam or the pharmaceutically acceptable salt, hydrate, solvate, or polymorph thereof is administered every 12 or 24 hours at a daily dose of 0.1 mg/kg to 5 mg/kg.

17. The composition for use according to claim 7 or claim 8, wherein the levetiracetam or the pharmaceutically acceptable salt, hydrate, solvate, or polymorph thereof is administered every 12 or 24 hours at a daily dose of 0.5 mg/kg to 5 mg/kg.

18. The composition for use according to claim 7 or claim 8, wherein the levetiracetam or the pharmaceutically acceptable salt, hydrate, solvate, or polymorph thereof is administered every 12 or 24 hours at a daily dose of less than 5 mg/kg, less than 2.5 mg/kg, less than 2 mg/kg, less than 1.5 mg/kg, less than 1 mg/kg, less than 0.5 mg/kg, less than 0.1 mg/kg, less than 0.05 mg/kg, less than 0.01 mg/kg, less than 0.005 mg/kg, or less than 0.001 mg/kg.

19. The composition for use according to claim 7 or claim 8, wherein the donepezil or the pharmaceutically acceptable salt, hydrate, solvate, or polymorph thereof is administered at a daily dose of 0.1 mg to 10 mg.

20. The composition for use according to claim 7 or claim 8, wherein the donepezil or the pharmaceutically acceptable salt, hydrate, solvate, or polymorph thereof is administered at a daily dose of less than 10 mg, less than 5 mg, less than 2 mg, less than 1 mg, less than 0.5 mg, or less than 0.1 mg.

21. The composition for use according to any one of claims 7-9 and 15-20, wherein the levetiracetam or the pharmaceutically acceptable salt, hydrate, solvate, or polymorph thereof and the donepezil or the pharmaceutically acceptable salt, hydrate, solvate, or polymorph thereof are administered:

a) simultaneously;
b) sequentially;
c) in a single formulation; or
d) in a separate formulation.

**22.** The composition for use according to any one of claims 7-9 and 15-21, wherein the treatment has a longer therapeutic effect in the subject than

a) the therapeutic effect attained by administering the donepezil or the pharmaceutically acceptable salt, hydrate, solvate, or polymorph thereof in the absence of the levetiracetam or the pharmaceutically acceptable salt, hydrate, solvate, or polymorph thereof by at least about 1.5x, or 2.0x, or 2.5x, or 3.0x, or 3.5x, or 4.0x, or 4.5x, or 5.0x, or 5.5x, or 6.0x, or 6.5x, or 7.0x, or 7.5x, or 8.0x, or 8.5x, or 9.0x, or 9.5x, or 10x, or greater than about 10x; or
b) the therapeutic effect attained by administering the levetiracetam or the pharmaceutically acceptable salt, hydrate, solvate, or polymorph thereof in the absence of the donepezil or the pharmaceutically acceptable salt, hydrate, solvate, or polymorph thereof by at least about 1.5x, or 2.0x, or 2.5x, or 3.0x, or 3.5x, or 4.0x, or 4.5x, or 5.0x, or 5.5x, or 6.0x, or 6.5x, or 7.0x, or 7.5x, or 8.0x, or 8.5x, or 9.0x, or 9.5x, or 10x, or greater than about 10x.

**23.** The composition for use according to any one of claims 7-9 and 15-22, wherein the CNS disorder is age-related cognitive impairment, Mild Cognitive Impairment, amnestic Mild Cognitive Impairment, Age-Associated Memory Impairment, Age Related Cognitive Decline, dementia, Alzheimer's Disease, prodromal Alzheimer's Disease, schizophrenia, amyotrophic lateral sclerosis, post traumatic stress disorder, or cancer-therapy-related cognitive impairment.

**24.** The composition for use according to any one of claims 7-9 and 15-23, wherein the treatment prevents or slows the progression of the CNS disorder with cognitive impairment, alleviates or ameliorates or slows the progression of one or more symptoms associated with the CNS disorder with cognitive impairment, prevents decline of cognitive function, or slows the conversion of age-related cognitive impairment into dementia or Alzheimer's disease.

**25.** A composition for use according to any one of claims 7-9 and 15-24, wherein the cognitive function is measured by Clinical Dementia Rating Scale.

**Patentansprüche**

**1.** Zusammensetzung, umfassend Levetiracetam oder ein pharmazeutisch verträgliches Salz, Hydrat, Solvat oder Polymorph davon und Donepezil oder ein pharmazeutisch verträgliches Salz, Hydrat, Solvat oder Polymorph davon in getrennter Arzneiform, verpackt zusammen oder in einer einzeldosierten Arzneiform.

**2.** Zusammensetzung nach Anspruch 1, wobei die Zusammensetzung in einer Festform, einer Flüssigform oder einer Suspensionsform vorliegt.

**3.** Zusammensetzung nach Anspruch 1, wobei die Zusammensetzung in einer hinhaltenden Freigabeform, einer verzögerten Freigabeform oder einer protrahierten Freigabeform vorliegt.

**4.** Zusammensetzung nach Anspruch 1 oder 2, wobei das Levetiracetam oder das pharmazeutisch verträgliche Salz, Hydrat, Solvat oder Polymorph davon in der Zusammensetzung zur protrahierten Freigabe formuliert ist.

**5.** Zusammensetzung nach einem der Ansprüche 1-4, wobei die Zusammensetzung zur einmal täglichen Verabreichung vorgesehen ist.

**6.** Zusammensetzung nach einem der Ansprüche 1-4, wobei die Zusammensetzung zur oralen Verabreichung vorgesehen ist.

**7.** Zusammensetzung zur Verwendung bei der Behandlung der kognitiven Störung oder zur Verwendung bei der Behandlung oder Verbesserung der kognitiven Funktion bei einem Patienten, der an einer Erkrankung des Zentralnervensystems (ZNS) mit kognitiver Störung oder einem Risiko davon leidet, wobei die Zusammensetzung Levetiracetam oder ein pharmazeutisch verträgliches Salz, Hydrat, Solvat oder Polymorph davon und Donepezil oder

ein pharmazeutisch verträgliches Salz, Hydrat, Solvat oder Polymorph davon umfasst.

**8.** Zusammensetzung zur Verwendung nach Anspruch 7, wobei das Levetiracetam oder das pharmazeutisch verträgliche Salz, Hydrat, Solvat oder Polymorph davon und/oder das Donepezil oder das pharmazeutisch verträgliche Salz, Hydrat, Solvat oder Polymorph davon an den Patienten in Dosen verabreicht werden, die subtherapeutisch sind im Vergleich zu den Dosen, bei denen sie therapeutisch wirksam sind, wenn sie in Abwesenheit des anderen verabreicht werden.

**9.** Zusammensetzung zur Verwendung nach Anspruch 7 oder Anspruch 8, wobei das Levetiracetam oder das pharmazeutisch verträgliche Salz, Hydrat, Solvat oder Polymorph davon in der Zusammensetzung zur protrahierten Freigabe formuliert ist.

**10.** Zusammensetzung nach einem der Ansprüche 1-3, wobei das Levetiracetam oder das pharmazeutisch verträgliche Salz, Hydrat, Solvat oder Polymorph davon in der Zusammensetzung in einer Menge von 0,07 - 350 mg vorliegt.

**11.** Zusammensetzung nach einem der Ansprüche 1-3, wobei das Levetiracetam oder das pharmazeutisch verträgliche Salz, Hydrat, Solvat oder Polymorph davon in der Zusammensetzung in einer Menge von 50 - 250 mg vorliegt.

**12.** Zusammensetzung nach einem der Ansprüche 1-3, wobei das Levetiracetam oder das pharmazeutisch verträgliche Salz, Hydrat, Solvat oder Polymorph davon in der Zusammensetzung in einer Menge von weniger als 350 mg, weniger als 250 mg, weniger als 200 mg, weniger als 150 mg, weniger als 100 mg, weniger als 50 mg, weniger als 10 mg, weniger als 5 mg, weniger als 1 mg, weniger als 0,5 mg, weniger als 0,1 mg oder weniger als 0,07 mg vorliegt.

**13.** Zusammensetzung nach einem der Ansprüche 1-3, wobei das Donepezil oder das pharmazeutisch verträgliche Salz, Hydrat, Solvat oder Polymorph davon in der Zusammensetzung in einer Menge von 0,1 mg bis 10 mg vorliegt.

**14.** Zusammensetzung nach einem der Ansprüche 1-3, wobei das Donepezil oder das pharmazeutisch verträgliche Salz, Hydrat, Solvat oder Polymorph davon in der Zusammensetzung in einer Menge von weniger als 10 mg, weniger als 5 mg, weniger als 2 mg, weniger als 1 mg oder weniger als 0,5 mg vorliegt.

**15.** Zusammensetzung zur Verwendung nach Anspruch 7 oder Anspruch 8, wobei das Levetiracetam oder das pharmazeutisch verträgliche Salz, Hydrat, Solvat oder Polymorph davon alle 12 oder 24 Stunden in einer Tagesdosis von 0,001 mg/kg bis 5 mg/kg verabreicht wird.

**16.** Zusammensetzung zur Verwendung nach Anspruch 7 oder Anspruch 8, wobei das Levetiracetam oder das pharmazeutisch verträgliche Salz, Hydrat, Solvat oder Polymorph davon alle 12 oder 24 Stunden in einer Tagesdosis von 0,1 mg/kg bis 5 mg/kg verabreicht wird.

**17.** Zusammensetzung zur Verwendung nach Anspruch 7 oder Anspruch 8, wobei das Levetiracetam oder das pharmazeutisch verträgliche Salz, Hydrat, Solvat oder Polymorph davon alle 12 oder 24 Stunden in einer Tagesdosis von 0,5 mg/kg bis 5 mg/kg verabreicht wird.

**18.** Zusammensetzung zur Verwendung nach Anspruch 7 oder Anspruch 8, wobei das Levetiracetam oder das pharmazeutisch verträgliche Salz, Hydrat, Solvat oder Polymorph davon alle 12 oder 24 Stunden in einer Tagesdosis von weniger als 5 mg/kg, weniger als 2,5 mg/kg, weniger als 2 mg/kg, weniger als 1,5 mg/kg, weniger als 1 mg/kg, weniger als 0,5 mg/kg, weniger als 0,1 mg/kg, weniger als 0,05 mg/kg, weniger als 0,01 mg/kg, weniger als 0,005 mg/kg oder weniger als 0,001 mg/kg verabreicht wird.

**19.** Zusammensetzung zur Verwendung nach Anspruch 7 oder Anspruch 8, wobei das Donepezil oder das pharmazeutisch verträgliche Salz, Hydrat, Solvat oder Polymorph davon in einer Tagesdosis von 0,1 mg bis 10 mg verabreicht wird.

**20.** Zusammensetzung zur Verwendung nach Anspruch 7 oder Anspruch 8, wobei das Donepezil oder das pharmazeutisch verträgliche Salz, Hydrat, Solvat oder Polymorph davon in einer Tagesdosis von weniger als 10 mg, weniger als 5 mg, weniger als 2 mg, weniger als 1 mg, weniger als 0,5 mg oder weniger als 0,1 mg verabreicht wird.

**21.** Zusammensetzung zur Verwendung nach einem der Ansprüche 7-9 und 15-20, wobei das Levetiracetam oder das pharmazeutisch verträgliche Salz, Hydrat, Solvat oder Polymorph davon und das Donepezil oder das pharmazeutisch verträgliche Salz, Hydrat, Solvat oder Polymorph davon wie folgt verabreicht werden:

a) simultan;
b) sequenziell;
c) in einer einzelnen Formulierung; oder
d) in einer getrennten Formulierung.

**22.** Zusammensetzung zur Verwendung nach einem der Ansprüche 7-9 und 15-21, wobei die Behandlung eine längere therapeutische Wirkung bei dem Patienten aufweist als

a) die durch Verabreichung des Donepezils oder des pharmazeutisch verträglichen Salzes, Hydrats, Solvats oder Polymorphs davon in Abwesenheit des Levetiracetams oder des pharmazeutisch verträglichen Salzes, Hydrats, Solvats oder Polymorphs davon erzielte therapeutische Wirkung, um mindestens ca. 1,5x, oder 2,0x, oder 2,5x, oder 3,0x, oder 3,5x, oder 4,0x, oder 4,5x, oder 5,0x, oder 5,5x oder 6,0x, oder 6,5x, oder 7,0x, oder 7,5x, oder 8,0x, oder 8,5x, oder 9,0x, oder 9,5x, oder 10x oder größer als ca. 10x; oder
b) die durch Verabreichung des Levetiracetams oder des pharmazeutisch verträglichen Salzes, Hydrats, Solvats oder Polymorphs davon in Abwesenheit des Donepezils oder des pharmazeutisch verträglichen Salzes, Hydrats, Solvats oder Polymorphs davon erzielte therapeutische Wirkung, um mindestens ca. 1,5x, oder 2,0x, oder 2,5x, oder 3,0x, oder 3,5x, oder 4,0x, oder 4,5x, oder 5,0x, oder 5,5x oder 6,0x, oder 6,5x, oder 7,0x, oder 7,5x, oder 8,0x, oder 8,5x, oder 9,0x, oder 9,5x, oder 10x oder größer als ca. 10x.

**23.** Zusammensetzung zur Verwendung nach einem der Ansprüche 7-9 und 15-22, wobei die ZNS-Erkrankung eine altersbedingte kognitive Störung, leichte kognitive Störung, leichte amnestische kognitive Störung, altersassoziierte Gedächtnisstörung, ein altersbedingter kognitiver Abbau, eine Demenz, Alzheimer-Krankheit, prodromale Alzheimer-Krankheit, Schizophrenie, amyotrophe Lateralsklerose, Posttraumatische Belastungsstörung oder durch Krebstherapie bedingte kognitive Störung ist.

**24.** Zusammensetzung zur Verwendung nach einem der Ansprüche 7-9 und 15-23, wobei die Behandlung die Progression der ZNS-Erkrankung mit kognitiver Störung verhindert oder verlangsamt, lindert oder verbessert oder die Progression von einem oder mehr mit der ZNS-Erkrankung mit kognitiver Störung assoziierten Symptom(en) verlangsamt, den Abbau der kognitiven Funktion verhindert oder die Konvertierung einer altersbedingten kognitiven Störung in eine Demenz oder Alzheimer-Krankheit verlangsamt.

**25.** Zusammensetzung zur Verwendung nach einem der Ansprüche 7-9 und 15-24, wobei die kognitive Funktion mittels der Clinical Dementia Rating Scale (Skala zur klinischen Beurteilung der Demenz) gemessen wird.

## Revendications

**1.** Composition comprenant du lévétiracétam ou un sel, hydrate, solvate ou polymorphe pharmaceutiquement acceptable de celui-ci et du donépézil ou un sel, hydrate, solvate ou polymorphe pharmaceutiquement acceptable de celui-ci sous la forme de doses distinctes emballées ensemble ou sous la forme de doses unitaires.

**2.** Composition selon la revendication 1, dans laquelle la composition est sous une forme solide, une forme liquide ou une forme de suspension.

**3.** Composition selon la revendication 1, dans laquelle la composition est sous une forme à libération soutenue, une forme à libération retard ou une forme à libération prolongée.

**4.** Composition selon la revendication 1 ou 2, dans laquelle le lévétiracétam ou le sel, hydrate, solvate ou polymorphe pharmaceutiquement acceptable de celui-ci dans la composition est mis en forme pour une libération prolongée.

**5.** Composition selon l'une quelconque des revendications 1 à 4, dans laquelle la composition est pour une administration une fois par jour.

**6.** Composition selon l'une quelconque des revendications 1 à 4, dans laquelle la composition est pour une administration par voie orale.

7. Composition pour utilisation dans le traitement d'un trouble cognitif ou pour utilisation dans le traitement ou l'amélioration de la fonction cognitive chez un sujet souffrant ou risquant de souffrir d'un trouble du système nerveux central (SNC) avec un trouble cognitif, dans laquelle la composition comprend du lévétiracétam ou un sel, hydrate, solvate ou polymorphe pharmaceutiquement acceptable de celui-ci et du donépézil ou un sel, hydrate, solvate ou polymorphe pharmaceutiquement acceptable de celui-ci.

8. Composition pour utilisation selon la revendication 7, dans laquelle le lévétiracétam ou le sel, hydrate, solvate ou polymorphe pharmaceutiquement acceptable de celui-ci et/ou le donépézil ou le sel, hydrate, solvate ou polymorphe pharmaceutiquement acceptable de celui-ci sont administrés au sujet à des doses qui sont subthérapeutiques par rapport aux doses auxquelles ils sont thérapeutiquement efficaces quand ils sont administrés en l'absence de l'autre.

9. Composition pour utilisation selon la revendication 7 ou la revendication 8, dans laquelle le lévétiracétam ou le sel, hydrate, solvate ou polymorphe pharmaceutiquement acceptable de celui-ci dans la composition est mis en forme pour une libération prolongée.

10. Composition selon l'une quelconque des revendications 1 à 3, dans laquelle le lévétiracétam ou le sel, hydrate, solvate ou polymorphe pharmaceutiquement acceptable de celui-ci dans la composition est présent dans une quantité de 0,07 à 350 mg.

11. Composition selon l'une quelconque des revendications 1 à 3, dans laquelle le lévétiracétam ou le sel, hydrate, solvate ou polymorphe pharmaceutiquement acceptable de celui-ci dans la composition est présent dans une quantité de 50 à 250 mg.

12. Composition selon l'une quelconque des revendications 1 à 3, dans laquelle le lévétiracétam ou le sel, hydrate, solvate ou polymorphe pharmaceutiquement acceptable de celui-ci dans la composition est présent dans une quantité inférieure à 350 mg, inférieure à 250 mg, inférieure à 200 mg, inférieure à 150 mg, inférieure à 100 mg, inférieure à 50 mg, inférieure à 10 mg, inférieure à 5 mg, inférieure à 1 mg, inférieure à 0,5 mg, inférieure à 0,1 mg ou inférieure à 0,07 mg.

13. Composition selon l'une quelconque des revendications 1 à 3, dans laquelle le donépézil ou le sel, hydrate, solvate ou polymorphe pharmaceutiquement acceptable de celui-ci dans la composition est présent dans une quantité de 0,1 mg à 10 mg.

14. Composition selon l'une quelconque des revendications 1 à 3, dans laquelle le donépézil ou le sel, hydrate, solvate ou polymorphe pharmaceutiquement acceptable de celui-ci dans la composition est présent dans une quantité inférieure à 10 mg, inférieure à 5 mg, inférieure à 2 mg, inférieure à 1 mg ou inférieure à 0,5 mg.

15. Composition pour utilisation selon la revendication 7 ou la revendication 8, dans laquelle le lévétiracétam ou le sel, hydrate, solvate ou polymorphe pharmaceutiquement acceptable de celui-ci est administré toutes les 12 ou 24 heures à une dose quotidienne de 0,001 mg/kg à 5 mg/kg.

16. Composition pour utilisation selon la revendication 7 ou la revendication 8, dans laquelle le lévétiracétam ou le sel, hydrate, solvate ou polymorphe pharmaceutiquement acceptable de celui-ci est administré toutes les 12 ou 24 heures à une dose quotidienne de 0,1 mg/kg à 5 mg/kg.

17. Composition pour utilisation selon la revendication 7 ou la revendication 8, dans laquelle le lévétiracétam ou le sel, hydrate, solvate ou polymorphe pharmaceutiquement acceptable de celui-ci est administré toutes les 12 ou 24 heures à une dose quotidienne de 0,5 mg/kg à 5 mg/kg.

18. Composition pour utilisation selon la revendication 7 ou la revendication 8, dans laquelle le lévétiracétam ou le sel, hydrate, solvate ou polymorphe pharmaceutiquement acceptable de celui-ci est administré toutes les 12 ou 24 heures à une dose quotidienne inférieure à 5 mg/kg, inférieure à 2,5 mg/kg, inférieure à 2 mg/kg, inférieure à 1,5 mg/kg, inférieure à 1 mg/kg, inférieure à 0,5 mg/kg, inférieure à 0,1 mg/kg, inférieure à 0,05 mg/kg, inférieure à 0,01 mg/kg, inférieure à 0,005 mg/kg ou inférieure à 0,001 mg/kg.

19. Composition pour utilisation selon la revendication 7 ou la revendication 8, dans laquelle le donépézil ou le sel, hydrate, solvate ou polymorphe pharmaceutiquement acceptable de celui-ci est administré à une dose quotidienne de 0,1 mg à 10 mg.

**20.** Composition pour utilisation selon la revendication 7 ou la revendication 8, dans laquelle le donépézil ou le sel, hydrate, solvate ou polymorphe pharmaceutiquement acceptable de celui-ci est administré à une dose quotidienne inférieure à 10 mg, inférieure à 5 mg, inférieure à 2 mg, inférieure à 1 mg, inférieure à 0,5 mg ou inférieure à 0,1 mg.

**21.** Composition pour utilisation selon l'une quelconque des revendications 7 à 9 et 15 à 20, dans laquelle le lévétiracétam ou le sel, hydrate, solvate ou polymorphe pharmaceutiquement acceptable de celui-ci et le donépézil ou le sel, hydrate, solvate ou polymorphe pharmaceutiquement acceptable de celui-ci sont administrés :

a) simultanément ;
b) successivement ;
c) dans une seule présentation ; ou
d) dans une présentation distincte.

**22.** Composition pour utilisation selon l'une quelconque des revendications 7 à 9 et 15 à 21, dans laquelle le traitement a un effet thérapeutique plus long chez le sujet que

a) l'effet thérapeutique obtenu en administrant le donépézil ou le sel, hydrate, solvate ou polymorphe pharmaceutiquement acceptable de celui-ci en l'absence du lévétiracétam ou du sel, hydrate, solvate ou polymorphe pharmaceutiquement acceptable de celui-ci d'au moins environ 1,5 fois, ou 2,0 fois, ou 2,5 fois, ou 3,0 fois, ou 3,5 fois, ou 4,0 fois, ou 4,5 fois, ou 5,0 fois, ou 5,5 fois, ou 6,0 fois, ou 6,5 fois, ou 7,0 fois, ou 7,5 fois, ou 8,0 fois, ou 8,5 fois, ou 9,0 fois, ou 9,5 fois, ou 10 fois, ou de plus de 10 fois supérieur ; ou
b) l'effet thérapeutique obtenu en administrant le lévétiracétam ou le sel, hydrate, solvate ou polymorphe pharmaceutiquement acceptable de celui-ci en l'absence du donépézil ou du sel, hydrate, solvate ou polymorphe pharmaceutiquement acceptable de celui-ci d'au moins environ 1,5 fois, ou 2,0 fois, ou 2,5 fois, ou 3,0 fois, ou 3,5 fois, ou 4,0 fois, ou 4,5 fois, ou 5,0 fois, ou 5,5 fois, ou 6,0 fois, ou 6,5 fois, ou 7,0 fois, ou 7,5 fois, ou 8,0 fois, ou 8,5 fois, ou 9,0 fois, ou 9,5 fois, ou 10 fois, ou de plus de 10 fois supérieur.

**23.** Composition pour utilisation selon l'une quelconque des revendications 7 à 9 et 15 à 22, dans laquelle le trouble du SNC est la déficience cognitive liée à l'âge, la déficience cognitive moyenne, la déficience cognitive moyenne amnésique, les troubles de la mémoire associés à l'âge, le déclin cognitif lié à l'âge, la démence, la maladie d'Alzheimer, la maladie d'Alzheimer prodromique, la schizophrénie, la sclérose latérale amyotrophique, le trouble de stress post-traumatique ou la déficience cognitive liée à une thérapie anticancéreuse.

**24.** Composition pour utilisation selon l'une quelconque des revendications 7 à 9 et 15 à 23, dans laquelle le traitement prévient ou ralentit l'évolution du trouble du SNC avec un trouble cognitif, soulage ou améliore ou ralentit l'évolution d'un ou plusieurs symptômes associés au trouble du SNC avec un trouble cognitif, prévient le déclin de la fonction cognitive ou ralentit la conversion de la déficience cognitive liée à l'âge en démence ou maladie d'Alzheimer.

**25.** Composition pour utilisation selon l'une quelconque des revendications 7 à 9 et 15 à 24, dans laquelle la fonction cognitive est mesurée par l'échelle d'évaluation de la démence connue sous le nom de Clinical Dementia Rating Scale.

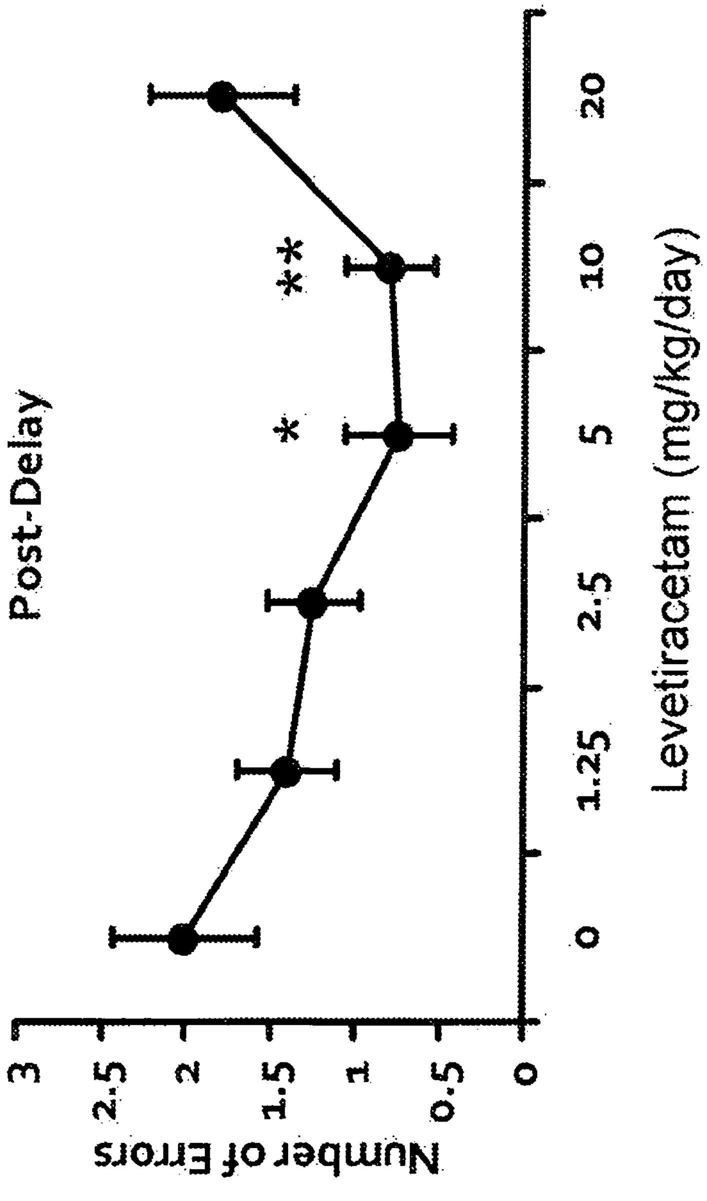


* $t(9) = 2.18$, $p = 0.057$

** $t(9) = 2.37$, $p = 0.042$

FIG. 1

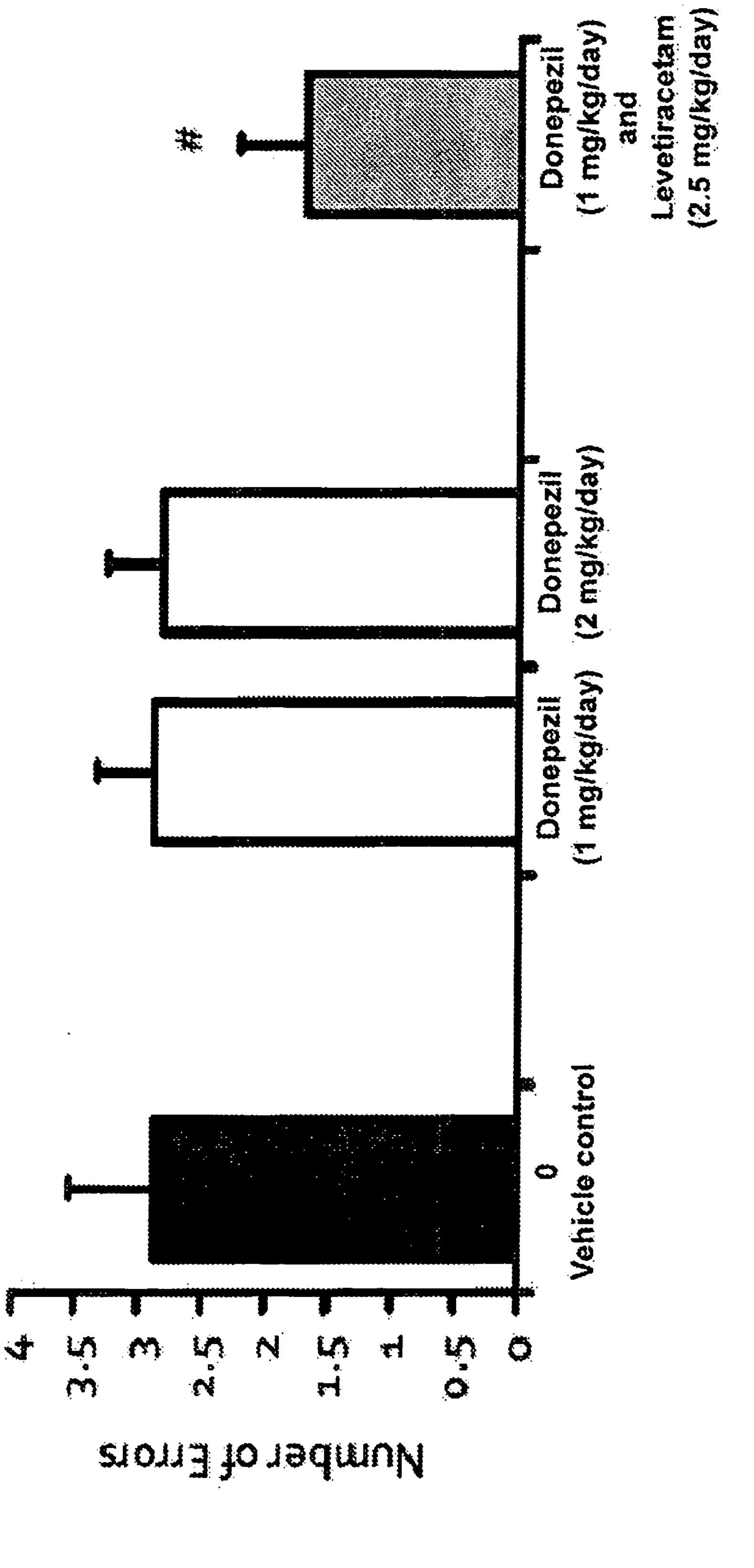


# $t(7) = 2.16$, $p = 0.034$

FIG. 2

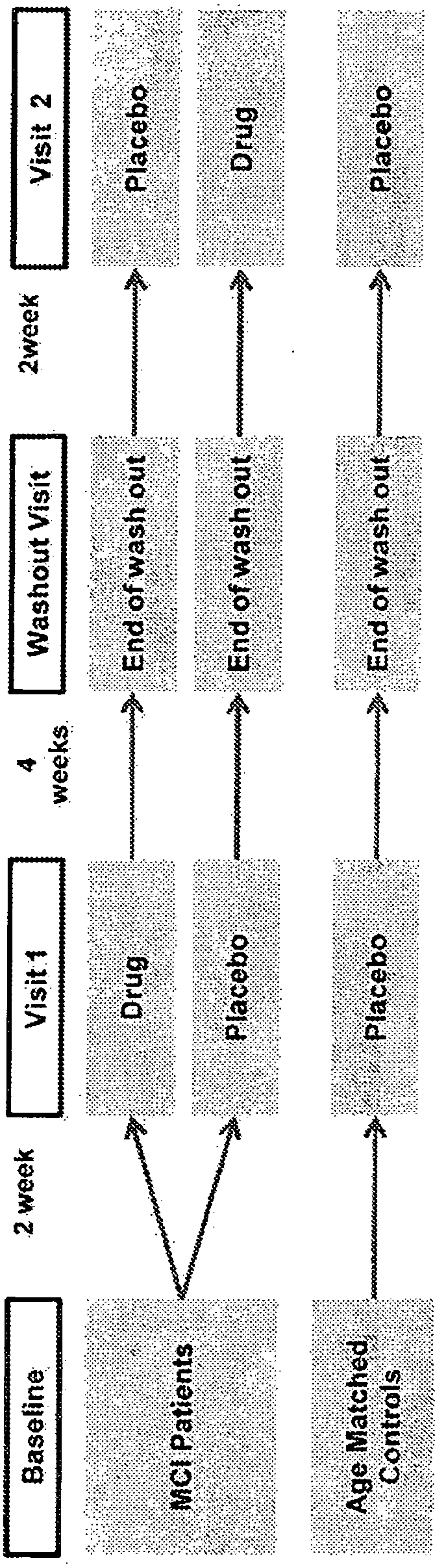
Baseline
2 week
Visit 1
4 weeks
Washout Visit
2week
Visit 2
MCI Patients
Drug
End of wash out
Placebo
Placebo
End of wash out
Drug
Age Matched Controls
Placebo
End of wash out
Placebo

FIG. 3

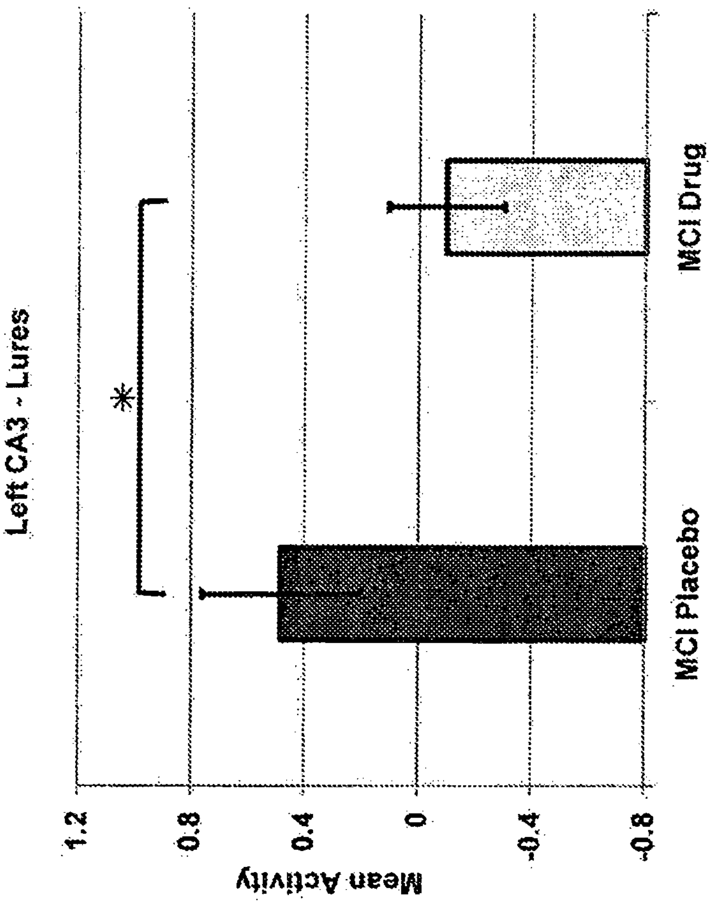


*Paired samples t-test: t = 2.276, p = 0.037

FIG. 4B

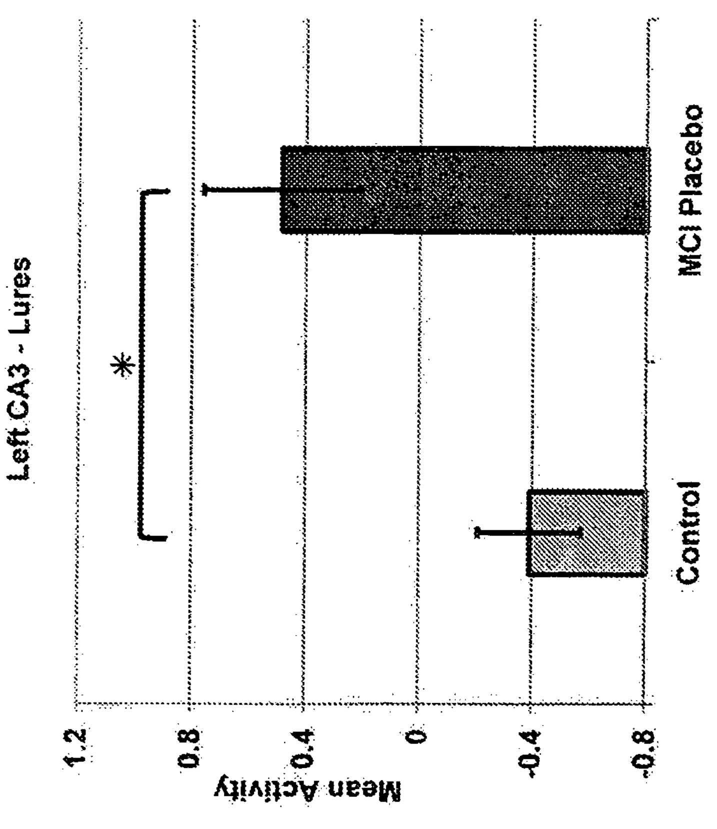


*Independent samples t-test: t = -2.636, p = 0.013

FIG. 4A

**Left CA3 Lures**

| Group | Mean Activity | Standard Error |
|---|---|---|
| Control | -0.39129 | 0.182628 |
| MCI Placebo | 0.48440 | 0.277487 |
| MCI Drug | -0.09653 | 0.205892 |

FIG. 4C

fMRI entorhinal activation in amnestic MCI is normalized by drug treatment

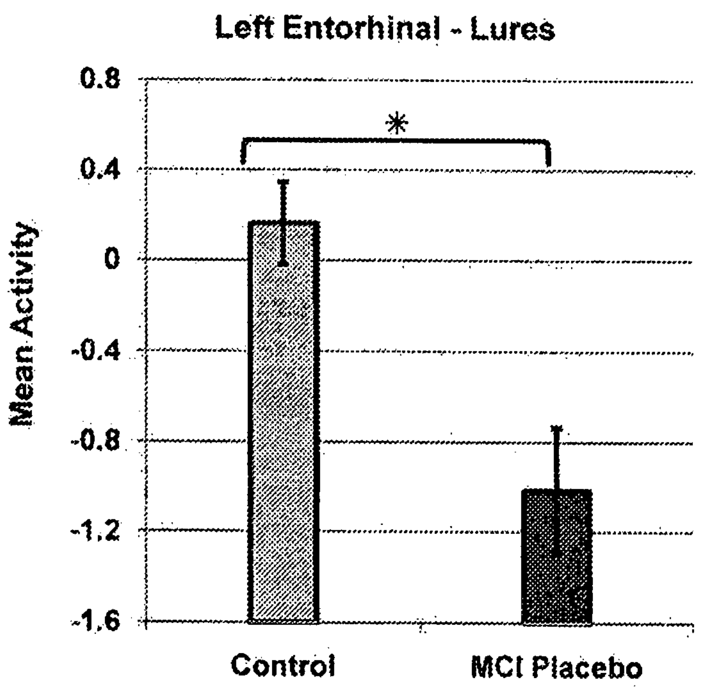


*Independent samples t-test: $t = 3.278$, $p = 0.003$

FIG. 5A

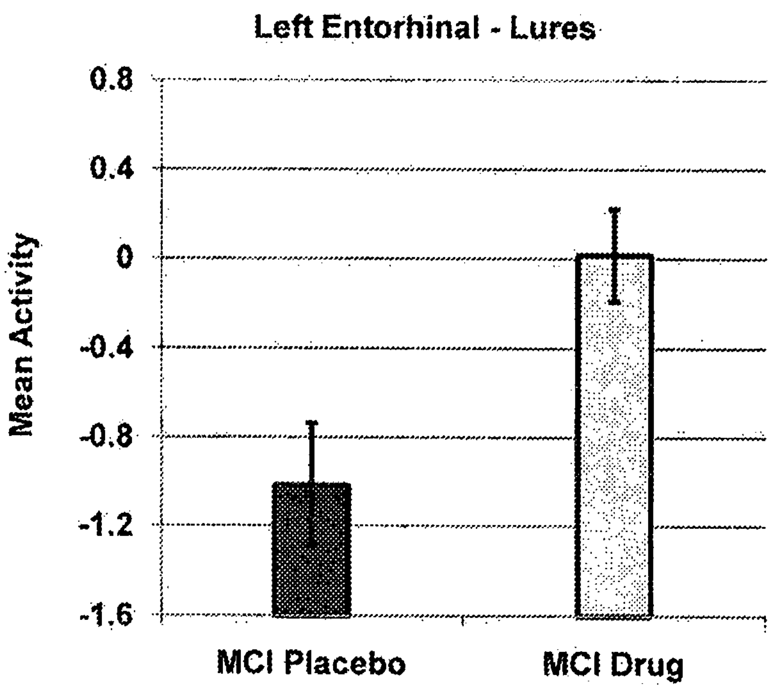


Paired samples t-test: $t = -1.600$, $p = 0.129$

FIG. 5B

**Left Entorhinal Lures**

| Group | Mean Activity | Standard Error |
|---|---|---|
| Control | 0.16444 | 0.143864 |
| MCI Placebo | -1.01273 | 0.329062 |
| MCI Drug | 0.016291 | 0.411762 |

FIG. 5C

Reduced memory in task that taxes pattern separation

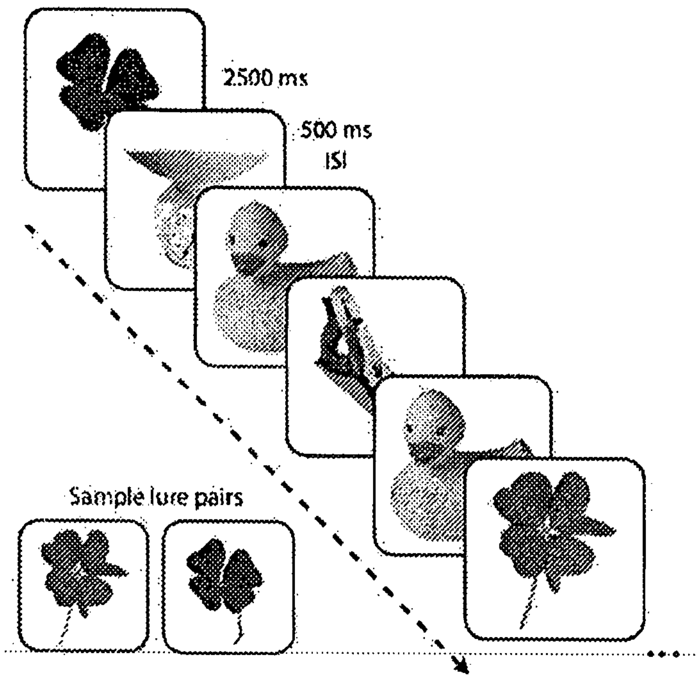


FIG. 6A

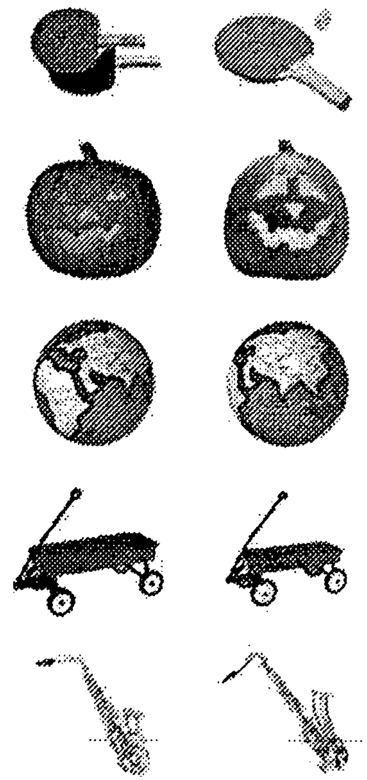

FIG. 6B

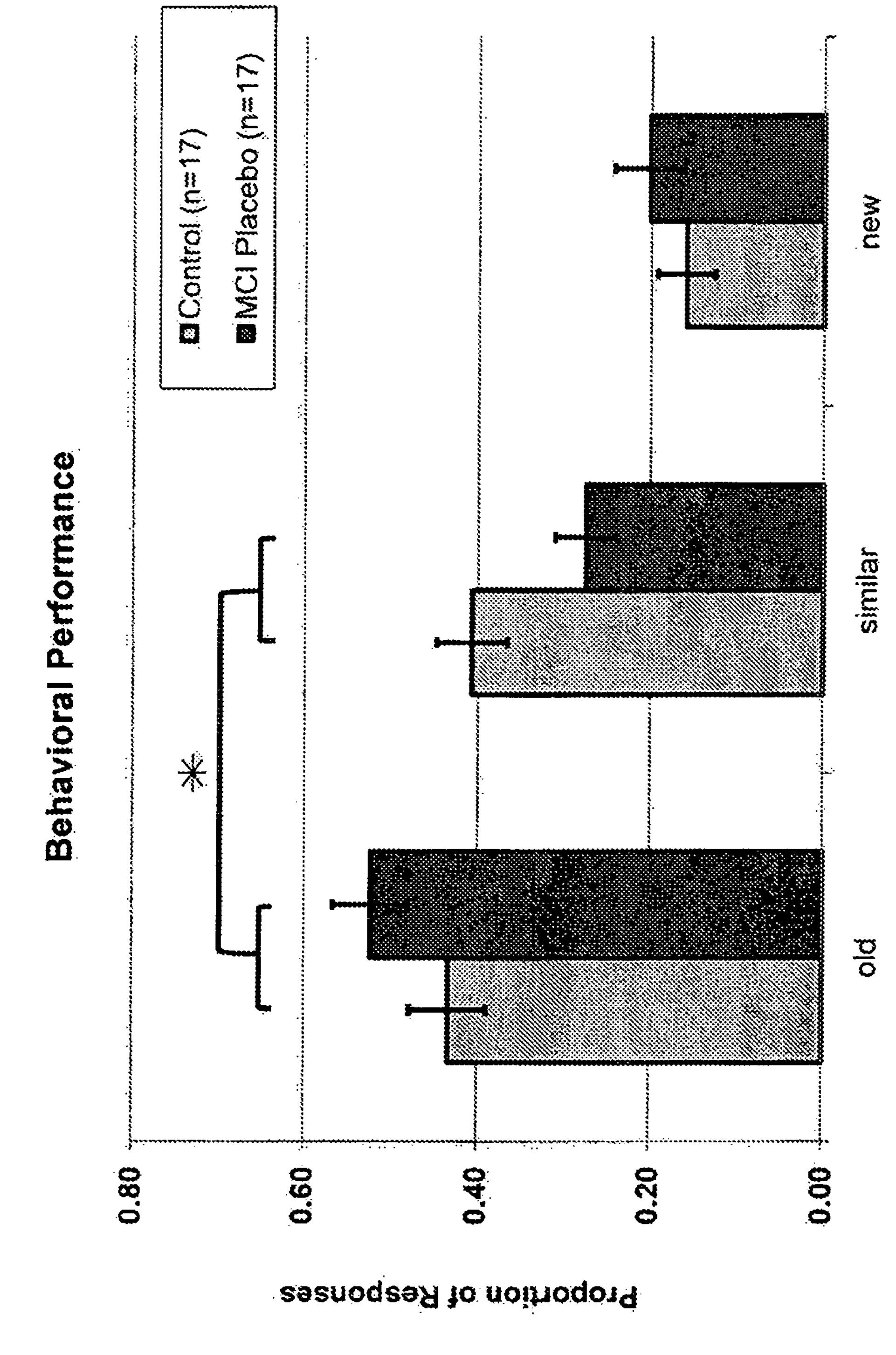


Control vs. MCI Placebo by Old vs. Similar: F= 7.687, p = 0.009

**FIG. 7**

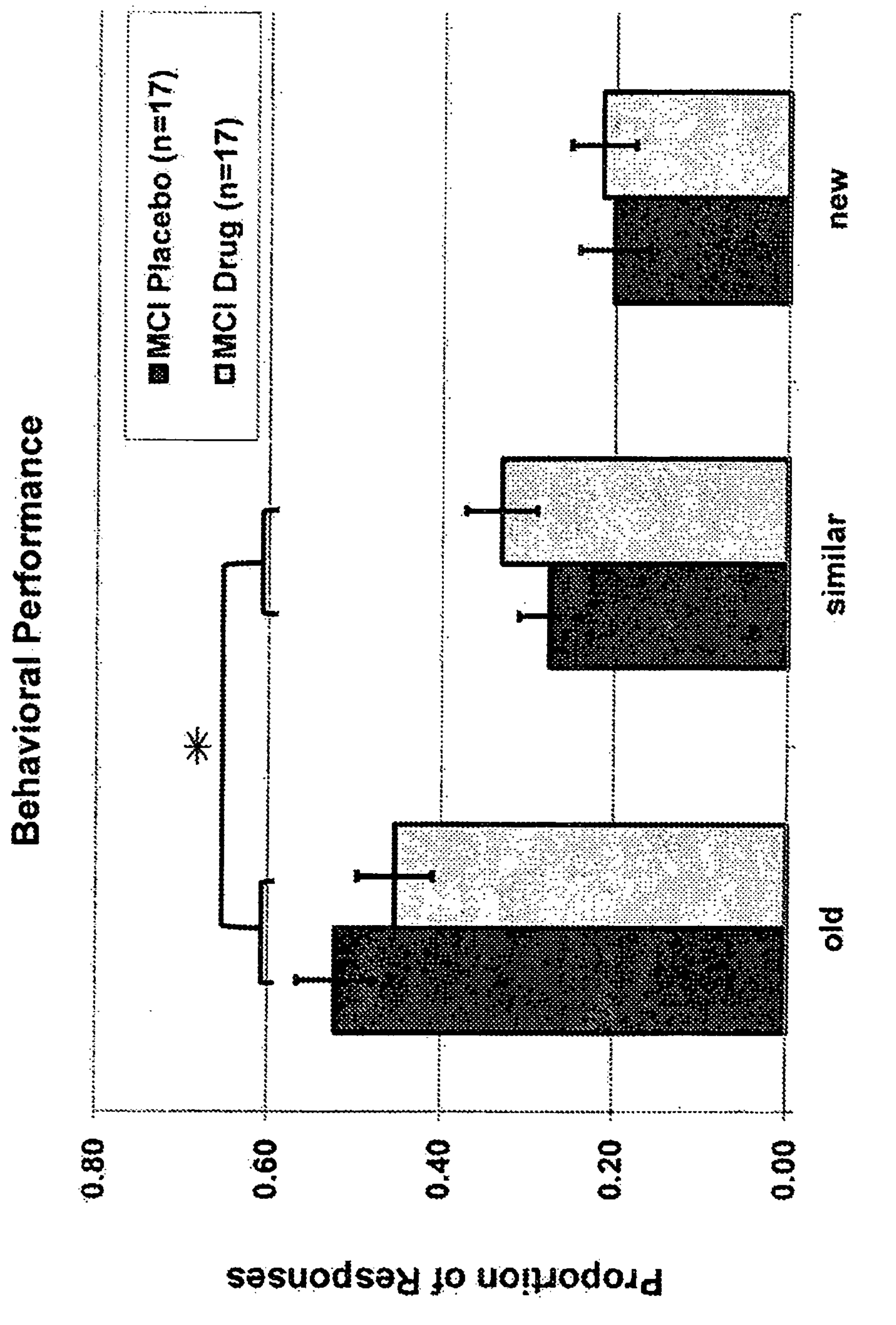


MCI Drug vs. MCI Placebo by Old vs. Similar: F = 5.028, p = 0.039

FIG. 8

## Behavioral Performance

| **Control Subjects** | **Proportion of Responses** | **Standard Error** |
|---|---|---|
| Old | 0.433676 | 0.04426 |
| Similar | 0.406771 | 0.04135 |
| New | 0.159553 | 0.03312 |
| **MCI Placebo Subjects** | **Proportion of Responses** | **Standard Error** |
| Old | 0.52262 | 0.04871 |
| Similar | 0.27549 | 0.03956 |
| New | 0.20188 | 0.04528 |
| **MCI Drug Subjects** | **Proportion of Responses** | **Standard Error** |
| Old | 0.45361 | 0.04825 |
| Similar | 0.33144 | 0.04592 |
| New | 0.21494 | 0.04202 |

FIG. 9

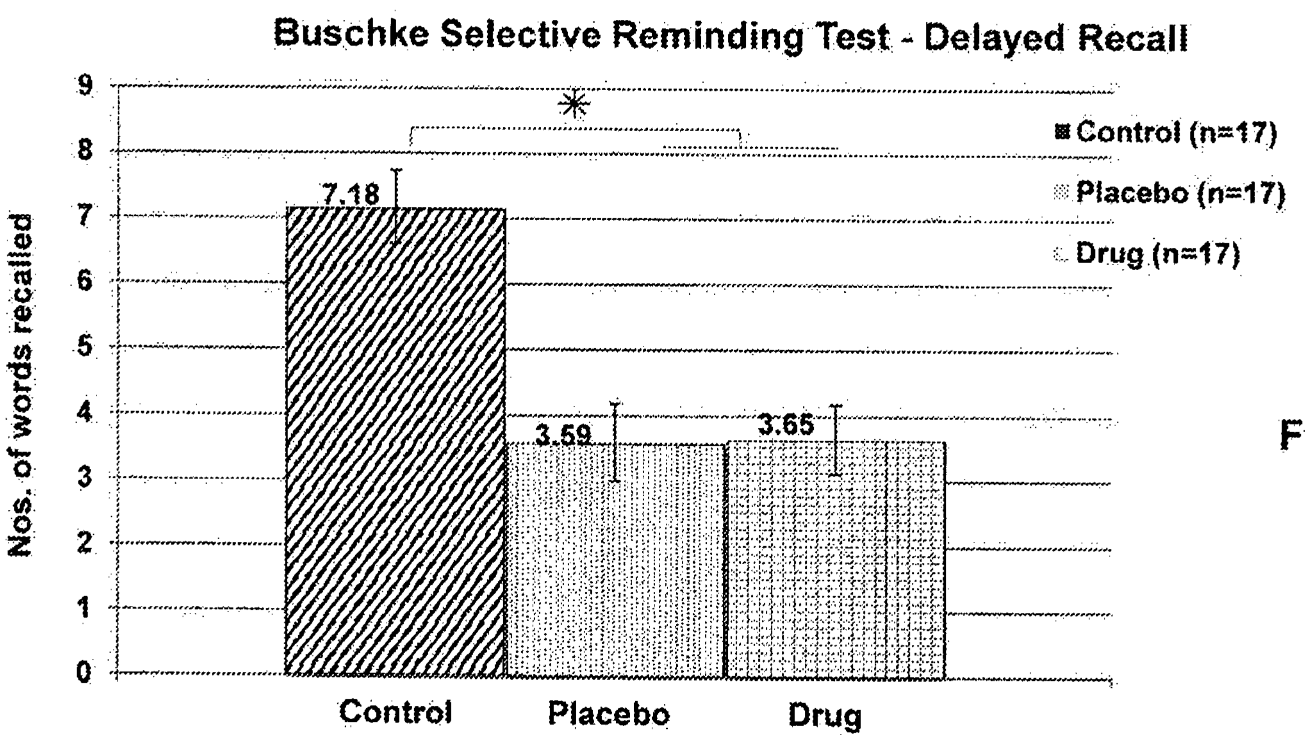


FIG. 10A

| Group | Mean | Standard Error | Delayed (p-value) |
|---|---|---|---|
| Control | 7.18 | 0.56 | Control vs. Placebo: <0.001<br>Control vs. Drug: < 0.001<br>Placebo vs. Drug: 0.887 |
| MCI Placebo | 3.59 | 0.59 | |
| MCI Drug | 3.65 | 0.53 | |

FIG. 10B

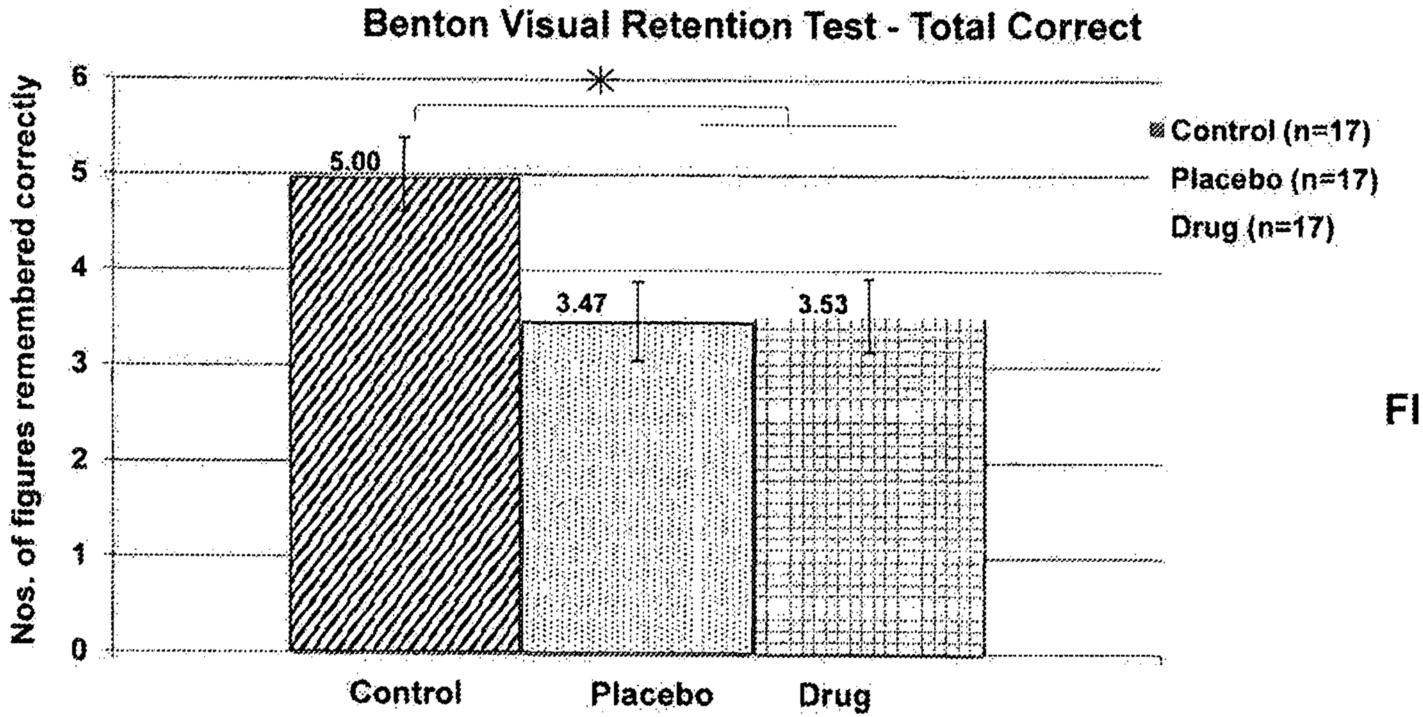


FIG. 11A

| Group | Mean | Standard Error | Total Correct (p-value) |
|---|---|---|---|
| Control | 5.00 | 0.38 | Control vs. Placebo: 0.011 |
| MCI Placebo | 3.47 | 0.41 | Control vs. Drug: 0.011 |
| MCI Drug | 3.53 | 0.38 | Placebo vs. Drug: 0.805 |

FIG. 11B

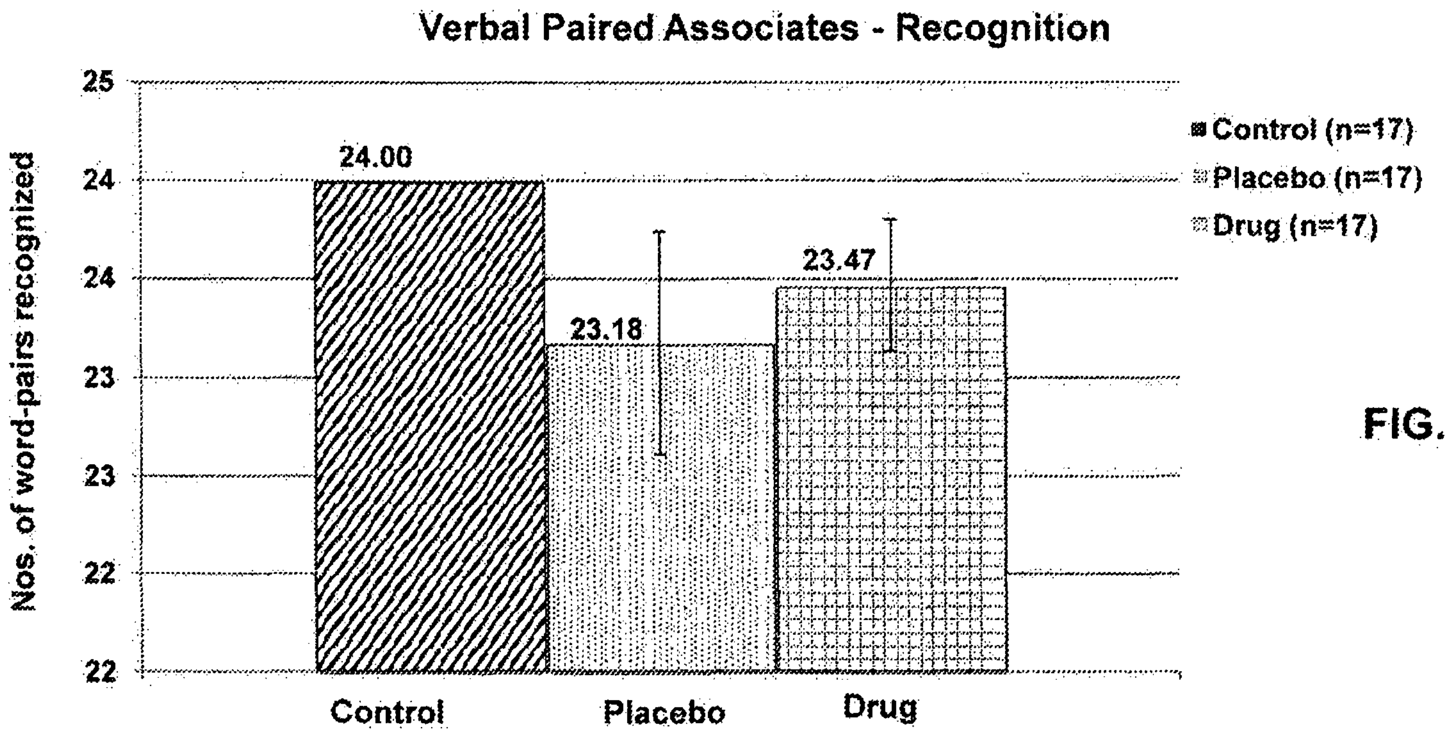


FIG. 12A

| Group | Mean | Standard Error | Recognition (p-value) |
|---|---|---|---|
| Control | 24.00 | 0.00 | Control vs. Placebo: 0.154 |
| MCI Placebo | 23.18 | 0.56 | Control vs. Drug: 0.122 |
| MCI Drug | 23.47 | 0.33 | Placebo vs. Drug: 0.428 |

FIG. 12B

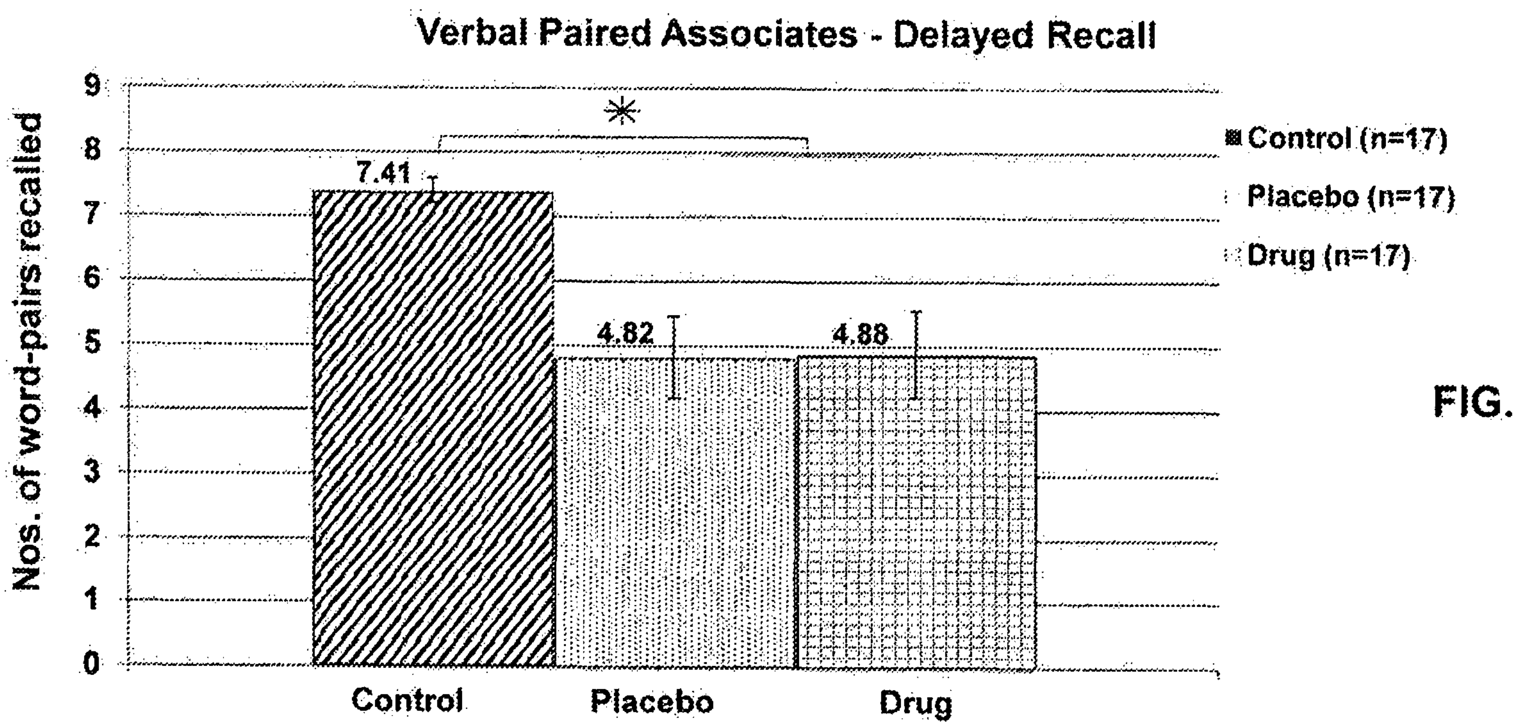


FIG. 13A

| Group | Mean | Standard Error | Delayed Recall (p-value) |
|---|---|---|---|
| Control | 7.41 | 0.19 | Control vs. Placebo: <0.001 |
| MCI Placebo | 4.82 | 0.63 | Control vs. Drug: 0.001 |
| MCI Drug | 4.88 | 0.67 | Placebo vs. Drug: 0.848 |

FIG. 13B

## Study Status

| | Control subjects | MCI subjects | Total subjects |
|---|---|---|---|
| Participated in screening | 26 | 32 | 58 |
| Screening failures | 4 | 9 | 13 |
| Enrolled | 22 | 23 | 45 |
| Removed or withdrew from study | 5 | 6 | 11 |
| Total subjects used in analysis | 17 | 17 | 34 |

FIG. 14A

Characteristics of Study Samples

| | Control Subjects | MCI Subjects | p |
|---|---|---|---|
| N | 17 | 17 | |
| Sex (M/F) | 9/8 | 6/11 | 0.307 |
| Age (yrs) | 69.3 (7.0) | 72.9 (8.9) | 0.201 |
| Education (yrs) | 15.9 (2.6) | 15.8 (2.9) | 0.951 |
| Race (Caucasian/African American) | 17/0 | 14/3 | 0.074 |
| Hispanic or Latino (y/n) | 0/17 | 1/16 | 0.317 |

FIG. 14B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 12580464 B
- US 2009005647 W
- US 61105847 A
- US 61152631 A
- US 61175536 A
- WO 2010144712 A
- WO 2010002869 A
- WO 2008132139 A
- WO 2007065595 A
- WO 2006128693 A
- WO 2006128692 A
- WO 2005054188 A
- WO 2004087658 A
- WO 2002094787 A
- WO 2001062726 A
- US 7465549 B
- US 7244747 B
- US 5334720 A
- US 4696943 A
- US 4696942 A
- US 20090312333 A
- US 20090018148 A
- US 20080081832 A
- US 2006258704 A
- GB 1039113 A
- GB 1309692 A
- WO 2010057088 A
- WO 2009008769 A
- WO 2008097546 A
- WO 2008074896 A
- WO 2008073452 A
- WO 2007127474 A
- WO 2007107846 A
- WO 2006097588 A
- WO 2006071274 A
- WO 2006070394 A
- WO 2006060082 A
- WO 2006040688 A
- WO 2005092009 A
- WO 2005079789 A
- WO 2005074535 A
- WO 2005072713 A
- WO 2005042475 A
- WO 2005039580 A
- WO 2005027975 A
- WO 2004084884 A
- WO 2004080393 A
- WO 2004052348 A
- WO 2004037234 A
- WO 2004034963 A
- WO 2004032929 A
- WO 2003101458 A
- WO 2003091220 A
- WO 2003082820 A
- WO 2003082794 A
- WO 2003020289 A
- WO 2002074293 A
- WO 2002032412 A
- WO 2001085145 A
- WO 2001078728 A
- WO 2001066114 A
- WO 2001066096 A
- WO 2001021590 A
- WO 2001000215 A
- WO 2000033840 A
- WO 2000030446 A
- WO 2000023057 A
- WO 2000015205 A
- WO 2000009483 A
- WO 2000007600 A
- WO 2000002549 A
- WO 1999047131 A
- WO 1999008672 A
- WO 1999007359 A
- WO 1998039000 A
- WO 1998030243 A
- WO 1997138993 A
- WO 1997119059 A
- WO 1997038993 A
- WO 1997029750 A
- WO 1997021681 A
- WO 1997013754 A
- WO 1997008146 A
- WO 1996040682 A
- WO 1994029255 A
- WO 1994020476 A
- WO 1994019356 A
- WO 1993116690 A
- WO 1993113100 A
- WO 1993007140 A
- WO 1993003041 A
- WO 1993003034 A
- WO 1992019238 A
- WO 1992017475 A
- WO 1991003467 A
- WO 1988008708 A
- US 7846930 B
- US 7732162 B

- US 7635709 B
- US 7378425 B
- US 6495700 B
- US 6479523 B
- US 6372760 B
- US 6245911 B
- US 6140321 A
- US 5985864 A
- US 5965571 A
- US 5965569 A
- US 5750542 A
- US 5744476 A
- US 5693668 A
- US 5668117 A
- US 5663448 A
- US 5622976 A
- US 5603176 A
- US 5602176 A
- US 5574046 A
- US 5455245 A
- US 5391553 A
- US 5389629 A
- US 5364864 A
- US 5338548 A
- US 5302593 A
- US 5300517 A
- US 5288758 A
- US 5246947 A
- US 5231093 A
- US 5187165 A
- US 5166181 A
- US 5106856 A
- US 5102891 A
- US 5100901 A
- US 4950658 A
- US 4948807 A
- US 4914102 A
- US 4895841 A
- US 4816456 A
- US 4663318 A
- US 2701225 A
- JP 4216704 A
- JP 4187674 A
- CA 2180703
- EP 298202 A
- EP 236684 A
- EP 409676 A
- EP 411534 A
- EP 468187 A
- EP 477903 A
- EP 481429 A
- EP 487071 A
- EP 611769 A
- EP 703901 A
- EP 1050303 A
- EP 2018874 A
- EP 2260839 A
- US 20010036949 A
- US 20020119963 A
- US 20030078252 A
- US 2003069289 A
- US 20040082644 A
- US 20050245504 A
- US 20050124642 A
- US 20060052428 A
- US 20070275959 A
- US 20080103105 A
- US 20080261950 A
- US 20090124659 A
- US 20100152108 A
- US 20100227852 A
- US 20100311697 A
- US 580464 A
- WO 2007019312 A
- WO 2004048551 A
- WO 200162726 A
- US 5468733 A
- US 5516759 A
- EP 0205503 W
- JP 2733203 B
- JP 2000319257 A
- JP 2000319258 A
- JP 2001139547 A
- WO 8808708 A

**Non-patent literature cited in the description**


- **SANSONE et al.** *Pharmacol. Biochem. Behav.,* 1993, vol. 44 (2), 451-455
- **HELMSTAEDTER et al.** *Epilepsy & Behavior,* 2008, vol. 13 (4), 642-649
- Principles of Neural Science. McGraw-Hill Medical, 2000
- **MOTULSKY.** Intuitive Biostatistics. Oxford University Press, Inc, 1995
- **LODISH et al.** Molecular Cell Biology. W. H. Freeman & Co, 2000
- **GRIFFITHS et al.** Introduction to Genetic Analysis. W. H. Freeman & Co, 1999
- **GILBERT et al.** Developmental Biology. Sinauer Associates, Inc, 2000
- The McGraw-Hill Dictionary of Chemical Terms. McGraw-Hill, 1985
- **FOLSTEIN et al.** *J Psychiatric Res,* 1975, vol. 12, 189-98
- **ROBBINS et al.** *Dementia,* 1994, vol. 5, 266-81
- **REY.** *L'examen clinique en psychologie,* 1964
- **KLUGER et al.** *J Geriatr Psychiatry Neurol,* 1999, vol. 12, 168-79

- **PETERSEN et al.** *Srch. Neurol.,* 1999, vol. 56, 303-308
- **PETERSEN.** Mild cognitive impairment: Aging to Alzheimer's Disease. Oxford University Press, 2003
- **KLUGER et al.** *J Geriatric Psychiatry Neurol,* 1999, vol. 12, 168-79
- **CHAPPELL et al.** *Neuropharmacology,* 1998, vol. 37, 481-487
- **DE HOZ et al.** *Eur. J. Neurosci.,* 2005, vol. 22, 745-54
- **STEELE ; MORRIS.** *Hippocampus,* 1999, vol. 9, 118-36
- **MRI. AISEN et al.** *Alzheimer's & Dementia,* 2010, vol. 6, 239-246
- **YASSA et al.** *PNAS,* 2010, vol. 107, 12687-12691
- **AISEN et al.** *Alzheimer's & Dementia,* 2010, vol. 6, 239-246
- **HERHOLZ et al.** *NeuroImage,* 2002, vol. 17, 302-316
- **GALLAGHER et al.** *Behav. Neurosci.,* 1993, vol. 107, 618-626
- **COLOMBO et al.** *Proc. Natl. Acad. Sci.,* 1997, vol. 94, 14195-14199
- **GALLAGHER ; BURWELL.** *Neurobiol. Aging,* 1989, vol. 10, 691-708
- **RAPP ; GALLAGHER.** *Proc. Natl. Acad. Sci.,* 1996, vol. 93, 9926-9930
- **NICOLLE et al.** *Neuroscience,* 1996, vol. 74, 741-756
- **NICOLLE et al.** *J. Neurosci.,* 1999, vol. 19, 9604-9610
- **SANKARANARAYANAN.** *Curr. Top. Medicinal Chem.,* 2006, vol. 6, 609-627
- **KOBAYASHI et al.** *Genes Brain Behav.,* 2005, vol. 4, 173-196
- **ASHE ; ZAHNS.** *Neuron.,* 2010, vol. 66, 631-45
- **WOON et al.** *Prog. Neuro-Psychopharm. & Biological Psych.,* vol. 34, 1181-1188
- **WANG et al.** *Arch. Gen. Psychiatry,* 2010, vol. 67, 296-303
- **YEHUDA et al.** *Bio. Psych.,* 2006, vol. 60, 714-721
- **YAFFE et al.** *Arch. Gen. Psych.,* 2010, vol. 678, 608-613
- **LIBERZON et al.** *Psychoneuroendocrinology,* 1997, vol. 22, 443-453
- **HARVERY et al.** *Psychopharmacology,* 2004, vol. 175, 494-502
- **KHAN ; LIBERZON.** *Psychopharmacology,* 2004, vol. 172, 225-229
- **GUIDOTTI et al.** *Psychopharmacology,* 2005, vol. 180, 191-205
- **ZIERHUT.** *Psych. Res. Neuroimag.,* 2010, vol. 183, 187-194
- **WOOD et al.** *NeuroImage,* 2010, vol. 52, 62-63
- **VINKERS et al.** *Expert Opin. Investig. Drugs,* 2009, vol. 19, 1217-1233
- **YOUNG et al.** *Pharmacol. Ther.,* 2009, vol. 122, 150-202
- **TREMONLIZZO et al.** *PNAS,* 2002, vol. 99, 17095-17100
- **LODGE et al.** *J. Neurosci.,* 2009, vol. 29, 2344-2354
- **DIETRICH et al.** *Oncologist,* 2008, vol. 13, 1285-95
- **SOUSSAIN et al.** *Lancet,* 2009, vol. 374, 1639-51
- **KIM et al.** *J. Radiat. Res.,* 2008, vol. 49, 517-526
- **YANG et al.** *Neurobiol. Learning and Mem.,* 2010, vol. 93, 487-494
- **KIM et al.** *J. Radiant. Res.,* 2008, vol. 49, 517-526
- **BAJJALIEH et al.** *Science,* 1992, vol. 257, 1271-1273
- **A. PADWA et al.** *J. Org. Chem.,* 2000, vol. 65, 5223-5232
- *Pure Appl. Chem.,* 1976, vol. 45, 11-30
- **T. HIGUCHI ; V. STELLA.** Pro-drugs as Novel Delivery System. *A.C.S. Symposium Series,* vol. 14
- Bioreversible Carriers in Drug Design. American Pharmaceutical Association and Pergamon Press, 1987
- **BRUFANI et al.** Alzheimer Disease: From Molecular Biology to Therapy. 1996, 171-177
- **SCHMIDT et al.** Alzheimer Disease: From Molecular Biology to Therapy. 1996, 217-221
- **VARGAS et al.** Alzheimer Disease: From Molecular Biology to Therapy. 1996, 261-255
- **GREIG et al.** Alzheimer Disease: From Molecular Biology to Therapy. 1996, 231-237
- Giacobini, Alzheimer Disease: From Molecular Biology to Therapy. 1996, 187-204
- **BORES ; KOSLEY.** *Drugs of the Future,* 1996, vol. 21, 621-631
- **KOH et al.** *Neuropsychopharmacology,* 2009
- Mild cognitive impairment: Aging to Alzheimer's Disease. Oxford University Press, 2003
- **STARK ; SQUIRE.** *PNAS,* 2001